(19) Europäisches Patentamt
European Patent Office
Office européen des brevets

(11) **EP 4 434 992 A1**

(12) **EUROPEAN PATENT APPLICATION**
published in accordance with Art. 153(4) EPC

(43) Date of publication:
**25.09.2024 Bulletin 2024/39**

(21) Application number: **22894936.8**

(22) Date of filing: **18.11.2022**

(51) International Patent Classification (IPC):
***C07D 498/04*** (2006.01) ***C07D 498/00*** (2006.01)
***C07D 491/04*** (2006.01) ***A61K 31/423*** (2006.01)
***A61K 31/343*** (2006.01) ***A61K 31/4523*** (2006.01)
***A61K 31/4525*** (2006.01) ***A61P 35/00*** (2006.01)
***A61P 37/00*** (2006.01)

(52) Cooperative Patent Classification (CPC):
**A61K 31/343; A61K 31/423; A61K 31/4523; A61K 31/4525; A61P 35/00; A61P 37/00; C07D 491/04; C07D 498/00; C07D 498/04**

(86) International application number:
**PCT/CN2022/132769**

(87) International publication number:
**WO 2023/088406 (25.05.2023 Gazette 2023/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **18.11.2021 CN 202111371514**
**13.07.2022 CN 202210824373**
**04.11.2022 CN 202211378992**
**11.11.2022 CN 202211413934**

(71) Applicant: **Chia Tai Tianqing Pharmaceutical Group Co., Ltd.**
**Lianyungang, Jiangsu 222062 (CN)**

(72) Inventors:
- **ZHANG, Yinsheng**
  **Lianyungang, Jiangsu 222062 (CN)**
- **REN, Jing**
  **Lianyungang, Jiangsu 222062 (CN)**
- **WANG, Jinan**
  **Lianyungang, Jiangsu 222062 (CN)**
- **XU, Sheng**
  **Lianyungang, Jiangsu 222062 (CN)**
- **YANG, Xiaojun**
  **Lianyungang, Jiangsu 222062 (CN)**
- **DENG, Li**
  **Lianyungang, Jiangsu 222062 (CN)**
- **HUANG, Yongkang**
  **Lianyungang, Jiangsu 222062 (CN)**
- **WANG, Qinglin**
  **Lianyungang, Jiangsu 222062 (CN)**

(74) Representative: **Müller Schupfner & Partner Patent- und Rechtsanwaltspartnerschaft mbB (Muc)**
**Bavariaring 11**
**80336 München (DE)**

(54) **FUSED IMIDE DERIVATIVE**

(57) Provided are a fused imide derivative as shown in formula I, a preparation method therefor, a pharmaceutical composition containing same, and the use thereof in the treatment of relevant diseases (such as cancers).

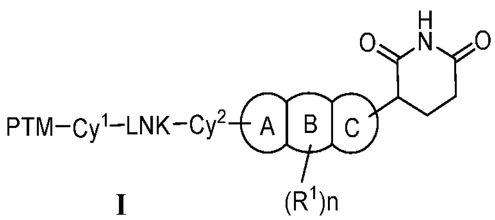

**I**

EP 4 434 992 A1

## Description

### CROSS-REFERENCE TO RELATED APPLICATIONS

The present application is an international application of Chinese Patent Application Nos. 202111371514.4, 202111667477.1, 202210824373.5, 202211261799.0, 202211378992.2, and 202211413934.9 filed on November 18, 2021, December 31, 2021, July 13, 2022, October 14, 2022, November 4, 2022, and November 11, 2022, respectively, and claims the priority and benefits of the Chinese Patent Applications. The disclosures of the Chinese Patent Applications are incorporated herein by reference in their entirety.

### TECHNICAL FIELD

The present application relates to a fused imide derivative and protein degrader, a preparation method therefor, a pharmaceutical composition comprising the same, and use thereof in treating a related disease (such as cancer).

### BACKGROUND

Bruton's tyrosine kinase (BTK) is mainly expressed in B cells, distributed in the lymphatic, hematopoietic and blood systems, and is a member of the non-receptor type tyrosine kinase Tec family, which also includes TEC, ITK/TSK/EMT, TXK and BMX, which have high structural homology. In recent years, researches on B cells, particularly on B cell non-Hodgkin lymphoma and rheumatoid arthritis find that BTK is often abnormally expressed. Since BTK is mainly expressed in B cells and marrow cells, BTK is a target with relatively good targeting property and safety.

Proteolysis targeting chimera (Protac) molecule is a bifunctional compound capable of binding to a target protein and E3 ubiquitin ligase simultaneously. Such compounds can induce the target protein to be recognized by proteasomes of cells, cause the degradation of the target protein and effectively reduce the content of the target protein in the cells. The introduction of ligands capable of binding different target proteins into Protac molecules has made it possible to apply Protac technology to the treatment of various diseases, and this technology has also received much attention in recent years.

### SUMMARY

In one aspect, the present application relates to a compound of formula I, a stereoisomer thereof or a pharmaceutically acceptable salt thereof:

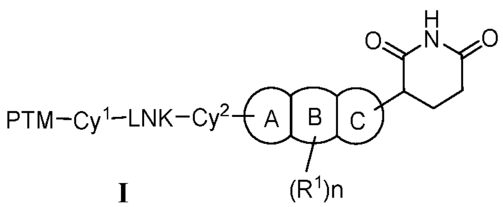

**I**

wherein,

ring A is absent or selected from the group consisting of $C_{5-10}$ cycloalkenyl, 5- to 10-membered heterocycloalkenyl, phenyl, and 5- to 6-membered heteroaryl, or
ring A is absent or selected from the group consisting of $C_{5-6}$ cycloalkenyl, 5- to 10-membered heterocycloalkenyl, phenyl, and 5- to 6-membered heteroaryl;
ring B is selected from phenyl;
ring C is selected from the group consisting of isoxazolyl and furanyl;
each R' is independently selected from the group consisting of halogen, -OH, $-NH_2$, -CN, $C_{1-4}$ alkyl, $C_{1-4}$ alkoxy, and $C_{1-4}$ haloalkyl;
n is selected from the group consisting of 0, 1, 2, and 3;
$Cy^1$ is selected from the group consisting of a bond, $C_{3-12}$ cycloalkyl, and 4- to 12-membered heterocycloalkyl, wherein the $C_{3-12}$ cycloalkyl or 4- to 12-membered heterocycloalkyl is optionally substituted with one or more $R^a$;
LNK is selected from the group consisting of a bond, $C_{1-12}$ alkylene, and $C_{1-12}$ heteroalkylene;
$Cy^2$ is absent or selected from the group consisting of $C_{3-12}$ cycloalkyl and 4- to 12-membered heterocycloalkyl, wherein the cycloalkyl or heterocycloalkyl is optionally substituted with one or more $R^b$;

each $R^a$ and each $R^b$ are independently selected from the group consisting of halogen, -OH, $-NH_2$, -CN, $C_{1-4}$ alkyl, $C_{1-4}$ alkoxy, $C_{1-4}$ haloalkyl, $C_{1-4}$ alkylamino, and di-$C_{1-4}$ alkylamino;
PTM is selected from the group consisting of drugs and derivatives thereof that bind to a target protein.

In some embodiments, the present application relates to a compound of formula I, a stereoisomer thereof, or a pharmaceutically acceptable salt thereof,

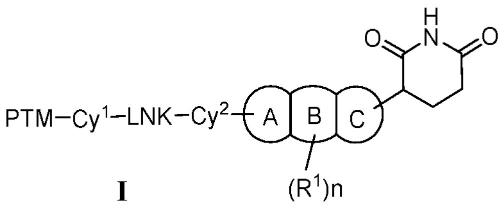

**I**

wherein,

ring A is absent or selected from the group consisting of $C_{5-6}$ cycloalkenyl, 5- to 10-membered heterocycloalkenyl, phenyl, and 5- to 6-membered heteroaryl;
ring B is selected from phenyl;
ring C is selected from the group consisting of isoxazolyl and furanyl;
each R’ is independently selected from the group consisting of halogen, -OH, $-NH_2$, -CN, $C_{1-4}$ alkyl, $C_{1-4}$ alkoxy, and $C_{1-4}$ haloalkyl;
n is selected from the group consisting of 0, 1, 2, and 3;
$Cy^1$ is selected from the group consisting of a bond, $C_{3-12}$ cycloalkyl, and 4- to 12-membered heterocycloalkyl, wherein the $C_{3-12}$ cycloalkyl or 4- to 12-membered heterocycloalkyl is optionally substituted with one or more $R^a$;
LNK is selected from the group consisting of a bond, $C_{1-12}$ alkylene, and $C_{1-12}$ heteroalkylene;
$Cy^2$ is absent or selected from the group consisting of $C_{3-12}$ cycloalkyl and 4- to 12-membered heterocycloalkyl, wherein the cycloalkyl or heterocycloalkyl is optionally substituted with one or more $R^b$;
each $R^a$ and each $R^b$ are independently selected from the group consisting of halogen, -OH, $-NH_2$, -CN, $C_{1-4}$ alkyl, $C_{1-4}$ alkoxy, $C_{1-4}$ haloalkyl, $C_{1-4}$ alkylamino, and di-$C_{1-4}$ alkylamino;
PTM is selected from the group consisting of drugs and derivatives thereof that bind to a target protein.

In some embodiments, in the compound of formula I, the stereoisomer thereof, or the pharmaceutically acceptable salt thereof, the structural moiety

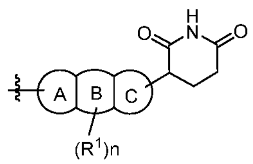

is selected from the group consisting of

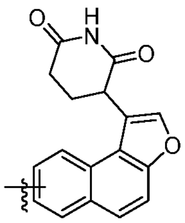 , 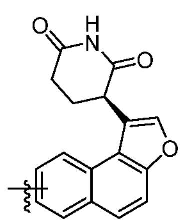 , 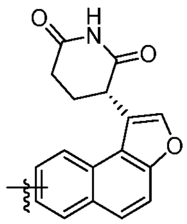 , 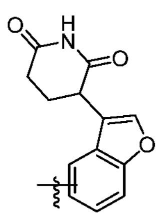 , 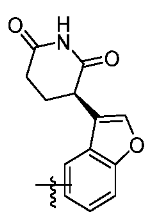 , and 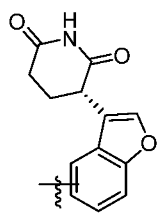 ,

and PTM is not selected from the group consisting of the following structural moieties:

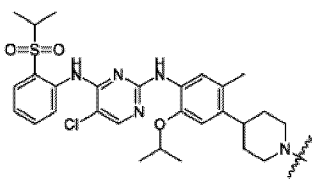
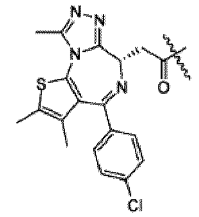
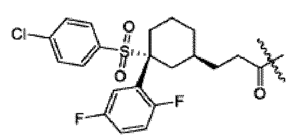

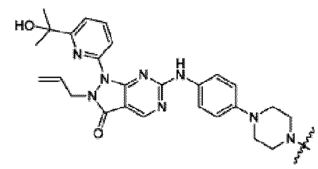
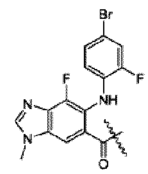

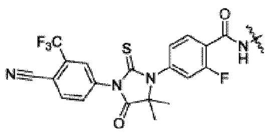
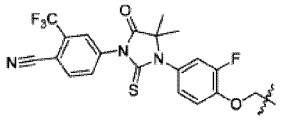
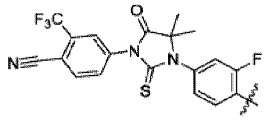

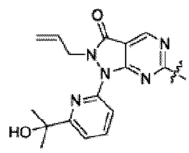
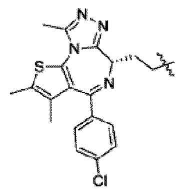
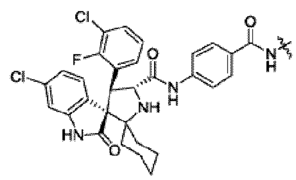

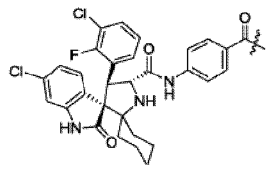
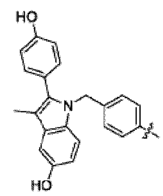
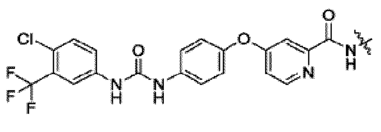

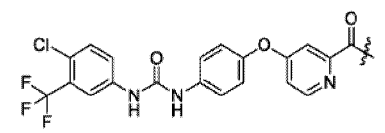
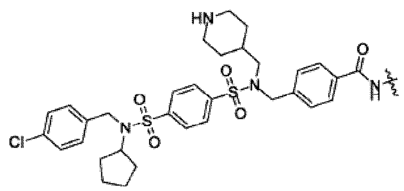

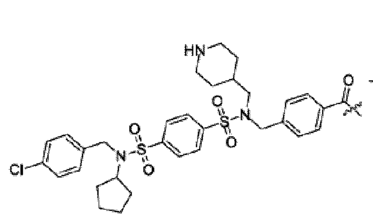
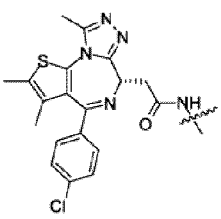
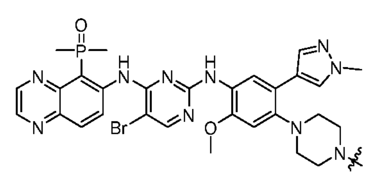

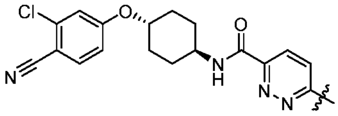
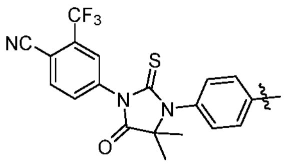
, or
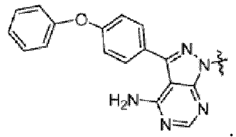
.

In some other embodiments, $Cy^1$ is selected from the group consisting of 4- to 12-membered heterocycloalkyl optionally substituted with one or more $R^a$.

In some embodiments of the present application, when ring A is present, $Cy^2$ or LNK when $Cy^2$ is absent may be directly covalently linked to ring B; likewise,

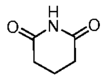

may also be directly covalently linked to ring B.

In some embodiments, PTM described herein is selected from the group consisting of drugs and derivatives thereof that act on AR, ER, kinase, phosphatase, MDM2, human BET bromodomain protein, Hsp90, HDAC, human lysine methyltransferase, RAF receptor, FKBP, angiogenic factor, immunosuppression-related receptor or protein, arene receptor, thyroid hormone receptor, HIV protease, HIV integrase, HCV protease, HBV protease, or acyl protein thioesterase 1 and/or acyl protein thioesterase 2.

In some embodiments, PTM described herein is selected from the group consisting of drugs and derivatives thereof that act on ALK, BET, CDK, PARP, EGFR, γ-secretase, CBFβ-SMMHC, WEE1, MEK, BCR-ABL, MET, RAS, BTK, VEGFR, JAK, HER2, HDAC, Akt, PI3K, mTOR, AR, ER, PDEδ, SRC, MDM2, RAF, IRAK4, STAT3, and c-Myc.

In some embodiments, PTM described herein is selected from the group consisting of drugs and derivatives thereof that act on ALK, BRD4, CDK4/6, PARP, EGFR, γ-secretase, CBFβ-SMMHC, WEEl, MEK, BCR-ABL, MET, KRAS, EGFR, BTK, AR, ER, PDEδ, JAK, MDM2, or RAF

In some embodiments, PTM described herein is selected from the group consisting of drugs and derivatives thereof that act on BTK or WEE1.

In some embodiments, PTM described herein is selected from the group consisting of drugs and derivatives thereof that act on BTK.

In another aspect, the present application relates to a compound of formula II-1, a stereoisomer thereof, or a pharmaceutically acceptable salt thereof,

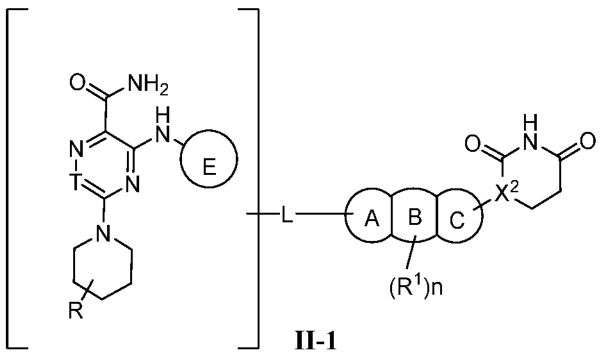

**II-1**

wherein,

- T is selected from the group consisting of CH and N;
- R is selected from the group consisting of hydrogen and 5- to 6-membered heterocycloalkyl, wherein the heterocycloalkyl is optionally substituted with =O or $C_{1-6}$ alkyl;
- ring E is selected from the group consisting of phenyl, benzocycloalkenyl (e.g., benzo 4- to 12-membered cycloalkenyl, or benzo 4- to 10-membered cycloalkenyl, or benzo 4- to 8-membered cycloalkenyl), and benzoheterocycloalkenyl (e.g., benzo 4- to 12-membered heterocycloalkenyl or benzo 5- to 11-membered heterocycloalkenyl);
- $X^2$ is selected from the group consisting of CH and N;
- L is selected from a connecting group;

ring A, ring B, ring C, $R^1$, and n are as defined herein.

In another aspect, the present application relates to a compound of formula II, a stereoisomer thereof, or a pharmaceutically acceptable salt thereof,

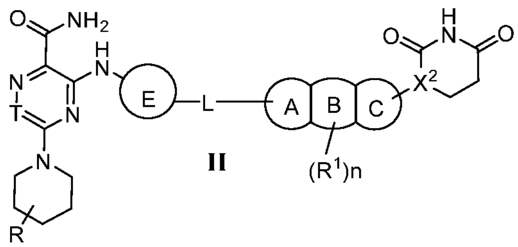

II

wherein,

T is selected from the group consisting of CH and N;
R is selected from the group consisting of hydrogen and 5- to 6-membered heterocycloalkyl, wherein the heterocycloalkyl is optionally substituted with =O or $C_{1-6}$ alkyl;
ring E is selected from the group consisting of phenyl, benzocycloalkenyl, and benzoheterocycloalkenyl;
$X^2$ is selected from the group consisting of CH and N;
L is selected from a connecting group;
ring A, ring B, ring C, $R^1$, and n are as defined herein.

In some embodiments, L is selected from the group consisting of -$Cy^1$-LNK-$Cy^2$-LNK-, -$Cy^1$-LNK-$Cy^2$-, and -$Cy^1$-$Cy^2$-LNK-, wherein $Cy^1$, LNK, and $Cy^2$ are as described herein. In some embodiments, L is selected from -$Cy^1$-LNK-$Cy^2$-, wherein $Cy^1$, LNK, and $Cy^2$ are as described herein.

In another aspect, the present application relates to a compound of formula I'a, a stereoisomer thereof, or a pharmaceutically acceptable salt thereof,

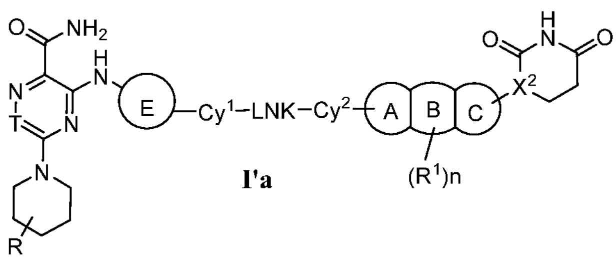

I'a

wherein,

T is selected from the group consisting of CH and N;
R is selected from the group consisting of hydrogen and 5- to 6-membered heterocycloalkyl, wherein the heterocycloalkyl is optionally substituted with =O or $C_{1-6}$ alkyl;
ring E is selected from the group consisting of phenyl, benzocycloalkenyl, and benzoheterocycloalkenyl;
$X^2$ is selected from the group consisting of CH and N;
ring A, ring B, ring C, $R^1$, n, $Cy^1$, LNK, and $Cy^2$ are as defined herein.

In some embodiments, $X^2$ is selected from CH; in some embodiments, $X^2$ is selected from N.

In some embodiments, ring E is selected from the group consisting of phenyl, benzo $C_{5-12}$ cycloalkenyl, and benzo 5- to 12-membered heterocycloalkenyl.

In some embodiments, ring E is selected from the group consisting of phenyl, benzo $C_{5-6}$ cycloalkenyl, and benzo 5- to 11-membered heterocycloalkenyl.

In some embodiments, ring E is selected from the group consisting of phenyl, benzo 5-membered heterocycloalkenyl, benzo 6-membered heterocycloalkenyl, benzo 10-membered heterocycloalkenyl, and benzo 11-membered heterocycloalkenyl.

In some embodiments, ring E is selected from the group consisting of phenyl,

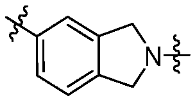

,

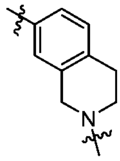

,

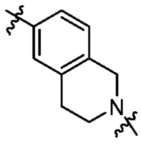

, 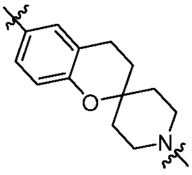

, and 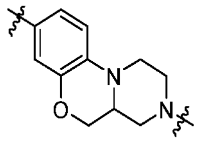

.

In some specific embodiments, ring E is selected from phenyl. In some specific embodiments, ring E is selected from the group consisting of

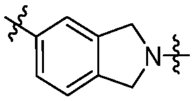

,

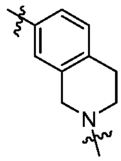

, 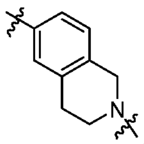

, 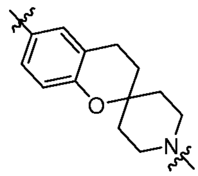

, and 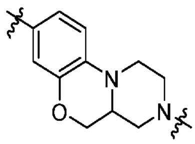

.

In another aspect, the compound of formula I, the stereoisomer thereof, or the pharmaceutically acceptable salt thereof described herein is selected from the group consisting of a compound of formula I', a stereoisomer thereof, and a pharmaceutically acceptable salt thereof,

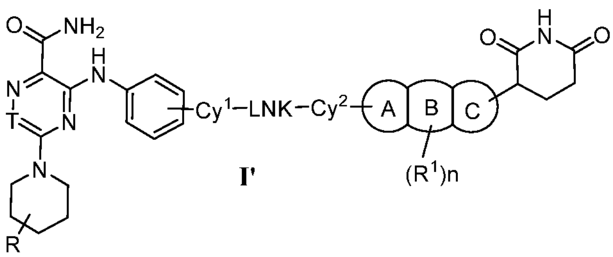

I'

wherein,

T is selected from the group consisting of CH and N;

R is selected from the group consisting of hydrogen and 5- to 6-membered heterocycloalkyl, wherein the heterocycloalkyl is optionally substituted with =O or $C_{1-6}$ alkyl.

In some embodiments, ring A is absent or selected from the group consisting of $C_{5-10}$ cycloalkenyl, 5- to 10-membered heterocycloalkenyl, phenyl, and 5- to 6-membered heteroaryl.

In some embodiments, ring A is absent or selected from the group consisting of $C_{5-8}$ cycloalkenyl, 5- to 10-membered heterocycloalkenyl, phenyl, and 5- to 6-membered heteroaryl.

In some embodiments, ring A is absent or selected from the group consisting of $C_{5-7}$ cycloalkenyl, 5- to 10-membered heterocycloalkenyl, phenyl, and 5- to 6-membered heteroaryl.

In some embodiments, ring A is absent or selected from the group consisting of $C_{5-6}$ cycloalkenyl, 5- to 10-membered heterocycloalkenyl, phenyl, and 5- to 6-membered heteroaryl.

In some embodiments, ring A is absent or selected from the group consisting of $C_{5-6}$ cycloalkenyl, 5- to 9-membered heterocycloalkenyl, phenyl, and 5-membered heteroaryl.

In some embodiments, ring A is absent or selected from the group consisting of $C_{5-6}$ cycloalkenyl, 5- to 9-membered heterocycloalkenyl, phenyl, pyrrolyl, pyrazolyl, furanyl, and oxazolyl.

In some embodiments, ring A is absent or selected from the group consisting of $C_5$ cycloalkenyl, $C_6$ cycloalkenyl, 5-, 6-, 7-, 8-, or 9-membered heterocycloalkenyl, phenyl, pyrrolyl, pyrazolyl, furanyl, and oxazolyl.

In some embodiments, ring A is absent or selected from the group consisting of cyclopentenyl, bicyclohexenyl, dihydropyrrolyl, tetrahydropyridinyl, tetrahydroazepinyl, dihydrooxazinyl, azaspirooctenyl, azaspirononenyl, phenyl, pyrrolyl, pyrazolyl, furanyl, and oxazolyl.

In some specific embodiments, ring A is selected from the group consisting of $C_{5-10}$ cycloalkenyl and 5- to 10-membered heterocycloalkenyl. In some specific embodiments, ring A is selected from the group consisting of $C_{5-8}$ cycloalkenyl and 5- to 9-membered heterocycloalkenyl. In some specific embodiments, ring A is selected from the group consisting of $C_{5-7}$ cycloalkenyl and 5- to 9-membered heterocycloalkenyl. In some specific embodiments, ring A is selected from the group consisting of $C_{5-6}$ cycloalkenyl and 5- to 9-membered heterocycloalkenyl. In some specific embodiments, ring A is selected from 5- to 9-membered heterocycloalkenyl. In some specific embodiments, ring A is selected from 5- to 8-membered heterocycloalkenyl. In some specific embodiments, ring A is selected from 5-membered heterocycloalkenyl. In some specific embodiments, ring A is selected from 6-membered heterocycloalkenyl. In some specific embodiments, ring A is selected from 7-membered heterocycloalkenyl. In some specific embodiments, ring A is selected from 8-membered heterocycloalkenyl. In some specific embodiments, ring A is selected from 9-membered heterocycloalkenyl. In some specific embodiments, ring A is absent or selected from the group consisting of $C_{5-6}$ cycloalkenyl, 5- to 9-membered heterocycloalkenyl (preferably 5- to 8-membered heterocycloalkenyl or 5- to 7-membered heterocycloalkenyl), and phenyl. In some preferred embodiments, ring A is absent or selected from the group consisting of $C_{5-6}$ cycloalkenyl, 5- to 7-membered heterocycloalkenyl containing 1-2 heteroatoms selected from the group consisting of N, O, and S (preferably containing 1-2 N atoms, e.g., 1 N atom), and phenyl. In some specific embodiments, ring A is absent or selected from the group consisting of cyclopentenyl, dihydropyrrolyl, tetrahydropyridinyl, tetrahydroazepinyl, and phenyl.

In some specific embodiments, ring A is selected from the group consisting of cyclopentenyl, bicyclohexenyl, dihydropyrrolyl, tetrahydropyridinyl, tetrahydroazepinyl, dihydrooxazinyl, azaspirooctenyl, and azaspirononenyl.

In some specific embodiments, ring A is selected from the group consisting of cyclopentenyl and bicyclohexenyl.

In some specific embodiments, ring A is selected from the group consisting of dihydropyrrolyl, dihydrooxazinyl, tetrahydropyridinyl, tetrahydroazepinyl, azaspirooctenyl, and azaspirononenyl.

In some specific embodiments, ring A is selected from the group consisting of phenyl, pyrrolyl, pyrazolyl, furanyl, and oxazolyl.

In some embodiments, ring A is absent or selected from the group consisting of $C_{5-7}$ cycloalkenyl, 5- to 8-membered heterocycloalkenyl, phenyl, and pyrrolyl.

In some other embodiments, ring A is absent or selected from the group consisting of $C_{5-6}$ cycloalkenyl, 5- to 8-membered heterocycloalkenyl, phenyl, and pyrrolyl.

In some other embodiments, ring A is absent or selected from the group consisting of $C_5$ cycloalkenyl, 5-, 6-, 7-, or 8-membered heterocycloalkenyl, phenyl, and pyrrolyl.

In some other embodiments, ring A is absent or selected from the group consisting of cyclopentenyl, dihydropyrrolyl, tetrahydropyridinyl, tetrahydroazepinyl, azaspirooctene, phenyl, and pyrrolyl.

In some other embodiments, ring A is absent.

In some other embodiments, ring A is selected from the group consisting of $C_{5-6}$ cycloalkenyl and 5- to 8-membered heterocycloalkenyl.

In some other embodiments, ring A is selected from the group consisting of cyclopentenyl, dihydropyrrolyl, tetrahydropyridinyl, tetrahydroazepinyl, and azaspirooctene. In some other embodiments, ring A is selected from cyclopentenyl. In some other embodiments, ring A is selected from the group consisting of dihydropyrrolyl, tetrahydropyridinyl, tetrahydroazepinyl, and azaspirooctene.

In some other embodiments, ring A is selected from the group consisting of phenyl and pyrrolyl.

In some embodiments, the structural moiety

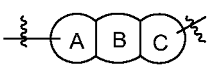

is selected from the group consisting of

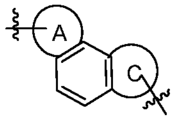 and 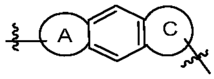.

Alternatively, in some embodiments, the structural moiety

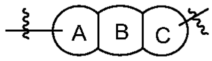

is selected from the group consisting of

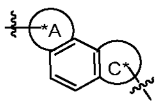, 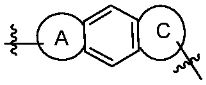, 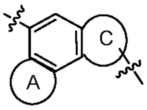,

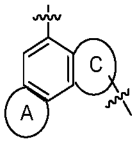, 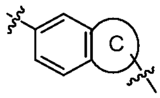, and 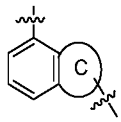,

wherein * in the bond

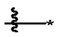

linked to ring A indicates that the bond is linked to an atom on ring A; * in the bond

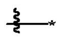

linked to ring C indicates that the bond is linked to an atom on ring C (hereinafter, a similarly positioned * indicates the same or similar meaning).

In some embodiments, the structural moiety

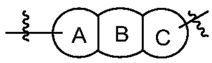

is selected from the group consisting of

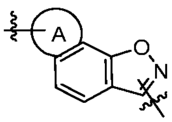, 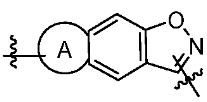, 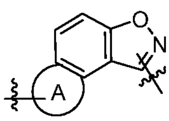, 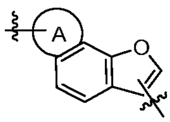, 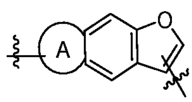, and 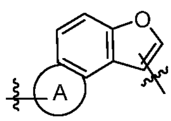.

In some embodiments, the structural moiety

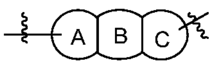

is selected from the group consisting of

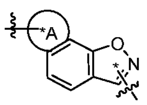, 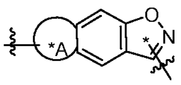, 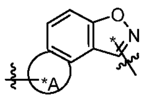, 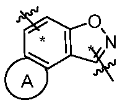 (e.g., 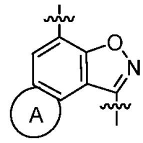 or 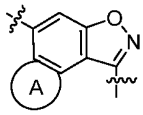), 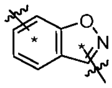

(e.g., 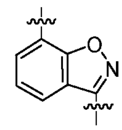, 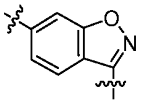, 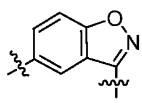, or 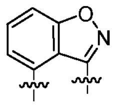), 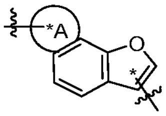, 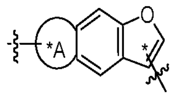,

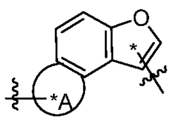, 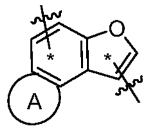, and 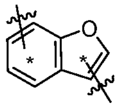,

wherein * is defined in the same or similar way as for * described above.

In some specific embodiments, the structural moiety

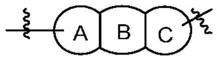

is selected from the group consisting of

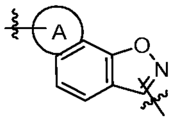 and 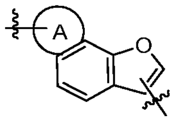.

In some specific embodiments, the structural moiety

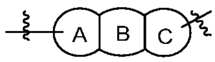

is selected from the group consisting of

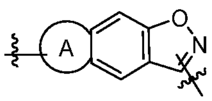

and

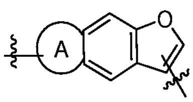.

In some specific embodiments, the structural moiety

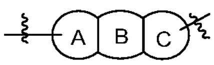

is selected from the group consisting of

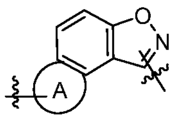

and

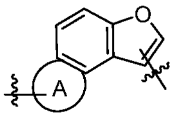.

In some embodiments, the structural moiety

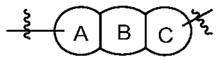

is selected from the group consisting of

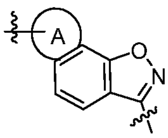, 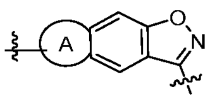, 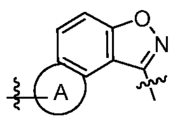, 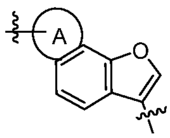, 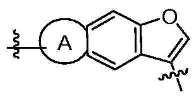, and 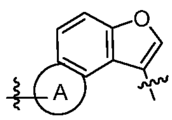.

In some specific embodiments, the structural moiety

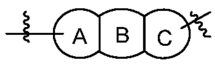

is selected from the group consisting of

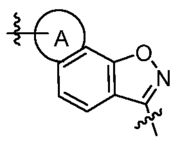 and 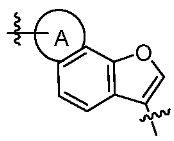.

In some specific embodiments, the structural moiety

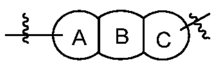

is selected from the group consisting of

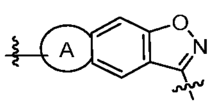 and 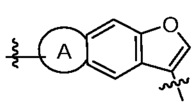.

In some specific embodiments, the structural moiety

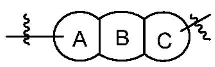

is selected from the group consisting of

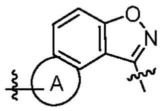

and

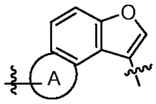.

In some embodiments, the structural moiety

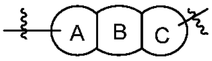

is selected from the group consisting of

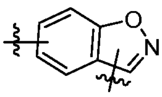, 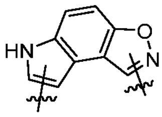, 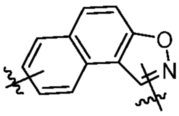, 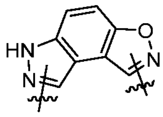, 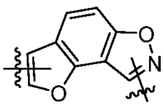, 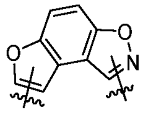,

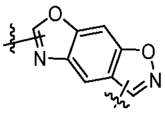, 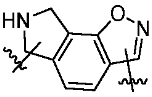, 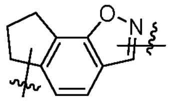, 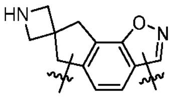, 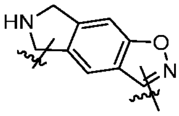, 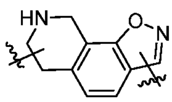,

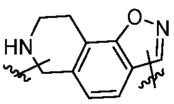, 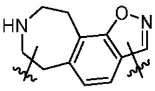, 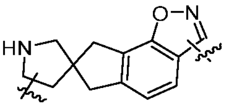, 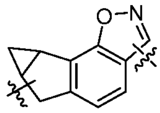, 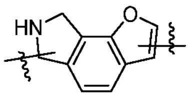, 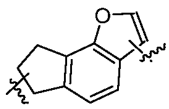,

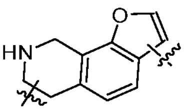, 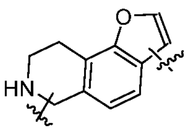, 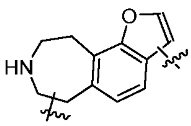, 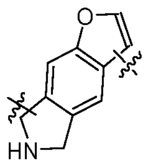, 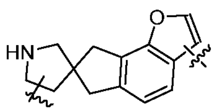, 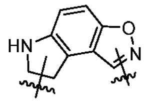,

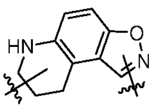, and 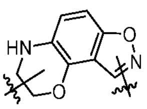.

In some specific embodiments, the structural moiety

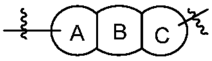

is selected from the group consisting of

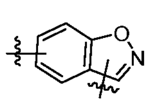, 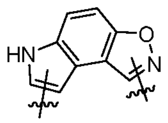, 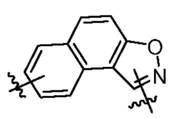, 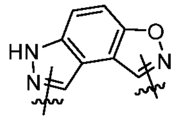, 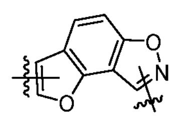, 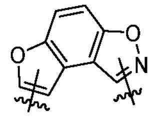,

and

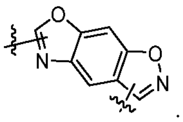.

In some specific embodiments, the structural moiety

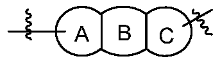

is selected from the group consisting of

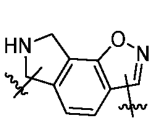, 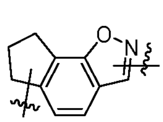, 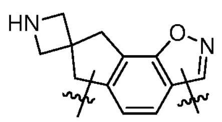, 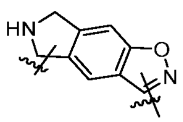, 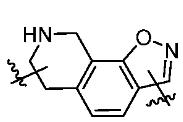, 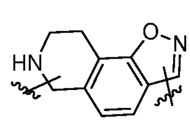,

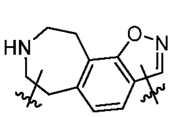, 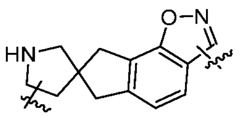, 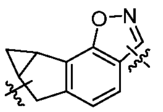, 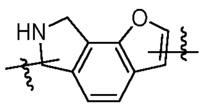, 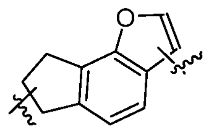,

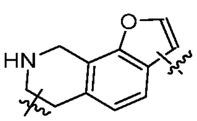, 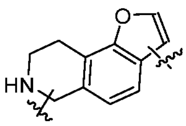, 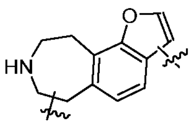, 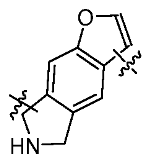, 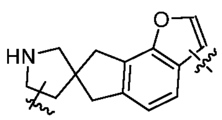, 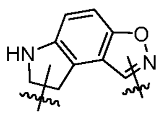,

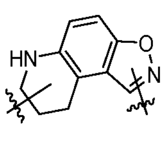, and 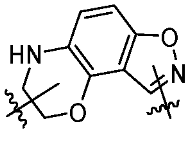.

In some embodiments, the structural moiety

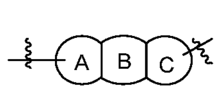

is selected from the group consisting of

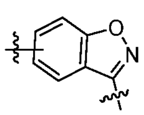, 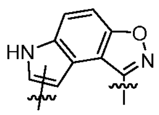, 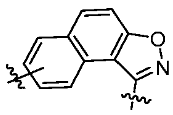, 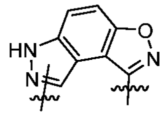, 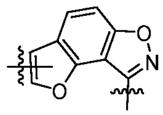, 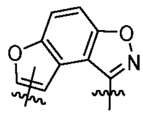, 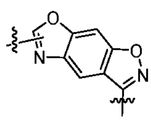,

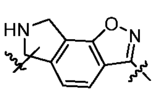, 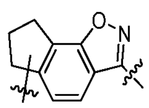, 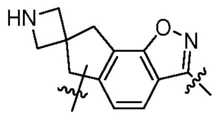, 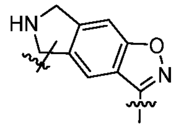, 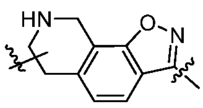, 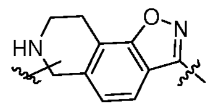,

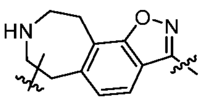, 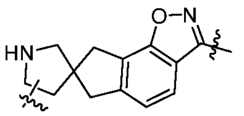, 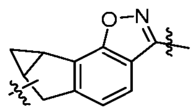, 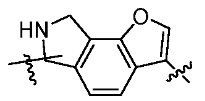, 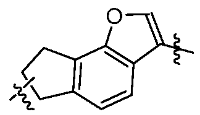,

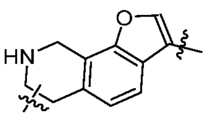, 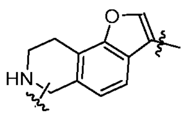, 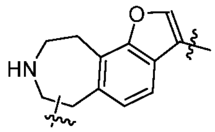, 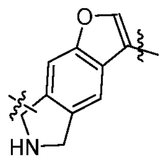, 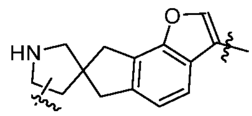,

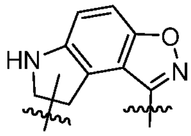, 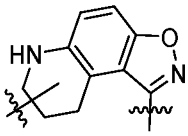, and 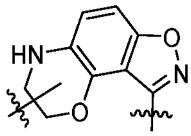.

In some specific embodiments, the structural moiety

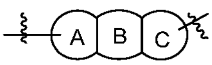

is selected from the group consisting of

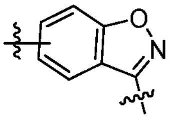, 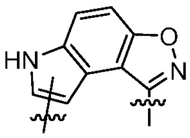 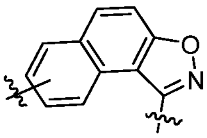, 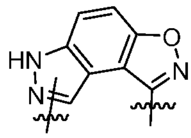, 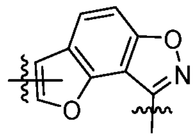, 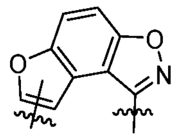,

and

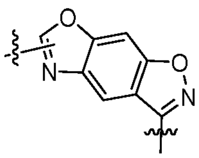.

In some specific embodiments, the structural moiety

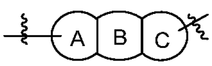

is selected from the group consisting of

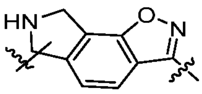, 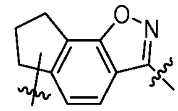, 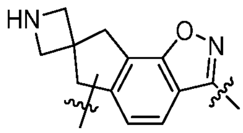, 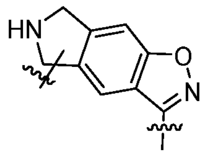, 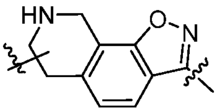,

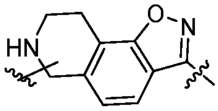, 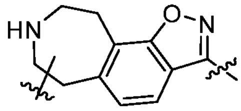, 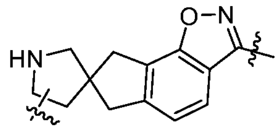, 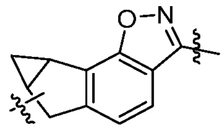,

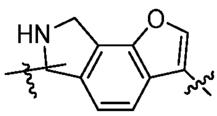, 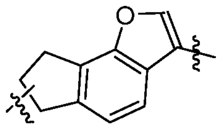, 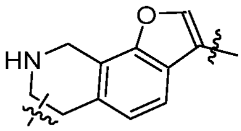, 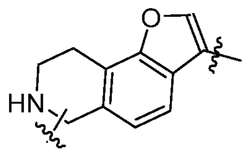,

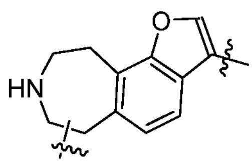, 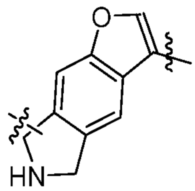, 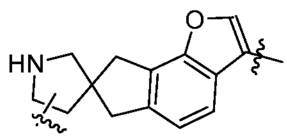, 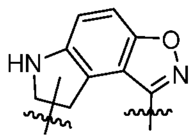, 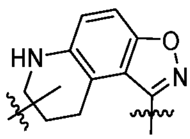,

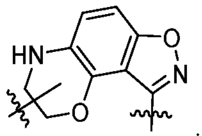.

and

In some embodiments, the structural moiety

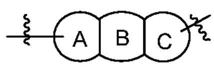

is selected from the group consisting of

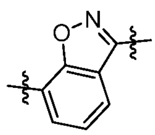, 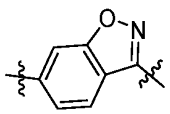, 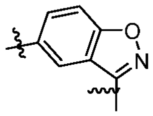, 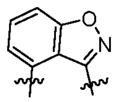, 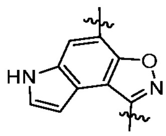, 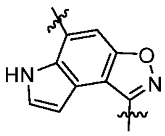, 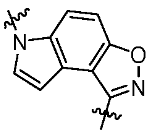,

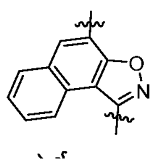 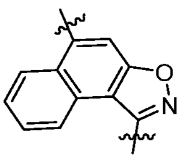, 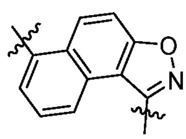, 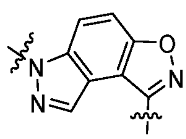, 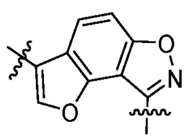, 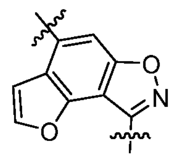,

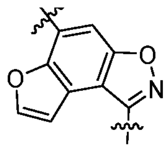, 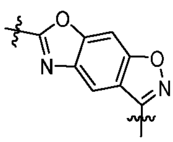, 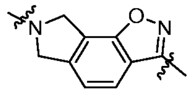, 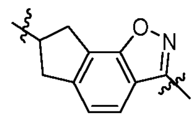, 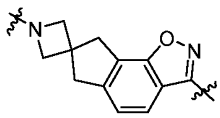, 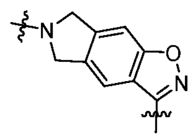,

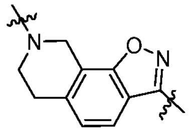, 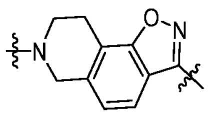, 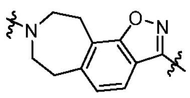, 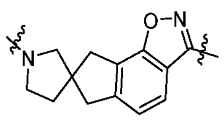, 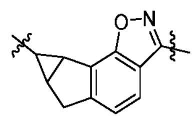,

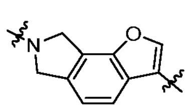, 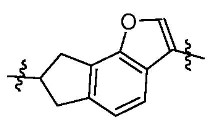, 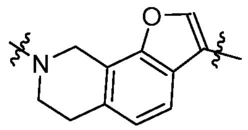, 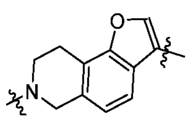, 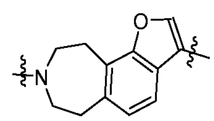,

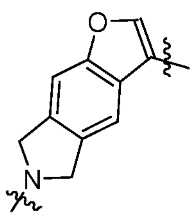, 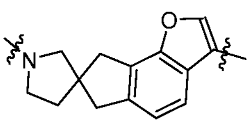, 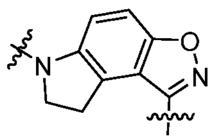, 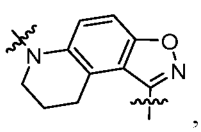, and 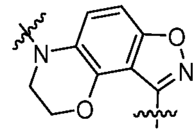.

In some specific embodiments, the structural moiety

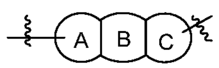

is selected from the group consisting of

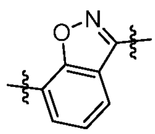, 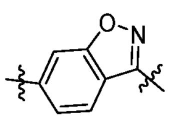, 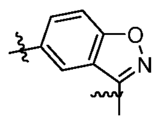, 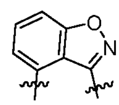, 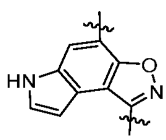, 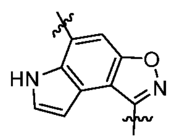, 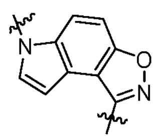,

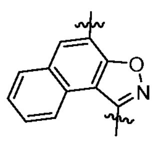 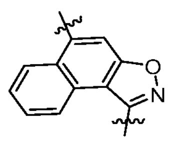, 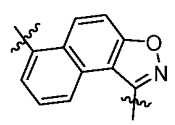, 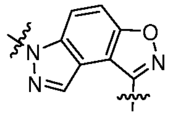, 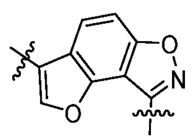, 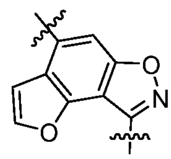,

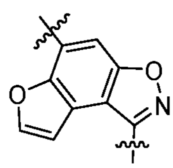, and 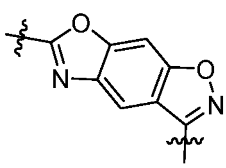.

In some specific embodiments, the structural moiety

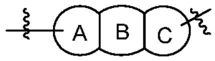

is selected from the group consisting of

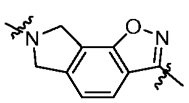, 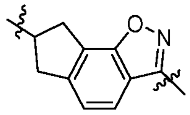, 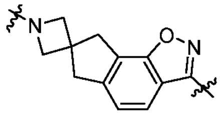, 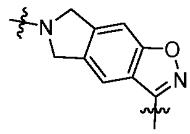, 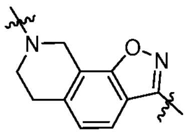, 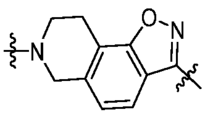, 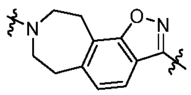, 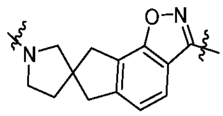, 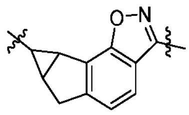, 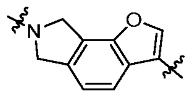, 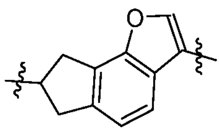, 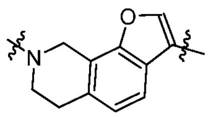, 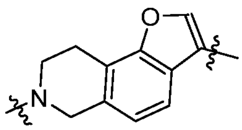, 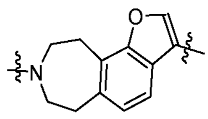, 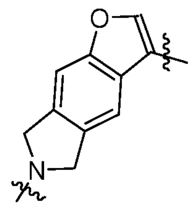, 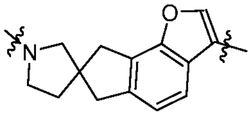, 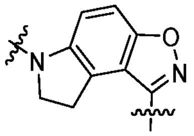, 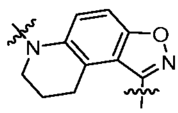, and 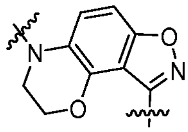.

In some other embodiments, the structural moiety

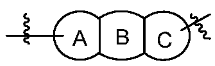

is selected from the group consisting of

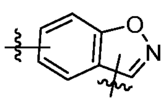, 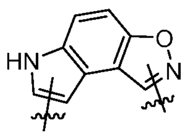, 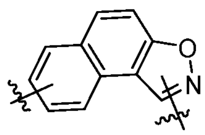, 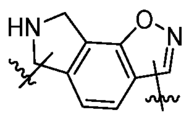, 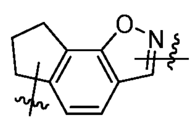, 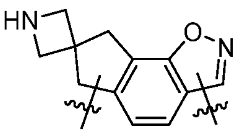, 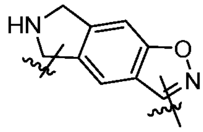, 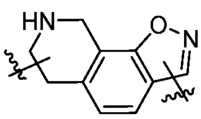, 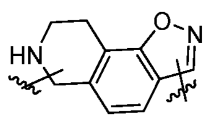, 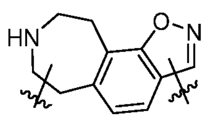, and

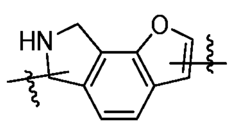.

In some other embodiments, the structural moiety

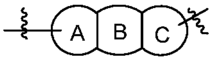

is selected from the group consisting of

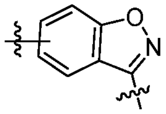, 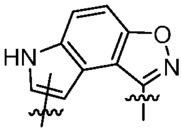, 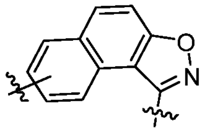, 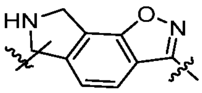, 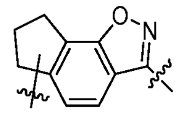,

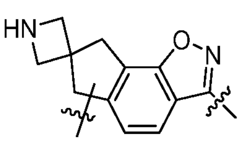, 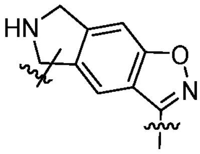, 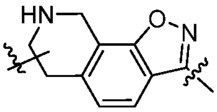, 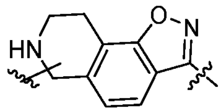, 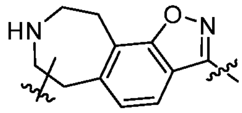,

and

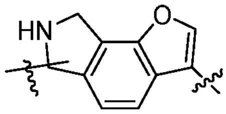.

In some other embodiments, the structural moiety

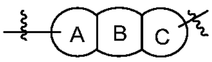

is selected from the group consisting of

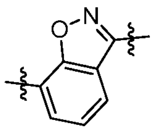, 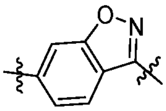, 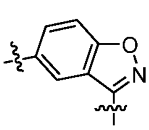, 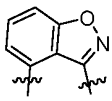, 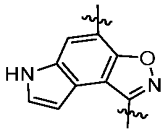, 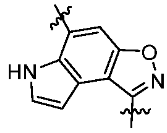, 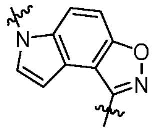,

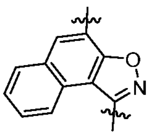, 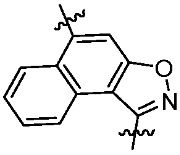, 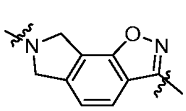, 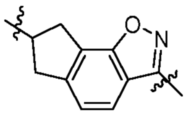, 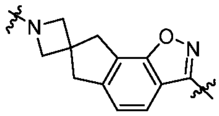, 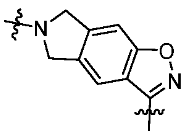,

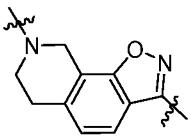, 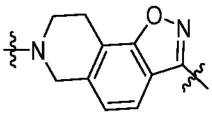, 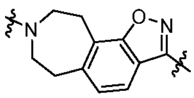, and 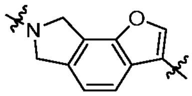.

In some embodiments, the structural moiety

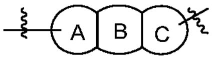

is selected from the group consisting of

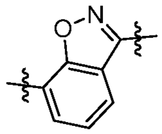, 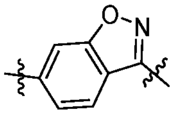,

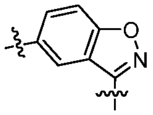, 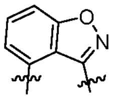, 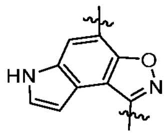, 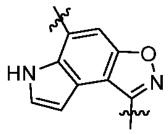, 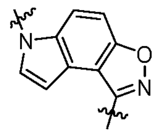, 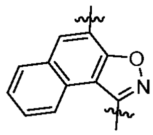, 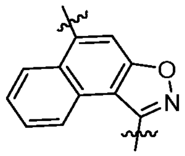,

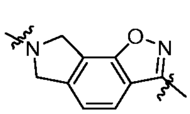, 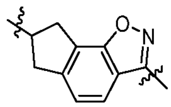, 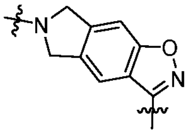, 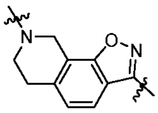, 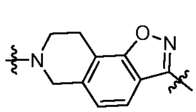, and 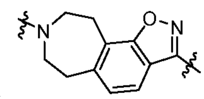.

In some embodiments, each R' is independently selected from the group consisting of halogen, -OH, $-NH_2$, -CN, $C_{1-3}$ alkyl, $C_{1-3}$ alkoxy, and $C_{1-3}$ haloalkyl.

In some embodiments, each R' is independently selected from the group consisting of fluorine, chlorine, bromine, -OH, $-NH_2$, and -CN. In some embodiments, each R' is independently selected from the group consisting of fluorine, chlorine, and bromine.

In some embodiments, each R' is independently selected from fluorine.

In some embodiments, n is selected from the group consisting of 0, 1, and 2. In some embodiments, n is selected from the group consisting of 0 and 1.

In some embodiments, the structural moiety

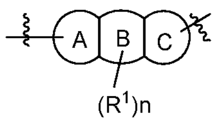

is selected from the group consisting of

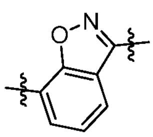, 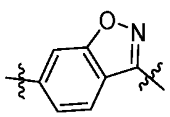, 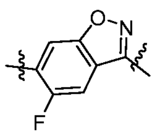, 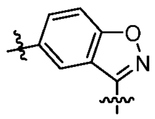, 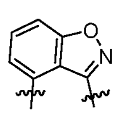, 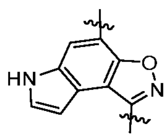, 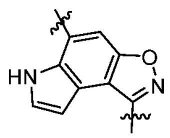,

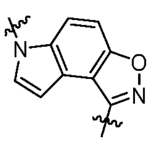, 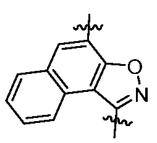, 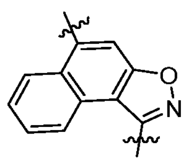, 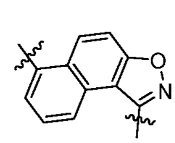, 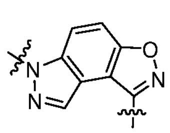, 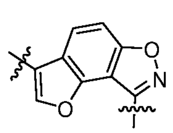,

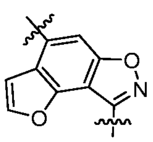, 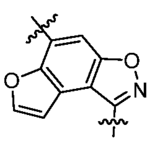, 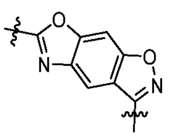, 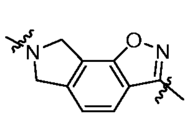, 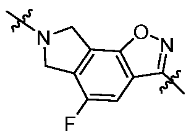, 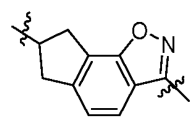,

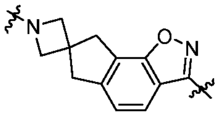, 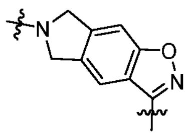, 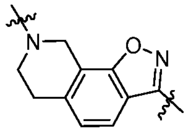, 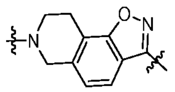, 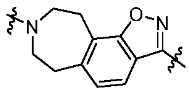,

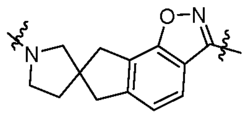, 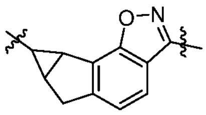, 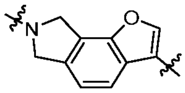, 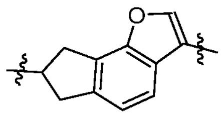, 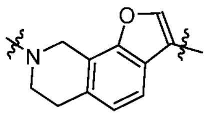,

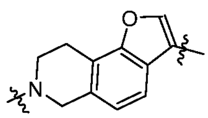, 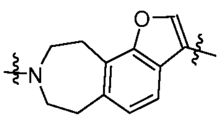, 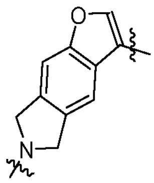, 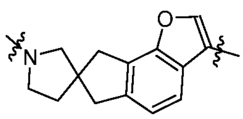, 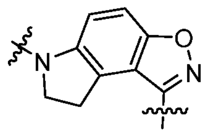,

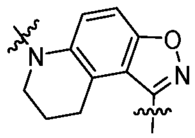, and 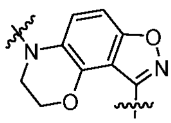.

In some specific embodiments, the structural moiety

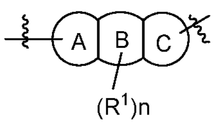

is selected from the group consisting of

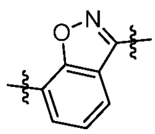, 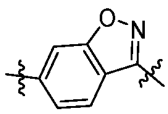, 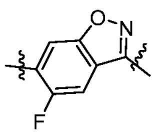, 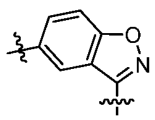, 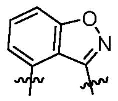, 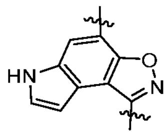, 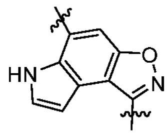,

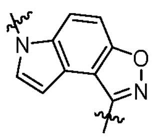, 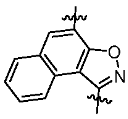, 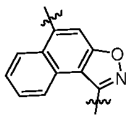, 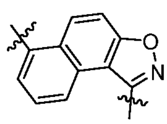, 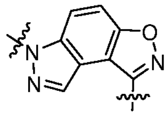, 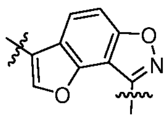, 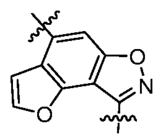,

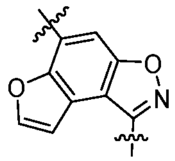, and 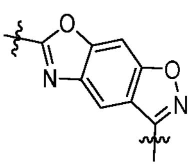.

In some specific embodiments, the structural moiety

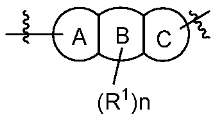

is selected from the group consisting of

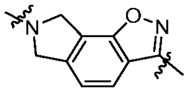, 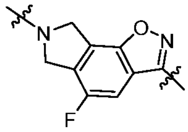, 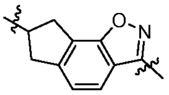, 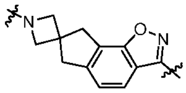, 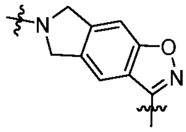, 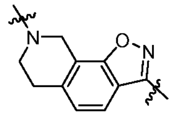,

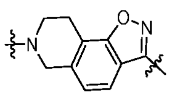, 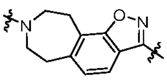, 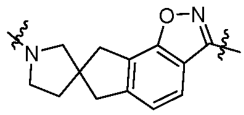, 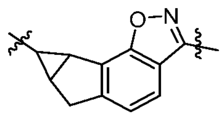, 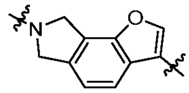,

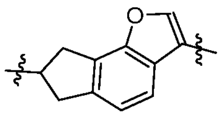, 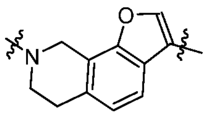, 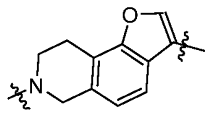, 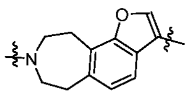, 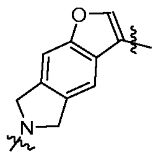,

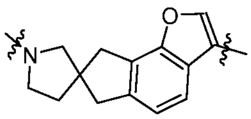, 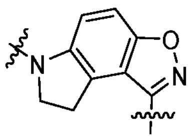, 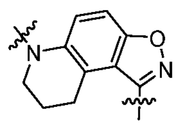, and 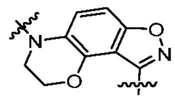.

In some specific embodiments, the structural moiety

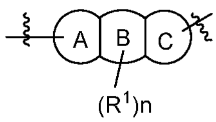

is selected from the group consisting of

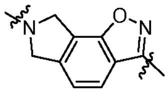, 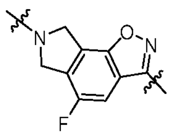, 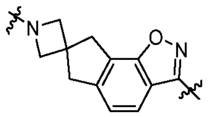, 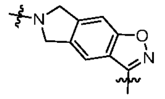, 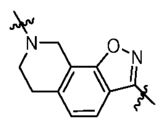, 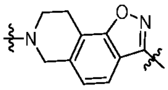,

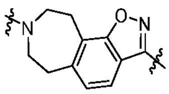, 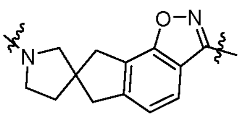, 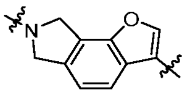, 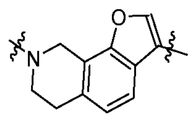, 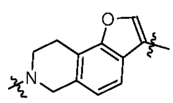, 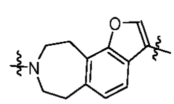,

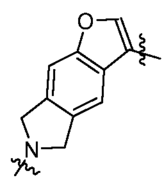, 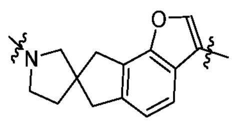, 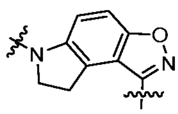, 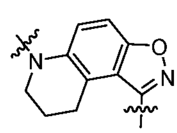, and 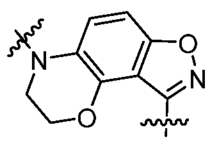.

In some specific embodiments, the structural moiety

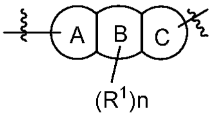

is selected from the group consisting of

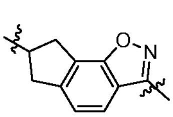, 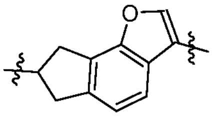, and 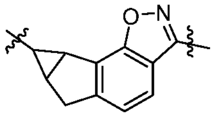.

In some specific embodiments, the structural moiety

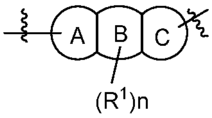

is selected from the group consisting of

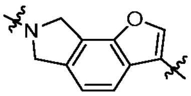, 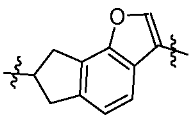, 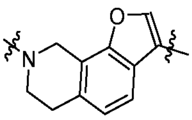, 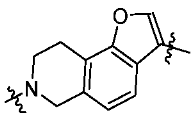, 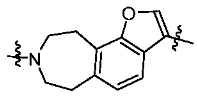, 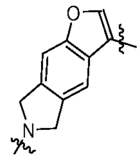,

and

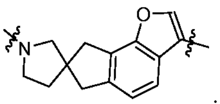.

In some other embodiments, the structural moiety

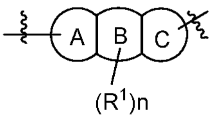

is selected from the group consisting of

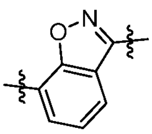, 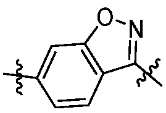, 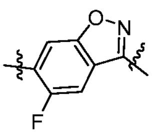, 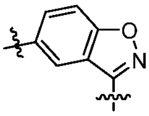, 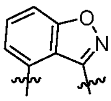, 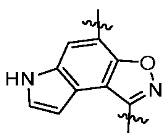, 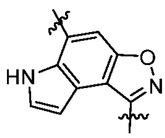,

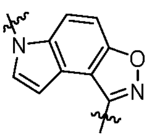, 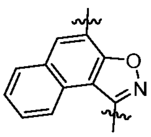, 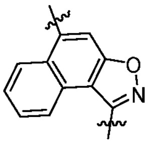, 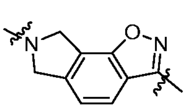, 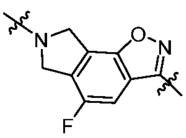, 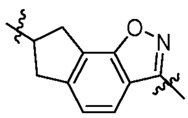,

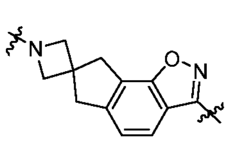, 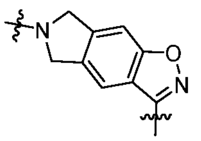, 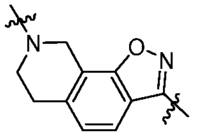, 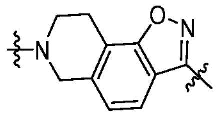, 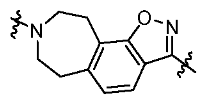,

and

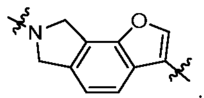.

In some embodiments, the structural moiety

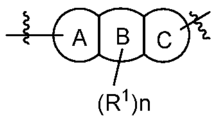

is selected from the group consisting of

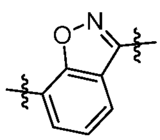, 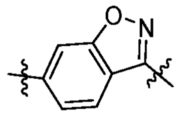, 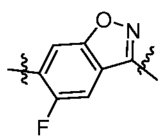, 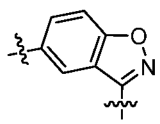, 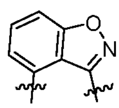, 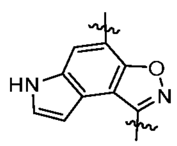,

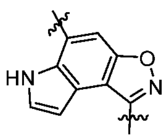, 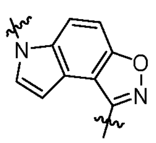, 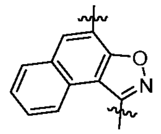, 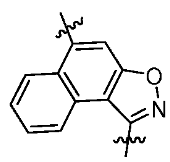, 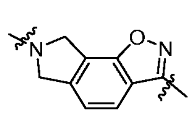, 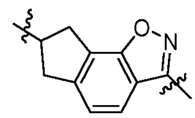,

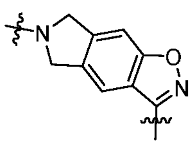, 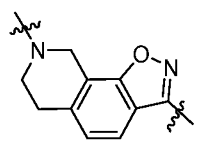, 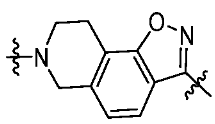, and 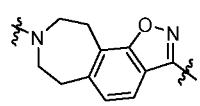.

In some embodiments, PTM is

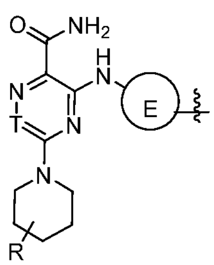,

wherein

T is selected from the group consisting of CH and N;

R is selected from the group consisting of hydrogen and 5- to 6-membered heterocycloalkyl, wherein the heterocycloalkyl is optionally substituted with =O or $C_{1-6}$ alkyl;

ring E is selected from the group consisting of phenyl, benzocycloalkenyl, and benzoheterocycloalkenyl.

In some specific embodiments, T is selected from CH.

In some specific embodiments, R is selected from the group consisting of hydrogen, imidazolidinonyl, and imidazolidinonyl substituted with $C_{1-4}$ alkyl; preferably, R is selected from the group consisting of hydrogen, imidazolidinonyl, 1-methyl-imidazolidinonyl, 1-ethyl-imidazolidinonyl, and 1-propyl-imidazolidinonyl.

In some specific embodiments, ring E is selected from the group consisting of phenyl, benzopiperidinyl, benzodihydropyrrolyl, spiro[benzopyran-piperidine], and benzodihydrooxazinopiperazine. In some specific embodiments, ring E is selected from phenyl.

In some specific embodiments, ring E is selected from phenyl, L is selected from $-Cy^1-LNK-Cy^2-$, and $X^2$ is selected from CH, wherein $Cy^1$, LNK, and $Cy^2$ are as described herein.

In some embodiments, PTM is

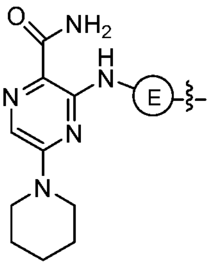
,
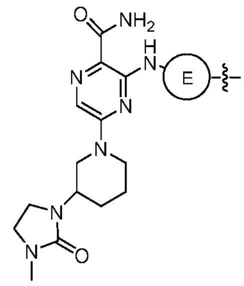
,
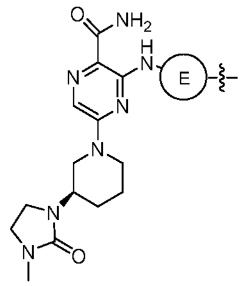
,

or

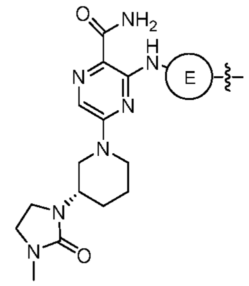
,

and ring E is as defined above. Preferably, PTM is

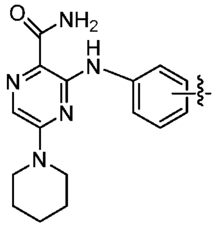
,

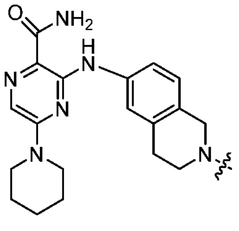
,
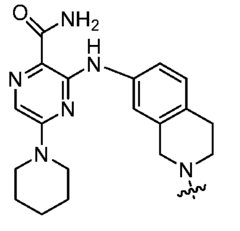
,
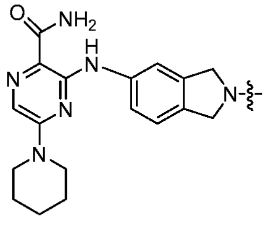
,
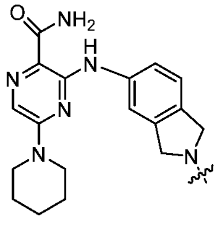
,

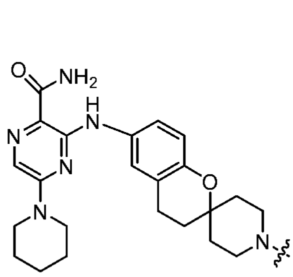, 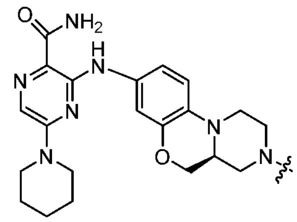, 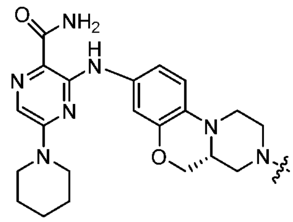, 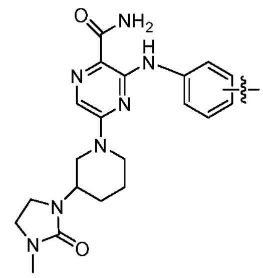,

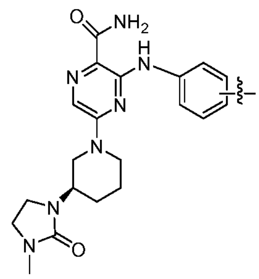, 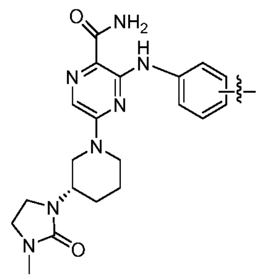, 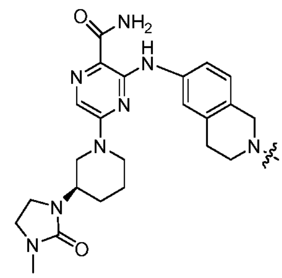, 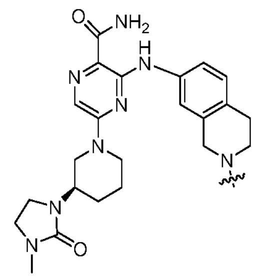,

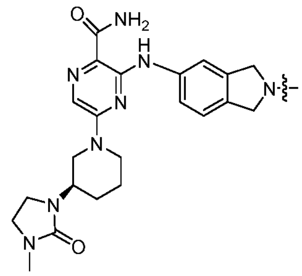, 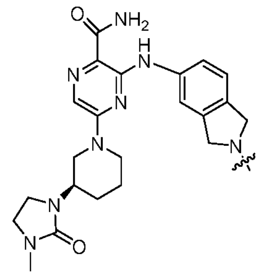, 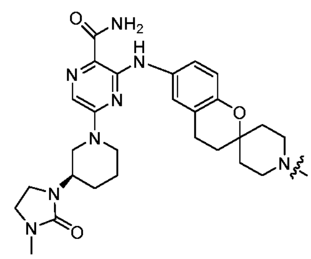, 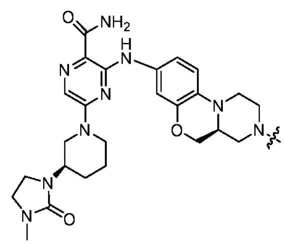, or

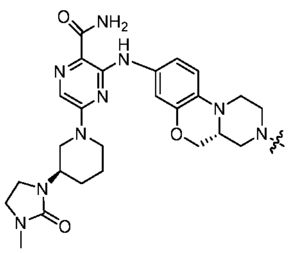.

More preferably, PTM is

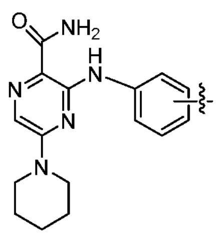, 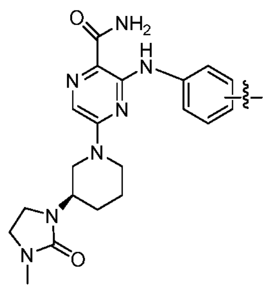,

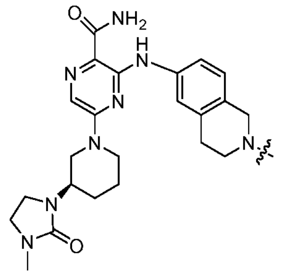, 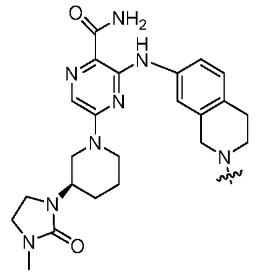, 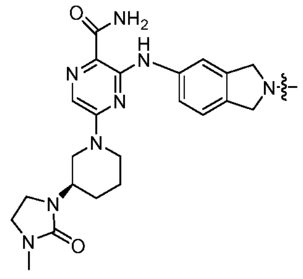, 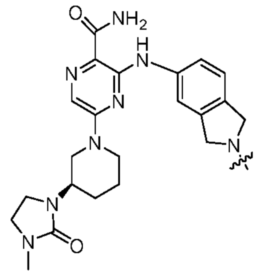,

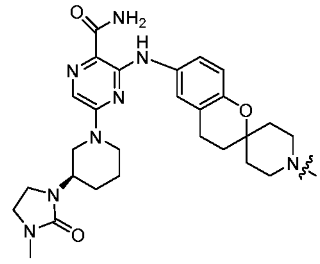, or 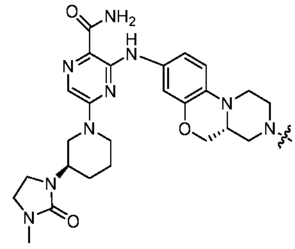.

In some embodiments, the structural moiety -$Cy^1$-LNK-$Cy^2$- is selected from the group consisting of -$Cy^1$-, -Cy'-LNK-, -$Cy^1$-$Cy^2$-, -$Cy^1$-LNK-$Cy^2$-, -$Cy^2$-, and -LNK-$Cy^2$-. In some embodiments, the structural moiety -$Cy^1$-LNK-$Cy^2$- is selected from the group consisting of -$Cy^1$-, -$Cy^1$-$Cy^2$-, and -$Cy^2$-. In some specific embodiments, the structural moiety -$Cy^1$-LNK-$Cy^2$- is selected from the group consisting of -Cy'-LNK-, -$Cy^1$-LNK-$Cy^2$-, and -LNK-$Cy^2$-. In some specific embodiments, the structural moiety -$Cy^1$-LNK-$Cy^2$- is selected from -Cy'-LNK-. In some specific embodiments, the structural moiety -$Cy^1$-LNK-$Cy^2$- is selected from -$Cy^1$-LNK-$Cy^2$-. In some specific embodiments, the structural moiety -$Cy^1$-LNK-$Cy^2$- is selected from -LNK-$Cy^2$-.

In some other embodiments, the structural moiety -$Cy^1$-LNK-$Cy^2$- is selected from the group consisting of -$Cy^1$-, -$Cy^1$-LNK, -$Cy^1$-$Cy^2$-, and -$Cy^1$-LNK-$Cy^2$-.

In some embodiments, $Cy^1$ is selected from the group consisting of a bond and the following groups optionally substituted with one or more $R^a$: $C_{4-11}$ cycloalkyl or 4- to 11-membered heterocycloalkyl.

In some embodiments, $Cy^1$ is selected from the group consisting of a bond and the following groups optionally substituted with one or more $R^a$: $C_{6-9}$ cycloalkyl or 4- to 11-membered heterocycloalkyl. In some embodiments, $Cy^1$ is selected from the group consisting of a bond and the following groups optionally substituted with one or more $R^a$: $C_{6-9}$ cycloalkyl (e.g., $C_6$ cycloalkyl and $C_9$ cycloalkyl) and 5- to 11-membered heterocycloalkyl containing 1-3 heteroatoms selected from the group consisting of N, O, and S (e.g., 1-3 or 1-2 heteroatoms selected from the group consisting of N and O).

In some embodiments, $Cy^1$ is selected from the group consisting of a bond and the following groups optionally substituted with one or more $R^a$: $C_6$ cycloalkyl, $C_9$ cycloalkyl, and 4-, 5-, 6-, 7-, 8-, 9-, 10-, or 11-membered heterocycloalkyl.

In some embodiments, $Cy^1$ is selected from the group consisting of a bond and the following groups optionally substituted with one or more $R^a$: $C_6$ cycloalkyl, $C_9$ cycloalkyl, and 4-, 6-, or 8- to 11-membered heterocycloalkyl. In some specific embodiments, $Cy^1$ is selected from a bond. In some specific embodiments, $Cy^1$ is selected from the group consisting of $C_6$ cycloalkyl and $C_9$ cycloalkyl, wherein the cycloalkyl is optionally substituted with one or more $R^a$. In some specific embodiments, $Cy^1$ is selected from the group consisting of 4-, 6-, and 8- to 11-membered heterocycloalkyl, wherein the heterocycloalkyl is optionally substituted with one or more $R^a$. In some specific embodiments, $Cy^1$ is selected from the group consisting of 4- and 6-membered heterocycloalkyl, wherein the heterocycloalkyl is optionally substituted with one or more $R^a$. In some specific embodiments, $Cy^1$ is selected from the group consisting of 8- to 11-membered heterocycloalkyl, wherein the heterocycloalkyl is optionally substituted with one or more $R^a$. In some specific embodiments, $Cy^1$ is selected from the group consisting of 8-, 9-, 10-, and 11-membered heterocycloalkyl, wherein the heterocycloalkyl is optionally substituted with one or more $R^a$.

In some embodiments, $Cy^1$ is selected from the group consisting of a bond and the following groups optionally substituted with one or more $R^a$: cyclohexyl, spirononanyl, azetidinyl, octahydrocyclopentapyrrolyl, piperidinyl, monoazaspirononanyl, diazaspirononanyl, azabicyclononanyl (e.g., monoazabicyclononanyl), monoazaspiroundecanyl, or diazaspiroundecanyl.

In some specific embodiments, $Cy^1$ is selected from the group consisting of cyclohexyl and spirononanyl. In some specific embodiments, $Cy^1$ is selected from the group consisting of azetidinyl and piperidinyl. In some specific embodiments, $Cy^1$ is selected from the group consisting of octahydrocyclopentapyrrolyl, monoazaspirononanyl, diazaspirononanyl, azabicyclononanyl, monoazaspiroundecanyl, and diazaspiroundecanyl. In some specific embodiments,

$Cy^1$ is selected from the group consisting of a bond, piperidinyl, cyclohexyl, spirononanyl, monoazaspirononanyl, octahydrocyclopentapyrrolyl, monoazaspiroundecanyl, monooxamonoazaspiroundecanyl, and azabicyclononanyl.

In some embodiments, $Cy^1$ is selected from the group consisting of a bond and the following groups optionally substituted with one or more $R^a$:

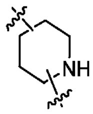, 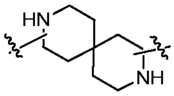, 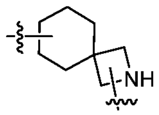, 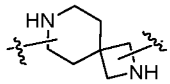, 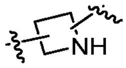,

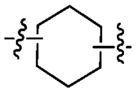, 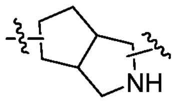, 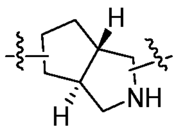, 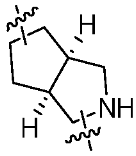, 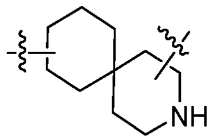, 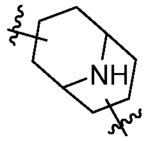,

or

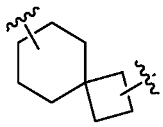.

In some embodiments, $Cy^1$ is selected from the group consisting of a bond and the following groups optionally substituted with one or more $R^a$:

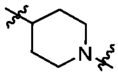, 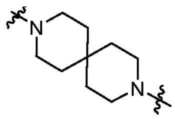, 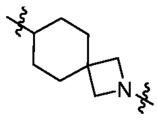, 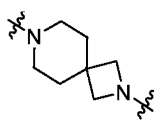, 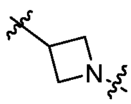,

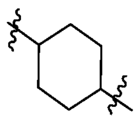, 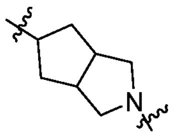, 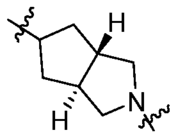, 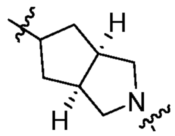, 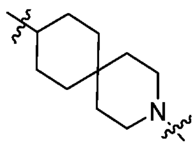, 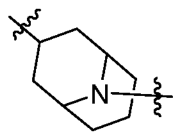,

or

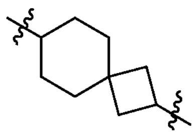.

In some specific embodiments, $Cy^1$ is selected from the group consisting of

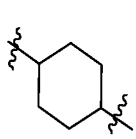 and 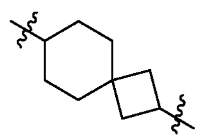.

In some specific embodiments, $Cy^1$ is selected from the group consisting of

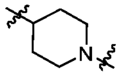 and 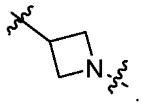.

In some specific embodiments, $Cy^1$ is selected from the group consisting of

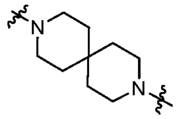, 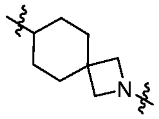,

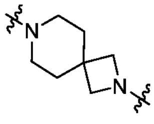, 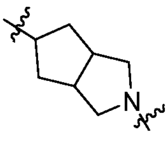, 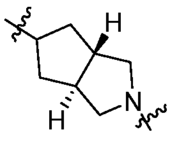, 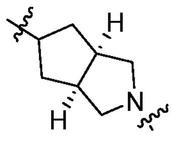, 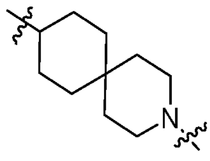, and 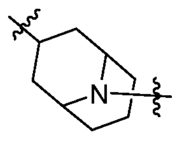.

In some specific embodiments, $Cy^1$ is selected from the group consisting of a bond,

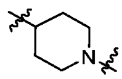, 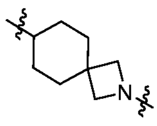,

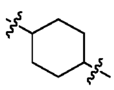, 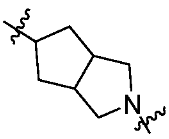, 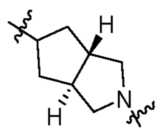, 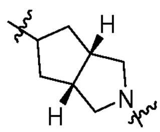, 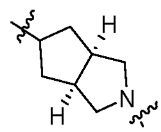, 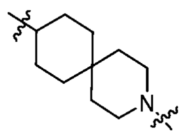, 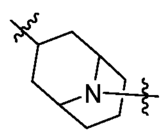,

and

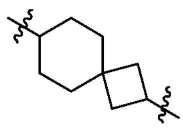.

In some other embodiments, $Cy^1$ is selected from the group consisting of 4- to 11-membered heterocycloalkyl optionally substituted with one or more $R^a$.

In some other embodiments, $Cy^1$ is selected from the group consisting of 5-, 6-, 7-, 8-, 9-, 10-, and 11-membered heterocycloalkyl optionally substituted with one or more $R^a$.

In some other embodiments, $Cy^1$ is selected from the group consisting of 6-, and 9- to 11-membered heterocycloalkyl optionally substituted with one or more $R^a$.

In some other embodiments, $Cy^1$ is selected from the group consisting of 6-, 9-, and 11-membered heterocycloalkyl optionally substituted with one or more $R^a$.

In some other embodiments, $Cy^1$ is selected from the group consisting of piperidinyl, monoazaspirononane, diazaspirononane, and diazaspiroundecane optionally substituted with one or more $R^a$.

In some other embodiments, $Cy^1$ is selected from the group consisting of

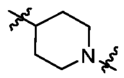, 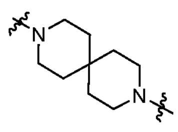,

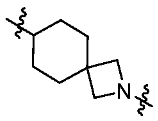, and 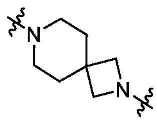

optionally substituted with one or more $R^a$.

In some embodiments, LNK is selected from the group consisting of a bond, $C_{1-6}$ alkylene, and $C_{1-6}$ heteroalkylene.

In some embodiments, LNK is selected from the group consisting of a bond and $C_{1-4}$ alkylene.

In some embodiments, LNK is selected from the group consisting of a bond and $C_{1-3}$ alkylene.

In some embodiments, LNK is selected from the group consisting of a bond and $-CH_2-$. In some specific embodiments, LNK is selected from a bond. In some specific embodiments, LNK is selected from $-CH_2-$.

In some embodiments, $Cy^2$ is absent or selected from the group consisting of $C_{4-11}$ cycloalkyl and 4- to 11-membered heterocycloalkyl, wherein the cycloalkyl or heterocycloalkyl is optionally substituted with one or more $R^b$. In some specific embodiments, $Cy^2$ is absent. In some specific embodiments, $Cy^2$ is selected from the group consisting of $C_{4-11}$ cycloalkyl and 4- to 11-membered heterocycloalkyl, wherein the cycloalkyl or heterocycloalkyl is optionally substituted with one or more $R^b$.

In some embodiments, $Cy^2$ is absent or selected from the group consisting of $C_{4-6}$ cycloalkyl and 4- to 6-membered heterocycloalkyl, wherein the cycloalkyl or heterocycloalkyl is optionally substituted with one or more $R^b$. In some specific embodiments, $Cy^2$ is selected from the group consisting of $C_{4-6}$ cycloalkyl and 4- to 6-membered heterocycloalkyl, wherein the cycloalkyl or heterocycloalkyl is optionally substituted with one or more $R^b$. In some embodiments, $Cy^2$ is absent or selected from the group consisting of $C_{4-6}$ cycloalkyl and 4- to 6-membered heterocycloalkyl, wherein the cycloalkyl or heterocycloalkyl is optionally substituted with one or more $R^b$, wherein the heterocycloalkyl comprises 1-3 (e.g., 1-2) heteroatoms selected from the group consisting of N, O, and S (e.g., Nor O).

In some embodiments, $Cy^2$ is absent or selected from the group consisting of cyclobutyl, cyclopentyl, cyclohexyl, azetidinyl, pyrrolidinyl, and piperidinyl, wherein the cyclobutyl, cyclopentyl, azetidinyl, pyrrolidinyl, or piperidinyl is optionally substituted with one or more $R^b$.

In some embodiments, $Cy^2$ is absent or selected from the group consisting of

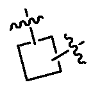, 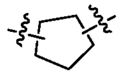, 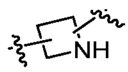,

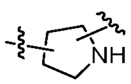, 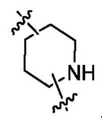, and 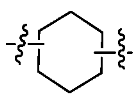.

In some embodiments, $Cy^2$ is absent or selected from the group consisting of

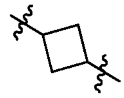, 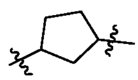,

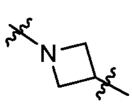, 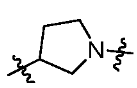, 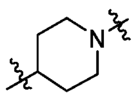, and 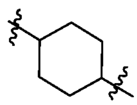.

In some other embodiments, $Cy^2$ is absent or selected from the group consisting of cyclobutyl, cyclopentyl, azetidinyl, pyrrolidinyl, and piperidinyl, wherein the cyclobutyl, cyclopentyl, azetidinyl, pyrrolidinyl, or piperidinyl is optionally substituted with one or more $R^b$.

In some other embodiments, $Cy^2$ is selected from the group consisting of

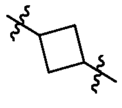, 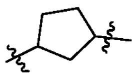, 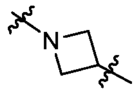,

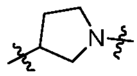, and 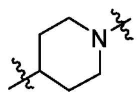.

In some embodiments, $R^a$ and $R^b$ are each independently selected from the group consisting of halogen, -OH, $-NH_2$, -CN, $C_{1-3}$ alkyl, $C_{1-3}$ alkoxy, $C_{1-3}$ haloalkyl, $C_{1-3}$ alkylamino, and di-$C_{1-3}$ alkylamino.

In some embodiments, $R^a$ and $R^b$ are each independently selected from the group consisting of halogen, -OH, $-NH_2$, -CN, and $C_{1-3}$ alkyl.

In some embodiments, $R^a$ and $R^b$ are each independently selected from the group consisting of halogen, -OH, $-NH_2$, and -CN.

In some embodiments, the structural moiety -Cy'-LNK- is selected from the group consisting of a bond, $-CH_2-$,

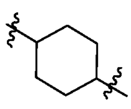, 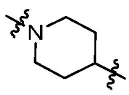, 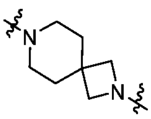, 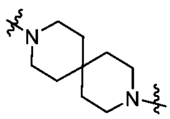, 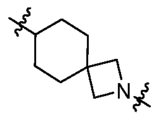, 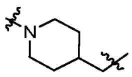, 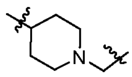,

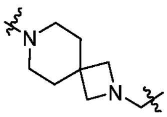, 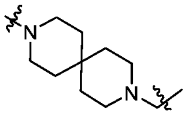, 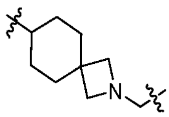, 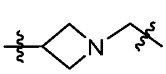, 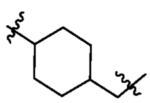, 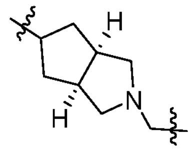,

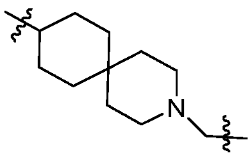, 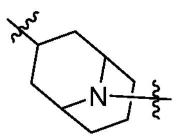, 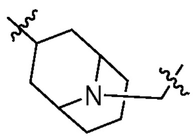, and 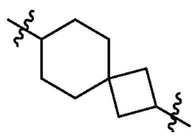.

In some specific embodiments, the structural moiety -Cy'-LNK- is selected from the group consisting of

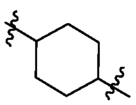, 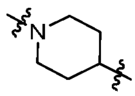, 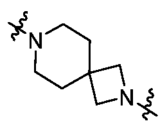, 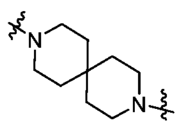, 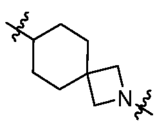, 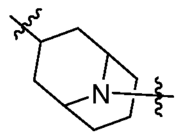,

and

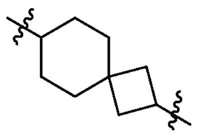.

In some specific embodiments, the structural moiety -Cy'-LNK- is selected from the group consisting of

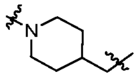, 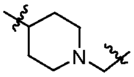, 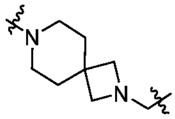, 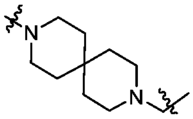, 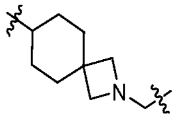, 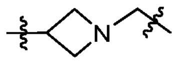,

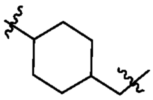, 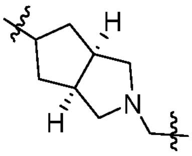, 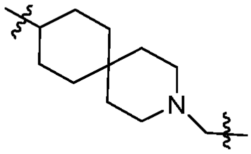, and 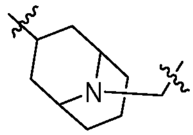.

In some specific embodiments, the structural moiety -Cy'-LNK- is selected from the group consisting of

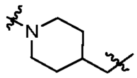, 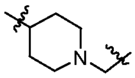,

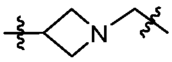, and 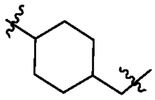.

In some specific embodiments, the structural moiety -Cy'-LNK- is selected from the group consisting of

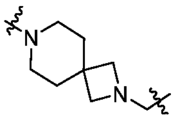, 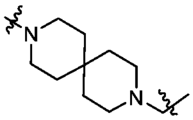, 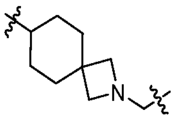, 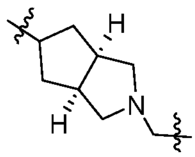,

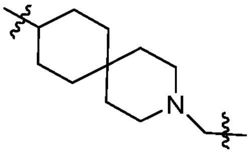, and 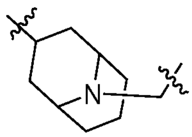.

In some other embodiments, the structural moiety -Cy'-LNK- is selected from the group consisting of

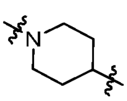, 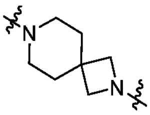, 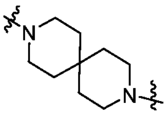, 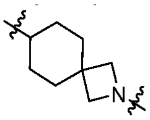, 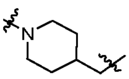, 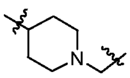, 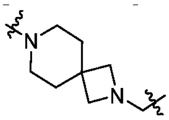,

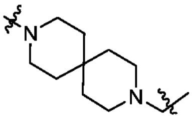, and 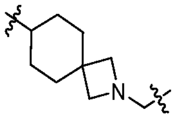.

In some embodiments, the structural moiety -LNK-$Cy^2$- is selected from the group consisting of a bond, $-CH_2-$,

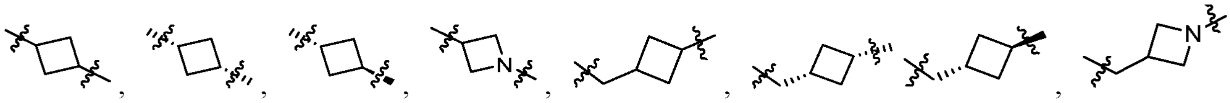

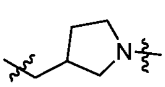, 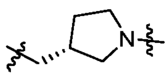, 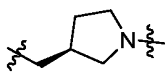, 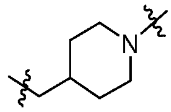, 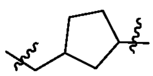, 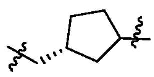,

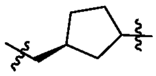, 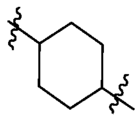, 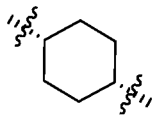, and 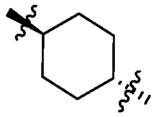.

In some other embodiments, the structural moiety -LNK-$Cy^2$- is selected from the group consisting of a bond,

-$CH_2$-, 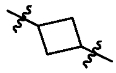, 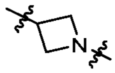, 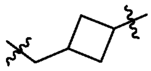, 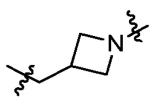, 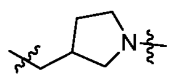, 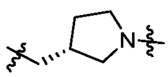,

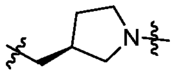, 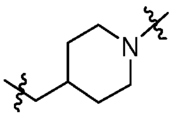, 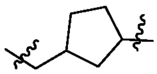, 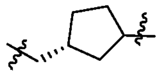, and 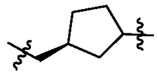.

In some embodiments, the structural moiety -$Cy^1$-$Cy^2$- is selected from the group consisting of

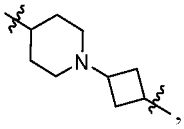,

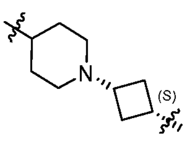, 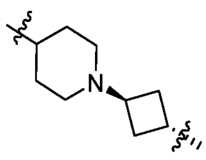, 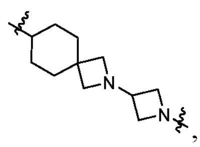, 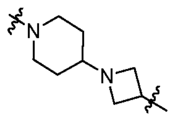, 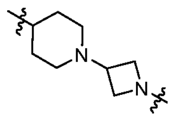, 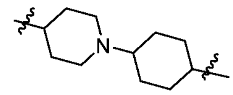,

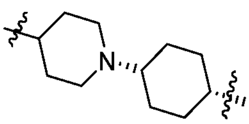, and 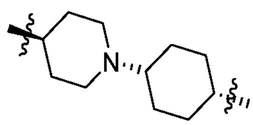.

In some other embodiments, the structural moiety -$Cy^1$-$Cy^2$- is selected from the group consisting of

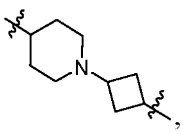, 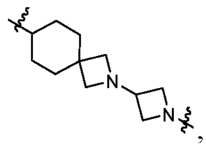, 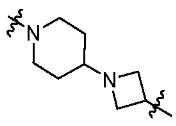, and 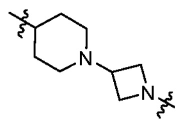.

In some embodiments, the structural moiety -$Cy^1$-LNK-$Cy^2$- is selected from the group consisting of a bond,

-$CH_2$-, 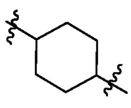, 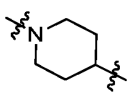, 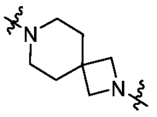, 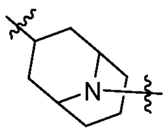, 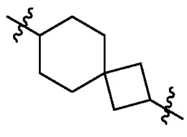, 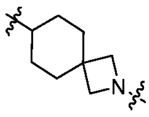,

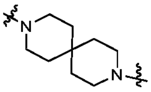, 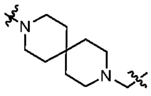, 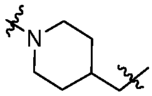, 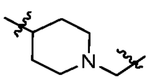, 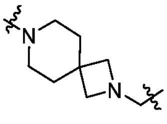, 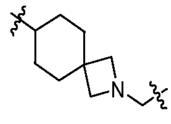, 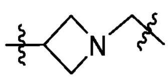,

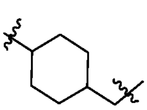, 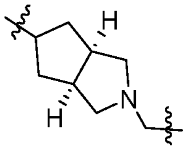, 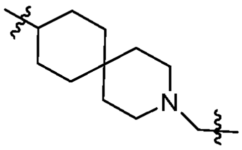, 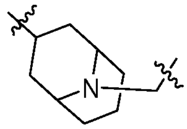 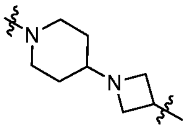, 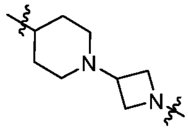,

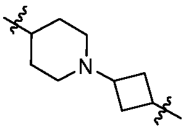, 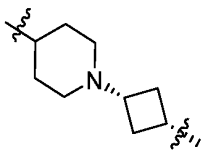, 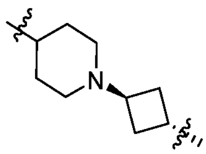, 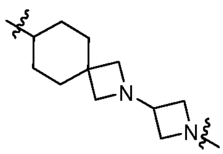, 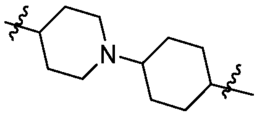,

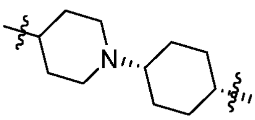, 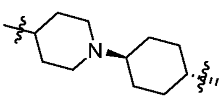, 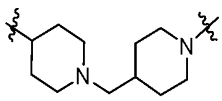, 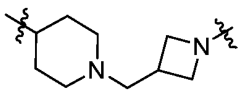,

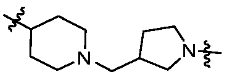, 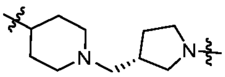, 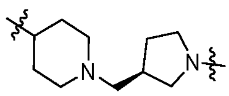, 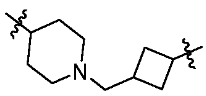, 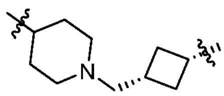,

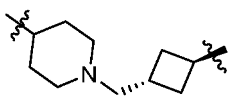, 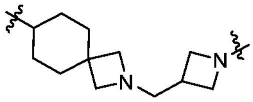, 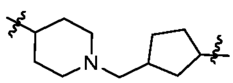, 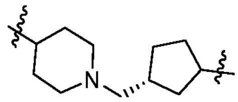,

and

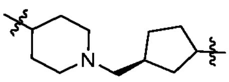.

In some specific embodiments, the structural moiety -$Cy^1$-LNK-$Cy^2$- is selected from the group consisting of

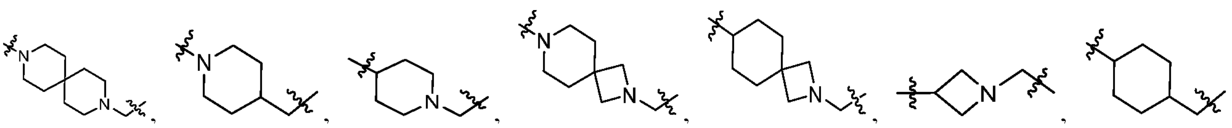

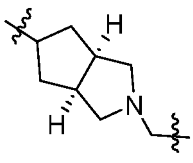, 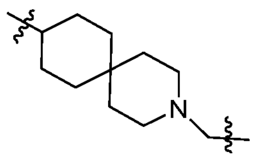, and 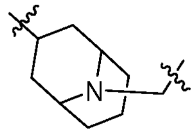.

In some specific embodiments, the structural moiety -$Cy^1$-LNK-$Cy^2$- is selected from the group consisting of

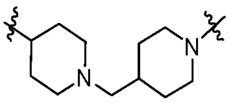, 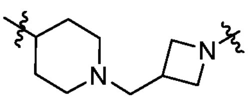,

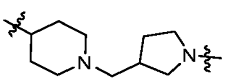, 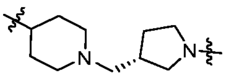, 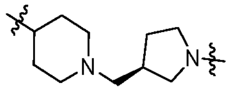, 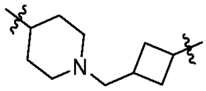, 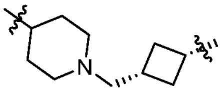,

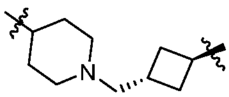, 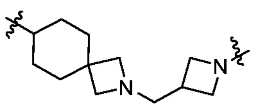, 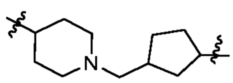, 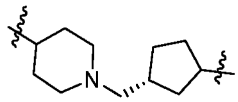,

and

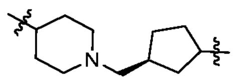.

In some other embodiments, the structural moiety -$Cy^1$-LNK-$Cy^2$- is selected from the group consisting of

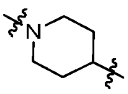, 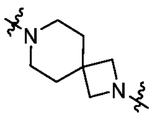, 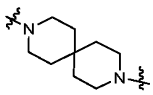, 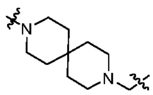, 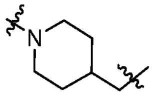, 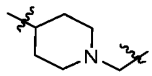, 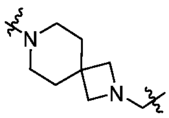,

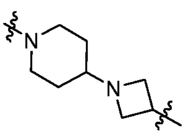, 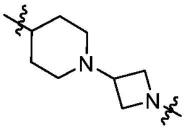, 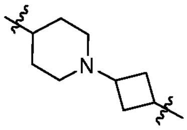, 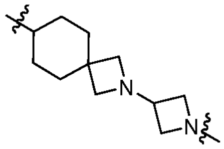, 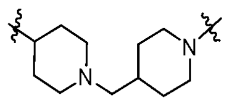,

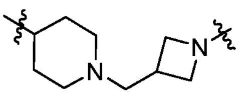, 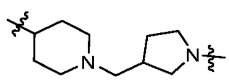, 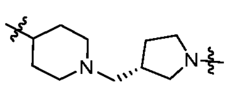, 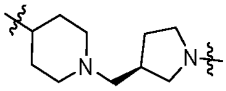, 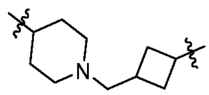,

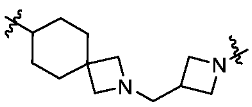, 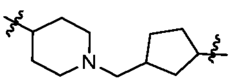, 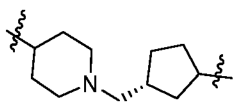, and 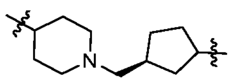.

In some embodiments, the structural moiety

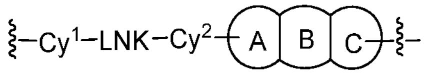

is selected from the group consisting of

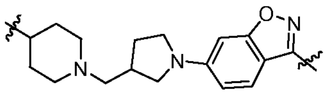 , 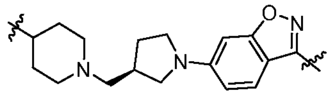 , 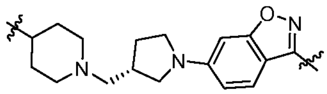 ,

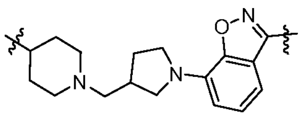 , 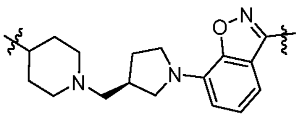 , 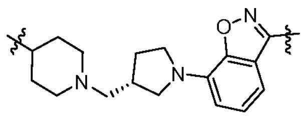 , 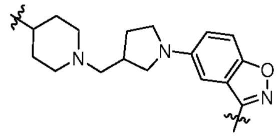 ,

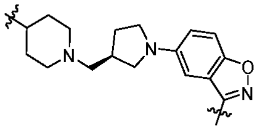 , 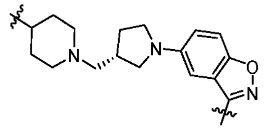 , 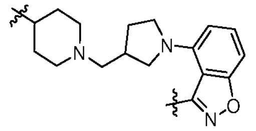 , 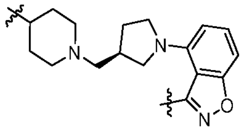 ,

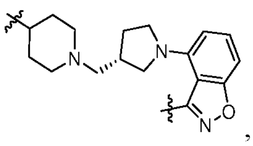 , 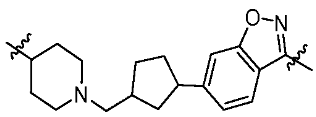 , 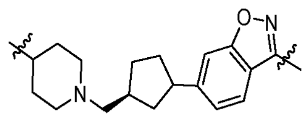 , 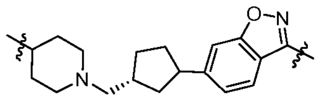 ,

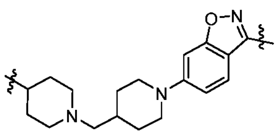 , 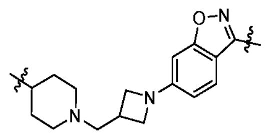 , 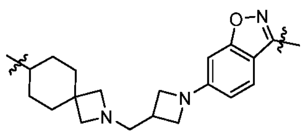 , 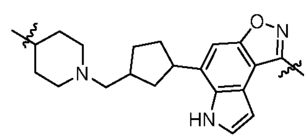 ,

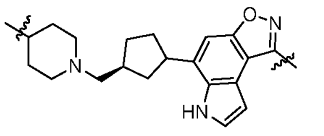 , 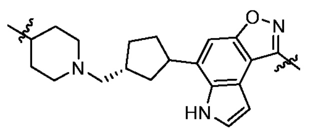 , 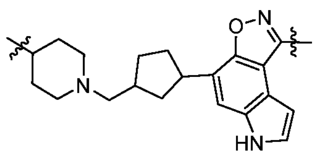 ,

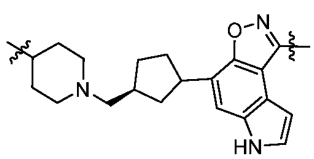 , 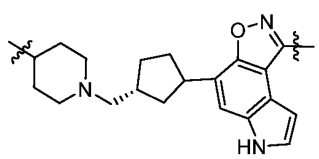 , 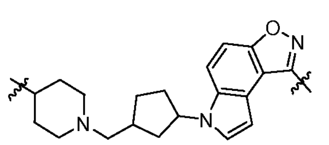 ,

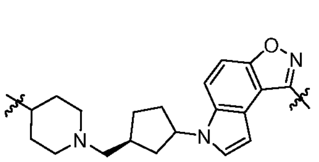 , 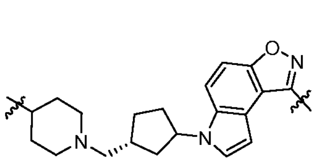 , 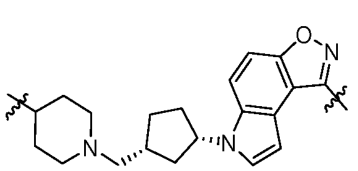 ,

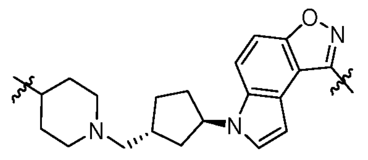 , 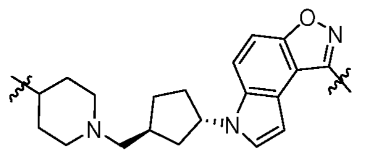 , 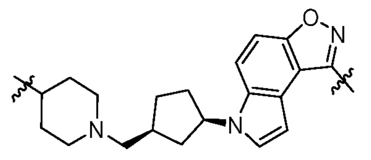 ,

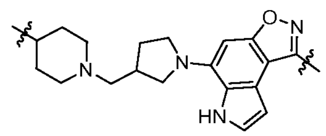 , 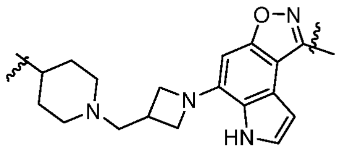 , 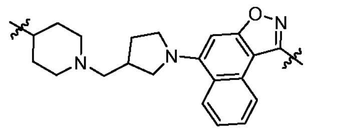 ,

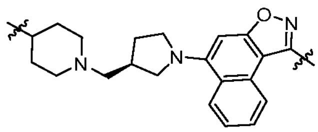 , 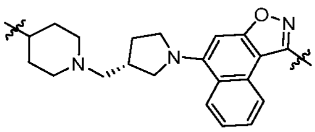 , 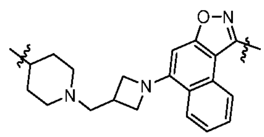 , 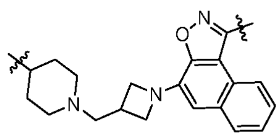 ,

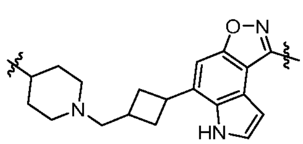 , 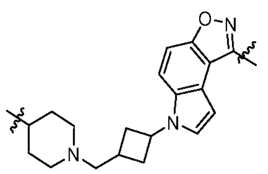 , 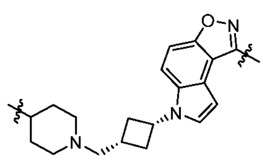 , 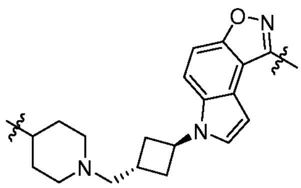 ,

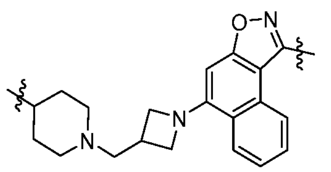 , 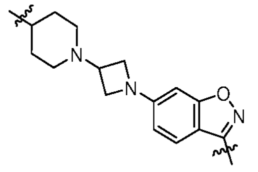 , 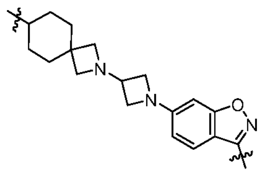 , 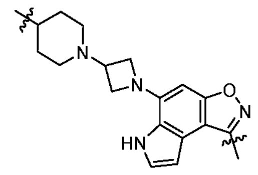 ,

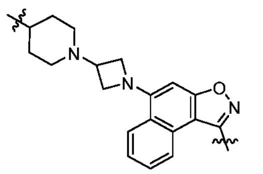 , 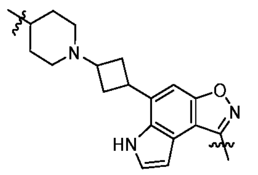 , 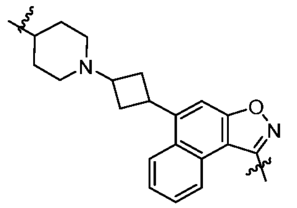 , 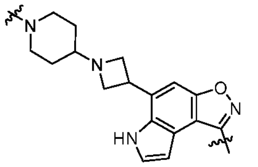 ,

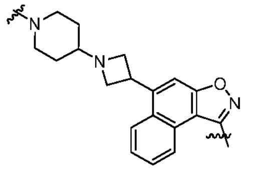 , 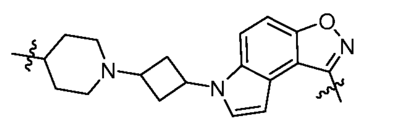 , 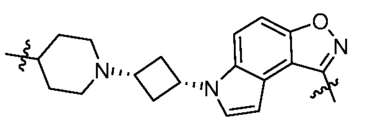 ,

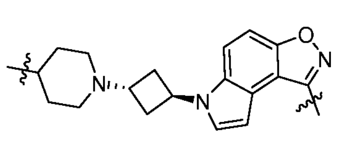 , 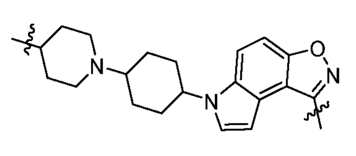 , 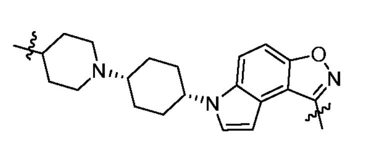 ,

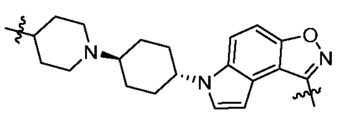, 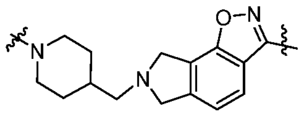, 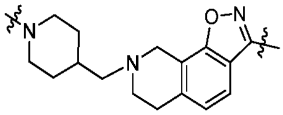, 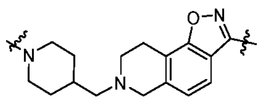,

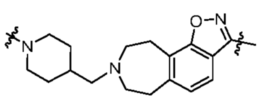, 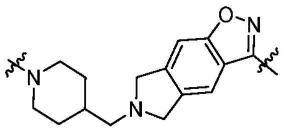, 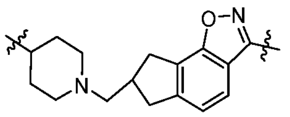, 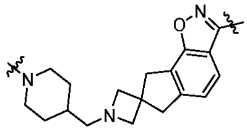,

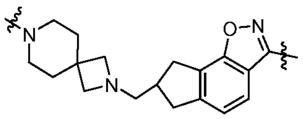, 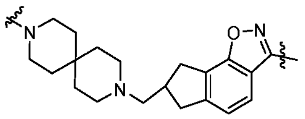, 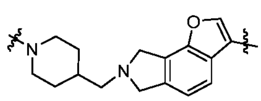, 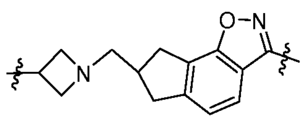,

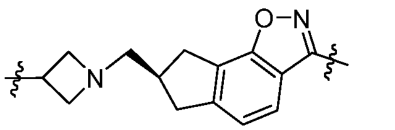, 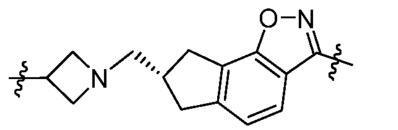, 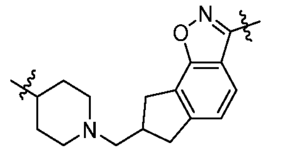,

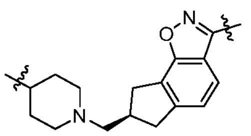, 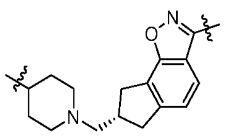, 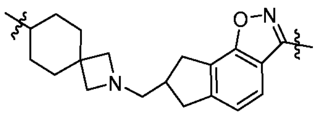, 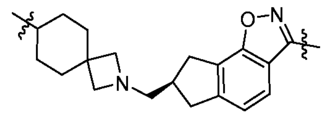,

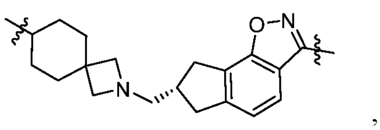, 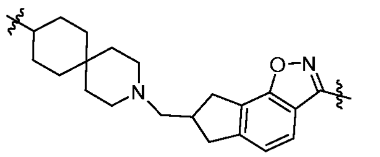, 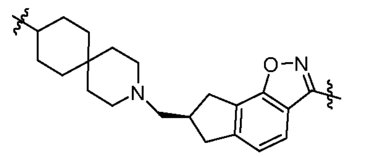,

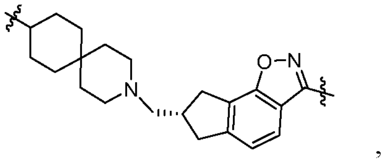, 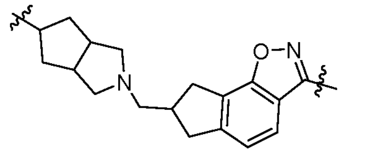, 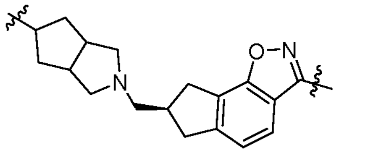,

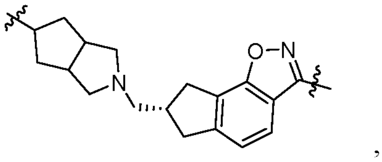, 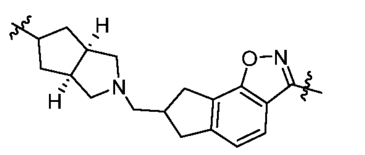, 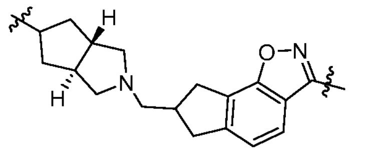,

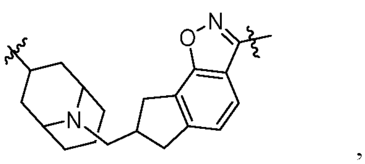, 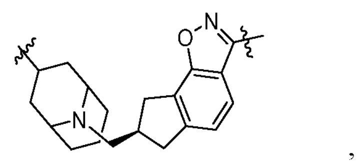, 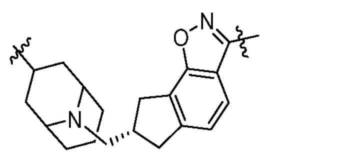,

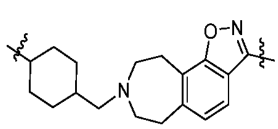 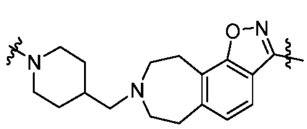, 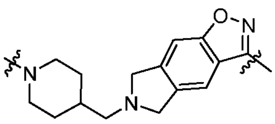, 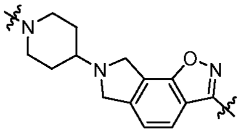,

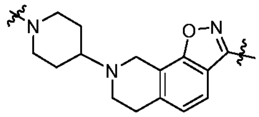, 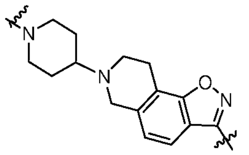, 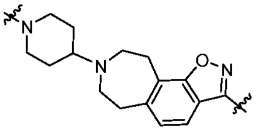, 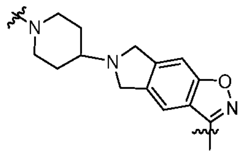,

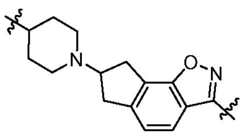, 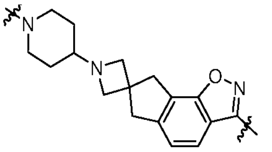, 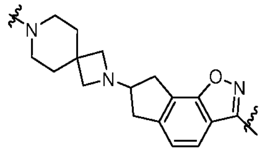, 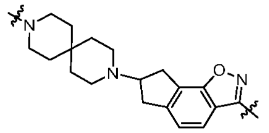,

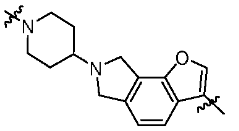, 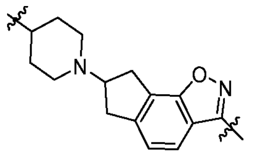, 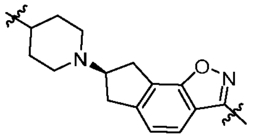, 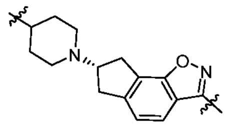,

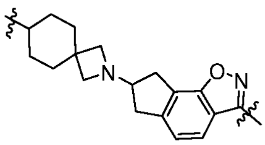, 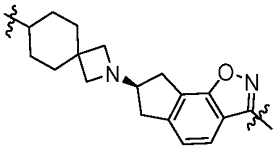, 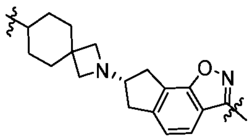, 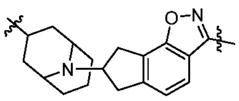,

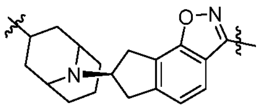, 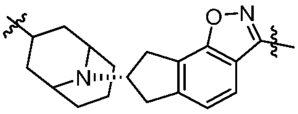, 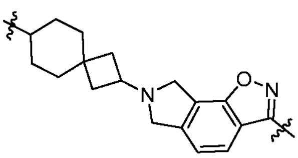, 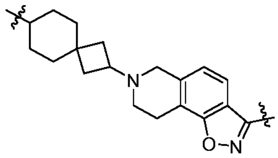,

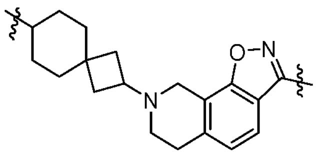, 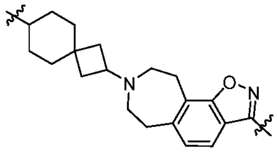, 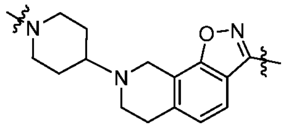, 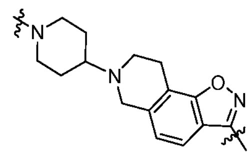,

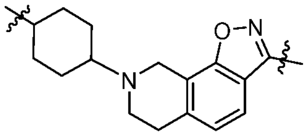, 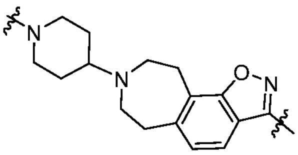, 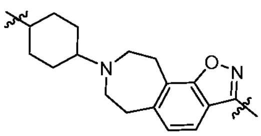, 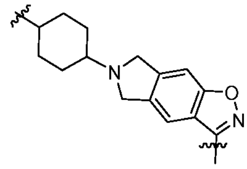,

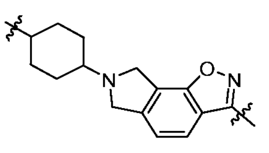, 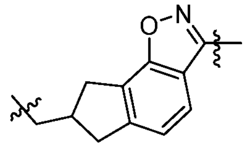, 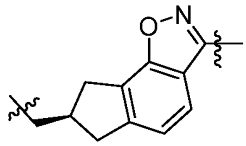, 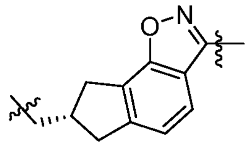,

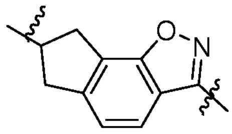, 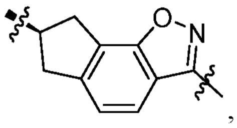, and 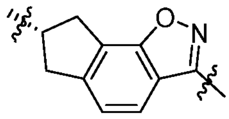.

In some other embodiments, the structural moiety

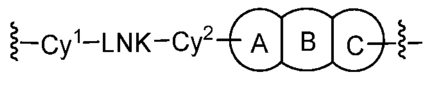

is selected from the group consisting of

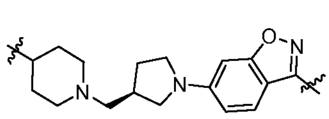, 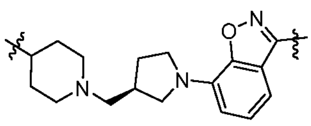, 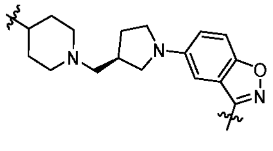,

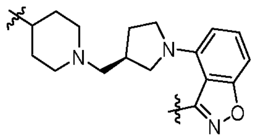, 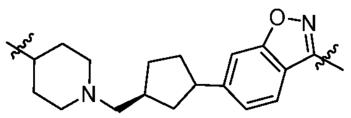, 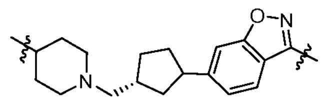,

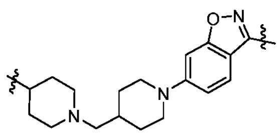, 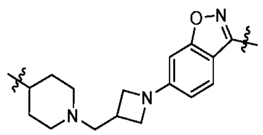, 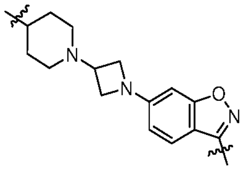, 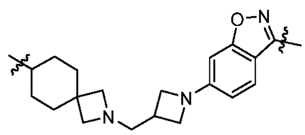,

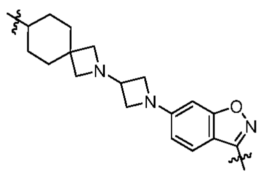, 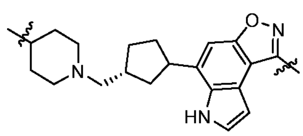, 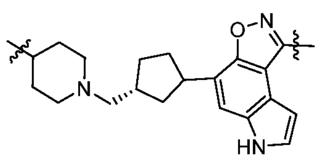,

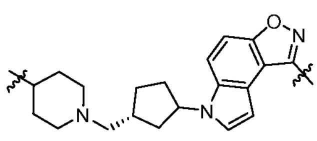, 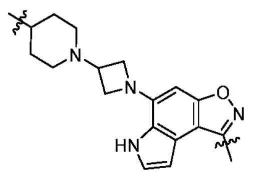, 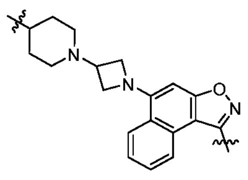, 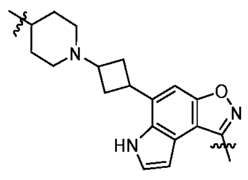,

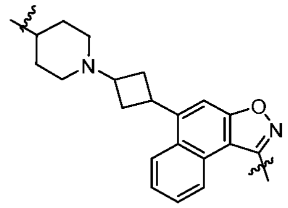, 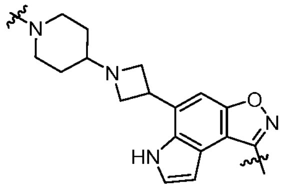, 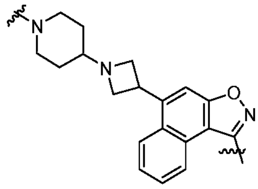, 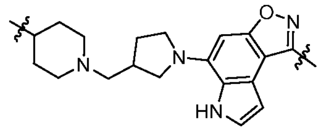,

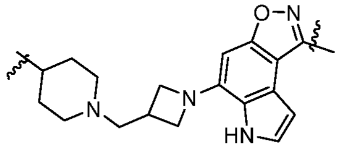, 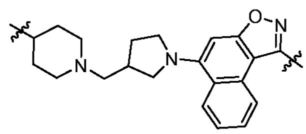, 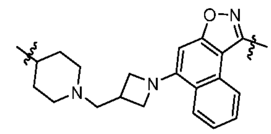, 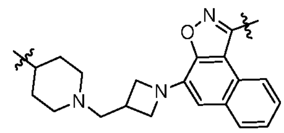,

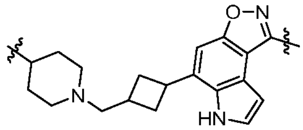, 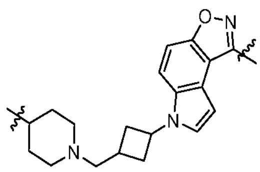, 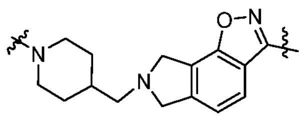, 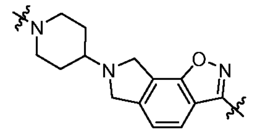,

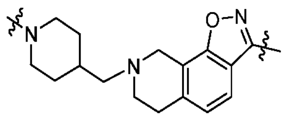, 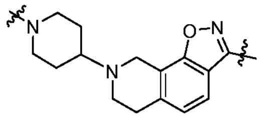, 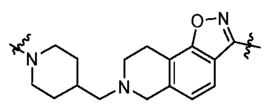, 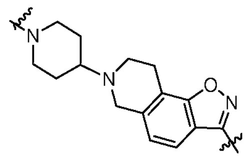,

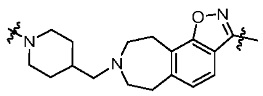, 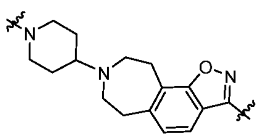, 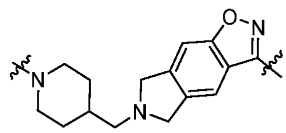, 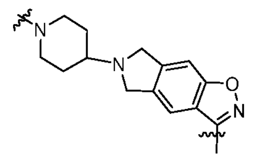,

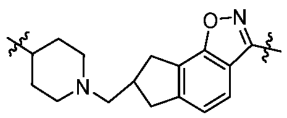, 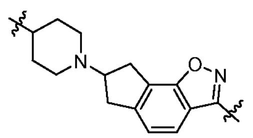, 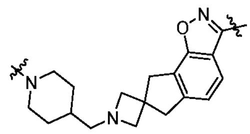, 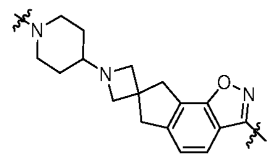,

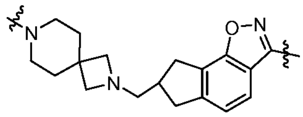, 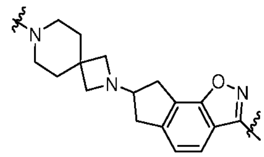, 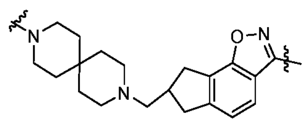, 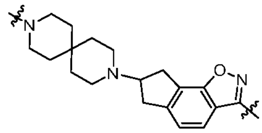,

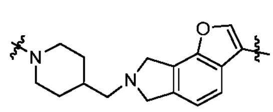, and 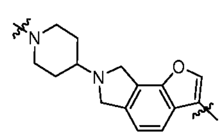.

In some embodiments, T is selected from CH. In some embodiments, T is selected from N.

In some embodiments, R is selected from the group consisting of hydrogen and 5- to 6-membered heterocycloalkyl, wherein the heterocycloalkyl is optionally substituted with =O or $C_{1-3}$ alkyl.

In some embodiments, R is selected from the group consisting of hydrogen and 5-membered heterocycloalkyl, wherein the heterocycloalkyl is optionally substituted with =O or $C_{1-3}$ alkyl.

In some embodiments, R is selected from the group consisting of hydrogen and imidazolinyl, wherein the imidazolinyl is optionally substituted with =O or methyl.

In some embodiments, R is selected from the group consisting of hydrogen and

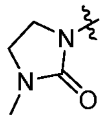.

In some embodiments, the present application relates to a compound of formula I, a stereoisomer thereof, or a pharmaceutically acceptable salt thereof, wherein, ring A is absent or selected from the group consisting of $C_{5-8}$ cycloalkenyl, 5- to 9-membered heterocycloalkenyl (preferably 5- to 8-membered heterocycloalkenyl or 5- to 7-membered heterocycloalkenyl), and phenyl; or, ring A is absent or selected from the group consisting of $C_{5-6}$ cycloalkenyl, 5- to 9-membered heterocycloalkenyl (preferably 5- to 8-membered heterocycloalkenyl or 5- to 7-membered heterocycloalkenyl), and phenyl; preferably, ring A is absent or selected from the group consisting of $C_{5-6}$ cycloalkenyl, 5- to 7-membered heterocycloalkenyl containing 1-2 heteroatoms selected from the group consisting of N, O, and S (preferably containing 1-2 N atoms, e.g., 1 N atom), and phenyl; or, preferably, ring A is selected from the group consisting of $C_{5-6}$ cycloalkenyl and 5- to 7-membered heterocycloalkenyl containing 1-2 heteroatoms selected from the group consisting of N, O, and S (preferably containing 1-2 N atoms, e.g., 1 N atom);

ring B is selected from phenyl;
ring C is selected from the group consisting of isoxazolyl and furanyl;
each R' is independently selected from the group consisting of fluorine, chlorine, and bromine; n is selected from the group consisting of 0, 1, and 2, preferably 0 and 1;
preferably,

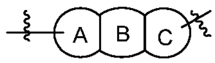

is selected from the group consisting of

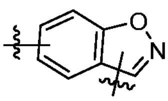, 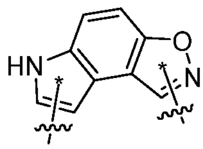,

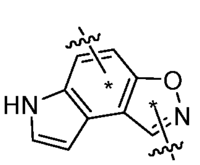, 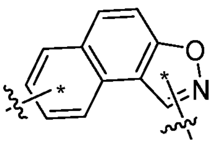, 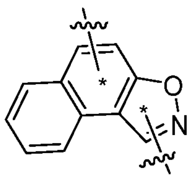, 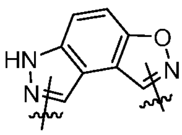, 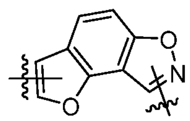,

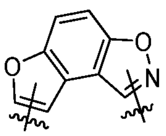, 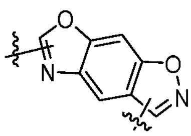, 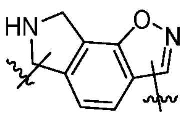, 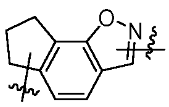, 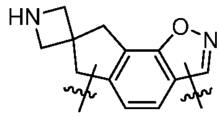,

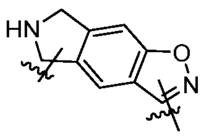, 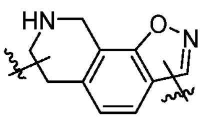, 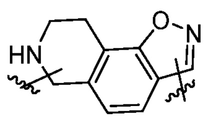, 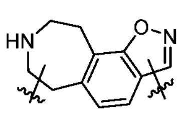, 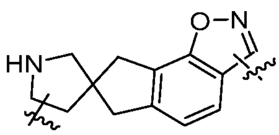,

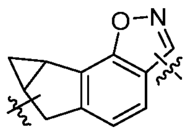, 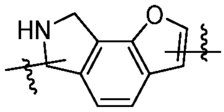, 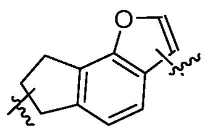, 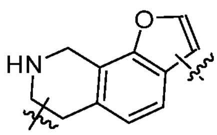, 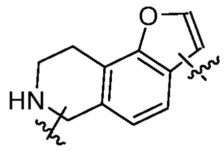,

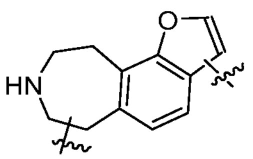, 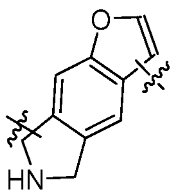, 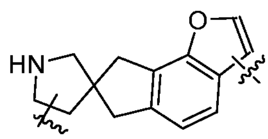, 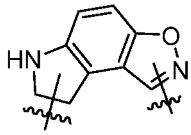, 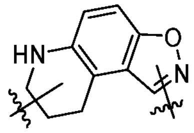,

and

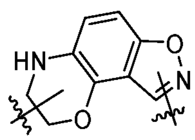;

PTM is 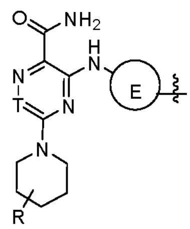;

preferably, PTM is

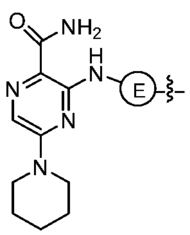, 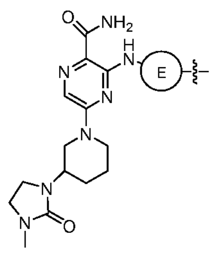, 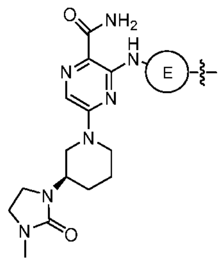,

or

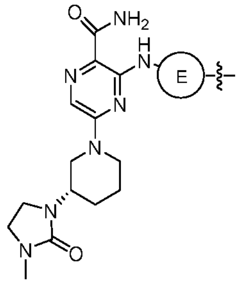

,

wherein

T is selected from the group consisting of CH and N, preferably selected from CH;
R is selected from the group consisting of hydrogen and 5- to 6-membered heterocycloalkyl, wherein the heterocycloalkyl is optionally substituted with =O or $C_{1-6}$ alkyl; preferably, R is selected from the group consisting of hydrogen, imidazolidinonyl, and imidazolidinonyl substituted with $C_{1-4}$ alkyl; more preferably, R is selected from the group consisting of hydrogen, imidazolidinonyl, 1-methyl-imidazolidinonyl, 1-ethyl-imidazolidinonyl, and 1-propyl-imidazolidinonyl;
ring E is selected from the group consisting of phenyl, benzocycloalkenyl, and benzoheterocycloalkenyl; preferably, ring E is selected from the group consisting of phenyl, benzopiperidinyl, benzodihydropyrrolyl, spiro[benzopyran-piperidine], and benzodihydrooxazinopiperazine;
$Cy^1$ is selected from the group consisting of a bond and the following groups optionally substituted with one or more $R^a$: $C_{6-9}$ cycloalkyl or 5- to 11-membered heterocycloalkyl containing 1-3 heteroatoms selected from the group consisting of N, O, and S (e.g., 1-3 or 1-2 heteroatoms selected from the group consisting of N and O);
LNK is selected from the group consisting of a bond and $C_{1-4}$ alkylene; preferably, LNK is selected from the group consisting of a bond and $C_{1-3}$ alkylene;
$Cy^2$ is absent or selected from the group consisting of $C_{4-6}$ cycloalkyl and 4- to 6-membered heterocycloalkyl, wherein the cycloalkyl or heterocycloalkyl is optionally substituted with one or more $R^b$; preferably, $Cy^2$ is absent or selected from the group consisting of $C_{4-6}$ cycloalkyl and 4- to 6-membered heterocycloalkyl, wherein the heterocycloalkyl comprises 1-2 heteroatoms selected from the group consisting of N and O.

In some specific embodiments, the present application relates to a compound of formula I, a stereoisomer thereof, or a pharmaceutically acceptable salt thereof, wherein

ring A is absent or selected from the group consisting of cyclopentenyl, dihydropyrrolyl, tetrahydropyridinyl, tetrahydroazepinyl, and phenyl; preferably, ring A is selected from the group consisting of cyclopentenyl, dihydropyrrolyl, tetrahydropyridinyl, and tetrahydroazepinyl, and more preferably, ring A is selected from the group consisting of dihydropyrrolyl, tetrahydropyridinyl, and tetrahydroazepinyl;

preferably, the structural moiety

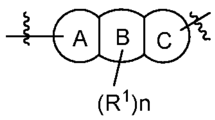

is selected from the group consisting of

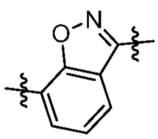

,

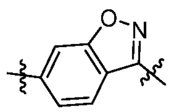, 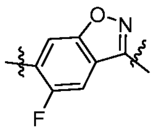, 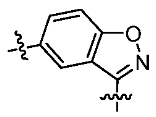, 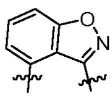, 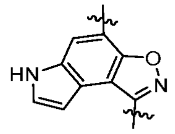, 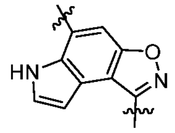, 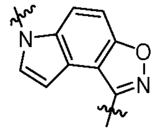,

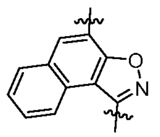, 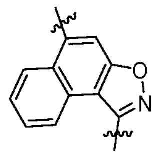, 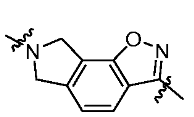, 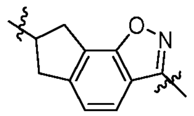, 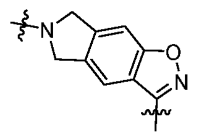, 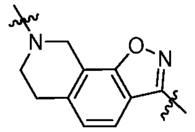,

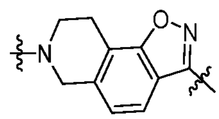, and 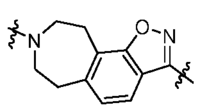;

PTM is

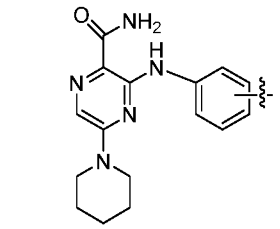, 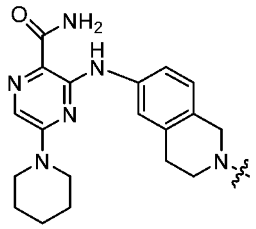, 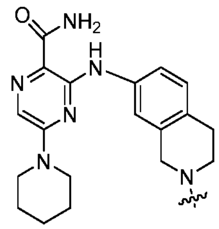, 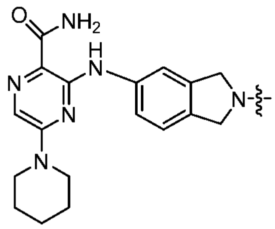,

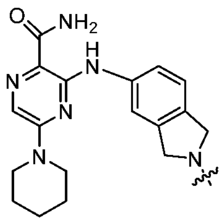, 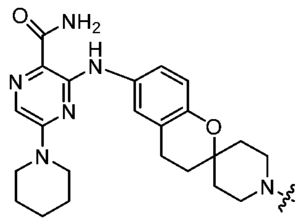, 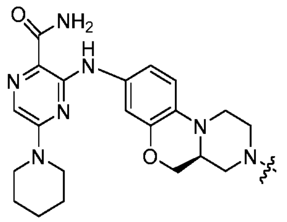, 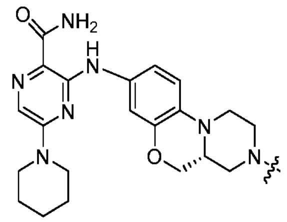,

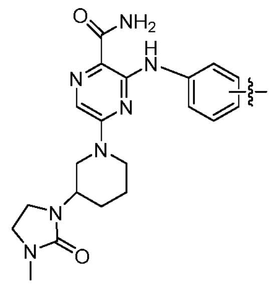, 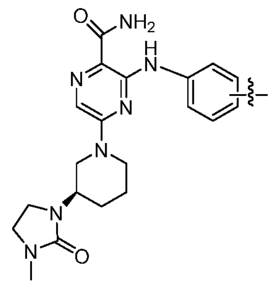, 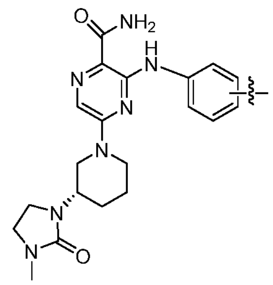, 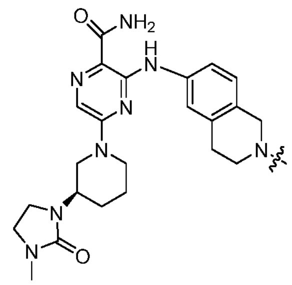,

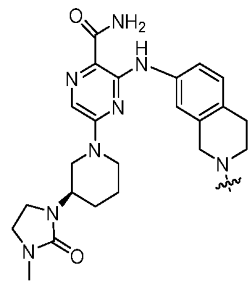, 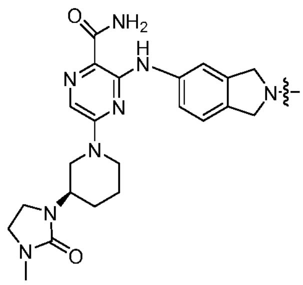, 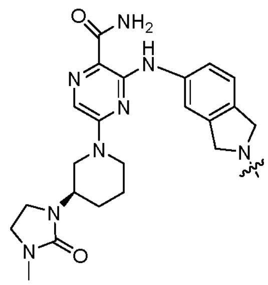, 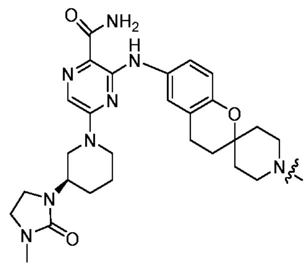,

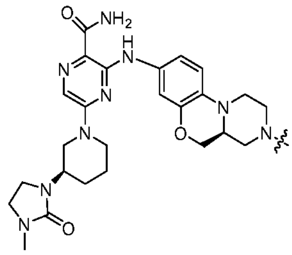, or 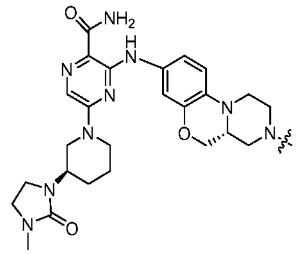;

preferably, PTM is

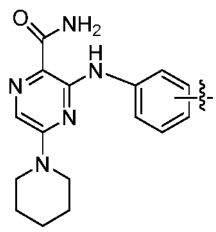,

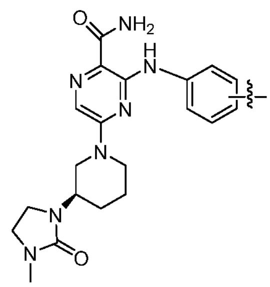, 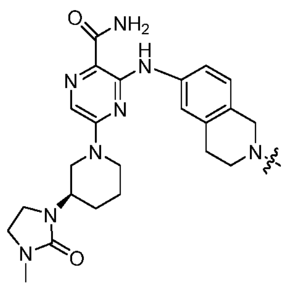, 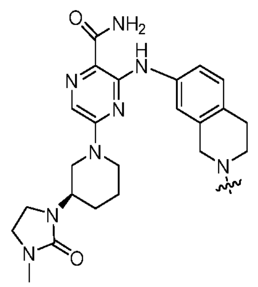, 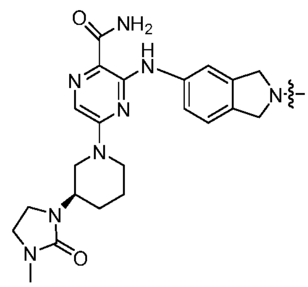,

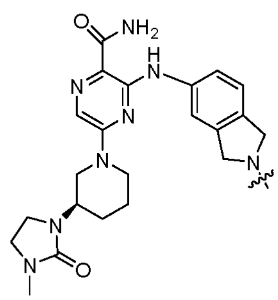, 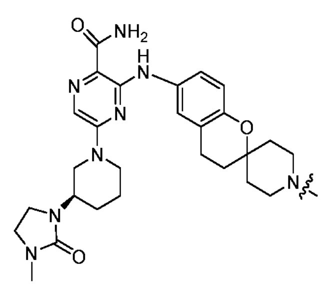, or 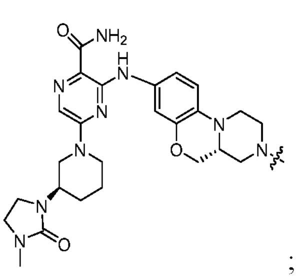;

$Cy^1$ is selected from the group consisting of a bond, piperidinyl, cyclohexyl, spirononanyl, monoazaspirononanyl, octahydrocyclopentapyrrolyl, monoazaspiroundecanyl, monooxamonoazaspiroundecanyl, and azabicyclononanyl;

preferably, $Cy^1$ is selected from the group consisting of a bond,

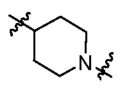, 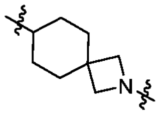, 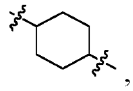,

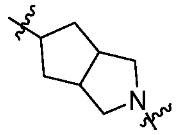, 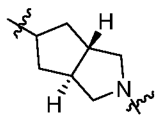, 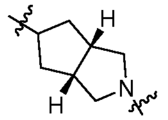, 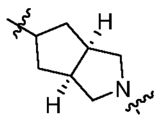, 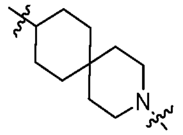, 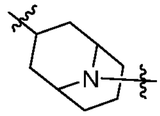,

and

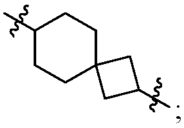;

LNK is selected from the group consisting of a bond and $C_{1-3}$ alkylene; preferably, LNK is selected from the group consisting of a bond and $-CH_2-$;

In some embodiments, $Cy^2$ is absent or selected from the group consisting of cyclobutyl, cyclopentyl, cyclohexyl, azetidinyl, pyrrolidinyl, and piperidinyl, wherein the cyclobutyl, cyclopentyl, azetidinyl, pyrrolidinyl, or piperidinyl is optionally substituted with one or more $R^b$; preferably, $Cy^2$ is absent or selected from the group consisting of

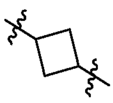, 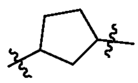, 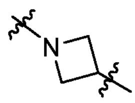, 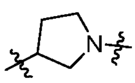, 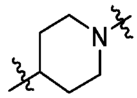, and 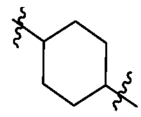.

In some embodiments, the heteroatom of the heterocycloalkenyl or heterocycloalkyl is selected from the group consisting of N, NH, O, and S. In some embodiments, the heteroatom of the heterocycloalkenyl or heterocycloalkyl is selected from the group consisting of N, O, and S. In some embodiments, the heteroatom of the heteroaryl is selected from the group consisting of N, O, and S. In some embodiments, the heteroatom of the heteroalkylene is selected from the group consisting of N, NH, O, S, S(O), and $S(O)_2$.

In some embodiments, the number of the heteroatom of the heterocycloalkenyl, heterocycloalkyl, heteroalkylene, or heteroaryl is selected from the group consisting of 1, 2, 3, 4, 5, and 6. In some embodiments, the number of the heteroatom of the heterocycloalkenyl, heterocycloalkyl, heteroalkylene, or heteroaryl is selected from the group consisting of 1, 2, 3, and 4. In some embodiments, the number of the heteroatom of the heterocycloalkenyl, heterocycloalkyl, heteroalkylene, or heteroaryl is selected from the group consisting of 1, 2, and 3.

The present application also relates to compounds of formula I'-1A, formula I'-2A, formula I'-1A-1, formula I'-2A-1, formula I'-3A-1, formula I'-3A-2, formula I'-4A-1, and formula I'-4A-2, stereoisomers thereof, or pharmaceutically acceptable salts thereof,

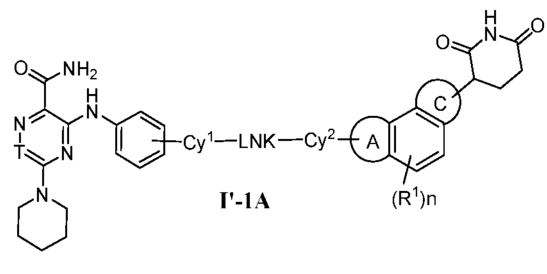
I'-1A

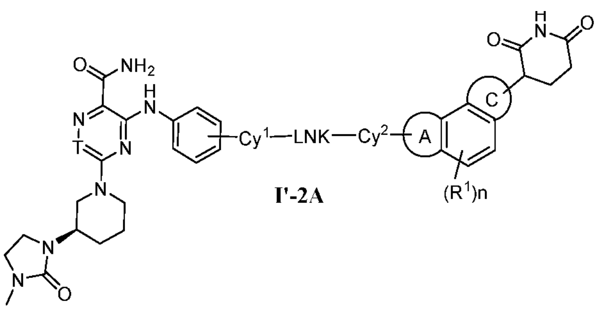
I'-2A

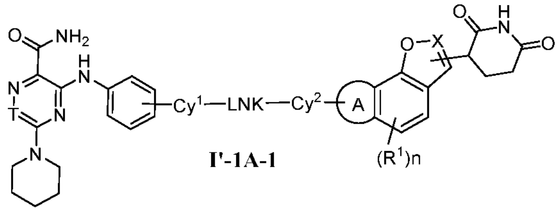
I'-1A-1

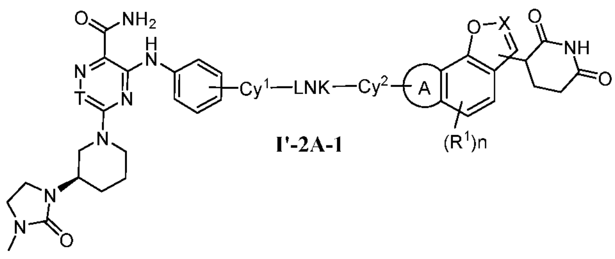
I'-2A-1

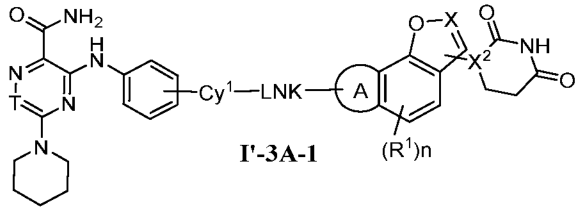
I'-3A-1

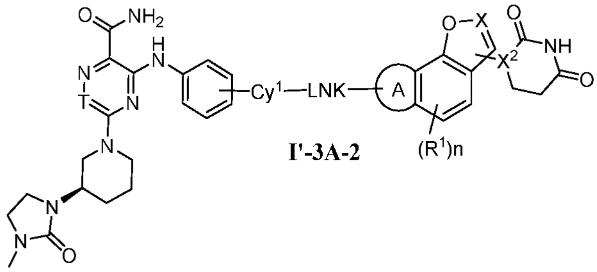
I'-3A-2

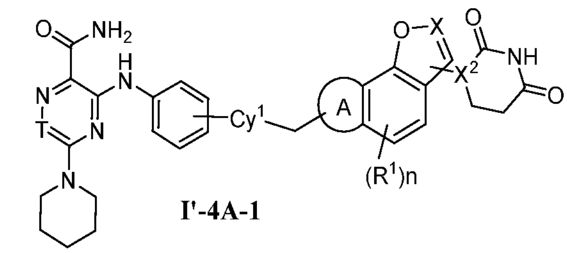
I'-4A-1

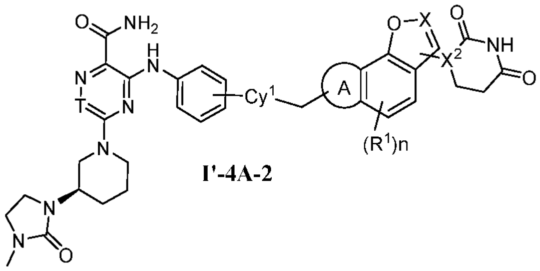
I'-4A-2

wherein ring A, ring C, $Cy^1$, $Cy^2$, LNK, $R^1$, n, T, and $X^2$ are as defined herein;
X is selected from the group consisting of CH and N.

In some embodiments, the structural moiety

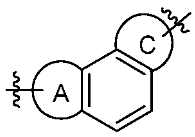

is selected from the group consisting of

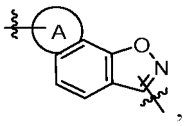, 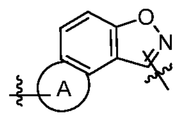, 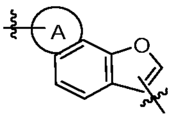, and 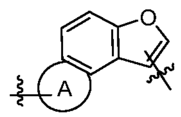.

In some embodiments, the structural moiety

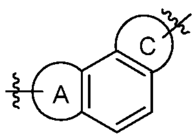

is selected from the group consisting of

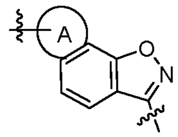 , 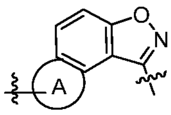 ,

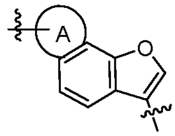 , and 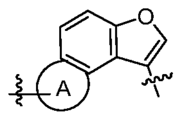 .

In some embodiments, the structural moiety

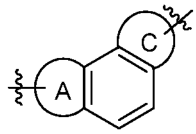 or 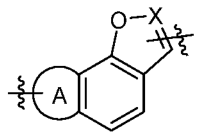

is selected from the group consisting of

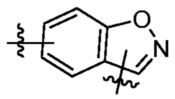 , 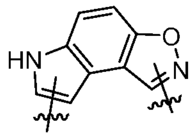 , 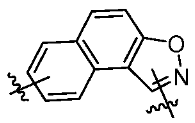 , 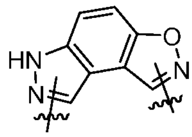 , 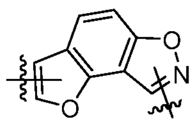 ,

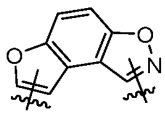 , 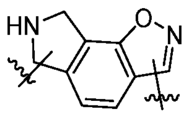 , 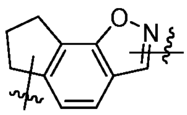 , 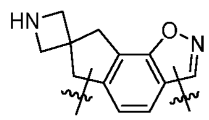 , 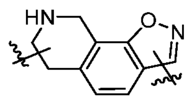 , 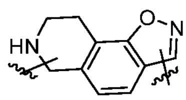 ,

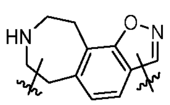 , 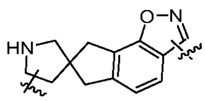 , 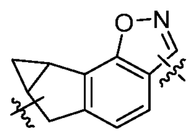 , 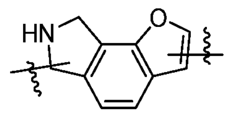 , 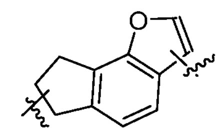 ,

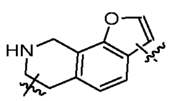 , 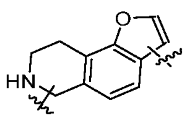 , 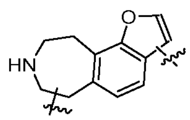 , 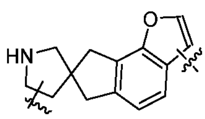 , 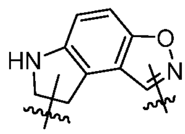 , 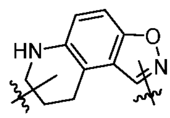 ,

and

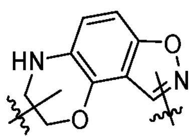 .

In some specific embodiments, the structural moiety

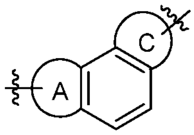 or 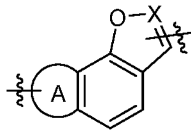

is selected from the group consisting of

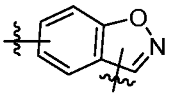, 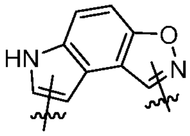, 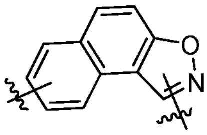, 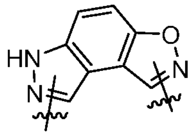, 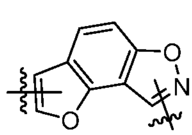,

and

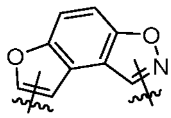.

In some specific embodiments, the structural moiety

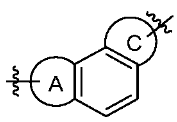 or 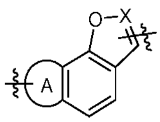

is selected from the group consisting of

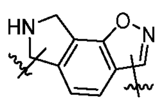, 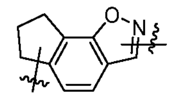, 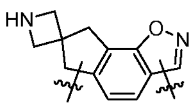, 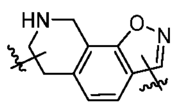,

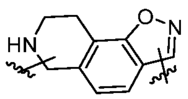, 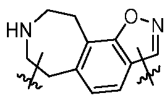, 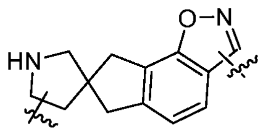, 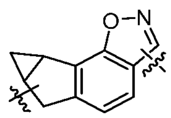, 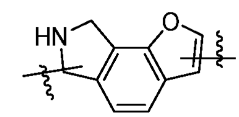,

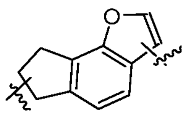, 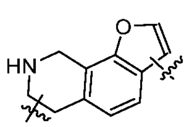, 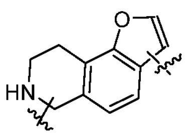, 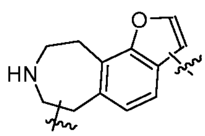, 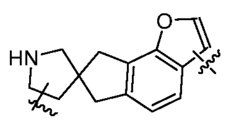,

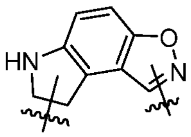, 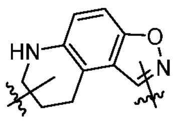, and 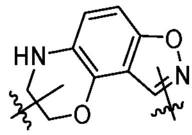.

In some embodiments, the structural moiety

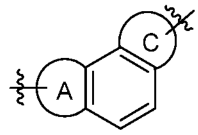 or 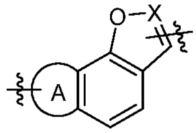

is selected from the group consisting of

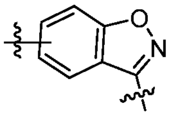, 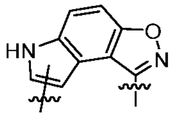, 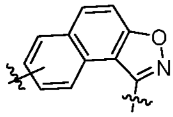, 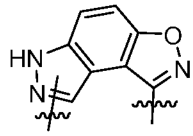, 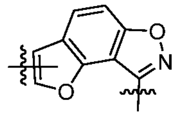,

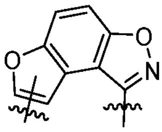, 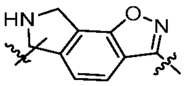, 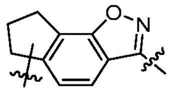, 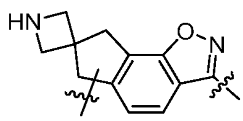, 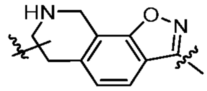,

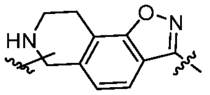, 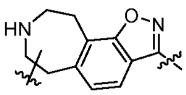, 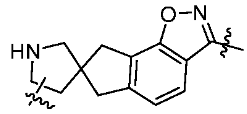, 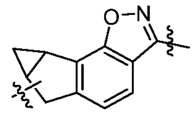, 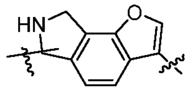,

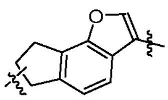, 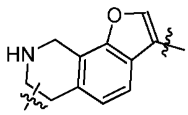, 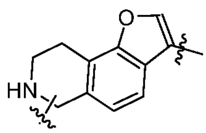, 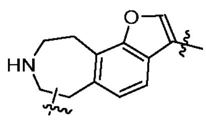, 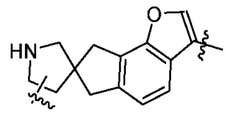,

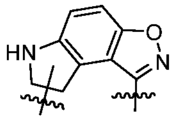, 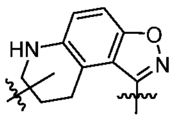, and 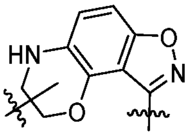.

In some specific embodiments, the structural moiety

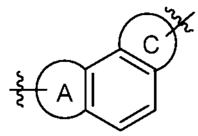 or 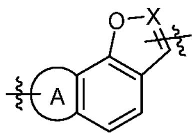

is selected from the group consisting of

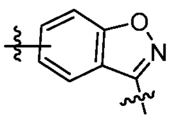, 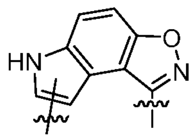, 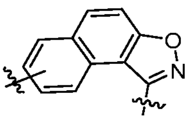, 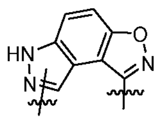, 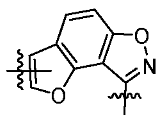,

and

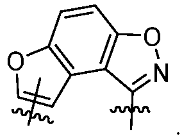.

In some specific embodiments, the structural moiety

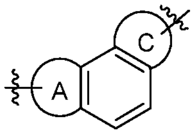 or 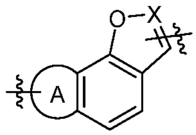

is selected from the group consisting of

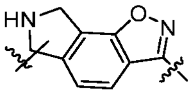, 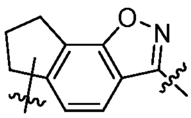, 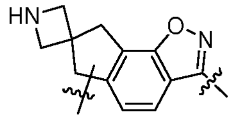,

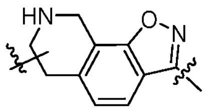, 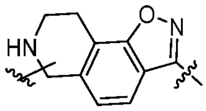, 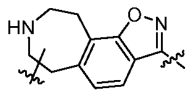, 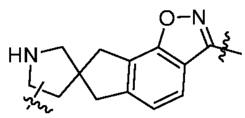, 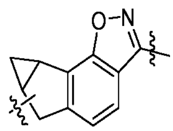,

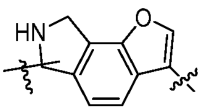, 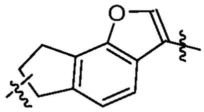, 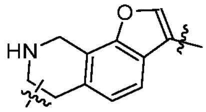, 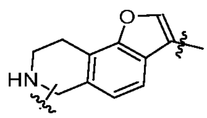, 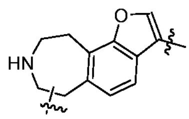,

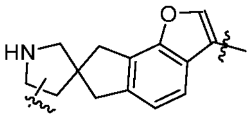, 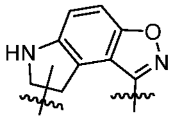, 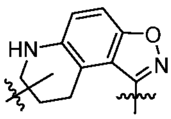, and 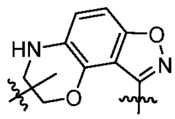.

In some embodiments, the structural moiety

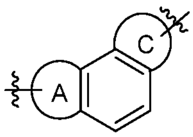 or 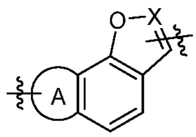

is selected from the group consisting of

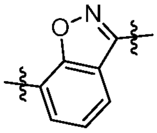, 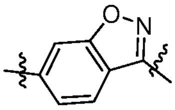, 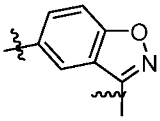, 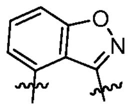, 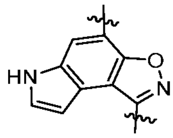, 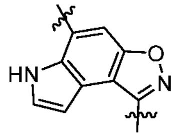,

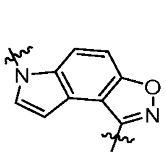, 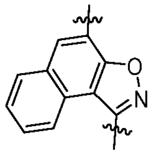, 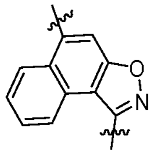, 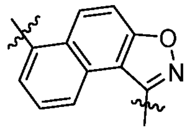, 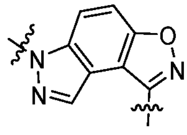, 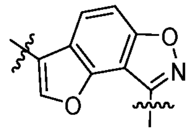,

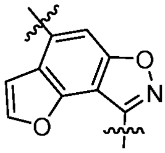, 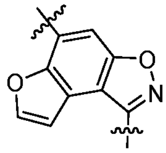, 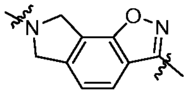, 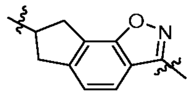, 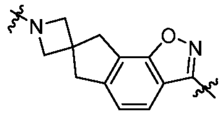, 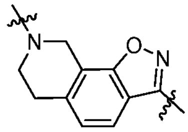,

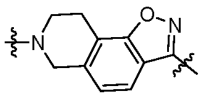, 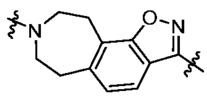, 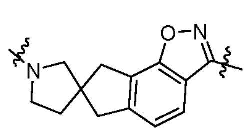, 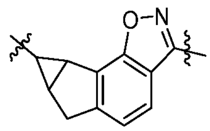, 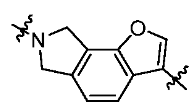,

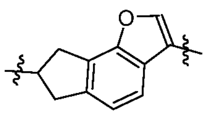, 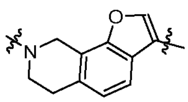, 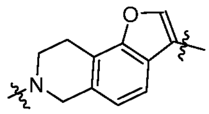, 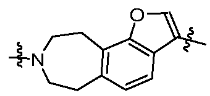, 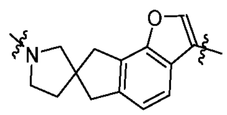,

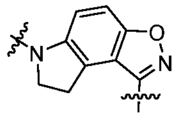, 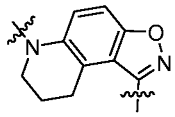, and 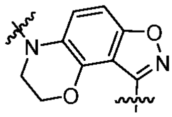.

In some specific embodiments, the structural moiety

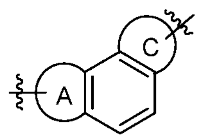 or 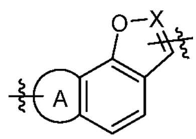

is selected from the group consisting of

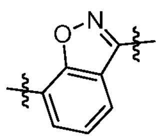, 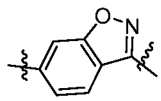, 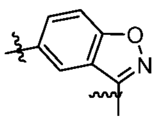, 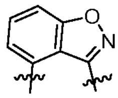, 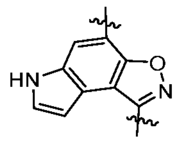, 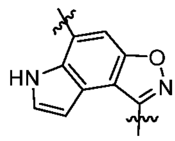,

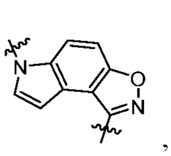, 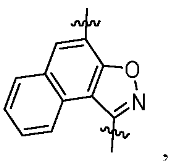, 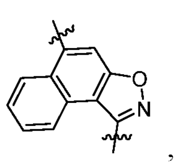, 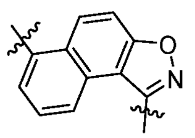, 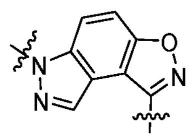, 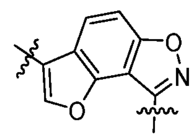,

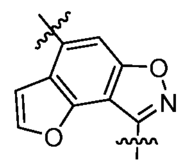, and 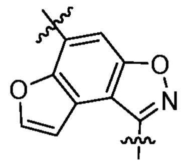.

In some specific embodiments, the structural moiety

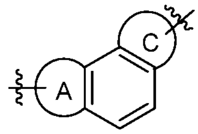

or

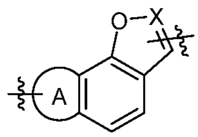

is selected from the group consisting of

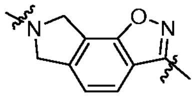, 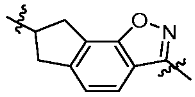,

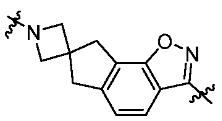, 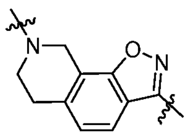, 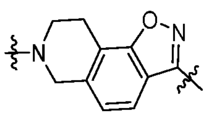, 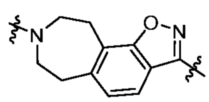, 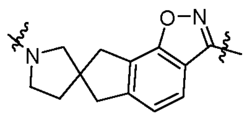,

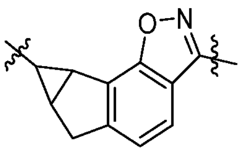, 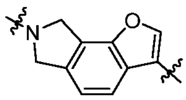, 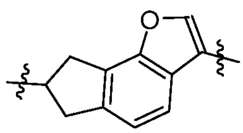, 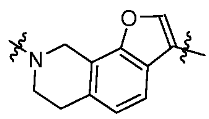, 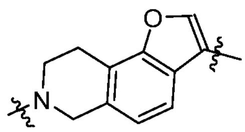,

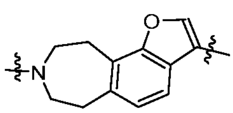, 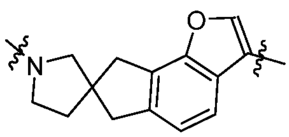, 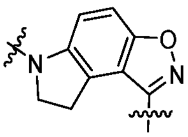, 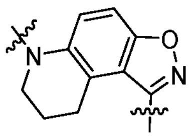, and 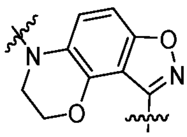.

In some specific embodiments, the structural moiety

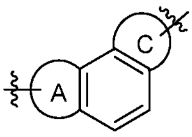 or 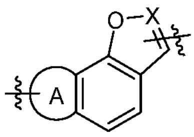

is selected from the group consisting of

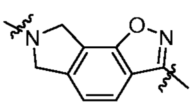, 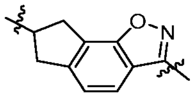, 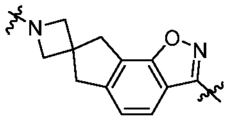, 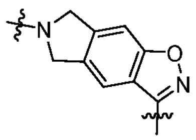,

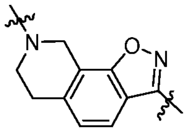, 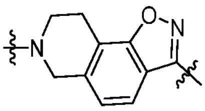, 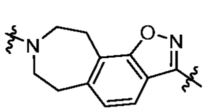, 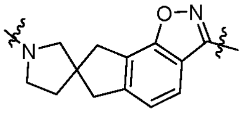, 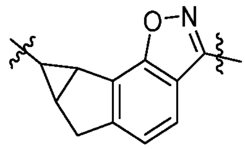,

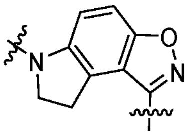, 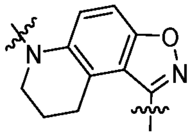, and 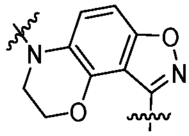.

In some other embodiments, the structural moiety

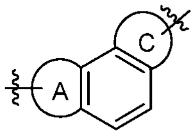 or 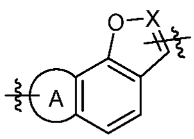

is selected from the group consisting of

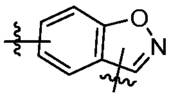, 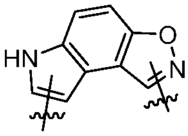, 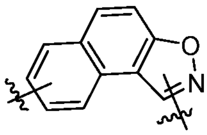, 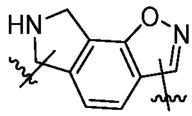, 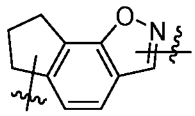,

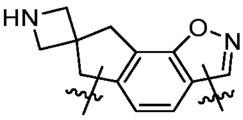, 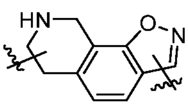, 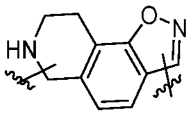, 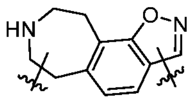, and 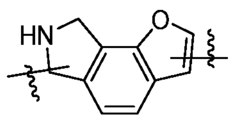.

In some other embodiments, the structural moiety

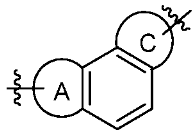 or 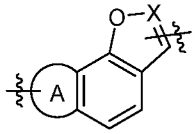

is selected from the group consisting of

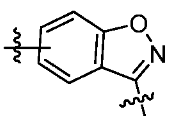, 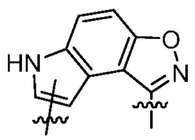, 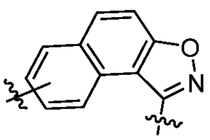, 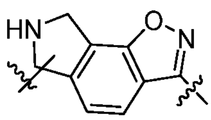,

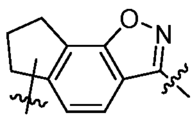, 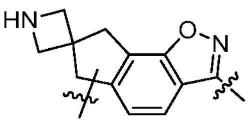, 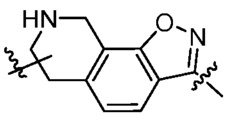, 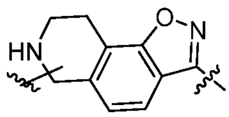, 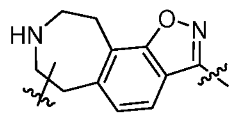,

and

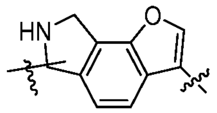.

In some other embodiments, the structural moiety

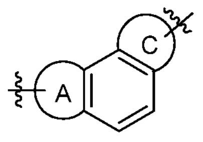 or 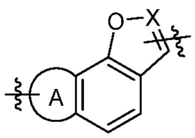

is selected from the group consisting of

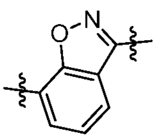, 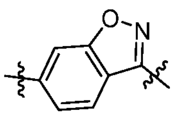, 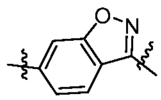, 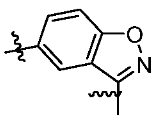, 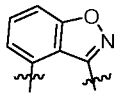, 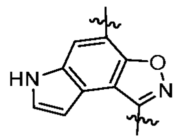,

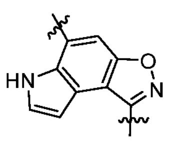, 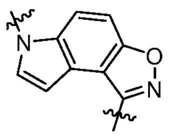, 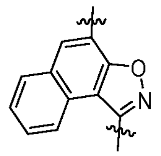, 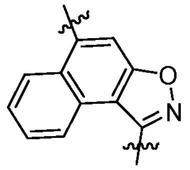, 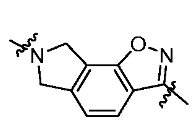, 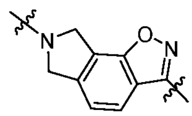,

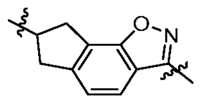, 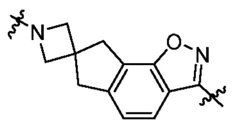, 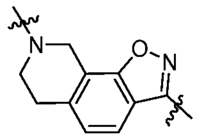, 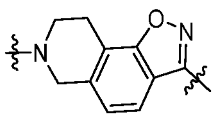, 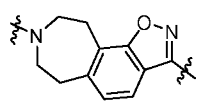,

and

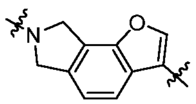.

In some embodiments, the structural moiety -Cy'-LNK-, -LNK-$Cy^2$-, -$Cy^1$-$Cy^2$-, or -$Cy^1$-LNK-$Cy^2$- are as described herein.

The present application also relates to a compound of a formula selected from the group consisting of the following formulas, a stereoisomer thereof, and a pharmaceutically acceptable salt thereof:

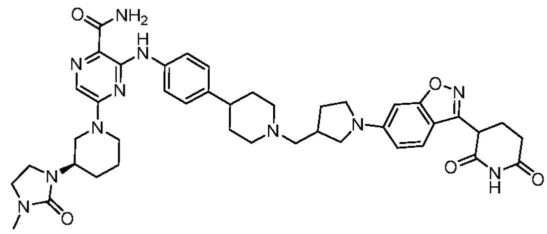

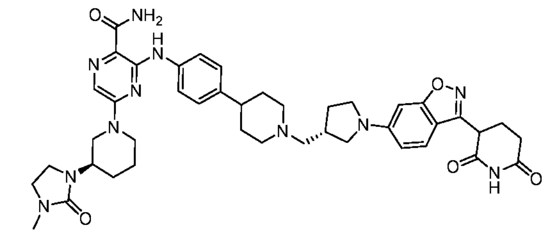

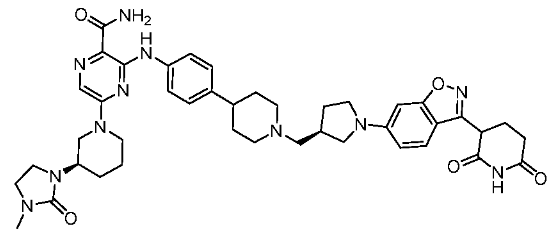

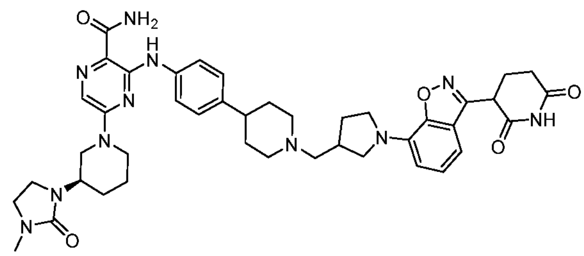

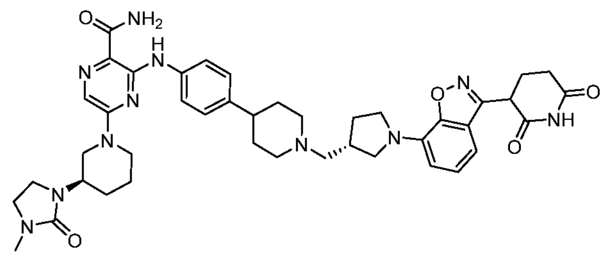

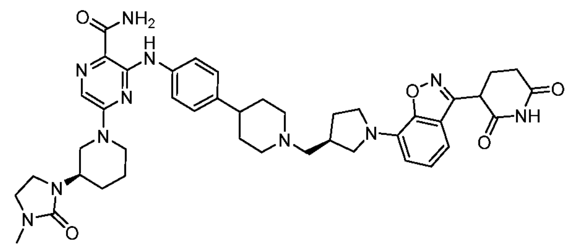

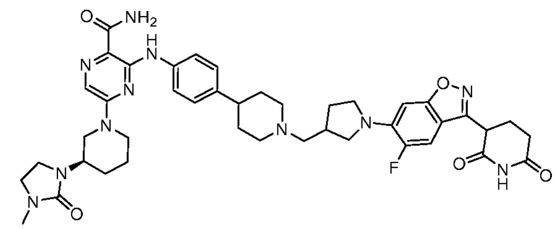

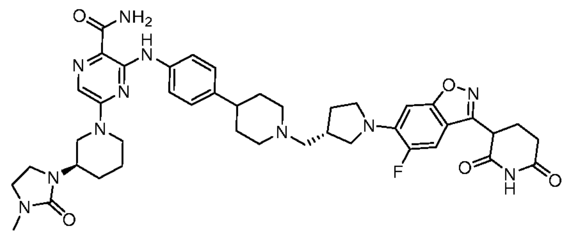

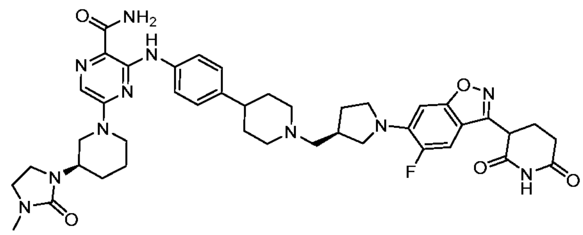

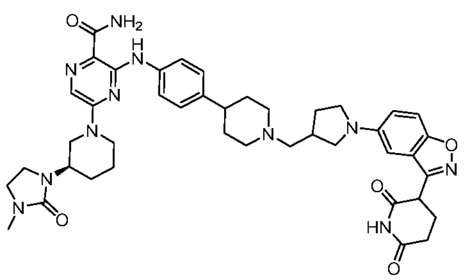

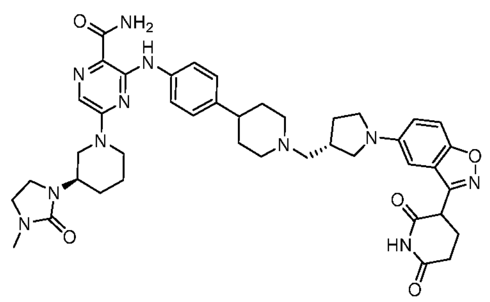

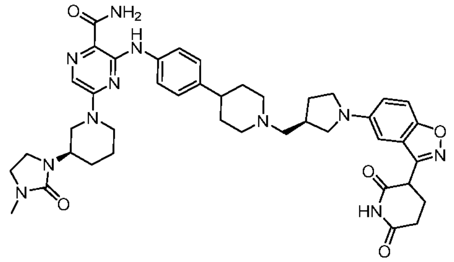

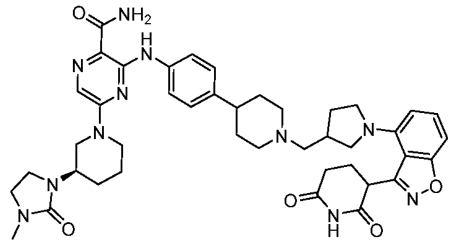

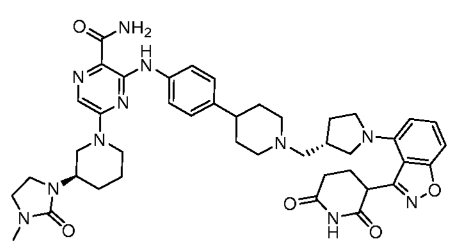

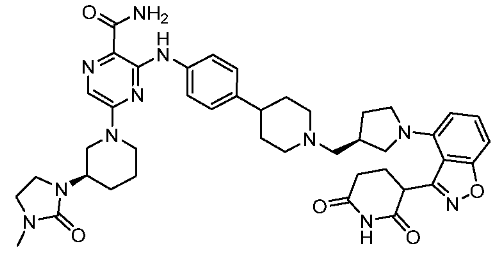

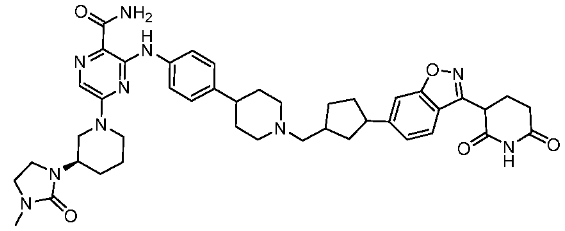

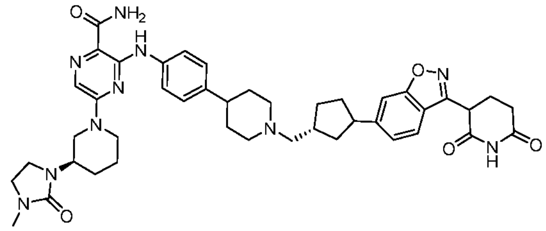

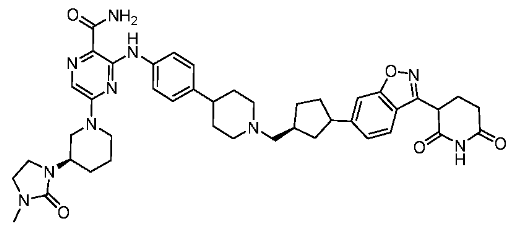

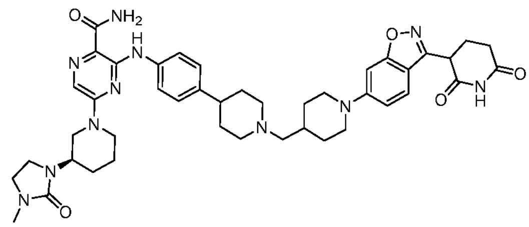

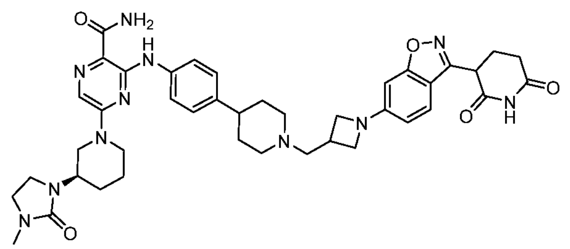

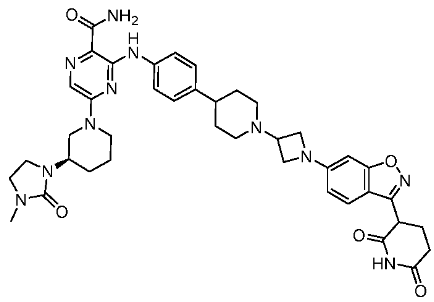

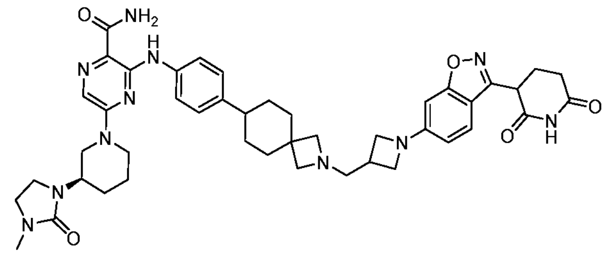

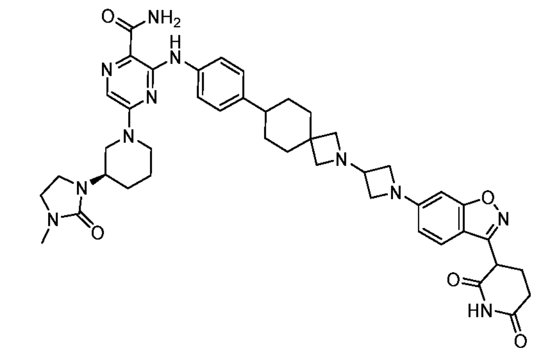

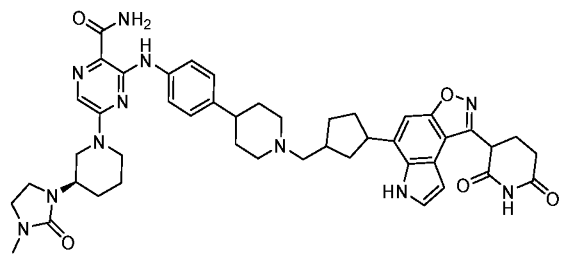

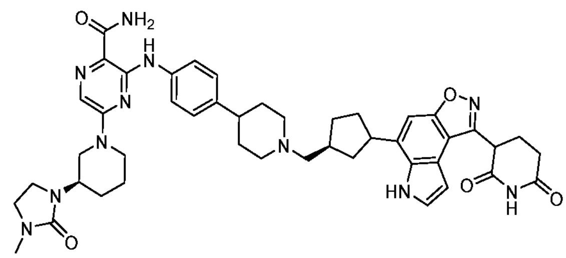

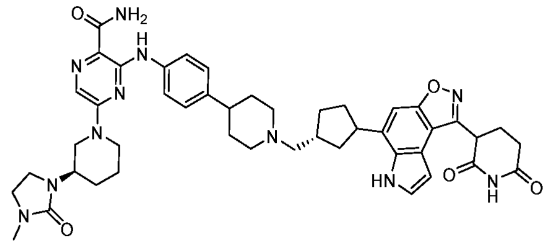

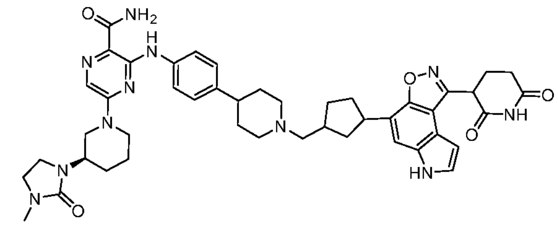

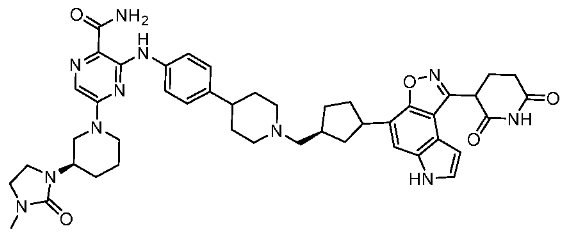

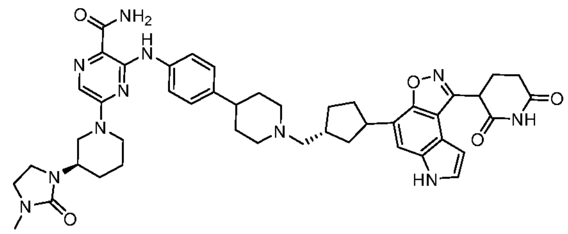

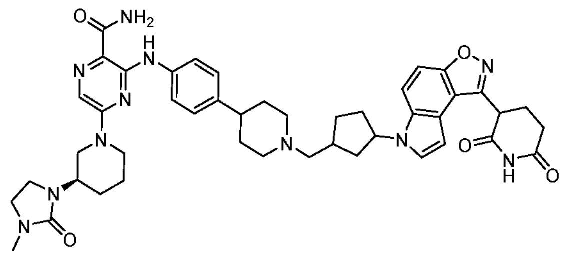

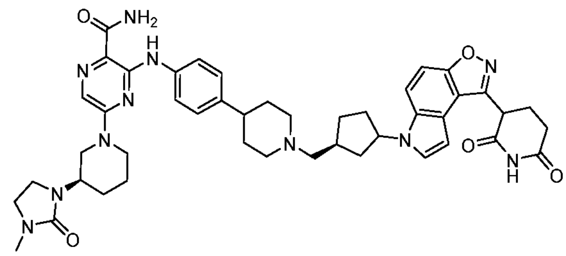

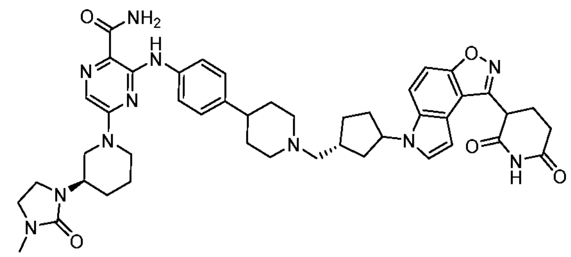

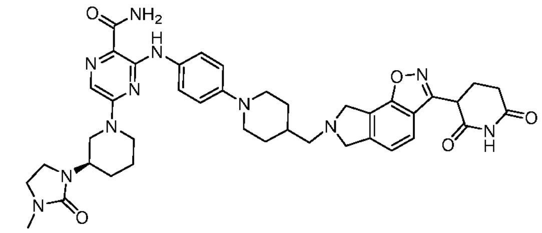

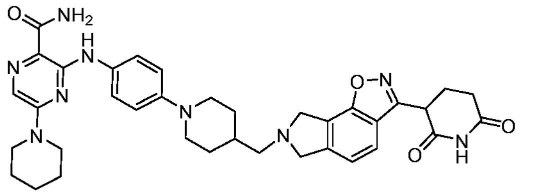

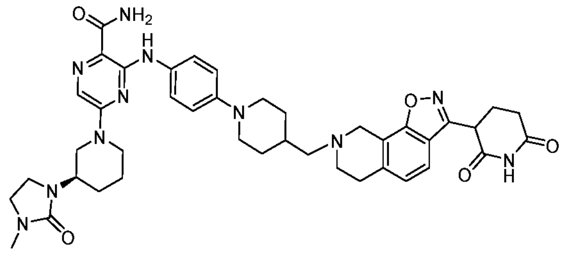

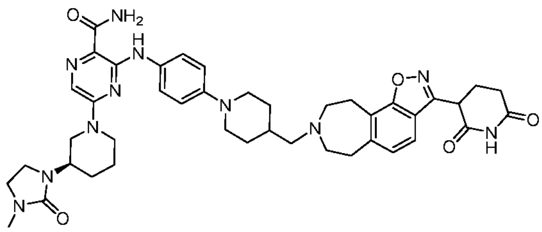

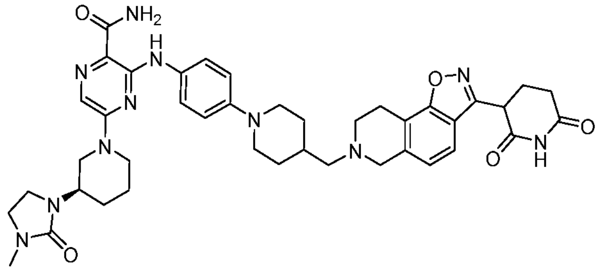

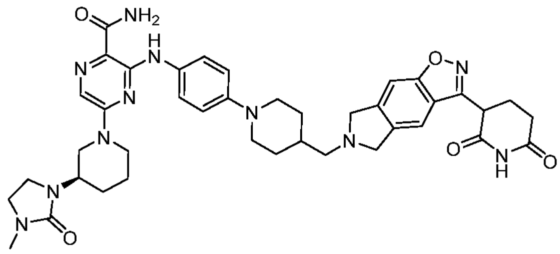

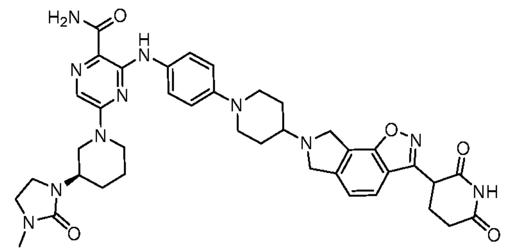

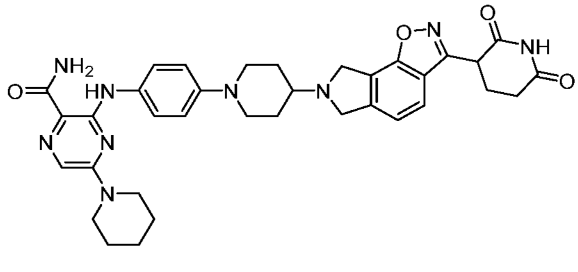

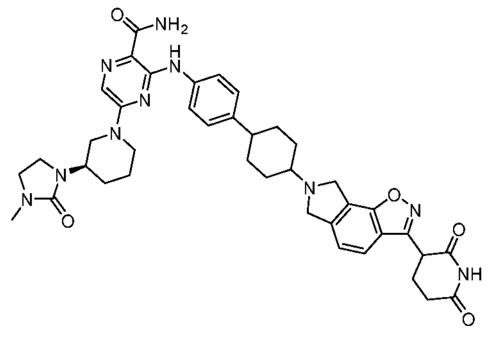

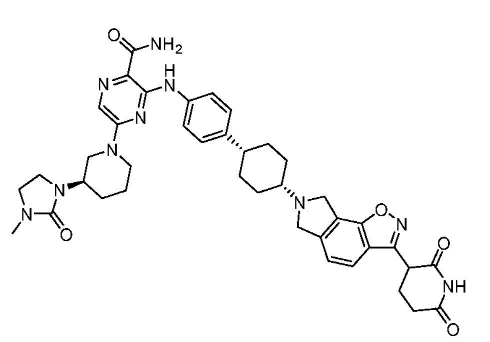

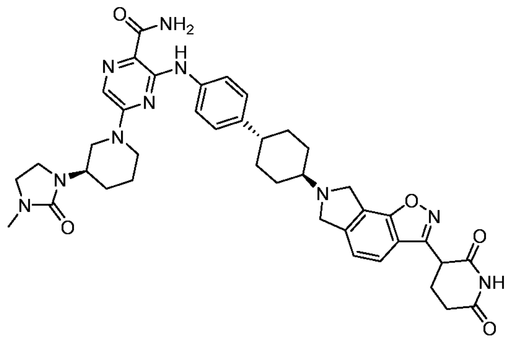

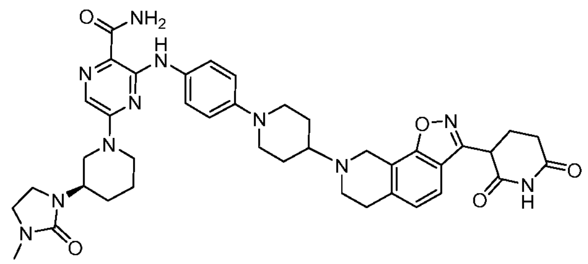

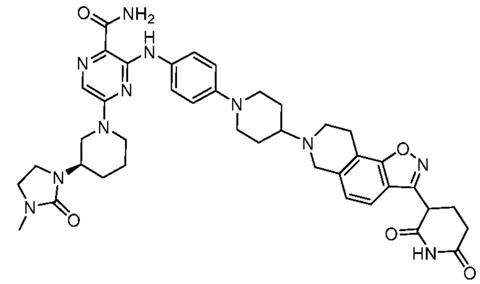

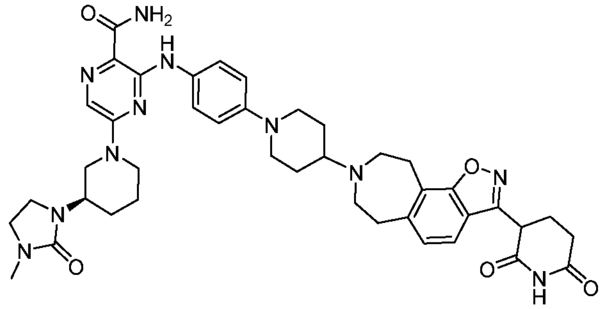

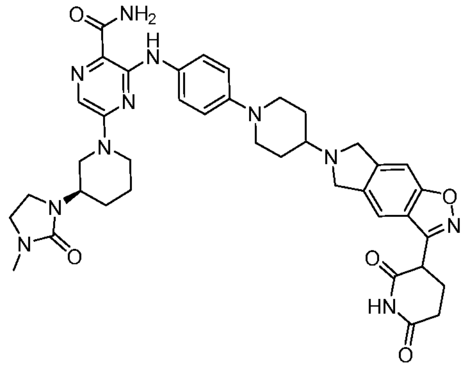

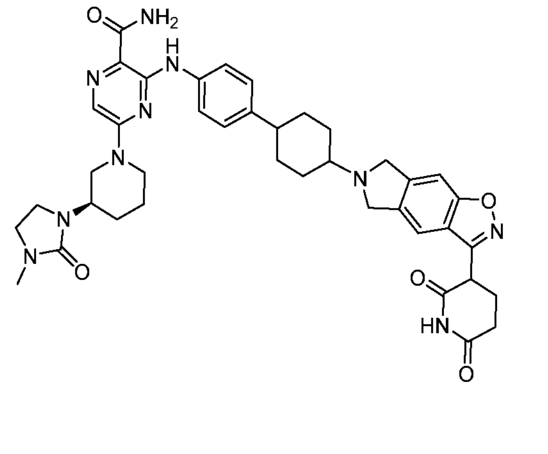

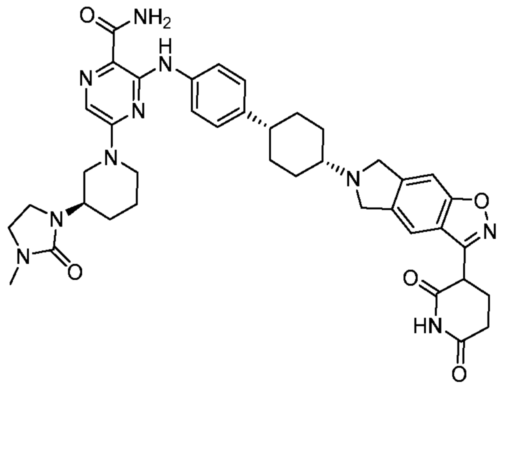

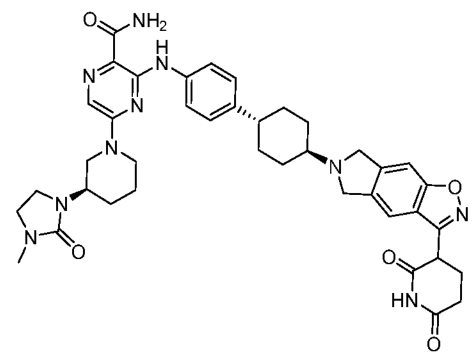

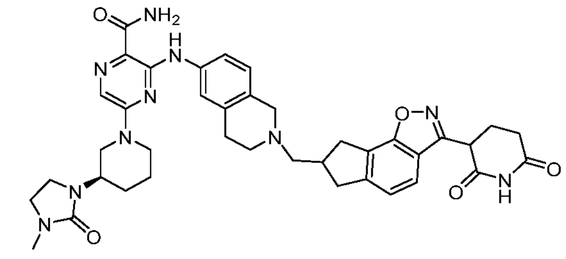

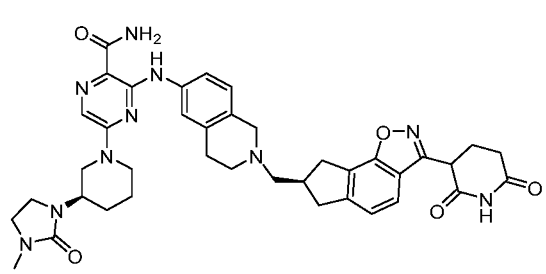

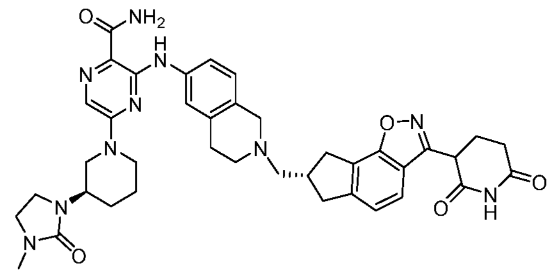

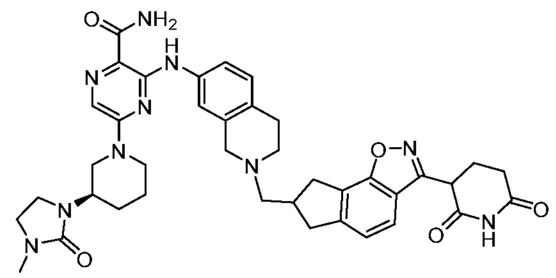

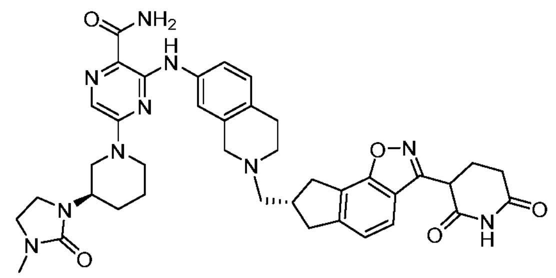

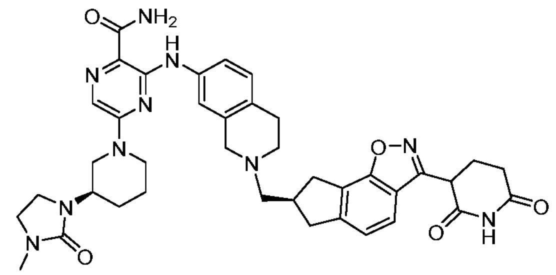

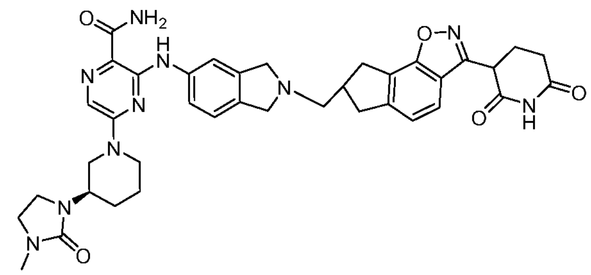

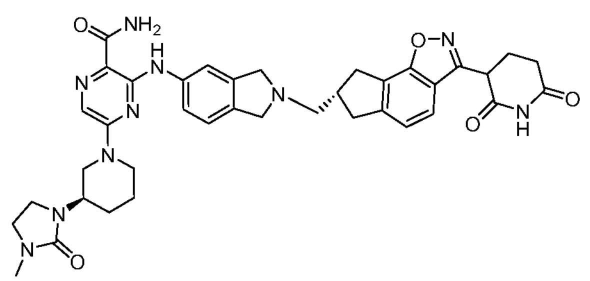

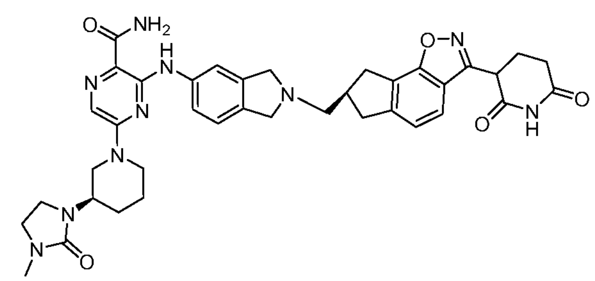

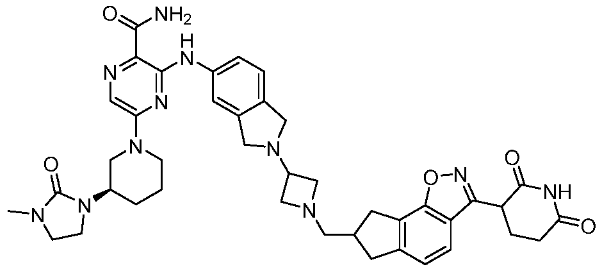

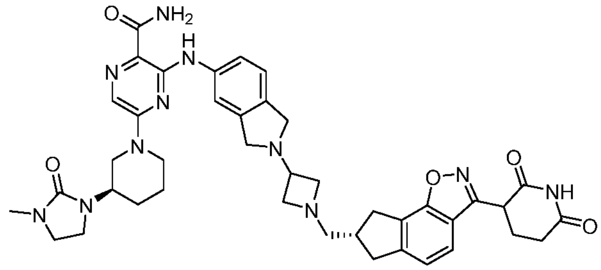

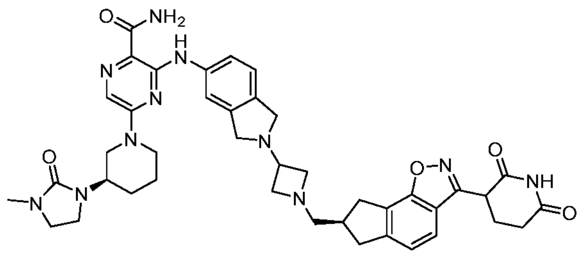

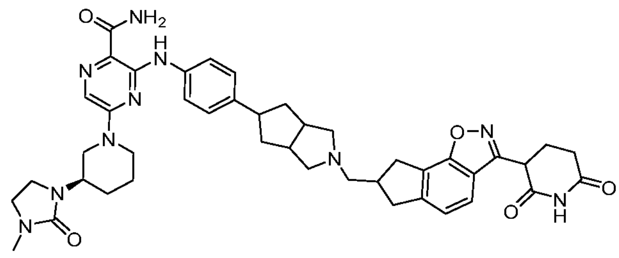

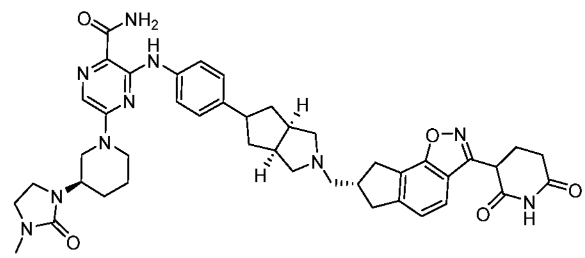

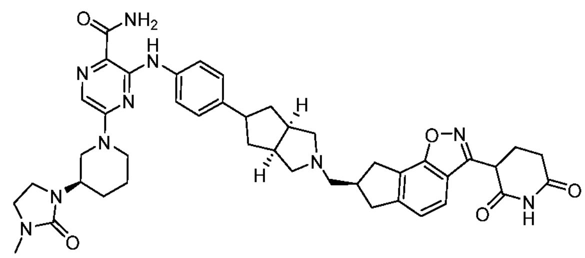

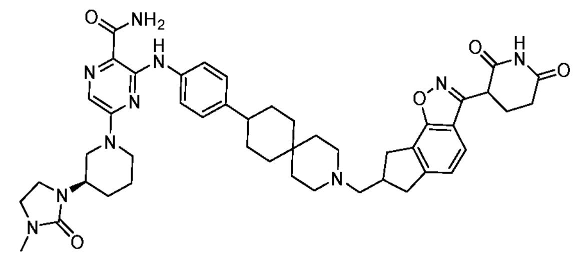

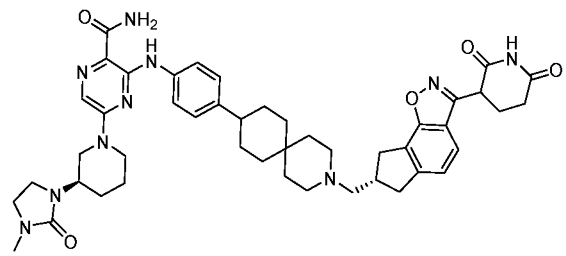

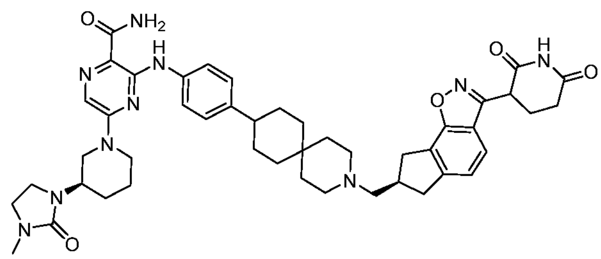

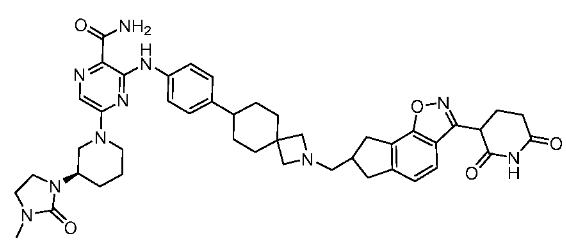

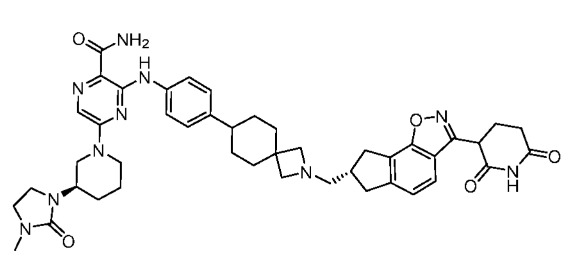

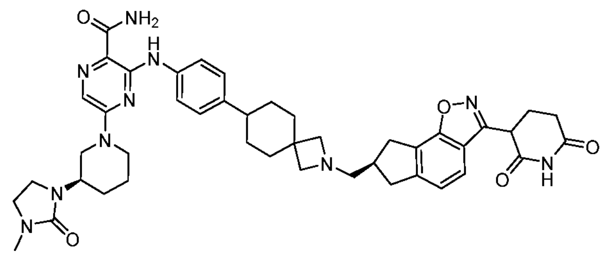

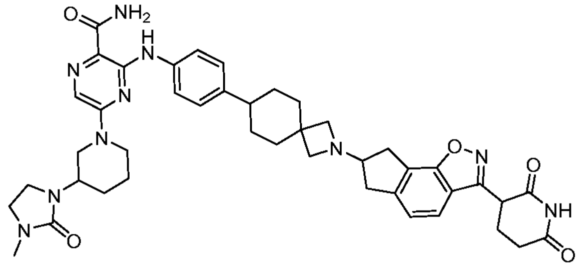

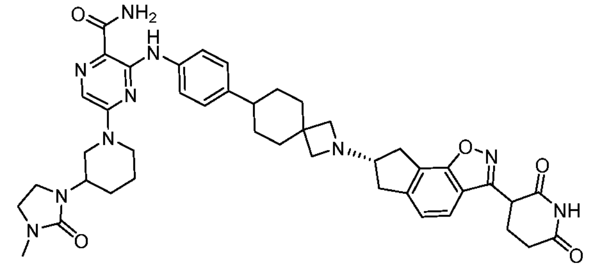

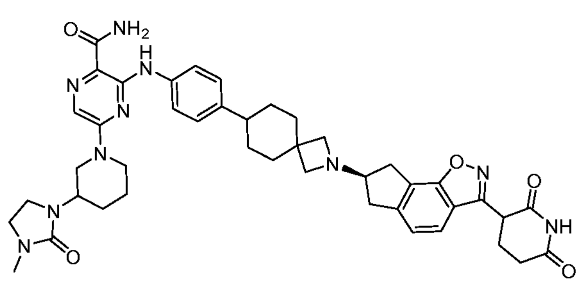

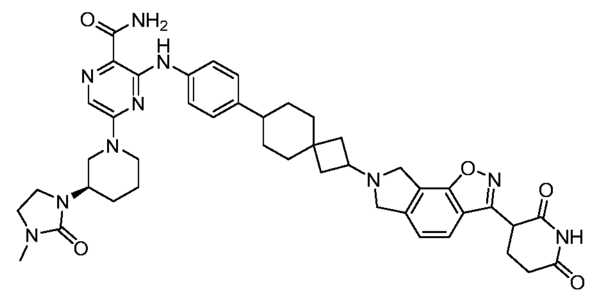

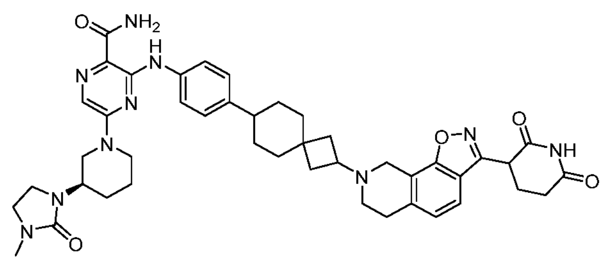

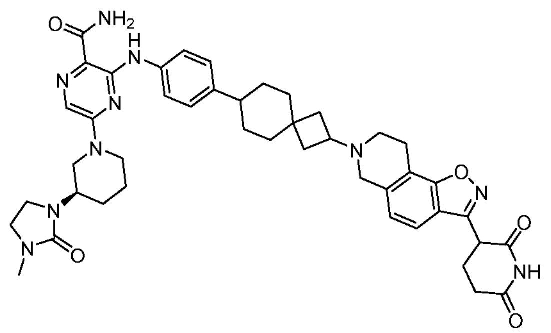

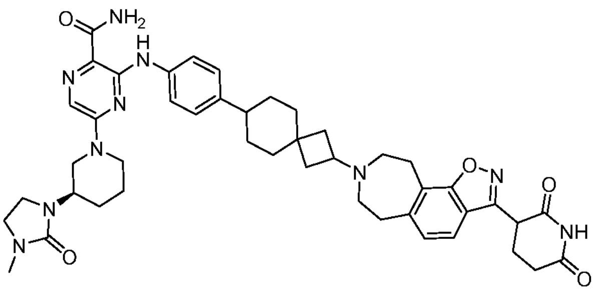

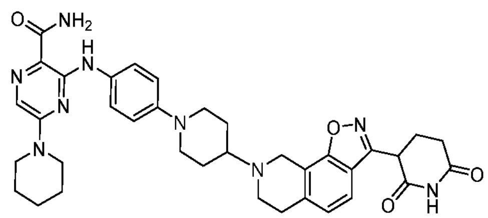

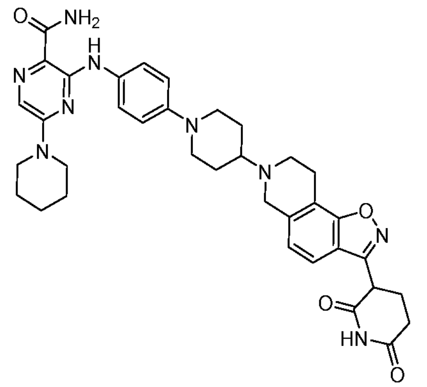

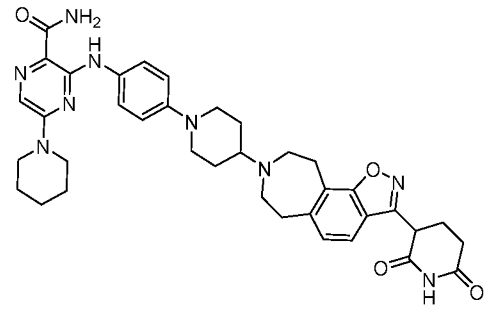

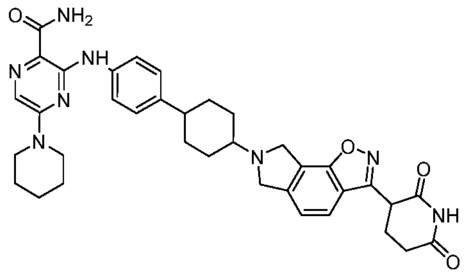

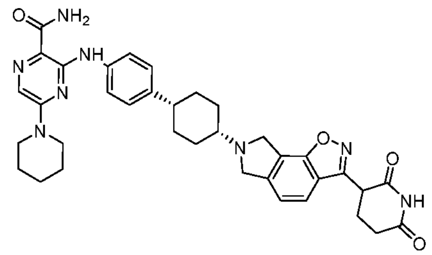

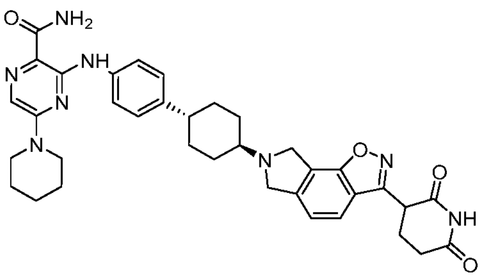

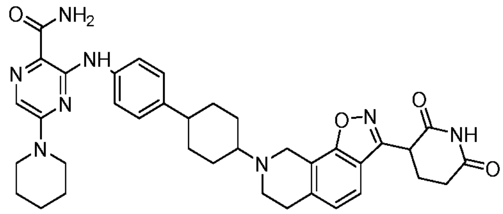

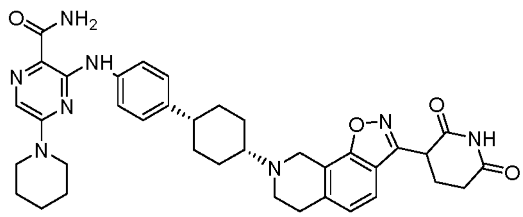

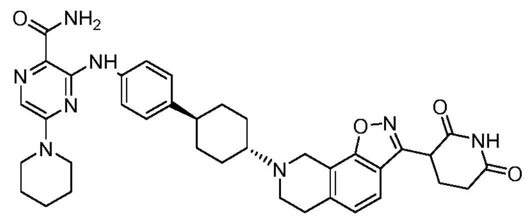

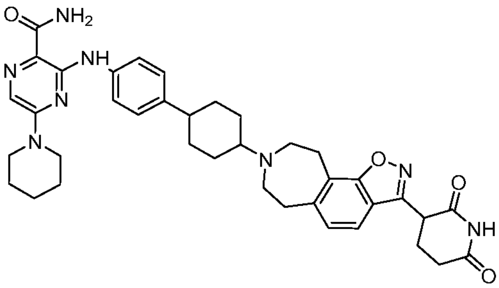

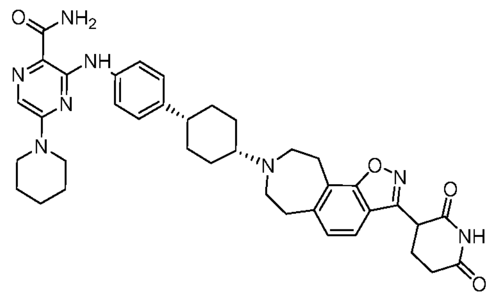

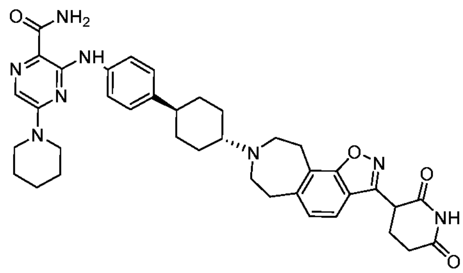

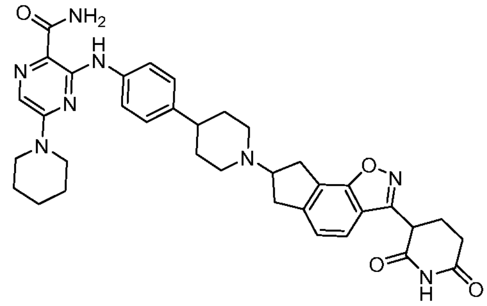

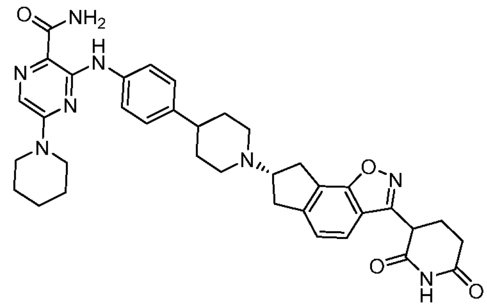

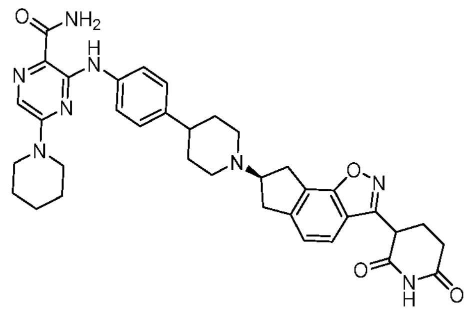

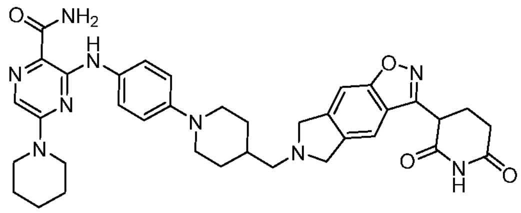

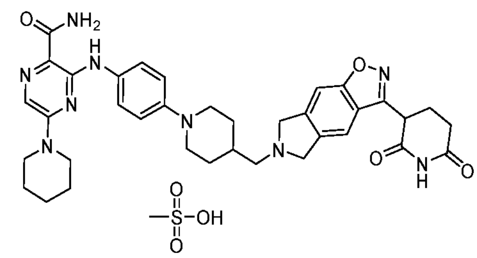

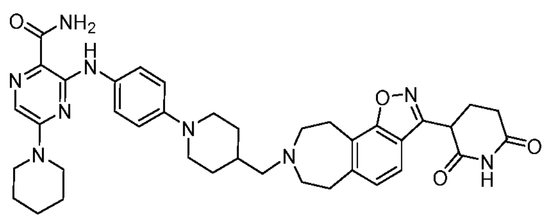

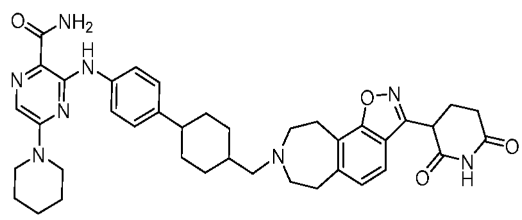

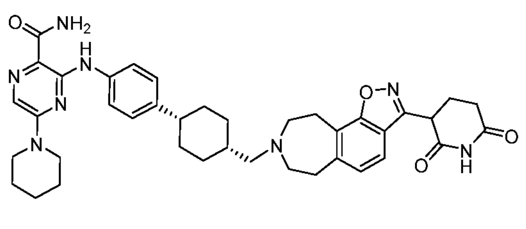

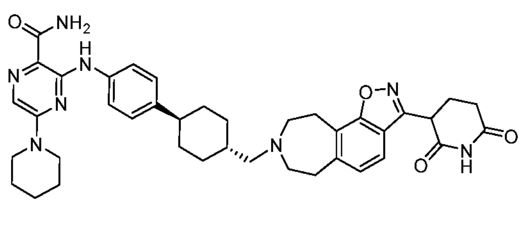

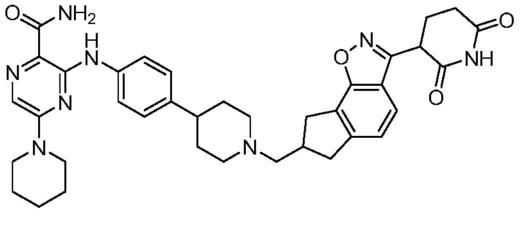

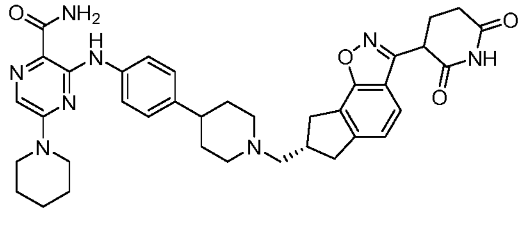

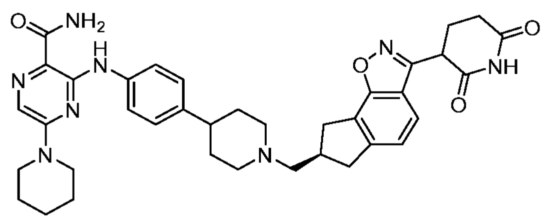

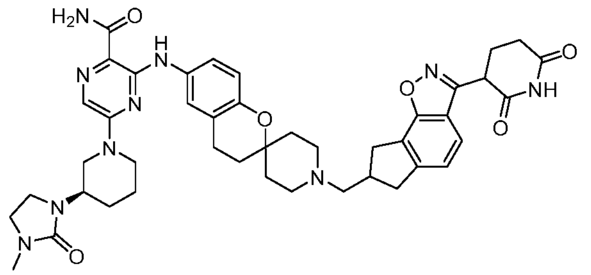

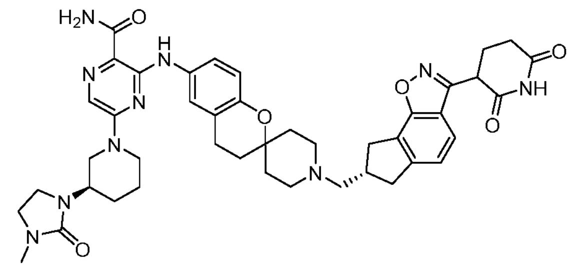

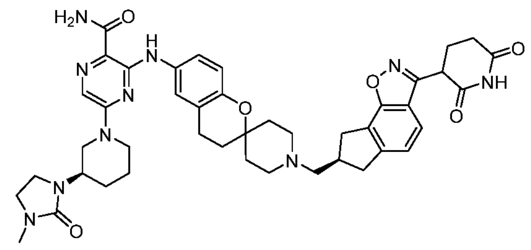

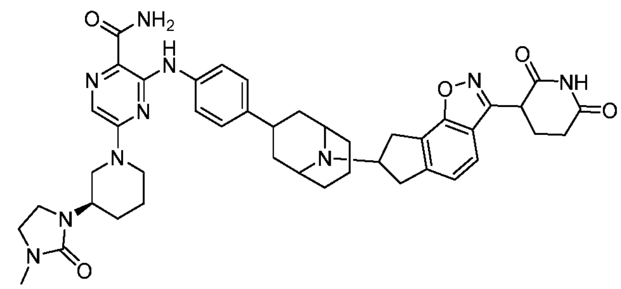

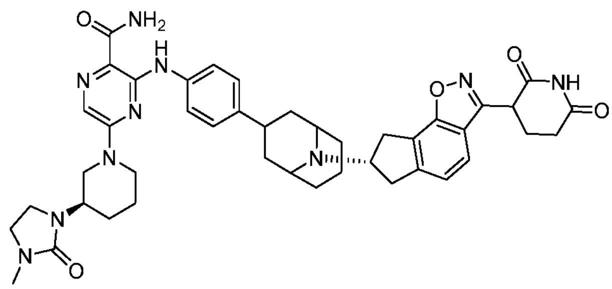

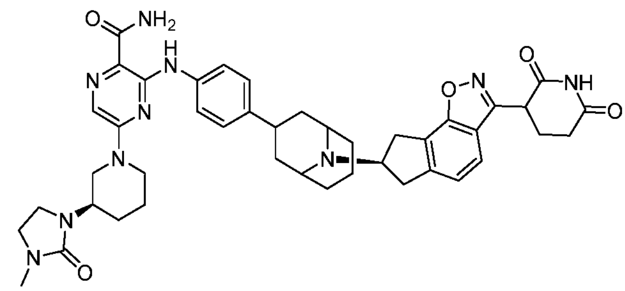

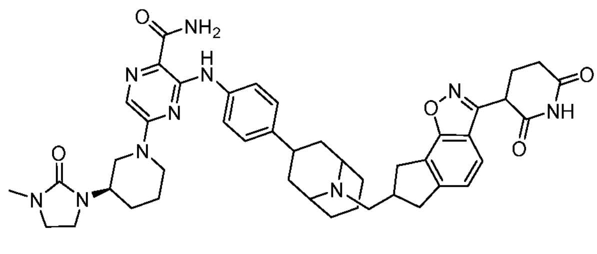

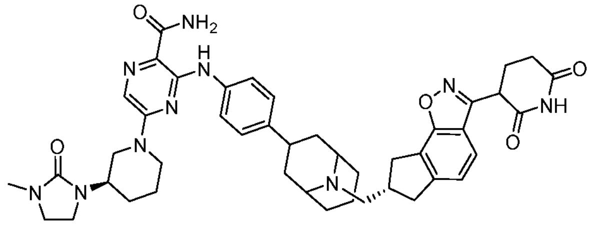

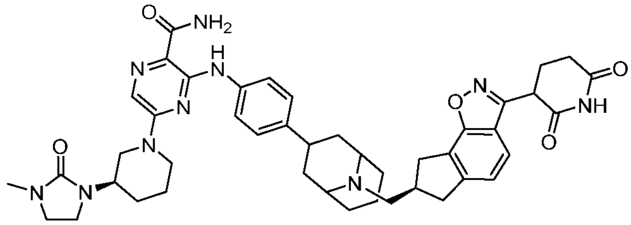

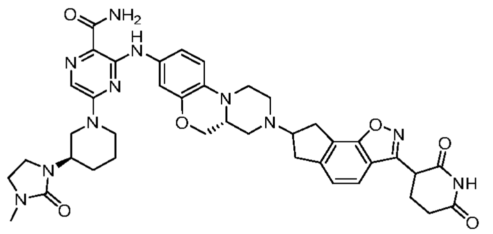

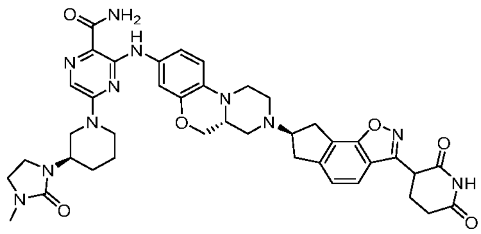

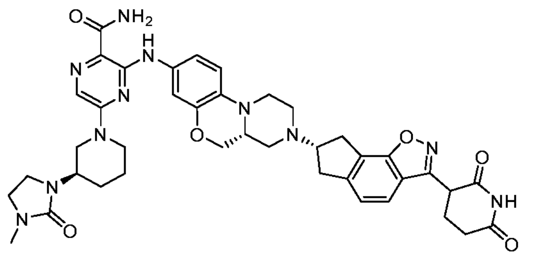

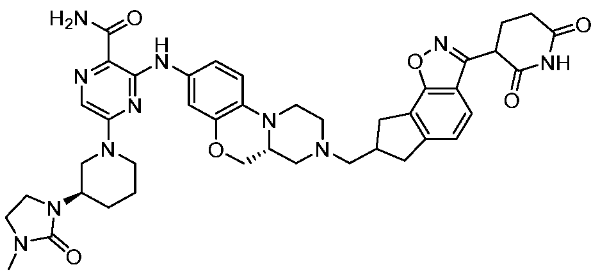

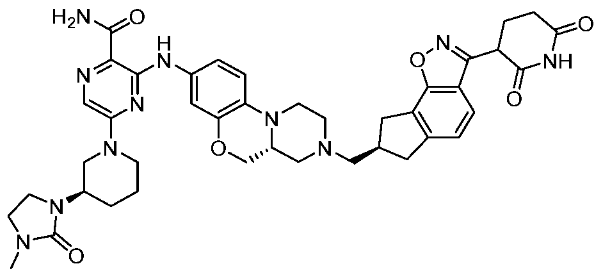

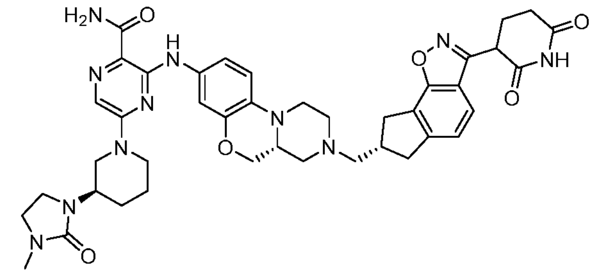

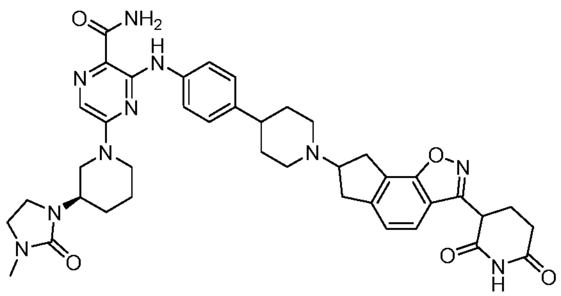

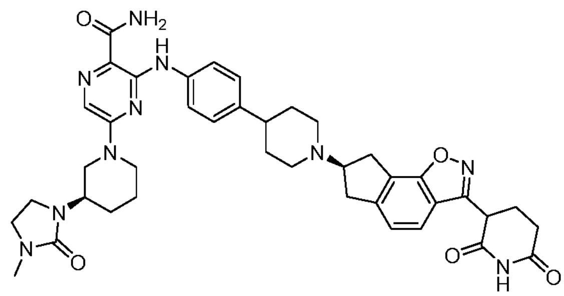

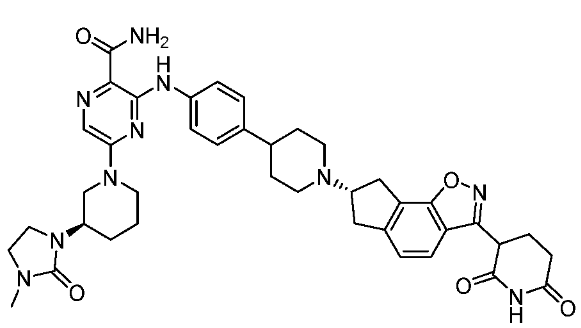

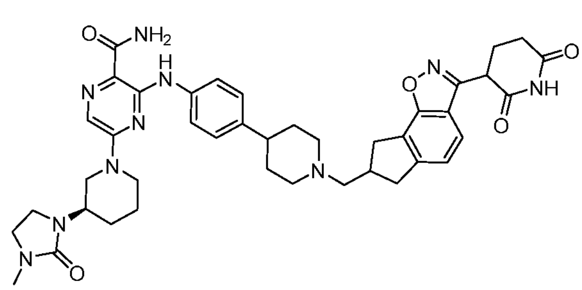

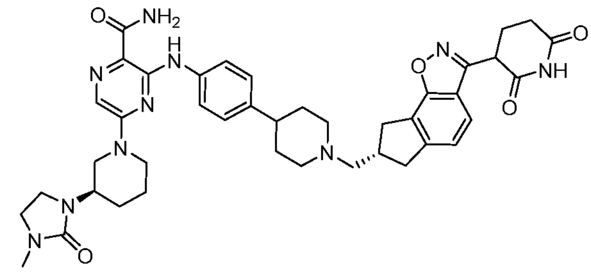

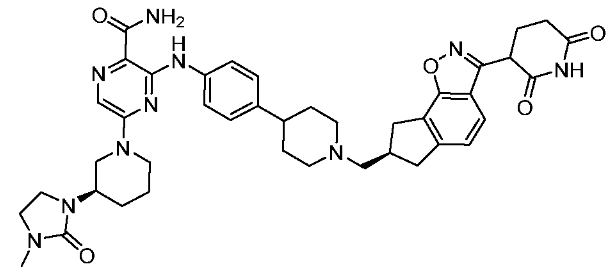

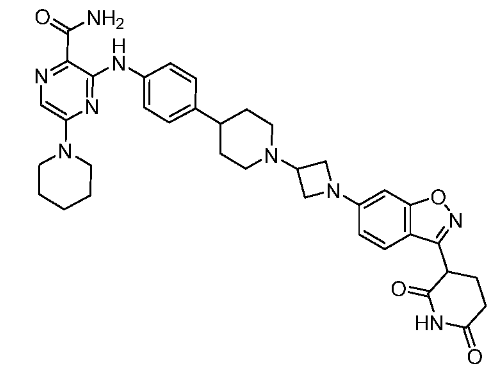

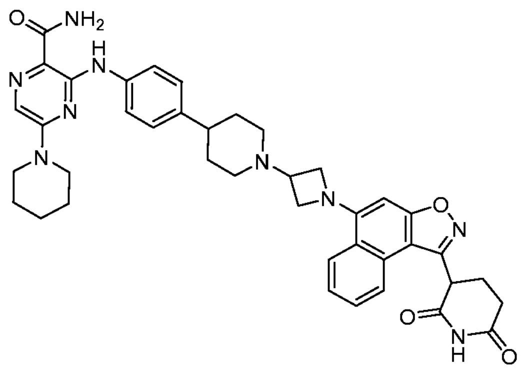

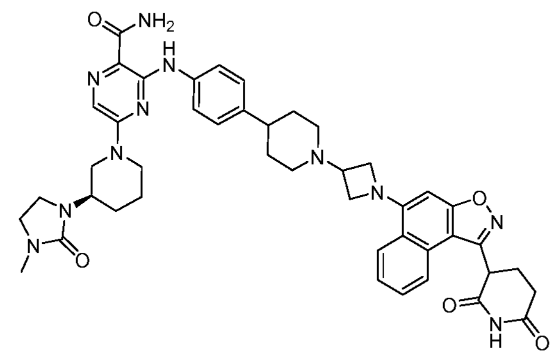

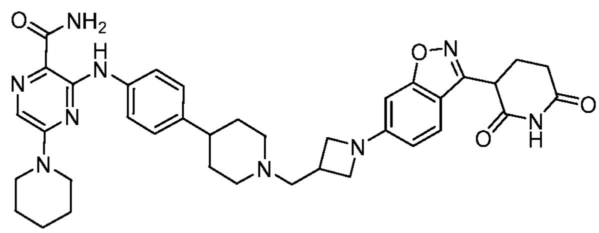

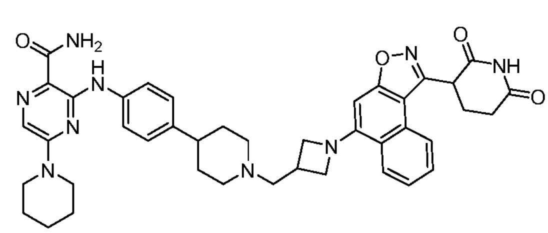

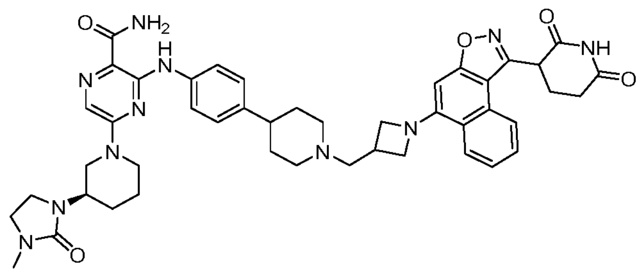

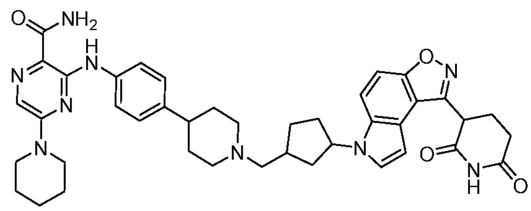

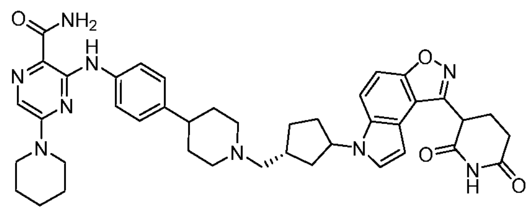

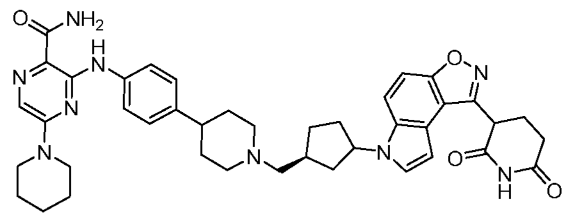

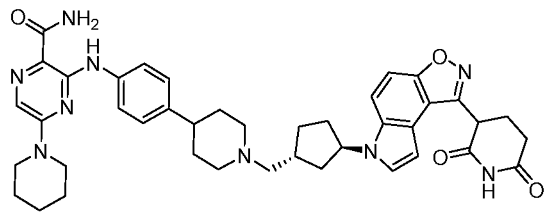

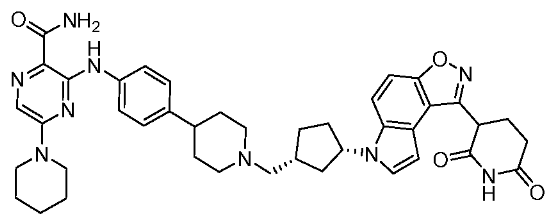

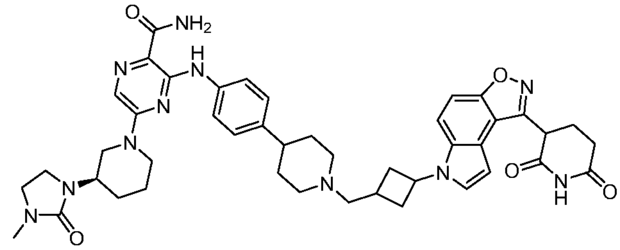

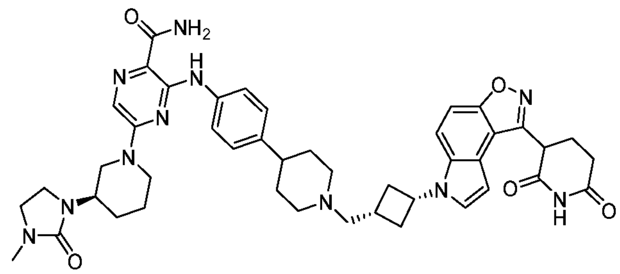

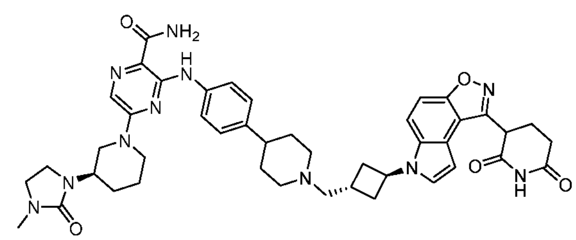

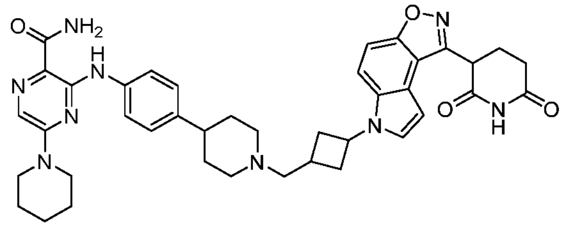

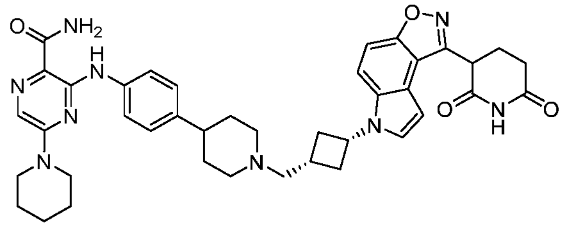

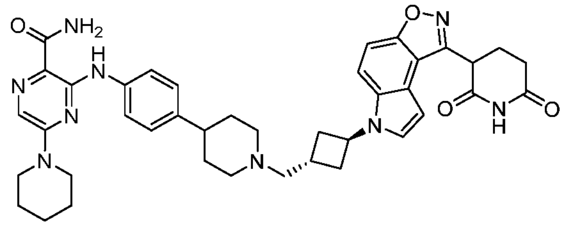

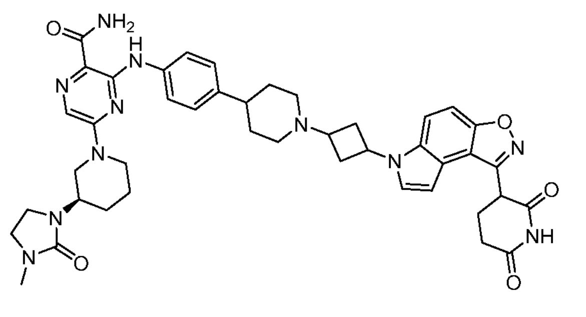

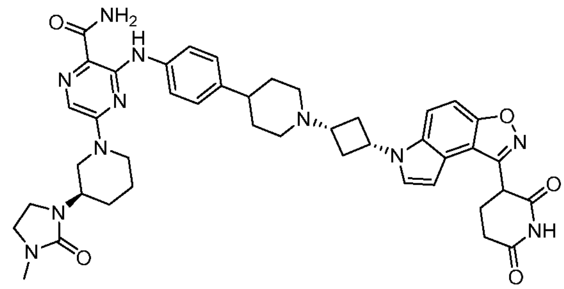

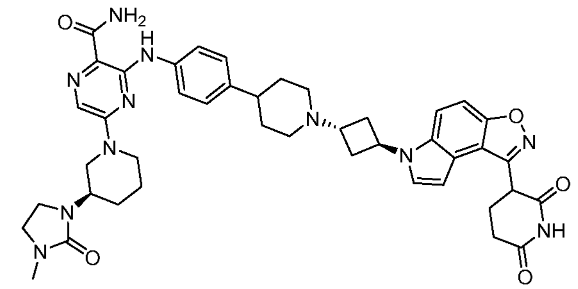

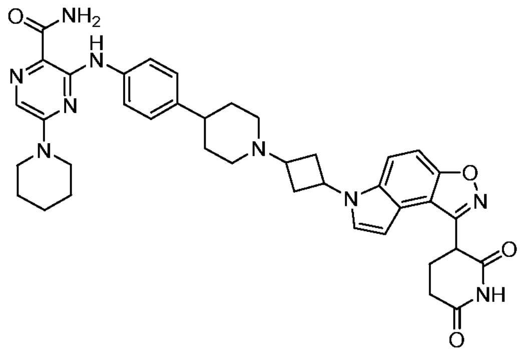

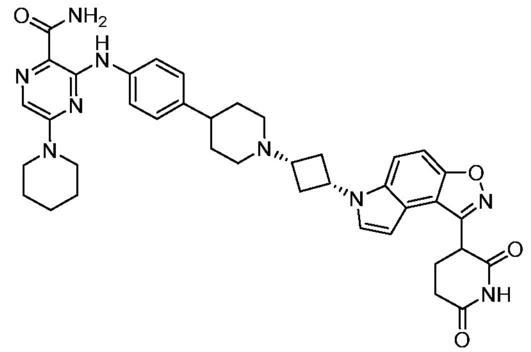

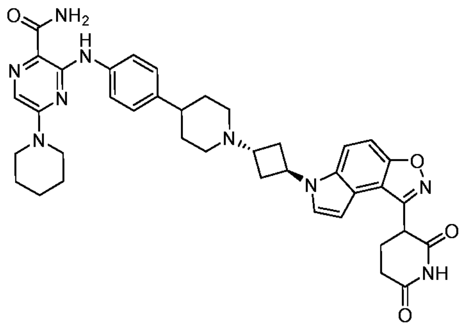

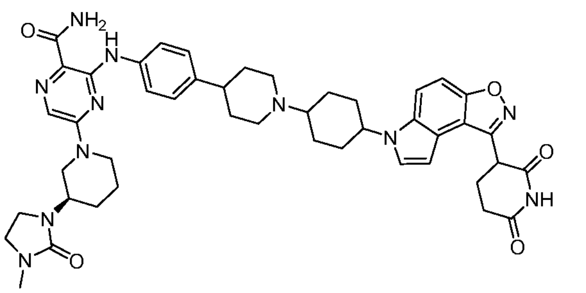

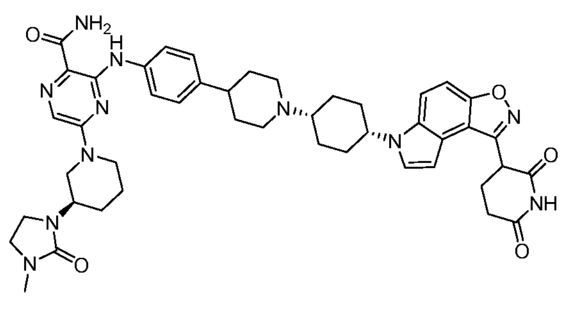

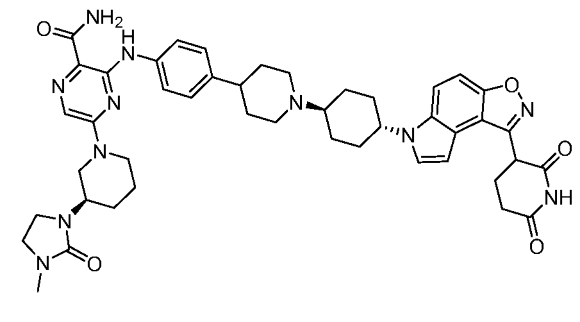

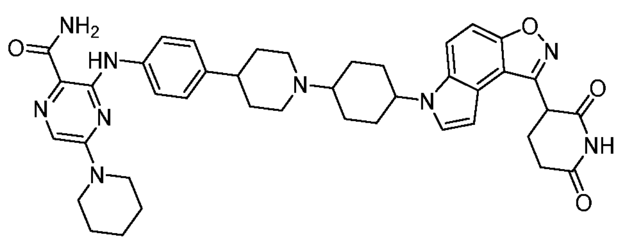

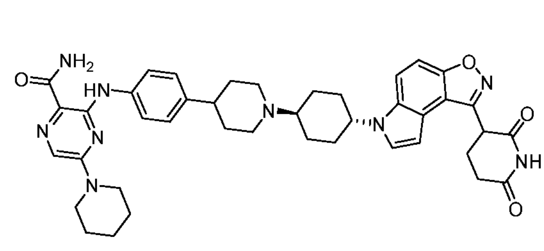

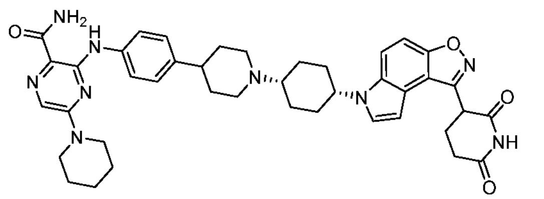

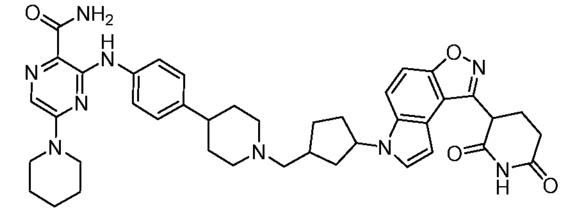

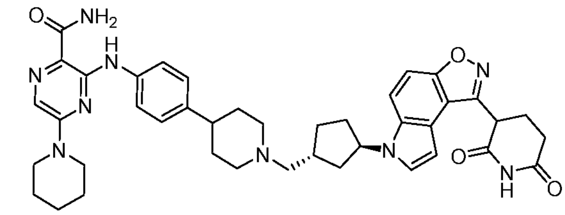

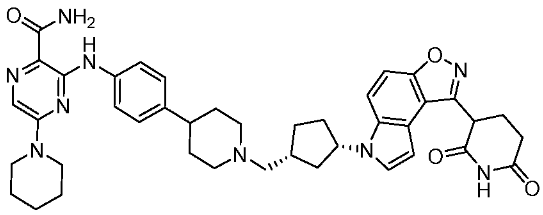

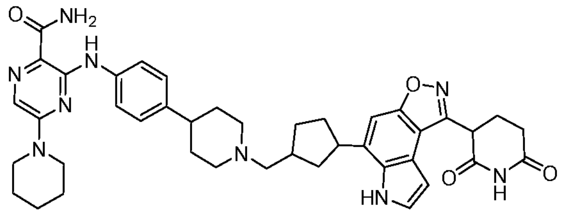

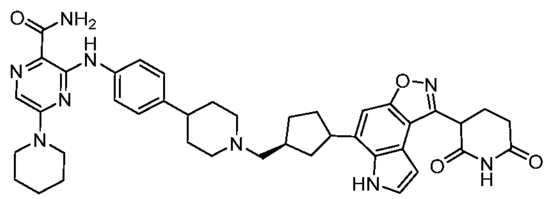

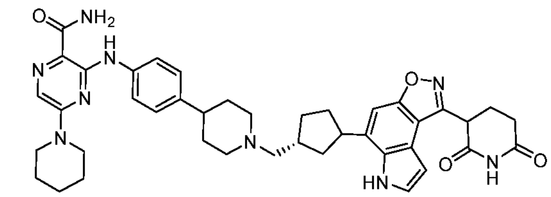

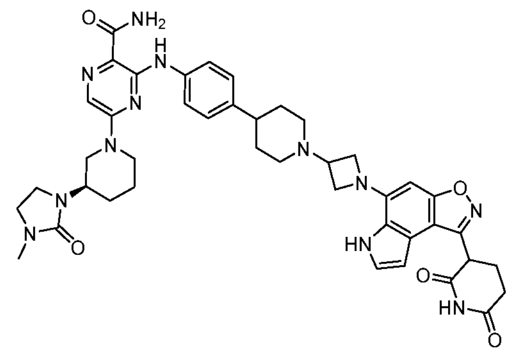

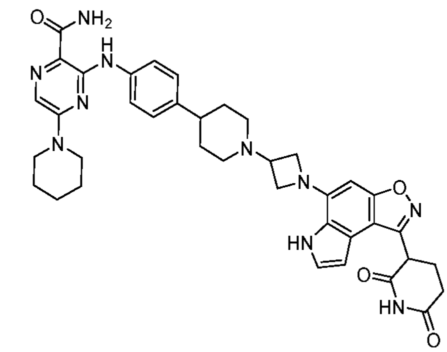

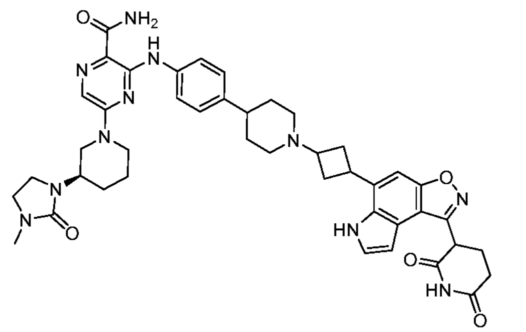

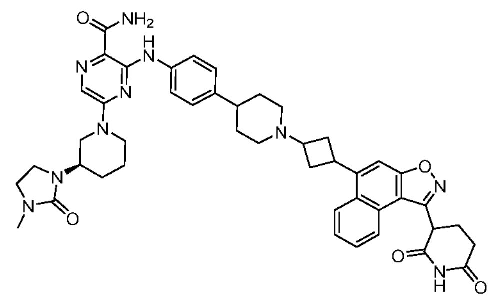

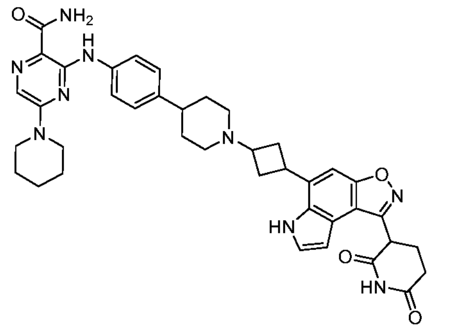

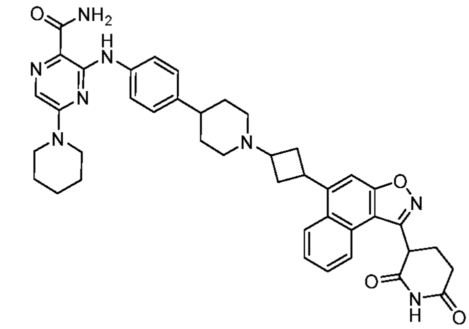

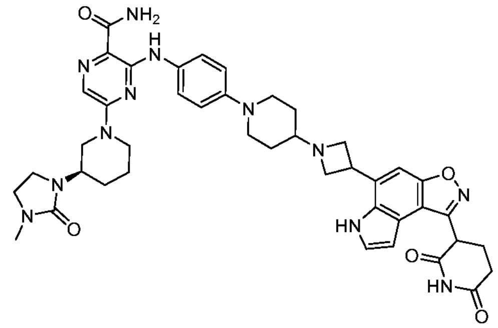

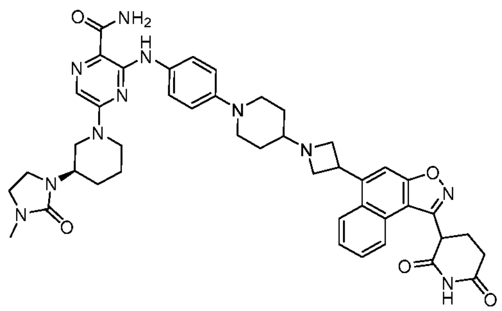

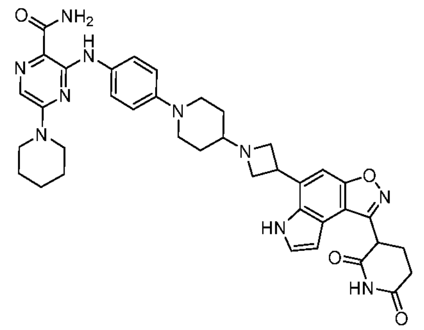

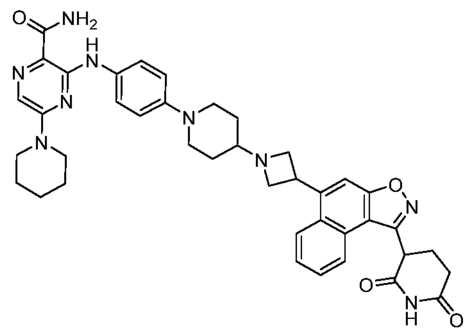

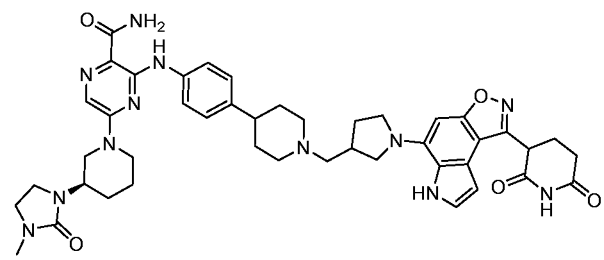

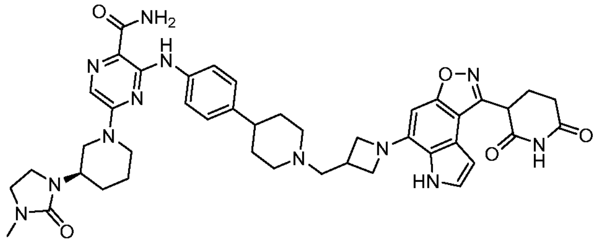

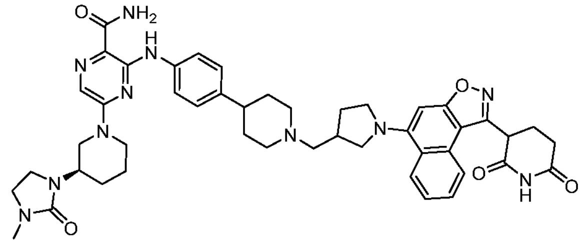

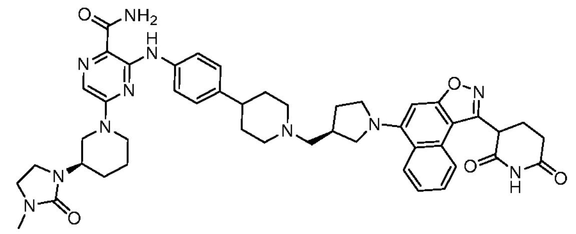
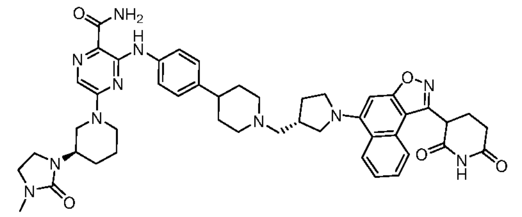

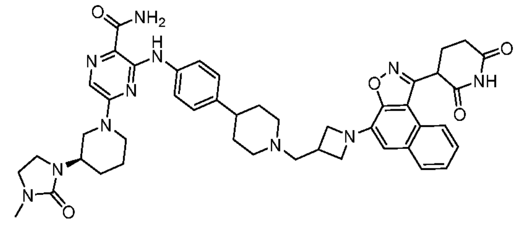
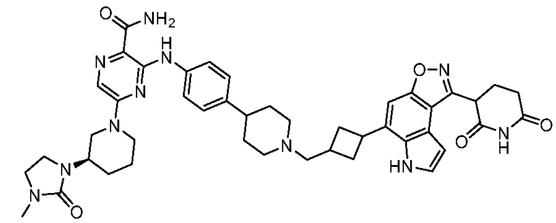

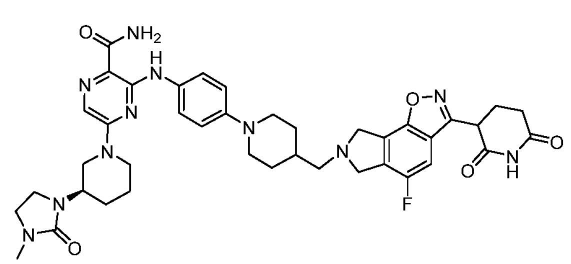
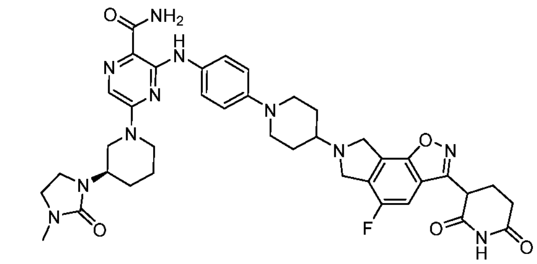

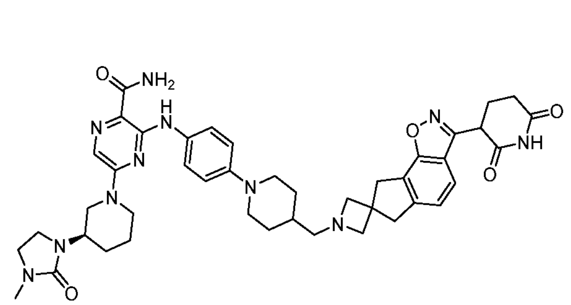
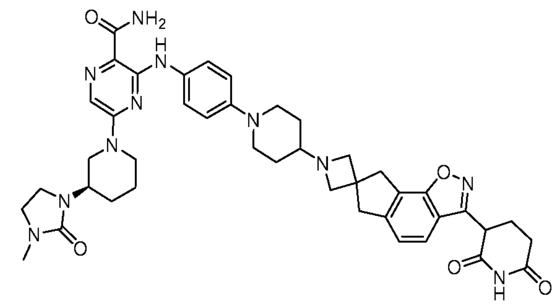

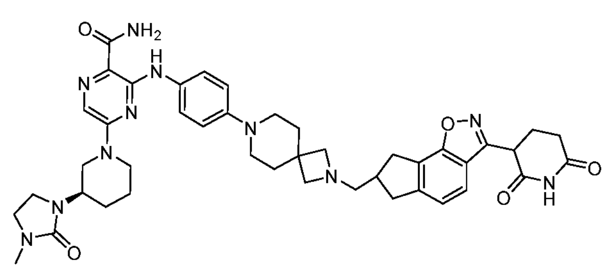
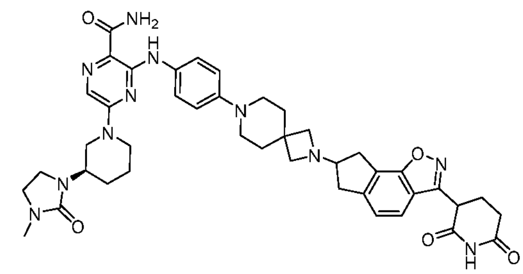

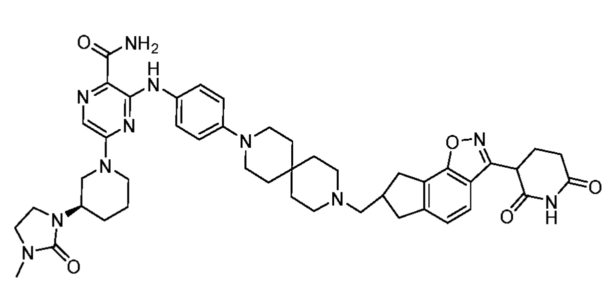
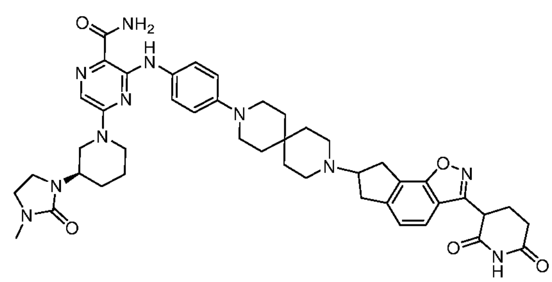

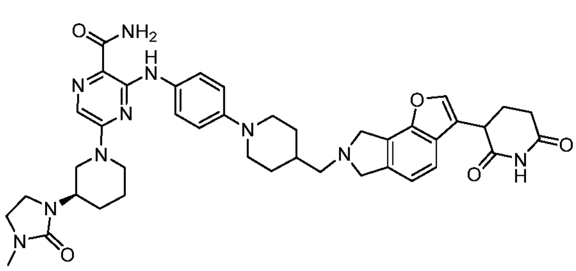

or

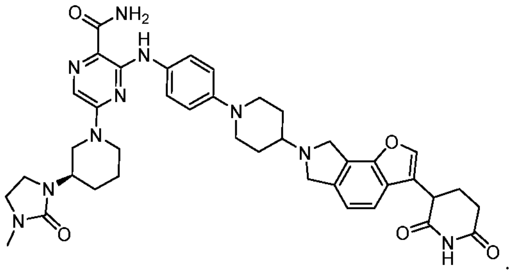.

In another aspect, the present application relates to a compound of formula I", a stereoisomer thereof, or a pharmaceutically acceptable salt thereof,

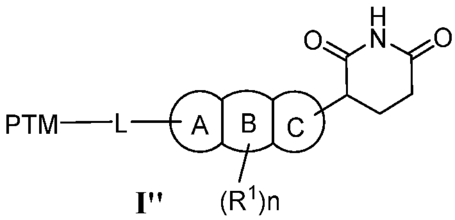

I"

wherein,

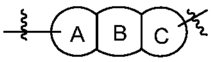

is selected from the group consisting of

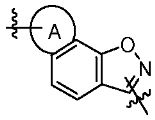, 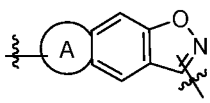, 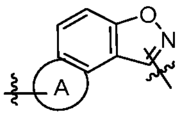,

and

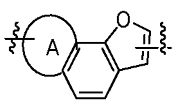;

ring A is selected from the group consisting of $C_{5-10}$ cycloalkenyl, 5- to 10-membered heterocycloalkenyl, phenyl, and 5- to 6-membered heteroaryl; or, ring A is selected from the group consisting of $C_{5-8}$ cycloalkenyl, 5- to 10-membered heterocycloalkenyl, phenyl, and 5- to 6-membered heteroaryl; or, ring A is selected from the group consisting of $C_{5-7}$ cycloalkenyl, 5- to 10-membered heterocycloalkenyl, phenyl, and 5- to 6-membered heteroaryl; or, ring A is selected from the group consisting of $C_{5-6}$ cycloalkenyl, 5- to 10-membered heterocycloalkenyl, phenyl, and 5- to 6-membered heteroaryl; or, ring A is selected from the group consisting of $C_{5-10}$ cycloalkenyl and 5- to 10-membered heterocycloalkenyl; or, ring A is selected from the group consisting of $C_{5-8}$ cycloalkenyl and 5-to 10-membered heterocycloalkenyl; or, ring A is selected from the group consisting of $C_{5-6}$ cycloalkenyl and 5- to 9-membered heterocycloalkenyl;

PTM is selected from the group consisting of drugs and derivatives thereof that bind to the target protein;

L is selected from a connecting group;

R’ and n are as defined herein.

In another aspect, the present application relates to a compound of formula I"-1, a moiety, a stereoisomer thereof, a derivative (such as a Protac molecule), or a pharmaceutically acceptable salt thereof,

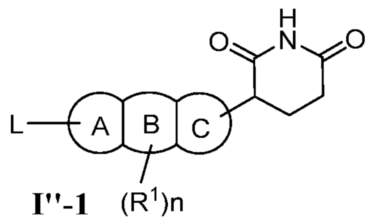

I''-1

wherein,

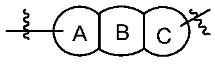

is selected from the group consisting of

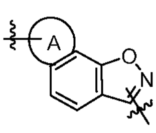, 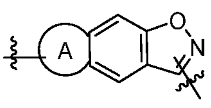, 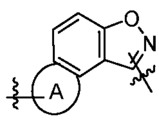,

and

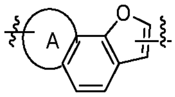;

ring A is selected from the group consisting of $C_{5-10}$ cycloalkenyl, 5- to 10-membered heterocycloalkenyl, phenyl, and 5- to 6-membered heteroaryl; or, ring A is selected from the group consisting of $C_{5-8}$ cycloalkenyl, 5- to 10-membered heterocycloalkenyl, phenyl, and 5- to 6-membered heteroaryl; or, ring A is selected from the group consisting of $C_{5-7}$ cycloalkenyl, 5- to 10-membered heterocycloalkenyl, phenyl, and 5- to 6-membered heteroaryl; or, ring A is selected from the group consisting of $C_{5-6}$ cycloalkenyl, 5- to 10-membered heterocycloalkenyl, phenyl, and 5- to 6-membered heteroaryl; or, ring A is selected from the group consisting of $C_{5-10}$ cycloalkenyl and 5- to 10-membered heterocycloalkenyl; or, ring A is selected from the group consisting of $C_{5-8}$ cycloalkenyl and 5-to 10-membered heterocycloalkenyl; or, ring A is selected from the group consisting of $C_{5-6}$ cycloalkenyl and 5- to 9-membered heterocycloalkenyl;

L is selected from a connecting group;

R’ and n are as defined herein.

In some embodiments of the present application, the compound of formula I"-1, the moiety, the stereoisomer thereof, the derivative (such as a Protac molecule), or the pharmaceutically acceptable salt thereof is selected from the group consisting of a compound of formula I", a stereoisomer thereof, and a pharmaceutically acceptable salt thereof.

In some embodiments, ring A is selected from the group consisting of $C_{5-8}$ cycloalkenyl (or $C_{5-7}$ cycloalkenyl), 5-to 9-membered heterocycloalkenyl, phenyl, pyrrolyl, pyrazolyl, furanyl, and oxazolyl.

In some embodiments, ring A is selected from the group consisting of $C_{5-6}$ cycloalkenyl, 5- to 9-membered heterocycloalkenyl, phenyl, pyrrolyl, pyrazolyl, furanyl, and oxazolyl.

In some embodiments, ring A is selected from the group consisting of $C_5$ cycloalkenyl, $C_6$ cycloalkenyl, 5-, 6-, 7-, 8-, or 9-membered heterocycloalkenyl, phenyl, pyrrolyl, pyrazolyl, furanyl, and oxazolyl.

In some embodiments, ring A is selected from the group consisting of cyclopentenyl, bicyclohexenyl, dihydropyrrolyl, tetrahydropyridinyl, tetrahydroazepinyl, azaspirooctenyl, dihydrooxazinyl, azaspirononenyl, phenyl, pyrrolyl, pyrazolyl, furanyl, and oxazolyl.

In some specific embodiments, ring A is selected from the group consisting of $C_{5-8}$ cycloalkenyl and 5- to 9-membered heterocycloalkenyl. In some specific embodiments, ring A is selected from the group consisting of $C_{5-7}$ cycloalkenyl and 5- to 9-membered heterocycloalkenyl. In some specific embodiments, ring A is selected from the group consisting of $C_{5-6}$ cycloalkenyl and 5- to 9-membered heterocycloalkenyl. In some specific embodiments, ring A is selected from 5- to 8-membered heterocycloalkenyl. In some specific embodiments, ring A is selected from the group

consisting of 6- to 8-membered heterocycloalkenyl. In some specific embodiments, ring A is selected from 5-membered heterocycloalkenyl. In some specific embodiments, ring A is selected from 6-membered heterocycloalkenyl. In some specific embodiments, ring A is selected from 7-membered heterocycloalkenyl. In some specific embodiments, ring A is selected from 8-membered heterocycloalkenyl. In some specific embodiments, ring A is selected from 9-membered heterocycloalkenyl.

In some specific embodiments, ring A is selected from the group consisting of cyclopentenyl, bicyclohexenyl, dihydropyrrolyl, tetrahydropyridinyl, dihydrooxazinyl, tetrahydroazepinyl, azaspirooctenyl, and azaspirononenyl. In some specific embodiments, ring A is selected from the group consisting of cyclopentenyl and bicyclohexenyl. In some embodiments, ring A is selected from the group consisting of dihydropyrrolyl, dihydrooxazinyl, tetrahydropyridinyl, tetrahydroazepinyl, azaspirooctenyl, and azaspirononenyl.

In some embodiments, ring A is selected from the group consisting of phenyl, pyrrolyl, pyrazolyl, furanyl, and oxazolyl.

In some other embodiments, ring A is selected from the group consisting of $C_{5-7}$ cycloalkenyl, 5- to 8-membered heterocycloalkenyl, phenyl, and pyrrolyl.

In some other embodiments, ring A is selected from the group consisting of $C_{5-6}$ cycloalkenyl, 5- to 8-membered heterocycloalkenyl, phenyl, and pyrrolyl.

In some other embodiments, ring A is selected from the group consisting of $C_5$ cycloalkenyl, 5-, 6-, 7-, or 8-membered heterocycloalkenyl, phenyl, and pyrrolyl.

In some other embodiments, ring A is selected from the group consisting of cyclopentenyl, dihydropyrrolyl, tetrahydropyridinyl, tetrahydroazepinyl, azaspirooctene, phenyl, and pyrrolyl.

In some other embodiments, ring A is selected from the group consisting of $C_{5-6}$ cycloalkenyl and 5- to 8-membered heterocycloalkenyl.

In some other embodiments, ring A is selected from the group consisting of cyclopentenyl, dihydropyrrolyl, tetrahydropyridinyl, tetrahydroazepinyl, and azaspirooctene.

In some other embodiments, ring A is selected from cyclopentenyl. In some embodiments, ring A is selected from the group consisting of dihydropyrrolyl, tetrahydropyridinyl, tetrahydroazepinyl, and azaspirooctene.

In some other embodiments, ring A is selected from the group consisting of phenyl and pyrrolyl.

In some embodiments, the structural moiety

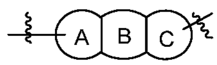

is selected from the group consisting of

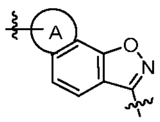 , 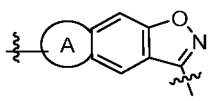 , 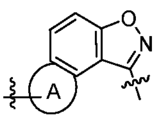 , and 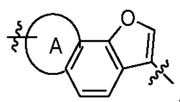 .

In some embodiments, the structural moiety

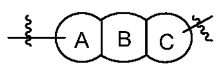

is selected from the group consisting of

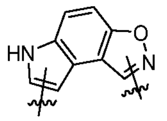 and 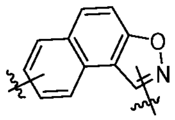 ;

in some embodiments, the structural moiety

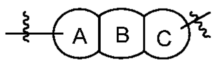

is selected from the group consisting of

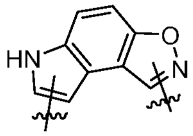, 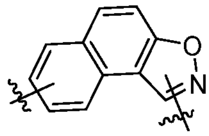, 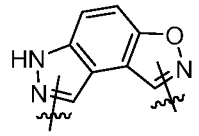, 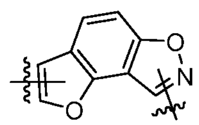,

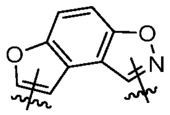, 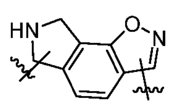, 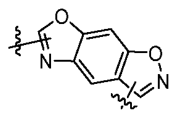, 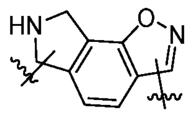, 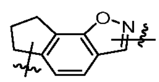, 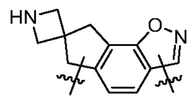,

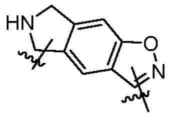, 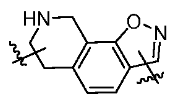, 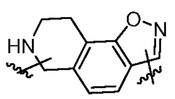, 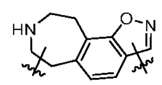, 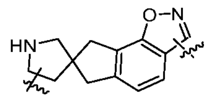, 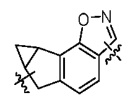,

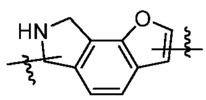, 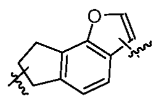, 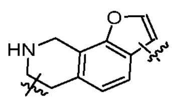, 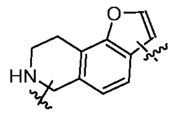, 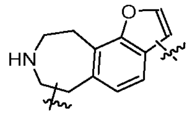, 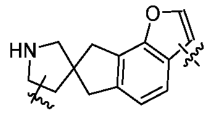,

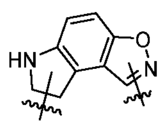, 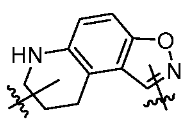, and 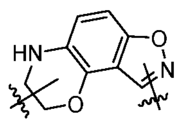.

In some other embodiments, the structural moiety

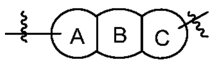

is selected from the group consisting of

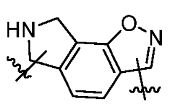, 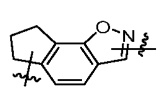, 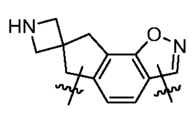, 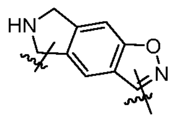, 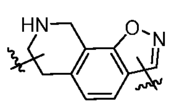, 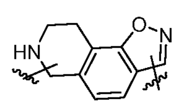,

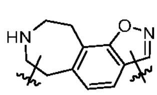, and 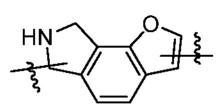.

In some embodiments, the structural moiety

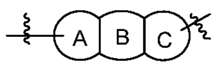

is selected from the group consisting of

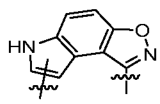, 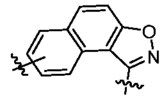, 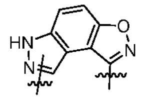, 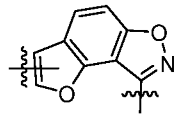, 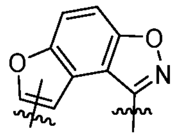, and 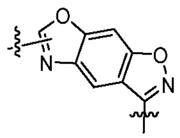.

In some embodiments, the structural moiety

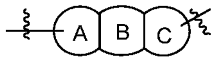

is selected from the group consisting of

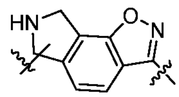,

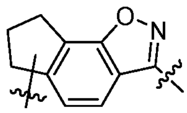, 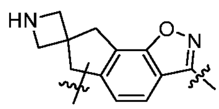, 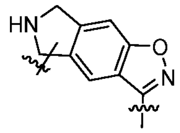, 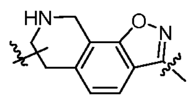, 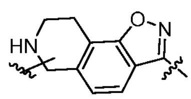, 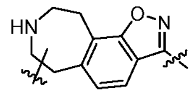,

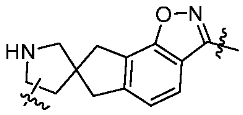, 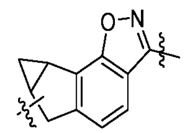, 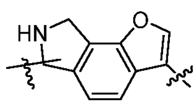, 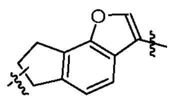, 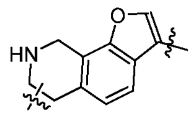,

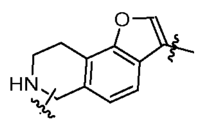, 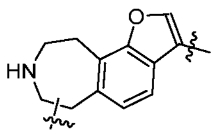, 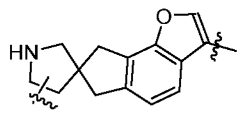, 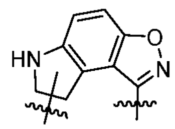, 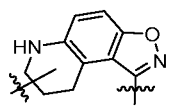,

and

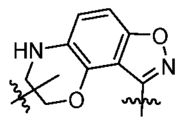.

In some embodiments, the structural moiety

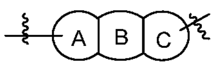

is selected from the group consisting of

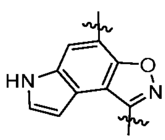, 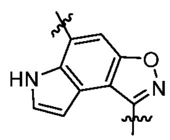, 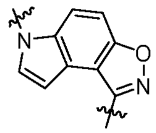, 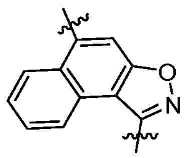, 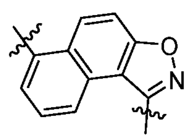, 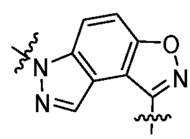,

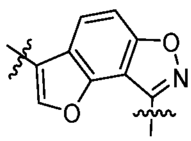, 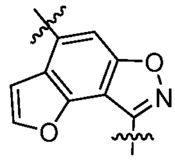, 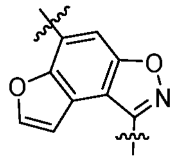, and 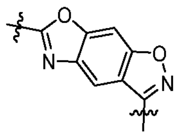.

In some embodiments, the structural moiety

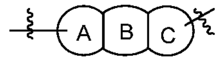

is selected from the group consisting of

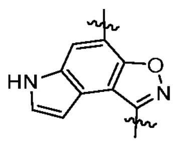, 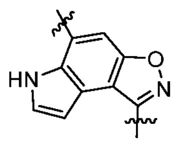,

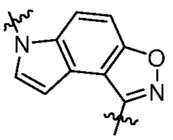, 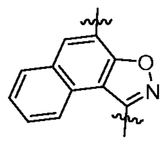, and 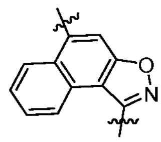.

In some embodiments, the structural moiety

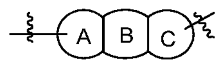

is selected from the group consisting of

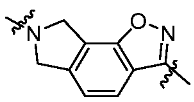, 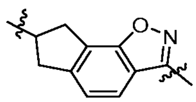,

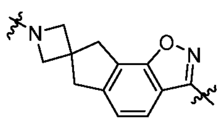, 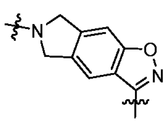, 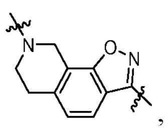, 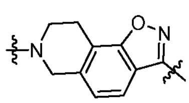, 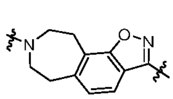, 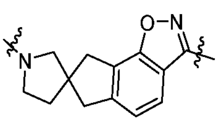,

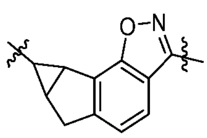, 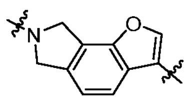, 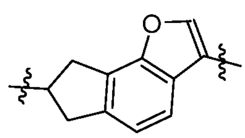, 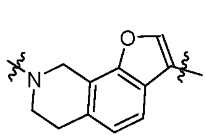, 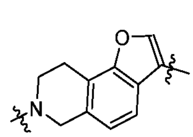,

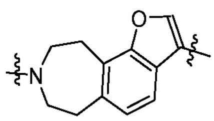, 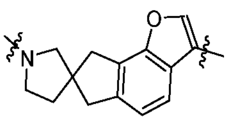, 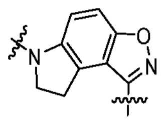, 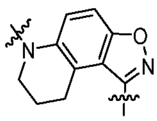, and 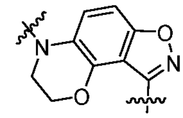.

In some other embodiments, the structural moiety

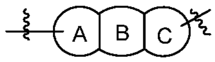

is selected from the group consisting of

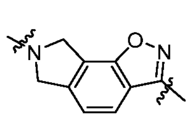, 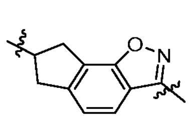, 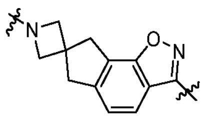, 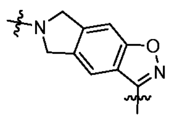, 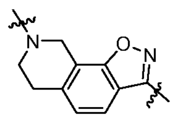, 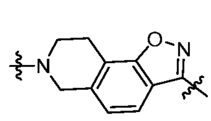,

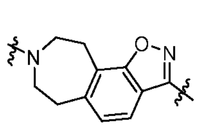, and 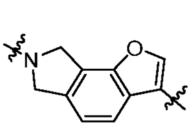.

In some embodiments, the structural moiety

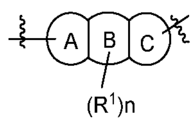

is selected from the group consisting of

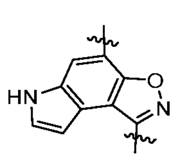, 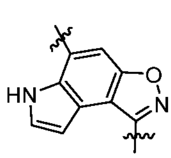, 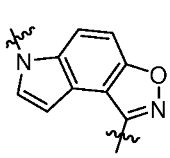, 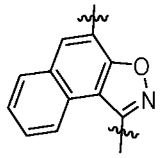, and 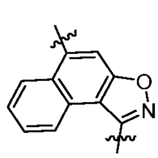;

in some embodiments, the structural moiety

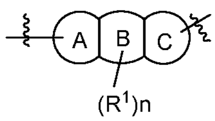

is selected from the group consisting of

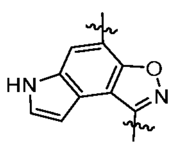, 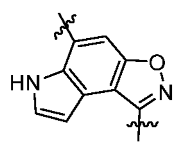,

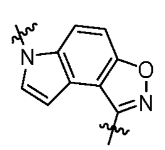, 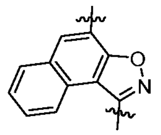, 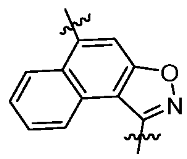, 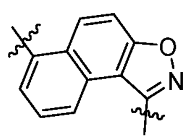, 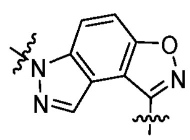, 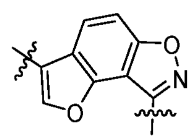,

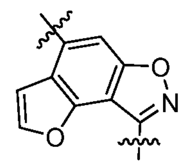, 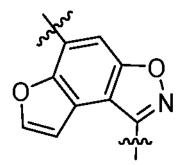, and 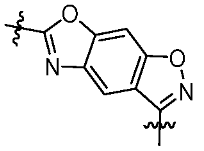.

In some embodiments, the structural moiety

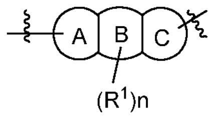

is selected from the group consisting of

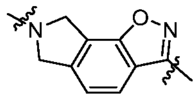, 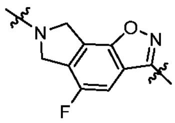, 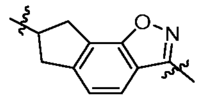,

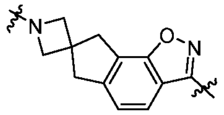, 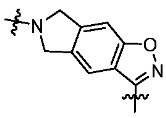, 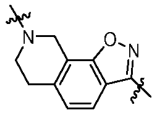, 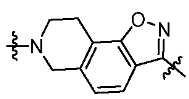, 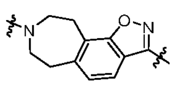, 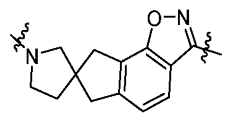,

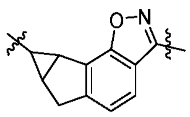, 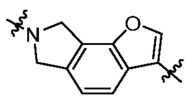, 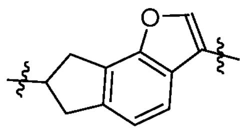, 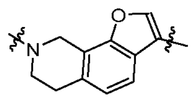, 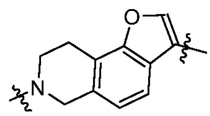,

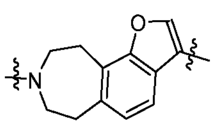, 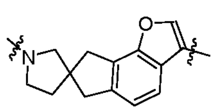, 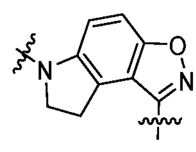, 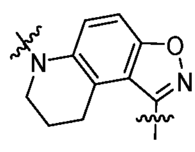, and 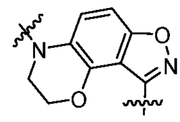.

In some embodiments, the structural moiety

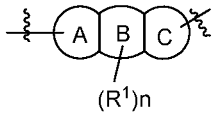

is selected from the group consisting of

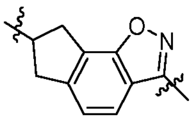, 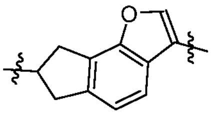, and 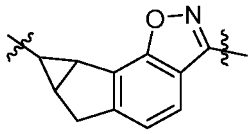.

In some embodiments, the structural moiety

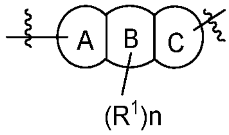

is selected from the group consisting of

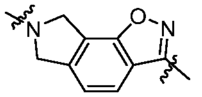, 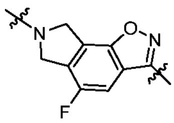,

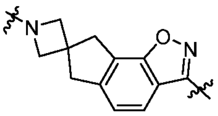, 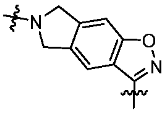, 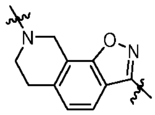, 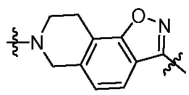, 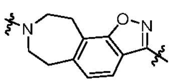, 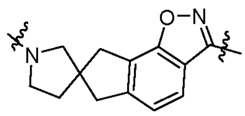,

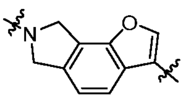, 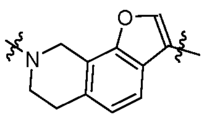, 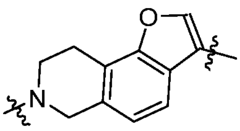, 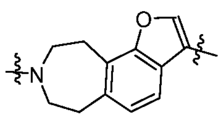, 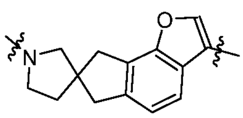,

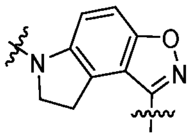, 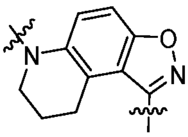, and 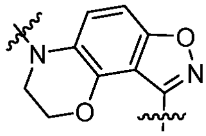.

In some other embodiments, the structural moiety

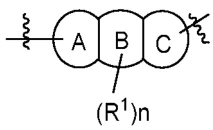

is selected from the group consisting of

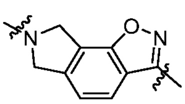, 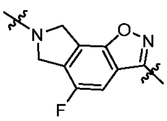, 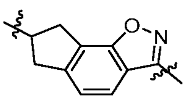, 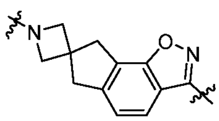, 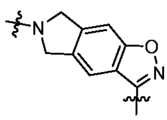, 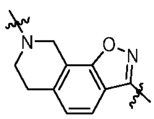,

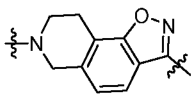, 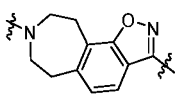, and 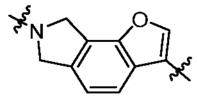.

In some embodiments, L is selected from the group consisting of -Cy$^1$-LNK-Cy$^2$-LNK-, -Cy$^1$-LNK-Cy$^2$-, and -Cy$^1$-Cy$^2$-LNK-; in some embodiments, L is selected from -Cy$^1$-LNK-Cy$^2$-; in some embodiments, Cy$^1$, LNK, Cy$^2$, -Cy'-LNK-, -LNK-Cy$^2$-, -Cy$^1$-Cy$^2$-, or -Cy$^1$-LNK-Cy$^2$- is as described herein; further, in some embodiments, the moiety

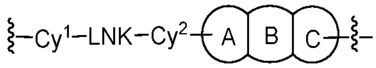

is selected from the group consisting of

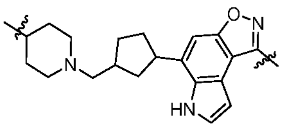,

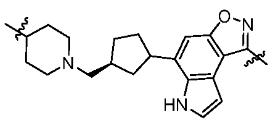, 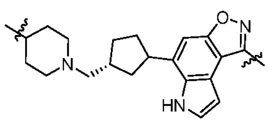, 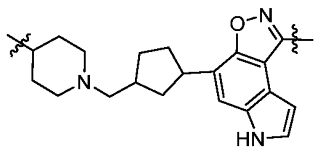, 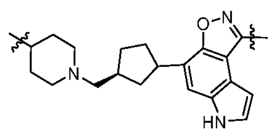,

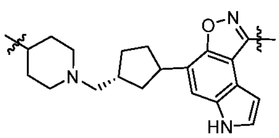, 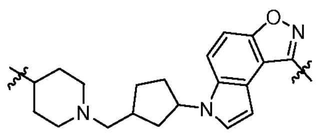, 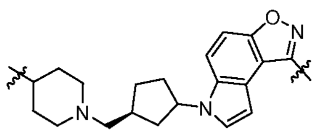,

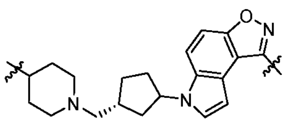, 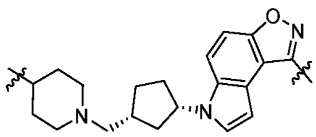, 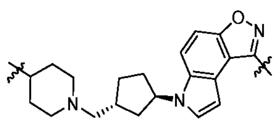, 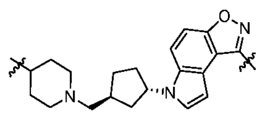,

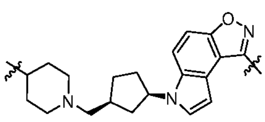, 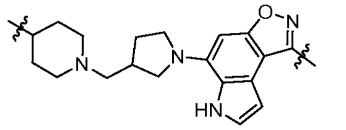, 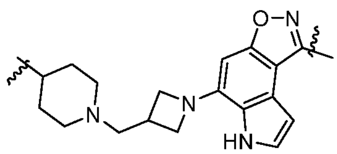,

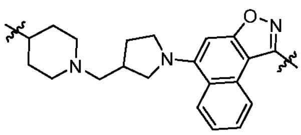, 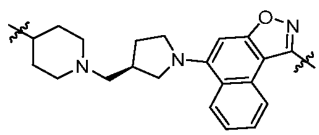, 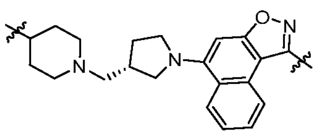,

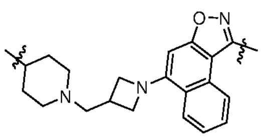, 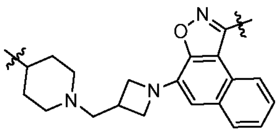, 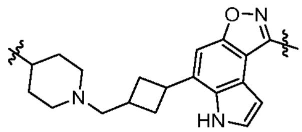, 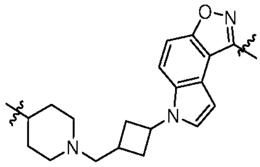,

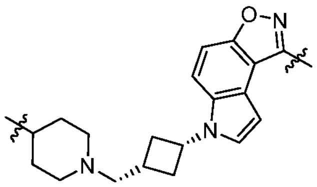, 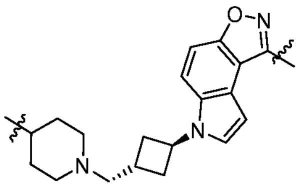, 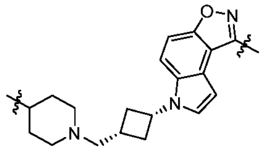, 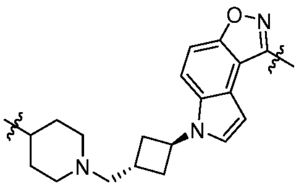,

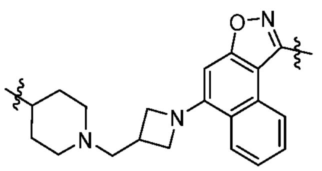, 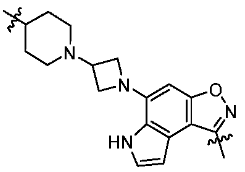, 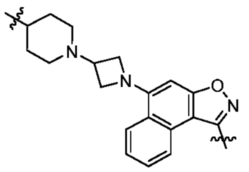, 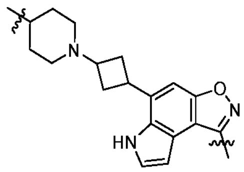,

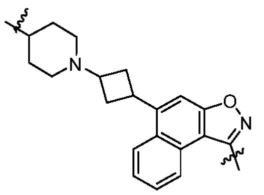, 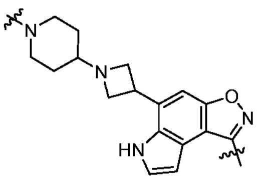, 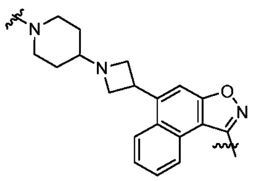, 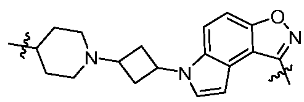,

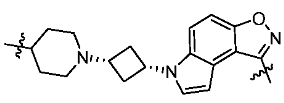, 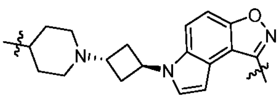, 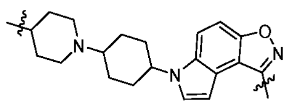, 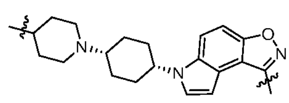,

and

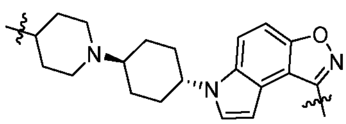.

Alternatively, the moiety

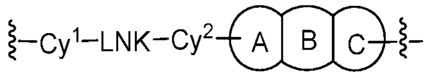

is selected from the group consisting of

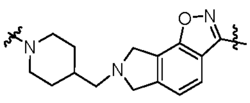, 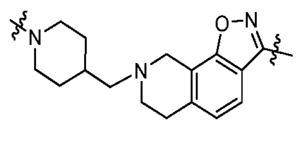, 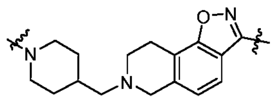,

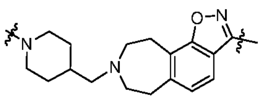, 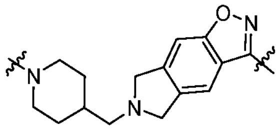, 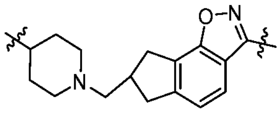, 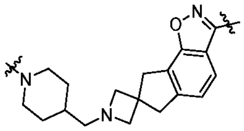,

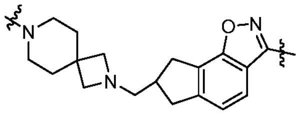, 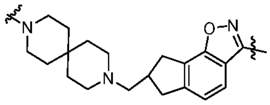, 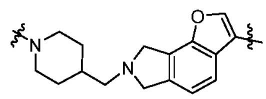, 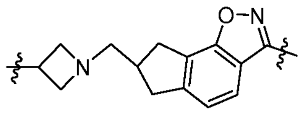,

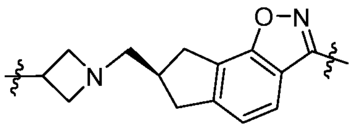, 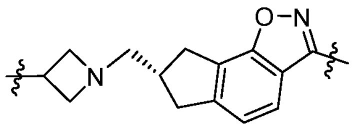, 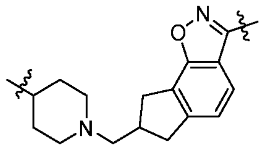,

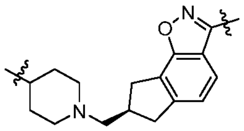, 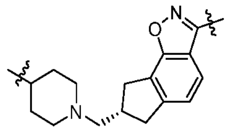, 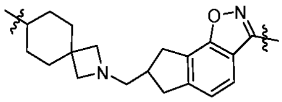, 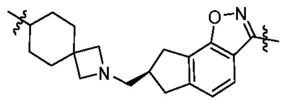,

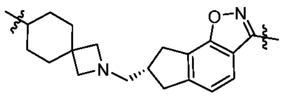, 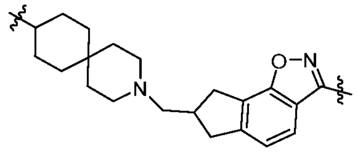, 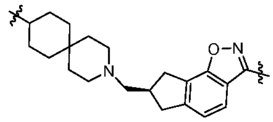,

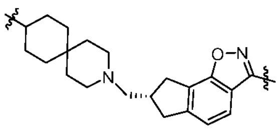, 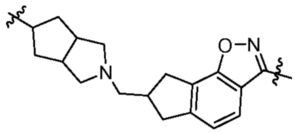, 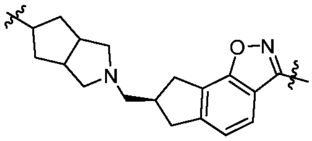, 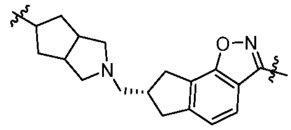,

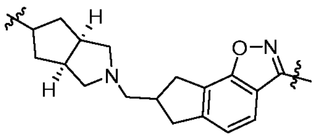, 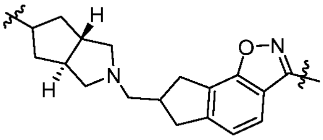, 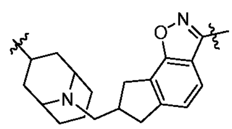, 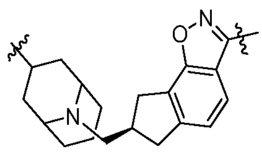,

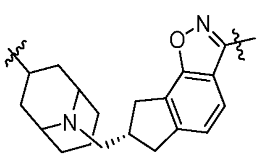, 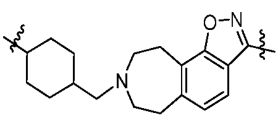 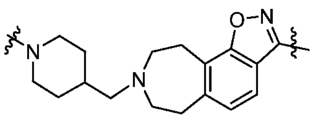, 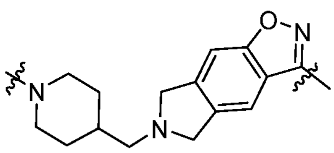,

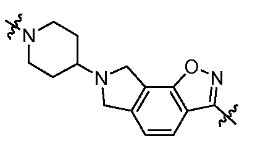, 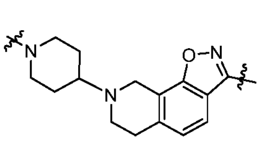, 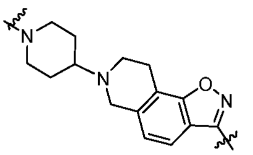, 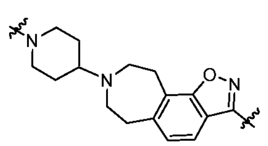,

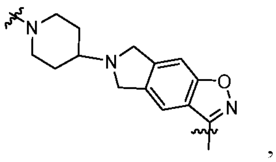, 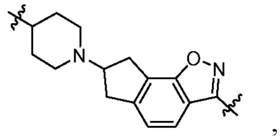, 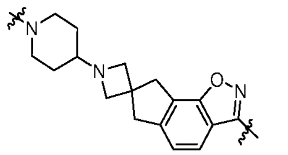, 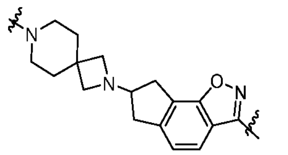,

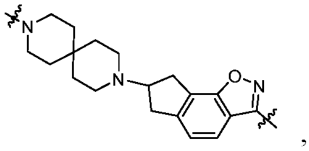, 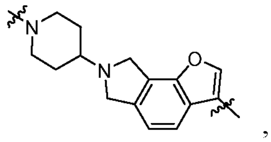, 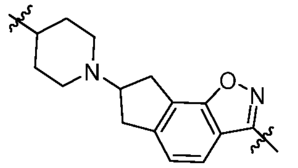, 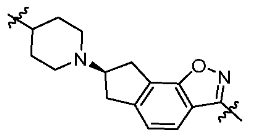,

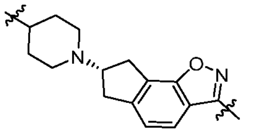, 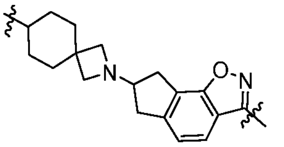, 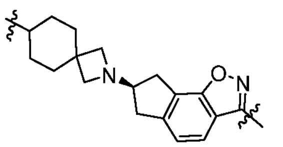, 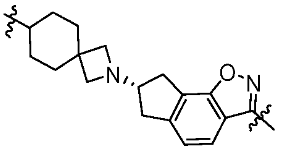,

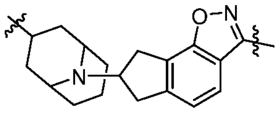, 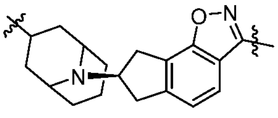, 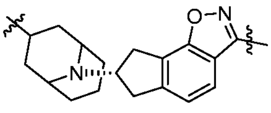, 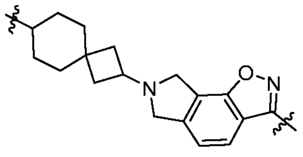,

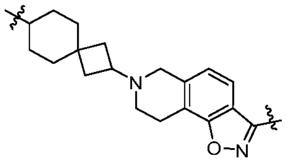, 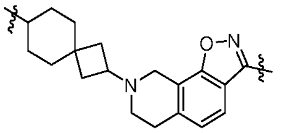, 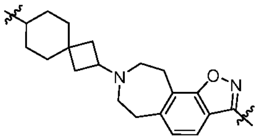, 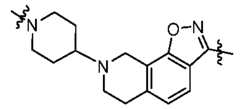,

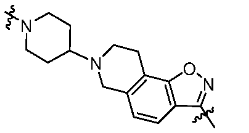, 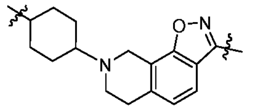, 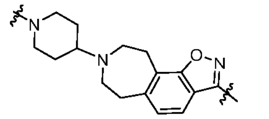, 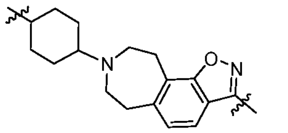,

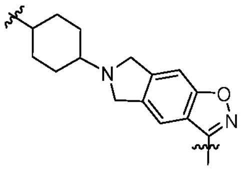, 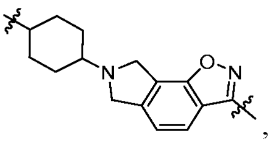, 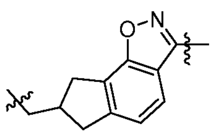, 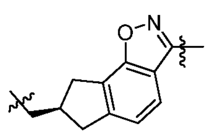, 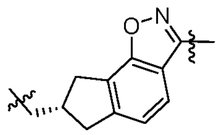,

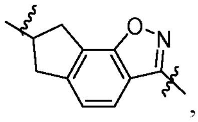, 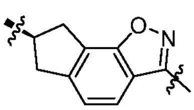, and 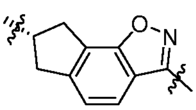.

Further, in some other embodiments, the moiety

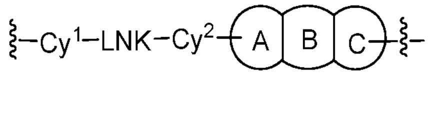

is selected from the group consisting of

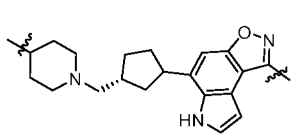, 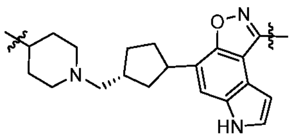, 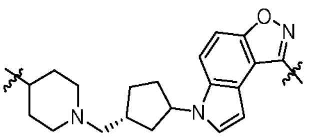,

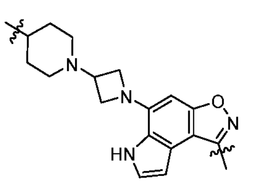, 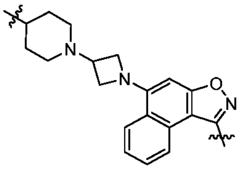, 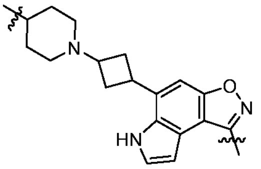, 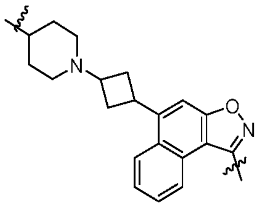,

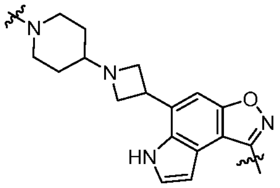, 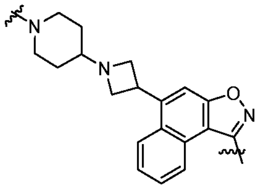, 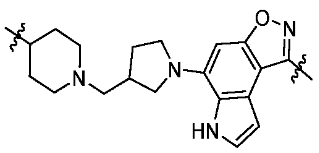, 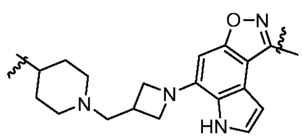,

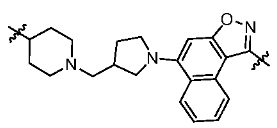, 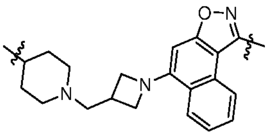, 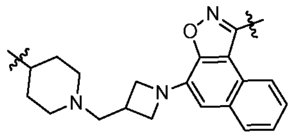, 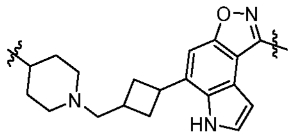,

and

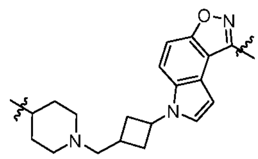;

or, the moiety

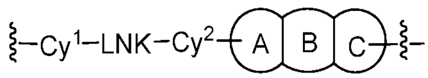

is selected from the group consisting

of 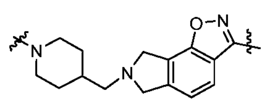, 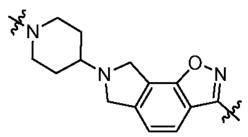, 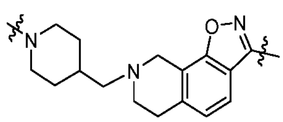, 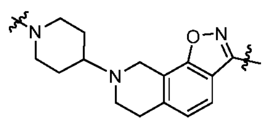,

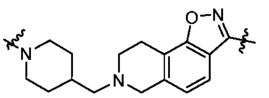, 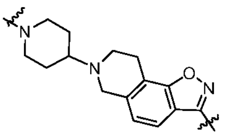, 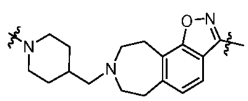, 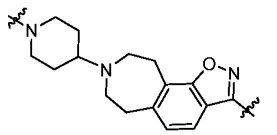,

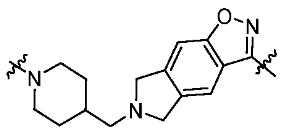, 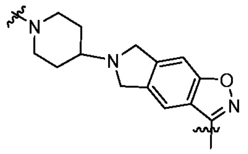, 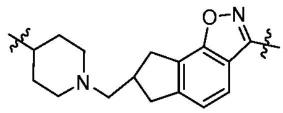, 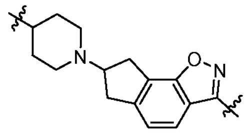,

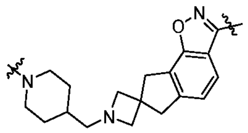, 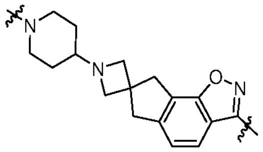, 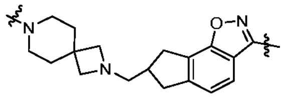, 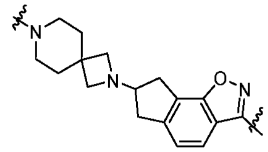,

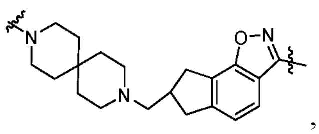, 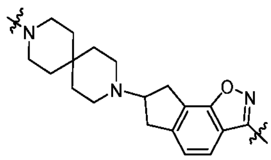, 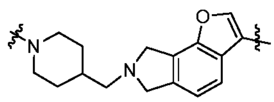, and 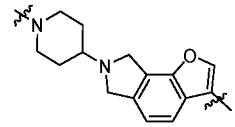.

In some embodiments, the PTM is as described herein.

In some embodiments, PTM is selected from

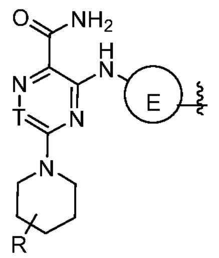

, wherein R, T, or ring E is as described herein.

In some embodiments, PTM is selected from

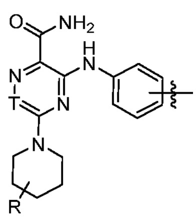

, wherein R or T is as described herein.

In another aspect, the present application relates to a compound of formula III below, a moiety, an isomer (e.g., a stereoisomer) thereof, a derivative (such as a Protac molecule) thereof, or a pharmaceutically acceptable salt thereof:

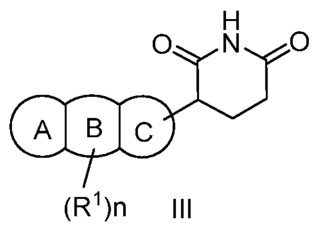

III ,

wherein ring A is selected from the group consisting of $C_{5-10}$ cycloalkenyl and 5- to 10-membered heterocycloalkenyl;
ring B is selected from phenyl;
ring C is selected from the group consisting of isoxazolyl and furanyl;
n is as defined herein;
each R' is independently selected from the group consisting of halogen, -OH, $-NH_2$, -CN, =O, $C_{1-4}$ alkoxy, -CHO, $C_{3-6}$ cycloalkyl, 3- to 10-membered heterocycloalkyl, and $C_{1-4}$ alkyl, wherein the $C_{3-6}$ cycloalkyl, 3- to 10-membered heterocycloalkyl, or $C_{1-4}$ alkyl is optionally substituted with halogen, -OH, $-NH_2$, or $C_{1-4}$ alkyl-OH. In some embodiments, the compound of formula III is not selected from the following compound:

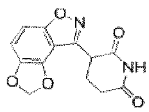 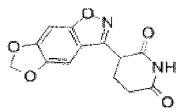.

In some embodiments, the heterocycloalkenyl comprises at least one N atom.

In some embodiments, ring A is selected from the group consisting of $C_{5-8}$ cycloalkenyl and 5- to 10-membered heterocycloalkenyl; or

ring A is selected from the group consisting of $C_{5-7}$ cycloalkenyl and 5- to 10-membered heterocycloalkenyl; or
ring A is selected from the group consisting of $C_{5-6}$ cycloalkenyl and 5- to 10-membered heterocycloalkenyl; or,
ring A is selected from the group consisting of $C_{5-6}$ cycloalkenyl and 5- to 9-membered heterocycloalkenyl.

In some embodiments, the structural moiety

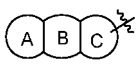

is selected from the group consisting of

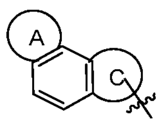

and

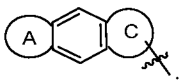.

In some embodiments, the structural moiety

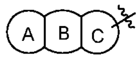

is selected from the group consisting of

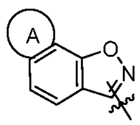,

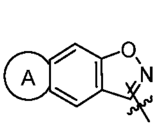, 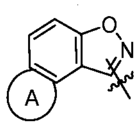, 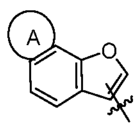, 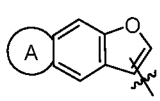, and 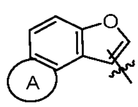.

In some embodiments, the structural moiety

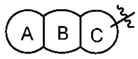

is selected from the group consisting of

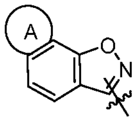

and

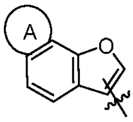

In some embodiments, the structural moiety

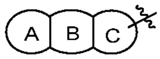

is selected from the group consisting of

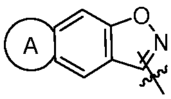

and

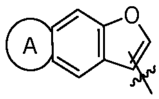

In some embodiments, the structural moiety

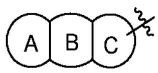

is selected from the group consisting of

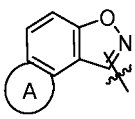

and

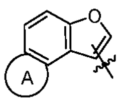

In some embodiments, the structural moiety

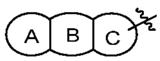

is selected from the group consisting of

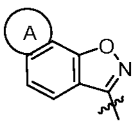

,

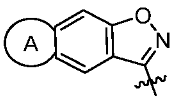, 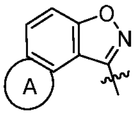, 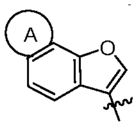, 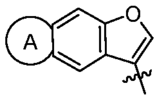, and 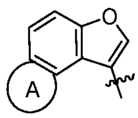.

In some specific embodiments, the structural moiety

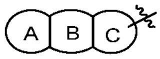

is selected from the group consisting of

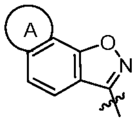 and 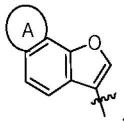

. In some specific embodiments, the structural moiety

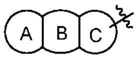

is selected from the group consisting of

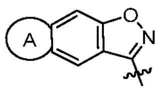 and 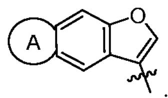.

In some specific embodiments, the structural moiety

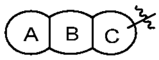

is selected from the group consisting of

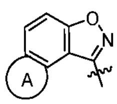 and 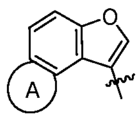.

In some embodiments, the structural moiety

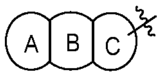

is selected from the group consisting of

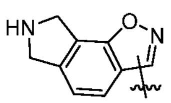, 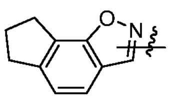, 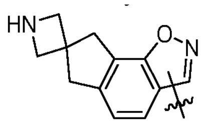, 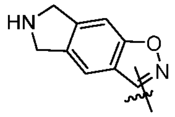, 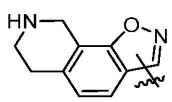, 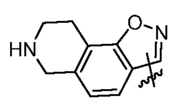,

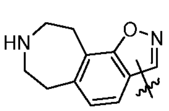, 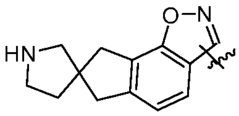, 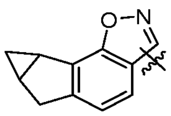, 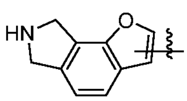, 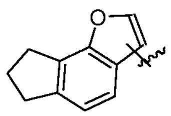, 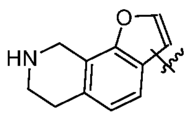,

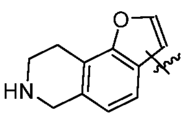, 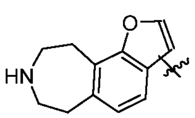, 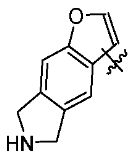, 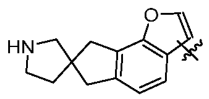, 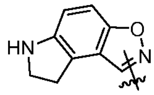, 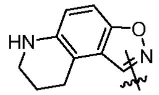, and

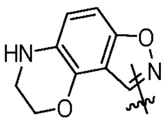.

In another aspect, the present application relates to a compound of formula III-1 below, a moiety, an isomer (e.g., a stereoisomer) thereof, a derivative (such as a Protac molecule) thereof, or a pharmaceutically acceptable salt thereof:

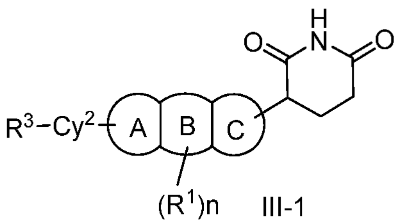
III-1

wherein $R^3$ is selected from the group consisting of oxo, hydroxy, $-(CH_2)_m-CHO$, and hydroxy $C_{1-5}$ alkylene, and m is 0, 1, 2, 3, 4, or 5, preferably 0; $Cy^2$, ring A, ring B, ring C, $R^1$, and n are as defined herein.

In another aspect, the present application relates to a compound of formula I'''-1a or I'''-2a below, a moiety, an isomer (e.g., a stereoisomer) thereof, a derivative (such as a Protac molecule) thereof, or a pharmaceutically acceptable salt thereof:

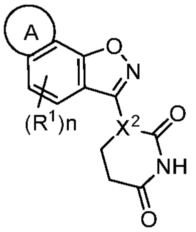 I'''-1a, 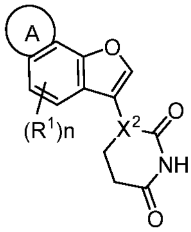 I'''-2a,

wherein,

ring A is selected from the group consisting of $C_{5-10}$ cycloalkenyl and 5- to 10-membered heterocycloalkenyl; or, ring A is selected from the group consisting of $C_{5-8}$ cycloalkenyl and 5- to 10-membered heterocycloalkenyl; or, ring A is selected from the group consisting of $C_{5-7}$ cycloalkenyl and 5- to 10-membered heterocycloalkenyl; or, ring A is selected from the group consisting of $C_{5-6}$ cycloalkenyl and 5- to 10-membered heterocycloalkenyl;

n is as defined herein;

each R' is independently selected from the group consisting of halogen, -OH, $-NH_2$, -CN, =O, $C_{1-4}$ alkoxy, -CHO, $C_{3-6}$ cycloalkyl, 3- to 10-membered heterocycloalkyl, and $C_{1-4}$ alkyl, wherein the $C_{3-6}$ cycloalkyl, 3- to 10-membered heterocycloalkyl, or $C_{1-4}$ alkyl is optionally substituted with halogen, -OH, $-NH_2$, or $C_{1-4}$ alkyl-OH; or, each R' is independently selected from the group consisting of halogen, -OH, $-NH_2$, -CN, =O, $C_{1-4}$ alkoxy, -CHO, $C_{3-6}$ cycloalkyl, and $C_{1-4}$ alkyl, wherein the $C_{3-6}$ cycloalkyl or $C_{1-4}$ alkyl is optionally substituted with halogen, -OH, $-NH_2$, or $C_{1-4}$

alkyl-OH;
$X^2$ is selected from the group consisting of CH and N.

In some embodiments, each R' is independently selected from the group consisting of halogen, -OH, $-NH_2$, -CN, =O, $C_{1-3}$ alkoxy, -CHO, $C_{3-4}$ cycloalkyl, and $C_{1-3}$ alkyl, wherein the $C_{3-4}$ cycloalkyl or $C_{1-3}$ alkyl is optionally substituted with halogen, -OH, $-NH_2$, or $C_{1-3}$ alkyl-OH.

In some embodiments, each R' is independently selected from the group consisting of halogen, -OH, $-NH_2$, -CN, =O, methoxy, -CHO, cyclobutyl, and methyl, wherein the cyclobutyl or methyl is optionally substituted with halogen, -OH, $-NH_2$, or $CH_2OH$.

In some embodiments, each R' is independently selected from the group consisting of F, -OH, $-NH_2$, $-CH_2OH$, =O, -CHO, $-CH_2NH_2$, and

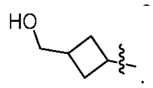

In some specific embodiments, each R' is independently selected from the group consisting of halogen, -OH, $-NH_2$, -CN, =O, methoxy, -CHO, and $C_{1-3}$ alkyl, wherein the $C_{1-3}$ alkyl is optionally substituted with halogen, -OH, or $-NH_2$.

In some specific embodiments, each R' is independently selected from the group consisting of halogen, -OH, $-NH_2$, -CN, =O, methoxy, -CHO, and methyl, wherein the methyl is optionally substituted with -OH or $-NH_2$.

In some specific embodiments, each R' is independently selected from the group consisting of F, -OH, $-NH_2$, $-CH_2OH$, =O, -CHO, and $-CH_2NH_2$.

In some embodiments, the moiety

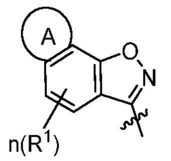 or 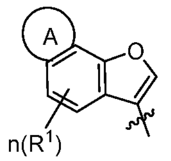

is selected from the group consisting of

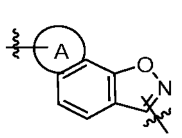, 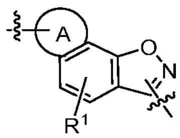, and 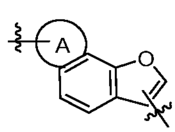;

preferably, the moiety is selected from the group consisting of

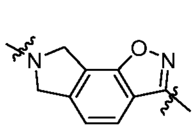, 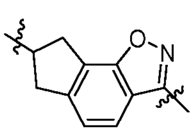, 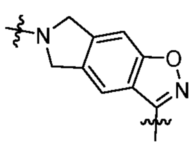, 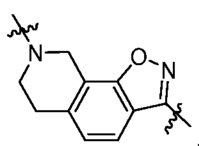, 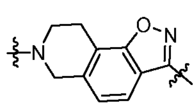, and

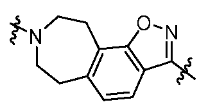.

In another aspect, the present application relates to a compound of formula I'''-1 or I'''-2 below, a moiety, an isomer (e.g., a stereoisomer) thereof, a derivative (such as a Protac molecule) thereof, or a pharmaceutically acceptable salt thereof:

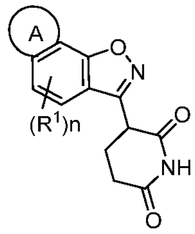 I'''-1, 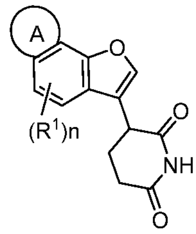 I'''-2,

wherein,

ring A is selected from the group consisting of $C_{5-10}$ cycloalkenyl and 5- to 10-membered heterocycloalkenyl; or, ring A is selected from the group consisting of $C_{5-8}$ cycloalkenyl and 5- to 10-membered heterocycloalkenyl; or, ring A is selected from the group consisting of $C_{5-7}$ cycloalkenyl and 5- to 10-membered heterocycloalkenyl; or, ring A is selected from the group consisting of $C_{5-6}$ cycloalkenyl and 5- to 10-membered heterocycloalkenyl; R’ and n are as defined herein.

In some embodiments, ring A is selected from the group consisting of $C_{5-6}$ cycloalkenyl and 5- to 9-membered heterocycloalkenyl. In some embodiments, ring A is selected from the group consisting of $C_5$ cycloalkenyl, $C_6$ cycloalkenyl, 5-, 6-, 7-, 8-, and 9-membered heterocycloalkenyl.

In some embodiments, ring A is selected from the group consisting of cyclopentenyl, bicyclohexenyl, dihydropyrrolyl, tetrahydropyridinyl, tetrahydroazepinyl, dihydrooxazinyl, azaspirooctenyl, and azaspirononenyl. In some embodiments, ring A is selected from the group consisting of cyclopentenyl and bicyclohexenyl. In some embodiments, ring A is selected from the group consisting of dihydropyrrolyl, tetrahydropyridinyl, tetrahydroazepinyl, dihydrooxazinyl, azaspirooctenyl, and azaspirononenyl.

In some embodiments, ring A is selected from the group consisting of

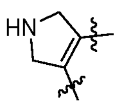, 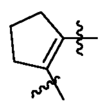, 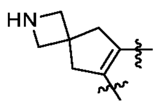,

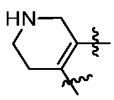, 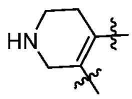, 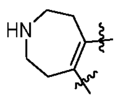, 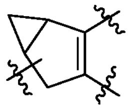, 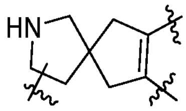, 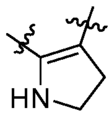, 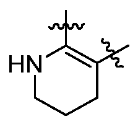, and 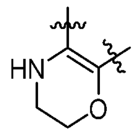.

In some embodiments, ring A is selected from the group consisting of

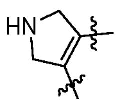, 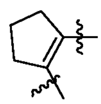, 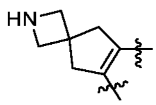,

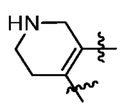, 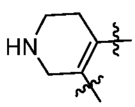, 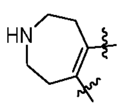, 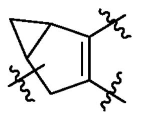, and 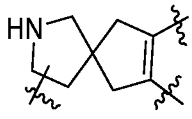.

In some other embodiments, ring A is selected from the group consisting of $C_{5-8}$ cycloalkenyl and 5- to 8-membered heterocycloalkenyl. In some other embodiments, ring A is selected from the group consisting of $C_{5-6}$ cycloalkenyl and 5- to 8-membered heterocycloalkenyl. In some embodiments, ring A is selected from the group consisting of $C_5$ cycloalkenyl, 5-, 6-, 7-, and 8-membered heterocycloalkenyl.

In some other embodiments, ring A is selected from the group consisting of cyclopentenyl, dihydropyrrolyl, tetrahydropyridinyl, tetrahydroazepinyl, and azaspirooctene.

In some other embodiments, ring A is selected from cyclopentenyl. In some embodiments, ring A is selected

from the group consisting of dihydropyrrolyl, tetrahydropyridinyl, tetrahydroazepinyl, and azaspirooctene.

In some other embodiments, ring A is selected from the group consisting of

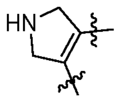 , 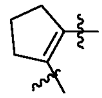 , 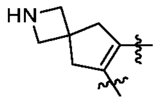 ,

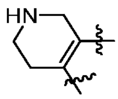 , 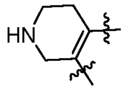 , and 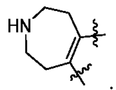 .

The present application relates to the following compound, a moiety, a stereoisomer thereof, a derivative (such as a Protac molecule) thereof, or a pharmaceutically acceptable salt thereof:

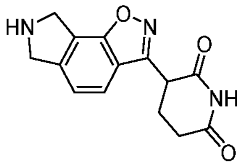 , 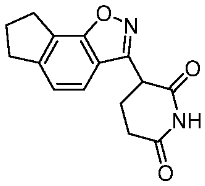 , 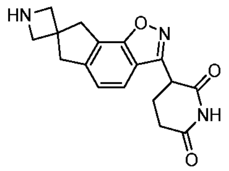 , 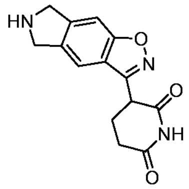 , 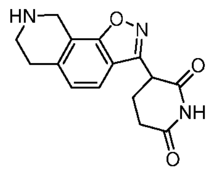 ,

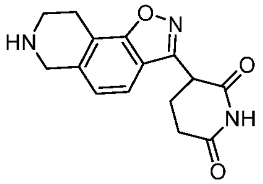 , 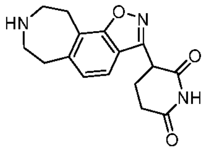 , 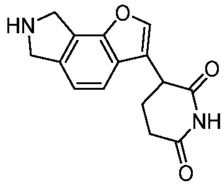 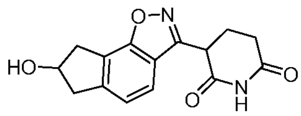 ,

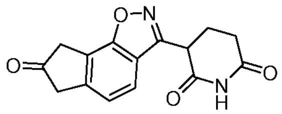 , 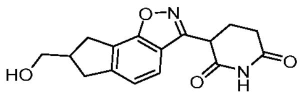 , 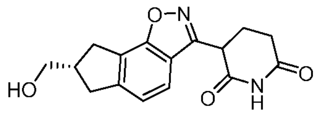 ,

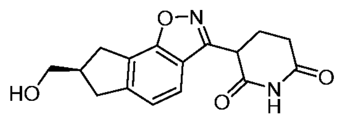 , 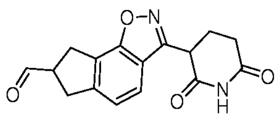 , 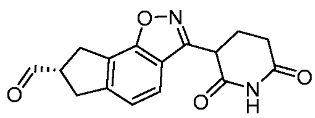 ,

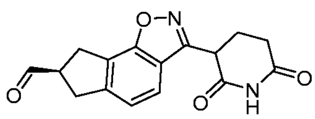 , 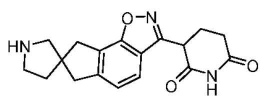 , 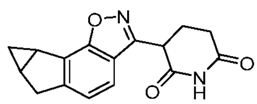 , 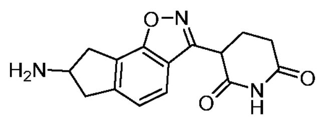 ,

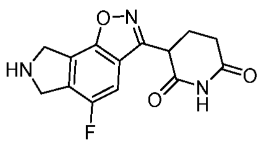 , 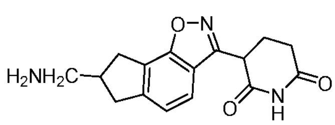 , 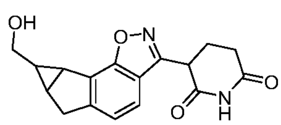 , 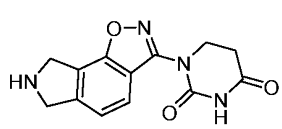 ,

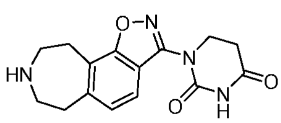 , 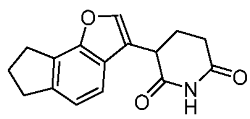 , 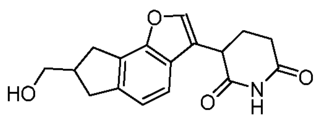 , 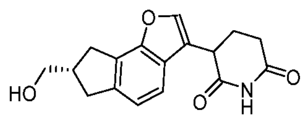 ,

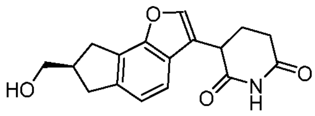 , 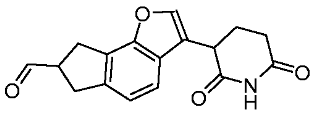 , 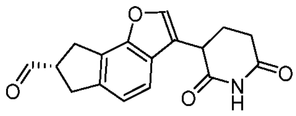 ,

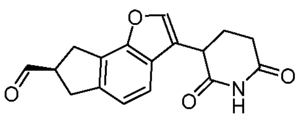 , 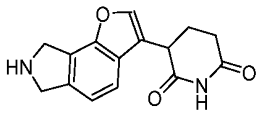 , 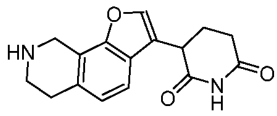 , 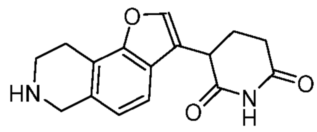 ,

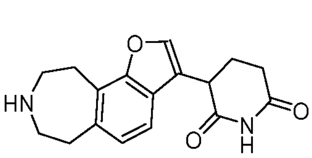 , 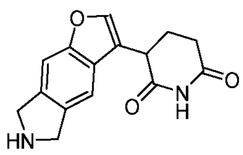 , 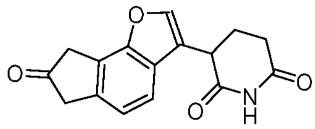 ,

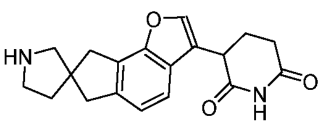 , 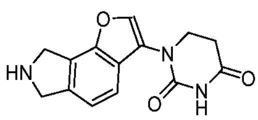 , 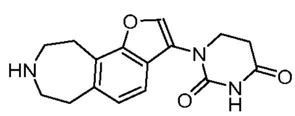 , 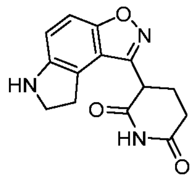 ,

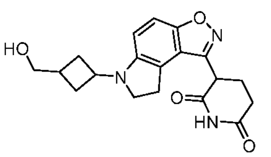 , 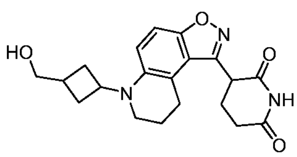 , 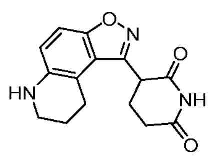 , 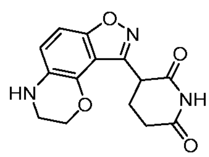 , or

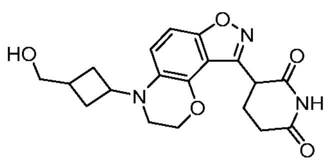 .

In another aspect, the present application relates to a compound of formula I‴-1c or I‴-2d below, a moiety, a stereoisomer thereof, a derivative (such as a Protac molecule) thereof, or a pharmaceutically acceptable salt thereof:

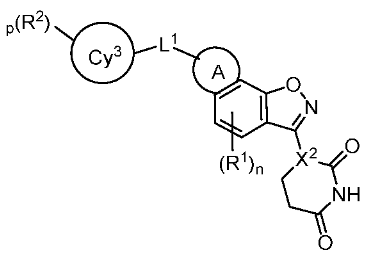 I‴-1c, 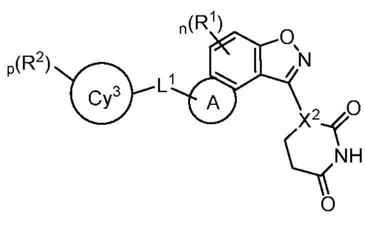 I‴-1d

wherein,

ring A, n, and $R^1$ are as defined herein;

$X^2$ is selected from the group consisting of CH and N;

L' is selected from $C_{0-3}$ alkylene;

$Cy^3$ is selected from the group consisting of $C_{3-8}$ cycloalkyl and 3- to 8-membered heterocycloalkyl;

$R^2$ is selected from the group consisting of -CHO, OH, SH, $NH_2$, COOH, and $C_{1-6}$ alkyl substituted with one or more SH, OH, or $NH_2$;

p is selected from the group consisting of 0, 1, 2, and 3.

In some embodiments, ring A is selected from the group consisting of $C_{5-9}$ cycloalkenyl, 5- to 9-membered heterocycloalkenyl, phenyl, and 5- to 6-membered heteroaryl.

In some embodiments, ring A is selected from the group consisting of $C_{5-7}$ cycloalkenyl, 5- to 9-membered heterocycloalkenyl, phenyl, and 5- to 6-membered heteroaryl.

In some embodiments, ring A is selected from the group consisting of $C_{5-6}$ cycloalkenyl, 5- to 9-membered heterocycloalkenyl, phenyl, and 5- to 6-membered heteroaryl.

In some embodiments, ring A is selected from the group consisting of 5- to 6-membered heterocycloalkenyl, phenyl, and 5-membered heteroaryl.

In some embodiments, ring A is selected from the group consisting of dihydropyrrolyl, tetrahydropyridinyl, dihydrooxazinyl, phenyl, pyrrolyl, pyrazolyl, and furanyl.

In some embodiments, the moiety

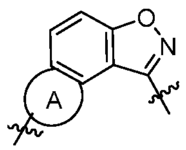

is selected from the group consisting of

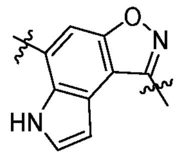 ,

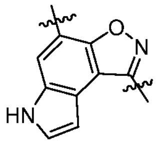 , 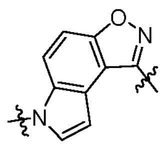 , 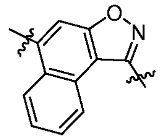 , 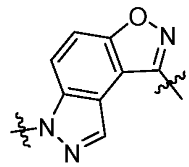 , 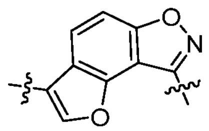 , 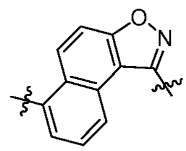 ,

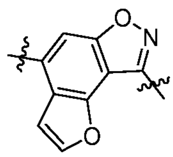 , 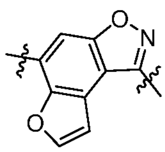 , 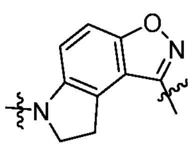 , 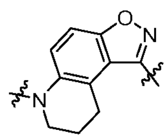 , and 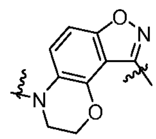 .

In some embodiments, L' is selected from the group consisting of a bond and $-CH_2-$.

In some embodiments, $Cy^3$ is selected from the group consisting of $C_{3-6}$ cycloalkyl and 4- to 6-membered heterocycloalkyl.

In some embodiments, $Cy^3$ is selected from the group consisting of $C_{4-6}$ cycloalkyl, 4-membered heterocycloalkyl, and 6-membered heterocycloalkyl.

In some embodiments, $Cy^3$ is selected from the group consisting of cyclobutyl, cyclopentyl, cyclohexyl, azetidinyl, and piperidinyl.

In some embodiments, $R^2$ is selected from the group consisting of -CHO, OH, and $C_{1-3}$ alkyl substituted with one or more OH or $NH_2$.

In some embodiments, $R^2$ is selected from the group consisting of -CHO, OH, and methyl substituted with one or more OH.

In some embodiments, $R^2$ is selected from the group consisting of -CHO, OH, and $-CH_2OH$.

In some embodiments, $X^2$ is selected from CH.

In some embodiments, p is selected from the group consisting of 0, 1, and 2.

In some embodiments, p is selected from the group consisting of 0 and 1. In some embodiments, p is selected from 0. In some embodiments, p is selected from 1.

In some embodiments, the structural moiety

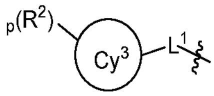

is selected from the group consisting of

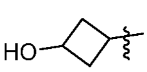, 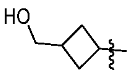, 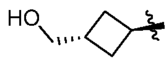, 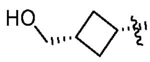, 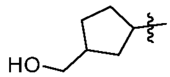, 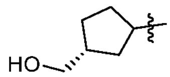, 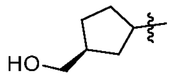,

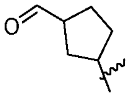, 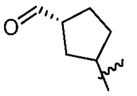, 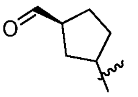, 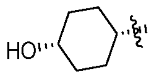, 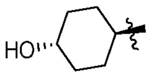, 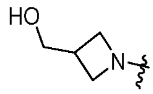, 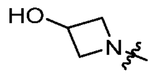,

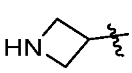, 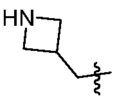, and 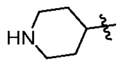.

The present application relates to the following compound, a moiety, a stereoisomer thereof, a derivative (such as a Protac molecule) thereof, or a pharmaceutically acceptable salt thereof,

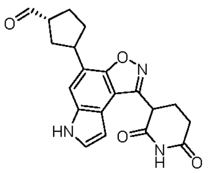
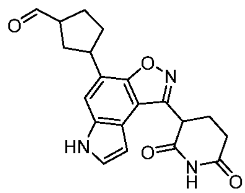
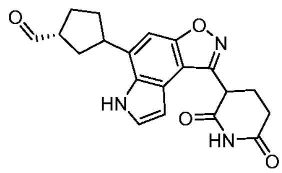

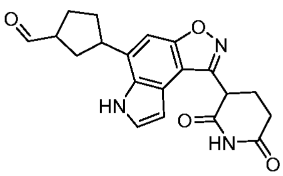
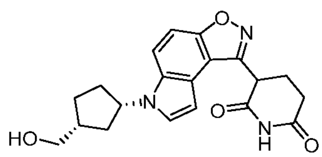
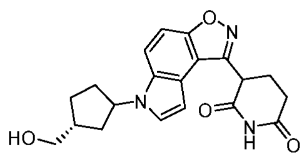
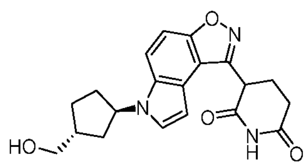

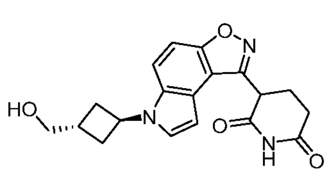
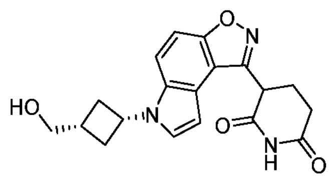
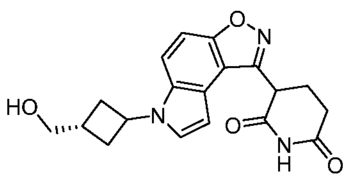

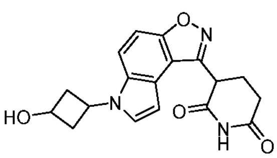
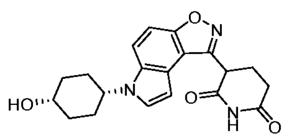
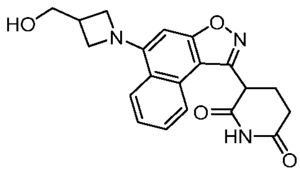
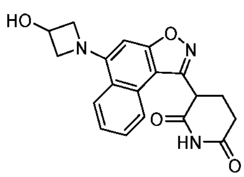

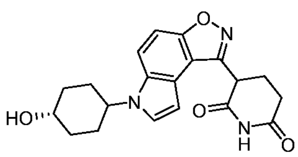 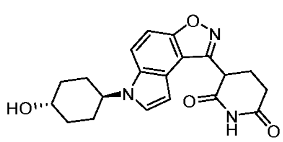 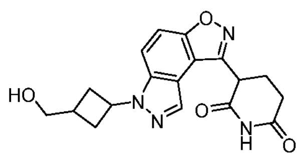 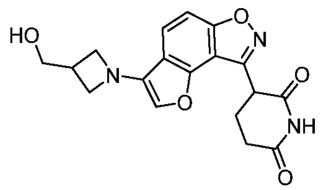

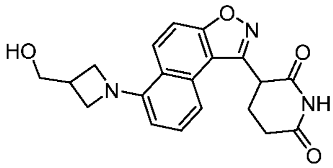 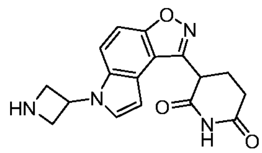 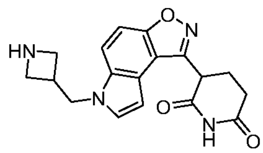 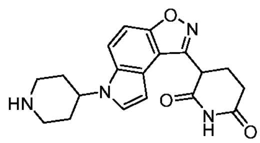

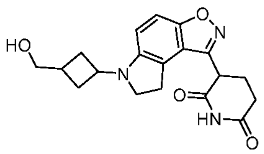 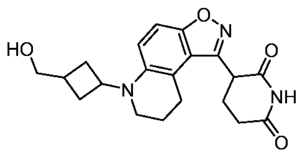 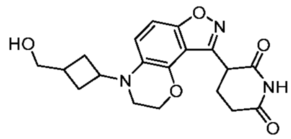 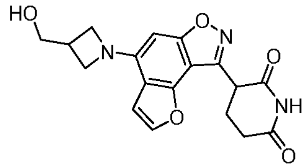

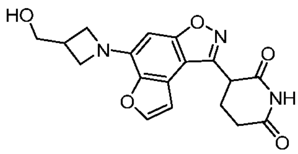 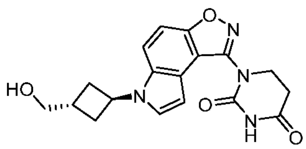 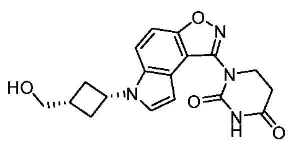 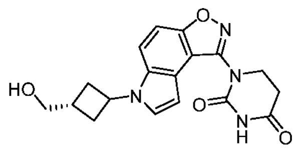

or 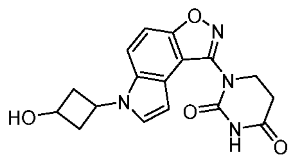.

The present application relates to the following compound, a moiety, a stereoisomer thereof, a derivative (such as a Protac molecule) thereof, or a pharmaceutically acceptable salt thereof,

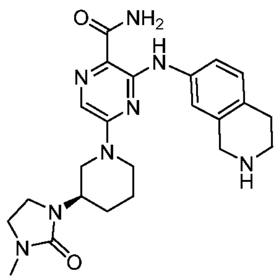 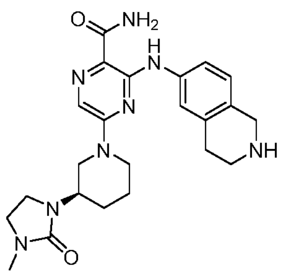 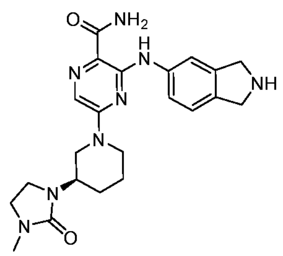 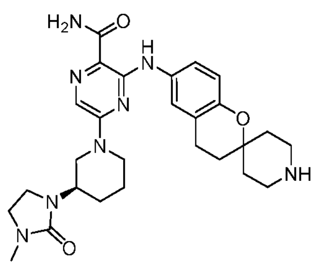

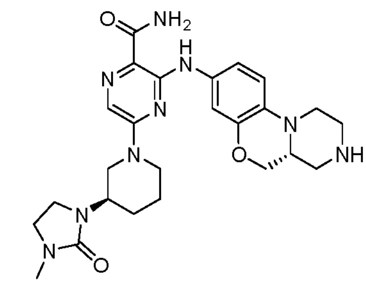 or 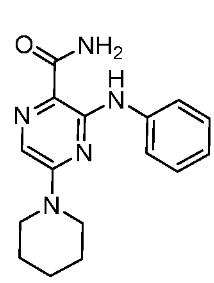.

In another aspect, the present application relates to use of the compound (e.g., formula III, III-1, I‴-1a, I‴-2a,

I‴-1, I‴-2, I‴-1c, I‴-2d, or a specific compound), the moiety, the isomer (e.g., a stereoisomer) thereof, and the derivative thereof in a Protac molecule. In another aspect, the present application relates to use of the compound (e.g., formula III, III-1, I‴-1a, I‴-2a, I‴-1, I‴-2, I‴-1c, I‴-2d, or a specific compound), the moiety, the isomer (e.g., a stereoisomer) thereof, and the derivative thereof for constituting part of a Protac molecule. In another aspect, the present application relates to the compound (e.g., formula III, III-1, I‴-1a, I‴-2a, I‴-1, I‴-2, I‴-1c, I‴-2d, or a specific compound), the moiety, the isomer (e.g., a stereoisomer) thereof, and the derivative thereof present in the form of a Protac molecule. In another aspect, the present application relates to use of the compound (e.g., formula III, III-1, I‴-1a, I‴-2a, I‴-1, I‴-2, I‴-1c, I‴-2d, or a specific compound), the moiety, the isomer (e.g., a stereoisomer) thereof, and the derivative thereof for degrading a protein. For example, the compound (e.g., formula III, III-1, I‴-1a, I‴-2a, I‴-1, I‴-2, I‴-1c, I‴-2d, or a specific compound), the moiety, the isomer (e.g., a stereoisomer) thereof, and the derivative thereof degrade the protein in the form of a Protac molecule. In another aspect, the present application relates to use of the compound (e.g., formula III, III-1, I‴-1a, I‴-2a, I‴-1, I‴-2, I‴-1c, I‴-2d, or a specific compound), the moiety, the isomer (e.g., a stereoisomer) thereof, and the derivative thereof for degrading a protein in the form of a Protac molecule. The present application relates to use of the compound (e.g., formula III, III-1, I‴-1a, I‴-2a, I‴-1, I‴-2, I‴-1c, I‴-2d, or a specific compound), the moiety, the isomer (e.g., a stereoisomer) thereof, and the derivative thereof (e.g., as a preparation intermediate) for preparing a Protac molecule. The present application relates to use of the compound (e.g., formula III, III-1, I‴-1a, I‴-2a, I‴-1, I‴-2, I‴-1c, I‴-2d, or a specific compound), the moiety, the isomer (e.g., a stereoisomer) thereof, and the derivative thereof (e.g., as a preparation intermediate) for preparing a protein degrader. Optionally, the Protac molecule may not include a Protac molecule that relates to AR.

Specifically, for example, in some embodiments, the present application relates to use of the following compound, a moiety thereof, a stereoisomer thereof, or a pharmaceutically acceptable salt thereof for preparing a Protac molecule:

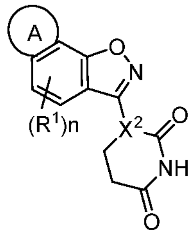 I‴-1a or 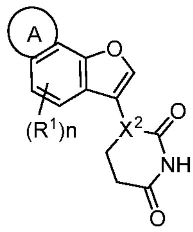 I‴-2a

wherein,

ring A is selected from the group consisting of $C_{5-10}$ cycloalkenyl and 5- to 10-membered heterocycloalkenyl; or, ring A is selected from the group consisting of $C_{5-8}$ cycloalkenyl and 5- to 10-membered heterocycloalkenyl; or, ring A is selected from the group consisting of $C_{5-7}$ cycloalkenyl and 5- to 10-membered heterocycloalkenyl; or, ring A is selected from the group consisting of $C_{5-6}$ cycloalkenyl and 5- to 10-membered heterocycloalkenyl; preferably, ring A is selected from the group consisting of cyclopentenyl, dihydropyrrolyl, tetrahydropyridinyl, and tetrahydroazepinyl;

n is selected from the group consisting of 0, 1, 2, and 3; preferably, n is selected from the group consisting of 0, 1, and 2;

each R’ is independently selected from the group consisting of halogen, -OH, $-NH_2$, -CN, =O, $C_{1-4}$ alkoxy, -CHO, $C_{3-6}$ cycloalkyl, and $C_{1-4}$ alkyl, wherein the $C_{3-6}$ cycloalkyl or $C_{1-4}$ alkyl is optionally substituted with halogen, -OH, $-NH_2$, or $C_{1-4}$ alkyl-OH; preferably, each R’ is independently selected from the group consisting of fluorine, chlorine, and bromine;

$X^2$ is selected from the group consisting of CH and N; preferably, $X^2$ is CH;

the moiety of the compound may be

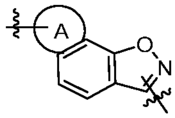, 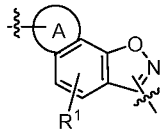, or 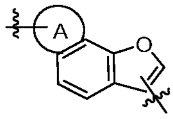;

preferably, the moiety is selected from the group consisting of

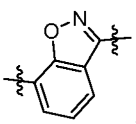, 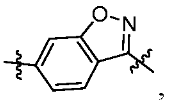, 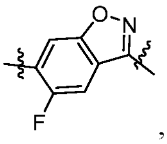, 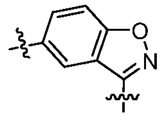, 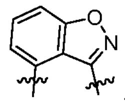,

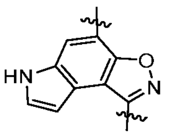, 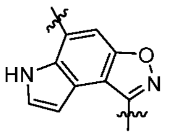, 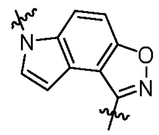, 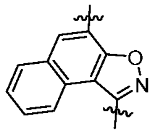, 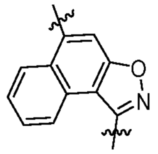, 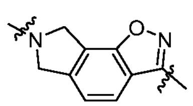,

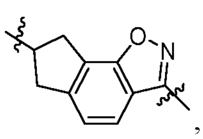, 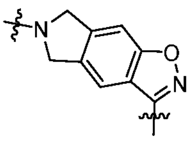, 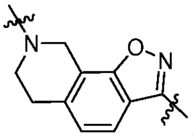, 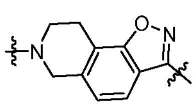, and 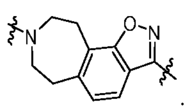.

In some embodiments, the present application relates to a method for preparing a Protac molecule, comprising: reacting the following compound, a moiety thereof, a stereoisomer thereof, or a pharmaceutically acceptable salt thereof to prepare the Protac molecule:

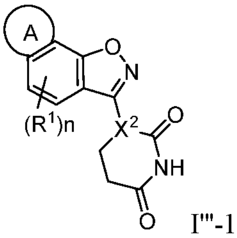 I'''-1a or 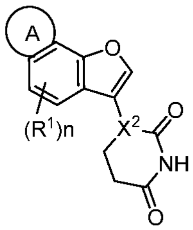 I'''-2a

wherein,

ring A is selected from the group consisting of $C_{5-10}$ cycloalkenyl and 5- to 10-membered heterocycloalkenyl; or, ring A is selected from the group consisting of $C_{5-8}$ cycloalkenyl and 5- to 10-membered heterocycloalkenyl; or, ring A is selected from the group consisting of $C_{5-7}$ cycloalkenyl and 5- to 10-membered heterocycloalkenyl; or, ring A is selected from the group consisting of $C_{5-6}$ cycloalkenyl and 5- to 10-membered heterocycloalkenyl; preferably, ring A is selected from the group consisting of cyclopentenyl, dihydropyrrolyl, tetrahydropyridinyl, and tetrahydroazepinyl;
n is selected from the group consisting of 0, 1, 2, and 3; preferably, n is selected from the group consisting of 0, 1, and 2;
each R' is independently selected from the group consisting of halogen, -OH, $-NH_2$, -CN, =O, $C_{1-4}$ alkoxy, -CHO, $C_{3-6}$ cycloalkyl, and $C_{1-4}$ alkyl, wherein the $C_{3-6}$ membered cycloalkyl or $C_{1-4}$ alkyl is optionally substituted with halogen, -OH, $-NH_2$, or $C_{1-4}$ alkyl-OH; preferably, each R' is independently selected from the group consisting of fluorine, chlorine, and bromine;
$X^2$ is selected from the group consisting of CH and N; preferably, $X^2$ is CH;
the moiety of the compound may be

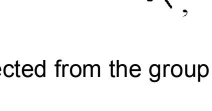, 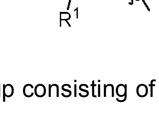, or ;

preferably, the moiety is selected from the group consisting of

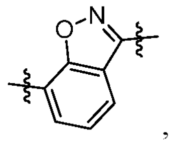, 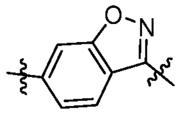, 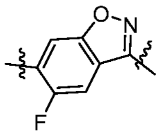, 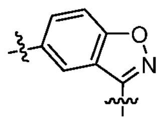, 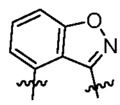,

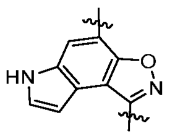, 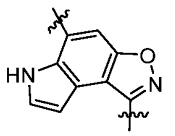, 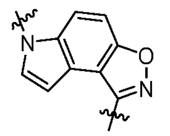, 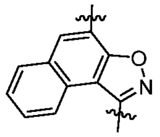, 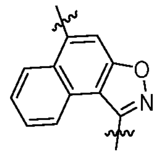, 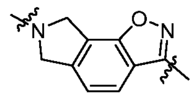,

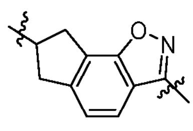, 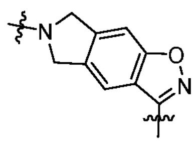, 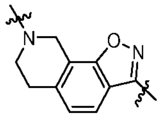, 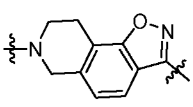, and 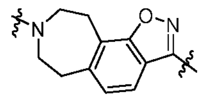.

The present application relates to use of the compound (e.g., formula III, III-1, I‴-1a, I‴-2a, I‴-1, I‴-2, I‴-1c, I‴-2d, or a specific compound), the moiety, the isomer (e.g., a stereoisomer) thereof, and the derivative thereof for degrading BTK protein. For example, the compound, the moiety, the isomer (e.g., a stereoisomer) thereof, and the derivative thereof degrade the BTK protein in the form of a Protac molecule. In another aspect, the present application relates to use of the compound (e.g., formula III, III-1, I‴-1a, I‴-2a, I‴-1, I‴-2, I‴-1c, I‴-2d, or a specific compound), the moiety, the isomer (e.g., a stereoisomer) thereof, and the derivative thereof for degrading BTK protein in the form of a Protac molecule.

In some embodiments, the Protac molecule or protein degrader is selected from a BTK protein degrader/molecule. In another aspect, the present application relates to a pharmaceutical composition comprising the compound, the moiety, the stereoisomer thereof, the derivative thereof, or the pharmaceutically acceptable salt thereof of the present application described above. The pharmaceutical composition of the present application also comprises a pharmaceutically acceptable excipient.

In another aspect, the present application relates to use of the compound, the moiety, the stereoisomer thereof, the derivative thereof or the pharmaceutically acceptable salt thereof, or the pharmaceutical composition thereof described above for preparing a medicament for preventing or treating a disorder treated by degrading a target protein that binds to a targeting ligand.

In another aspect, the present application relates to use of the compound, the moiety, the stereoisomer thereof, the derivative thereof or the pharmaceutically acceptable salt thereof, or the pharmaceutical composition thereof described above for preparing a medicament for preventing or treating a disorder treated by binding to cereblon protein *in vivo.*

In another aspect, the present application relates to use of the compound, the moiety, the stereoisomer thereof, the derivative thereof or the pharmaceutically acceptable salt thereof, or the pharmaceutical composition thereof described above for preparing a medicament for preventing or treating a BTK-related disease.

The present application relates to a method for treating or preventing a disorder treated by degrading a target protein that binds to a targeting ligand in a mammal, comprising administering to a mammal, preferably a human, in need of such treatment a therapeutically effective amount of the compound, the stereoisomer thereof, the derivative thereof or the pharmaceutically acceptable salt thereof, or the pharmaceutical composition thereof of the present application described above.

The present application relates to a method for treating or preventing a disorder treated by binding to cereblon protein *in vivo,* comprising administering to a mammal, preferably a human, in need of such treatment a therapeutically effective amount of the compound, the stereoisomer thereof, the derivative thereof or the pharmaceutically acceptable salt thereof, or the pharmaceutical composition thereof of the present application described above.

In another aspect, the present application relates to a method for treating a BTK-related disease in a mammal, comprising administering to a mammal, preferably a human, in need of such treatment a therapeutically effective amount of the compound, the stereoisomer thereof, the derivative thereof or the pharmaceutically acceptable salt thereof, or the pharmaceutical composition thereof of the present application described above.

In another aspect, the present application relates to the compound, the moiety, the stereoisomer thereof, the derivative thereof or the pharmaceutically acceptable salt thereof, or the pharmaceutical composition thereof described above for use in preventing or treating a disorder treated by degrading a target protein that binds to a targeting ligand.

In another aspect, the present application relates to the compound, the moiety, the stereoisomer thereof, the

derivative thereof or the pharmaceutically acceptable salt thereof, or the pharmaceutical composition thereof described above for use in preventing or treating a disorder treated by binding to cereblon protein *in vivo.*

In another aspect, the present application relates to the compound, the moiety, the stereoisomer thereof, the derivative thereof or the pharmaceutically acceptable salt thereof, or the pharmaceutical composition thereof described above for use in preventing or treating a BTK-related disease.

In another aspect, the present application relates to use of the compound, the moiety, the stereoisomer thereof, the derivative thereof or the pharmaceutically acceptable salt thereof, or the pharmaceutical composition thereof described above for preventing or treating a disorder treated by degrading a target protein that binds to a targeting ligand.

In another aspect, the present application relates to use of the compound, the moiety, the stereoisomer thereof, the derivative thereof or the pharmaceutically acceptable salt thereof, or the pharmaceutical composition thereof described above for preventing or treating a disorder treated by binding to cereblon protein *in vivo.*

In another aspect, the present application relates to use of the compound, the moiety, the stereoisomer thereof, the derivative thereof or the pharmaceutically acceptable salt thereof, or the pharmaceutical composition thereof described above for preventing or treating a BTK-related disease. In some specific embodiments, the BTK-related disease described above is selected from a disorder treated by degrading a protein that binds to a ligand of a BTK target protein; in some specific embodiments, the BTK-related disease described above is selected from a disorder treated by binding to cereblon protein *in vivo;* in some embodiments, the disease or disorder described above is selected from the group consisting of autoimmune diseases, inflammatory diseases, and cancer.

In some specific embodiments, the disorder treated by binding to cereblon protein *in vivo* and/or the disorder treated by binding to cereblon protein *in vivo* described above is selected from a BTK-related disease; in some specific embodiments, the BTK-related disease described above is selected from the group consisting of autoimmune diseases, inflammatory diseases, and cancer.

In some embodiments, the "one or more" is selected from the group consisting of one, two, three, four, five, and six. In some embodiments, the "one or more" is selected from the group consisting of one, two, and three. In some embodiments, the "one or more" is selected from the group consisting of one and two.

In some embodiments, the present application encompasses the variables defined above and embodiments thereof, as well as any combination thereof.

**Technical Effects**

The compound of the present application has a degradation effect on BTK of OCI-LY10 cells, can inhibit cell (OCI-LY10 cells, TMD8-$BTK^{C481S}$ cells, or OCI-LY10-$BTK^{C481S}$ cells) proliferation *in vitro,* has selectivity for BTK and/or $BTK^{C481S}$ kinase as compared to EGFR and TEC kinases, has stable *in vitro* metabolism and good *in vivo* pharmacokinetic properties, has an inhibition effect on mouse TMD-8 xenograft tumor *in vivo,* has binding activity to CRBN protein, and can exhibit the desired IKZF1, IKZF3, and GSPT1 protein degradation activity. The compound provided by the present application can bind to a cereblon receptor of a $CRL4^{CRBN}$ E3 ubiquitin ligase, so that a new binding site is generated on a protein new substrate serving as a human disease medium, thereby causing the protein degradation of the new substrate. The new morphology surface generated by these compounds can interact directly with a target protein or target protein complex, thereby directly or indirectly reducing protein levels. In various embodiments, the compound of the present application can produce a reduction in the level of a new substrate target protein through direct ubiquitination of the target protein, or ubiquitinate a new substrate target protein cofactor or target protein complex or other proteins responsible for controlling target protein homeostasis. These compounds may lead to degradation of a new substrate target protein of cerebrum that binds to a direct binding ligand, degradation of a new substrate that acts as a cofactor for the binding ligand to bind to cerebrum, degradation of cerebrum that binds to a complex cofactor and target protein surface binding ligand, degradation of a new substrate target protein complex of CRBN that binds to a binding ligand, or reduce the target protein levels by degrading the cofactor of a protein or new substrate protein not in the complex.

**Definitions**

Unless otherwise stated, the following terms used in the present application shall have the following meanings. A certain term, unless otherwise specifically defined, should not be considered ambiguous or unclear, but construed according to its common meaning in the art. When referring to a trade name, it is intended to refer to its corresponding commercial product or its active ingredient.

The term "substituted" means that any one or more hydrogen atoms on a specific atom are substituted with substituents, as long as the valence of the specific atom is normal and the resulting compound is stable. When the substituent is oxo (i.e., =O), it means that two hydrogen atoms are substituted and oxo is not possible on an aromatic group.

The term "optional" or "optionally" means that the subsequently described event or circumstance may, but does not necessarily, occur. The description includes instances where the event or circumstance occurs and instances where

it does not. An "optionally substituted" group means that the group is substituted or unsubstituted. For example, ethyl being "optionally" substituted with halogen means that the ethyl may be unsubstituted ($CH_2CH_3$), monosubstituted (for example, $CH_2CH_2F$), polysubstituted (for example, $CHFCH_2F$, $CH_2CHF_2$, and the like), or fully substituted ($CF_2CF_3$). It will be appreciated by those skilled in the art that for any groups comprising one or more substituents, any substitutions or substituting patterns that may not exist spatially and/or cannot be synthesized are not introduced.

$C_{m-n}$ as used herein means that the portion has an integer number of carbon atoms in the given range (m-n). For example, "$C_{1-6}$" means that the group may have 1 carbon atom, 2 carbon atoms, 3 carbon atoms, 4 carbon atoms, 5 carbon atoms, or 6 carbon atoms.

When any variable (e.g., R) occurs once or more in the constitution or structure of a compound, the definition of the variable in each case is independent. For example, if a group comprises 2 R, the definition of each R is independent.

When a bond is crosslinked to two atoms of a ring (including monocyclic, fused or spiro ring), the bond may be bonded to any atom on the ring (including monocyclic, fused or spiro ring). For example, the structural unit

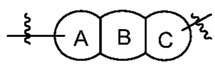

indicates that the bonds on two sides may be linked to any two different atoms on ring A, ring B, or ring C; for another example,

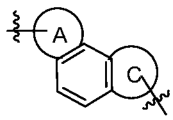

indicates that the bonds on two sides may be linked to any two different atoms on ring A, the middle benzene ring, or ring C; for further example,

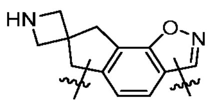

indicates that the bonds on two sides may be linked to any two different atoms of the four rings in the system.

The term "halo-" or "halogen" refers to fluorine, chlorine, bromine, and iodine.

The term "hydroxy" refers to -OH group.

The term "amino" refers to $-NH_2$ group.

The term "alkyl" refers to hydrocarbyl with a general formula of $C_nH_{2n+1}$. The alkyl may be linear or branched. For example, the term "$C_{1-6}$ alkyl" refers to alkyl containing 1 to 6 carbon atoms (for example, methyl, ethyl, n-propyl, isopropyl, n-butyl, isobutyl, sec-butyl, *tert*-butyl, *n*-pentyl, 1-methylbutyl, 2-methylbutyl, 3-methylbutyl, neopentyl, hexyl, 2-methylpentyl, and the like). The alkyl moieties (i.e., alkyl) of alkoxy, alkylamino, dialkylamino, alkylsulfonyl, and alkylthio are similarly defined as above.

The term "alkylene" is alkyl which has lost one hydrogen.

The term "heteroalkylene" refers to alkylene in which one or more C atoms are substituted with a heteroatom and which comprises at least one C atom. Specific heteroatoms may be selected from the group consisting of N, NH, O, S, S(O), and $S(O)_2$. The number of the heteroatom is selected from the group consisting of 1, 2, 3, 4, 5, and 6. For example, $C_{1-12}$ heteroalkylene means that the heteroalkylene comprises 1 to 12 C atoms and one or more heteroatoms (e.g., 1-6, 1-3, 1, 2, or 3 heteroatoms). For example, $C_{1-6}$ heteroalkylene means that the heteroalkylene comprises 1 to 6 C atoms and one or more heteroatoms.

The term "alkoxyl" refers to -O-alkyl.

The term "alkenyl" refers to linear or branched unsaturated aliphatic hydrocarbyl consisting of carbon atoms and hydrogen atoms with at least one double bond. Non-limiting examples of alkenyl include, but are not limited to, ethenyl, 1-propenyl, 2-propenyl, 1-butenyl, isobutenyl, 1,3-butadienyl, and the like.

The term "cycloalkenyl" refers to a non-aromatic carbon ring that is not fully saturated and may exist as a monocyclic, (e.g., bicyclic) bridged cyclic or spiro cyclic structure. Unless otherwise specified, the carbon ring is usually a 4- to 12-membered ring, a 4- to 10-membered ring, or a 4- to 8-membered ring. Non-limiting examples of cycloalkenyl include, but are not limited to, cyclopentenyl, cyclopentadienyl, cyclohexenyl, cyclohexadienyl, cycloheptenyl, cycloheptadienyl, and the like.

Non-limiting examples of "benzocycloalkenyl" include benzo 4- to 12-membered cycloalkenyl, benzo 4- to 10-membered cycloalkenyl, or benzo 4- to 8-membered (e.g., 4-, 5-, 6-, 7-, or 8-membered) cycloalkenyl.

The term "cycloalkyl" refers to a carbon ring that is fully saturated and may exist as a single ring, a bridged ring, or a spiro ring. Unless otherwise specified, the carbon ring is usually a 3- to 10-membered ring (e.g., a 5- to 8-membered ring). Non-limiting examples of cycloalkyl include, but are not limited to, cyclopropyl, cyclobutyl, cyclopentyl, cyclohexyl, norbornyl(bicyclo[2.2.1]heptyl), bicyclo[2.2.2]octyl, adamantyl, and the like.

The term "heterocycloalkyl" refers to a fully saturated cyclic group that may exist in the form of a monocyclic, bridged cyclic, or spiro cyclic structure. Unless otherwise specified, the heterocyclyl is usually a 3- to 12-membered, 3- to 10-membered, 3- to 8-membered, 3- to 7-membered, 3- to 6-membered, or 3- to 5-membered ring containing 1 to 3 heteroatoms (preferably 1 or 2 heteroatoms) independently selected from the group consisting of sulfur, oxygen, and nitrogen. Examples of 3-membered heterocycloalkyl include, but are not limited to, oxiranyl, thiiranyl, and aziranyl; non-limiting examples of 4-membered heterocycloalkyl include, but are not limited to, azetidinyl, oxetanyl, and thietanyl; examples of 5-membered heterocycloalkyl include, but are not limited to, tetrahydrofuranyl, tetrahydrothienyl, pyrrolidinyl, isoxazolidinyl, oxazolidinyl, isothiazolidinyl, thiazolidinyl, imidazolidinyl, and tetrahydropyrazolyl; examples of 6-membered heterocycloalkyl include, but are not limited to, piperidinyl, tetrahydropyranyl, tetrahydrothiopyranyl, morpholinyl, piperazinyl, 1,4-oxathianyl, 1,4-dioxanyl, thiomorpholinyl, 1,3-dithianyl, and 1,4-dithianyl; examples of 7-membered heterocycloalkyl include, but are not limited to, azacycloheptanyl, oxacycloheptanyl, and thiocycloheptanyl. Monocyclic heterocycloalkyl having 5 or 6 ring atoms is preferred.

The term "heterocycloalkenyl" includes cycloalkenyl in which one or more carbon atoms are substituted with a heteroatom, such as, specifically, cycloalkenyl in which up to 3 carbon atoms, in one embodiment up to 2 carbon atoms, and in another embodiment 1 carbon atom, are each independently replaced by O, S(O), NH, or N, provided that at least one cycloalkenyl carbon-carbon double bond is preserved. A cyclic group that may exist in the form of a monocyclic, bridged cyclic, or spiro cyclic structure may be a 3- to 12-membered ring (e.g., a 5- to 8-membered ring). Examples of heterocycloalkenyl include, but are not limited to, dihydropyrrolyl, tetrahydropyridinyl, tetrahydroazepinyl, or azaspirooctene.

Non-limiting examples of "benzoheterocycloalkenyl" include benzo 4- to 12-membered heterocycloalkenyl (e.g., 5-, 6-, 10-, or 11-membered), benzo 5- to 11-membered heterocycloalkenyl, or benzo 5- to 8-membered (e.g., 4-, 5-, 6-, 7-, or 8-membered) heterocycloalkenyl. Specific examples are

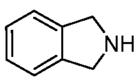, 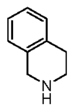, 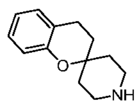,

or

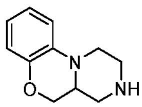.

Unless otherwise specified, the carbon ring is usually a 4- to 8-membered ring. Non-limiting examples of cycloalkenyl include, but are not limited to, cyclopentenyl, cyclopentadienyl, cyclohexenyl, cyclohexadienyl, cycloheptenyl, cycloheptadienyl, and the like.

The term "heteroaryl" refers to a monocyclic or fused polycyclic system which comprises at least one ring atom selected from the group consisting of N, O and S, with the remaining ring atoms being C, and which has at least one aromatic ring. Preferably, the heteroaryl has a single 4- to 8-membered ring, in particular a 5- to 8-membered ring, or has a plurality of fused rings comprising 6-14 ring atoms, in particular 6-10 ring atoms. Non-limiting examples of heteroaryl include, but are not limited to, pyrrolyl, furanyl, thienyl, imidazolyl, oxazolyl, pyrazolyl, pyridinyl, pyrimidinyl, pyrazinyl, quinolyl, isoquinolyl, tetrazolyl, triazolyl, triazinyl, benzofuranyl, benzothienyl, indolyl, isoindolyl, and the like.

In the present application, one of the absolute configurations (e.g., one of

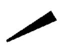

or

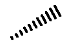 ;

specifically

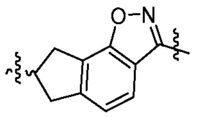

represents

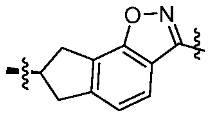

or

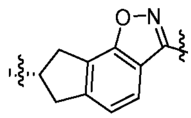

) or one of the relative configurations (e.g.,

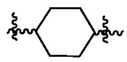

represents

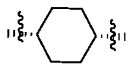 or 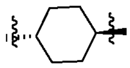

) of a stereogenic center is represented by a wavy line

( 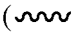 ).

Unless otherwise specified, the compounds disclosed herein include both *E* and *Z* geometric isomers when they contain olefinic double bonds or other centers of geometric asymmetry. Likewise, all tautomeric forms are included within the scope of the present application.

The group or moiety in the present application such as LNK, $Cy^1$, $Cy^2$, -$Cy^1$-LNK-$Cy^2$-, -Cy'-LNK-, or -LNK-$Cy^2$-, and specific options thereof, optionally can be linked to the left group and the right group of the group or moiety respectively in the general formula in a left-to-right reading order. For example, when $Cy^1$ is selected from

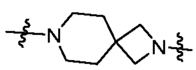,

according to a left-to-right reading order, the left side of $Cy^1$ is linked to the structural moiety

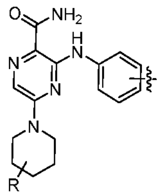

in the general formula corresponding to the left side, and the right side is linked to the right moiety

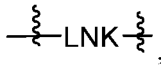

, forming the structural moiety

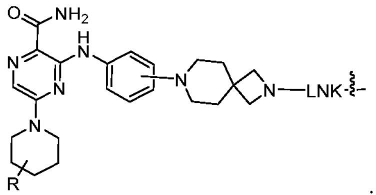

Optionally, the group or moiety in the present application such as LNK, $Cy^1$, $Cy^2$, $-Cy^1$-LNK-$Cy^2$-, -Cy'-LNK-, or -LNK-$Cy^2$-, and specific options thereof, can be linked to the left group and the right group of the group or moiety respectively in the general formula in a right-to-left reading order. For example, when $Cy^1$ is selected from

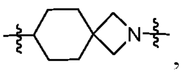

according to a right-to-left reading order, the right side of $Cy^1$ is linked to the structural moiety

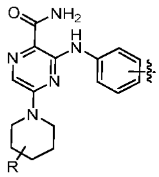

in the general formula corresponding to the left side, and the left side of $Cy^1$ is linked to the structural moiety

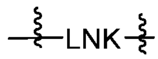

in the general formula corresponding to the right side to form the structural moiety

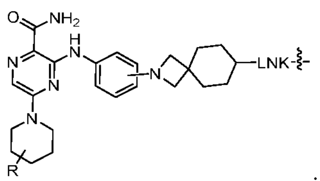

The other groups are as described above.

The term "proteolysis targeting chimera (Protac) molecule" is a bifunctional compound capable of combining a target protein and E3 ubiquitin ligase simultaneously. Such compounds can induce the target protein to be recognized

by proteasomes of cells, cause the degradation of the target protein and effectively reduce the content of the target protein in the cells.

The term "derivative" refers to one or a group of new compounds produced by substituting or replacing one or more hydrogen atoms in the basic structure of the parent compound with other groups or structural moieties. The derivative of the present application refers to a derivative compound that retains the parent structure. For example, the parent compound is derived into a Protac molecule, in particular a PTM-linker-ULM molecule, wherein PTM is a protein target moiety binding to the target protein and target polypeptide; the linker is a connecting group, and ULM refers to a moiety binding to a ubiquitin ligase.

The term "protein-targeting drug or derivative thereof' or PTM is used to describe small molecules that bind to a target protein or other proteins or polypeptides of interest and bring the localization/presence of the protein or polypeptide close to the ubiquitin ligase so that degradation of the protein or polypeptide by the ubiquitin ligase can occur. Non-limiting examples of small molecule target protein binding moieties include drugs or derivatives thereof, among numerous others, that target AR, ER, kinase, phosphatase, MDM2, human BET bromodomain protein, Hsp90, HDAC, human lysine methyltransferase, RAF receptor, FKBP, angiogenic factor, immunosuppression-related receptor or protein, arene receptor, thyroid hormone receptor, HIV protease, HIV integrase, HCV protease, HBV protease, or acyl protein thioesterase 1 and/or 2, or include drugs or derivatives thereof, among numerous others, that target ALK, BET, CDK, PARP, EGFR, γ-secretase, CBFβ-SMMHC, WEE1, MEK, BCR-ABL, MET, RAS, BTK, VEGFR, JAK, HER2, HDAC, Akt, PI3K, mTOR, AR, ER, PDEδ, SRC, MDM2, RAF, IRAK4, STAT3, and c-Myc, or include drugs or derivatives thereof, among numerous others, that target ALK, BRD4, CDK4/6, PARP, EGFR, γ-secretase, CBFβ-SMMHC, WEEl, MEK, BCR-ABL, MET, KRAS, EGFR, BTK, AR, ER, PDEδ, JAK, MDM2, or RAF

The term "PTM group" is a drug or a derivative thereof that binds to a target protein. There are many types of targets of PTM groups, and the PTM groups are selected from the group consisting of proteins that are expressed in a cell such that at least a portion of the sequence is found in the cell, and the protein can bind to the PTM group. The term "protein" includes oligopeptide and polypeptide sequences with sufficient length that can bind to the PTM group according to the present application. Any protein in eukaryotic systems or microbial systems (including viruses, bacteria, or fungi) as further described herein is a target for ubiquitination mediated by the compounds according to the present application. The target protein is preferably a eukaryotic protein.

"Target proteins" are used to describe below as proteins or polypeptides that bind to the compounds according to the present application and are targets for degradation by ubiquitin ligases. Such small molecule target protein binding moieties also include pharmaceutically acceptable salts, enantiomers, solvates, and polymorphs of the compositions, and other small molecules that can target the protein of interest. The binding moieties are linked to the group

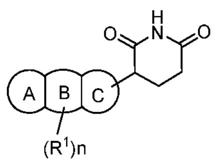

via a linker group L.

Unless otherwise specified,

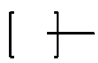

is used to indicate that a hydrogen atom at any position of a group within

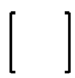

may be substituted with a group linked by "-".

The term "treat" or "treatment" means administering a compound or formulation described herein to ameliorate or eliminate a disease or one or more symptoms associated with the disease, including:

(i) inhibiting a disease or disease state, i.e., arresting its progression; and
(ii) alleviating a disease or disease state, i.e., causing its regression.

The term "prevent", "preventing", or "prevention" means administering the compound or formulation of the present application to prevent a disease or one or more symptoms associated with the disease, and includes: preventing the occurrence of the disease or disease state in a mammal, particularly when such a mammal is predisposed to the disease state but has not yet been diagnosed with it.

The term "therapeutically effective amount" refers to an amount of the compound disclosed herein for (i) treating or preventing a specific disease, condition, or disorder; (ii) alleviating, ameliorating, or eliminating one or more symptoms of a specific disease, condition or disorder, or (iii) preventing or delaying onset of one or more symptoms of a specific disease, condition, or disorder described herein. The amount of the compound disclosed herein composing the "therapeutically effective amount" varies depending on the compound, the disease state and its severity, the administration regimen, and the age of the mammal to be treated, but can be determined routinely by those skilled in the art in accordance with their knowledge and the present disclosure.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

A pharmaceutically acceptable salt, for example, may be a metal salt, an ammonium salt, a salt formed with an organic base, a salt formed with an inorganic acid, a salt formed with an organic acid, a salt formed with a basic or acidic amino acid, and the like.

The term "pharmaceutical composition" refers to a mixture consisting of one or more of the compounds or the salts thereof of the present application and a pharmaceutically acceptable excipient. The pharmaceutical composition is intended to facilitate the administration of the compound of the present application to an organism. The term "pharmaceutically acceptable excipient" refers to those that do not have a significant irritating effect on an organism and do not impair the biological activity and properties of the active compound. Suitable excipients are well known to those skilled in the art, for example, carbohydrate, wax, water-soluble and/or water-swellable polymers, hydrophilic or hydrophobic material, gelatin, oil, solvent, or water.

The word "comprise" and variations thereof such as "comprises" or "comprising" will be understood in an open, non-exclusive sense, i.e., "including but not limited to".

The compounds and intermediates disclosed herein may also exist in different tautomeric forms, and all such forms are included within the scope of the present application. The term "tautomer" or "tautomeric form" refers to structural isomers of different energies that can interconvert via a low energy barrier. For example, a proton tautomer (also referred to as prototropic tautomer) includes interconversion via proton transfer, such as keto-enol isomerism and imine-enamine isomerization. A specific example of a proton tautomer is an imidazole moiety where a proton can transfer between two ring nitrogens. A valence tautomer includes the interconversion via recombination of some bonding electrons.

Unless otherwise specified clearly herein, singular terms encompass plural terms, and vice versa. Similarly, unless otherwise specified clearly herein, the word "or" is intended to include "and".

Unless otherwise stated herein, parameter values representing amounts of ingredients or physicochemical properties or reaction conditions and the like are to be understood as being modified in all cases by the term "about". When the term "about" is used to describe the present application, the term "about" indicates that there is an error value; for example, it means varying within a range of $\pm5\%$, such as $\pm1\%$ or $\pm0.1\%$, of a particular value. The present application also comprises isotopically labeled compounds of the present application which are identical to those recited herein but have one or more atoms replaced by an atom having an atomic mass or mass number different from the atomic mass or mass number generally found in nature. Examples of isotopes that can be incorporated into the compounds of the present application include isotopes of hydrogen, carbon, nitrogen, oxygen, phosphorus, sulfur, fluorine, iodine and chlorine, such as $^{2}H$, $^{3}H$, $^{11}C$, $^{13}C$, $^{14}C$, $^{13}N$, $^{15}N$, $^{15}O$, $^{17}O$, $^{18}O$, $^{31}P$, $^{32}P$, $^{35}S$, $^{18}F$, $^{123}I$, $^{125}I$, and $^{36}Cl$.

Certain isotopically labeled compounds disclosed herein (e.g., those labeled with $^{3}H$ and $^{14}C$) can be used to analyze compounds and/or substrate tissue distribution. Tritiated (i.e., $^{3}H$) and carbon-14 (i.e., $^{14}C$) isotopes are particularly preferred for their ease of preparation and detectability. Positron emitting isotopes, such as $^{15}O$, $^{13}N$, $^{11}C$, and $^{18}F$, can be used in positron emission tomography (PET) studies to determine substrate occupancy. Isotopically labeled compounds of the present application can generally be prepared by following procedures analogous to those disclosed in the schemes and/or examples below while substituting a non-isotopically labeled reagent with an isotopically labeled reagent.

Furthermore, substitution with heavier isotopes such as deuterium (*i.e.*, $^{2}H$) may provide certain therapeutic advantages (*e.g.,* increased *in vivo* half-life or reduced dosage requirement) resulting from greater metabolic stability and hence may be preferred in some circumstances in which deuterium substitution may be partial or complete, wherein partial deuterium substitution refers to substitution of at least one hydrogen with at least one deuterium.

The compound disclosed herein can be asymmetrical, for example, having one or more stereoisomers. Unless otherwise stated, all stereoisomers include, for example, enantiomers and diastereoisomers. The compound with asymmetrical carbon atoms disclosed herein can be separated in an optically pure form or in a racemic form. The optically

pure form can be separated from a racemic mixture or can be synthesized using a chiral raw material or a chiral reagent.

The pharmaceutical composition of the present application can be prepared by combining the compound of the present application with a suitable pharmaceutically acceptable excipient, and can be formulated, for example, into a solid, semisolid, liquid or gaseous formulation such as tablet, pill, capsule, powder, granule, ointment, emulsion, suspension, suppository, injection, inhalant, gel, microsphere, and aerosol.

Typical routes of administration of the compound or the pharmaceutically acceptable salt thereof or the pharmaceutical composition thereof of the present application include, but are not limited to, oral, rectal, local, inhalation, parenteral, sublingual, intravaginal, intranasal, intraocular, intraperitoneal, intramuscular, subcutaneous and intravenous administration.

The pharmaceutical composition of the present application can be manufactured using methods well known in the art, such as conventional mixing, dissolving, granulating, dragee-making, levigating, emulsifying and lyophilizing.

In some embodiments, the pharmaceutical composition is in an oral form. For oral administration, the pharmaceutical composition can be formulated by mixing the active compounds with pharmaceutically acceptable excipients well known in the art. These excipients enable the compounds of the present application to be formulated into tablets, pills, pastilles, dragees, capsules, liquids, gels, slurries, suspensions, and the like for oral administration to a patient.

A solid oral composition can be prepared by conventional mixing, filling or tableting. For example, it can be obtained by the following method: mixing the active compounds with solid excipients, optionally grinding the resulting mixture, adding additional suitable excipients if desired, and processing the mixture into granules to get the core parts of tablets or dragees. Suitable excipients include, but are not limited to: binders, diluents, disintegrants, lubricants, glidants, sweeteners or flavoring agents, and the like.

The pharmaceutical composition may also be suitable for parenteral administration, such as a sterile solution, a suspension, or a lyophilized product in a suitable unit dosage form.

In all of the administration methods of the compound of general formula I described herein, the daily dose administered is from 0.01 to 200 mg/kg body weight, given in individual or separated doses.

The compounds disclosed herein can be prepared using a variety of synthetic methods well known to those skilled in the art, including the specific embodiments listed below, embodiments formed by combinations thereof with other chemical synthetic methods, and equivalents thereof known to those skilled in the art. The preferred embodiments include, but are not limited to, the examples of the present application.

The chemical reactions of the embodiments of the present application are carried out in a proper solvent that should be suitable for the chemical changes in the present application and the reagents and materials required. In order to obtain the compounds of the present application, it is sometimes necessary for those skilled in the art to modify or select a synthesis procedure or a reaction process based on the existing embodiments.

An important consideration in synthetic route planning in the art is the selection of suitable protecting groups for reactive functional groups (e.g., amino in the present application). For example, reference may be made to Greene's Protective Groups in Organic Synthesis (4th Ed.) Hoboken, New Jersey: John Wiley & Sons, Inc.

In some embodiments, the compound of formula I disclosed herein can be prepared by one skilled in the art of organic synthesis through the following routes:

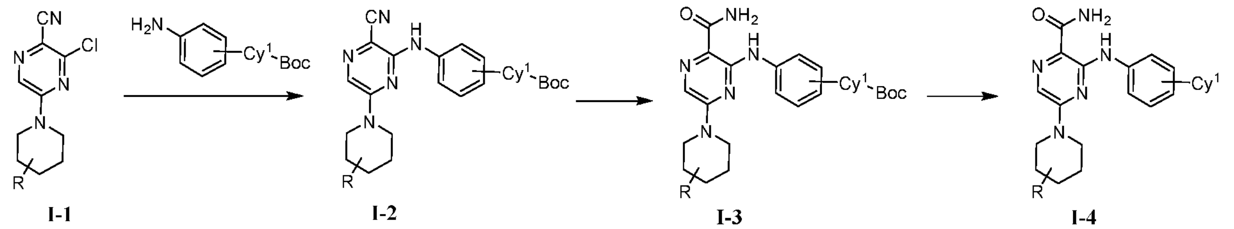

I-1 I-2 I-3 I-4

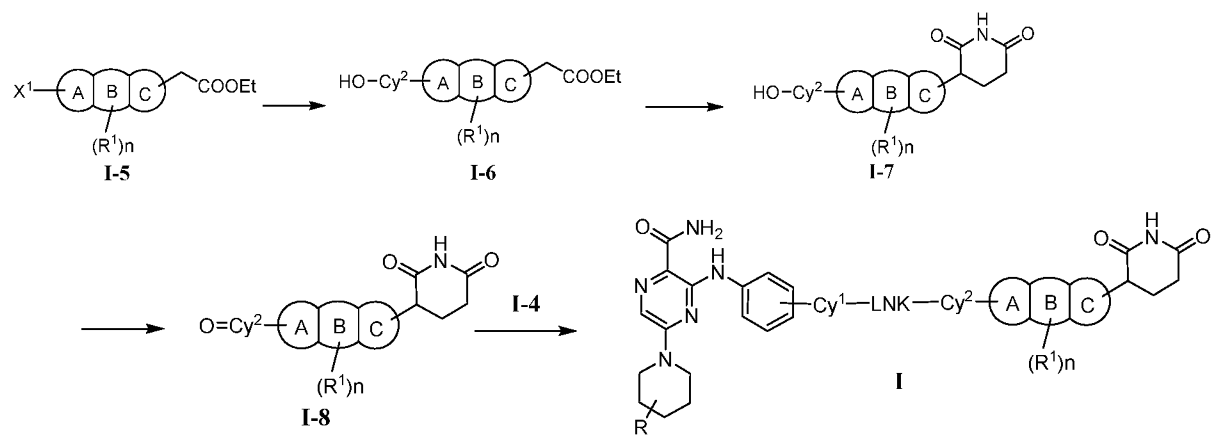

I-5 I-6 I-7

I-4

I-8 I

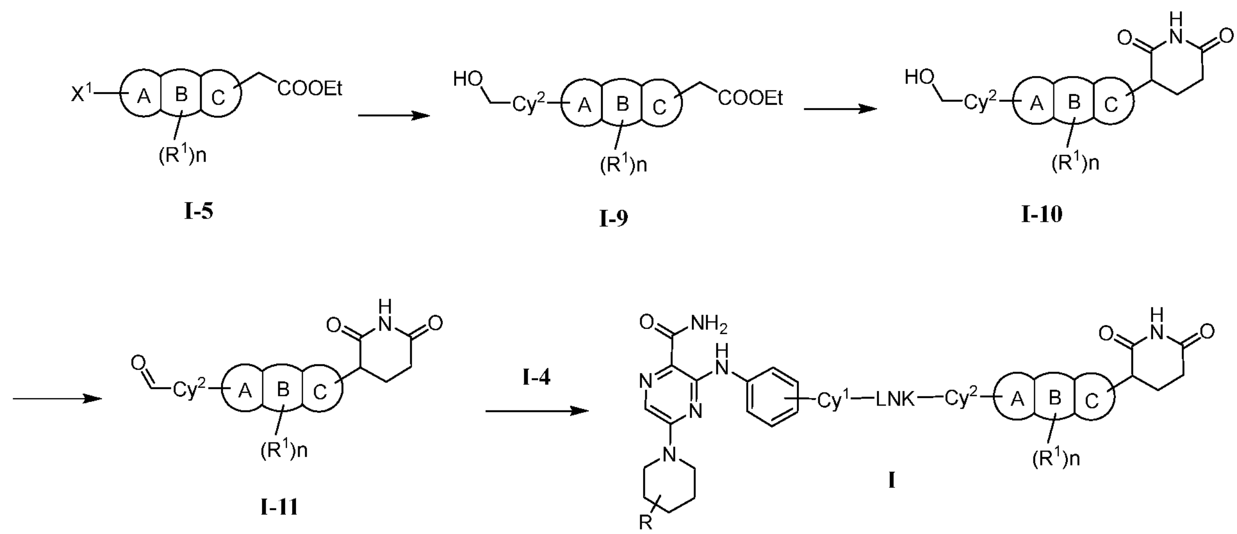

I-5 I-9 I-10

I-4

I-11 I

wherein $X^1$ is selected from the group consisting of halogens.

A compound of general formula **(I-1)** is subjected to a coupling reaction to give a compound of general formula **(I-2),** the compound of general formula **(I-2)** is subjected to a hydrolysis reaction to give a compound of general formula **(I-3),** and then a protecting group is removed to give a compound of general formula **(I-4).**

A compound of general formula **(I-5)** is subjected to a coupling reaction with hydroxy-substituted $Cy^2$ to give a compound of general formula **(I-6),** and the compound of general formula **(I-6)** is subjected to an addition reaction with acrylamide and then an amine-ester exchange reaction to give a compound of general formula **(I-7).** The compound of general formula **(I-7)** is subjected to an oxidation reaction to give a compound of general formula **(I-8).** The compound of general formula **(I-4)** and the compound of general formula **(I-8)** are subjected to a reductive amination reaction to give a compound of general formula **I.**

The compound of general formula **(I-5)** is subjected to a coupling reaction with hydroxymethyl-substituted $Cy^2$ to give a compound of general formula **(I-9),** and the compound of general formula **(I-9)** is subjected to an addition reaction with acrylamide and then an amine-ester exchange reaction to give a compound of general formula **(I-10).** The compound of general formula **(I-10)** is subjected to an oxidation reaction to give a compound of general formula **(I-11).** The compound of general formula **(I-4)** and the compound of general formula **(I-11)** are subjected to a reductive amination reaction to give a compound of general formula **I.**

The following abbreviations are used in this application:
Boc represents *tert*-butyloxycarbonyl; Et represents ethyl; EA represents ethyl acetate; DMSO represents dimethyl sulfoxide; DMF represents *N*,*N*-dimethylformamide; BINAP represents 2,2'-bis(diphenylphosphino)-1,1'-binaphthyl; DCM represents dichloromethane; $Pd_2(dba)_3$ represents tris(dibenzylideneacetone)dipalladium; THF represents tetrahydrofuran; MeOH represents methanol; PE represents petroleum ether; IBX represents 2-iodoxybenzoic acid; DIPEA represents *N*,*N*'-diisopropylethylamine; DIBAL-H represents diisobutylaluminum hydride; NIS represents N-iodosuccinimide; NBS represents N-bromosuccinimide; Tf represents $-OSO_2CF_3$; $H_2O_2$ represents hydrogen peroxide; $Pd(OAc)_2$ represents palladium acetate; DMA represents *N*,*N*-dimethylacetamide; TBSCl represents *tert*-butyldimethylsilyl chloride; TFA represents trifluoroacetic acid; $PdCl_2(dppf)$ represents [1,1'-bis(diphenylphosphino)ferrocene]palladium dichloride; Ruphos represents 2-dicyclohexylphosphino-2',6'-diisopropoxybiphenyl.

All patents, patent applications and other identified publications are explicitly incorporated herein by reference for the purpose of description and disclosure. These publications are provided solely because they were disclosed prior to the filing date of the present application. All statements as to the dates of these documents or descriptions as to the contents of these documents are based on the information available to the applicant and do not constitute any admission as to the correctness of the dates or the content of these documents. Moreover, in any country or region, any reference to these publications herein is not to be construed as an admission that the publications form part of the commonly recognized knowledge in the art.

For clarity, the present application is further described with the following examples, which are, however, not intended to limit the scope of the present application. All reagents used in the present application are commercially available and can be used without further purification.

## DETAILED DESCRIPTION

**Example 1:** Synthesis of Compound **1**

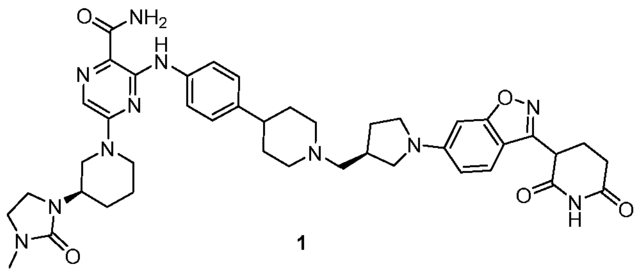

**1**

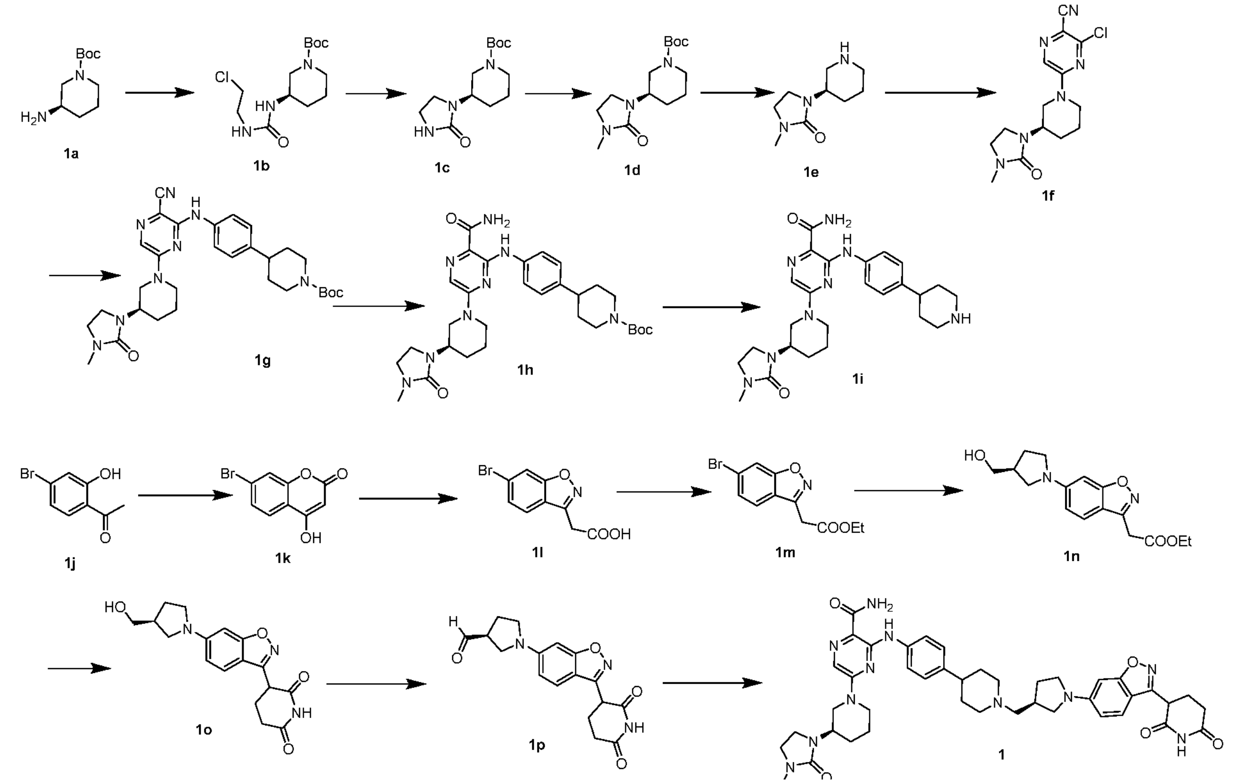

1a 1b 1c 1d 1e 1f 1g 1h 1i 1j 1k 1l 1m 1n 1o 1p 1

Step 1: Preparation of intermediate **1b**

Intermediate **1a** (25 g), triethylamine (25.3 g, 34.8 mL), and dichloromethane (250 mL) were added to a reaction flask in sequence. 1-Chloro-2-isocyanate (15.81 g) was slowly added dropwise, and after the dropwise addition was carried out for 20 min and completed, the mixture was reacted at room temperature for 4 h. 500 mL of water was added to the reaction system, the organic phase was separated, and the aqueous phase was extracted 2 times with 200 mL of dichloromethane. The organic phases were combined, dried over sodium sulfate, and filtered. The filtrate was distilled under reduced pressure to remove the solvent, and the crude product was separated by silica gel column chromatography (eluent: EA) to give the target intermediate **1b** (40.8 g).

$^1$H NMR (500 MHz, DMSO-$d_6$) $\delta$ 6.11 (q, $J$ = 7.1, 6.5 Hz, 2H), 3.56 (s, 1H), 3.43 (dq, $J$ = 8.6, 4.5 Hz, 1H), 3.33 - 3.27 (m, 2H), 3.14 - 2.84 (m, 1H), 1.80 - 1.71 (m, 1H), 1.60 (ddq, $J$ = 12.2, 6.0, 3.5, 3.0 Hz, 1H), 1.38 (s, 9H), 1.37 - 1.24 (m, 2H).

Step 2: Preparation of intermediate **1c**

Sodium hydride (10.31 g) was slowly added to a stirred solution of intermediate **1b** (40 g) in tetrahydrofuran (300 mL) at 0 °C. After the addition was completed, the mixture was reacted overnight at room temperature. After the reaction was completed, 75 mL of water was added to the reaction solution to quench the reaction. After the mixture was vigorously stirred for 10 min, liquid separation was performed. The aqueous phase was extracted 3 times with 200 mL of dichloromethane, dried over anhydrous sodium sulfate, and concentrated by rotary evaporation. The concentrate was extracted with 300 mL of acetonitrile and 300 mL of petroleum ether, and liquid separation was performed. The acetonitrile phase was subjected to rotary evaporation to give intermediate 1c (32.2 g).

Step 3: Preparation of intermediate **1d**

Sodium hydride (5.71 g) was slowly added to a stirred solution of intermediate 1c (20 g) in tetrahydrofuran (200 mL) at 0 °C. After the mixture was stirred for 5 min, ice bath was removed. After the mixture was stirred at room temperature for 1 h, ice bath was added. Iodomethane (15.21 g, 6.70 mL) was slowly added dropwise to the reaction system, and after the dropwise addition was completed, the mixture was reacted overnight at room temperature. After the reaction was completed, 75 mL of water was added to the reaction solution to quench the reaction. After the mixture was vigorously stirred for 10 min, liquid separation was performed. The aqueous phase was extracted 3 times with 200 mL of dichloromethane, dried over anhydrous sodium sulfate, and concentrated by rotary evaporation. The concentrate was extracted

with 300 mL of acetonitrile and 300 mL of petroleum ether, and liquid separation was performed. The acetonitrile phase was subjected to rotary evaporation to give intermediate **1d** (22.5 g).

Step 4: Preparation of intermediate **1e**

Intermediate **1d** (20 g) and a solution of 4 M hydrochloric acid in dioxane (25.7 g, 176 mL, 705 mmol) were added to a single-necked flask in sequence, and the mixture was reacted at room temperature. After the reaction was completed, the solvent was removed by evaporation at reduced pressure to give intermediate **1e** (15.5 g).

Step 5: Preparation of intermediate **1f**

3,5-Dichloropyrazine-2-carbonitrile (11.82 g) was added to a stirred solution of intermediate **1e** (15 g) and *N*,*N*-diisopropylethylamine (35.1 g, 47.5 mL) in DMF (300 mL) at 0 °C. Ice bath was removed after 15 min, and the mixture was stirred at room temperature overnight. 300 mL of ethyl acetate and 300 mL of water were added to the reaction solution. The organic phase was separated, extracted 2 times with 100 mL of ethyl acetate, washed with 200 mL of saturated brine, dried over anhydrous sodium sulfate, filtered, and concentrated by evaporation at reduced pressure to remove the solvent, and the crude product was separated by silica gel column chromatography (eluent: dichloromethane/methanol = 100:1, v/v) to give the target intermediate **1f** (14.5 g).

MS(ESI, $[M+H]^+$) *m/z*: 321.2.

$^1H$ NMR (500 MHz, DMSO-$d_6$) $\delta$ 7.94 (s, 1H), 3.30 (dtd, *J* = 14.7, 7.5, 6.8, 4.9 Hz, 2H), 3.25 - 3.22 (m, 1H), 2.89 (s, 4H), 2.73 (s, 3H), 2.65 (s, 2H), 1.92 - 1.69 (m, 3H), 1.62 - 1.48 (m, 1H).

Step 6: Preparation of intermediate **1g**

Intermediate **1f** (13 g), *tert*-butyl 4-(4-aminophenyl)piperidine-1-carboxylate (9.61 g), cesium carbonate (34.0 g), BINAP (2.166 g), palladium acetate (0.781 g), and 1,4-dioxane (200 mL) were added to a single-necked flask in sequence, and the mixture was heated to 100 °C and reacted under $N_2$ atmosphere. After the reaction was completed, the reaction solution was cooled to room temperature and filtered, and the filter cake was washed with 150 mL of dichloromethane. The filtrate was concentrated by evaporation at reduced pressure to remove the solvent, and the crude product was separated by silica gel column chromatography (eluent: dichloromethane/methanol = 100:1, v/v) to give the target intermediate **1g** (14.2 g).

MS(ESI, $[M+H]^+$) *m/z*: 561.5

$^1H$ NMR (500 MHz, DMSO-$d_6$) $\delta$ 8.97 (s, 1H), 7.82 (s, 1H), 7.48 - 7.43 (m, 2H), 7.16 - 7.10 (m, 2H), 4.37 - 4.19 (m, 2H), 4.07 (d, *J* = 13.1 Hz, 2H), 3.64 - 3.59 (m, 2H), 3.42 (dq, *J* = 11.6, 3.0 Hz, 3H), 3.34 - 3.29 (m, 1H), 3.26 - 3.23 (m, 2H), 2.94 (d, *J* = 12.9 Hz, 1H), 2.71 (s, 3H), 2.62 (ddt, *J* = 15.5, 12.0, 3.3 Hz, 1H), 1.82 - 1.71 (m, 5H), 1.58 - 1.43 (m, 3H), 1.42 (s, 9H).

Step 7: Preparation of intermediate **1h**

Intermediate **1g** (8 g), DMSO (25 mL), and cesium carbonate (4.08 g) were added to a single-necked flask in sequence. Hydrogen peroxide (21.32 g, 19.21 mL) was added under an ice bath. After the mixture was stirred for 5 min, the ice bath was removed, and the mixture was reacted at room temperature. After the reaction was completed, 300 mL of a saturated sodium sulfite solution was added to the residue to quench the reaction. The color was not changed as detected using a potassium iodide starch reagent, and then 300 mL of ethyl acetate was added for extraction. 100 mL of ethyl acetate was then added for 3 times of extraction. The organic phases were combined, dried over anhydrous sodium sulfate, filtered, and concentrated by evaporation at reduced pressure to remove the solvent, and the crude product was separated by silica gel column chromatography (eluent: dichloromethane/methanol = 100:1, v/v) to give the target intermediate **1h** (7.22 g).

MS(ESI, $[M+H]^+$) *m/z*: 579.51.

$^1H$ NMR (500 MHz, DMSO-$d_6$) $\delta$11.19 (s, 1H), 7.75 (d, J = 2.8 Hz, 1H), 7.66 (s, 1H), 7.55 - 7.46 (m, 2H), 7.32 (d, J = 2.8 Hz, 1H), 7.15 (d, J = 8.6 Hz, 2H), 4.36 (d, J = 12.4 Hz, 1H), 4.27 (d, J = 13.4 Hz, 1H), 4.06 (d, J = 12.8 Hz, 2H), 3.61 (tt, J = 11.0, 4.0 Hz, 1H), 3.25 (dd, J = 9.5, 7.2 Hz, 2H), 3.06 - 2.91 (m, 2H), 2.70 (s, 3H), 2.62 (tt, J = 12.1, 3.6 Hz, 1H), 2.54 (s, 4H), 1.82 (dt, J = 14.8, 3.5 Hz, 2H), 1.79 - 1.70 (m, 3H), 1.54 (s, 1H), 1.45 (dq, J = 12.9, 4.2 Hz, 2H), 1.42 (s, 9H).

Step 8: Preparation of intermediate **1i**

Intermediate **1h** (3 g), dichloromethane (30 mL), and trifluoroacetic acid (17.06 g, 11.53 mL) were added to a single-necked flask in sequence, and the mixture was reacted at room temperature. After the reaction was completed, the solvent was removed by evaporation at reduced pressure to give a product of trifluoroacetate salt. 50 mL of dichloromethane was added to dissolve the crude product, and a saturated aqueous sodium bicarbonate solution was slowly added. After the system was adjusted to weak alkalinity, liquid separation was performed. The organic phase was concentrated by evaporation at reduced pressure to remove the solvent, thus giving intermediate **1i** (2.4 g).

MS(ESI, $[M+H]^+$) *m/z*: 479.44
$^1$H NMR (500 MHz, DMSO-$d_6$) $\delta$ 11.25 (s, 1H), 7.77 (d, *J* = 2.8 Hz, 1H), 7.67 (s, 1H), 7.54 (d, *J* = 8.3 Hz, 2H), 7.34 (d, *J* = 2.8 Hz, 1H), 7.19 - 7.12 (m, 2H), 4.32 (dd, *J* = 33.8, 12.7 Hz, 2H), 3.62 (tt, *J* = 11.0, 4.0 Hz, 2H), 3.39 - 3.31 (m, 3H), 3.30 - 3.23 (m, 3H), 3.05 (t, *J* = 11.7 Hz, 1H), 3.00 - 2.88 (m, 3H), 2.72 (s, 3H), 1.95 - 1.86 (m, 2H), 1.85 - 1.79 (m, 2H), 1.79 - 1.64 (m, 3H), 1.57 (dtd, *J* = 16.9, 8.4, 3.7 Hz, 1H).

Step 9: Preparation of intermediate **1k**

A solution of intermediate **1j** (30 g) in toluene (100 mL) was slowly added dropwise to a solution of sodium hydride (27.9 g) in toluene (50 mL) at 0 °C. After the dropwise addition was completed, the mixture was stirred at 0 °C for 15 min. Dimethyl carbonate (126 g) was added dropwise to the reaction solution. After the dropwise addition was completed and the mixture was stirred at 0 °C for 15 min, the mixture was heated to 100 °C and reacted for 2 h. The reaction solution was washed once with water (500 mL). The organic layer was discarded, and the aqueous layer was adjusted to pH = 2 to 3 with diluted hydrochloric acid (3 mol/L) at a low temperature, and a large amount of solid was precipitated. After filtration under vacuum and drying, 30.63 g of intermediate **1k** was obtained.

MS (ESI, $[M-H]^-$) *m/z*: 239.0
$^1$H NMR (500 MHz, DMSO-$d_6$) $\delta$ 12.74 (s, 1H), 7.77 - 7.65 (m, 2H), 7.54 (dd, *J* = 8.4, 1.9 Hz, 1H), 5.63 (s, 1H).

Step 10: Preparation of intermediate **1l**

Intermediate **1k** (29.5 g), hydroxylamine hydrochloride (15.85 g), sodium ethoxide (15.52 g), and ethanol (150 mL) were added to a reaction flask in sequence, and the mixture was heated to 85 °C for 2 h under $N_2$ atmosphere. The reaction solution was subjected to rotary evaporation to dryness, 500 mL of a saturated sodium carbonate solution was added to the residue, and the mixture was extracted with DCM (300 mL $\times$ 2). The organic layer was discarded. The pH of the aqueous layer was adjusted to < 3 with diluted hydrochloric acid (3 mol/L). A large amount of solid was precipitated. Filter under vacuum was performed, and the resulting filter cake was washed with water and dried under atmospheric pressure to give 26.53 g of intermediate **1l**.

MS (ESI, $[M-H]^-$) *m/z:* 254.0.

$^1$H NMR (500 MHz, DMSO-$d_6$) $\delta$ 12.95 (s, 1H), 8.12 (d, *J* = 1.5 Hz, 1H), 7.82 (d, *J* = 8.4 Hz, 1H), 7.59 (dd, *J* = 8.4, 1.6 Hz, 1H), 4.12 (s, 2H).

Step 11: Preparation of intermediate **1m**

Intermediate **1l** (26.5 g), and ethanol (530 mL) were added to a reaction flask in sequence. After concentrated sulfuric acid (49.9 g, 27.1 mL, 508 mmol) was slowly added dropwise, the mixture was heated to 85 °C and reacted for 4 h. The reaction solution was subjected to rotary evaporation to dryness. After ice water was added to the residue, an NaOH solution was added dropwise at a low temperature to adjust the pH to be approximately equal to 9, and then the mixture was extracted 2 times with 300 mL of ethyl acetate. The organic layers were combined, dried over anhydrous sodium sulfate, and filtered under vacuum. The filtrate was subjected to rotary evaporation to dryness to give 24.87 g of intermediate **1m.**

MS (ESI, $[M-H]^-$) *m/z*: 282.0
$^1$H NMR (500 MHz, DMSO-$d_6$) $\delta$ 8.13 (d, *J* = 1.5 Hz, 1H), 7.82 (d, *J* = 8.4 Hz, 1H), 7.60 (dd, *J* = 8.4, 1.5 Hz, 1H), 4.23 (s, 2H), 4.15 (q, *J* = 7.1 Hz, 2H), 1.19 (t, *J* = 7.1 Hz, 3H).

Step 12: Preparation of intermediate **1n**

Intermediate **1m** (2 g) and (*S*)-pyrrolidine-3-methanol (2.087 g) were added in sequence to a reaction flask.

$Pd_2(dba)_3$ (1.260 g) and potassium phosphate (5.84 g) were then added, and finally toluene (60 mL) was added. The mixture was heated at 80 °C for 4 h under $N_2$ atmosphere. The reaction solution was washed twice with 100 mL of water. The organic layer was dried over anhydrous sodium sulfate and filtered under vacuum, and the resulting filtrate was concentrated, separated and purified by silica gel column chromatography to give intermediate **1n** (0.35 g).

MS (ESI, $[M+H]^+$) *m/z:* 305.20.

Step 13: Preparation of intermediate **1o**

Intermediate **1n** (360 mg) and THF (30 mL) were first added to a reaction flask, acrylamide (101 mg) and potassium *tert*-butoxide (199 mg) were added at 0 °C, and the mixture was slowly warmed to 10 °C under $N_2$ atmosphere and stirred. After the reaction was completed, 100 mL of a saturated ammonium chloride solution was added to the reaction solution at a low temperature, and the mixture was extracted twice with EA (50 mL). The organic layer was dried over anhydrous sodium sulfate and filtered, the filtrate was subjected to rotary evaporation to dryness and purified by silica gel column chromatography (eluent: DCM:MeOH = 95:5, v/v) to give 245 mg of intermediate **1o**.
MS (ESI, $[M+H]^+$) *m/z*: 330.1

Step 14: Preparation of intermediate **1p**

Intermediate **1o** (330 mg) and DCM (30 mL) were added to a reaction flask in sequence, Dess-Martin reagent (1.224 g) was added with stirring, and the mixture was reacted at room temperature under $N_2$ atmosphere. After the reaction was completed, an aqueous NazSzOs solution (30 mL) was added at a low temperature to quench the reaction solution, and the mixture was extracted with an aqueous $NaHCO_3$ solution (30 mL). The organic layer was dried over anhydrous sodium sulfate and filtered under vacuum, and the resulting filtrate was subjected to rotary evaporation to dryness to give 290 mg of intermediate **1p**, which was directly used in the next step.

Step 15: Preparation of compound **1**

Intermediate **1i** (150 mg), intermediate **1p** (103 mg), DCM (10 mL), and glacial acetic acid (31.4 mg, 0.030 mL) were added to a reaction flask in sequence. After the mixture was stirred at room temperature for 20 min, sodium cyanoborohydride (59.1 mg) was added, and the mixture was stirred at room temperature. After the reaction was completed, 20 mL of a saturated $NaHCO_3$ solution was added to the reaction solution, and the mixture was extracted twice with 20 mL of DCM-MeOH (10:1). The extracts were combined, dried over anhydrous sodium sulfate, and filtered under vacuum, and the resulting filtrate was subjected to rotary evaporation to dryness and purified by $C_{18}$ reversed-phase column (10 nM aqueous ammonium acetate solution-acetonitrile = 50%:50%, v/v) to give 55 mg of compound **1.**

Q-TOF (ESI, $[M+H]^+$) *m/z*: 790.4146
$^1H$ NMR (500 MHz, DMSO-$d_6$) δ 11.18 (s, 1H), 11.02 (s, 1H), 7.75 (d, *J* = 2.8 Hz, 1H), 7.66 (s, 1H), 7.52 (dd, *J* = 13.9, 8.4 Hz, 3H), 7.35 - 7.30 (m, 1H), 7.17 (d, *J* = 8.1 Hz, 2H), 6.66 (dd, *J* = 8.9, 1.8 Hz, 1H), 6.58 (d, *J* = 1.8 Hz, 1H), 4.45 - 4.34 (m, 2H), 4.28 (d, *J* = 13.3 Hz, 1H), 3.62 (dt, *J* = 11.1, 7.0 Hz, 1H), 3.48 (t, *J* = 8.5 Hz, 1H), 3.41 (d, *J* = 4.7 Hz, 1H), 3.26 (dd, *J* = 10.3, 7.0 Hz, 3H), 3.12 - 2.92 (m, 5H), 2.73 (s, 4H), 2.60 (ddd, *J* = 21.9, 12.8, 5.5 Hz, 2H), 2.41 (ddd, *J* = 25.0, 11.8, 4.7 Hz, 4H), 2.20 - 2.10 (m, 2H), 1.87 - 1.51 (m, 10H).

**Example 2:** Synthesis of Compound **2**

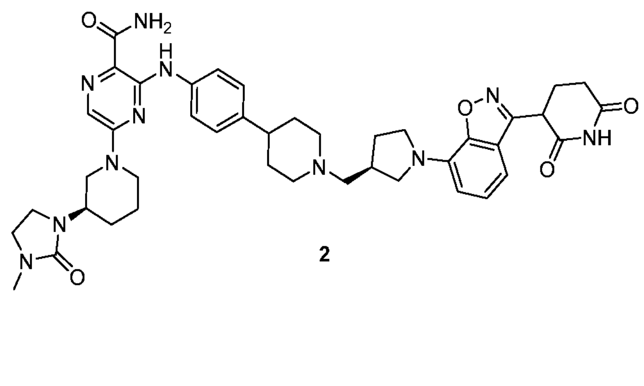

**2**

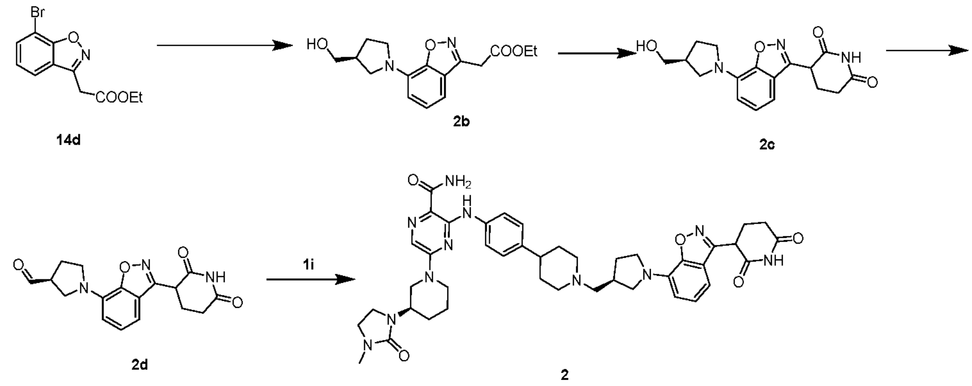


14d 2b 2c 2d 2

Step 1: Preparation of intermediate **2b**

Intermediate **14d** (2 g) and (*S*)-pyrrolidine-3-methanol (1.424 g) were added in sequence to a reaction flask. $Pd_2(dba)_3$ (1.289 g) and potassium phosphate (5.98 g) were then added, and finally toluene (50 mL) was added. The mixture was reacted at 80 °C under $N_2$ atmosphere. After the reaction was completed, the reaction solution was washed twice with 50 mL of water. The organic layer was dried over anhydrous sodium sulfate and filtered under vacuum, and the filtrate was subjected to rotary evaporation to dryness, purified by silica gel column (PE-EA = 95:5, v/v, 1500 mL), and further purified by $C_{18}$ reversed-phase column (10 nM aqueous ammonium acetate solution-acetonitrile = 50%:50%, v/v) to give 480 mg of intermediate **2b.**

MS (ESI, $[M+H]^+$) *m/z*: 305.1.

$^1H$ NMR (500 MHz, DMSO-$d_6$) $\delta$ 7.17 (t, *J* = 7.8 Hz, 1H), 6.96 (dd, *J* = 7.9, 0.8 Hz, 1H), 6.58 (dd, *J* = 7.8, 0.9 Hz, 1H), 4.73 (t, *J* = 5.2 Hz, 1H), 4.17 - 4.09 (m, 4H), 3.67 (dd, *J* = 9.7, 7.5 Hz, 1H), 3.60 (ddd, *J* = 9.5, 8.1, 5.0 Hz, 1H), 3.56 - 3.35 (m, 4H), 2.45 (dq, *J* = 14.0, 7.0 Hz, 1H), 2.10 - 2.01 (m, 1H), 1.76 (dq, *J* = 12.2, 7.5 Hz, 1H), 1.18 (t, *J* = 7.1 Hz, 3H).

Step 2: Preparation of intermediate **2c**

Intermediate **2b** (450 mg) and THF (40 mL) were first added to a reaction flask, acrylamide (126 mg) and potassium *tert*-butoxide (249 mg) were added at 0 °C, and the mixture was slowly warmed to 10 °C under $N_2$ atmosphere and stirred. After the reaction was completed, 100 mL of a saturated ammonium chloride solution was added to the reaction solution at a low temperature, and the mixture was extracted twice with EA (50 mL). The organic layer was dried over anhydrous sodium sulfate and filtered under vacuum, the filtrate was subjected to rotary evaporation to dryness and purified by silica gel column (DCM:MeOH = 95:5, v/v) to give 450 mg of intermediate **2c.**

MS (ESI, $[M+H]^+$) *m/z:* 330.1.

Step 3: Preparation of intermediate **2d**

Intermediate **2c** (440 mg) and DCM (30 mL) were added to a reaction flask in sequence, Dess-Martin reagent (1.7 g) was added with stirring, and the mixture was reacted at room temperature under $N_2$ atmosphere. After the reaction was completed, an aqueous NazSzOs solution (30 mL) was added at a low temperature to quench the reaction, and the organic layer separated by extraction was washed with an aqueous $NaHCO_3$ solution (30 mL). The separated organic layer was dried over anhydrous sodium sulfate and filtered under vacuum, and the resulting filtrate was subjected to rotary evaporation to dryness to give 342 mg of intermediate **2d,** which was directly used in the next step.

Step 4: Preparation of compound **2**

Intermediate **1i** (250 mg), intermediate **2d** (171 mg), DCM (10 mL), and glacial acetic acid (31.4 mg, 0.030 mL) were added to a reaction flask in sequence. After the mixture was stirred at room temperature for 20 min, sodium cyanoborohydride (197 mg) was added, and the mixture was stirred at room temperature. After the reaction was completed, 20 mL of a saturated $NaHCO_3$ solution was added to the reaction solution, and the mixture was extracted twice with 20 mL of DCM-MeOH (10:1). The extracts were combined, dried over anhydrous sodium sulfate, and filtered under vacuum, and the resulting filtrate was subjected to rotary evaporation to dryness and purified by $C_{18}$ reversed-phase column (10 nM aqueous ammonium acetate solution-acetonitrile = 50%:50%, v/v) to give 60 mg of compound **2.**

Q-TOF(ESI, $[M+H]^+$) *m/z*: 790.4157.

$^1H$ NMR (500 MHz, DMSO-$d_6$) $\delta$ 11.17 (s, 1H), 11.07 (s, 1H), 7.74 (d, *J* = 2.8 Hz, 1H), 7.65 (s, 1H), 7.52 - 7.47

(m, 2H), 7.32 (d, *J* = 2.8 Hz, 1H), 7.20 - 7.12 (m, 3H), 6.97 (d, *J* = 7.9 Hz, 1H), 6.59 (d, *J* = 7.7 Hz, 1H), 4.53 (dd, *J* = 11.6, 5.0 Hz, 1H), 4.37 (d, *J* = 12.6 Hz, 1H), 4.28 (d, *J* = 13.3 Hz, 1H), 3.74 (ddd, *J* = 9.9, 7.2, 2.6 Hz, 1H), 3.67 - 3.59 (m, 2H), 3.55 (qd, *J* = 7.4, 3.7 Hz, 1H), 3.40 - 3.32 (m, 3H), 3.30 - 3.23 (m, 3H), 3.07 (d, *J* = 10.9 Hz, 1H), 3.04 - 2.90 (m, 3H), 2.77 (td, *J* = 11.8, 6.0 Hz, 1H), 2.67 - 2.55 (m, 2H), 2.48 - 2.34 (m, 4H), 2.21 - 1.97 (m, 4H), 1.85 - 1.52 (m, 9H).

$^{13}$C NMR (126 MHz, DMSO) δ 173.58, 171.99, 169.70, 160.77, 157.13, 154.01, 153.30, 150.98, 140.44, 137.77, 134.30, 127.46, 125.70, 122.03, 120.33, 118.64, 114.69, 111.16, 108.46, 62.25, 55.11, 54.25, 54.10, 49.11, 48.88, 47.20, 45.46, 44.86, 41.79, 36.13, 33.90, 31.55, 31.41, 29.70, 28.25, 24.50, 23.23.

**Example 3:** Synthesis of Compound **3**

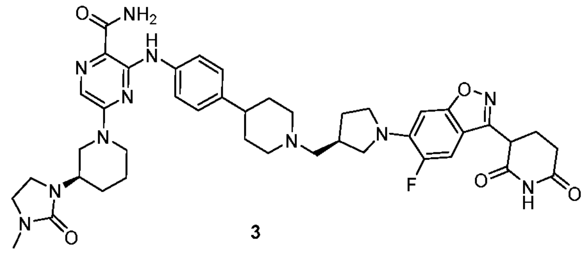

**3**

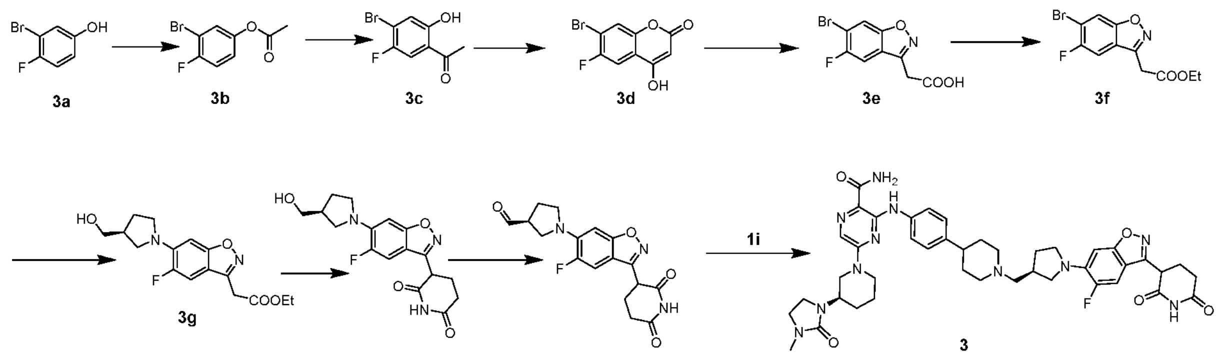


**3a** **3b** **3c** **3d** **3e** **3f** **3g** **3h** **3i** **3**

Step 1: Preparation of intermediate **3b**

In a single-necked flask, intermediate **3a** (20 g) was dissolved in dichloromethane (200 mL). Triethylamine (15.89 g, 21.89 mL) was added at 0 °C, and acetyl chloride (12.33 g) was slowly added dropwise. After the addition was completed, the mixture was reacted at room temperature for 1 h. After the reaction was completed, 300 mL of dichloromethane was added to the system for dilution, and a 3 M aqueous hydrochloric acid solution was then added to adjust the pH of the system to weak acidity. Extraction and liquid separation were performed, and the organic phase was collected. The pH of the organic phase was adjusted to weak alkalinity with a saturated aqueous sodium bicarbonate solution. Extraction and liquid separation were performed, and the organic phase was collected. The organic phase was dried over anhydrous sodium sulfate, filtered and concentrated by evaporation at reduced pressure to remove the solvent, thus giving intermediate **3b** (28.5 g).

$^1$H NMR (500 MHz, DMSO-$d_6$) δ 7.60 (dd, *J* = 5.9, 2.7 Hz, 1H), 7.44 (t, *J* = 8.7 Hz, 1H), 7.23 (ddd, *J* = 8.9, 4.1, 2.8 Hz, 1H), 2.26 (s, 3H).

Step 2: Preparation of intermediate **3c**

Intermediate **3b** (8.61 g) and aluminum trichloride (8.92 g) were added to a single-necked flask in sequence, and the mixture was heated to 170 °C and reacted for 2 h. The reaction solution was cooled to room temperature, 50 mL of dichloromethane was added, and about 500 mL of 3 M hydrochloric acid was slowly added. Liquid separation was performed, the organic phase was collected, and the aqueous phase was then extracted 2 times with 250 mL of dichloromethane. The organic phases were combined, dried over anhydrous sodium sulfate, filtered and concentrated by evaporation at reduced pressure to remove the solvent, thus giving intermediate **3c** (11.8 g).

MS(ESI, $[M-H]^+$) $m/z$: 231.0

$^1H$ NMR (500 MHz, DMSO-$d_6$) $\delta$ 11.64 (s, 1H), 7.79 (d, $J$ = 9.3 Hz, 1H), 7.33 (d, $J$ = 5.8 Hz, 1H), 2.62 (s, 3H).

Step 3: Preparation of intermediate **3d**

Intermediate **3c** (10 g), diethyl carbonate (25.3 g), and toluene (100 mL) were added to a single-necked flask in sequence. The reaction solution was cooled to 0 °C and sodium hydride (8.58 g) was added in portions. The mixture was first heated to 90 °C and then heated to 120 °C and reacted for 5 h. After the reaction was completed, the reaction solution was cooled to room temperature and slowly poured into 1 L of stirred ice water. The mixture was extracted with 500 mL of ethyl acetate, and the organic phase was discarded. The aqueous phase was adjusted to pH = 3 with 3 N hydrochloric acid, the mixture was extracted 3 times with 300 mL of ethyl acetate, and the organic phases were combined. The organic phase was dried over anhydrous sodium sulfate, filtered and concentrated by evaporation at reduced pressure to remove the solvent, thus giving intermediate **3d** (9.8 g).

MS(ESI, $[M-H]^+$) $m/z$: 256.95.

$^1H$ NMR (500 MHz, DMSO-$d_6$) $\delta$ 12.86 (s, 1H), 7.88 (d, $J$ = 5.6 Hz, 1H), 7.67 (d, $J$ = 8.5 Hz, 1H), 5.65 (s, 1H).

Step 4: Preparation of intermediate **3e**

Intermediate **3d** (9 g), hydroxylamine hydrochloride (7.95 g), sodium ethoxide (2.224 g), and ethanol (50 mL) were added to a single-necked flask in sequence, and the mixture was heated to 85 °C and reacted overnight. The reaction solution was cooled to room temperature, 3 N hydrochloric acid was added to adjust the pH to 5, the mixture was concentrated by evaporation at reduced pressure to remove the solvent, 100 L of water was added, the mixture was cooled, and meanwhile the pH was adjusted to 3 with 3 N hydrochloric acid. The mixture was stirred for 30 min and filtered. The filter cake was collected and dried to give intermediate **3e** (8.1 g).

MS(ESI, $[M+H]^+$) $m/z$: 273.86.

$^1H$ NMR (500 MHz, DMSO-$d_6$) $\delta$ 12.97 (s, 1H), 8.30 (d, $J$ = 5.3 Hz, 1H), 7.93 (d, $J$ = 7.9 Hz, 1H), 4.12 (s, 2H).

Step 5: Preparation of intermediate **3f**

Intermediate **3e** (6 g), ethanol (40 mL), and sulfuric acid (9.27 g, 5.04 mL) were added to a single-necked flask in sequence, and the mixture was heated to 90 °C and reacted for 2 h. The reaction solution was cooled to room temperature and concentrated by evaporation at reduced pressure to remove the solvent, and 100 mL of ethyl acetate and 100 mL of water were added to the residue for dilution. A saturated aqueous sodium bicarbonate solution was added to adjust the pH to 7, and the organic phase was separated. The aqueous phase was extracted 2 times with 100 mL of ethyl acetate, and the organic phases were combined, dried over anhydrous sodium sulfate, filtered and concentrated by evaporation at reduced pressure to remove the solvent, thus giving intermediate **3f** (5.4 g).

MS(ESI, $[M+H]^+$) $m/z$: 302.21.

$^1H$ NMR (500 MHz, DMSO-$d_6$) $\delta$ 8.32 (d, $J$ = 5.3 Hz, 1H), 7.94 (d, $J$ = 8.0 Hz, 1H), 4.22 (s, 2H), 4.16 (q, $J$ = 7.1 Hz, 2H), 1.21 (t, $J$ = 7.1 Hz, 3H).

Step 6: Preparation of intermediate **3g**

Intermediate **3f** (4 g), (*S*)-pyrrolidine-3-methanol (2.68 g), palladium acetate (0.595 g), potassium phosphate (8.43 g), and 1,4-dioxane (20 mL) were added to a single-necked flask in sequence, and the mixture was heated to 100 °C and reacted under $N_2$ atmosphere overnight. The reaction solution was cooled to room temperature and concentrated by evaporation at reduced pressure to remove the solvent, and the crude product was separated by silica gel column chromatography (eluent: petroleum ether/ethyl acetate = 1:1, v/v) to give the target intermediate **3g** (0.5 g).

MS(ESI, $[M+H]^+$) $m/z$: 323.11.

$^1H$ NMR (500 MHz, DMSO-$d_6$) $\delta$ 7.45 (d, $J$ = 13.3 Hz, 1H), 6.81 (d, $J$ = 7.0 Hz, 1H), 4.73 (t, $J$ = 5.2 Hz, 1H), 4.13 (q, $J$ = 7.2 Hz, 2H), 4.03 (s, 2H), 3.52 (ddd, $J$ = 10.5, 7.4, 3.2 Hz, 1H), 3.49 - 3.38 (m, 4H), 3.27 (ddd, $J$ = 10.0, 6.7, 3.1 Hz, 1H), 2.41 (td, $J$ = 14.9, 14.1, 7.9 Hz, 1H), 2.01 (dtd, $J$ = 12.2, 7.9, 7.5, 5.3 Hz, 1H), 1.72 (dq, $J$ = 12.2, 7.7 Hz, 1H), 1.19 (t, $J$ = 7.1 Hz, 3H).

Step 7: Preparation of intermediate **3h**

Intermediate **3g** (500 mg), acrylamide (98 mg), and anhydrous tetrahydrofuran (5 mL) were added to a three-necked flask in sequence. Potassium *tert*-butoxide (233 mg) was slowly added at -15 °C, and the mixture was reacted at -15 °C for 2 h. A saturated aqueous ammonium chloride solution was added dropwise to the reaction solution to

quench the reaction, and the mixture was extracted with 50 mL of ethyl acetate. The organic phase was separated. The aqueous phase was extracted 3 times with 50 mL of ethyl acetate, the organic phases were combined, dried over anhydrous sodium sulfate, filtered and concentrated by evaporation at reduced pressure to remove the solvent, thus giving intermediate **3h** (120 mg).

MS(ESI, $[M-H]^+$) *m/z*: 345.9.

$^1H$ NMR (500 MHz, DMSO-$d_6$) $\delta$ 11.03 (s, 1H), 7.52 (d, *J* = 13.3 Hz, 1H), 6.82 (d, *J* = 6.9 Hz, 1H), 4.72 (t, *J* = 5.3 Hz, 1H), 4.41 (dd, *J* = 11.6, 5.0 Hz, 1H), 3.45 (td, *J* = 11.7, 6.2 Hz, 5H), 2.72 (ddd, *J* = 17.8, 12.3, 5.7 Hz, 1H), 2.57 (dt, *J* = 17.3, 4.1 Hz, 1H), 2.39 (tq, *J* = 13.0, 7.7, 6.9 Hz, 2H), 2.14 (dt, *J* = 13.5, 4.6 Hz, 1H), 2.00 (tq, *J* = 13.4, 8.3, 7.1 Hz, 2H), 1.77 - 1.67 (m, 1H).

Step 8: Preparation of intermediate **3i**

Intermediate **3h** (80 mg), acetonitrile (5 mL), and IBX (129 mg) were added to a single-necked flask in sequence, and the mixture was heated to 85 °C and reacted for 1 h. The reaction solution was cooled to room temperature and filtered, and the filtrate was collected. The resulting solution (containing intermediate 3i) was directly used in the next step.

Step 9: Preparation of compound **3**

Intermediate **3i** (the solution of intermediate 3i obtained in the previous step), intermediate **1i** (60 mg), and methanol (1 mL) were added to a single-necked flask in sequence, and 1 drop of acetic acid was added dropwise. After the mixture was stirred at room temperature for 1 h, sodium cyanoborohydride (23.63 mg) was added, and the mixture was reacted at room temperature for 2 h. The mixture was concentrated by evaporation at reduced pressure to remove the solvent and purified by preparative liquid chromatography. Compound **3** (25 mg) was obtained.

MS(ESI, $[M+H]^+$) *m/z:* 808.6.

$^1H$ NMR (500 MHz, DMSO-$d_6$) $\delta$ 11.18 (s, 1H), 11.03 (s, 1H), 7.75 (s, 1H), 7.66 (s, 1H), 7.51 (dd, *J* = 18.1, 10.6 Hz, 3H), 7.33 (s, 1H), 7.17 (d, *J* = 8.1 Hz, 2H), 6.84 (d, *J* = 7.0 Hz, 1H), 4.51 - 4.33 (m, 2H), 4.28 (d, *J* = 13.3 Hz, 1H), 3.60 (dd, *J* = 25.9, 10.7 Hz, 2H), 3.47 (d, *J* = 9.5 Hz, 2H), 3.26 (t, *J* = 8.1 Hz, 3H), 2.98 (dt, *J* = 32.9, 12.0 Hz, 4H), 2.73 (s, 4H), 2.57 (dt, *J* = 17.3, 4.1 Hz, 2H), 2.42 - 2.30 (m, 2H), 2.21 - 1.96 (m, 4H), 1.91 - 1.45 (m, 10H).

$^{13}C$ NMR (126 MHz, DMSO-$d_6$) $\delta$ 173.59, 172.00, 169.71, 161.59, 160.79, 156.67, 154.02, 152.29, 150.98, 148.61, 127.45, 120.32, 118.68, 114.69, 109.76, 109.50, 106.13, 93.49, 54.85, 54.21, 49.77, 49.10, 47.19, 45.46, 44.85, 39.00, 35.05, 34.96, 33.69, 31.76, 31.62, 31.55, 31.45, 30.86, 30.30, 29.71, 29.46, 28.24, 24.50, 23.01.

**Example 4:** Synthesis of Compound **4**

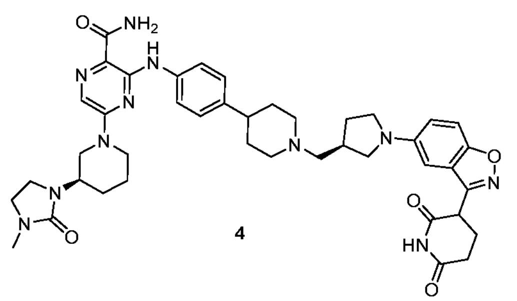

4

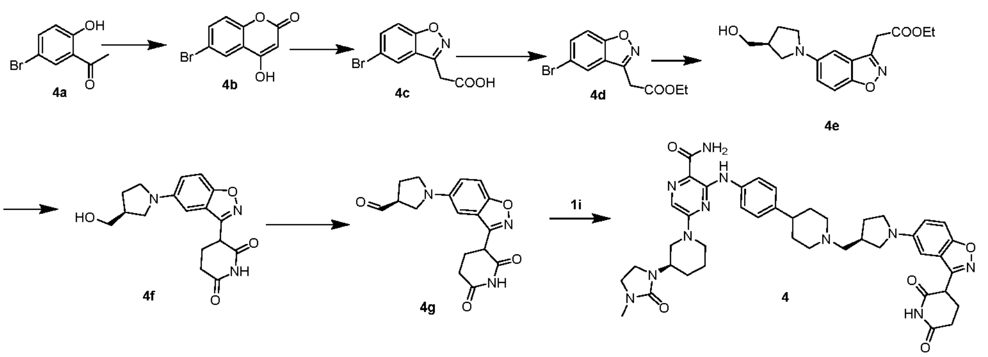


Step 1: Preparation of intermediate **4b**

**4a** (25 g), diethyl carbonate (68.7 g), and toluene (200 mL) were added to a reaction flask in sequence. The reaction solution was cooled to 0 °C and sodium hydride (23.25 g) was added in portions. The mixture was heated to 120 °C and reacted for 5 h. The reaction was stopped, the reaction solution was cooled to room temperature and slowly poured into 1.5 L of stirred ice water. The mixture was extracted with 400 mL of ethyl acetate. The aqueous phase was adjusted to pH = 3 with 3 N hydrochloric acid, the mixture was extracted 3 times with 400 mL of ethyl acetate, and the organic phases were combined. The organic phase was dried over anhydrous sodium sulfate, filtered and concentrated by evaporation at reduced pressure to remove the solvent, thus giving 25 g of intermediate **4b.**

MS(ESI, $[M-H]^+$) *m/z*: 239.0
$^1H$ NMR (500 MHz, DMSO-$d_6$) $\delta$ 12.78 (s, 1H), 7.90 (d, *J* = 2.4 Hz, 1H), 7.80 (dd, *J* = 8.8, 2.5 Hz, 1H), 7.36 (d, *J* = 8.8 Hz, 1H), 5.62 (s, 1H).

Step 2: Preparation of intermediate **4c**

**4b** (13 g), hydroxylamine hydrochloride (7.50 g), sodium methoxide (5.83 g), and ethanol (100 mL) were added to a reaction flask in sequence, and the reaction solution was heated to 85 °C and reacted for 15.5 h under $N_2$ atmosphere. The reaction solution was cooled to room temperature and concentrated by evaporation at reduced pressure to remove the solvent. After 200 mL of water was added to the residue, 3 M hydrochloric acid was added. The mixture was filtered, and the filter cake was purified by silica gel column chromatography (eluent: DCM:$CH_3OH$ = 9:1, v/v) to give 5.5 g of intermediate **4c.**

MS(ESI, $[M-H]^+$) *m/z*: 254.1.

$^1H$ NMR (500 MHz, DMSO-$d_6$) $\delta$ 12.96 (s, 1H), 8.14 (d, *J* = 2.0 Hz, 1H), 7.81 (dd, *J* = 8.9, 2.0 Hz, 1H), 7.75 (d, *J* = 8.8 Hz, 1H), 4.13 (s, 2H).

Step 3: Preparation of intermediate **4d**

**4c** (5.5 g), ethanol (50 mL), and concentrated sulfuric acid (12.64 g, 6.87 mL) were added to a reaction flask in sequence, and the mixture was heated to 90 °C and reacted for 15 h. The reaction solution was cooled to room temperature and concentrated by evaporation at reduced pressure to remove the solvent, and 250 mL of ethyl acetate and 250 mL of water were added to the residue. A saturated aqueous sodium bicarbonate solution was added to adjust the pH to 7, and the organic phase was separated. The aqueous phase was extracted 2 times with 280 mL of ethyl acetate, the organic phases were combined, dried over anhydrous sodium sulfate, filtered and concentrated by evaporation at reduced pressure to remove the solvent, thus giving 5.65 g of intermediate **4d.**

MS(ESI, $[M+H]^+$) *m/z:* 282.0
$^1H$ NMR (500 MHz, DMSO-$d_6$) $\delta$ 8.16 (dd, *J* = 2.0, 0.6 Hz, 1H), 7.82 (dd, *J*= 8.9, 1.9 Hz, 1H), 7.76 (dd, *J* = 8.9, 0.6 Hz, 1H), 4.23 (s, 2H), 4.15 (q, *J* = 7.1 Hz, 2H), 1.21 (t, *J* = 7.1 Hz, 3H).

Step 4: Preparation of intermediate **4e**

(S)-Pyrrolidine-3-methanol (3.52 g) and intermediate **4d** (3.3 g) were added in sequence to a reaction flask. $Pd_2(dba)_3$ (2.127 g) and potassium phosphate (9.86 g) were then added, and finally toluene (100 mL) was added. The mixture was heated to 80 °C for 3.5 h under $N_2$ atmosphere. The reaction solution was cooled to room temperature with

stirring and filtered, and the filtrate was purified by silica gel column chromatography (PE:EA = 3:2, v/v) to give 0.8 g of intermediate **4e.**

MS(ESI, $[M+H]^+$) *m/z*: 305.2.

$^1$H NMR (500 MHz, DMSO-$d_6$) $\delta$ 7.54 (d, *J* = 9.0 Hz, 1H), 6.98 (dd, *J* = 9.0, 2.4 Hz, 1H), 6.70 (d, *J* = 2.3 Hz, 1H), 4.72 (t, *J* = 5.2 Hz, 1H), 4.14 (q, *J* = 7.1 Hz, 2H), 3.51 - 3.37 (m, 2H), 3.31 - 3.21 (m, 2H), 3.06 (dd, *J* = 9.3, 6.2 Hz, 1H), 2.48 - 2.40 (m, 1H), 2.06 (dtd, *J* = 12.3, 7.3, 4.9 Hz, 1H), 1.76 (dq, *J* = 12.3, 7.5 Hz, 1H), 120 (t, *J* = 7.1 Hz, 3H).

Step 5: Preparation of intermediate **4f**

Intermediate **4e** (800 mg) and THF (20 mL) were added to a reaction flask. Acrylamide (185 mg) and potassium *tert*-butoxide (292 mg) were then added in sequence at 0 °C, and the mixture was reacted for 3 h under an ice-water bath under $N_2$ atmosphere. The reaction solution was added dropwise to an aqueous ammonium chloride solution, and the reaction was neutralized. After 100 mL of ethyl acetate was added, the mixture was extracted, the organic phase was washed with saturated brine, dried over anhydrous sodium sulfate and filtered, and the filtrate was purified by silica gel column chromatography (eluent EA) to give 0.4 g of intermediate **4f.**

MS(ESI, $[M+H]^+$) *m/z:* 330.2.

$^1$H NMR (500 MHz, DMSO-$d_6$) $\delta$ 11.10 - 10.97 (m, 1H), 7.55 (d, *J* = 9.0 Hz, 1H), 6.98 (dd, *J* = 9.1, 2.3 Hz, 1H), 6.68 (t, *J* = 2.5 Hz, 1H), 4.72 (td, *J* = 5.2, 1.6 Hz, 1H), 4.52 (ddd, *J* = 11.4, 5.0, 1.2 Hz, 1H), 3.51 - 3.39 (m, 2H), 3.31 - 3.18 (m, 2H), 3.05 (ddd, *J* = 17.5, 9.5, 6.2 Hz, 1H), 2.75 (ddd, *J* = 16.9, 11.5, 5.4 Hz, 1H), 2.62 - 2.51 (m, 2H), 2.45 (dt, *J* = 13.9, 7.0 Hz, 1H), 2.19 - 2.01 (m, 2H), 1.76 (ddt, *J* = 12.0, 7.6, 3.4 Hz, 1H), 1.19 (dt, *J* = 11.6, 7.1 Hz, 1H).

Step 6: Synthesis of compound **4**

Intermediate **4f** (200 mg) and DCM (2 mL) were added to a reaction flask, Dess-Martin periodinane (515 mg) was added at 0 °C reaction, and the mixture was reacted at room temperature for 1 h. The reaction solution was filtered. After the filtrate was evaporated under reduced pressure to remove part of the solvent, methanol (5 mL), sodium cyanoborohydride (76 mg), and intermediate **1i** (291 mg) were directly added, and the mixture was reacted at room temperature for 2 h. The reaction solution was purified by silica gel column chromatography (DCM:$CH_3OH$ = 10:1, v/v) and then purified by C18 reversed-phase column ($H_2O$ (1 vol% ammonium acetate):$CH_3CN$ = 40%:60%, v/v) to give 0.44 g of compound **4.**

MS(ESI, $[M+H]^+$) *m/z:* 790.46.

$^1$H NMR (500 MHz, DMSO-$d_6$) $\delta$ 11.20 (s, 1H), 11.05 (s, 1H), 7.76 (d, *J* = 2.9 Hz, 1H), 7.66 (s, 1H), 7.56 (d, *J* = 9.0 Hz, 1H), 7.50 (d, *J* = 8.1 Hz, 2H), 7.33 (d, *J* = 2.8 Hz, 1H), 7.17 (d, *J* = 8.1 Hz, 2H), 6.98 (dd, *J* = 9.2, 2.3 Hz, 1H), 6.70 (d, *J* = 2.4 Hz, 1H), 4.54 (dd, *J* = 11.5, 5.0 Hz, 1H), 4.32 (dd, *J* = 36.1, 11.7 Hz, 2H), 3.62 (tt, *J* = 11.3, 4.0 Hz, 1H), 3.44 - 3.35 (m, 2H), 3.26 (ddd, *J* = 16.0, 13.1, 7.5 Hz, 4H), 3.09 - 2.90 (m, 5H), 2.80 - 2.74 (m, 1H), 2.72 (s, 3H), 2.67 - 2.51 (m, 3H), 2.47 - 2.28 (m, 3H), 2.23 - 2.09 (m, 2H), 2.07 - 1.92 (m, 2H), 1.87 - 1.46 (m, 10H).

$^{13}$C NMR (126 MHz, DMSO-$d_6$) $\delta$ 173.67, 172.08, 169.70, 160.78, 156.52, 156.26, 154.02, 150.97, 145.56, 137.82, 127.44, 122.01, 121.98, 120.28, 118.66, 117.61, 114.70, 110.26, 100.53, 62.34, 55.38, 54.42, 53.24, 49.11, 48.14, 47.21, 45.45, 44.85, 39.01, 36.21, 33.75, 31.57, 31.47, 29.98, 28.22, 24.47, 22.96.

**Example 5:** Synthesis of Compound **5**

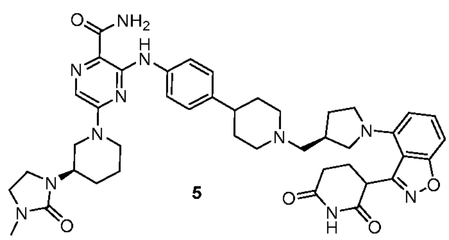

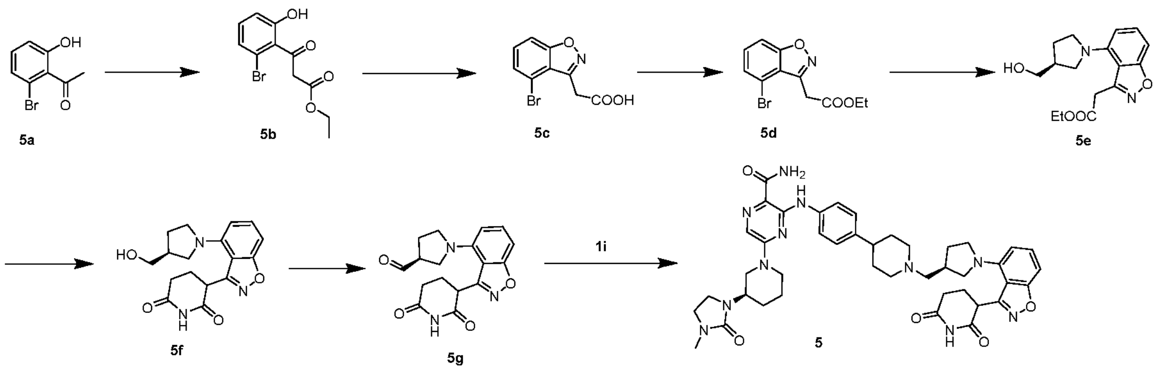


5a 5b 5c 5d 5e 5f 5g 5

Step 1: Preparation of intermediate **5b**

1-(2-Bromo-6-hydroxyphenyl)ethan-1-one (9.5 g), diethyl carbonate (26.1 g), and toluene (200 mL) were added to a reaction flask in sequence. The reaction solution was cooled to 0 °C, and sodium hydride (8.83 g, 60%, 221 mmol) was added in portions. The mixture was heated to 120 °C and reacted overnight, cooled to room temperature, and slowly poured into 1.5 L of stirred ice water. The mixture was extracted with 400 mL of ethyl acetate. The aqueous phase was adjusted to pH = 3 with 3 N hydrochloric acid and extracted 3 times with 400 mL of ethyl acetate, the organic phases were combined, washed once with saturated brine, dried over anhydrous sodium sulfate, filtered, and concentrated by evaporation at reduced pressure to remove the solvent, thus giving intermediate **5b** (12.4 g).

MS(ESI, $[M-H]^-$) *m/z:* 285.0.

$^1H$ NMR (500 MHz, DMSO-d6) δ 10.60 (s, 1H), 7.19 (t, *J* = 8.2 Hz, 1H), 7.09 (dd, *J* = 7.9, 0.9 Hz, 1H), 6.91 (dd, *J* = 8.2, 0.9 Hz, 1H), 4.09 (q, *J* = 7.1 Hz, 2H), 3.87 (s, 2H), 1.20 - 1.14 (m, 3H).

Step 2: Preparation of intermediate **5c**

Intermediate **5b** (11.2 g), MeOH (200 mL), hydroxylamine hydrochloride (9.49 g), and sodium acetate (11.20 g) were added to a reaction flask in sequence, and the mixture was heated to 80 °C and reacted for 3 h. The reaction solution was cooled to room temperature, 3 N hydrochloric acid was added to adjust the pH to 5, the mixture was concentrated by evaporation at reduced pressure to remove the solvent, 1 L of water was added, the reaction flask was placed under an ice-water bath to cool, and meanwhile the pH was adjusted to 3 with 3 N hydrochloric acid. The mixture was stirred for 30 min and filtered. The filter cake was collected and dried to give intermediate **5c** (8.0 g).

$^1H$ NMR (500 MHz, DMSO-*d6*) δ 12.99 (s, 1H), 7.81 (dd, *J* = 8.2, 0.8 Hz, 1H), 7.66 - 7.55 (m, 2H), 4.15 (s, 2H).

Step 3: Preparation of intermediate **5d**

Intermediate **5c** (8 g), ethanol (100 mL), and concentrated sulfuric acid (6.13 g, 3.33 mL) were added to a reaction flask in sequence, and the mixture was heated to 90 °C and reacted overnight. The reaction solution was cooled to room temperature and concentrated by evaporation at reduced pressure to remove the solvent, and 250 mL of ethyl acetate and 250 mL of water were added to the residue for dilution. A saturated aqueous sodium bicarbonate solution was added to adjust the pH to 7, and the organic phase was separated. The aqueous phase was extracted 2 times with 250 mL of ethyl acetate to give intermediate **5d** (8.64 g).

$^1H$ NMR (500 MHz, DMSO-d6) δ 7.83 (dd, *J* = 8.1, 0.9 Hz, 1H), 7.69 - 7.56 (m, 2H), 4.25 (s, 2H), 4.16 (q, *J* = 7.1 Hz, 2H), 1.20 (t, *J* = 7.1 Hz, 3H).

Step 4: Preparation of intermediate **5e**

Intermediate **5d** (500 mg), (S)-pyrrolidin-3-ylmethanol (267 mg), palladium acetate (39.5 mg), and potassium phosphate (747 mg) were added to a reaction flask in sequence, a solvent 1,4-dioxane (30 mL) was added, and the mixture was heated to 100 °C and reacted under nitrogen atmosphere overnight. The above reaction procedure was repeated 4 times. After the reaction was completed, the mixtures were cooled to room temperature, combined, filtered under vacuum, concentrated, and purified by silica gel column chromatography to give intermediate **5e** (240 mg).

MS(ESI, $[M+H]^+$) *m/z*: 305.0.

$^1H$ NMR (500 MHz, DMSO-*d6*) δ 7.44 (t, *J* = 8.0 Hz, 1H), 7.15 (d, *J* = 8.2 Hz, 1H), 6.73 (d, *J* = 7.8 Hz, 1H), 4.68 (t, *J* = 5.2 Hz, 1H), 4.18 (s, 2H), 4.10 (q, *J* = 7.1 Hz, 2H), 3.46 - 3.37 (m, 2H), 3.30 - 3.20 (m, 3H), 3.08 - 3.01 (m, 1H), 2.45 - 2.33 (m, 1H), 2.04 - 1.94 (m, 1H), 1.69 - 1.58 (m, 1H), 1.16 (t, *J* = 7.1 Hz, 3H).

Step 5: Preparation of intermediate **5f**

Intermediate **5e** (120 mg), acrylamide (32.2 mg), and THF (5 mL) were added to a reaction flask in sequence. After the mixture was cooled to an internal temperature of -10 °C, a potassium *tert*-butoxide solution (88 mg, 0.784 mL, 0.784 mmol) was slowly added, and the mixture was reacted at -10 °C. After the reaction was completed, the reaction was quenched with saturated ammonium chloride. The mixture was extracted with 20 mL of EA, washed once with saturated brine, dried over anhydrous sodium sulfate, filtered, concentrated, and purified by silica gel column chromatography to give intermediate **5f** (46 mg).
MS(ESI, $[M+H]^+$) *m/z:* 330.0.

Step 6: Preparation of compound **5**

Intermediate **5f** (57 mg), acetonitrile (5 mL), and IBX (145 mg) were added to a reaction flask in sequence, and the mixture was reacted at 85 °C for 1 h. After the reaction was completed, the mixture was cooled to room temperature, and filtered under vacuum, and the filtrate was directly used in the next step (the filtrate contained 5 g of the intermediate). MeOH (5 mL) was added to the filtrate. Intermediate **1i** (68.7 mg) and acetic acid (4.31 mg) were then added in sequence. After the mixture was stirred at room temperature for 20 min, sodium cyanoborohydride (27.1 mg) was added, and the mixture was reacted at room temperature for another 3 h. After the reaction was completed, 5 mL of a saturated sodium bicarbonate solution was added to the reaction solution. The mixture was extracted with DCM, washed once with saturated sodium chloride, dried over anhydrous sodium sulfate, filtered, concentrated, and purified by normal-phase silica gel column chromatography and C18 reversed-phase column chromatography in sequence to give compound **5** (28 mg).
MS(ESI, $[M+H]^+$) *m/z:* 790.6.
$^1H$ NMR (500 MHz, DMSO-*d*6) δ 11.19 (s, 1H), 11.08 (d, *J* = 17.4 Hz, 1H), 7.83 - 7.73 (m, 1H), 7.66 (s, 1H), 7.58 - 7.47 (m, 3H), 7.39 - 7.27 (m, 2H), 7.19 - 7.13 (m, 2H), 7.04 (s, 1H), 4.63 - 4.56 (m, 1H), 4.42 - 4.25 (m, 2H), 3.65 - 3.56 (m, 1H), 3.31 - 3.14 (m, 6H), 3.12 - 2.88 (m, 5H), 2.81 - 2.73 (m, 1H), 2.69 (s, 3H), 2.63 - 2.54 (m, 1H), 2.48 - 2.22 (m, 5H), 2.16 - 1.90 (m, 3H), 1.89 - 1.43 (m, 9H).

**Example 6:** Preparation of Compound 6

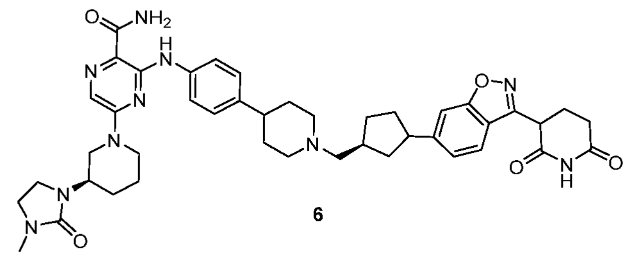

**6**

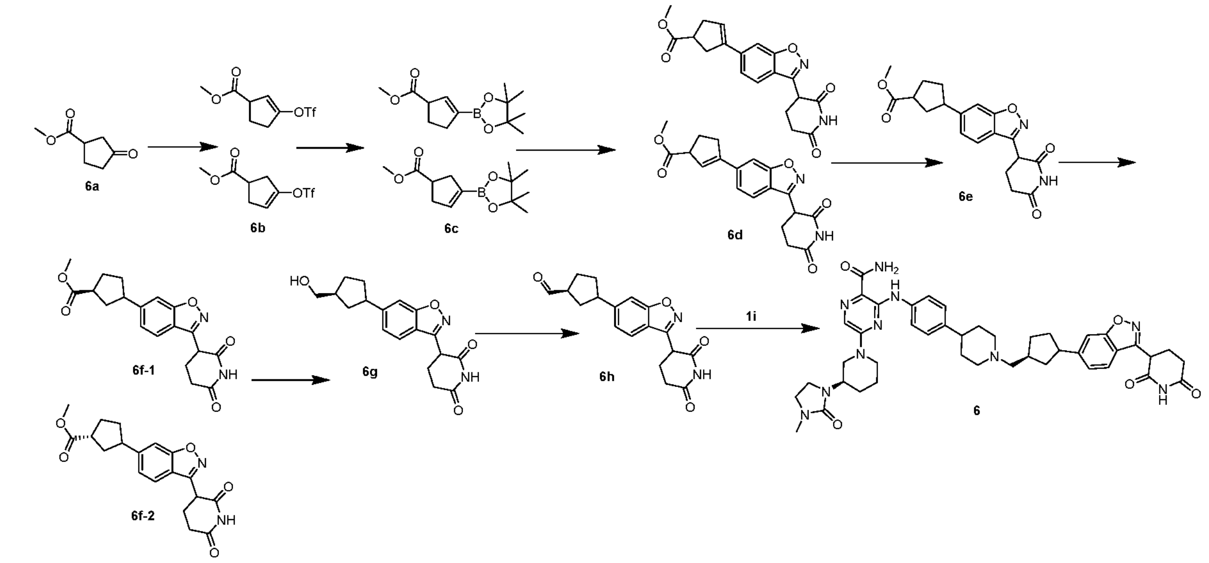


6a 6b 6c 6d 6e 6f-1 6g 6h 6 6f-2

Step 1: Preparation of intermediate **6b**

Intermediate **6a** (3.0 g), DIPEA (5.73 g), and DCM (30 mL) were added to a reaction flask, trifluoromethanesulfonic anhydride (11.31 g) was slowly added under an ice bath, and then the mixture was slowly warmed to room temperature and reacted for 2 h. 100 mL of water and 100 mL of DCM were added to the reaction solution. The organic phase was separated, washed with 100 mL of a saturated sodium chloride solution, dried over anhydrous sodium sulfate, filtered, and concentrated. The concentrate was separated and purified by silica gel column chromatography to give mixture intermediate **6b** (5.2 g).

$^1$H NMR (500 MHz, Chloroform-*d*) δ 5.59 (p, *J* = 2.3 Hz, 1H), 3.73 (s, 3H), 3.29 (ddt, *J* = 10.1, 8.6, 7.0 Hz, 1H), 2.98 (ddq, *J* = 16.3, 6.7, 2.7 Hz, 1H), 2.76 - 2.70 (m, 2H), 2.37 - 2.26 (m, 1H).

Step 2: Preparation of intermediate **6c**

Intermediate **6b** (2.6 g), bis(pinacolato)diboron (2.89 g), potassium acetate (0.694 g), and 1,4-dioxane (30 mL) were added to a reaction flask in sequence, [1,1'-bis(diphenylphosphino)ferrocene]palladium dichloride dichloromethane complex (0.265 g) was added, and the mixture was purged with $N_2$ 3 times. The mixture was then heated to 85 °C and reacted for 2 h, and the heating was stopped. 200 mL of water was added to the reaction solution, and the mixture was extracted 2 times with 100 mL of EA. The combined organic layers were washed twice with 100 mL of a saturated sodium chloride solution. The mixture was washed, dried over anhydrous sodium sulfate, filtered, and concentrated. The concentrate was separated and purified by silica gel column chromatography to give mixture intermediate **6c** (2.1 g).

Step 3: Preparation of intermediate **6d**

Intermediate **6c** (1.22 g), intermediate **1m** (1.0 g), potassium carbonate (1.34 g), $H_2O$ (3 mL), and 1,4-dioxane (15 mL) were added to a reaction flask in sequence, [1,1'-bis(diphenylphosphino)ferrocene]palladium dichloride dichloromethane complex (0.355 g) was added, and the mixture was purged with $N_2$ 3 times. The mixture was then heated to 70 °C and reacted for 2 h, and the heating was stopped. 200 mL of water was added to the reaction solution, and the mixture was extracted 2 times with 100 mL of DCM. The combined organic layers were washed twice with 100 mL of a saturated sodium chloride solution. The mixture was washed, dried over anhydrous sodium sulfate, filtered, and concentrated. The concentrate was separated and purified by silica gel column chromatography to give mixture intermediate **6d** (0.328 g).

MS (ESI, $[M-H]^-$) m/z: 353.2.

Step 4: Preparation of intermediate **6e**

Intermediate **6d** (0.32 g), palladium on carbon catalyst (0.032 g), and MeOH (10 mL) were added to a reaction flask in sequence, and the mixture was reacted at room temperature for 16 h under $H_2$ atmosphere. After the reaction was completed, the reaction solution was filtered, and the filtrate was concentrated to give intermediate **6e** (0.17 g).

MS (ESI, $[M-H]^-$) m/z: 355.2.

Step 5: Preparation of intermediates **6f-1 and 6f-2**

Intermediate **6e** (0.9 g) was resolved by preparative SFC to give intermediate **6f-1** (0.17 g) with S configuration and intermediate **6f-2** (0.1 g) with R configuration.
MS (ESI, $[M-H]^-$) m/z: 355.2.

Step 6: Preparation of intermediate **6g**

Intermediate **6f-1** (0.17 g) and THF (10 mL) were added to a reaction flask, lithium aluminum hydride (0.02 g) was slowly added under an ice bath, and then the mixture was slowly warmed to room temperature and reacted for 1 h. After the reaction was completed, a small amount of ice water was added to the reaction solution under an ice bath to quench the reaction, and 100 mL of DCM and 100 mL of water were then added. The mixture was filtered, and the organic phase of the filtrate was separated, washed with 100 mL of a saturated sodium chloride solution, dried over anhydrous sodium sulfate, filtered, and concentrated. The concentrate was separated and purified by silica gel column chromatography to give intermediate **6g** (0.093 g).
MS (ESI, $[M-H]^-$) m/z: 327.1.

Step 7: Preparation of intermediate **6h**

Intermediate **6g** (0.09 g) and dichloromethane (5 mL) were added to a reaction flask, Dess-Martin periodinane (0.233 g) was added, and the mixture was reacted at room temperature for 1 h. After the reaction was completed, dichloromethane (50 mL) and water (50 mL) were added to the system. The organic phase was separated, and the aqueous phase was extracted 2 times with 50 mL of dichloromethane. The organic phases were combined, dried over anhydrous sodium sulfate, filtered and concentrated to give intermediate **6h** (0.08 g).
MS (ESI, $[M-H]^-$) m/z: 325.1.

Step 8: Preparation of compound **6**

Intermediate **6h** (0.09 g), intermediate **1i** (0.12 g), and methanol (5 mL) were added to a reaction flask, 1 drop of acetic acid was added, and sodium cyanoborohydride (0.032 g) was then added. The mixture was reacted at room temperature for 2 h. After the reaction was completed, 50 mL of dichloromethane and 50 mL of water were added to the system. The organic phase was separated, and the aqueous phase was extracted 2 times with 50 mL of dichloromethane. The organic phases were combined, dried over anhydrous sodium sulfate, filtered and concentrated. The concentrate was separated and purified by silica gel column chromatography to give compound **6** (0.08 g).
MS (ESI, $[M+H]^+$) m/z: 789.5.
$^1H$ NMR (500 MHz, DMSO-$d_6$) $\delta$ 11.18 (s, 1H), 11.09 (s, 1H), 7.75 (d, *J* = 8.6 Hz, 2H), 7.64 (d, *J* = 17.9 Hz, 2H), 7.49 (d, *J* = 8.0 Hz, 2H), 7.32 (d, *J* = 8.2 Hz, 2H), 7.16 (d, *J* = 8.1 Hz, 2H), 4.56 (dd, *J* = 11.9, 5.0 Hz, 1H), 4.39 - 4.33 (m, 1H), 4.31 - 4.24 (m, 1H), 3.64 - 3.58 (m, 1H), 3.27 - 3.22 (m, 4H), 3.05 - 2.91 (m, 4H), 2.78 (td, *J* = 11.9, 6.1 Hz, 1H), 2.70 (s, 3H), 2.64 - 2.59 (m, 1H), 2.46 (s, 2H), 2.39 - 2.31 (m, 2H), 2.21 (ddt, *J* = 13.7, 9.5, 5.7 Hz, 3H), 2.12 - 2.01 (m, 3H), 1.90 (s, 3H), 1.84 - 1.74 (m, 8H), 1.55 (d, *J* = 12.6 Hz, 2H).

**Example 7:** Preparation of Compound **7**

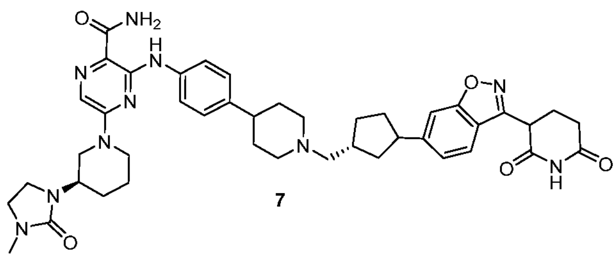

7

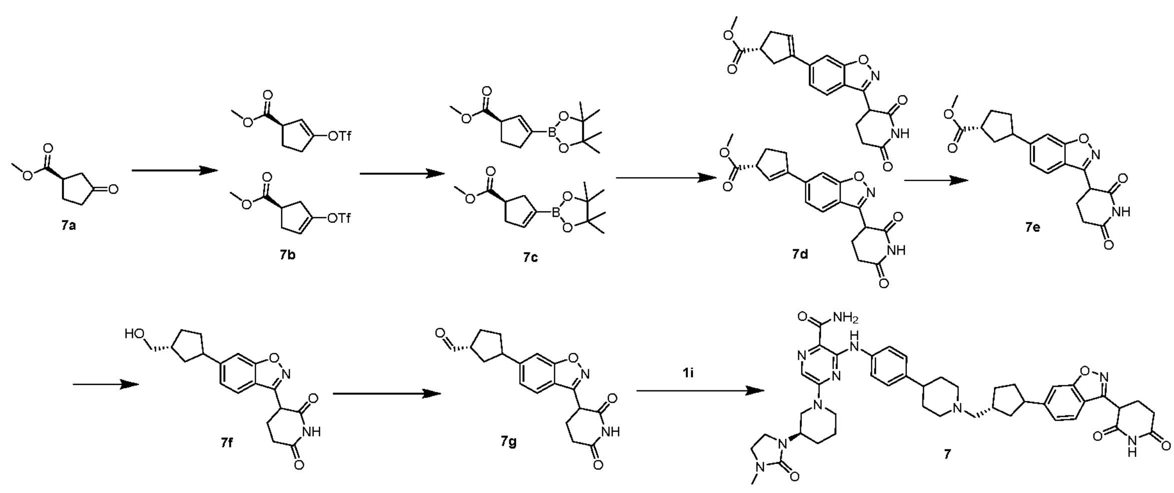


7a 7b 7c 7d 7e 7f 7g 7

Step 1: Preparation of compound **7**

Referring to the procedures of the first step to the eighth step in Example **6,** Example **7** was synthesized, except that intermediate **6a** was replaced by intermediate **7a** as a starting material.

MS (ESI, $[M+H]^+$) m/z: 789.5.

$^1$H NMR (500 MHz, DMSO-$d_6$) δ 11.20 (s, 1H), 11.09 (s, 1H), 7.78 - 7.72 (m, 2H), 7.64 (d, *J* = 16.6 Hz, 2H), 7.50 (d, *J* = 8.1 Hz, 2H), 7.36 - 7.29 (m, 2H), 7.16 (d, *J* = 8.1 Hz, 2H), 4.57 (dd, *J* = 11.9, 5.0 Hz, 1H), 4.36 (d, *J* = 12.2 Hz, 1H), 4.28 (d, *J* = 13.1 Hz, 1H), 3.60 (ddt, *J* = 12.0, 5.3, 2.9 Hz, 1H), 3.30 (s, 2H), 3.27 - 3.19 (m, 3H), 2.99 (dt, *J* = 37.1, 11.7 Hz, 4H), 2.78 (ddd, *J* = 17.2, 12.0, 5.3 Hz, 1H), 2.70 (d, *J* = 2.4 Hz, 3H), 2.61 (dt, *J* = 17.3, 4.3 Hz, 1H), 2.49 - 2.44 (m, 1H), 2.19 (dq, *J* = 13.8, 5.0 Hz, 2H), 2.08 (d, *J* = 8.6 Hz, 2H), 2.00 (d, *J* = 7.5 Hz, 1H), 1.91 (s, 1H), 1.86 - 1.62 (m, 8H), 1.56 (d, *J* = 13.2 Hz, 2H).

**Example 8:** Preparation of Compound **8**

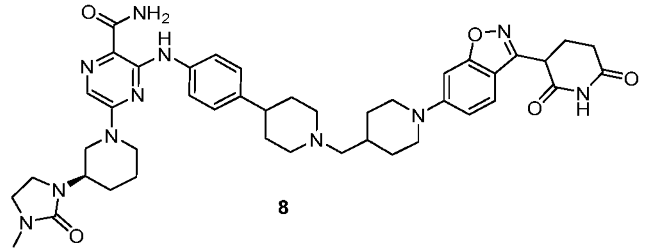

8

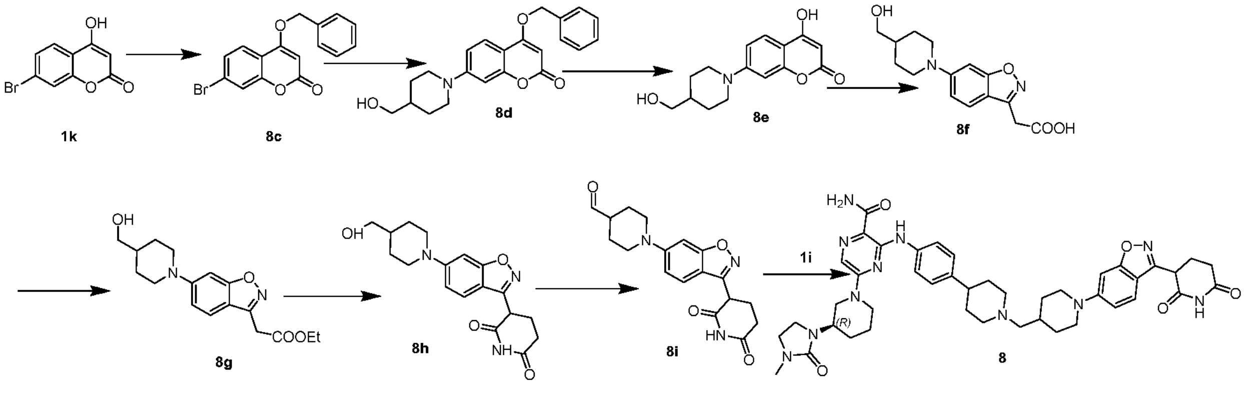


1k 8c 8d 8e 8f 8g 8h 8i 8

Step 1: Preparation of intermediate **8c**

Intermediate **8b** (27 g), benzyl bromide (36.8 g), potassium carbonate (29.8 g), and 700 mL of acetonitrile were added to a single-necked flask in sequence, and the mixture was heated to 80 °C and reacted for 5 h. After the reaction solution was concentrated by evaporation at reduced pressure to remove the solvent, the crude product was separated by silica gel column chromatography (eluent: EA) to give 2.06 g of intermediate **8c** as the target product.

MS(ESI, $[M+H]^+$) *m/z*: 331.03.

$^1$H NMR (500 MHz, DMSO-$d_6$) δ 7.76 - 7.70 (m, 2H), 7.54 (dt, *J* = 7.8, 1.8 Hz, 3H), 7.49 - 7.43 (m, 2H), 7.43 - 7.38 (m, 1H), 6.09 (s, 1H), 5.35 (s, 2H).

Step 2: Preparation of intermediate **8d**

Intermediate **8c** (4 g), 4-hydroxymethylpiperidine (2.77 g), cesium carbonate (7.82 g), palladium chloride (0.213 g), and 1,4-dioxane (150 mL) were added to a single-necked flask in sequence, and the mixture was reacted at 80 °C for 16 h under $N_2$ atmosphere. The reaction solution was cooled to room temperature and concentrated by evaporation at reduced pressure to remove the solvent, and the crude product was separated and purified by silica gel column chromatography (eluent: PE:EA = 1:1, v/v) to give 4.82 g of intermediate **8d.**

MS(ESI, $[M+H]^+$) *m/z:* 366.

$^1$H NMR (500 MHz, DMSO-$d_6$) δ 7.55 (d, *J* = 9.0 Hz, 1H), 7.53 - 7.47 (m, 3H), 7.46 - 7.42 (m, 2H), 7.40 (dd, *J* = 3.8, 2.0 Hz, 1H), 6.93 (dd, *J* = 9.1, 2.5 Hz, 1H), 5.70 (s, 1H), 5.30 (s, 2H), 4.49 (t, *J* = 5.3 Hz, 1H), 3.93 (dt, *J* = 13.3, 3.5 Hz, 2H), 3.27 (t, *J* = 5.8 Hz, 2H), 2.84 (td, *J* = 12.7, 2.7 Hz, 2H), 1.76 - 1.69 (m, 2H), 1.65 - 1.58 (m, 1H), 1.21 - 1.15 (m, 2H).

Step 3: Preparation of intermediate **8e**

Intermediate **8d** (4.00 g), palladium on carbon (2.0 g), and methanol (300 mL) were added to a single-necked flask in sequence. After the mixture was purged several times with $H_2$, the mixture was reacted at room temperature for 16 h. The mixture was filtered under vacuum, the filtrate was concentrated by evaporation at reduced pressure to remove the solvent, and the crude product was separated by silica gel column chromatography (PE:EA = 2:1, v/v) to give 1.70 g of intermediate **8e.**

MS(ESI, $[M+H]^+$) *m/z*: 276.1

$^1$H NMR (500 MHz, DMSO-$d_6$)δ 12.01 (s, 1H), 7.56 (d, J = 8.9 Hz, 1H), 6.92 (dd, J = 9.0, 2.4 Hz, 1H), 6.73 (d, J = 2.4 Hz, 1H), 5.31 (s, 1H), 4.46 (s, 1H), 3.93 (dt, J = 13.2, 3.2 Hz, 2H), 3.27 (d, J = 6.2 Hz, 2H), 2.84 (td, J = 12.7, 2.7 Hz, 2H), 1.76 - 1.69 (m, 2H), 1.66 - 1.57 (m, 1H), 1.18 (qd, J = 12.4, 4.1 Hz, 2H).

Step 4: Preparation of intermediate **8f**

Intermediate **8e** (1.9 g), hydroxylamine hydrochloride (1.555 g), sodium ethoxide (1.523 g), and ethanol (150 mL) were added to a single-necked flask in sequence, and the mixture was reacted at 85 °C for 22 h. The reaction solution was cooled to room temperature and concentrated by evaporation at reduced pressure to remove the solvent. Dichloromethane and a saturated aqueous sodium carbonate solution were added to the crude product. The mixture was extracted and separated, the organic phase was discarded, and the pH of the aqueous phase was adjusted with 6 M HCl to 6. The mixture was extracted several times with dichloromethane. The organic phases were combined, washed with saturated brine, dried over anhydrous sodium sulfate, and filtered. The filtrate was concentrated by evaporation at reduced pressure to remove the solvent, thus giving 1.32 g of intermediate 8f. MS(ESI, $[M+H]^-$) *m/z:* 289.1.

$^1$H NMR (500 MHz, DMSO-$d_6$) δ 12.74 (s, 1H), 7.53 (d, *J* = 8.9 Hz, 1H), 7.07 (dd, *J* = 8.9, 2.1 Hz, 1H), 7.01 (d, *J* = 2.0 Hz, 1H), 4.49 (t, *J* = 5.4 Hz, 1H), 3.94 - 3.87 (m, 4H), 3.34 (s, 2H), 2.80 (td, *J* = 12.5, 2.6 Hz, 2H), 1.77 - 1.71 (m, 2H), 1.63 - 1.57 (m, 1H), 1.22 (dd, *J* = 12.4, 3.7 Hz, 2H).

Step 5: Preparation of intermediate **8g**

Intermediate **8f** (1.3 g), ethanol (100 mL), and sulfuric acid (1.471 g, 15.00 mmol) were added to a single-necked flask in sequence, and the mixture was reacted at 85 °C for 5 h. The reaction solution was cooled to room temperature, and dichloromethane and a saturated aqueous sodium bicarbonate solution were added to the reaction solution to adjust the pH to 8. The organic phase was separated, and the aqueous phase was extracted with dichloromethane. The organic phases were combined, washed with saturated brine, dried over anhydrous sodium sulfate, and filtered. The filtrate was concentrated by evaporation at reduced pressure to remove the solvent, and the crude

product was purified by silica gel column chromatography (PE:EA = 1:4, v/v) to give 1.01 g of intermediate **8g.**

MS(ESI, [M+H]$^+$) *m/z*: 319.16.

$^1$H NMR (500 MHz, DMSO-$d_6$) δ 7.53 (d, *J* = 9.0 Hz, 1H), 7.08 (dd, *J* = 9.0, 2.1 Hz, 1H), 7.02 (d, *J* = 2.1 Hz, 1H), 4.49 (t, *J* = 5.3 Hz, 1H), 4.13 (q, *J* = 7.1 Hz, 2H), 4.04 (s, 2H), 3.89 (dt, *J* = 12.2, 3.4 Hz, 2H), 3.28 (t, *J* = 5.8 Hz, 2H), 3.17 (d, *J* = 5.2 Hz, 1H), 2.81 (td, *J* = 12.6, 2.7 Hz, 2H), 1.74 (dd, *J* = 13.7, 3.7 Hz, 2H), 1.26 - 1.17 (m, 5H).

Step 6: Preparation of intermediate **8h**

Acrylamide (48.7 mg) was slowly added dropwise to a stirred solution of intermediate **8g** (200 mg) in THF (50 mL) at 0 °C under $N_2$ atmosphere. After the dropwise addition was carried out for 1 min and completed, a solution of potassium *tert*-butoxide in THF (1 M) (0.935 mL) was added dropwise, and after the dropwise addition was carried out for about 1 min and completed, the mixture was stirred and reacted at 0 °C for 3 h. The reaction solution was poured into an aqueous ammonium chloride solution. After the mixture was vigorously stirred for 1 min, the mixture was extracted with EA, washed with saturated brine, dried over anhydrous sodium sulfate, and filtered. The filtrate was concentrated by evaporation at reduced pressure to remove the solvent, and the crude product was separated by silica gel column chromatography (PE:EA = 1:4, v/v) to give 0.06 g of intermediate **8h.** MS(ESI, [M+H]$^+$) *m/z:* 344.20.

Step 7: Preparation of intermediate **8i**

Intermediate **8h** (45 mg), IBX oxidant (108 mg), and acetonitrile (10 mL) were added to a single-necked flask in sequence, and the mixture was reacted at 85 °C for 0.5 h. The reaction solution was cooled to room temperature and filtered, and the filtrate (containing intermediate **8i)** was directly used in the next step.

MS(ESI, [M+H]$^+$) *m/z*: 342.16.

Step 8: Preparation of compound **8**

The intermediate **8i** reaction solution (the solution of intermediate 8i obtained in the previous step) and MeOH (10.00 mL) were added to a single-necked flask. Intermediate **1i** (68.7 mg) and acetic acid (3.85 mg) were added in sequence with stirring at room temperature. After the mixture was stirred at room temperature for 30 min, sodium cyanoborohydride (16.11 mg) was added, and the mixture was reacted at room temperature for 21 h. The reaction solution was poured into a mixed solution of dichloromethane and water, and the pH was adjusted to 8 with saturated sodium bicarbonate. The organic phase was separated, and the aqueous phase was extracted several times with dichloromethane. The organic phases were combined, washed with saturated brine, dried over anhydrous sodium sulfate, and filtered. The filtrate was concentrated by evaporation at reduced pressure to remove the solvent, and the crude product was dissolved in DMSO, purified by $C_{18}$ reversed-phase column, and purified by Biotage medium and low pressure chromatography (1 mM aqueous ammonium acetate solution:acetonitrile = 1:1, v/v) to give 27.6 mg of compound **8.**

HR-MS(ESI, [M+H]$^+$) *m/z*: 804.43146.

$^1$H NMR (500 MHz, DMSO-$d_6$)δ 11.21 (s, 1H), 11.05 (s, 1H), 7.79 - 7.73 (m, 1H), 7.66 (s, 1H), 7.53 (dd, J = 22.1, 8.4 Hz, 3H), 7.36 - 7.32 (m, 1H), 7.17 (d, J = 8.1 Hz, 2H), 7.09 - 7.04 (m, 2H), 4.45 (dd, J = 11.4, 5.0 Hz, 1H), 4.39 - 4.32 (m, 1H), 4.29 (d, J = 13.1 Hz, 1H), 3.90 (d, J = 12.3 Hz, 2H), 3.62 (dq, J = 11.2, 6.6, 5.5 Hz, 1H), 3.26 (dd, J = 11.5, 5.1 Hz, 4H), 3.06 - 2.81 (m, 6H), 2.61 - 2.56 (m, 1H), 2.54 - 2.36 (m, 5H), 2.18 (dt, J = 13.2, 5.0 Hz, 2H), 1.99 - 1.68 (m, 11H), 1.64 - 1.51 (m, 2H), 1.24 (q, J = 11.4, 9.3 Hz, 4H).

**Example 9:** Synthesis of Compound **9**

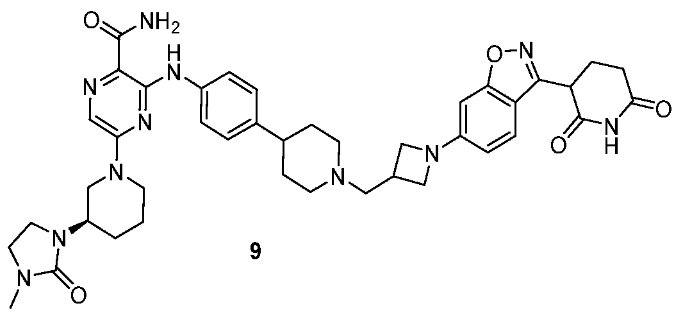

9

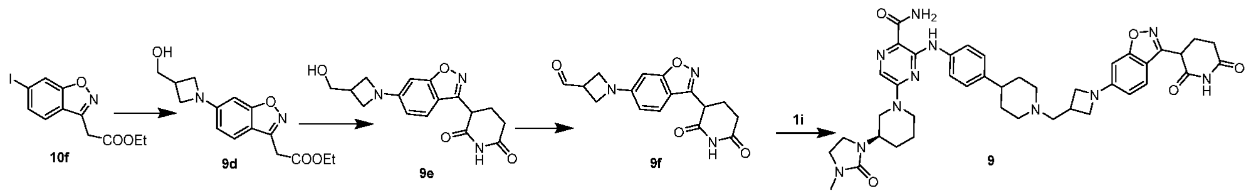

Step 1: Preparation of intermediate **9d**

Intermediate **10f** (10 g), azetidin-3-ylmethanol (3.51 g), L-proline (1.547 g), copper(I) iodide (1.280 g), DMF (100 mL), and sodium carbonate (8.55 g) were added to a reaction flask in sequence, and the mixture was heated to 100 °C and reacted for 4 h under $N_2$ atmosphere. The reaction was stopped, and the reaction solution was cooled to room temperature and extracted three times with the organic solvent DCM (200 mL) and water (500 mL). The organic phase was separated, washed with 500 mL of water, washed with 500 mL of saturated brine, dried over anhydrous sodium sulfate, and filtered. The filtrate was purified by silica gel column chromatography (eluent: EA) to give 2.16 g of intermediate **9d.**

MS(ESI, $[M+H]^+$) *m/z*: 291.2.

$^1$H NMR (500 MHz, DMSO-$d_6$) δ 7.45 - 7.38 (m, 1H), 7.36 (ddt, *J* = 6.6, 4.9, 2.6 Hz, 1H), 6.37 (d, *J* = 7.9 Hz, 1H), 4.73 (t, *J* = 5.3 Hz, 1H), 4.02 (q, *J* = 7.1 Hz, 2H), 3.91 (s, 1H), 3.83 (t, *J* = 7.9 Hz, 1H), 3.55 (dd, *J* = 7.8, 5.4 Hz, 2H), 3.52 - 3.46 (m, 2H), 2.71 (ttd, *J* = 12.4, 8.6, 7.2, 4.3 Hz, 1H), 1.08 (t, *J* = 7.1 Hz, 3H).

Step 2: Preparation of intermediate **9e**

Intermediate **9d** (200 mg) and THF (10 mL) were added to a reaction flask in sequence. The mixture was cooled to about 0 °C, acrylamide (53.9 mg) and potassium *tert*-butoxide (116 mg) were added, and the mixture was reacted at 0 °C for 3.5 h under $N_2$ atmosphere. The reaction was stopped, and the reaction solution was added dropwise to a saturated aqueous ammonium chloride solution. The mixture was extracted with 50 mL of ethyl acetate, the organic phase was separated, washed with 50 mL of saturated brine, dried over anhydrous sodium sulfate and filtered, and the filtrate was purified by silica gel column chromatography (eluent EA) to give 0.035 g of intermediate **9e.**

MS(ESI, $[M+H]^+$) *m/z*: 316.1.

$^1$H NMR (500 MHz, DMSO-$d_6$) δ 11.03 (s, 1H), 7.53 (d, *J* = 8.6 Hz, 1H), 6.49 (d, *J* = 1.8 Hz, 1H), 6.46 (dd, *J* = 8.6, 1.9 Hz, 1H), 4.80 (t, *J* = 5.2 Hz, 1H), 4.42 (dd, *J* = 11.3, 5.0 Hz, 1H), 3.94 (t, *J* = 7.8 Hz, 2H), 3.65 (dd, *J* = 7.7, 5.4 Hz, 2H), 3.59 (t, *J* = 5.8 Hz, 2H), 2.82 (tdd, *J* = 8.2, 6.8, 6.0, 3.6 Hz, 1H), 2.73 (ddd, *J* = 17.0, 11.5, 5.3 Hz, 1H), 2.58 (dt, *J* = 17.2, 4.4 Hz, 1H), 2.41 (dtd, *J* = 13.1, 11.4, 4.6 Hz, 1H), 2.16 (dq, *J* = 13.3, 5.0 Hz, 1H).

Step 3: Preparation of Example **9**

Intermediate **9e** (200 mg), acetonitrile (10.00 mL), and IBX oxidant (533 mg) were added to a reaction flask in sequence, and the mixture was reacted at 80 °C for 1 h. The reaction solution was filtered and evaporated to remove a small amount of the solvent (containing intermediate 9f). MeOH (10 mL), intermediate **1i** (304 mg), and glacial acetic acid (19.04 mg) were added. After the mixture was stirred at room temperature for 30 min, sodium cyanoborohydride (80 mg) was added, and the mixture was reacted at room temperature for 3 h. The mixture was purified by silica gel column chromatography (DCM:$CH_3OH$ = 10:1, v/v). The mixture was then purified by 120 g $C_{18}$ reversed-phase column (10 mM aqueous ammonium acetate solution:$CH_3CN$ = 40%:60%) to give 0.09 g of compound **9.**

MS(ESI, $[M+H]^+$) *m/z*: 776.5.

$^1$H NMR (500 MHz, DMSO-$d_6$) δ 11.20 (s, 1H), 11.04 (s, 1H), 7.76 (d, *J* = 2.9 Hz, 1H), 7.66 (s, 1H), 7.52 (dd, *J* = 22.5, 8.4 Hz, 3H), 7.33 (d, *J* = 2.9 Hz, 1H), 7.16 (d, *J* = 8.2 Hz, 2H), 6.53 - 6.45 (m, 2H), 4.43 (dd, *J* = 11.4, 5.0 Hz, 1H), 4.35 (d, *J* = 12.2 Hz, 1H), 4.29 (d, *J* = 13.4 Hz, 1H), 4.04 (t, *J* = 7.7 Hz, 2H), 3.60 (ddd, *J* = 13.0, 8.6, 5.4 Hz, 3H), 3.32 - 3.23 (m, 4H),3.04 (d, *J* = 11.8 Hz, 1H), 3.01 - 2.91 (m, 4H), 2.72 (s, 4H), 2.64 - 2.52 (m, 3H), 2.48 - 2.36 (m, 2H), 2.17 (dq, *J* = 13.2, 5.0 Hz, 1H), 2.05 (t, *J* = 11.6 Hz, 2H), 1.87 - 1.80 (m, 2H), 1.80 - 1.70 (m, 3H), 1.67 - 1.52 (m, 3H).

$^{13}$C NMR (126 MHz, DMSO-$d_6$) δ 173.60, 172.48, 172.09, 169.70, 165.08, 160.79, 156.41, 154.04, 154.02, 150.98, 140.26, 137.83, 127.44, 122.90, 120.26, 118.66, 114.71, 111.21, 110.23, 89.41, 56.68, 55.38, 54.31, 49.12, 47.24, 45.46, 44.85, 41.51, 39.03, 33.59, 31.56, 31.40, 28.21, 27.80, 24.45, 23.29, 21.54.

**Example 10:** Synthesis of Compound 10

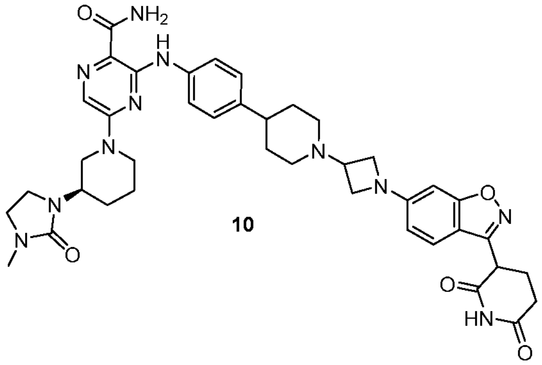

10

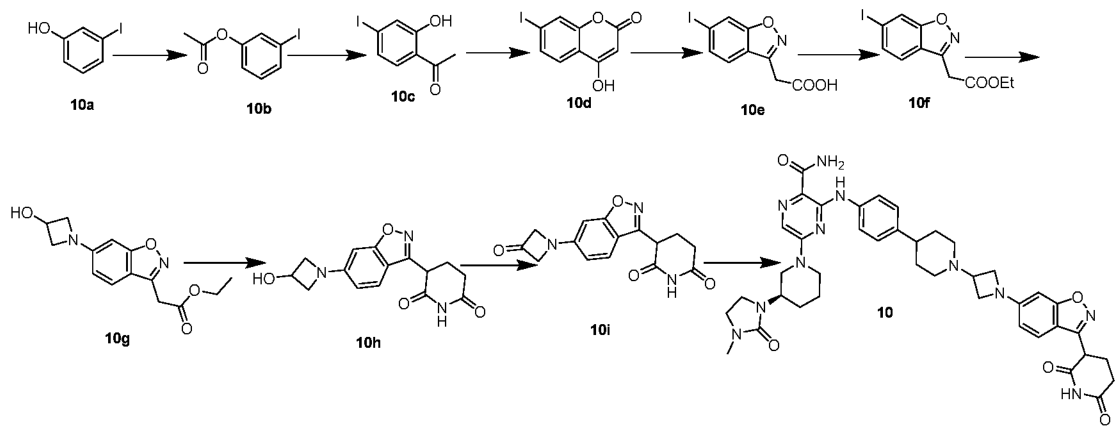

10a 10b 10c 10d 10e 10f

10g 10h 10i 10

Step 1: Preparation of intermediate **10b:**

Intermediate **10a** (100 g), triethylamine (92 g), and DCM (1 L) were added to a single-necked flask in sequence, acetyl chloride (39.2 g) was added at 0 °C, and the mixture was reacted at room temperature for 2.5 h. The system was concentrated to remove DCM, 300 mL × 3 of petroleum ether was added to the system, and the mixture was extracted with 1000 mL of saturated brine. The organic phase was separated, dried over anhydrous sodium sulfate, filtered, and concentrated to give 123 g of intermediate **10b.**

MS(ESI, $[M+H]^+$) *m/z*: 263.0.

Step 2: Preparation of intermediate **10c:**

Intermediate **10b** (123 g) and anhydrous aluminum trichloride (94 g) were added to a single-necked flask in sequence, and the mixture was heated to 170 °C and reacted for 3 h. The reaction solution was cooled to room temperature. About 500 mL of 6 M hydrochloric acid was slowly added to quench the reaction. After a solid present therein was crushed, the mixture was extracted with DCM and filtered. Liquid separation was performed, the organic phase was collected, and the aqueous phase was then extracted 2 times with 250 mL of dichloromethane. The organic phases were combined, dried over anhydrous sodium sulfate, filtered and concentrated by evaporation at reduced pressure to remove the solvent, thus giving 112 g of intermediate **10c.**

MS(ESI, $[M+H]^+$) *m/z*: 263.1.

Step 3: Preparation of intermediate **10d:**

Intermediate **10c** (107 g), diethyl carbonate (174 g), and toluene (1000 mL) were added to a three-necked flask in sequence. After dissolution, the mixture was cooled to about 0 °C. Sodium hydride (58.8 g) was added in portions, and the mixture was warmed to 100 °C. The mixture was warmed to 120 °C and reacted for about 1.5 h after the system was stable. The reaction solution was cooled to room temperature and slowly poured into 2 L of stirred ice water. The mixture was extracted with 500 mL of ethyl acetate, and the organic phase was discarded. The aqueous phase was adjusted to pH = 1-2 with 3 N hydrochloric acid and filtered to give 90 g of intermediate **10d.**

MS(ESI, $[M+H]^+$) *m/z:* 289.1.

Step 4: Preparation of intermediate **10e:**

Intermediate **10d** (90 g), hydroxylamine hydrochloride (43.4 g), and absolute ethanol (1000 mL) were added to a single-necked flask in sequence at 0 °C. After dissolution, sodium ethoxide (42.5 g) was added in portions under $N_2$ atmosphere, and the mixture was heated to 90 °C and reacted for 4.5 h. The reaction solution was cooled to room temperature, and 3 N hydrochloric acid was added to adjust the pH to 1-3. The mixture was concentrated by evaporation at reduced pressure to remove the solvent, 500 mL of water was added to the residue, and the mixture was stirred at room temperature for 30 min and filtered. The filter cake was washed with 200 mL of water and then transferred to a reduced-pressure oven to be dried, thus giving 91.6 g of intermediate **10e.**

MS(ESI, $[M+H]^+$) *m/z*: 304.1.

$^1H$ NMR (500 MHz, DMSO-$d_6$) $\delta$ 8.22 (d, *J* = 1.2 Hz, 1H), 7.71 (dd, *J* = 8.3, 1.3 Hz, 1H), 7.64 (d, *J* = 8.3 Hz, 1H), 4.00 (s, 2H).

Step 5: Preparation of intermediate **10f:**

Intermediate **10e** (91.6 g) and absolute ethanol (1000 mL) were added to a single-necked flask in sequence, concentrated sulfuric acid (148 g, 1511 mmol) was added dropwise at 0 °C over about 3 min (the system was about 50 °C), and the mixture was heated to 85 °C and reacted for 3.5 h under $N_2$ atmosphere. The reaction solution was cooled to room temperature with stirring. The solvent was removed from the reaction solution, 1500 mL of ice water and 1000 mL of ethyl acetate were added to the residue, a 10% aqueous NaOH solution was added dropwise under an ice-water bath, and the pH was adjusted to about 9 (internal temperature < 5 °C). The mixture was extracted and separated three times by a separating funnel, and the organic phases were combined, washed with saturated brine, dried over anhydrous sodium sulfate, and subjected to rotary evaporation to remove the solvent, thus giving 102.6 g of intermediate **10f.**

MS(ESI, $[M+H]^+$) *m/z:* 332.0.

Step 6: Preparation of intermediate **10g:**

Intermediate **10f** (30 g), azetidin-3-ol (7.29 g), L-proline (5.22 g), copper(I) iodide (4.31 g), anhydrous sodium carbonate (28.8 g), and DMF (300 mL) were added to a single-necked flask in sequence, and the mixture was heated to 100 °C and reacted for 2.5 h under $N_2$ atmosphere. The reaction solution was cooled to room temperature and extracted three times with 200 mL $\times$ 3 of the organic solvent ethyl acetate and 1000 mL of water. After the organic phases were separated and combined, the organic phase was washed with 500 mL of water, washed with 500 mL of saturated brine, dried over anhydrous sodium sulfate, and filtered. The filtrate was concentrated by evaporation at reduced pressure to remove the solvent, thus giving a crude product, which was purified by silica gel column chromatography to give 12 g of intermediate **10g.**

MS(ESI, $[M-H]^-$) *m/z:* 275.1.

Step 7: Preparation of intermediate **10h:**

Intermediate **10g** (10 g) and anhydrous tetrahydrofuran (200 mL) were added to a three-necked flask in sequence. After dissolution, acrylamide (1.976 g) was added. Under $N_2$ atmosphere, the mixture was cooled to about -15 °C, potassium *tert*-butoxide (3.894 g) was added dropwise, and the system was kept at -15 °C and reacted for about 1.5 h. The reaction solution was added dropwise to a saturated ammonium chloride solution to quench the reaction, and the mixture was extracted with 100 mL $\times$ 3 of ethyl acetate. The organic phase was separated, washed with 100 mL of saturated brine, dried over anhydrous sodium sulfate, concentrated by evaporation at reduced pressure to remove the solvent, and purified by silica gel column chromatography to give 6.3 g of intermediate **10h.**

MS(ESI, $[M+H]^+$) *m/z*: 302.1.

Step 8: Preparation of intermediate **10i:**

At room temperature, 2-iodoxybenzoic acid (IBX, 9.32 g) was added to a stirred solution of intermediate **10h** in anhydrous acetonitrile (50 mL), and the mixture was reacted at 90 °C for about 3.5 h and filtered. After the filter cake was washed twice with acetonitrile, the filtrates were combined, concentrated by evaporation at reduced pressure to remove the solvent, and purified by silica gel column chromatography to give 4.6 g of intermediate **10i.**

MS(ESI, $[M+H]^+$) *m/z*: 300.1.

Step 9: Preparation of compound **10**

Intermediate **1i** (150 mg), intermediate **10i** (133 mg), dichloromethane (10 mL), and glacial acetic acid (50.4 mg, 0.048 mL) were first added to a reaction flask. After the mixture was stirred at room temperature for 30 min, sodium cyanoborohydride (70.3 mg) was added, and the mixture was stirred at room temperature overnight. After the reaction was completed, 20 mL of a saturated aqueous $NaHCO_3$ solution was added to the reaction solution, and the mixture was extracted twice with 20 mL of DCM-MeOH (10:1). The extracts were combined, dried over anhydrous sodium sulfate, and filtered under vacuum, and the resulting filtrate was subjected to rotary evaporation to dryness and purified by $C_{18}$ reversed-phase column (10 nM aqueous ammonium acetate solution-acetonitrile = 50%:50%, v/v) to give 59 mg of compound **10.**

Q-TOF(ESI, $[M+H]^+$) *m/z*: 762.3847.

$^1$H NMR (500 MHz, DMSO-$d_6$)δ 11.20 (s, 1H), 11.04 (s, 1H), 7.76 (d, *J* = 2.5 Hz, 1H), 7.65 (s, 1H), 7.56 (d, *J* = 8.6 Hz, 1H), 7.49 (d, *J* = 8.3 Hz, 2H), 7.33 (d, *J* = 2.8 Hz, 1H), 7.16 (d, *J* = 8.2 Hz, 2H), 6.56 - 6.47 (m, 2H), 4.44 (dd, *J* = 11.4, 5.0 Hz, 1H), 4.34 (d, *J* = 12.6 Hz, 1H), 4.28 (d, *J* = 13.2 Hz, 1H), 4.04 (t, *J* = 7.3 Hz, 2H), 3.72 (dd, *J* = 8.0, 5.3 Hz, 2H), 3.61 (td, *J* = 11.8, 10.6, 5.1 Hz, 1H), 3.32 - 3.20 (m, 4H), 3.03 (t, *J* = 11.8 Hz, 1H), 2.99 - 2.90 (m, 3H), 2.70 (s, 4H), 2.59 (dt, *J* = 17.4, 4.5 Hz, 1H), 2.49 - 2.37 (m, 2H), 2.17 (dq, *J* = 14.1, 4.9 Hz, 1H), 1.96 (t, *J* = 11.2 Hz, 2H), 1.85 - 1.69 (m, 5H), 1.59 (dq, *J* = 15.5, 12.0 Hz, 3H).

$^{13}$C NMR (126 MHz, DMSO-$d_6$) δ 173.61, 172.09, 169.70, 165.06, 160.78, 156.44, 154.04, 153.82, 150.97, 140.19, 137.84, 127.43, 122.97, 120.26, 118.67, 114.71, 111.34, 110.33, 89.58, 56.40, 54.97, 50.51, 49.13, 47.25, 45.44, 44.85, 41.48, 33.25, 31.55, 31.41, 28.19, 24.44, 23.29.

**Example 11:** Synthesis of Compound **11**

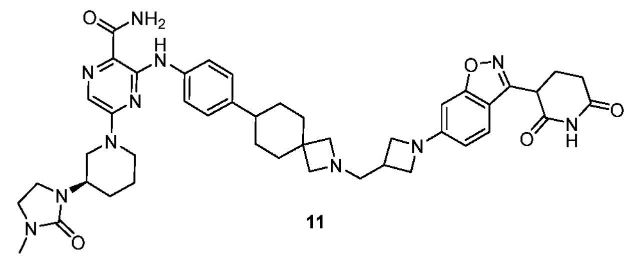

**11**

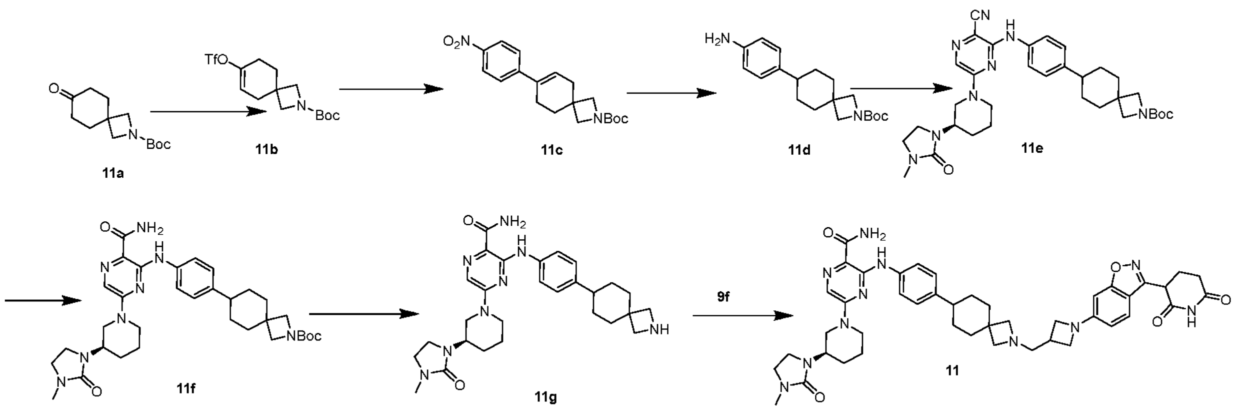


11a 11b 11c 11d 11e 11f 11g 11

Step 1: Preparation of intermediate **11b**

Lithium bis(trimethylsilyl)amide (9.44 g) was slowly added dropwise to a stirred solution of intermediate **11a** (9 g) in THF (150 mL) at -78 °C under $N_2$ atmosphere. After the dropwise addition was carried out for 5 min and completed, the mixture was stirred and reacted at -78 °C for 0.5 h. A solution of N phenylbis(trifluoromethanesulfonyl)imide (17.47 g) in THF (150 mL) was slowly added to the reaction solution, and the temperature was controlled below -60 °C. After the addition was completed, the mixture was reacted at -78 °C for 3.5 h. After the reaction was completed, the reaction solution was poured into a saturated ammonium chloride crushed ice solution, and the mixture was extracted twice with 200 mL of EA. The organic phases were combined, washed once with saturated brine, dried over anhydrous sodium

sulfate, filtered, concentrated, and purified by silica gel column chromatography (PE:EA = 3:2, v/v) to give 13 g of intermediate **11b.**

$^1$H NMR (500 MHz, DMSO-$d_6$) δ 5.87 - 5.77 (m, 1H), 3.57 (d, *J* = 51.4 Hz, 4H), 2.40 (d, *J* = 4.3 Hz, 4H), 1.90 (t, *J* = 6.3 Hz, 2H), 1.38 (s, 9H).

Step 2: Preparation of intermediate **11c**

4-Nitrophenylboronic acid pinacol ester (10.06 g), intermediate **11b** (10 g), potassium carbonate (11.16 g), [1,1'-bis(diphenylphosphino)ferrocene]palladium dichloride dichloromethane complex (4.40 g), 1,4-dioxane (300 mL), and water (50 mL) were added to a single-necked flask in sequence. After the mixture was purged several times with $N_2$, the mixture was heated to 90 °C and reacted for 5 h. After the reaction was completed, the reaction solution was filtered, the filter cake was washed several times with EA, and 500 mL of EA and 200 mL of water were added to the filtrate. The organic phase was separated, washed with 500 mL of saturated brine, dried over anhydrous sodium sulfate, and filtered. The filtrate was purified by silica gel column chromatography (PE:EA = 4:1, v/v). 5.58 g of intermediate **11c** was obtained.

MS(ESI, $[M+H]^+$) *m/z:* 345.05

$^1$H NMR (500 MHz, DMSO-$d_6$)δ 8.26 - 8.15 (m, 2H), 7.75 - 7.61 (m, 2H), 6.43 - 6.33 (m, 1H), 3.60 (d, *J* = 49.0 Hz, 4H), 2.52 (d, *J* = 3.8 Hz, 2H), 2.46 (dt, *J* = 4.5, 2.4 Hz, 2H), 1.89 (t, *J* = 6.2 Hz, 2H), 1.38 (s, 9H).

Step 3: Preparation of intermediate **11d**

Pd/C (10%, 0.015 g) was added to a solution of intermediate **11c** (1 g) in MeOH (50 mL). The reaction solution was first purged 2-3 times with nitrogen, then purged 2-3 times with hydrogen, and stirred at room temperature and reacted for 90 min. After the reaction was completed, the reaction solution was filtered, and the filter cake was rinsed with 50 mL of DCM solvent. The filtrate was concentrated by evaporation at reduced pressure to remove the solvent, thus giving 1.013 g of intermediate **11d.**

MS(ESI, $[M+H]^+$) *m/z*: 317.2.

$^1$H NMR (500 MHz, DMSO-$d_6$)δ 6.87 - 6.79 (m, 2H), 6.51 - 6.42 (m, 2H), 4.79 (s, 2H), 3.58 (d, *J* = 13.2 Hz, 2H), 3.47 (s, 2H), 2.24 (tt, *J* = 11.9, 3.4 Hz, 1H), 1.87 (dd, *J* = 12.9, 3.8 Hz, 2H), 1.66 - 1.57 (m, 2H), 1.48 (td, *J* = 13.0, 3.5 Hz, 2H), 1.38 (s, 9H), 1.35 - 1.27 (m, 2H).

Step 4: Preparation of intermediate **11e**

Intermediate **1f** (1.1 g), intermediate **11d** (1.053 g), BINAP (0.207 g), $Cs_2CO_3$ (3.25 g), $Pd(OAc)_2$ (0.075 g), and 1,4-dioxane (50 mL) were added to a single-necked flask in sequence, and the mixture was heated to 100 °C and reacted under $N_2$ atmosphere for 1.5 h. The reaction was stopped, the reaction solution was cooled to room temperature and filtered, and the filter cake was washed with 150 mL of dichloromethane. The filtrate was concentrated by evaporation at reduced pressure to remove the solvent and purified by silica gel column chromatography (DCM:$CH_3OH$ = 10:1). 1.2 g of intermediate **11e** was obtained.

MS(ESI, $[M+H]^+$) *m/z:* 601.6.

$^1$H NMR (500 MHz, DMSO-$d_6$)δ 8.93 (s, 1H), 7.81 (s, 1H), 7.47 - 7.41 (m, 2H), 7.10 (d, *J* = 8.6 Hz, 2H), 4.24 (d, *J* = 13.1 Hz, 1H), 3.66 - 3.43 (m, 6H), 3.25 (s, 4H), 3.00 - 2.86 (m, 2H), 2.71 (d, *J* = 2.2 Hz, 3H), 2.37 (d, *J* = 12.2 Hz, 1H), 1.91 (d, *J* = 12.7 Hz, 2H), 1.82 - 1.65 (m, 6H), 1.53 (td, *J* = 12.9, 3.5 Hz, 4H), 1.38 (s, 9H).

Step 5: Preparation of intermediate **11f**

Intermediate **11e** (1.2 g), DMSO (100 mL), MeOH (50 mL), and $Cs_2CO_3$ (0.532 g) were added to a single-necked flask in sequence. $H_2O_2$ (0.926 g) was added under an ice bath. After the mixture was stirred for 5 min, the ice bath was removed, and the mixture was reacted at room temperature for 1.5 h. The reaction was stopped, 100 mL of a saturated sodium sulfite solution was added to the reaction solution to quench the reaction. The color was not changed as detected using a potassium iodide starch reagent, and then 100 mL of ethyl acetate was added for extraction. The organic phases were combined, dried over anhydrous sodium sulfate, filtered and concentrated by evaporation at reduced pressure to remove the solvent, thus giving 1.2 g of intermediate **11f**.

MS(ESI, $[M+H]^+$) *m/z*: 619.41.

Step 6: Preparation of intermediate **11g**

Intermediate **11f** (1 g), DCM (20 mL), and trifluoroacetic acid (5.53 g, 3.74 mL) were added to a reaction flask in sequence, and the mixture was reacted at room temperature for 3 h. The solvent was removed by evaporation at reduced pressure to give a product of trifluoroacetate salt. A saturated aqueous sodium bicarbonate solution was slowly added. After the system was adjusted to weak alkalinity, the mixture was stirred for 1 h. The mixture was filtered, and the filter cake was washed with a small amount of water and dried in a vacuum drying oven to give 0.8 g of intermediate **11g.**
MS(ESI, $[M+H]^+$) *m/z:* 519.4.
$^1$H NMR (500 MHz, DMSO-$d_6$) δ 11.22 (s, 1H), 7.76 (d, *J* = 2.7 Hz, 1H), 7.66 (s, 1H), 7.52 - 7.46 (m, 2H), 7.33 (d, *J* = 2.8 Hz, 1H), 7.15 - 7.10 (m, 2H), 4.41 - 4.26 (m, 2H), 3.77 (s, 2H), 3.65 (s, 2H), 3.60 (dt, *J* = 10.9, 4.0 Hz, 1H), 3.40 - 3.33 (m, 2H), 3.30 - 3.23 (m, 2H), 3.05 (t, *J* = 11.8 Hz, 1H), 2.95 (t, *J* = 12.2 Hz, 1H), 2.71 (s, 3H), 2.39 (tt, *J* = 11.8, 3.0 Hz, 1H), 2.08 (d, *J* = 12.8 Hz, 2H), 1.86 - 1.69 (m, 5H), 1.54 (td, *J* = 13.3, 3.6 Hz, 3H), 1.38 (qd, *J* = 13.1, 3.1 Hz, 2H).

Step 11: Preparation of compound **11**

Intermediate **9f** (50 mg), intermediate **11g** (82 mg), MeOH (5 mL), and acetic acid (4.76 mg) were added to a reaction flask in sequence. After the mixture was stirred at room temperature for 30 min, sodium cyanoborohydride (19.93 mg) was added, and the mixture was stirred at room temperature for 3.5 h. The reaction solution was purified by silica gel column chromatography (DCM:$CH_3OH$ = 10:1) to give 0.07 g of Example **11.**
MS(ESI, $[M+H]^+$) *m/z:* 816.7.
$^1$H NMR (500 MHz, DMSO-*$d_6$*) δ 11.20 (s, 1H), 11.04 (s, 1H), 7.75 (d, *J* = 2.9 Hz, 1H), 7.65 (s, 1H), 7.54 (d, *J*= 8.6 Hz, 1H), 7.49 (d, *J* = 8.2 Hz, 2H), 7.33 (d, *J* = 2.9 Hz, 1H), 7.12 (d, *J* = 8.2 Hz, 2H), 6.52 - 6.43 (m, 2H), 4.43 (dd, *J* = 11.4, 5.0 Hz, 1H), 4.37 (d, *J* = 12.2 Hz, 1H), 4.28 (d, *J* = 12.7 Hz, 1H), 3.99 (t, *J* = 7.5 Hz, 2H), 3.67 - 3.55 (m, 3H), 3.36 - 3.30 (m, 6H), 3.26 (dd, *J* = 9.2, 7.2 Hz, 2H), 2.98 (dt, *J* = 35.3, 11.6 Hz, 4H), 2.75 (dd, *J* = 11.8, 5.5 Hz, 2H), 2.72 (s, 3H), 2.58 (dt, *J* = 17.2, 4.4 Hz, 1H), 2.45 - 2.33 (m, 2H), 2.16 (dq, *J* = 13.0, 5.0 Hz, 1H), 1.96 (d, *J* = 11.0 Hz, 2H), 1.85 - 1.73 (m, 3H), 1.69 (d, *J* = 12.4 Hz, 2H), 1.59 - 1.45 (m, 3H), 1.37 (q, *J* = 11.1, 9.5 Hz, 2H).
$^{13}$C NMR (126 MHz, DMSO-*$d_6$*) δ 173.60, 172.08, 169.70, 165.05, 160.78, 156.42, 154.03, 154.01, 150.97, 140.88, 137.73, 127.40, 122.90, 120.14, 118.61, 114.69, 111.25, 110.24, 89.45, 65.83, 56.17, 55.39, 49.12, 47.19, 45.45, 44.85, 42.69, 39.02, 35.91, 31.54, 31.40, 30.98, 28.22, 24.47, 23.29.

**Example 12:** Synthesis of Compound **12**

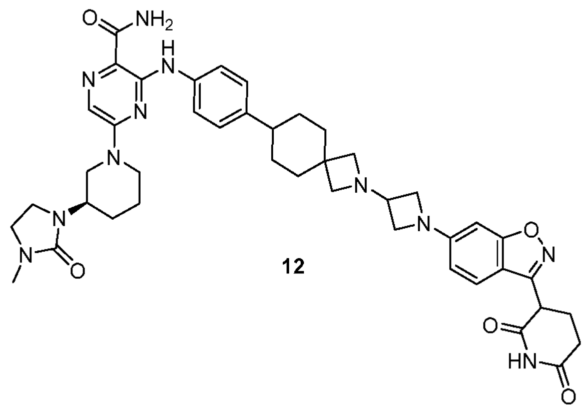

12

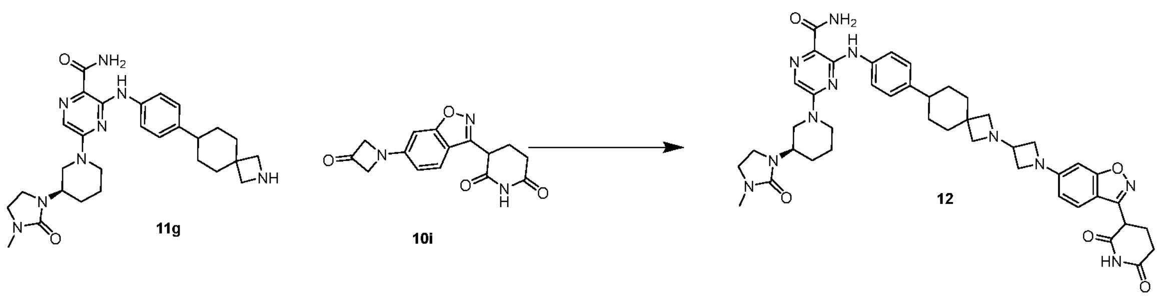

11g

10i

12

Step 1: Preparation of compound **12**

Intermediate **11g** (200 mg), DCM (30 mL), intermediate **10i** (115 mg), and 3 drops of acetic acid were added to a single-necked flask in sequence. The mixture was reacted at room temperature for 1 h and cooled under an ice bath. $NaBH_4$ (36.5 mg) was added, and the mixture was reacted at room temperature for 20 h. The reaction solution was poured into a mixed solvent of a DCM/MeOH = 10/1 solution and water, and the pH was adjusted to 8 with a saturated aqueous sodium bicarbonate solution. The organic phase was separated, and the aqueous phase was extracted several times with DCM/MeOH = 10/1. The organic phases were combined, washed with saturated brine, dried over anhydrous sodium sulfate, and filtered. The filtrate was concentrated by evaporation at reduced pressure to remove the solvent, and the crude product was purified by silica gel column chromatography (dichloromethane:methanol = 20:1, v/v). The resulting purified product was dissolved in DMSO, purified by (120 g) commercial $C_{18}$ reversed-phase column, and purified by Biotage medium and low pressure chromatography (1 M aqueous ammonium acetate solution:acetonitrile = 1:1, v/v) to give 61 mg of compound **12.**

HRMS (ESI) *m/z* $[M+H]^+$:802.41917.

$^1H$ NMR (500 MHz, DMSO-$d_6$)δ 11.20 (s, 1H), 11.04 (s, 1H), 7.75 (d, *J* = 2.9 Hz, 1H), 7.65 (s, 1H), 7.55 (d, *J* = 8.6 Hz, 1H), 7.48 (d, *J* = 8.3 Hz, 2H), 7.33 (d, *J* = 2.9 Hz, 1H), 7.11 (d, *J* = 8.3 Hz, 2H), 6.52 (d, *J* = 1.8 Hz, 1H), 6.48 (dd, *J* = 8.6, 1.9 Hz, 1H), 4.43 (dd, *J* = 11.4, 5.0 Hz, 1H), 4.36 (d, *J* = 12.5 Hz, 1H), 4.28 (d, *J* = 13.3 Hz, 1H), 3.94 (t, *J* = 7.6 Hz, 2H), 3.77 - 3.68 (m, 2H), 3.61 (ddt, *J* = 15.4, 11.2, 4.6 Hz, 2H), 3.32 - 3.20 (m, 4H), 3.08 - 2.92 (m, 5H), 2.74 (td, *J* = 11.5, 5.7 Hz, 1H), 2.69 (s, 3H), 2.58 (dt, *J* = 17.3, 4.5 Hz, 1H), 2.41 (dddt, *J* = 20.9, 14.7, 9.7, 3.9 Hz, 2H), 2.17 (dq, *J* = 13.3, 5.0 Hz, 1H), 1.97 (d, *J* = 12.3 Hz, 2H), 1.84 - 1.66 (m, 5H), 1.59 - 1.47 (m, 3H), 1.42 - 1.33 (m, 2H), 1.23 (s, 1H).

$^{13}C$ NMR (126 MHz, DMSO-$d_6$) δ 173.62, 172.10, 169.71, 165.08, 160.78, 156.44, 154.04, 153.66, 150.98, 140.92, 137.72, 127.42, 122.93, 120.16, 118.62, 114.69, 111.25, 110.34, 89.52, 61.21, 59.19, 55.12, 53.85, 49.11, 47.18, 45.44, 44.85, 42.71, 39.01, 36.60, 35.11, 31.51, 31.40, 31.01, 28.22, 24.47, 23.29.

**Example 13:** Preparation of Compound **13**

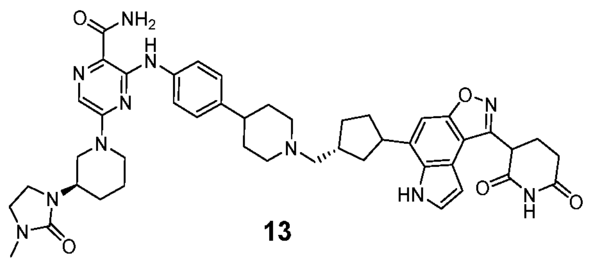

**13**

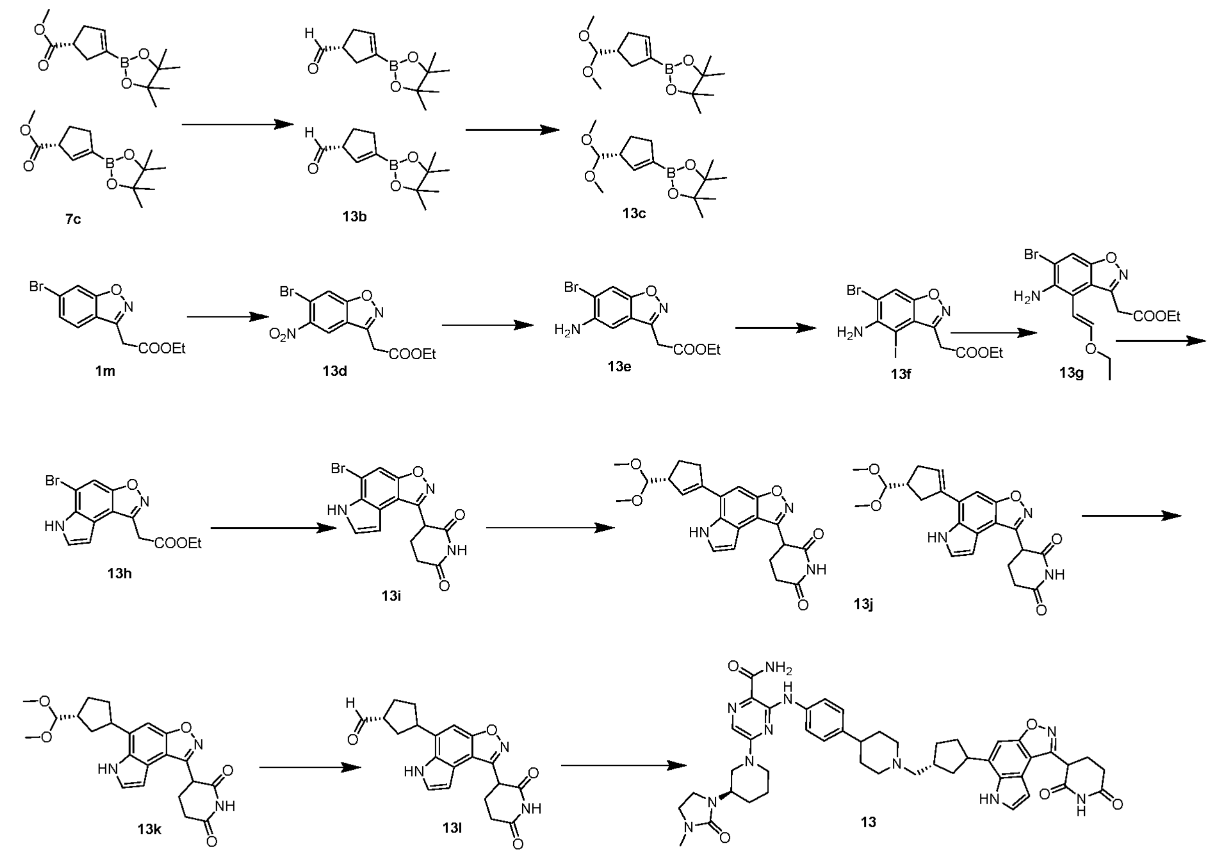

7c 13b 13c

1m 13d 13e 13f 13g

13h 13i 13j

13k 13l 13

Step 1: Preparation of intermediate **13b**

DIBAL-H (0.311 g) was slowly added dropwise to a stirred solution of intermediate **7c** (0.5 g) in DCM (10 mL) at -78 °C under $N_2$ atmosphere. After the dropwise addition was carried out for 3 min and completed, the mixture was stirred and reacted at -78 °C for 1 h. 2 mL of methanol was slowly added to the reaction solution at -78 °C to quench the reaction. The reaction solution was placed at room temperature, diluted with 20 mL of petroleum ether, stirred for 5 min, and filtered. The filtrate was concentrated to give mixture intermediate **13b** (0.4 g).

Step 2: Preparation of intermediate **13c**

Intermediate **13b** (0.4 g), trimethyl orthoformate (0.261 g), *p*-toluenesulfonic acid (0.028 g), and methanol (10 mL) were added to a reaction flask in sequence, and the mixture was reacted at room temperature overnight. About 100 mL of a saturated aqueous sodium bicarbonate solution was added to the system, and the mixture was extracted 3 times with 50 mL of ethyl acetate. The organic phases were combined, dried over anhydrous sodium sulfate, filtered, and concentrated. The concentrate was separated and purified by silica gel column chromatography to give mixture intermediate **13c** (0.32 g).

$^1$H NMR (500 MHz, DMSO-$d_6$) $\delta$ 6.33 - 6.28 (m, 1H), 4.13 (d, *J* = 7.6 Hz, 1H), 3.23 (d, *J* = 1.3 Hz, 6H), 2.50 - 2.47 (m, 1H), 2.40 (dddt, *J* = 18.3, 15.5, 8.8, 2.4 Hz, 2H), 2.27 - 2.14 (m, 2H), 1.19 (s, 12H).

Step 3: Preparation of intermediate **13d**

Intermediate **1m** (57 g) and sulfuric acid (200 mL) were added to a reaction flask in sequence, and a mixed solution of nitric acid (25.28 g, 401.25 mmol) and sulfuric acid (20 mL) was slowly added under an ice bath. After the dropwise addition was completed, the mixture was slowly warmed to room temperature and reacted for 1 h. The reaction solution was slowly poured into 2 L of ice water, the mixture was extracted with 500 mL of ethyl acetate, the organic phase was collected, and the aqueous phase was extracted 2 times with 500 m of ethyl acetate. The organic phases were combined, dried over anhydrous sodium sulfate, filtered, and concentrated. The concentrate was separated and purified by silica gel column chromatography to give intermediate **13d** (53.3 g).

MS (ESI, [M-H]$^-$) m/z: 326.9.

$^1$H NMR (500 MHz, DMSO-$d_6$) δ 8.75 (s, 1H), 8.51 (s, 1H), 4.29 (s, 2H), 4.15 (q, *J* = 7.1 Hz, 2H), 1.20 (t, *J*= 7.1 Hz, 3H).

Step 4: Preparation of intermediate **13e**

Intermediate **13d** (40 g), ethanol (400 mL), and stannous chloride dihydrate (115 g) were added to a reaction flask in sequence, and the mixture was stirred at room temperature for 4 h. The reaction solution was concentrated, and 2 L of dichloromethane and 2 L of water were added to the residue. A saturated aqueous sodium bicarbonate solution was slowly added under an ice bath to adjust the pH to 9-10. The mixture was filtered, and the organic phase of the filtrate was separated, dried over anhydrous sodium sulfate, filtered, and concentrated. The concentrate was separated and purified by silica gel column chromatography to give intermediate **13e** (35.1 g).
MS(ESI, [M+H]$^+$) *m/z:* 299.0.
$^1$H NMR (500 MHz, DMSO-$d_6$) δ 7.93 (s, 1H), 7.04 (s, 1H), 5.35 (s, 2H), 4.14 (q, *J* = 7.1 Hz, 2H), 4.09 (s, 2H), 1.20 (t, *J* = 7.1 Hz, 3H).

Step 5: Preparation of intermediate **13f**

Silver sulfate (29.46 g), elemental iodine (23.98 g), and acetonitrile (300 mL) were added to a reaction flask in sequence, intermediate **13e** (35 g) was then added, and the mixture was reacted at room temperature for 1 h. The reaction solution was filtered, and after the filtrate was concentrated, 500 mL of ethyl acetate and 500 mL of water were added. The organic phase was separated, dried over anhydrous sodium sulfate, filtered, and concentrated. The concentrate was separated and purified by silica gel column chromatography to give intermediate **13f** (27.1 g).
MS(ESI, [M+H]$^+$) *m/z:* 423.0.
$^1$H NMR (500 MHz, DMSO-$d_6$) δ 8.10 (s, 1H), 5.27 (s, 2H), 4.15 (d, *J* = 6.6 Hz, 4H), 1.22 (t, *J* = 7.1 Hz, 3H).

Step 6: Preparation of intermediate **13g**

Intermediate **13f** (26.8 g), (*E*)-1-ethoxyvinyl-2-boronic acid pinacol ester (14.99 g), [1,1'-bis(diphenylphosphino)ferrocene]palladium dichloride dichloromethane complex (4.61 g), potassium carbonate (26.14 g), 1,4-dioxane (200 mL), and water (30.00 mL) were added to a reaction flask in sequence, and the mixture was heated to 70 °C and reacted under $N_2$ atmosphere for 6 h. The reaction solution was cooled to room temperature, and 500 mL of ethyl acetate and 500 mL of water were added. The organic phase was separated, dried over anhydrous sodium sulfate, filtered, and concentrated. The concentrate was separated and purified by silica gel column chromatography to give intermediate **13g** (17 g).
MS(ESI, [M+H]$^+$) *m/z:* 369.1.

Step 7: Preparation of intermediate **13h**

Intermediate **13g** (17 g), DCM (200 mL), hydrochloric acid (57.55 mL, 4 mol/L, 230.22 mmol) were added to a reaction flask in sequence, and the mixture was reacted at room temperature for 5 h. 200 mL of DCM and 200 mL of a saturated sodium bicarbonate solution were added to the system. The organic phase was separated, and the aqueous phase was then extracted 2 times with 50 mL of dichloromethane. The organic phases were combined, dried over anhydrous sodium sulfate, filtered and concentrated. The concentrate was separated and purified by silica gel column chromatography to give intermediate **13h** (7.1 g).
MS(ESI, [M+H]$^+$) *m/z:* 323.0.
$^1$H NMR (500 MHz, DMSO-$d_6$) δ 11.89 (s, 1H), 7.84 (d, *J* = 1.7 Hz, 1H), 7.63 (t, *J* = 2.8 Hz, 1H), 6.77 (dd, *J* = 2.9, 1.6 Hz, 1H), 4.29 (s, 2H), 4.13 (q, *J* = 7.1 Hz, 2H), 1.19 - 1.16 (m, 3H).

Step 8: Preparation of intermediate **13i**

Intermediate **13h** (3 g), acrylamide (0.792 g), and anhydrous tetrahydrofuran (50 mL) were added to a reaction flask in sequence. Potassium *tert*-butoxide (1.56 g) was slowly added at 0 °C, and the mixture was reacted for 2 h. 200 mL of a saturated aqueous ammonium chloride solution was added to the reaction solution to quench the reaction, and the mixture was extracted with 200 mL of DCM. The organic phase was separated, and the aqueous phase was extracted 2 times with 50 mL of DCM. The organic phases were combined, dried over anhydrous sodium sulfate, filtered and concentrated. The concentrate was separated and purified by silica gel column chromatography to give intermediate **13i** (1.34 g).
MS(ESI, [M-H]$^-$) *m/z:* 345.9.

$^1$H NMR (500 MHz, DMSO-$d_6$) δ 11.91 (s, 1H), 11.19 (s, 1H), 7.86 (s, 1H), 7.63 (t, *J* = 2.9 Hz, 1H), 6.71 (dd, *J* = 3.0, 1.8 Hz, 1H), 4.72 (dd, *J* = 12.0, 5.1 Hz, 1H), 2.85 (ddd, *J* = 17.5, 12.2, 5.4 Hz, 1H), 2.64 (dt, *J* = 17.3, 4.1 Hz, 1H), 2.48 - 2.36 (m, 1H), 2.24 (dtd, *J* = 13.7, 5.2, 3.6 Hz, 1H).

Step 9: Preparation of intermediate **13j**

Intermediate **13i** (0.2 g), intermediate **13c** (0.185 g), [1,1'-bis(diphenylphosphino)ferrocene]palladium dichloride dichloromethane complex (0.042 g), potassium carbonate (0.183 g), 1,4-dioxane (10 mL), and water (2.00 mL) were added to a reaction flask in sequence, and the mixture was heated to 120 °C and reacted under $N_2$ atmosphere for 3 h. The reaction solution was cooled to room temperature, and 500 mL of ethyl acetate and 500 mL of water were added. The organic phase was separated, dried over anhydrous sodium sulfate, filtered, and concentrated. The concentrate was separated and purified by silica gel column chromatography to give mixture intermediate **13j** (0.205 g).
MS (ESI, $[M-H]^-$) m/z: 408.2.

Step 10: Preparation of intermediate **13k**

Intermediate **13j** (0.39 g), palladium on carbon catalyst (0.039 g), and MeOH (20 mL) were added to a reaction flask in sequence, and the mixture was reacted at room temperature overnight under $H_2$ atmosphere. The reaction solution was filtered, and the filtrate was concentrated to give intermediate **13k** (0.36 g).
MS (ESI, $[M-H]^-$) m/z: 410.2.
$^1$H NMR (500 MHz, DMSO-$d_6$) δ 11.61 (s, 1H), 11.17 (s, 1H), 7.52 (t, *J* = 2.8 Hz, 1H), 7.33 (d, *J* = 9.7 Hz, 1H), 6.57 - 6.52 (m, 1H), 4.64 (dd, *J* = 11.8, 5.1 Hz, 1H), 4.29 (d, *J* = 7.6 Hz, 1H), 3.61 (ddd, *J* = 24.3, 10.6, 5.4 Hz, 1H), 3.28 (dd, *J* = 7.6, 5.4 Hz, 6H), 2.84 (ddd, *J* = 17.2, 12.0, 5.4 Hz, 1H), 2.62 (dt, *J* = 17.3, 4.2 Hz, 1H), 2.43 (tt, *J*= 12.1, 7.0 Hz, 2H), 2.22 (dq, *J* = 13.8, 7.7, 5.9 Hz, 2H), 2.16 - 2.09 (m, 1H), 1.87 - 1.67 (m, 3H), 1.60 - 1.51 (m, 1H).

Step 11: Preparation of intermediate **13l**

Intermediate **13k** (0.1 g), acetone (5 mL), and p-toluenesulfonic acid (0.021 g) were added to a reaction flask in sequence, and the mixture was reacted at room temperature for 2 h. 50 mL of a saturated aqueous sodium bicarbonate solution and 50 mL of DCM were added to the system. The organic phase was separated, dried over anhydrous sodium sulfate, filtered, and concentrated to give intermediate **13l** (0.085 g).
MS (ESI, $[M-H]^-$) m/z: 364.2.

Step 12: Preparation of compound **13**

Intermediate **13l** (0.08 g), intermediate **1i** (0.104 g), and dichloroethane (5 mL) were added to a reaction flask, 1 drop of acetic acid was added, and sodium cyanoborohydride (0.026 g) was then added. The mixture was reacted at room temperature for 2 h. After the reaction was completed, dichloromethane (50 mL) and water (50 mL) were added to the system. The organic phase was separated, and the aqueous phase was extracted 2 times with dichloromethane (50 mL). The organic phases were combined, dried over anhydrous sodium sulfate, filtered and concentrated. The concentrate was separated and purified by silica gel column chromatography to give compound **13** (0.042 g).
MS (ESI, $[M+H]^+$) m/z: 828.4.
$^1$H NMR (500 MHz, DMSO-$d_6$) δ 11.73 (d, *J* = 27.7 Hz, 1H), 11.28 (s, 1H), 11.17 (s, 1H), 7.78 (s, 1H), 7.67 (s, 1H), 7.56 (d, *J* = 7.5 Hz, 3H), 7.40 (s, 1H), 7.35 (s, 1H), 7.18 (d, *J* = 8.0 Hz, 2H), 6.56 (s, 1H), 4.66 (dd, *J* = 11.7, 5.2 Hz, 1H), 4.39 - 4.27 (m, 2H), 3.71 (s, 1H), 3.62 (s, 3H), 3.26 (s, 3H), 3.07 (d, *J* = 11.6 Hz, 2H), 2.97 (t, *J* = 12.5 Hz, 1H), 2.84 (d, *J* = 14.6 Hz, 1H), 2.71 (s, 3H), 2.63 (d, *J* = 16.9 Hz, 2H), 2.43 (s, 1H), 2.23 (s, 2H), 2.00 (s, 3H), 1.82 (d, *J* = 12.9 Hz, 4H), 1.62 (d, *J* = 45.5 Hz, 4H).

**Example 14:** Synthesis of Compound **14**

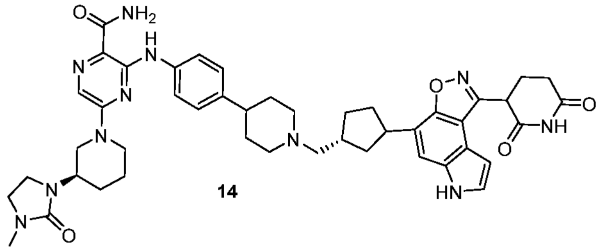

14

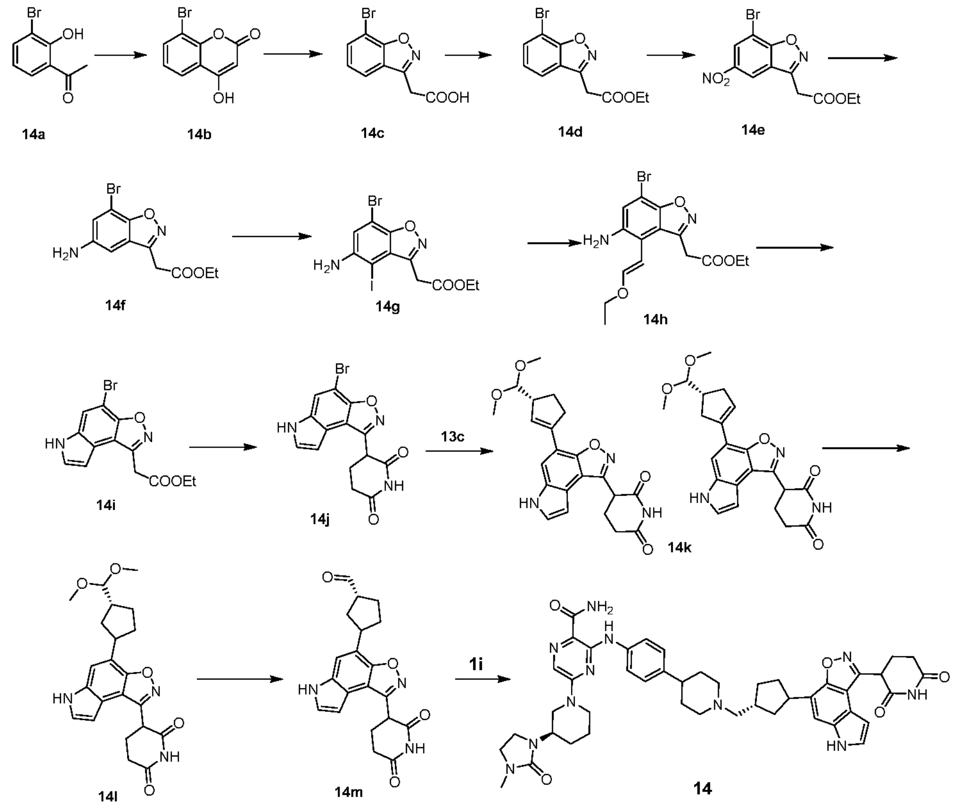


14a 14b 14c 14d 14e 14f 14g 14h 14i 14j 14k 14l 14m 14

Step 1: Preparation of intermediate **14b**

**14a** (50 g), diethyl carbonate (137 g), and toluene (200 mL) were added to a single-necked flask in sequence. The reaction solution was cooled to 0 °C and sodium hydride (46.5 g) was added in portions. The mixture was first heated to 80 °C and reacted for about 10 min. The mixture was heated to 120 °C and reacted for 5 h. The reaction solution was cooled to room temperature and slowly poured into 2 L of stirred ice water. The mixture was extracted with 1 L of ethyl acetate, and the organic phase was discarded. The aqueous phase was adjusted to pH = 3 with 3 N hydrochloric acid, the mixture was extracted 3 times with 500 mL of ethyl acetate, and the organic phases were combined. The organic phase was dried over anhydrous sodium sulfate, filtered and concentrated by evaporation at reduced pressure to remove the solvent, thus giving intermediate **14b** (52 g).

MS(ESI, $[M-H]^+$) m/z: 239.0.

$^1$H NMR (500 MHz, DMSO-$d_6$) $\delta$ 12.77 (s, 1H), 7.94 (dd, *J* = 7.8, 1.5 Hz, 1H), 7.83 (dd, *J* = 7.9, 1.5 Hz, 1H), 7.29 (t, *J* = 7.9 Hz, 1H), 5.64 (s, 1H).

Step 2: Preparation of intermediate **14c**

Intermediate **14b** (52 g), methanol (300 mL), hydroxylamine hydrochloride (52.5 g), and sodium ethoxide (61.9 g) were added to a single-necked flask in sequence, and the mixture was heated to 80 °C and reacted overnight. The reaction solution was cooled to room temperature, 3 N hydrochloric acid was added to adjust the pH to 5, the mixture was concentrated by evaporation at reduced pressure to remove the solvent, 2 L of water was added, the reaction flask was placed under an ice-water bath to cool, and meanwhile the pH was adjusted to 3 with 3 N hydrochloric acid. The

mixture was stirred for 30 min and filtered. The filter cake was collected and dried to give intermediate **14c** (46 g).
MS(ESI, $[M-H]^+$) *m/z:* 254.0.

Step 3: Preparation of intermediate **14d**

Intermediate **14c** (46 g), ethanol (400 mL), and sulfuric acid (106 g, 57.5 mL, 1078 mmol) were added to a single-necked flask in sequence, and the mixture was heated to 90 °C and reacted for 2 h. The reaction solution was cooled to room temperature and concentrated by evaporation at reduced pressure to remove the solvent, and 1 L of ethyl acetate and 1 L of water were added to the residue for dilution. A saturated aqueous sodium bicarbonate solution was added to adjust the pH to 7, and the organic phase was separated. The aqueous phase was extracted 2 times with 500 mL of ethyl acetate, the organic phases were combined, dried over anhydrous sodium sulfate, filtered and concentrated by evaporation at reduced pressure to remove the solvent, thus giving intermediate **14d** (51 g).
MS(ESI, $[M+H]^+$) *m/z:* 283.0.
$^1$H NMR (500 MHz, DMSO-$d_6$) δ 7.93 (dd, *J* = 7.6, 0.9 Hz, 1H), 7.89 (dd, *J* = 7.9, 0.9 Hz, 1H), 7.38 (t, *J* = 7.8 Hz, 1H), 4.26 (s, 2H), 4.15 (q, *J* = 7.1 Hz, 2H), 1.20 (t, *J* = 7.1 Hz, 3H).

Step 4: Preparation of intermediate **14e**

Intermediate **14d** (51 g), sulfuric acid (176 g, 96 mL, 1795 mmol), and potassium nitrate (27.2 g) were added to a single-necked flask in sequence under an ice bath. After the feeding was completed, the mixture was reacted at room temperature for 1 h. The reaction solution was slowly poured into 2 L of ice water, the mixture was extracted with 500 mL of ethyl acetate, the organic phase was collected, and the aqueous phase was extracted 2 times with 500 mL of ethyl acetate. The organic phases were combined, dried over anhydrous sodium sulfate, filtered, and concentrated by evaporation at reduced pressure to remove the solvent. The crude product was separated by silica gel column chromatography (petroleum ether/ethyl acetate = 20:1, v/v) to give the target intermediate **14e** (40 g).
$^1$H NMR (500 MHz, DMSO-$d_6$) δ 8.98 (d, *J* = 2.1 Hz, 1H), 8.75 (d, *J* = 2.1 Hz, 1H), 4.38 (s, 2H), 4.17 (q, *J* = 7.1 Hz, 2H), 1.22 (t, *J* = 7.1 Hz, 3H).

Step 5: Preparation of intermediate **14f**

Intermediate **14e** (40 g), ethanol (400 mL), and stannous chloride dihydrate (115 g) were added to a single-necked flask in sequence, and the mixture was stirred at room temperature for 4 h. The mixture was concentrated by evaporation at reduced pressure to remove the solvent, and 2 L of dichloromethane and 2 L of water were added to the residue. A saturated aqueous sodium bicarbonate solution was slowly added under an ice bath to adjust the pH to weak alkalinity. The mixture was filtered, the filter cake was washed 2 times with 500 mL of dichloromethane, and the filtrate was collected. After liquid separation was performed, the organic phase was collected, and the aqueous phase was then extracted 2 times with 500 mL of dichloromethane. The organic phases were combined, dried over anhydrous sodium sulfate, filtered, and concentrated by evaporation at reduced pressure to remove the solvent. The crude product was separated by silica gel column chromatography (petroleum ether/ethyl acetate = 2:1, v/v) to give the target intermediate **14f** (23 g).
MS(ESI, $[M+H]^+$) *m/z*: 299.0.

Step 6: Preparation of intermediate **14g**

Intermediate **14f** (6 g), DMF (50 mL), and NIS (4.51 g) were added to a single-necked flask in sequence, and the mixture was reacted at room temperature for 1 h. 100 mL of ethyl acetate and 200 mL of water were added to the system. The organic phase was separated. The aqueous phase was then extracted 2 times with 50 mL of ethyl acetate, and the organic phases were combined, dried over anhydrous sodium sulfate, filtered and concentrated by evaporation at reduced pressure to remove the solvent. The crude product was separated by silica gel column chromatography (petroleum ether/ethyl acetate = 4:1, v/v) to give the target intermediate **14g** (8 g).
MS(ESI, $[M-H]^+$) *m/z*: 423.0.
$^1$H NMR (500 MHz, DMSO-$d_6$) δ 7.36 (s, 1H), 5.54 (s, 2H), 4.21 - 4.10 (m, 4H), 1.22 (t, *J* = 7.1 Hz, 3H).

Step 7: Preparation of intermediate **14h**

Intermediate **14g** (4 g), (*E*)-1-ethoxyvinyl-2-boronic acid pinacol ester (2.237 g), [1,1'-bis(diphenylphosphino)ferrocene]palladium dichloride dichloromethane complex (1.377 g), potassium carbonate (3.90 g), 1,4-dioxane (40 mL), and water (10.00 mL) were added to a single-necked flask in sequence, and the mixture was heated to 80 °C and

reacted under $N_2$ atmosphere overnight. The reaction solution was cooled to room temperature, and ethyl acetate (100 mL) and water (100 mL) were added to the system. The organic phase was separated and then extracted 3 times with 50 mL of ethyl acetate, and the organic phases were combined, dried over anhydrous sodium sulfate, filtered and concentrated by evaporation at reduced pressure to remove the solvent. The crude product was separated by silica gel column chromatography (petroleum ether/ethyl acetate = 4:1, v/v) to give the target intermediate **14h** (2.4 g).

MS(ESI, $[M+H]^+$) *m/z:* 369.1.

Step 8: Preparation of intermediate **14i**

Intermediate **14h** (1 g), dichloromethane (10 mL), and trifluoroacetic acid (1.544 g, 1.043 mL) were added to a single-necked flask in sequence, and the mixture was reacted at room temperature overnight. Dichloromethane (100 mL) and water (100 mL) were added to the system. The organic phase was separated. The aqueous phase was then extracted 2 times with dichloromethane (50 mL), and the organic phases were combined, dried over anhydrous sodium sulfate, filtered and concentrated by evaporation at reduced pressure to remove the solvent. The crude product was separated by silica gel column chromatography (petroleum ether/ethyl acetate = 4:1, v/v) to give the target intermediate **14i** (0.5 g).

MS(ESI, $[M+H]^+$) *m/z:* 323.0.

$^1$H NMR (500 MHz, DMSO-$d_6$) $\delta$ 11.78 (s, 1H), 7.96 (s, 1H), 7.61 (t, *J* = 2.9 Hz, 1H), 6.66 (t, *J* = 2.3 Hz, 1H), 4.31 (s, 2H), 4.14 (q, *J* = 7.1 Hz, 2H), 1.16 (t, *J* = 7.1 Hz, 3H).

Step 9: Preparation of intermediate **14j**

Intermediate **14i** (300 mg), acrylamide (66 mg), and anhydrous tetrahydrofuran (5 mL) were added to a three-necked flask in sequence. Potassium *tert*-butoxide (208 mg) was slowly added at 0 °C, and the mixture was reacted at 0 °C for 2 h. A saturated aqueous ammonium chloride solution was added dropwise to the reaction solution to quench the reaction, and the mixture was extracted with 50 mL of ethyl acetate. The organic phase was separated. The aqueous phase was extracted 3 times with ethyl acetate (50 mL), the organic phases were combined, dried over anhydrous sodium sulfate, filtered and concentrated by evaporation at reduced pressure to remove the solvent. The crude product was separated by silica gel column chromatography (petroleum ether/ethyl acetate = 3:2, v/v) to give the target intermediate **14j** (0.18 g).

MS(ESI, $[M-H]^+$) *m/z*: 345.9.

$^1$H NMR (500 MHz, DMSO-$d_6$) $\delta$ 11.80 (s, 1H), 11.20 (s, 1H), 7.97 (d, *J* = 0.8 Hz, 1H), 7.61 (t, *J* = 2.8 Hz, 1H), 6.60 (t, *J* = 2.3 Hz, 1H), 4.73 (dd, *J* = 12.2, 5.1 Hz, 1H), 2.86 (ddd, *J* = 17.5, 12.3, 5.4 Hz, 1H), 2.65 (dt, *J* = 17.3, 4.0 Hz, 1H), 2.45 (qd, *J* = 12.5, 4.4 Hz, 1H), 2.25 (dtd, *J* = 13.6, 5.2, 3.4 Hz, 1H).

Step 10: Preparation of intermediate **14k**

Intermediate **14j** (480 mg), intermediate **13c** (370 mg), [1,1'-bis(diphenylphosphino)ferrocene]palladium dichloride dichloromethane complex (202 mg), potassium carbonate (572 mg), 1,4-dioxane (5 mL), and water (0.5 mL) were added to a single-necked flask in sequence, and the mixture was heated to 85 °C and reacted under $N_2$ atmosphere for 2 h. The reaction solution was cooled to room temperature, and ethyl acetate (50 mL) and water (100 mL) were added to the system for extraction. The organic phase was separated, then extracted 3 times with ethyl acetate (50 mL), dried over anhydrous sodium sulfate, filtered, and concentrated by evaporation at reduced pressure to remove the solvent. The crude product was separated by silica gel column chromatography (dichloromethane/methanol = 60:1, v/v) to give mixture intermediate **14k** (0.35 g).

MS(ESI, $[M-H]^+$) *m/z:* 408.19.

$^1$H NMR (500 MHz, DMSO-$d_6$) $\delta$ 11.63 (d, *J* = 30.9 Hz, 1H), 11.18 (s, 1H), 7.57 (dt, *J* = 9.7, 3.5 Hz, 2H), 6.64 (d, *J* = 6.3 Hz, 1H), 6.62 - 6.48 (m, 1H), 4.71 (dt, *J* = 11.4, 5.0 Hz, 1H), 4.38 - 4.21 (m, 1H), 3.33 - 3.25 (m, 6H), 3.00 - 2.79 (m, 2H), 2.79 - 2.58 (m, 3H), 2.44 (tt, *J* = 12.2, 6.3 Hz, 1H), 2.25 (dp, *J* = 13.1, 5.2 Hz, 1H), 2.16 - 1.96 (m, 1H), 1.88 - 1.72 (m, 1H).

Step 11: Preparation of intermediate **14l**

Intermediate **14k** (50 mg), methanol (3 mL), and Pd/C (10 mg) were added to a single-necked flask in sequence, and the mixture was purged 3 times with hydrogen and reacted at room temperature for 3 h. The reaction solution was filtered to remove palladium on carbon, the filter cake was washed with 5 mL each of dichloromethane, methanol, and ethyl acetate, and the filtrate was distilled under reduced pressure to remove the solvent, thus giving intermediate **14l** (40 mg).

MS(ESI, $[M-H]^+$) *m/z:* 410.21.

$^1$H NMR (500 MHz, DMSO-$d_6$) δ 11.52 (d, *J* = 2.3 Hz, 1H), 11.18 (s, 1H), 7.54 (s, 1H), 7.47 (t, *J* = 2.8 Hz, 1H), 6.49 (q, *J*= 2.3 Hz, 1H), 4.68 (ddd, *J* = 11.9, 9.1, 5.2 Hz, 1H), 4.28 (d, *J*= 7.5 Hz, 1H), 3.55 - 3.41 (m, 1H), 3.29 (dd, *J* = 7.2, 2.3 Hz, 6H), 2.86 (ddd, *J* = 17.3, 12.1, 5.4 Hz, 1H), 2.64 (dt, *J* = 17.3, 4.2 Hz, 1H), 2.49 - 2.38 (m, 2H), 2.29 - 2.09 (m, 3H), 1.89 - 1.75 (m, 2H), 1.74 - 1.61 (m, 2H).

Step 12: Preparation of intermediate **14m**

Intermediate **14l** (100 mg), bis(acetonitrile) palladium dichloride (31.5 mg), and acetone (10 mL) were added to a single-necked flask in sequence, and the mixture was reacted at room temperature for 1 h. Ethyl acetate (30 mL) and water (50 mL) were added to the system for extraction. The organic phase was separated. The aqueous phase was then extracted 3 times with ethyl acetate (30 mL), and the organic phases were combined, dried over anhydrous sodium sulfate, filtered and concentrated by evaporation at reduced pressure to remove the solvent, thus giving intermediate **14m** (70 mg).
MS(ESI, $[M-H]^+$) *m/z:* 364.11

Step 13: Preparation of compound **14**

Intermediate **1i** (90 mg), intermediate **14m** (68.7 mg), and 1,2-dichloroethane (2 mL) were added to a single-necked flask in sequence, 1 drop of acetic acid was added dropwise, and the mixture was stirred at room temperature for 20 min. Sodium cyanoborohydride (35.5 mg) was added, and the mixture was reacted at room temperature. Dichloromethane (20 mL) and water (50 mL) were added to the system for extraction. The organic phase was separated, and the aqueous phase was extracted 3 times with DCM/MeOH = 10:1 (30 mL). The organic phases were combined, dried over anhydrous sodium sulfate, filtered, concentrated by evaporation at reduced pressure to remove the solvent, and purified by silica gel column chromatography to give compound **14** (20 mg).

MS(ESI, $[M+H]^+$) *m/z:* 828.52.

$^1$H NMR (500 MHz, DMSO-$d_6$) δ 11.62 (d, *J* = 27.7 Hz, 1H), 11.28 (s, 1H), 11.19 (s, 1H), 7.89 (s, 1H), 7.68 (s, 1H), 7.56 (d, *J* = 7.5 Hz, 3H), 7.49 (s, 1H), 7.35 (s, 1H), 7.18 (d, *J* = 8.0 Hz, 2H), 6.51 (s, 1H), 4.68 (dd, *J* = 11.7, 5.2 Hz, 1H), 4.35 - 4.25 (m, 2H), 3.76 (s, 1H), 3.61 (s, 3H), 3.31 (s, 3H), 3.13 (d, *J* = 11.6 Hz, 2H), 2.91 (t, *J* = 12.5 Hz, 1H), 2.79 (d, *J* = 14.6 Hz, 1H), 2.79 (s, 3H), 2.59 (d, *J* = 16.9 Hz, 2H), 2.49 (s, 1H), 2.26 (s, 2H), 2.05 (s, 3H), 1.83 (d, *J* = 12.9 Hz, 4H), 1.66 (d, *J* = 45.5 Hz, 4H).

**Example 15:** Preparation of Compound **15**

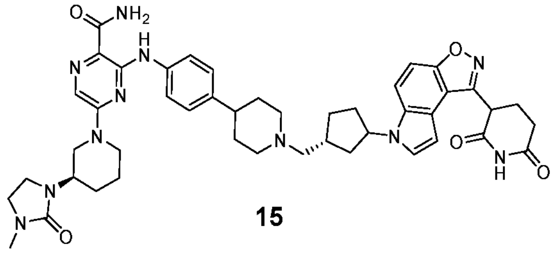

**15**

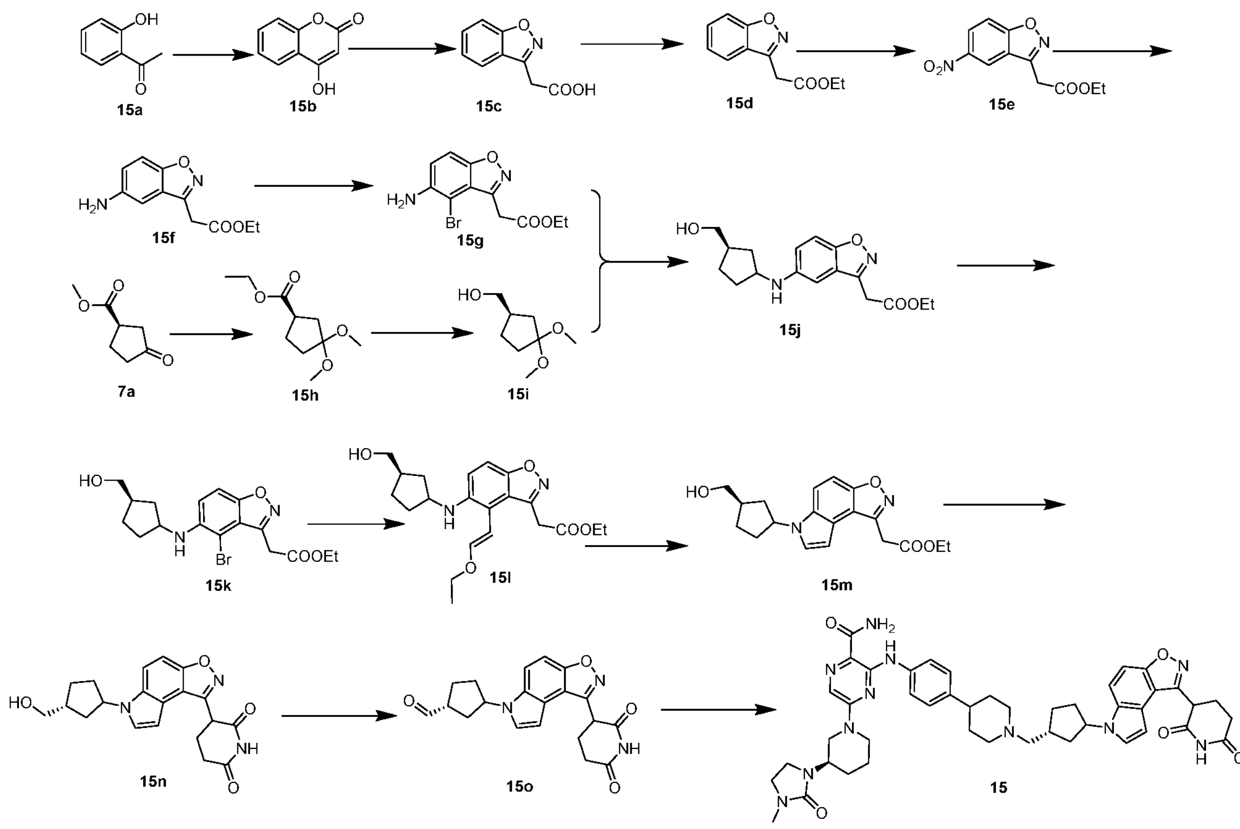

15a 15b 15c 15d 15e 15f 15g 7a 15h 15i 15j 15k 15l 15m 15n 15o 15

Step 1: Preparation of intermediate **15b**

**15a** (50 g), diethyl carbonate (216.9 g), and toluene (500 mL) were added to a reaction flask in sequence. The reaction solution was cooled to 0 °C and sodium hydride (44.06 g) was added in portions. The mixture was first heated to 70 °C and reacted for about 10 min. The mixture was heated to 120 °C and reacted for 5 h. The reaction solution was cooled to room temperature and slowly poured into 2 L of stirred ice water. The mixture was extracted with 1 L of ethyl acetate, and the organic phase was discarded. The aqueous phase was adjusted to pH = 3 with 3 N hydrochloric acid, the mixture was extracted 3 times with 500 mL of ethyl acetate, and the organic phases were combined. The organic phase was dried over anhydrous sodium sulfate, filtered and concentrated to give intermediate **15b** (55 g).

$^1$H NMR (500 MHz, DMSO-$d_6$) δ 12.52 (s, 1H), 7.83 (dd, *J* = 7.8, 1.7 Hz, 1H), 7.65 (ddd, *J* = 8.6, 7.2, 1.7 Hz, 1H), 7.43 - 7.32 (m, 2H), 5.61 (s, 1H).

Step 2: Preparation of intermediate **15c**

Intermediate **15b** (55 g), methanol (500 mL), hydroxylamine hydrochloride (63.5 g), and sodium ethoxide (80.8 g) were added to a reaction flask in sequence, and the mixture was heated to 80 °C and reacted overnight. The reaction solution was cooled to room temperature, 3 N hydrochloric acid was added to adjust the pH to 5, the mixture was concentrated, 2 L of water was added, the reaction flask was placed under an ice-water bath to cool, and meanwhile the pH was adjusted to 3 with 3 N hydrochloric acid. The mixture was stirred for 30 min and filtered. The filter cake was collected and dried to give intermediate **15c** (54.5 g).

$^1$H NMR (500 MHz, DMSO-$d_6$) δ 12.90 (s, 1H), 7.86 (dt, *J* = 7.9, 1.0 Hz, 1H), 7.74 (d, *J* = 8.4 Hz, 1H), 7.66 (ddd, *J* = 8.3, 7.0, 1.2 Hz, 1H), 7.40 (td, *J* = 7.4, 7.0, 0.9 Hz, 1H), 4.11 (s, 2H).

Step 3: Preparation of intermediate **15d**

Intermediate **15c** (54 g), ethanol (400 mL), and sulfuric acid (106 g) were added to a reaction flask in sequence, and the mixture was heated to 90 °C and reacted for 2 h. The reaction solution was cooled to room temperature and concentrated by evaporation at reduced pressure to remove the solvent, and 1 L of ethyl acetate and 1 L of water were added to the residue for dilution. A saturated aqueous sodium bicarbonate solution was added to adjust the pH to 7, and the organic phase was separated. The aqueous phase was extracted 2 times with 500 mL of ethyl acetate, the

organic phases were combined, dried over anhydrous sodium sulfate, filtered and concentrated to give intermediate **15d** (62 g).

MS(ESI, $[M+H]^+$) *m/z:* 206.1.

Step 4: Preparation of intermediate **15e**

Intermediate **15d** (30 g) and sulfuric acid (200 mL) were added to a reaction flask in sequence, and a mixed solution of nitric acid (11.05 g) and sulfuric acid (4 mL) was slowly added under an ice bath. After the dropwise addition was completed, the mixture was slowly warmed to room temperature and reacted for 1 h. The reaction solution was slowly poured into 2 L of ice water, the mixture was extracted with 300 mL of ethyl acetate, the organic phase was collected, and the aqueous phase was extracted 2 times with 100 m of ethyl acetate. The organic phases were combined, dried over anhydrous sodium sulfate, filtered, and concentrated. The concentrate was separated and purified by silica gel column chromatography to give intermediate **15e** (29.3 g).

MS(ESI, $[M+H]^+$) *m/z:* 251.1.

Step 5: Preparation of intermediate **15f**

Intermediate **15e** (29 g), ethanol (300 mL), and stannous chloride dihydrate (130.76 g) were added to a reaction flask in sequence, and the mixture was stirred at room temperature for 4 h. The reaction solution was concentrated, and 1 L of dichloromethane and 0.5 L of water were added to the residue. A saturated aqueous sodium bicarbonate solution was slowly added under an ice bath to adjust the pH to 9-10. The mixture was filtered, and the organic phase of the filtrate was separated, dried over anhydrous sodium sulfate, filtered, and concentrated. The concentrate was separated and purified by silica gel column chromatography to give intermediate **15f** (24.3 g).

MS(ESI, $[M-H]^-$) *m/z:* 219.1.

Step 6: Preparation of intermediate **15g**

Intermediate **15f** (24 g) and DCM (200 mL) were added to a reaction flask in sequence, NBS (21.34 g) was added at 0 °C, and the mixture was reacted at room temperature for 1 h. 500 mL of DCM and 500 mL of water were added to the reaction solution. The organic phase was separated, washed with a saturated sodium chloride solution, dried over anhydrous sodium sulfate, filtered, and concentrated. The concentrate was separated and purified by silica gel column chromatography to give intermediate **15g** (22.4 g).

MS(ESI, $[M+H]^+$) *m/z:* 299.2.

$^1H$ NMR (500 MHz, DMSO-$d_6$) δ 7.52 (d, *J* = 8.9 Hz, 1H), 7.17 (d, *J* = 8.9 Hz, 1H), 4.87 (s, 2H), 4.19 - 4.11 (m, 4H), 1.20 (t, *J* = 7.1 Hz, 3H).

Step 7: Preparation of intermediate **15h**

Intermediate **7a** (5 g), trimethyl orthoformate (11.2 g), p-toluenesulfonic acid (0.606 g), and ethanol (50 mL) were added to a reaction flask in sequence, and the mixture was reacted at room temperature overnight. About 100 mL of a saturated aqueous sodium bicarbonate solution was added to the system, and the mixture was extracted 3 times with 100 mL of ethyl acetate. The organic phases were combined, dried over anhydrous sodium sulfate, filtered, and concentrated. The concentrate was separated and purified by silica gel column chromatography to give intermediate **15h** (4.5 g).

$^1H$ NMR (500 MHz, Chloroform-d) δ 4.14 (q, *J* = 7.1 Hz, 2H), 3.21 (d, *J* = 10.6 Hz, 6H), 2.92 - 2.81 (m, 1H), 2.14 - 2.03 (m, 2H), 2.01 - 1.91 (m, 2H), 1.91 - 1.78 (m, 2H), 1.25 (t, *J* = 7.1 Hz, 3H).

Step 8: Preparation of intermediate **15i**

Intermediate **15h** (4.5 g) and THF (50 mL) were added to a reaction flask, lithium aluminum hydride (0.998 g) was slowly added under an ice bath, and then the mixture was slowly warmed to room temperature and reacted for 1 h. After the reaction was completed, a small amount of ice water was added to the reaction solution under an ice bath to quench the reaction, and 200 mL of DCM and 200 mL of water were then added. The mixture was filtered, and the organic phase of the filtrate was separated, washed with 200 mL of a saturated sodium chloride solution, dried over anhydrous sodium sulfate, filtered, and concentrated to give intermediate **15i** (3.9 g).

$^1H$ NMR (500 MHz, Chloroform-d) δ 3.55 (dhept, *J* = 15.8, 5.4, 4.9 Hz, 2H), 3.21 (d, *J* = 3.0 Hz, 6H), 2.28 (dddd, *J* = 15.1, 8.8, 7.6, 3.9 Hz, 1H), 2.05 - 1.96 (m, 1H), 1.90 (dddd, *J* = 10.2, 8.8, 3.7, 1.5 Hz, 2H), 1.85 - 1.81 (m, 1H), 1.80 - 1.73 (m, 1H), 1.58 (ddd, *J* = 13.4, 7.3, 1.3 Hz, 1H), 1.51 - 1.40 (m, 1H).

Step 9: Preparation of intermediate **15j**

Intermediate **15g** (2.2 g), intermediate **15i** (1.3 g), triethylsilane (1.71 g), and acetonitrile (20 mL) were added to a reaction flask, elemental iodine (1.87 g) was then added, and the mixture was warmed to 90 °C and reacted overnight. The reaction solution was cooled to room temperature and concentrated, and 200 mL of DCM and 200 mL of water were added. The organic phase was separated, washed with 200 mL of a saturated sodium chloride solution, dried over anhydrous sodium sulfate, filtered, and concentrated. The concentrate was separated and purified by silica gel column chromatography to give intermediate **15j** (1.9 g).

MS (ESI, $[M+H]^+$) m/z: 319.2.

Step 10: Preparation of intermediate **15k**

Intermediate **15j** (1.9 g) and DCM (30 mL) were added to a reaction flask in sequence, NBS (1.06 g) was then added, and the mixture was reacted at room temperature for 1 h. 100 mL of DCM and 100 mL of water were added to the reaction solution. The organic phase was separated, washed with a saturated sodium chloride solution, dried over anhydrous sodium sulfate, filtered, and concentrated. The concentrate was separated and purified by silica gel column chromatography to give intermediate **15k** (0.75 g).

MS(ESI, $[M+H]^+$) *m/z:* 397.2.

$^1$H NMR (500 MHz, DMSO-$d_6$) δ 7.63 (dd, *J* = 9.0, 7.4 Hz, 1H), 7.18 (dd, *J* = 9.2, 3.4 Hz, 1H), 4.96 (d, *J* = 7.6 Hz, 1H), 4.70 - 4.63 (m, 1H), 4.19 - 4.12 (m, 4H), 3.92 (hept, *J* = 6.3 Hz, 1H), 3.40 - 3.36 (m, 1H), 2.18 (ddd, *J* = 12.4, 8.4, 6.6 Hz, 1H), 2.14 - 2.02 (m, 1H), 1.94 - 1.76 (m, 1H), 1.75 - 1.61 (m, 1H), 1.51 (dddd, *J* = 20.0, 15.4, 10.7, 6.6 Hz, 2H), 1.35 - 1.23 (m, 1H), 1.20 (t, *J* = 7.1 Hz, 3H).

Step 11: Preparation of intermediate **15l**

Intermediate **15k** (0.7 g), (*E*)-1-ethoxyvinyl-2-boronic acid pinacol ester (0.419 g), [1,1'-bis(diphenylphosphino)ferrocene]palladium dichloride dichloromethane complex (0.129 g), potassium carbonate (0.731 g), 1,4-dioxane (10 mL), and water (2 mL) were added to a microwave tube in sequence, $N_2$ was bubbled, and the mixture was microwaved at 120 °C and reacted for 2 h. The reaction solution was cooled to room temperature, and 100 mL of ethyl acetate and 100 mL of water were added. The organic phase was separated, dried over anhydrous sodium sulfate, filtered, and concentrated. The concentrate was separated and purified by silica gel column chromatography to give intermediate **15l** (0.51 g).

MS(ESI, $[M+H]^+$) *m/z:* 389.2.

Step 12: Preparation of intermediate **15m**

Intermediate **15l** (0.5 g), DCM (20 mL), hydrochloric acid (0.644 mL, 4 mol/L, 2.57 mmol) were added to a reaction flask in sequence, and the mixture was reacted at room temperature for 5 h. 100 mL of DCM and 100 mL of a saturated sodium bicarbonate solution were added to the system. The organic phase was separated, and the aqueous phase was then extracted 2 times with DCM (50 mL). The organic phases were combined, dried over anhydrous sodium sulfate, filtered and concentrated. The concentrate was separated and purified by silica gel column chromatography to give intermediate **15m** (0.24 g).
MS(ESI, $[M+H]^+$) *m/z*: 343.1

Step 13: Preparation of intermediate **15n**

Intermediate **15m** (0.18 g), acrylamide (0.041 g), and anhydrous tetrahydrofuran (10 mL) were added to a reaction flask in sequence. Potassium *tert*-butoxide (0.071 g) was slowly added at 0 °C, and the mixture was reacted for 1 h. 50 mL of a saturated aqueous ammonium chloride solution was added to the reaction solution to quench the reaction, and the mixture was extracted with 50 mL of DCM. The organic phase was separated, dried over anhydrous sodium sulfate, filtered and concentrated. The concentrate was separated and purified by silica gel column chromatography to give intermediate **15n** (0.095 g).

MS(ESI, $[M+H]^+$) *m/z:* 368.2.

Step 14: Preparation of intermediate **15o**

Intermediate **15n** (0.09 g) and dichloromethane (5 mL) were added to a reaction flask, Dess-Martin periodinane (0.233 g) was added, and the mixture was reacted at room temperature for 1 h. After the reaction was completed,

dichloromethane (50 mL) and water (50 mL) were added to the system. The organic phase was separated, and the aqueous phase was extracted 2 times with dichloromethane (50 mL). The organic phases were combined, dried over anhydrous sodium sulfate, filtered and concentrated to give intermediate **15o** (0.08 g).

MS (ESI, $[M-H]^-$) m/z: 364.1.

Step 15: Preparation of compound **15**

Intermediate **15o** (0.08 g), intermediate **1i** (0.104 g), and dichloroethane (5 mL) were added to a reaction flask, 1 drop of acetic acid was added, and sodium cyanoborohydride (0.026 g) was then added. The mixture was reacted at room temperature for 2 h. After the reaction was completed, dichloromethane (50 mL) and water (50 mL) were added to the system. The organic phase was separated, and the aqueous phase was extracted 2 times with dichloromethane (50 mL). The organic phases were combined, dried over anhydrous sodium sulfate, filtered and concentrated. The concentrate was separated and purified by silica gel column chromatography to give compound **15** (0.042 g).

MS (ESI, $[M+H]^+$) m/z: 828.4.

$^1H$ NMR (500 MHz, DMSO-$d_6$) δ 11.18 (s, 2H), 7.91 (dd, *J* = 9.2, 2.0 Hz, 1H), 7.80 - 7.69 (m, 2H), 7.66 (s, 1H), 7.56 - 7.42 (m, 3H), 7.37 - 7.29 (m, 1H), 7.15 (d, *J* = 8.1 Hz, 2H), 6.58 (dd, *J* = 7.9, 3.0 Hz, 1H), 5.09 (d, *J* = 38.6 Hz, 1H), 4.69 (dd, *J* = 11.9, 5.1 Hz, 1H), 4.31 (dd, *J* = 38.9, 12.7 Hz, 2H), 3.60 (dq, *J* = 10.9, 5.8, 4.5 Hz, 1H), 3.30 (d, *J* = 7.9 Hz, 2H), 3.23 (d, *J* = 8.1 Hz, 2H), 3.05 - 2.92 (m, 3H), 2.85 (ddd, *J* = 17.4, 12.1, 5.5 Hz, 1H), 2.68 (s, 3H), 2.63 (dt, *J* = 17.2, 4.2 Hz, 1H), 2.47 - 2.33 (m, 4H), 2.28 - 2.19 (m, 2H), 1.99 (s, 4H), 1.85 - 1.70 (m, 5H), 1.58 (d, *J* = 36.0 Hz, 3H).

**Example 16:** Synthesis of Compound **16**

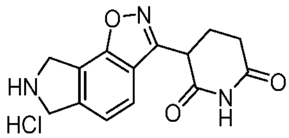

**16**

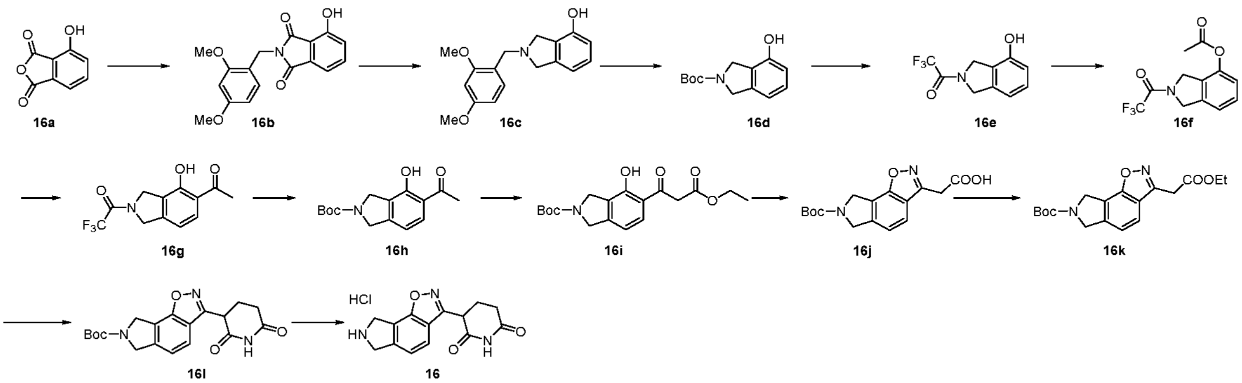

16a
16b
16c
16d
16e
16f
16g
16h
16i
16j
16k
16l
16

Step 1: Preparation of intermediate **16b**

At 10 °C, **16a** (81 g), 2,4-dimethoxybenzylamine (83 g), and acetic acid (500 mL) were added to a reaction flask in sequence, and the mixture was warmed to 80 °C and reacted. After the reaction was completed, as confirmed by TLC, water was added to the reaction solution, and a solid was precipitated. Filter under vacuum was performed, and the filter cake was washed with water and dried to give (95.78 g) intermediate **16b.**

MS (ESI, $[M+H]^-$) *m/z:* 312.02

$^1H$ NMR (500 MHz, DMSO-$d_6$) δ 11.03 (s, 1H), 7.62 (dd, *J* = 8.4, 7.1 Hz, 1H), 7.29 (d, *J* = 7.1 Hz, 1H), 7.22 (d, *J* = 8.4 Hz, 1H), 6.90 (d, *J* = 8.4 Hz, 1H), 6.56 (d, *J* = 2.4 Hz, 1H), 6.43 (dd, *J* = 8.4, 2.4 Hz, 1H), 4.60 (s, 2H), 3.80 (s, 3H), 3.73 (s, 3H).

Step 2: Preparation of intermediate **16c**

At 10 °C, a 2.5 M solution of lithium aluminum hydride in tetrahydrofuran (227 mL) was slowly added dropwise to a solution of **16b** (96.00 g) in tetrahydrofuran (1000 mL), and the mixture was reacted at 80 °C. After the reaction was completed, as confirmed by TLC, a 15 wt% aqueous sodium hydroxide solution and water were added to the reaction solution. Filter under vacuum was performed, the filter cake was washed with a dichloromethane:MeOH = 1:1 solution, the filtrate was concentrated, and the crude product was separated and purified by silica gel column chromatography to give (55.87 g) intermediate **16c.**

MS (ESI, $[M+H]^+$) *m/z:* 286.01
$^1$H NMR (500 MHz, DMSO-$d_6$) δ 9.28 (s, 1H), 7.24 (d, *J* = 8.3 Hz, 1H), 6.97 (t, *J* = 7.7 Hz, 1H), 6.64 (d, *J* = 7.4 Hz, 1H), 6.59 (d, *J* = 8.0 Hz, 1H), 6.55 (d, *J* = 2.4 Hz, 1H), 6.51 (dd, *J* = 8.3, 2.4 Hz, 1H), 3.81 - 3.71 (m, 12H). Step 3: Preparation of intermediate **16d**
**16c** (48.00 g), methanol (350 mL), palladium hydroxide (4.8 g), and di-tert-butyl dicarbonate (41.4 g) were added to a reaction flask in sequence, and the mixture was reacted at 25 °C under hydrogen atmosphere. After the reaction was completed, as confirmed by TLC, the reaction solution was filtered under vacuum. The filtrate was concentrated, extracted with water and ethyl acetate, washed with saturated brine, dried over anhydrous sodium sulfate, and filtered. The filtrate was concentrated, and the crude product was separated by silica gel column chromatography and purified to give 34.43 g of intermediate **16d.**
$^1$H NMR (500 MHz, DMSO-$d_6$) δ 7.07 (t, *J* = 7.7 Hz, 1H), 6.68 (ddd, *J* = 18.9, 7.8, 2.7 Hz, 2H), 4.56 - 4.50 (m, 2H), 4.48 - 4.42 (m, 2H), 1.45 (s, 9H).

Step 4: Preparation of intermediate **16e**

**16d** (35.00 g), methanol (250 mL), and a solution of 4 M hydrochloric acid in dioxane (123 mL) were added to a reaction flask in sequence, and the mixture was reacted at 25 °C. After the reaction was completed, as confirmed by TLC, the reaction solution was concentrated, pyridine (200 mL) and trifluoroacetic anhydride (25.20 g) were added, and the mixture was stirred and reacted at 25 °C. After the reaction was completed, as confirmed by TLC, the reaction solution was poured into a 3 M aqueous hydrochloric acid solution, vigorously stirred, extracted with ethyl acetate, washed with saturated brine, dried over anhydrous sodium sulfate, and filtered. The filtrate was concentrated and dried to give 32.48 g of intermediate **16e.**

MS (ESI, $[M+H]^-$) *m/z:* 229.99
$^1$H NMR (500 MHz, DMSO-$d_6$)δ 9.82 (d, *J* = 24.4 Hz, 1H), 7.16 (td, *J* = 7.7, 3.7 Hz, 1H), 6.81 (dd, *J* = 11.5, 7.5 Hz, 1H), 6.74 (d, *J* = 8.0 Hz, 1H), 4.99 (s, 1H), 4.89 (s, 1H), 4.80 (s, 1H), 4.70 (s, 1H).

Step 5: Preparation of intermediate **16f**

**16e** (32.48 g), dichloromethane (300 mL), triethylamine (28.40 g), 4-dimethylaminopyridine (1.72 g), and acetic anhydride (15.78 g) were added to a reaction flask in sequence, and the mixture was reacted at 25 °C. After the reaction was completed, as confirmed by TLC, the reaction solution was poured into water, vigorously stirred, extracted with dichloromethane, washed with saturated brine, dried over anhydrous sodium sulfate, and filtered. The filtrate was concentrated, and the crude product was separated by silica gel column chromatography and purified to give 33.82 g net weight of intermediate **16f.**

$^1$H NMR (500 MHz, DMSO-$d_6$)δ 7.41 (td, *J* = 7.8, 2.3 Hz, 1H), 7.30 (t, *J* = 8.5 Hz, 1H), 7.12 (dd, *J* = 7.9, 2.9 Hz, 1H), 5.09 (s, 1H), 4.90 (d, *J* = 12.3 Hz, 2H), 4.72 (s, 1H), 2.31 (d, *J* = 3.4 Hz, 3H).

Step 6: Preparation of intermediate **16g**

**16f** (30.00 g), aluminum trichloride (29.30 g), and orthodichlorobenzene (200 mL) were added to a reaction flask in sequence, and the mixture was reacted at 150 °C. After the reaction was completed, as confirmed by TLC, the reaction solution was poured into an aqueous citric acid solution, vigorously stirred, extracted with ethyl acetate, washed with saturated brine, dried over anhydrous sodium sulfate, and filtered. The filtrate was concentrated, and the crude product was separated by silica gel column chromatography and purified to give (18.19 g) net weight of intermediate **16g.**

$^1$H NMR (500 MHz, DMSO-$d_6$) δ 12.38 (s, 1H), 7.94 (dd, *J* = 8.2, 4.7 Hz, 1H), 7.01 (dd, *J* = 12.7, 8.1 Hz, 1H), 5.07 (s, 1H), 4.94 (s, 1H), 4.86 (s, 1H), 4.74 (d, *J* = 1.6 Hz, 1H), 2.66 (d, *J* = 1.0 Hz, 3H).

Step 7: Preparation of intermediate **16h**

**16g** (18.19 g), sodium hydroxide (7.56 g), methanol (150 mL), and water (150 mL) were added to a reaction flask in sequence, and the mixture was reacted at 25 °C for 2.5 h. The reaction solution was concentrated to remove methanol, 1,4-dioxane (150 mL) and di-*tert*-butyl dicarbonate (15.14 g) were added to the residue, and the mixture was stirred and reacted at 25 °C for 2 h. The reaction solution was poured into water, vigorously stirred, extracted with ethyl acetate, washed with saturated brine, dried over anhydrous sodium sulfate, and filtered. The filtrate was concentrated, and the crude product was separated by silica gel column chromatography and purified to give (14.10 g) intermediate **16h.**

MS (ESI, $[M+H]^-$) *m/z:* 276.07
$^1$H NMR (500 MHz, DMSO-$d_6$) δ 12.36 (s, 1H), 7.89 (dd, J = 8.1, 2.9 Hz, 1H), 6.95 (t, J = 8.0 Hz, 1H), 4.62 (dt, J = 13.4, 2.2 Hz, 2H), 4.51 (dt, J = 13.9, 2.3 Hz, 2H), 2.65 (s, 3H), 1.46 (s, 9H).

Step 8: Preparation of intermediate **16i**

**16h** (14.10 g), diethyl carbonate (27.00 g), and toluene (200 mL) were added to a reaction flask in sequence, and the mixture was cooled to 0 °C. After 60 wt% sodium hydride (9.16 g) was added, the mixture was warmed to 120 °C, stirred, and reacted. After the reaction was completed, as confirmed by TLC, the reaction solution was poured into a 3 M aqueous hydrochloric acid solution, vigorously stirred, extracted with ethyl acetate, washed with saturated brine, dried over anhydrous sodium sulfate, and filtered. The filtrate was concentrated, and the crude product was separated by silica gel column chromatography and purified to give (14.96 g) net weight of intermediate **16i.**

MS (ESI, $[M+H]^+$) *m/z:* 250.20
$^1$H NMR (500 MHz, DMSO-$d_6$) δ 11.72 (d, *J* = 9.6 Hz, 1H), 7.82 (dd, *J* = 8.2, 4.7 Hz, 1H), 6.97 (t, *J* = 8.4 Hz, 1H), 4.63 (dt, *J* = 12.4, 2.1 Hz, 2H), 4.53 (dt, *J* = 13.7, 2.1 Hz, 2H), 4.22 (d, *J* = 2.5 Hz, 2H), 4.13 (q, *J* = 7.1 Hz, 2H), 1.46 (d, *J* = 1.9 Hz, 9H), 1.19 (t, *J* = 7.1 Hz, 3H).

Step 9: Preparation of intermediate **16j**

**16i** (14.96 g), a 50% aqueous hydroxylamine solution (6.36 g), and ethanol (150 mL) were added to a reaction flask in sequence, and the mixture was stirred and reacted at 85 °C. After the reaction was completed, as confirmed by TLC, the reaction solution was concentrated, and water and ethyl acetate were then added. The mixture was extracted and separated, and the organic phase was washed with saturated brine, dried over anhydrous sodium sulfate, and filtered. The filtrate was concentrated to give a crude product of intermediate 16j (10.13 g).

MS (ESI, $[M+H]^-$) *m/z:* 317.20
$^1$H NMR (500 MHz, DMSO-$d_6$) δ 12.59 (s, 1H), 7.78 (d, *J* = 8.2 Hz, 1H), 7.38 (t, *J* = 7.6 Hz, 1H), 4.85 (dt, *J* = 13.5, 2.5 Hz, 2H), 4.75 (dt, *J* = 12.2, 2.4 Hz, 2H), 4.11 (s, 2H), 1.49 (d, *J* = 2.1 Hz, 9H).

Step 10: Preparation of intermediate **16k**

**16j** (10.13 g), potassium carbonate (12.55 g), *N*,*N*-dimethylacetamide (150 mL), and iodoethane (7.08 g) were added to a reaction flask in sequence, and the mixture was stirred and reacted at 80 °C. After the reaction was completed, as confirmed by TLC, the reaction solution was poured into water, vigorously stirred, extracted with ethyl acetate, washed with saturated brine, dried over anhydrous sodium sulfate, and filtered. The filtrate was concentrated, and the crude product was separated by silica gel column chromatography and purified to give (11.22 g) net weight of intermediate **16k.**

MS (ESI, $[M+H]^+$) *m/z:* 247.16
$^1$H NMR (500 MHz, DMSO-$d_6$)δ 7.78 (d, *J* = 8.0 Hz, 1H), 7.39 (t, *J* = 7.5 Hz, 1H), 4.89 - 4.82 (m, 2H), 4.75 (dd, *J* = 11.8, 2.8 Hz, 2H), 4.22 (d, *J* = 4.3 Hz, 2H), 4.13 (p, *J* = 7.2 Hz, 2H), 1.48 (d, *J* = 2.1 Hz, 9H), 1.21 - 1.17 (m, 3H).

Step 11: Preparation of intermediate **16l**

Acrylamide (1.32 g) was slowly added to a stirred solution of **16k** (10.72 g) in tetrahydrofuran (50 mL) at 0 °C under $N_2$ atmosphere, a 1 M solution of potassium *tert*-butoxide in tetrahydrofuran (18.63 mL) was then added dropwise, and the mixture was stirred and reacted at 0 °C. After the reaction was completed, as confirmed by TLC, the reaction solution was poured into an aqueous ammonium chloride solution, vigorously stirred, extracted with ethyl acetate, washed with saturated brine, dried over anhydrous sodium sulfate, and filtered. The filtrate was concentrated, and the crude

product was separated by silica gel column chromatography and purified to give (7.52 g) net weight of intermediate **16l**.

MS (ESI, $[M+H]^+$) *m/z:* 272.02

$^1$H NMR (500 MHz, DMSO-$d_6$) $\delta$ 11.11 (s, 1H), 7.80 (dd, *J* = 8.1, 2.7 Hz, 1H), 7.37 (t, *J* = 7.9 Hz, 1H), 4.85 (dd, *J* = 12.2, 2.6 Hz, 2H), 4.75 (dt, *J* = 12.0, 2.3 Hz, 2H), 4.63 (dd, *J* = 12.1, 4.9 Hz, 1H), 2.78 (ddd, *J* = 17.3, 12.1, 5.3 Hz, 1H), 2.67 - 2.51 (m, 2H), 2.20 (dq, *J* = 13.5, 4.7 Hz, 1H), 1.49 (d, *J* = 2.6 Hz, 9H).

Step 12: Preparation of compound **16**

**16l** (1.00 g), a solution of 4 M hydrochloric acid in dioxane (10 mL), and ethyl acetate (50 mL) were added to a reaction flask in sequence, and the mixture was reacted at 25 °C. After the reaction was completed, as confirmed by TLC, the reaction solution was directly filtered, and the filter cake was washed with ethyl acetate and dried to give 0.83 g of compound **16.**

MS (ESI, $[M+H]^+$) *m/z:* 272.11

$^1$H NMR (500 MHz, DMSO-*$d_6$*) $\delta$ 11.13 (s, 1H), 10.36 (s, 2H), 7.90 (d, *J* = 8.1 Hz, 1H), 7.45 (d, *J* = 8.2 Hz, 1H), 4.81 (s, 2H), 4.70 - 4.63 (m, 3H), 2.79 (ddd, *J* = 17.4, 12.2, 5.3 Hz, 1H), 2.62 (dt, *J* = 17.3, 4.0 Hz, 1H), 2.59 - 2.52 (m, 1H), 2.21 (ddt, *J* = 13.2, 5.1, 2.5 Hz, 1H).

**Example 17:** Preparation of Compound **17**

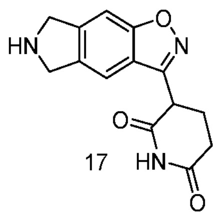

17

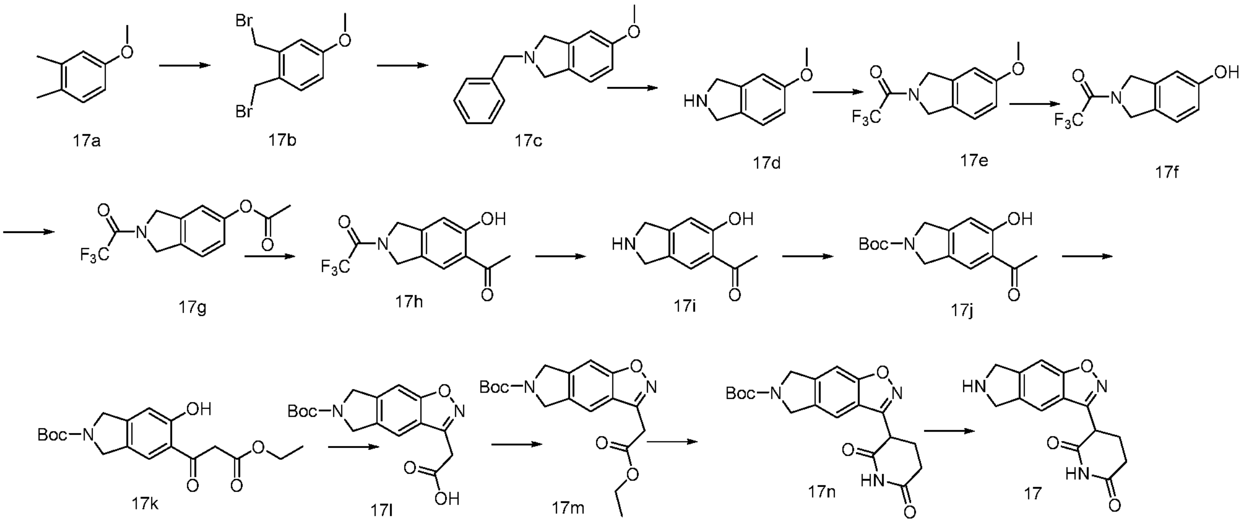

17a 17b 17c 17d 17e 17f 17g 17h 17i 17j 17k 17l 17m 17n 17

Step 1: Preparation of intermediate **17b**

**17a** (18 g), AIBN (0.738 g), carbon tetrachloride (500 mL), and NBS (47.8 g) were added to a reaction flask in sequence, and the mixture was warmed to 60 °C and reacted. After the reaction was completed, as confirmed by TLC, the reaction solution was cooled to room temperature and concentrated by evaporation at reduced pressure to remove the solvent, and dichloromethane was added to the residue. The mixture was washed with saturated brine, dried over anhydrous sodium sulfate, and filtered. The filtrate was concentrated by evaporation at reduced pressure to remove the solvent and purified by silica gel column chromatography to give intermediate **17b** (18.7 g).

Step 2: Preparation of intermediate **17c**

**17b** (18.7 g), benzylamine (1.78 mL), *N*,*N*-diisopropylethylamine (7.13 mL), and toluene (50 mL) were added to a reaction flask in sequence, and the mixture was warmed to 50 °C and reacted. After the reaction was completed, as confirmed by TLC, the reaction solution was cooled to room temperature. After the reaction was completed, the stirring was stopped, and the reaction was carried out at room temperature for a total of 2 h. Ethyl acetate and 1 M solution of HCl in ice water were added to the reaction solution for extraction. The aqueous phase was collected, the pH was adjusted to about 9 with sodium bicarbonate solid, and ethyl acetate was added for extraction. The organic phase was collected, dried over anhydrous sodium sulfate, filtered, and concentrated by evaporation at reduced pressure to remove the solvent, thus giving intermediate **17c** (9.6 g).

MS(ESI, $[M+H]^+$) *m*/*z:* 240.1.

Step 3: Preparation of intermediate **17d**

**17c** (9.6 g), methanol (200 mL), 10% palladium on carbon (5 g), and toluene (50 mL) were added to a reaction flask in sequence, and the mixture was purged 3 times with hydrogen and reacted at room temperature under hydrogen atmosphere. The mixture was reacted at room temperature. After the reaction was completed, as confirmed by TLC, the palladium on carbon was filtered, and the filter cake was washed 2 times with methanol. The filtrate was collected and concentrated by evaporation at reduced pressure to remove the solvent, thus giving **17d** (4.5 g).

MS(ESI, $[M+H]^+$) *m*/*z:* 149.9.

Step 4: Preparation of intermediate **17e**

**17d** (4 g), tetrahydrofuran (50 mL), and trifluoroacetic anhydride (5.63 g) were added to a reaction flask in sequence, and the mixture was reacted at room temperature. After the reaction was completed, as confirmed by TLC, an aqueous solution was added to the reaction solution to quench the reaction, and ethyl acetate was then added for extraction. The organic phase was separated, dried over anhydrous sodium sulfate, and purified by silica gel column chromatography to give **17e** (4.58 g).

$^1H$ NMR (500 MHz, DMSO-*d*6) δ 7.29 (t, *J* = 9.3 Hz, 1H), 6.98 (d, *J* = 8.6 Hz, 1H), 6.93 - 6.86 (m, 1H), 4.97 (d, *J* = 21.7 Hz, 2H), 4.77 (dd, *J* = 21.4, 5.2 Hz, 2H), 3.76 (dd, *J* = 3.6, 1.6 Hz, 3H).

Step 5: Preparation of intermediate **17f**

**17e** (4.6 g), dichloromethane (200 mL), and boron tribromide (1 M, 18.76 mL) were added to a reaction flask in sequence, and the mixture was reacted at room temperature. After the reaction was completed, as confirmed by TLC, water was added to the reaction solution under an ice bath to quench the reaction. The organic phase was separated, and the aqueous phase was extracted with dichloromethane. The organic phases were combined, dried over anhydrous sodium sulfate, and concentrated by evaporation at reduced pressure to remove the solvent, thus giving **17f** (4.2 g).

$^1H$ NMR (500 MHz, DMSO-d6) δ 7.16 (t, *J* = 8.8 Hz, 1H), 6.87 - 6.59 (m, 2H), 4.92 (d, *J* = 20.8 Hz, 2H), 4.72 (d, *J* = 19.9 Hz, 2H).

Step 6: Preparation of intermediate **17g**

**17f** (6.5 g), dichloromethane (60 mL), triethylamine (7.8 mL), and acetic anhydride (2.94 mL) were added to a reaction flask in sequence, and the mixture was reacted at room temperature. After the reaction was completed, as confirmed by TLC, dichloromethane and water were added to the reaction solution. The organic phase was separated, washed with saturated brine, dried over anhydrous sodium sulfate, filtered, and concentrated by evaporation at reduced pressure to remove the solvent, thus giving **17g** (7 g).

$^1H$ NMR (500 MHz, DMSO-d6) δ 7.42 (dd, *J* = 10.0, 8.3 Hz, 1H), 7.17 (dd, *J* = 14.0, 2.1 Hz, 1H), 7.09 (d, *J* = 8.2 Hz, 1H), 5.03 (d, *J* = 6.6 Hz, 2H), 4.83 (d, *J* = 6.9 Hz, 2H), 2.27 (s, 3H).

Step 7: Preparation of intermediate **17h**

**17g** (6 g) and aluminum trichloride (4.39 g) were added to a reaction flask in sequence, and the mixture was gradually heated from room temperature to 150 °C and reacted. After the reaction was completed, as confirmed by TLC, the reaction solution was cooled to room temperature, and water and a 3 M aqueous hydrochloric acid solution were added to the residue. The organic phase was separated, washed with saturated brine, dried over anhydrous sodium sulfate, and filtered. The filtrate was concentrated under reduced pressure to remove the solvent and purified by silica

gel column chromatography to give **17h** (3.78 g).

MS(ESI, $[M-H]^-$) *m/z:* 271.9.

Step 7: Preparation of intermediate **17j**

**17h** (550 mg), MeOH (5.00 mL), and an aqueous solution (5.00 mL) of sodium hydroxide (242 mg) were added to a reaction flask in sequence, and the mixture was reacted at room temperature. After the reaction was completed, as confirmed by TLC, the reaction solution was concentrated to remove methanol, and the aqueous phase was retained. **17i** was obtained. 1,4-Dioxane (5 mL) and Boc anhydride (439 mg, 0.462 mL) were added to the system, and the mixture was reacted at room temperature. After the reaction was completed, as confirmed by TLC, the reaction solution was extracted with ethyl acetate and saturated brine, and the organic phase was dried over anhydrous sodium sulfate, filtered, and concentrated to give **17j** (200 mg).

$^1$H NMR (500 MHz, DMSO-d6) δ 12.11 (d, *J* = 19.3 Hz, 1H), 7.88 (d, *J* = 9.5 Hz, 1H), 6.74 - 6.56 (m, 1H), 4.60 - 4.50 (m, 4H), 2.63 (d, *J* = 6.5 Hz, 3H), 1.45 (s, 9H).

Step 8: Preparation of intermediate **17k**

**17j** (3.5 g) and THF (300 mL) were added to a reaction flask in sequence, and diethyl carbonate (14.91 g, 15.29 mL) was added. The mixture was cooled to about 0 °C, and 60 wt% sodium hydride (5.05 g, 126 mmol) was added in portions. The mixture was heated to 85 °C and reacted. After the reaction was completed, as confirmed by TLC, the reaction solution was cooled to room temperature and slowly poured into ice water. The mixture was extracted with ethyl acetate, and the organic phase was discarded. The aqueous phase was adjusted to pH = 1-2 with 3 M hydrochloric acid, then extracted with ethyl acetate, dried over anhydrous sodium sulfate, filtered, and concentrated by evaporation at reduced pressure to remove the solvent, thus giving **17k** (7.0 g).

MS(ESI, $[M-H]^-$) *m/z:* 348.3.

Step 9: Preparation of intermediate **17l**

**17k** (4.4 g), an aqueous hydroxylamine solution (4.16 g, 63.0 mmol), and ethanol (50 mL) were added to a reaction flask in sequence, and the mixture was reacted at 85 °C. After the reaction was completed, as confirmed by TLC, the reaction solution was cooled to room temperature, ethyl acetate and a saturated aqueous sodium carbonate solution were added to the residue for extraction, and the organic phase was discarded. The aqueous phase was adjusted to pH = 2-3 with a 1 M aqueous HCl solution, extracted with ethyl acetate, dried over anhydrous sodium sulfate, filtered, and concentrated by evaporation at reduced pressure to remove the solvent, thus giving **17l** (3.25 g).

$^1$H NMR (500 MHz, DMSO-$d_6$) δ 12.83 (s, 1H), 7.74 (d, *J* = 13.0 Hz, 1H), 7.68 (d, *J* = 3.5 Hz, 1H), 4.71 (d, *J* = 13.6 Hz, 2H), 4.66 (d, *J* = 11.4 Hz, 2H), 4.07 (s, 2H), 1.47 (s, 9H).

Step 10: Preparation of intermediate **17m**

**17l** (3.14 g), potassium carbonate (1.500 g), DMA (5 mL), and iodoethane (2.308 g, 1.183 mL) were added to a reaction flask in sequence, and the mixture was heated to 80 °C and reacted. After the reaction was completed, as confirmed by TLC, the reaction solution was cooled to room temperature and poured into a mixed solution of ethyl acetate and water. The organic phase was separated, dried over anhydrous sodium sulfate, and filtered. The filtrate was concentrated by evaporation at reduced pressure to remove the solvent, thus giving **17m** (2.47 g).

$^1$H NMR (500 MHz, DMSO-$d_6$) δ 7.73 (d, *J* = 15.9 Hz, 1H), 7.69 (d, *J* = 3.5 Hz, 1H), 4.69 (dd, *J* = 23.8, 12.4 Hz, 4H), 4.19 - 4.11 (m, 4H), 1.47 (s, 9H), 1.20 (t, *J* = 7.1 Hz, 3H).

Step 11: Preparation of intermediate **17n**

**17m** (1.5 g) and THF (75 mL) were added to a reaction flask in sequence, acrylamide (0.215 g) was added, and the mixture was cooled to about -15 °C. A 1 M solution of potassium *tert*-butoxide in tetrahydrofuran (2.60 mL) was added, and the system was warmed to 0 °C and reacted. After the reaction was completed, as confirmed by TLC, the mixture was added dropwise to an ammonium chloride solution to quench the reaction and extracted with ethyl acetate. The organic phase was separated, dried over anhydrous sodium sulfate, and filtered, and the filtrate was concentrated and purified by silica gel column chromatography to give **17n** (0.88 g).

$^1$H NMR (500 MHz, DMSO-$d_6$) δ 11.11 (d, *J* = 3.4 Hz, 1H), 7.78 (d, *J* = 12.4 Hz, 1H), 7.70 (s, 1H), 4.68 (dd, *J* = 29.4, 13.3 Hz, 4H), 4.58 (dd, *J* = 12.0, 4.9 Hz, 1H), 2.79 (ddd, *J* = 17.3, 12.1, 5.3 Hz, 1H), 2.62 (dt, *J* = 17.3, 4.1 Hz, 1H), 2.56 - 2.50 (m, 1H), 2.31 - 2.14 (m, 1H), 1.47 (d, *J* = 1.5 Hz, 9H).

Step 12: Preparation of compound **17**

**17n** (0.428 g) and dichloromethane (10.00 mL) were added to a reaction flask in sequence, then trifluoroacetic acid (3.29 g, 2.211 mL) was added, and the mixture was reacted at room temperature. After the reaction was completed, as confirmed by TLC, water was added to the reaction solution, and the pH was adjusted to 7-8 with saturated sodium bicarbonate. The mixture was extracted with dichloromethane, dried over anhydrous sodium sulfate, and subjected to rotary evaporation to remove the solvent, thus giving **17** (0.439 g).

MS(ESI, $[M+H]^+$) *m/z:* 272.24.

$^1H$ NMR (500 MHz, DMSO-d6) δ 7.81 - 7.77 (m, 1H), 7.74 (s, 1H), 4.59 (dd, *J* = 12.1, 5.0 Hz, 1H), 4.49 (s, 2H), 4.43 (s, 2H), 2.79 (ddd, *J* = 17.4, 12.2, 5.3 Hz, 1H), 2.62 (dt, *J* = 17.3, 4.0 Hz, 1H), 2.47 (dd, *J* = 12.4, 4.4 Hz, 1H), 2.20 (ddt, *J* = 13.3, 5.2, 2.6 Hz, 1H).

Example 18: Synthesis of Compound **18**

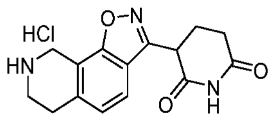

**18**

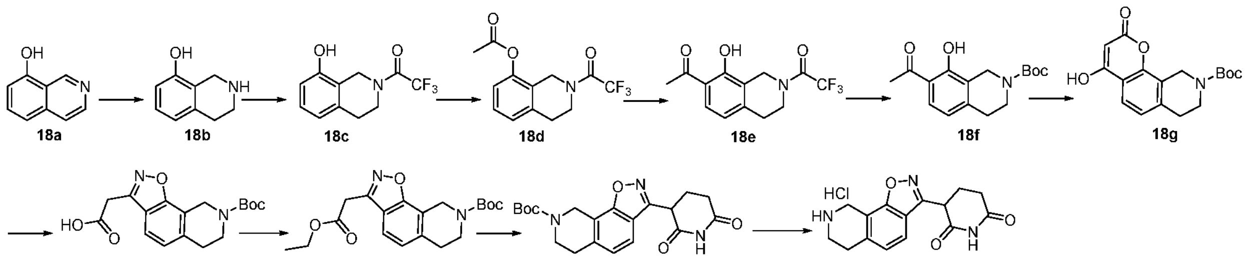

18a 18b 18c 18d 18e 18f 18g 18h 18i 18j 18

Step 1: Preparation of intermediate **18b**

Intermediate **18a** (25 g) was completely dissolved in methanol (1000 mL) and acetic acid (103 g, 99 mL) and then added to a high pressure reactor under a hydrogen pressure of 3 Mpa and a temperature of 110 °C. After the reaction was completed, as confirmed by TLC, the reaction solution was concentrated by evaporation at reduced pressure to remove the solvent, and a solution of hydrochloric acid in dioxane (4 mol/L, 100 mL, 400 mmol) was added to the residue. The mixture was concentrated by evaporation at reduced pressure to remove the solvent, the residue was slurried with ethyl acetate and filtered, and the filter cake was collected to give the target intermediate **18b** (28.97 g).

MS(ESI, $[M+H]^+$) m/z: 150.0.

1H NMR (500 MHz, DMSO-d6) δ 10.01 (s, 1H), 7.06 (t, J = 7.8 Hz, 1H), 6.76 (d, J = 8.0 Hz, 1H), 6.64 (d, J = 7.6 Hz, 1H), 4.01 (t, J = 4.9 Hz, 2H), 3.33 - 3.25 (m, 2H), 2.94 (t, J = 6.2 Hz, 2H).

Step 2: Preparation of intermediate **18c**

Intermediate **18b** (28.97 g) and tetrahydrofuran (300 mL) were added to a reaction flask in sequence, and trifluoroacetic anhydride (27.0 mL) was added under an ice bath. The mixture was reacted at room temperature. After the reaction was completed, as confirmed by TLC, the reaction solution was extracted with ethyl acetate and water. The organic phase was separated, and the aqueous phase was extracted twice with ethyl acetate. The organic phases were combined, washed with saturated brine, dried over anhydrous sodium sulfate, filtered, and concentrated by evaporation at reduced pressure to remove the solvent, and the filter cake was collected by filtration to give the target intermediate **18c** (22.67 g).

MS(ESI, $[M+H]^-$) *m/z:* 244.0.

$^1H$ NMR (500 MHz, DMSO-d6) δ 7.03 (q, J = 7.9 Hz, 1H), 6.74 - 6.68 (m, 1H), 6.64 (t, J = 6.9 Hz, 1H), 4.61 (d, J = 23.5 Hz, 2H), 3.78 (td, J = 6.0, 3.7 Hz, 2H), 2.84 (dt, J = 16.8, 5.9 Hz, 2H).

Step 3: Preparation of intermediate **18d**

Intermediate **18c** (22.67 g), dichloromethane (200 mL), triethylamine (28.1 g, 38.6 mL), and DMAP (0.282 g, 2.311 mmol) were added to a reaction flask in sequence, and acetic anhydride (10.38 g, 9.68 mL) was added under an ice bath. The mixture was allowed to return to room temperature and react. After the reaction was completed, as confirmed by TLC, the reaction solution was concentrated by evaporation at reduced pressure to remove the solvent, and the residue was extracted with ethyl acetate and water. The organic phase was separated, washed with a saturated ammonium chloride solution and saturated brine, dried over anhydrous sodium sulfate, and filtered, and the filtrate was concentrated by evaporation at reduced pressure to remove the solvent, thus giving the target intermediate **18d** (21.94 g).

$^1$H NMR (500 MHz, DMSO-d6) δ 7.30 (dt, J = 11.1, 7.8 Hz, 1H), 7.18 - 7.11 (m, 1H), 7.05 (dt, J = 8.0, 2.2 Hz, 1H), 4.59 (s, 2H), 3.81 (q, J = 6.1 Hz, 2H), 2.95 (dt, J = 10.1, 6.0 Hz, 2H), 2.33 (d, J = 9.5 Hz, 3H).

Step 4: Preparation of intermediate **18e**

Intermediate **18d** (21 g) and aluminum trichloride (14.62 g) were added to a reaction flask in sequence and the mixture was heated to 170 °C and reacted under $N_2$ atmosphere. After the reaction was completed, as confirmed by TLC, the reaction solution was cooled to room temperature, water was added to quench the reaction, and dichloromethane was added for extraction. The organic phase was separated, washed with 500 mL of saturated brine, dried over anhydrous sodium sulfate, and filtered, and the filtrate was purified by silica gel column chromatography to give the target intermediate **18e** (10.16 g).

MS(ESI, $[M+H]^-$) *m/z:* 286.0.

$^1$H NMR (500 MHz, DMSO-d6) δ 12.76 (d, J = 8.4 Hz, 1H), 7.83 (t, J = 8.8 Hz, 1H), 6.86 (dd, J = 8.3, 5.7 Hz, 1H), 4.67 (d, J = 25.1 Hz, 2H), 3.86 - 3.78 (m, 2H), 2.94 (dt, J = 13.3, 5.9 Hz, 2H), 2.64 (d, J = 1.2 Hz, 3H).

Step 5: Preparation of intermediate **18f**

Intermediate **18e** (10.16 g) and MeOH (100 mL) were added to a reaction flask in sequence, and a solution of sodium hydroxide (4.24 g) in water (100 mL) was added dropwise under an ice bath. The mixture was allowed to return to room temperature and react. After the reaction was completed, as confirmed by TLC, the reaction solution was concentrated by evaporation at reduced pressure to remove methanol, and 1,4-dioxane (100 mL) and di-*tert*-butyl dicarbonate (8.49 g, 9.03 mL) were added. The mixture was allowed to return to room temperature and react. After the reaction was completed, as confirmed by TLC, the reaction solution was extracted with ethyl acetate and water. The organic phase was separated, washed with 1000 mL of saturated brine, dried over anhydrous sodium sulfate, and filtered, and the filtrate was concentrated by evaporation at reduced pressure to remove the solvent, thus giving the target intermediate **18f** (8.96 g).

MS(ESI, $[M+H]^+$) m/z: 192.0.

$^1$H NMR (500 MHz, DMSO-d6) δ 12.72 (s, 1H), 7.76 (d, J = 8.2 Hz, 1H), 6.80 (d, J = 8.2 Hz, 1H), 4.41 (s, 2H), 3.55 (t, J = 5.8 Hz, 2H), 2.80 (t, J = 5.8 Hz, 2H), 2.63 (s, 3H), 1.43 (s, 9H).

Step 6: Preparation of intermediate **18g**

Intermediate **18f** (8.76 g), diethyl carbonate (17.76 g, 18.21 mL), and toluene (90 mL) were added to a reaction flask in sequence, 60 wt% sodium hydride (6.01 g, 150 mmol) was added in portions under an ice bath. The mixture was heated to 120 °C and reacted. After the reaction was completed, as confirmed by TLC, the reaction solution was cooled to room temperature and poured into ice water to quench the reaction. The pH was adjusted to 1-2 with a 1 M hydrochloric acid solution, and ethyl acetate was added for extraction. The organic phase was separated, washed with 600 mL of saturated brine, dried over anhydrous sodium sulfate, and filtered, and the filtrate was concentrated by evaporation at reduced pressure to remove the solvent. The residue was slurried with petroleum ether and filtered, and the filter cake was collected to give the target intermediate **18g** (12.03 g).

Step 7: Preparation of intermediate **18h**

Intermediate **18g** (9.54 g), EtOH (100 mL), and an aqueous hydroxylamine solution (9.93 g, 9.21 mL, 150 mmol) were added to a reaction flask in sequence, and the mixture was heated to 85 °C and reacted. After the reaction was completed, as confirmed by TLC, a saturated sodium carbonate solution was added to the reaction solution to adjust the pH to 8, and ethyl acetate was added for extraction. The organic phase was separated and extracted twice with water. Then the organic phases were combined, the pH was adjusted to 3 with 1 M hydrochloric acid, and ethyl acetate was added for extraction three times. The organic phases were combined, washed with saturated brine, dried over

anhydrous sodium sulfate, and filtered, and the filtrate was concentrated by evaporation at reduced pressure to remove the solvent, thus giving the target intermediate **18h** (9.01 g).

MS(ESI, $[M+H]^-$) *m/z*: 331.0.

Step 8: Preparation of intermediate **18i**

Intermediate **18h** (9.01 g), potassium carbonate (11.24 g), DMA (90 mL), and iodoethane (5.07 g, 2.63 mL, 32.5 mmol) were added to a reaction flask in sequence, and the mixture was heated to 80 °C and reacted under $N_2$ atmosphere. After the reaction was completed, as confirmed by TLC, the reaction solution was cooled to room temperature and extracted with ethyl acetate and water. The organic phase was separated, washed with saturated brine, dried over anhydrous sodium sulfate, and filtered, and the filtrate was purified by silica gel column chromatography to give the target intermediate **18i** (7.33 g).

MS(ESI, $[M+H]^+$) m/z: 361.0.

$^1$H NMR (500 MHz, DMSO-d6) δ 7.63 (d, J = 8.1 Hz, 1H), 7.21 (d, J = 8.2 Hz, 1H), 4.76 (s, 2H), 4.18 (s, 2H), 4.13 (q, J = 7.1 Hz, 2H), 3.66 (t, J = 5.8 Hz, 2H), 2.93 (t, J = 5.8 Hz, 2H), 1.45 (s, 9H), 1.19 (t, J = 7.1 Hz, 3H).

Step 9: Preparation of intermediate **18j**

Intermediate **18i** (7.3 g), tetrahydrofuran (80 mL), and acrylamide (0.864 g, 12.15 mmol) were added to a reaction flask in sequence, the mixture was cooled to -15 °C under $N_2$ atmosphere, and then a solution of potassium *tert*-butoxide in tetrahydrofuran (1 mol/L, 11.14 mL, 11.14 mmol) was added dropwise. After the dropwise addition was completed, the mixture was warmed to 0 °C and reacted. After the reaction was completed, as confirmed by TLC, the system was added to a saturated ammonium chloride solution and extracted with ethyl acetate. The organic phase was separated, dried over anhydrous sodium sulfate, and filtered, and the filtrate was purified by silica gel column chromatography to give the target intermediate **18j** (4.54 g).

MS(ESI, $[M+H]^-$) *m/z:* 384.3

$^1$H NMR (500 MHz, DMSO-d6) δ 11.09 (s, 1H), 7.65 (d, J = 8.1 Hz, 1H), 7.20 (d, J = 8.2 Hz, 1H), 4.76 (s, 2H), 4.58 (dd, J = 12.0, 5.0 Hz, 1H), 3.66 (t, J = 5.8 Hz, 2H), 2.93 (t, J = 5.8 Hz, 2H), 2.77 (ddd, J = 17.3, 12.1, 5.3 Hz, 1H), 2.61 (dt, J = 17.3, 4.1 Hz, 1H), 2.54 (d, J = 4.5 Hz, 1H), 2.18 (dtd, J = 13.5, 5.2, 3.6 Hz, 1H), 1.45 (s, 9H).

Step 10: Preparation of compound **18**

Intermediate **18j** (300 mg) and ethyl acetate (5 mL) were added to a reaction flask in sequence, and a solution of hydrochloric acid in dioxane (4 moL/L, 3.89 mL, 15.58 mmol) was added. The mixture was reacted at room temperature. After the reaction was completed, as confirmed by TLC, the reaction solution was concentrated to give compound **18** (235 mg).

MS(ESI, $[M+H]^+$) m/z: 286.10.

$^1$H NMR (500 MHz, DMSO-d6) δ 11.08 (s, 1H), 7.55 (d, J = 8.1 Hz, 1H), 7.10 (d, J = 8.2 Hz, 1H), 4.55 (dd, J = 11.9, 5.0 Hz, 1H), 4.07 (s, 2H), 3.00 (t, J = 5.7 Hz, 2H), 2.81 (t, J = 5.7 Hz, 2H), 2.78 - 2.71 (m, 1H), 2.60 (dt, J = 17.3, 4.2 Hz, 1H), 2.46 (dd, J = 12.2, 4.5 Hz, 1H), 2.18 (dq, J = 13.6, 4.9 Hz, 1H).

**Example 19:** Synthesis of Compound **19**

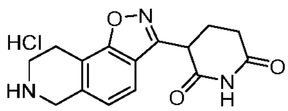

**19**

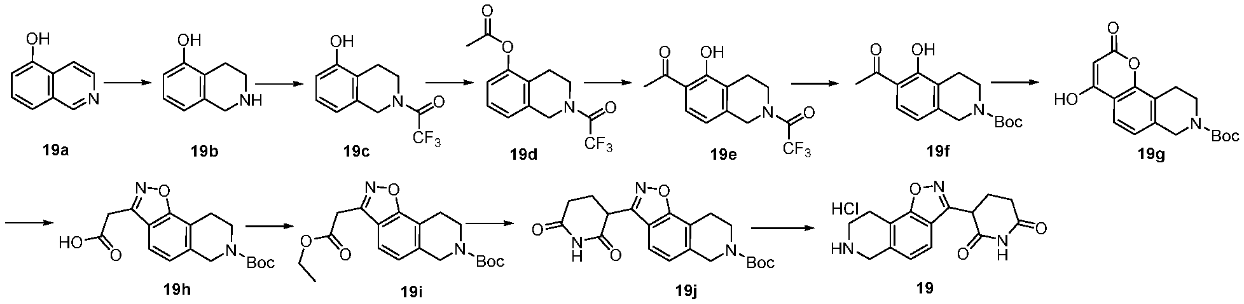

19a 19b 19c 19d 19e 19f 19g 19h 19i 19j 19

Step 1: Preparation of intermediate **19b**

Intermediate **19a** was completely dissolved in methanol (1680 mL) and acetic acid (166 mL) and then added to a high pressure reactor under a hydrogen pressure of 3 Mpa and a temperature of 110 °C. After the reaction was completed, as confirmed by TLC, the reaction solution was concentrated by evaporation at reduced pressure to remove the solvent, and a solution of hydrochloric acid in dioxane (4 moL/L, 200 mL, 798 mmol) was added to the residue. The mixture was concentrated by evaporation at reduced pressure to remove the solvent, the residue was slurried with ethyl acetate and filtered, and the filter cake was collected to give the target intermediate **19b** (46.96 g).
MS(ESI, $[M+H]^+$) m/z: 150.0.
$^1H$ NMR (500 MHz, DMSO-d6) δ 9.81 (s, 1H), 7.03 (t, J = 7.7 Hz, 1H), 6.78 (d, J = 7.8 Hz, 1H), 6.62 (d, J = 7.6 Hz, 1H), 4.15 (s, 2H), 3.35 (s, 2H), 2.79 (s, 2H).

Step 2: Preparation of intermediate **19c**

Intermediate **19b** (40 g) and tetrahydrofuran (400 mL) were added to a reaction flask in sequence, and trifluoroacetic anhydride (61.9 g, 41.0 mL, 295 mmol) was added under an ice bath. The mixture was allowed to return to room temperature and react. After the reaction was completed as confirmed by TLC, the reaction solution was extracted with ethyl acetate and water. The organic phase was separated, washed with 1000 mL of saturated brine, dried over anhydrous sodium sulfate, and filtered, and the filtrate was concentrated by evaporation at reduced pressure to remove the solvent, thus giving the target intermediate **19c** (81 g).
MS(ESI, $[M+H]^-$) *m/z*: 244.04.

Step 3: Preparation of intermediate **19d**

Intermediate **19c** (65.3 g), dichloromethane (650 mL), triethylamine (81 g, 111 mL, 799 mmol), and DMAP (0.813 g) were added to a reaction flask in sequence, and acetic anhydride (29.9 g, 27.9 mL, 293 mmol) was added under an ice bath. The mixture was allowed to return to room temperature and react. After the reaction was completed, as confirmed by TLC, the reaction solution was concentrated by evaporation at reduced pressure to remove the solvent, and the residue was extracted with ethyl acetate and water. The organic phase was separated, washed with a saturated ammonium chloride solution and saturated brine, dried over anhydrous sodium sulfate, and filtered, and the filtrate was concentrated by evaporation at reduced pressure to remove the solvent, thus giving the target intermediate **19d** (55.6 g).
$^1H$ NMR (500 MHz, Chloroform-d) δ 7.28 (d, J = 7.9 Hz, 1H), 7.10 - 7.02 (m, 1H), 7.02 - 6.95 (m, 1H), 4.79 (d, J = 27.0 Hz, 2H), 3.92 - 3.78 (m, 2H), 2.82 - 2.72 (m, 2H), 2.33 (d, J = 2.0 Hz, 3H).

Step 4: Preparation of intermediate **19e**

Intermediate **19d** (30.73 g) and aluminum trichloride (21.40 g) were added to a reaction flask in sequence and the mixture was heated to 170 °C and reacted under $N_2$ atmosphere. After the reaction was completed, as confirmed by TLC, the reaction solution was cooled to room temperature, water was added to quench the reaction, and dichloromethane was added for extraction. The organic phase was separated, and the aqueous phase was extracted twice with dichloromethane. The organic phases were combined, washed with saturated brine, dried over anhydrous sodium sulfate, and filtered, and the filtrate was purified by silica gel column chromatography to give the target intermediate **19e** (16.21 g).
MS(ESI, $[M+H]^-$) *m/z:* 286.0.
1H NMR (500 MHz, DMSO-d6) δ 12.74 (d, J = 11.3 Hz, 1H), 7.82 (d, J = 8.3 Hz, 1H), 6.91 (dd, J = 14.9, 8.3 Hz, 1H), 4.80 (d, J = 9.9 Hz, 2H), 3.86 (dt, J = 8.2, 5.9 Hz, 2H), 2.77 (dt, J = 17.7, 6.1 Hz, 2H), 2.64 (s, 3H).

Step 5: Preparation of intermediate **19f**

Intermediate **19e** (15.7 g) and MeOH (160 mL) were added to a reaction flask in sequence, and a solution of sodium hydroxide (6.56 g) in water (160 mL) was added dropwise under an ice bath. The mixture was allowed to return to room temperature and react. After the reaction was completed, as confirmed by TLC, the reaction solution was concentrated by evaporation at reduced pressure to remove methanol, and 1,4-dioxane (160 mL) and di-tert-butyl dicarbonate (13.12 g, 13.96 mL) were added. The mixture was reacted at room temperature. After the reaction was completed, as confirmed by TLC, the reaction solution was extracted with ethyl acetate and water. The organic phase was separated, and the aqueous phase was extracted twice with ethyl acetate. The organic phases were combined, washed with saturated brine, dried over anhydrous sodium sulfate, and filtered, and the filtrate was concentrated by evaporation at reduced pressure to remove the solvent, thus giving the target intermediate **19f** (19.83 g).
MS(ESI, $[M+H]^+$) m/z: 192.0.
$^1H$ NMR (500 MHz, DMSO-d6) δ 12.76 (s, 1H), 7.76 (d, J = 8.3 Hz, 1H), 6.80 (d, J = 8.3 Hz, 1H), 4.52 (s, 2H), 3.57 (d, J = 1.8 Hz, 2H), 2.64 (d, J = 6.1 Hz, 5H), 1.43 (s, 9H).

Step 6: Preparation of intermediate **19g**

Intermediate **19f** (15.92 g, 54.6 mmol), diethyl carbonate (32.3 g, 33.1 mL), and toluene (200 mL) were added to a reaction flask in sequence, 60 wt% sodium hydride (10.93 g, 273 mmol) was added in portions under an ice bath. The mixture was heated to 120 °C and reacted. After the reaction was completed, as confirmed by TLC, the reaction solution was cooled to room temperature and poured into ice water to quench the reaction. The pH was adjusted to 1-2 with a 1 M hydrochloric acid solution, and ethyl acetate was added for extraction. The organic phase was separated, and the aqueous phase was extracted twice with ethyl acetate. The organic phases were combined, washed with saturated brine, dried over anhydrous sodium sulfate, and filtered, and the filtrate was concentrated by evaporation at reduced pressure to remove the solvent. The residue was slurried with petroleum ether and filtered, and the filter cake was collected to give the target intermediate **19g** (12 g).
$^1H$ NMR (500 MHz, DMSO-d6) δ 12.46 (s, 1H), 7.64 (d, J = 8.1 Hz, 1H), 7.17 (d, J = 8.2 Hz, 1H), 5.56 (s, 1H), 4.60 (s, 2H), 3.62 (t, J = 6.0 Hz, 2H), 2.83 (t, J = 5.8 Hz, 2H), 1.44 (s, 9H).

Step 7: Preparation of intermediate **19h**

Intermediate **19g** (12 g), EtOH (120 mL), and an aqueous hydroxylamine solution (12.49 g, 11.59 mL, 189 mmol) were added to a reaction flask in sequence, and the mixture was heated to 85 °C and reacted. After the reaction was completed, as confirmed by TLC, a saturated sodium bicarbonate solution was added to the reaction solution to adjust the pH to 8, and ethyl acetate was added for extraction. The organic phase was separated, and the aqueous phase was adjusted to pH of 3 with 1 M hydrochloric acid and extracted three times with ethyl acetate. The organic phases were combined, washed with saturated brine, dried over anhydrous sodium sulfate, and filtered, and the filtrate was concentrated by evaporation at reduced pressure to remove the solvent, thus giving the target intermediate **19h** (10.44 g).
MS(ESI, $[M+H]^-$) *m*/*z*: 331.0.
$^1H$ NMR (500 MHz, DMSO-d6) δ 12.85 (s, 1H), 7.63 (d, J = 8.1 Hz, 1H), 7.21 (d, J = 8.2 Hz, 1H), 4.67 (s, 2H), 4.07 (s, 2H), 3.69 (t, J = 5.9 Hz, 2H), 2.98 (t, J = 5.9 Hz, 2H), 1.44 (s, 9H).

Step 8: Preparation of intermediate **19i**

Intermediate **19h** (10.44 g), potassium carbonate (13.02 g), DMA (110 mL), and iodoethane (5.88 g, 3.05 mL, 37.7 mmol) were added to a reaction flask in sequence, and the mixture was heated to 80 °C and reacted under $N_2$ atmosphere. After the reaction was completed, as confirmed by TLC, the reaction solution was cooled to room temperature and extracted with ethyl acetate and water. The organic phase was separated, washed with 800 mL of saturated brine, dried over anhydrous sodium sulfate, and filtered, and the filtrate was purified by silica gel column chromatography to give the target intermediate **19i** (8.31 g).
MS(ESI, $[M+H]^+$) m/z: 361.2.

Step 9: Preparation of intermediate **19j**

Intermediate **19i** (5.5 g), tetrahydrofuran (50 mL), and acrylamide (0.759 g, 10.68 mmol) were added to a reaction flask in sequence, the mixture was cooled to -15 °C under $N_2$ atmosphere, and then a solution of potassium tert-butoxide in tetrahydrofuran (1 mol/L, 9.92 mL, 9.92 mmol) was added dropwise. After the dropwise addition was

completed, the mixture was warmed to 0 °C and reacted. After the reaction was completed, as confirmed by TLC, the system was added to a saturated ammonium chloride solution and extracted with ethyl acetate. The organic phase was separated, dried over anhydrous sodium sulfate, and filtered, and the filtrate was purified by silica gel column chromatography to give the target intermediate **19j** (2.81 g).

MS(ESI, $[M+H]^-$) *m/z:* 384.34.

$^1$H NMR (500 MHz, DMSO-d6) δ 11.09 (s, 1H), 7.64 (d, J = 8.2 Hz, 1H), 7.20 (d, J = 8.3 Hz, 1H), 4.67 (s, 2H), 4.57 (dd, J = 12.0, 5.0 Hz, 1H), 3.69 (t, J = 5.9 Hz, 2H), 2.98 (t, J = 5.9 Hz, 2H), 2.77 (ddd, J = 17.3, 12.1, 5.3 Hz, 1H), 2.61 (dt, J = 17.3, 4.1 Hz, 1H), 2.54 (d, J = 4.5 Hz, 1H), 2.18 (dtd, J = 13.5, 5.2, 3.7 Hz, 1H), 1.44 (s, 9H).

Step 10: Preparation of compound **19**

Intermediate **19j** (850 mg) and ethyl acetate (20 mL) were added to a reaction flask in sequence, and a solution of hydrochloric acid in dioxane (4 moL/L, 11.03 mL, 44.1 mmol) was added. The mixture was reacted at room temperature. After the reaction was completed, as confirmed by TLC, the reaction solution was concentrated to give compound **19** (760 mg).

MS(ESI, $[M+H]^+$) m/z: 286.12.

$^1$H NMR (500 MHz, DMSO-d6) δ 11.08 (s, 1H), 7.54 (d, J = 8.1 Hz, 1H), 7.05 (d, J = 8.2 Hz, 1H), 4.54 (dd, J = 11.8, 5.0 Hz, 1H), 3.97 (s, 2H), 3.03 (t, J = 5.8 Hz, 2H), 2.86 (t, J = 5.8 Hz, 2H), 2.76 (td, J = 12.0, 5.9 Hz, 1H), 2.60 (dt, J = 17.3, 4.2 Hz, 1H), 2.46 (dd, J = 12.2, 4.4 Hz, 1H), 2.18 (dq, J = 13.5, 4.8 Hz, 1H).

**Example 20:** Synthesis of Compound **20**

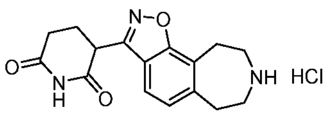

**20**

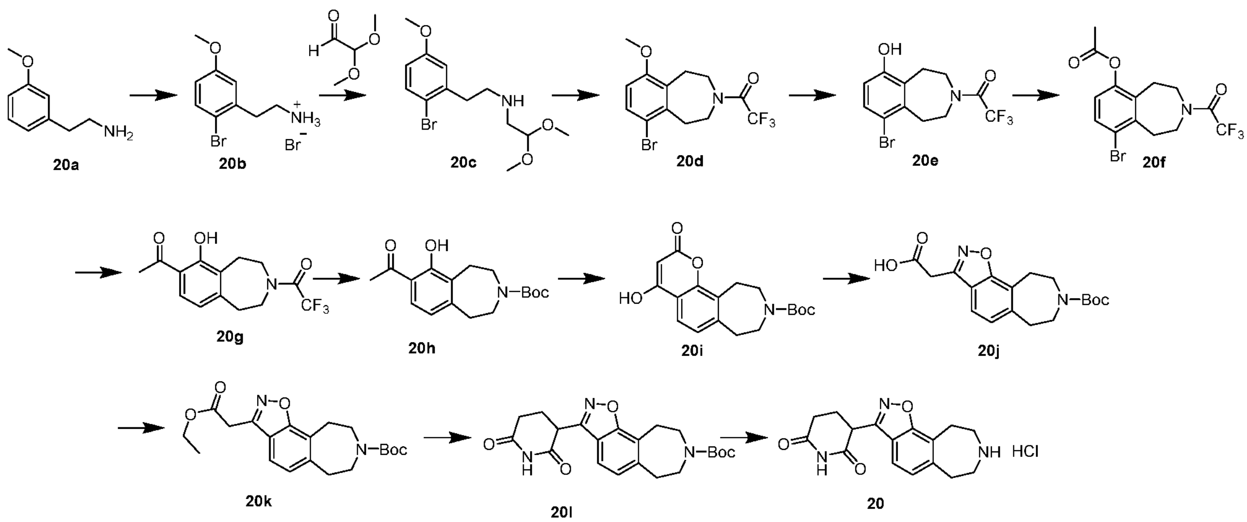

20a 20b 20c 20d 20e 20f

20g 20h 20i 20j

20k 20l 20

Step 1: Preparation of intermediate **20b**

Liquid bromine (55.5 g) was added dropwise into a solution of **20a** (50 g) in acetic acid (180 mL) at 15 °C. After the dropwise addition was completed, the mixture was allowed to react at room temperature. After the reaction was completed, as confirmed by TLC, methyl *tert*-butyl ether was added dropwise to the reaction solution, and the mixture was stirred and filtered. The filter cake was collected and dried to give intermediate **20b** (95 g).

MS(ESI, $[M+H]^+$) *m/z:* 231.9.

Step 2: Preparation of intermediate **20c**

**20b** (80 g), glyoxal dimethyl acetal (66.9 g), triethylamine (27.3 g), anhydrous sodium sulfate (80 g), and methanol (600 mL) were added to a reaction flask in sequence, and the mixture was reacted overnight at room temperature. The reaction solution was cooled to -15 °C, and sodium borohydride (14.6 g) was added in portions. After the addition was completed, the mixture was allowed to react at room temperature. After the reaction was completed, as confirmed by TLC, the reaction solution was concentrated to remove about half of the methanol, and dichloromethane and water were added to the reaction solution. The organic phase was separated, washed with a saturated sodium chloride solution, dried over anhydrous sodium sulfate, filtered, and concentrated. The concentrate was separated and purified by silica gel column chromatography to give intermediate **20c** (60 g).

MS(ESI, $[M+H]^+$) *m/z:* 319.9.

Step 3: Preparation of intermediate **20d**

**20c** (47 g) was added dropwise to trifluoroacetic anhydride (148 g) at 0 °C under nitrogen atmosphere. After the dropwise addition was completed, the mixture was allowed to react at room temperature for 1 h. Trifluoroacetic acid (87 g) was added dropwise, and the mixture was warmed to 40 °C and reacted for 1 h. Triethylsilane (68 g) was added dropwise, and the mixture was warmed to 60 °C and reacted. After the reaction was completed, as confirmed by TLC, 400 mL of ethyl acetate and 600 mL of water were added to the reaction solution. The organic phase was separated, washed three times with a saturated sodium chloride solution, dried over anhydrous sodium sulfate, filtered, and concentrated. The concentrate was separated and purified by silica gel column chromatography to give intermediate **20d** (20.5 g).

1H NMR (500 MHz, DMSO-d6) δ 7.46 (dd, *J* = 8.9, 1.7 Hz, 1H), 6.87 (d, *J* = 8.9 Hz, 1H), 3.77 (d, *J* = 2.8 Hz, 3H), 3.67 (ddt, *J* = 14.4, 5.7, 3.4 Hz, 4H), 3.22 (ddd, *J* = 11.9, 6.4, 4.6 Hz, 2H), 3.16 - 3.06 (m, 2H).

Step 4: Preparation of intermediate **20e**

A solution of boron tribromide in dichloromethane (146 mL, 1 M) was slowly added dropwise to a stirred solution of **20d** (20.5 g) in dichloroethane (200 mL) at 0 °C under nitrogen atmosphere. After the dropwise addition was completed, the mixture was allowed to react at room temperature. After the reaction was completed, as confirmed by TLC, the reaction solution was slowly poured into ice water, stirred for 10 min, and filtered, and the filter cake was collected and dried to give **20e** (18.5 g).

MS(ESI, $[M-H]^+$) *m/z:* 337.9.

1H NMR (500 MHz, DMSO-*d*6) δ 9.74 (s, 1H), 7.26 (dd, *J*= 8.7, 1.3 Hz, 1H), 6.68 (dd, *J*= 8.7, 3.1 Hz, 1H), 3.73 - 3.61 (m, 4H), 3.23 - 3.13 (m, 2H), 3.12 - 3.02 (m, 2H).

Step 5: Preparation of intermediate **20f**

Acetic anhydride (5.65 g) was slowly added dropwise to a stirred solution of **20e** (17.0 g) and triethylamine (7.63 g) in dichloroethane (200 mL) at 0 °C under nitrogen atmosphere. After the dropwise addition was completed, the mixture was allowed to react at room temperature. After the reaction was completed, as confirmed by TLC, the reaction solution was slowly poured into water. The organic phase was separated, washed with a saturated sodium chloride solution, dried over anhydrous sodium sulfate, filtered, and concentrated to give **20f** (19.8 g).

MS(ESI, $[M+H]^+$) *m/z:* 381.7.

Step 6: Preparation of intermediate **20g**

**20f** (19.5 g), aluminum trichloride (18.7 g), and orthodichlorobenzene (80 mL) were added to a reaction flask in sequence, and the mixture was warmed to 150 °C and reacted. After the reaction was completed, as confirmed by TLC, the reaction solution was cooled to room temperature, and 250 mL of 3 N diluted hydrochloric acid was added, followed by extraction with ethyl acetate. The organic phases were combined, washed with saturated brine, dried over anhydrous sodium sulfate, and filtered. The concentrate was separated and purified by silica gel column chromatography to give intermediate **20g** (11.2 g).

MS(ESI, $[M-H]^+$) *m/z:* 300.0.

1H NMR (500 MHz, DMSO-*d*6) δ 12.83 (d, *J* = 4.8 Hz, 1H), 7.78 (dd, *J* = 8.1, 3.5 Hz, 1H), 6.83 (t, *J* = 8.3 Hz, 1H), 3.69 (ddd, *J* = 12.9, 9.6, 5.9 Hz, 4H), 3.11 - 3.00 (m, 4H), 2.64 (s, 3H).

Step 7: Preparation of intermediate **20h**

**20g** (9.5 g), methanol (100 mL), water (20 mL), and sodium hydroxide (1.9 g) were added to a reaction flask in sequence, and the mixture was reacted at room temperature for 1 h. Di-*tert*-butyl dicarbonate (8.2 g) was added, and the mixture was reacted at room temperature. After the reaction was completed, as confirmed by TLC, ethyl acetate and water were added to the reaction solution. The organic phase was separated, washed with saturated brine, dried over anhydrous sodium sulfate, and filtered. The concentrate was separated and purified by silica gel column chromatography to give intermediate **20h** (8.5 g).

$^1$H NMR (500 MHz, DMSO-$d_6$) δ 12.80 (s, 1H), 7.74 (d, *J* = 8.1 Hz, 1H), 6.79 (d, *J* = 8.2 Hz, 1H), 3.45 (dt, *J* = 11.6, 5.0 Hz, 4H), 2.92 (q, *J* = 5.0 Hz, 4H), 2.63 (s, 3H), 1.38 (s, 9H).

Step 8: Preparation of intermediate **20i**

**20h** (8.5 g), diethyl carbonate (16.4 g), and toluene (100 mL) were added to a reaction flask in sequence, and 60 wt% sodium hydride (5.57 g) was added in portions. The reaction solution was warmed to 115 °C and reacted. After the reaction was completed, as confirmed by TLC, the reaction solution was cooled to room temperature, and ethyl acetate and water were added to the reaction solution. The organic phase was separated, washed with saturated brine, dried over anhydrous sodium sulfate, and filtered. The concentrate was separated and purified by silica gel column chromatography to give intermediate **20i** (9.0 g).

MS(ESI, [M-H]$^+$) *m/z:* 330.1.

Step 9: Preparation of intermediate **20j**

**20i** (9.0 g), an aqueous hydroxylamine solution (8.7 g), and ethanol (100 mL) were added to a reaction flask in sequence, and the reaction solution was warmed to 80 °C and reacted. After the reaction was completed, as confirmed by TLC, the reaction solution was cooled to room temperature, and ethyl acetate and water were added to the reaction solution. The organic phase was separated, washed with saturated brine, dried over anhydrous sodium sulfate, and filtered. The concentrate was separated and purified by silica gel column chromatography to give intermediate **20j** (8.5 g).

MS(ESI, [M-H]$^+$) *m/z:* 345.4.

$^1$H NMR (500 MHz, DMSO-$d_6$) δ 7.53 (d, *J* = 8.0 Hz, 1H), 7.12 (d, *J* = 8.0 Hz, 1H), 3.73 (q, *J* = 13.9, 11.5 Hz, 2H), 3.59 - 3.54 (m, 2H), 3.52 - 3.47 (m, 2H), 3.13 (t, *J* = 5.2 Hz, 2H), 3.07 - 2.98 (m, 2H), 1.40 (s, 9H).

Step 10: Preparation of intermediate **20k**

**20j** (8.5 g), potassium carbonate (3.3 g), iodoethane (5.1 g), and DMA (70 mL) were added to a reaction flask in sequence, and the reaction solution was warmed to 80 °C and reacted. After the reaction was completed, as confirmed by TLC, the reaction solution was cooled to room temperature, and ethyl acetate and water were added to the reaction solution. The organic phase was separated, washed with saturated brine, dried over anhydrous sodium sulfate, and filtered. The concentrate was separated and purified by silica gel column chromatography to give intermediate **20k** (6.5 g).

MS(ESI, [M-H]$^+$) *m/z:* 373.1.

$^1$H NMR (500 MHz, DMSO-$d_6$) δ 7.56 (d, *J* = 8.0 Hz, 1H), 7.22 (d, *J* = 8.0 Hz, 1H), 4.13 (dd, *J* = 13.7, 6.6 Hz, 4H), 3.55 (dt, *J* = 28.2, 5.0 Hz, 4H), 3.17 (s, 2H), 3.05 (t, *J* = 5.2 Hz, 2H), 1.38 (dd, *J* = 9.3, 4.4 Hz, 9H), 1.19 (t, *J* = 6.5 Hz, 3H).

Step 11: Preparation of intermediate **20l**

A solution of sodium *tert*-butoxide in tetrahydrofuran (14 mL, 1 M) was slowly added dropwise to a stirred solution of **20k** (5.6 g) in tetrahydrofuran (70 mL) at -10 °C under nitrogen atmosphere. After the dropwise addition was completed, the mixture was maintained at this temperature and reacted for 30 min. Acrylamide (0.71 g) was weighed out and dissolved in 5 mL of tetrahydrofuran, and the solution was added dropwise to the reaction solution. The mixture was reacted. After the reaction was completed, as confirmed by TLC, the reaction solution was slowly poured into saturated ammonium chloride, and ethyl acetate was added. The organic phase was separated, washed with a saturated sodium chloride solution, dried over anhydrous sodium sulfate, and filtered. The concentrate was separated and purified by silica gel column chromatography to give intermediate **20l** (2.5 g). MS(ESI, [M-H]$^+$) *m/z:* 397.9.

$^1$H NMR (500 MHz, DMSO-$d_6$) δ 11.08 (s, 1H), 7.58 (d, *J* = 8.0 Hz, 1H), 7.21 (d, *J* = 8.1 Hz, 1H), 4.55 (dd, *J* = 12.0, 4.9 Hz, 1H), 3.55 (dt, *J* = 31.4, 5.0 Hz, 4H), 3.22 - 3.00 (m, 4H), 2.77 (ddd, *J* = 17.3, 12.0, 5.3 Hz, 1H), 2.60 (dt, *J* = 17.3, 4.1 Hz, 1H), 2.46 (dd, *J* = 12.2, 4.4 Hz, 1H), 2.20 - 2.12 (m, 1H), 1.38 (d, *J* = 6.3 Hz, 9H).

Step 12: Preparation of compound **20**

**20l** (2.5 g), ethyl acetate (30 mL), and a solution of hydrochloric acid in dioxane (15 mL, 4 M) were added to a reaction flask in sequence, and the mixture was reacted at room temperature. After the reaction was completed, as confirmed by TLC, the reaction solution was filtered, and the filter cake was collected and dried to give compound **20** (18.5 g).

MS(ESI, $[M+H]^+$) *m/z:* 300.2.

1H NMR (500 MHz, DMSO-*d6*) δ 11.10 (s, 1H), 9.53 (s, 2H), 7.65 (d, *J* = 8.1 Hz, 1H), 7.27 (d, *J* = 8.1 Hz, 1H), 4.59 (dd, *J* = 12.1, 4.9 Hz, 1H), 3.43 (dd, *J* = 7.0, 3.3 Hz, 2H), 3.37 - 3.19 (m, 6H), 2.78 (ddd, *J* = 17.3, 12.1, 5.3 Hz, 1H), 2.61 (dt, *J* = 17.3, 4.1 Hz, 1H), 2.17 (dtd, *J* = 13.4, 5.2, 3.6 Hz, 1H).

**Example 21:** Synthesis of Compound **21**

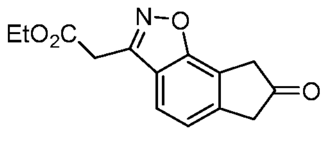

21

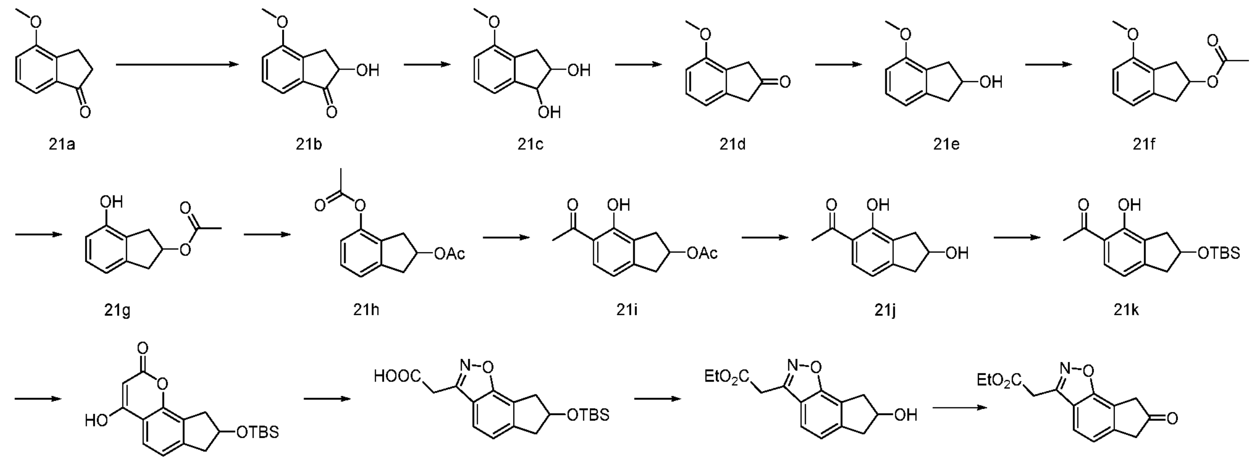

21a 21b 21c 21d 21e 21f

21g 21h 21i 21j 21k

21l 21m 21n 21

Step 1: Preparation of compound **21b**

**21a** (33.3 g), MeOH (500 mL), iodobenzenediacetic acid (82 g, 246 mmol), and potassium hydroxide (127 g) were added to a reaction flask in sequence under an ice bath, and the mixture was reacted at room temperature. After the reaction was completed, as confirmed by TLC, the reaction solution was concentrated by evaporation at reduced pressure to remove the solvent, and the residue was extracted with ethyl acetate and a sodium bicarbonate solution. The organic phase was separated, dried over anhydrous sodium sulfate, filtered, and concentrated by evaporation at reduced pressure to remove the solvent. The residue was dissolved in THF (500 mL), and hydrochloric acid (6 M, 68.4 mL) was added. The mixture was reacted at room temperature for 0.5 h, and the reaction solution was adjusted to pH of 8 with a saturated sodium bicarbonate solution, extracted with 200 mL of ethyl acetate, dried over anhydrous sodium sulfate, concentrated, and purified by silica gel column chromatography to give **21b** (16 g).

MS(ESI, $[M+H]^+$) *m/z:* 178.9.

Step 2: Preparation of compound **21c**

**21b** (80 g) and MeOH (1000 mL) were added to a reaction flask in sequence, and after complete dissolution, sodium borohydride (17.83 g, 471 mmol) was added. The mixture was reacted at room temperature. After the reaction was completed, as confirmed by TLC, a saturated ammonium chloride solution was added dropwise to the reaction solution to quench the reaction, and ethyl acetate was added for extraction. The organic phase was dried over anhydrous

sodium sulfate, filtered, and concentrated by evaporation at reduced pressure to remove the solvent, thus giving **21c** (82 g).

$^1$H NMR (500 MHz, DMSO-d6) δ 7.17 (t, J = 7.8 Hz, 1H), 6.87 (d, J = 7.4 Hz, 1H), 6.80 (d, J = 8.1 Hz, 1H), 5.34 (d, J = 6.3 Hz, 1H), 5.13 (d, J = 5.0 Hz, 1H), 4.67 (t, J = 5.8 Hz, 1H), 4.06 (ddd, J = 12.0, 6.8, 5.1 Hz, 1H), 3.75 (s, 3H), 3.03 (dd, J = 15.8, 7.1 Hz, 1H), 2.43 (dd, J = 15.8, 6.5 Hz, 1H).

Step 3: Preparation of compound **21d**

**21c** (35 g), toluene (300 mL), and p-toluenesulfonic acid (66.9 g) were added to a reaction flask in sequence, and the mixture was heated to 120 °C and reacted. After the reaction was completed, as confirmed by TLC, the reaction solution was cooled to room temperature, and the organic solvent ethyl acetate and water were added to quench the reaction. The organic phase was separated, dried over anhydrous sodium sulfate, filtered, and concentrated by evaporation at reduced pressure to remove the solvent, thus giving **21d** (35.5 g).

$^1$H NMR (500 MHz, DMSO-d6) δ 7.26 - 7.22 (m, 1H), 6.93 - 6.87 (m, 2H), 3.79 (s, 3H), 3.53 (s, 2H), 3.37 (s, 2H).

Step 4: Preparation of compound **21e**

**21d** (35 g), MeOH (400 mL), and sodium borohydride (5.83 g) were added to a reaction flask in sequence, and the mixture was reacted at room temperature. After the reaction was completed, as confirmed by TLC, a saturated ammonium chloride solution was added dropwise to the reaction solution to quench the reaction, and ethyl acetate was added for extraction. The organic phase was dried over anhydrous sodium sulfate, filtered, and concentrated by evaporation at reduced pressure to remove the solvent, thus giving **21e** (18 g).

$^1$H NMR (500 MHz, DMSO-d6) δ 7.13 - 7.07 (m, 1H), 6.83 - 6.78 (m, 1H), 6.74 (d, J = 8.1 Hz, 1H), 4.81 (d, J = 3.8 Hz, 1H), 4.49 (tq, J = 6.5, 3.4 Hz, 1H), 3.75 (s, 3H), 3.04 (dd, J = 16.1, 6.1 Hz, 1H), 2.94 (dd, J = 16.3, 6.2 Hz, 1H), 2.79 - 2.61 (m, 2H).

Step 5: Preparation of compound **21f**

**21e** (60 g), dichloromethane (500 mL), triethylamine (111 g, 152 mL), and acetic anhydride (41.0 g, 38.2 mL) were added to a reaction flask in sequence, and the mixture was reacted at room temperature. After the reaction was completed, as confirmed by TLC, the reaction solution was washed with a saturated ammonium chloride solution and saturated brine, dried over anhydrous sodium sulfate, filtered, and concentrated under reduced pressure to give **21f** (37.2 g).

$^1$H NMR (500 MHz, DMSO-d6) δ 7.22 - 7.06 (m, 1H), 6.82 (dd, J = 27.3, 8.3 Hz, 2H), 5.41 (s, 1H), 3.78 (d, J = 13.2 Hz, 3H), 3.32 - 3.20 (m, 1H), 3.18 - 3.09 (m, 1H), 2.85 (dd, J = 34.6, 17.1 Hz, 2H), 1.97 (d, J = 15.3 Hz, 3H).

Step 6: Preparation of compound **21g**

Boron trichloride (19.22 g, 164 mL) was slowly added dropwise to a stirred solution of **21f** (17 g) in dichloromethane (500 mL) under an ice bath. After the dropwise addition was completed, the mixture was naturally warmed to room temperature and reacted. After the reaction was completed, as confirmed by TLC, 160 mL of 1 M HCl and 200 mL of an aqueous solution were added to the reaction solution to quench the reaction, followed by extraction with dichloromethane. The organic phase was separated, dried over anhydrous sodium sulfate, and concentrated by evaporation at reduced pressure to remove the solvent, thus giving **21g** (15 g).

MS(ESI, $[M-H]^-$) *m/z:* 190.9.

Step 7: Preparation of compound **21h**

**21g** (16 g), dichloromethane (200 mL), triethylamine (9.50 g, 13.01 mL), and acetic anhydride (5.27 g, 4.91 mL) were added to a reaction flask in sequence, and the mixture was reacted at room temperature. After the reaction was completed, as confirmed by TLC, the reaction solution was washed with a saturated ammonium chloride solution and saturated brine, dried over anhydrous sodium sulfate, filtered, and concentrated under reduced pressure to give **21h** (14.8 g).

MS(ESI, $[M-H]^-$) *m/z:* 233.01.

Step 8: Preparation of compound **21i**

**21h** (11.6 g), dichloromethane (300 mL), and zirconium tetrachloride (46.2 g, 198 mmol) were added to a

reaction flask in sequence, and the mixture was reacted at 50 °C. After the reaction was completed, as confirmed by TLC, the reaction solution was cooled to room temperature, and a 3 M aqueous hydrochloric acid solution, water, and dichloromethane were added to the residue. The organic phase was separated, dried over anhydrous sodium sulfate, filtered, and concentrated by evaporation at reduced pressure to remove the solvent, thus giving **21i** (11.4 g).

$^1$H NMR (500 MHz, DMSO-*d*6) δ 12.34 (s, 1H), 7.80 (d, *J* = 8.0 Hz, 1H), 6.91 (d, *J* = 8.0 Hz, 1H), 5.45 (tt, *J* = 6.3, 2.2 Hz, 1H), 3.37 - 3.33 (m, 1H), 3.19 (dd, *J* = 17.3, 6.3 Hz, 1H), 2.95 (dd, *J* = 17.9, 2.2 Hz, 1H), 2.86 (dd, *J* = 17.3, 2.1 Hz, 1H), 2.63 (s, 3H), 1.97 (s, 3H).

Step 9: Preparation of compound **21k**

**21i** (15.4 g), ethanol (200 mL), and an aqueous solution (10.00 mL) of sodium hydroxide (2.63 g) were added to a reaction flask in sequence, and the mixture was reacted at room temperature. After the reaction was completed, as confirmed by TLC, the reaction solution was adjusted to pH of 2-3 with a 2 M aqueous HCl solution, extracted with ethyl acetate and water, dried over anhydrous sodium sulfate, filtered, and concentrated by evaporation at reduced pressure to remove the solvent, thus giving the crude intermediate **21j.** Dichloroethane (200 mL), imidazole (17.90 g), and TBSCl (39.6 g) were added, and the mixture was reacted overnight under reflux. The reaction solution was cooled to room temperature, and 100 mL of dichloromethane and 300 mL of water were added for extraction. The organic phase was dried over anhydrous sodium sulfate, filtered, and concentrated by evaporation at reduced pressure to remove the solvent, thus giving **21k** (15.4 g).

$^1$H NMR (500 MHz, DMSO-d6) δ 12.25 (s, 1H), 7.69 (d, *J* = 8.0 Hz, 1H), 6.79 (d, *J* = 8.0 Hz, 1H), 4.64 (dq, *J* = 6.2, 3.2 Hz, 1H), 3.05 (ddd, *J* = 56.3, 16.6, 6.2 Hz, 2H), 2.71 (dd, *J* = 17.0, 3.6 Hz, 1H), 2.61 (dd, *J* = 16.3, 3.5 Hz, 1H), 2.51 (s, 3H), 0.78 (s, 9H), 0.00 (s, 6H).

Step 10: Preparation of compound **21l**

**21k** (8.4 g) and THF (300 mL) were added to a reaction flask in sequence, and diethyl carbonate (16.19 g, 16.52 mL) was added. The mixture was cooled to about 0 °C, and 60 wt% sodium hydride (5.48 g, 137 mmol) was added in portions. The mixture was heated to 85 °C and reacted. After the reaction was completed, as confirmed by TLC, the reaction solution was cooled to room temperature and slowly poured into ice water. The mixture was extracted with ethyl acetate, and the organic phase was discarded. The aqueous phase was adjusted to pH = 1-2 with 3 M hydrochloric acid, then extracted with ethyl acetate, dried over anhydrous sodium sulfate, filtered, and concentrated by evaporation at reduced pressure to remove the solvent, thus giving **21l** (10 g).

MS(ESI, [M-H]-) m/z: 331.2.

Step 11: Preparation of intermediate **21m**

**21l** (10 g), an aqueous hydroxylamine solution (9.93 g, 9.93 mL, 150 mmol), and ethanol (100 mL) were added to a reaction flask in sequence, and the mixture was reacted at 85 °C. After the reaction was completed, as confirmed by TLC, the reaction solution was cooled to room temperature, ethyl acetate and a saturated aqueous sodium carbonate solution were added to the residue for extraction, and the organic phase was discarded. The aqueous phase was adjusted to pH = 2-3 with a 1 M aqueous HCl solution, extracted with ethyl acetate, dried over anhydrous sodium sulfate, filtered, and concentrated by evaporation at reduced pressure to remove the solvent, thus giving **21m** (11 g).

MS(ESI, [M-H]-) m/z: 346.2.

Step 12: Preparation of intermediate **21n**

**21m** (10 g), ethanol (150 mL), and sulfuric acid (14.40 g, 7.83 mL, 144 mmol) were added to a reaction flask in sequence, and the mixture was reacted at 85 °C. After the reaction was completed, as confirmed by TLC, the reaction solution was cooled to room temperature and adjusted to pH = 7 with dichloromethane and saturated aqueous sodium bicarbonate. The organic phase was separated, dried over anhydrous sodium sulfate, filtered, and concentrated by evaporation at reduced pressure to remove the solvent, thus giving **21n** (5.8 g).

MS(ESI, [M+H]+) m/z: 261.97.

$^1$H NMR (500 MHz, DMSO-d6) δ 7.65 (d, *J* = 8.0 Hz, 1H), 7.33 (d, *J* = 8.0 Hz, 1H), 5.09 (d, *J* = 4.0 Hz, 1H), 4.71 (dt, *J* = 6.4, 3.1 Hz, 1H), 4.26 - 4.12 (m, 4H), 3.30 (ddd, *J* = 31.0, 16.5, 6.0 Hz, 2H), 2.98 (ddd, *J* = 31.6, 16.5, 3.0 Hz, 2H), 1.22 (t, *J* = 7.1 Hz, 3H).

Step 13: Preparation of intermediate **21**

Compound **21n** (300 mg), dichloromethane (10 mL), and Dess-Martin periodinane (974 mg) were added to a reaction flask in sequence, and the mixture was stirred and reacted at room temperature. After the reaction was completed, as confirmed by TLC, the reaction solution was poured into a saturated sodium sulfite solution to quench the reaction, and ethyl acetate was added for extraction. The organic phase was separated, washed with 200 mL of a saturated sodium bicarbonate solution and 200 mL of saturated brine, dried over anhydrous sodium sulfate, and filtered, and the filtrate was concentrated by evaporation at reduced pressure to remove the solvent, thus giving compound **21** (320 mg).

MS(ESI, $[M+H]^+$) m/z: 260.0.

$^1H$ NMR (500 MHz, DMSO-d6) δ 7.74 (d, J = 8.1 Hz, 1H), 7.38 (d, J = 8.1 Hz, 1H), 4.21 (s, 2H), 4.14 (q, J = 7.1 Hz, 2H), 3.79 (s, 2H), 3.72 (s, 2H), 1.19 (t, J = 7.1 Hz, 3H).

**Examples 22** and **23:** Synthesis of Compounds **22** and **23**

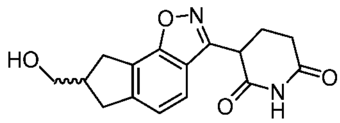
22

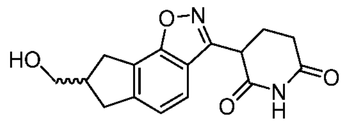
23

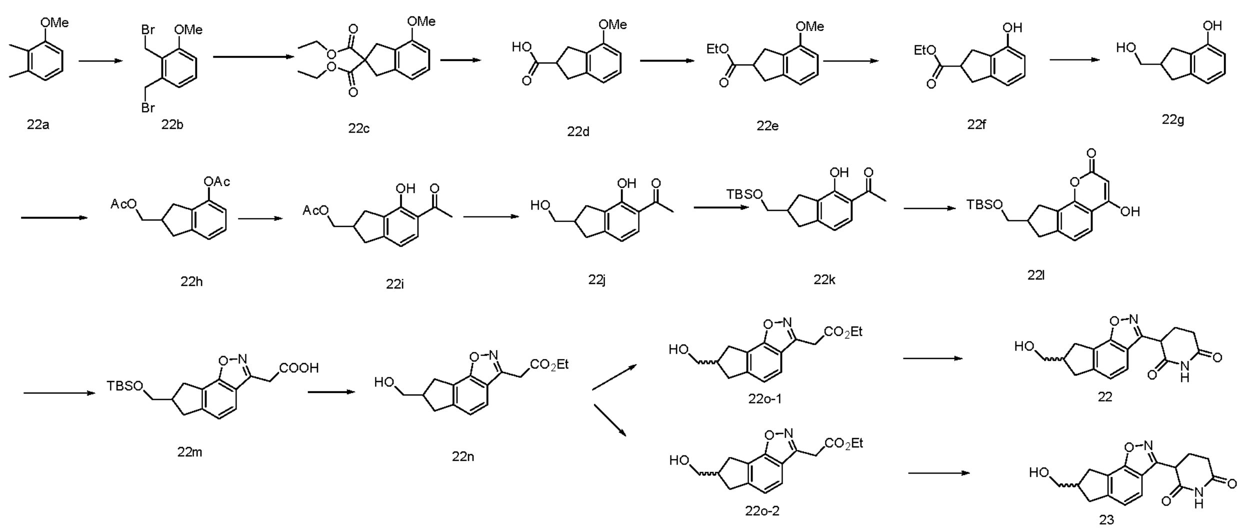
22a 22b 22c 22d 22e 22f 22g
22h 22i 22j 22k 22l
22m 22n 22o-1 22 22o-2 23

Step 1: Preparation of intermediate **22b**

$CCl_4$ (6750 mL), **22a** (450 g), 2,2-azobisisobutyronitrile (18.45 g), and N-bromosuccinimide (1194 g) were added to a reaction flask in sequence. The mixture was heated to 80 °C and reacted under reflux. After the reaction was completed, as confirmed by TLC, the reaction solution was filtered, and the filtrate was concentrated by evaporation at reduced pressure to remove the solvent. The residue was slurried with petroleum ether and filtered, and the filter cake was collected to give intermediate **22b** (833 g).

$^1H$ NMR (500 MHz, DMSO-d6) δ 7.34 (d, J = 8.1 Hz, 1H), 7.06 (ddd, J = 17.8, 8.1, 1.1 Hz, 2H), 4.77 (d, J = 9.5 Hz, 4H), 3.87 (s, 3H).

Step 2: Preparation of intermediate **22c**

60 wt% NaH (187 g) and THF (2000 mL) were added to a reaction flask in sequence, and diethyl malonate (300 g, 284 mL) was added under an ice bath. The mixture was stirred at room temperature for 30 min, and then **22b** (606 g) was added. The mixture was stirred and reacted at room temperature. After the reaction was completed, as confirmed by TLC, the reaction solution was slowly added dropwise to a saturated ammonium chloride solution to quench the reaction, and 2000 mL of petroleum ether and 2000 mL of water were added for extraction. The aqueous phase was extracted twice with 1000 mL of petroleum ether, and the organic phases were combined, washed twice with 500 mL

of a saturated ammonium chloride solution, washed with saturated brine, dried over anhydrous sodium sulfate, and filtered. The filtrate was concentrated by evaporation at reduced pressure to remove the solvent, and the residue was purified by silica gel column chromatography to give 262 g of intermediate **22c.**

MS(ESI, [M-H]+) m/z: 293.2
$^1$H NMR (500 MHz, DMSO-d6) δ 7.16 (t, J = 7.8 Hz, 1H), 6.80 (dd, J = 15.3, 7.8 Hz, 2H), 4.14 (q, J = 7.1 Hz, 4H), 3.77 (s, 3H), 3.48 (s, 2H), 3.38 (s, 2H), 1.17 (t, J = 7.0 Hz, 6H).

Step 3: Preparation of intermediate **22d**

**22c** (130 g), DMSO (1000 mL), $H_2O$ (300 mL), and lithium chloride (42.6 g) were added to a reaction flask, and the mixture was stirred at 180 °C. After the reaction was completed, as confirmed by TLC, the reaction solution was poured into 1000 mL of ice water to quench the reaction, adjusted to pH = 2-3 with 1 M hydrochloric acid, extracted 3 times with 1 L of ethyl acetate, washed with saturated brine, dried over anhydrous sodium sulfate, and filtered, and the filtrate was concentrated by evaporation at reduced pressure to remove the solvent, thus giving 191 g of intermediate **22d.** MS(ESI, [M+H]+) m/z: 193.05

Step 4: Preparation of intermediate **22e**

**22d** (85 g), ethanol (1000 mL), and concentrated sulfuric acid (44 g) were added to a reaction flask in sequence, and the mixture was heated to 70 °C and reacted. After the reaction was completed, as confirmed by TLC, the reaction solution was cooled to room temperature and concentrated by evaporation at reduced pressure to remove the solvent. The residue was poured into ice water and neutralized with a saturated aqueous sodium bicarbonate solution. After neutralization, the mixture was extracted 3 times with 1000 mL of petroleum ether. The organic phase was separated, washed with saturated brine, dried over anhydrous sodium sulfate, and filtered, and the filtrate was concentrated by evaporation at reduced pressure to remove the solvent, thus giving 99 g of intermediate **22e.**

MS(ESI, [M+H]+) m/z: 221.1
$^1$H NMR (500 MHz, DMSO-d6) δ 7.13 (t, J = 7.8 Hz, 1H), 6.81 (d, J = 7.5 Hz, 1H), 6.76 (d, J = 8.2 Hz, 1H), 4.09 (q, J = 7.1 Hz, 2H), 3.76 (s, 3H), 3.36 - 3.31 (m, 1H), 3.20 - 3.10 (m, 2H), 3.10 - 2.96 (m, 2H), 1.20 (t, J = 7.1 Hz, 3H).

Step 5: Preparation of intermediate **22f**

In a reaction flask, boron tribromide (415 g, 1657 mL) was added dropwise to a stirred solution of **22e** (150 g) in dichloromethane (750 mL) under $N_2$ atmosphere, and the reaction was carried out at a temperature of not higher than 0 °C. MeOH (500 mL) was added at 0 °C, and the mixture was reacted for 30 min, then gradually allowed to return to room temperature, and stirred for 2 h. After the reaction was completed, the reaction solution was poured into a mixed solvent of 1000 mL of ice water and 1000 mL of dichloromethane, and the mixture was stirred, separated by a separating funnel, washed with saturated brine, dried over anhydrous sodium sulfate, and filtered, and the filtrate was concentrated by evaporation at reduced pressure to remove the solvent, thus giving 122 g of intermediate **22f.**

$^1$H NMR (500 MHz, DMSO-d6) δ 9.24 (s, 1H), 6.95 (t, J = 7.7 Hz, 1H), 6.70 - 6.60 (m, 1H), 6.62 - 6.52 (m, 1H), 4.10 (q, J = 7.1 Hz, 2H), 3.33 - 3.27 (m, 1H), 3.13 - 3.01 (m, 3H), 2.96 (dd, J = 16.1, 7.1 Hz, 1H), 1.20 (t, J = 7.1 Hz, 3H).

Step 6: Synthesis of intermediate **22g**

**22f** (130 g) and tetrahydrofuran (2000 mL) were added to a reaction flask in sequence at 0 °C under $N_2$ atmosphere, and a solution of lithium aluminum hydride in tetrahydrofuran (1 M, 438 mL) was slowly added dropwise. The mixture was reacted under an ice-water bath. After the reaction was completed, as confirmed by TLC, 3 L of water was slowly added dropwise to quench the reaction. The reaction solution was adjusted to pH = 1-2 with concentrated hydrochloric acid and extracted 3 times with 2 L of ethyl acetate. The organic phases were combined, washed with saturated brine, dried over anhydrous sodium sulfate, and filtered, and the filtrate was concentrated by evaporation at reduced pressure to remove the solvent, thus giving 129 g of intermediate **22g.**

$^1$H NMR (500 MHz, DMSO-d6) δ 6.88 (t, J = 7.7 Hz, 1H), 6.58 (d, J = 7.3 Hz, 1H), 6.52 (d, J = 7.9 Hz, 1H), 3.38 - 3.32 (m, 2H), 2.84 (ddd, J = 33.5, 16.2, 8.3 Hz, 2H), 2.60 (dq, J = 13.9, 8.1, 6.6 Hz, 1H), 2.55 - 2.49 (m, 3H).

Step 7: Preparation of intermediate **22h**

**22g** (120 g), 4-dimethylaminopyridine (7.14 g), dichloromethane (2000 mL), and triethylamine (177 g, 244 mL)

were added to a reaction flask in sequence, and acetyl chloride (101 g, 91 mL) was slowly added dropwise at 0 °C. After the dropwise addition was completed, the mixture was reacted at room temperature. After the reaction was completed as confirmed by TLC, the reaction solution was poured into a mixed solvent of dichloromethane and water. The organic phase was separated, and the aqueous phase was extracted with dichloromethane. The organic phases were combined, washed with saturated brine, dried over anhydrous sodium sulfate, and filtered, and the filtrate was purified by silica gel column chromatography to give 127 g of intermediate **22h.**

MS(ESI, [M+H]+) m/z: 249.3
$^1$H NMR (500 MHz, DMSO-d6) δ 7.18 (t, J = 7.7 Hz, 1H), 7.11 (d, J = 7.4 Hz, 1H), 6.88 (d, J = 7.9 Hz, 1H), 4.07 - 3.96 (m, 2H), 3.11 - 3.00 (m, 1H), 2.87 (dd, J = 15.9, 7.8 Hz, 1H), 2.81 - 2.69 (m, 2H), 2.56 - 2.50 (m, 1H), 2.27 (s, 3H), 2.02 (s, 3H).

Step 8: Preparation of intermediate **22i**

**22h** (91 g), dichloromethane (2000 mL), and zirconium tetrachloride (342 g) were added to a reaction flask in sequence, and the mixture was stirred and reacted overnight at 50 °C under $N_2$ atmosphere. After the reaction was completed, the reaction solution was cooled to room temperature and then poured into a mixed solvent of 1000 mL of ice water and 1000 mL of dichloromethane. The organic phase was separated, and the aqueous phase was extracted with dichloromethane. The organic phases were combined, dried over anhydrous sodium sulfate, and filtered, and the filtrate was purified by silica gel column chromatography to give 90 g of intermediate **22i.**

MS(ESI, [M-H]+) m/z: 247.2
$^1$H NMR (500 MHz, DMSO-d6) δ 12.34 (s, 1H), 7.73 (d, J = 8.0 Hz, 1H), 6.83 (d, J = 8.0 Hz, 1H), 4.68 (t, J = 5.3 Hz, 1H), 3.36 (ddd, J = 7.1, 5.3, 2.0 Hz, 2H), 3.03 - 2.94 (m, 1H), 2.92 - 2.82 (m, 1H), 2.76 - 2.68 (m, 1H), 2.61 (s, 3H).

Step 9: Preparation of intermediate **22j**

**22i** (95 g) and ethanol (900 mL) were added to a reaction flask in sequence, and a solution of sodium hydroxide (77 g) in $H_2O$ (800 mL) was added dropwise under an ice bath. The mixture was reacted at room temperature under $N_2$ atmosphere. After the reaction was completed, as confirmed by TLC, the reaction solution was diluted with 2 L of ethyl acetate and 1 L of water, 3 M hydrochloric acid was slowly added to adjust pH = 3, and liquid separation was performed. The aqueous phase was extracted 3 times with ethyl acetate, the organic phases were combined, washed with saturated brine, dried over anhydrous sodium sulfate, and filtered, and the filtrate was concentrated by evaporation at reduced pressure to remove the solvent, thus giving 86 g of intermediate **22j.**

MS(ESI, [M-H]+) m/z: 205.1
$^1$H NMR (500 MHz, DMSO-d6) δ 12.34 (s, 1H), 7.73 (d, J = 8.0 Hz, 1H), 6.84 (d, J = 8.0 Hz, 1H), 4.68 (t, J = 5.3 Hz, 1H), 3.36 (ddd, J = 7.0, 5.2, 2.0 Hz, 2H), 2.98 (dd, J = 17.0, 8.2 Hz, 1H), 2.92 - 2.82 (m, 1H), 2.72 (dd, J = 16.9, 5.6 Hz, 1H), 2.61 (s, 3H), 2.61 - 2.53 (m, 2H).

Step 10: Preparation of intermediate **22k**

**22j** (37 g), 1,2-dichloroethane (700 mL), imidazole (36.6 g), and tert-butyldimethylsilyl chloride (29.7 g) were added to a reaction flask in sequence, and the mixture was reacted at 75 °C. After the reaction was completed, as confirmed by TLC, the reaction solution was cooled to room temperature, and dichloromethane and water were added. The organic phase was separated, washed with saturated brine, dried over anhydrous sodium sulfate, and filtered, and the filtrate was concentrated by evaporation at reduced pressure to remove the solvent, thus giving 60 g of intermediate **22k.**

$^1$H NMR (500 MHz, DMSO-d6) δ 12.31 (s, 1H), 7.71 (d, J = 8.0 Hz, 1H), 6.82 (d, J = 8.0 Hz, 1H), 3.52 (d, J = 6.4 Hz, 2H), 2.97 (dd, J = 16.9, 8.0 Hz, 1H), 2.85 (dd, J = 15.4, 7.5 Hz, 1H), 2.69 (dd, J = 16.9, 5.6 Hz, 1H), 2.64 - 2.59 (m, 1H), 2.58 (s, 3H), 2.55 (d, J = 5.6 Hz, 1H), 0.82 (s, 9H), 0.00 (s, 6H).

Step 11: Preparation of intermediate **22l**

**22k** (55 g), diethyl carbonate (101 g, 103 mL), and toluene (1000 mL) were added to a reaction flask in sequence. The reaction solution was cooled to 0 °C, and 60 wt% sodium hydride (34.3 g, 858 mmol) was added in portions. After the addition was completed, the mixture was slowly heated to 120 °C and reacted. After the reaction was completed as confirmed by TLC, the reaction solution was slowly poured into ice water and extracted with ethyl acetate. The aqueous

phase was adjusted to pH = 3 with 3 N hydrochloric acid and extracted 3 times with ethyl acetate, and the organic phases were combined, dried over anhydrous sodium sulfate, filtered, and concentrated by evaporation at reduced pressure to remove the solvent, thus giving 53.6 g of intermediate **22l**.

MS(ESI, [M-H]+) m/z: 345.1.

$^1$H NMR (500 MHz, DMSO-*d*6) δ 12.31 (s, 1H), 7.57 (d, J = 7.9 Hz, 1H), 7.16 (d, J = 7.9 Hz, 1H), 5.49 (s, 1H), 3.56 (d, J = 6.4 Hz, 2H), 3.04 (ddd, J = 16.0, 13.3, 8.1 Hz, 2H), 2.75 (td, J = 14.8, 13.3, 4.4 Hz, 2H), 2.71 - 2.63 (m, 1H), 0.81 (s, 9H), 0.00 (s, 6H).

Step 12: Preparation of intermediate **22m**

**22l** (51 g), hydroxylamine hydrochloride (61.4 g), sodium ethoxide (61.1 g), and ethanol (2000 mL) were added to a reaction flask in sequence, and the mixture was heated to 85 °C and reacted under $N_2$ atmosphere. After the reaction was completed, as confirmed by TLC, the reaction solution was concentrated by evaporation at reduced pressure to remove the solvent, 2 L of water was added, the pH was adjusted to 8-9 with a saturated sodium carbonate solution, and ethyl acetate was added for extraction. The aqueous phase was collected, adjusted to pH = 6-7 with 1 M hydrochloric acid, and extracted with ethyl acetate. The organic phase was collected, washed with saturated brine, dried over anhydrous sodium sulfate, and filtered, and the filtrate was concentrated by evaporation at reduced pressure to remove the solvent, thus giving 47 g of intermediate **22m.**

MS(ESI, [M-H]+) m/z: 360.2.

$^1$H NMR (500 MHz, DMSO-d6) δ 7.55 (d, J = 8.0 Hz, 1H), 7.22 (d, J = 8.0 Hz, 1H), 3.97 (s, 2H), 3.57 (d, J = 6.6 Hz, 2H), 3.16 - 3.07 (m, 2H), 2.88 - 2.72 (m, 3H), 0.81 (s, 9H), 0.00 (s, 6H).

Step 13: Preparation of intermediate **22n**

**22m** (47 g), ethanol (1500 mL), and concentrated sulfuric acid (65.1 g, 35.4 mL) were added to a reaction flask in sequence, and the mixture was heated to 85 °C under $N_2$ atmosphere. After the reaction was completed, as confirmed by TLC, the reaction solution was cooled to room temperature and subjected to rotary evaporation to remove the solvent. Dichloromethane was added, and a saturated aqueous sodium bicarbonate solution was added dropwise for neutralization. The mixture was washed with saturated brine, dried over anhydrous sodium sulfate, and filtered, and the filtrate was concentrated by evaporation at reduced pressure to remove the solvent, thus giving 43 g of intermediate **22n.**

MS(ESI, [M-H]+) m/z: 276.1

$^1$H NMR (500 MHz, DMSO-d6) δ 7.58 (d, J = 8.0 Hz, 1H), 7.27 (d, J = 8.0 Hz, 1H), 4.74 (q, J = 4.9 Hz, 1H), 4.16 (s, 2H), 4.12 (t, J = 7.1 Hz, 2H), 3.43 (dd, J = 6.8, 5.3 Hz, 2H), 3.15 (ddd, J = 29.7, 16.4, 8.3 Hz, 2H), 2.93 - 2.81 (m, 2H), 2.79 - 2.70 (m, 1H), 1.19 (t, J = 7.1 Hz, 3H).

Step 14: Preparation of intermediates **22o-1** and **22o-2**

Preparative resolution was performed. 43 g of intermediate **22n** was dissolved in 430 mL of a solution of dichloromethane in ethanol, the concentration was about 100.0 mg/mL, and the mixture was filtered through a 0.45 μm organic filter membrane and the filtrate was collected. Instrument: YMC high pressure preparative chromatograph; chromatographic column: CHIRALPAK IG (30*250 mm, S-10 μm); mobile phases: A: ethanol, B: n-hexane. The prepeak gave 9.057 g of intermediate **22o-1** and the postpeak gave 8.833 g of intermediate **22o-2.**

**22o-1:** MS(ESI, [M-H]+) m/z: 276.1.
**22o-2:** MS(ESI, [M-H]+) m/z: 276.1.

Step 15: Preparation of compound **22**

**22o-1** (11.93 g), THF (200 mL), and acrylamide (3.39 g) were added to a reaction flask in sequence under $N_2$ atmosphere. After the mixture was cooled to 0 °C, potassium *tert*-butoxide (3.89 g, 34.7 mL) was added. The mixture was reacted at 0 °C. After the reaction was completed, as confirmed by TLC, the reaction solution was added dropwise to an icy saturated aqueous ammonium chloride solution, and ethyl acetate was added for extraction. The aqueous phase was extracted twice with ethyl acetate, and the organic phases were combined, washed with saturated brine, dried over anhydrous sodium sulfate, and filtered. The filtrate was concentrated by evaporation at reduced pressure to remove the solvent and filtered, and the filter cake was collected to give 6.77 g of compound **22.**

$^1$H NMR (500 MHz, DMSO-d6) δ 11.08 (s, 1H), 7.60 (d, J = 8.0 Hz, 1H), 7.25 (d, J = 8.1 Hz, 1H), 4.74 (td, J = 5.3, 2.2 Hz, 1H), 4.56 (dd, J = 11.8, 5.0 Hz, 1H), 3.43 (dd, J = 6.8, 5.3 Hz, 2H), 3.22 - 3.09 (m, 2H), 2.94 - 2.83 (m, 2H),

2.76 (dq, J = 16.9, 6.3 Hz, 2H), 2.60 (dt, J = 17.3, 4.2 Hz, 1H), 2.50 - 2.44 (m, 1H), 2.23 - 2.13 (m, 1H).

Step 16: Preparation of compound of Example **23**

**22o-2** (12.83 g), THF (200 mL), and acrylamide (3.64 g) were added to a reaction flask in sequence. After the mixture was cooled to 0 °C, potassium *tert*-butoxide (4.18 g, 37.3 mL) was added. The mixture was reacted at 0 °C under $N_2$ atmosphere. After the reaction was completed, as confirmed by TLC, the reaction solution was added dropwise to an icy saturated aqueous ammonium chloride solution, and ethyl acetate was added for extraction. The aqueous phase was extracted twice with ethyl acetate, and the organic phases were combined, washed with saturated brine, dried over anhydrous sodium sulfate, and filtered. The filtrate was concentrated by evaporation at reduced pressure to remove the solvent and filtered, and the filter cake was collected to give 7.454 g of the compound of Example 23.

$^1$H NMR (500 MHz, DMSO-d6) δ 11.08 (s, 1H), 7.60 (d, J = 8.2 Hz, 1H), 7.25 (d, J = 8.1 Hz, 1H), 4.74 (td, J = 5.3, 2.2 Hz, 1H), 4.56 (dd, J = 11.8, 5.0 Hz, 1H), 3.43 (dd, J = 6.8, 5.2 Hz, 2H), 3.21 - 3.09 (m, 2H), 2.94 - 2.83 (m, 2H), 2.80 - 2.71 (m, 2H), 2.60 (dt, J = 17.3, 4.2 Hz, 1H), 2.46 (dd, J = 12.1, 4.5 Hz, 1H), 2.23 - 2.15 (m, 1H).

**Example 24:** Synthesis of Compound **24**

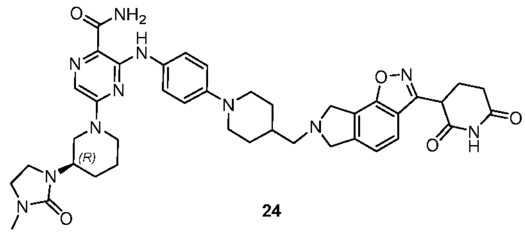

**24**

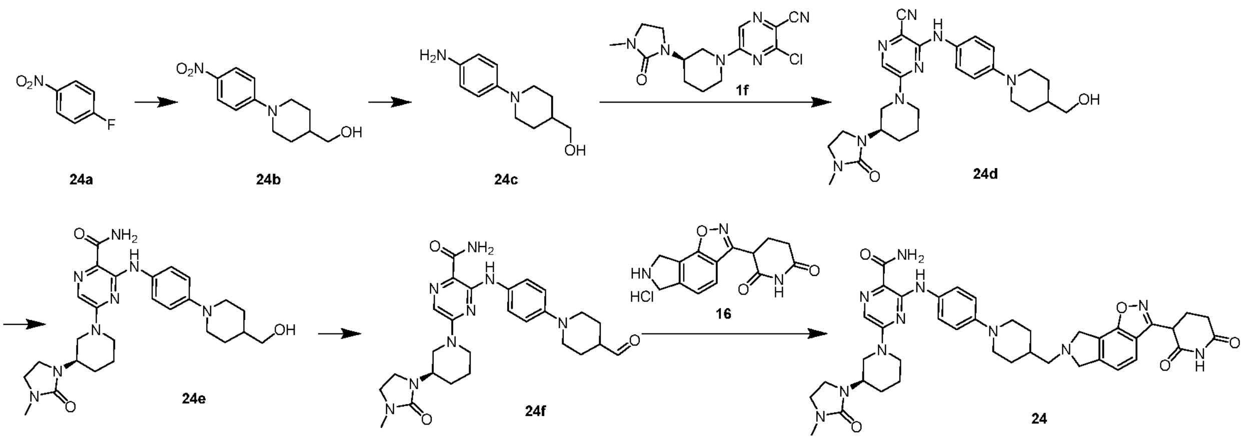

24a 24b 24c 1f 24d 24e 24f 16 24

Step 1: Preparation of intermediate **24b**

**24a** (7.00 g), 4-piperidinemethanol (6.86 g), N,N-diisopropylethylamine (9.62 g), and dimethyl sulfoxide (70 mL) were added to a reaction flask in sequence, and the mixture was heated to 100 °C and reacted. After the reaction was completed, as confirmed by TLC, the reaction solution was cooled to room temperature, and water was added to the reaction solution. The mixture was stirred for 10 min and filtered, and the filter cake was collected and dried to give intermediate **24b** (13.7 g).

MS(ESI, [M+H]$^+$) *m/z:* 237.1.

Step 2: Preparation of intermediate **24c**

Intermediate **24b** (13.5 g), 10% palladium on carbon (2.70 g), and methanol (200 mL) were added to a reaction flask in sequence, and the mixture was purged three times with hydrogen and reacted overnight. The palladium on carbon was removed by filtration under vacuum, and the residue was concentrated to give intermediate **24c** (9.0 g).

MS(ESI, [M+H]$^+$) *m/z:* 207.3.

1H NMR (500 MHz, DMSO-*d*6) δ 6.68 (d, *J* = 8.2 Hz, 2H), 6.47 (d, *J* = 8.2 Hz, 2H), 4.59 (s, 2H), 4.45 (t, *J* = 5.3 Hz, 1H), 3.34 (s, 2H), 3.28 (dd, *J* = 6.5, 3.1 Hz, 2H), 2.44 (t, *J* = 11.7 Hz, 2H), 1.71 (d, *J* = 12.5 Hz, 2H), 1.40 (ddt, *J* = 11.5, 8.6, 4.1 Hz, 1H), 1.23 (qd, *J* = 12.2, 4.0 Hz, 2H).

Step 3: Preparation of intermediate **24d**

Intermediate **1f** (6.0 g), **24c** (3.86 g), BINAP (1.16 g), cesium carbonate (18.28 g), palladium acetate (0.42 g), and 1,4-dioxane (80 mL) were added to a reaction flask in sequence, and the mixture was purged three times with nitrogen, heated to 100 °C, and reacted. After the reaction was completed, as confirmed by TLC, the reaction solution was cooled to room temperature, and ethyl acetate and water were added to the residue. The organic phase was separated, washed with a saturated sodium chloride solution, dried over anhydrous sodium sulfate, filtered, and concentrated. The concentrate was separated and purified by silica gel column chromatography to give intermediate **24d** (7.1 g).

MS(ESI, $[M+H]^+$) *m/z*: 491.5.

$^1$H NMR (500 MHz, DMSO-$d_6$) δ 8.79 (s, 1H), 7.75 (s, 1H), 7.42 - 7.31 (m, 2H), 6.92 - 6.79 (m, 2H), 4.47 (t, *J* = 5.3 Hz, 1H), 4.39 - 4.17 (m, 2H), 3.66 - 3.52 (m, 3H), 3.31 - 3.21 (m, 5H), 2.99 - 2.86 (m, 2H), 2.70 (s, 3H), 2.61 - 2.53 (m, 2H), 1.81 - 1.69 (m, 5H), 1.57 - 1.43 (m, 2H), 1.29 - 1.16 (m, 3H).

Step 4: Preparation of intermediate **24e**

Intermediate **24d** (6.9 g), cesium carbonate (4.58 g), water (20 mL), DMSO (20 mL), and methanol (70 mL) were added to a reaction flask in sequence. The reaction solution was cooled to 0 °C, and 30 wt% hydrogen peroxide (4.78 g) was added dropwise. After the dropwise addition was completed, the mixture was allowed to react at room temperature. After the reaction was completed as confirmed by TLC, water and a saturated aqueous sodium sulfite solution were added to the reaction solution. The mixture was stirred for 10 min and filtered, and the filter cake was collected and dried to give intermediate **24e** (6.0 g).

MS(ESI, $[M+H]^+$) *m/z*: 509.5.

$^1$H NMR (500 MHz, DMSO-$d_6$) δ 10.97 (s, 1H), 7.70 (d, *J* = 2.8 Hz, 1H), 7.60 (s, 1H), 7.44 - 7.36 (m, 2H), 7.27 (d, *J* = 2.8 Hz, 1H), 6.91 - 6.83 (m, 2H), 4.47 (t, *J* = 5.3 Hz, 1H), 4.31 (dd, *J* = 49.3, 12.9 Hz, 2H), 3.66 - 3.54 (m, 3H), 3.32 - 3.21 (m, 6H), 3.03 - 2.88 (m, 2H), 2.70 (s, 3H), 2.57 (td, *J* = 12.1, 2.6 Hz, 2H), 1.84 - 1.72 (m, 5H), 1.59 - 1.44 (m, 2H), 1.25 (tt, *J* = 12.1, 2.7 Hz, 2H).

Step 5: Preparation of intermediate **24f**

Intermediate **24e** (2.0 g), dichloromethane (30 mL), and *N*,*N*-diisopropylethylamine (1.36 g) were added to a reaction flask in sequence, and the mixture was cooled to 0 °C. Sulfur trioxide pyridine (1.88 g) was dissolved in DMSO (6 mL), and the solution was added dropwise to the reaction solution. After the dropwise addition was completed, the mixture was allowed to react at room temperature. After the reaction was completed, as confirmed by TLC, ethyl acetate and water were added to the reaction solution. The organic phase was separated, washed with a saturated sodium chloride solution, dried over anhydrous sodium sulfate, filtered, and concentrated. The concentrate was separated and purified by silica gel column chromatography to give intermediate **24f** (1.1 g).

MS(ESI, $[M+H]^+$) *m/z:* 507.4.

Step 6: Preparation of compound **24**

Intermediate **24f** (165 mg), **16** (100 mg), sodium acetate (26.7 mg), and dichloroethane/isopropanol (5:1, 20 mL) were added to a reaction flask in sequence. After the mixture was stirred at room temperature for 30 min, sodium cyanoborohydride (40.8 mg) was added, and the mixture was reacted at room temperature. After the reaction was completed as confirmed by TLC, dichloromethane and water were added to the reaction solution. The organic phase was separated, washed with a saturated sodium chloride solution, dried over anhydrous sodium sulfate, filtered, and concentrated. The concentrate was separated and purified by silica gel column chromatography to give compound **24** (100 mg).

MS(ESI, $[M+H]^+$) *m/z:* 761.6.

1H NMR (500 MHz, DMSO-d6) δ 11.10 (s, 1H), 10.98 (s, 1H), 7.72 (d, *J* = 7.9 Hz, 2H), 7.60 (s, 1H), 7.47 - 7.37 (m, 2H), 7.35 - 7.21 (m, 2H), 6.93 - 6.85 (m, 2H), 4.60 (dd, *J* = 11.9, 5.0 Hz, 1H), 4.32 (dd, *J* = 50.6, 12.8 Hz, 2H), 4.15 (s, 2H), 4.04 (t, *J* = 2.4 Hz, 2H), 3.69 - 3.55 (m, 3H), 3.35 (dd, *J* = 8.6, 6.2 Hz, 1H), 3.32 - 3.19 (m, 3H), 2.96 (dt, *J* = 28.7, 11.7 Hz, 2H), 2.77 (ddd, *J* = 17.2, 12.0, 5.3 Hz, 1H), 2.70 (s, 3H), 2.68 - 2.54 (m, 5H), 2.53 (s, 1H), 2.20 (dq, *J* = 13.5, 4.3 Hz, 1H), 1.96 - 1.64 (m, 6H), 1.61 - 1.48 (m, 1H), 1.36 - 1.27 (m, 2H).

**Example 25:** Synthesis of Compound **25**

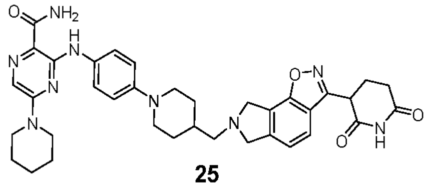

**25**

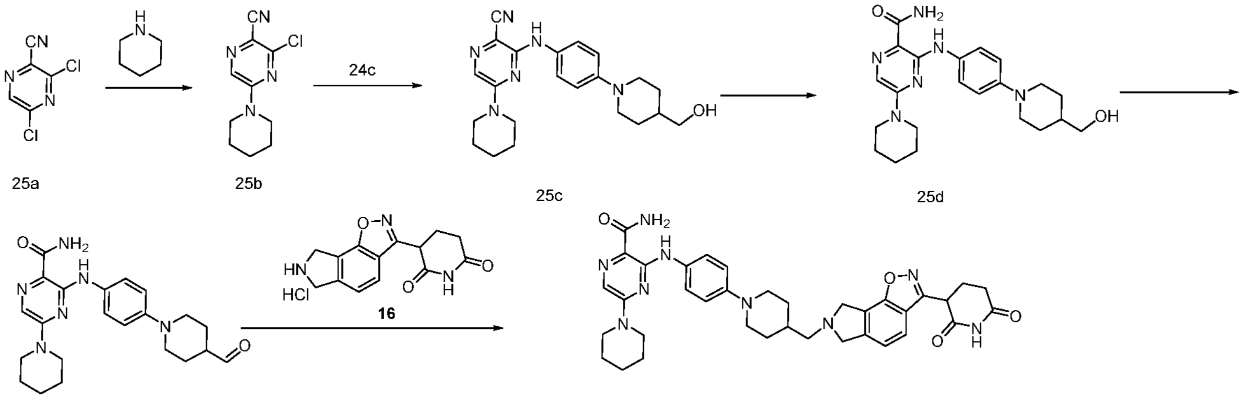


25a 25b 25c 25d

25e 25

Step 1: Preparation of intermediate **25b**

**25a** (5.0 g), tetrahydropyridine (3.49 g), DMF (40 mL), and N,N-diisopropylethylamine (18.57 g, 25.10 mL) were added to a reaction flask in sequence, and the mixture was reacted at room temperature. After the reaction was completed, as confirmed by TLC, water was added to quench the reaction, and ethyl acetate was added for extraction. The organic phase was dried over anhydrous sodium sulfate, filtered, and concentrated by evaporation at reduced pressure to remove the solvent, thus giving **25b** (6.6 g).

MS(ESI, [M+H]+) m/z: 223.23.

Step 2: Preparation of intermediate **25c**

**25b** (2.00 g), **24c** (6.86 g), BINAP (0.51 g), cesium carbonate (8.12 g), palladium acetate (0.18 g), and 1,4-dioxane (50 mL) were added to a reaction flask in sequence, and the mixture was purged three times with nitrogen, heated to 100 °C, and reacted. After the reaction was completed, as confirmed by TLC, the reaction solution was cooled to room temperature, and ethyl acetate and water were added to the residue. The organic phase was separated, washed with a saturated sodium chloride solution, dried over anhydrous sodium sulfate, filtered, and concentrated. The concentrate was separated and purified by silica gel column chromatography to give intermediate **25c** (2.3 g).

MS(ESI, $[M+H]^+$) *m/z:* 393.4.

Step 3: Preparation of intermediate **25d**

Intermediate **25c** (2.1 g), cesium carbonate (1.74 g), water (10 mL), DMSO (15 mL), and methanol (30 mL) were added to a reaction flask in sequence. The reaction solution was cooled to 0 °C, and 30 wt% hydrogen peroxide (1.82 g) was added dropwise. After the dropwise addition was completed, the mixture was allowed to react at room temperature. After the reaction was completed, as confirmed by TLC, water and a saturated aqueous sodium sulfite solution were added to the reaction solution. The mixture was stirred for 10 min and filtered, and the filter cake was collected and dried to give intermediate **25d** (2.1 g).

MS(ESI, $[M+H]^+$) *m/z:* 411.3.

Step 4: Preparation of intermediate **25e**

Intermediate **25d** (0.75 g), dichloromethane (20 mL), and N,N-diisopropylethylamine (0.63 g) were added to a reaction flask in sequence, and the mixture was cooled to 0 °C. Sulfur trioxide pyridine (0.87 g) was dissolved in DMSO (2 mL), and the solution was added dropwise to the reaction solution. After the dropwise addition was completed, the mixture was allowed to react at room temperature. After the reaction was completed, as confirmed by TLC, ethyl acetate and water were added to the reaction solution. The organic phase was separated, washed with a saturated sodium chloride solution, dried over anhydrous sodium sulfate, filtered, and concentrated. The concentrate was separated and purified by silica gel column chromatography to give intermediate **25e** (0.45 g).
MS(ESI, $[M+H]^+$) *m/z:* 409.3.

Step 5: Preparation of compound **25**

Intermediate **25e** (80 mg), **16** (50 mg), sodium acetate (13.3 mg), and dichloroethane/isopropanol (5:1, 20 mL) were added to a reaction flask in sequence. After the mixture was stirred at room temperature for 30 min, sodium cyanoborohydride (20.4 mg) was added, and the mixture was reacted at room temperature. After the reaction was completed, as confirmed by TLC, dichloromethane and water were added to the reaction solution. The organic phase was separated, washed with a saturated sodium chloride solution, dried over anhydrous sodium sulfate, filtered, and concentrated. The concentrate was separated and purified by silica gel column chromatography to give compound **25** (25 mg).
MS(ESI, $[M+H]^+$) *m/z:* 644.4.
1H NMR (500 MHz, DMSO-*d*6) $\delta$ 11.10 (s, 1H), 11.06 (s, 1H), 7.75 - 7.66 (m, 2H), 7.60 (s, 1H), 7.43 (d, *J* = 8.6 Hz, 2H), 7.31 (d, *J* = 8.1 Hz, 1H), 7.26 (d, *J* = 2.9 Hz, 1H), 6.92 (d, *J* = 8.6 Hz, 2H), 4.60 (dd, *J* = 11.9, 5.0 Hz, 1H), 4.16 (s, 2H), 4.05 (s, 2H), 3.73 - 3.55 (m, 6H), 2.77 (ddd, *J* = 17.3, 12.0, 5.4 Hz, 1H), 2.63 (ddd, *J* = 24.4, 12.5, 5.4 Hz, 5H), 2.55 (d, *J* = 4.9 Hz, 1H), 2.21 (dt, *J* = 13.3, 4.6 Hz, 1H), 1.93 - 1.85 (m, 2H), 1.74 - 1.62 (m, 3H), 1.57 (p, *J* = 5.5, 5.1 Hz, 4H), 1.31 (td, *J* = 11.7, 3.3 Hz, 2H).

**Example 26:** Synthesis of Compound **26**

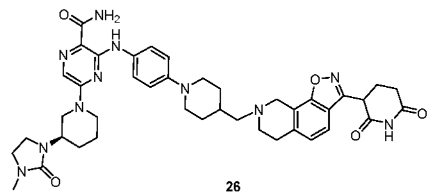

26

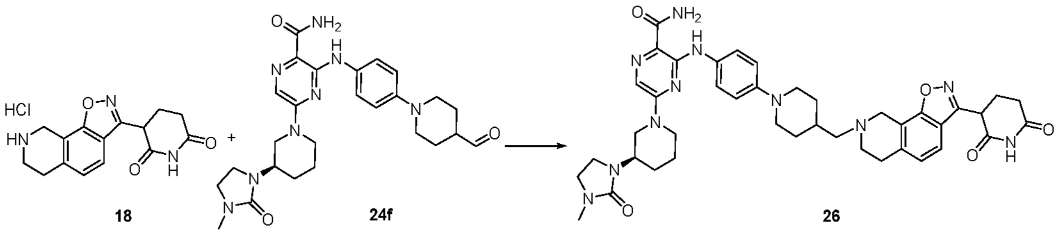

18 24f 26

Compound 18 (107 mg), intermediate **24f** (60 mg), acetic acid (6.31 mg, 6.02 μL), 1,2-dichloroethane (5 mL), and isopropanol (2 mL) were added to a reaction flask in sequence. After the mixture was stirred and reacted at room temperature for 0.5 h, sodium cyanoborohydride (39.6 mg) was added, and the mixture was stirred and reacted at room temperature. After the reaction was completed, as confirmed by TLC, a saturated sodium bicarbonate solution was added to the reaction solution to neutralize acetic acid, and then 50 mL of dichloromethane and 100 mL of water were added for extraction. The organic phase was separated, and the aqueous phase was extracted twice with 20 mL of dichloromethane. The organic phases were combined, washed with saturated brine, dried over anhydrous sodium sulfate, and filtered, and the filtrate was purified by silica gel column chromatography to give compound **26** (124 mg).
MS(ESI, $[M+H]^+$) m/z: 776.58.
$^1$H NMR (500 MHz, DMSO-d6) $\delta$ 11.09 (s, 1H), 10.97 (s, 1H), 7.71 (d, J = 2.9 Hz, 1H), 7.63 - 7.56 (m, 2H),

7.41 (d, J = 8.6 Hz, 2H), 7.28 (d, J = 2.9 Hz, 1H), 7.15 (d, J = 8.2 Hz, 1H), 6.87 (d, J = 8.6 Hz, 2H), 4.56 (dd, J = 11.9, 5.0 Hz, 1H), 4.36 (d, J = 12.6 Hz, 1H), 4.26 (d, J = 13.2 Hz, 1H), 3.81 (s, 2H), 3.64 - 3.56 (m, 3H), 3.27 (ddd, J = 29.3, 13.9, 6.4 Hz, 4H), 3.02 - 2.89 (m, 4H), 2.81 - 2.72 (m, 3H), 2.70 (s, 3H), 2.62 (ddd, J = 21.5, 10.8, 7.6 Hz, 3H), 2.53 (d, J = 4.9 Hz, 1H), 2.45 (d, J = 6.5 Hz, 2H), 2.18 (dq, J = 13.6, 4.8 Hz, 1H), 1.90 - 1.69 (m, 6H), 1.59 - 1.49 (m, 1H), 1.27 (d, J = 12.3 Hz, 2H).

**Example 27:** Synthesis of Compound **27**

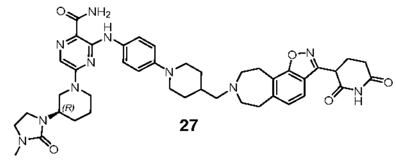

27

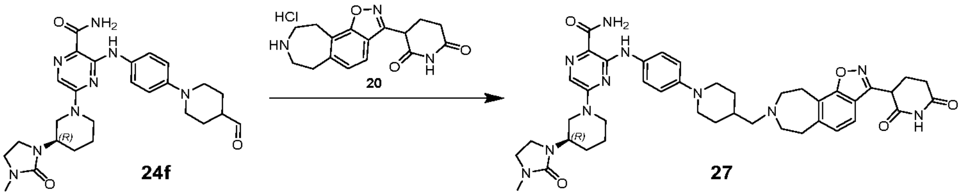

20

24f

27

Step 1: Preparation of compound **27**

Intermediate **24f** (113 mg), **20** (75 mg), sodium acetate (18.3 mg), and dichloroethane/isopropanol (5:1, 20 mL) were added to a reaction flask in sequence. After the mixture was stirred at room temperature for 30 min, sodium cyanoborohydride (28.1 mg) was added, and the mixture was reacted at room temperature for 1 h. 50 mL of dichloromethane and 100 mL of water were added to the reaction solution. The organic phase was separated, washed with a saturated sodium chloride solution, dried over anhydrous sodium sulfate, filtered, and concentrated. The concentrate was separated and purified by silica gel column chromatography to give compound **27** (90 mg).

MS(ESI, $[M+H]^+$) *m/z:* 790.6.

$^1$H NMR (500 MHz, DMSO-*d*6) δ 11.09 (s, 1H), 10.98 (s, 1H), 7.71 (d, *J* = 3.0 Hz, 1H), 7.60 (s, 1H), 7.54 (d, *J* = 8.0 Hz, 1H), 7.45 - 7.39 (m, 2H), 7.28 (d, *J* = 2.9 Hz, 1H), 7.18 (d, *J* = 8.2 Hz, 1H), 6.88 (d, *J* = 9.0 Hz, 2H), 4.55 (dd, *J* = 11.9, 5.0 Hz, 1H), 4.32 (dd, *J* = 47.1, 12.9 Hz, 2H), 3.61 (dd, *J* = 9.7, 5.8 Hz, 3H), 3.35 (dd, *J* = 7.4, 2.1 Hz, 1H), 3.31 - 3.22 (m, 3H), 3.16 (dd, *J* = 6.9, 2.9 Hz, 2H), 3.05 (dd, *J* = 6.5, 3.3 Hz, 2H), 2.97 (dt, *J* = 32.0, 12.3 Hz, 2H), 2.71 (s, 4H), 2.70 - 2.55 (m, 7H), 2.47 (dd, *J* = 12.2, 4.4 Hz, 1H), 2.36 (d, *J* = 7.1 Hz, 2H), 2.18 (dt, *J* = 9.1, 3.1 Hz, 1H), 1.88 - 1.63 (m, 6H), 1.61 - 1.50 (m, 1H), 1.32 - 1.24 (m, 2H).

**Example 28:** Synthesis of Compound **28**

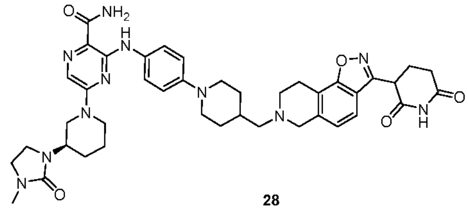

28

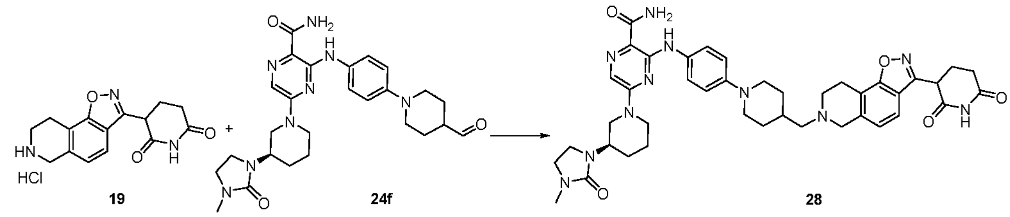

19 24f 28

Compound **19** (107 mg), intermediate **24f** (60 mg), acetic acid (6.31 mg, 6.02 μL, 0.105 mmol), 1,2-dichloroethane (5 mL), and isopropanol (2 mL) were added to a reaction flask in sequence. After the mixture was stirred and reacted at room temperature for 0.5 h, sodium cyanoborohydride (39.6 mg) was added, and the mixture was stirred and reacted at room temperature for 1 h. After the reaction was completed, 2 mL of a saturated sodium bicarbonate solution was added to the reaction solution to neutralize acetic acid, and then 50 mL of dichloromethane and 100 mL of water were added for extraction. The organic phase was separated, and the aqueous phase was extracted twice with 20 mL of dichloromethane. The organic phases were combined, washed with saturated brine, dried over anhydrous sodium sulfate, and filtered, and the filtrate was purified by silica gel column chromatography (DCM:MeOH = 10:1, v/v) to give compound **28** (96 mg).

MS(ESI, $[M+H]^+$) m/z: 776.40.

$^1H$ NMR (500 MHz, DMSO-d6) δ 11.09 (s, 1H), 10.98 (s, 1H), 7.71 (d, J = 3.0 Hz, 1H), 7.58 (d, J = 14.0 Hz, 2H), 7.41 (d, J = 8.5 Hz, 2H), 7.28 (d, J = 2.9 Hz, 1H), 7.11 (d, J = 8.2 Hz, 1H), 6.87 (d, J = 8.5 Hz, 2H), 4.56 (dd, J = 11.9, 5.0 Hz, 1H), 4.36 (d, J = 12.5 Hz, 1H), 4.26 (d, J = 13.3 Hz, 1H), 3.70 (s, 2H), 3.65 - 3.55 (m, 3H), 3.27 (ddd, J = 28.5, 15.0, 7.7 Hz, 4H), 3.05 - 2.88 (m, 4H), 2.77 (p, J = 6.4 Hz, 3H), 2.69 (s, 3H), 2.66 - 2.57 (m, 3H), 2.49 - 2.44 (m, 1H), 2.40 (d, J = 6.8 Hz, 2H), 2.19 (dt, J = 13.2, 4.7 Hz, 1H), 1.88 - 1.69 (m, 6H), 1.61 - 1.49 (m, 1H), 1.27 (q, J = 8.8, 5.1 Hz, 2H).

**Example 29:** Synthesis of Compound **29**

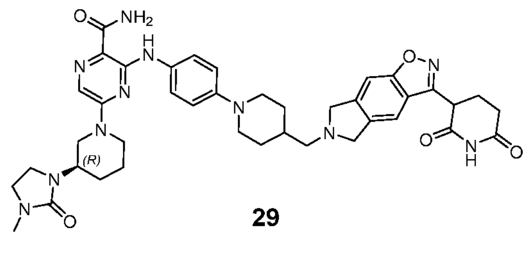

**29**

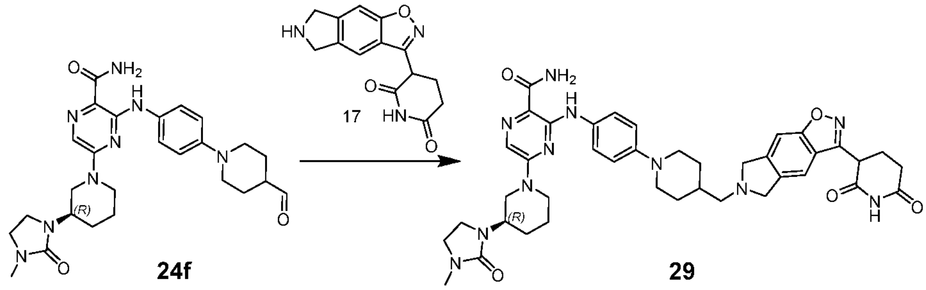

17

**24f** **29**

Step 1: Preparation of compound **29**

Intermediate **24f** (118 mg), **17** (100 mg), sodium acetate (21.2 mg), and dichloroethane/isopropanol (5:1, 20 mL) were added to a reaction flask in sequence. After the mixture was stirred at room temperature for 30 min, sodium cyanoborohydride (32.6 mg) was added, and the mixture was reacted at room temperature for 1 h. 50 mL of dichloromethane and 100 mL of water were added to the reaction solution. The organic phase was separated, washed with a saturated sodium chloride solution, dried over anhydrous sodium sulfate, filtered, and concentrated. The concentrate was separated and purified by silica gel column chromatography to give compound **29** (60 mg).

MS(ESI, $[M+H]^+$) *m/z:* 762.5.

1H NMR (500 MHz, DMSO-d6) δ 11.09 (s, 1H), 10.98 (s, 1H), 7.71 (d, *J* = 2.9 Hz, 1H), 7.65 (s, 1H), 7.60 (d, *J* = 2.9 Hz, 2H), 7.45 - 7.38 (m, 2H), 7.28 (d, *J* = 2.9 Hz, 1H), 6.94 - 6.83 (m, 2H), 4.55 (dd, *J* = 11.9, 5.0 Hz, 1H), 4.45 - 4.20 (m, 2H), 3.91 (d, *J* = 25.4 Hz, 4H), 3.60 (dd, *J* = 9.9, 5.9 Hz, 3H), 3.32 - 3.21 (m, 4H), 2.96 (dt, *J* = 28.1, 11.7 Hz, 2H), 2.77 (ddd, *J* = 17.3, 12.0, 5.4 Hz, 1H), 2.70 (s, 3H), 2.67 - 2.56 (m, 5H), 2.53 (d, *J* = 4.6 Hz, 1H), 2.19 (dq, *J* = 13.6, 4.9 Hz, 1H), 1.93 - 1.71 (m, 5H), 1.70 - 1.49 (m, 2H), 1.35 - 1.26 (m, 2H).

**Example 30:** Synthesis of Compound **30**

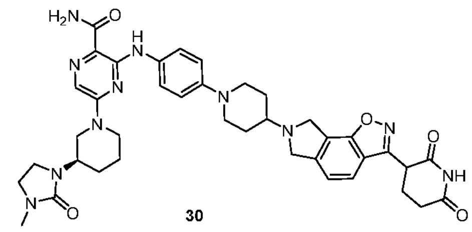

30

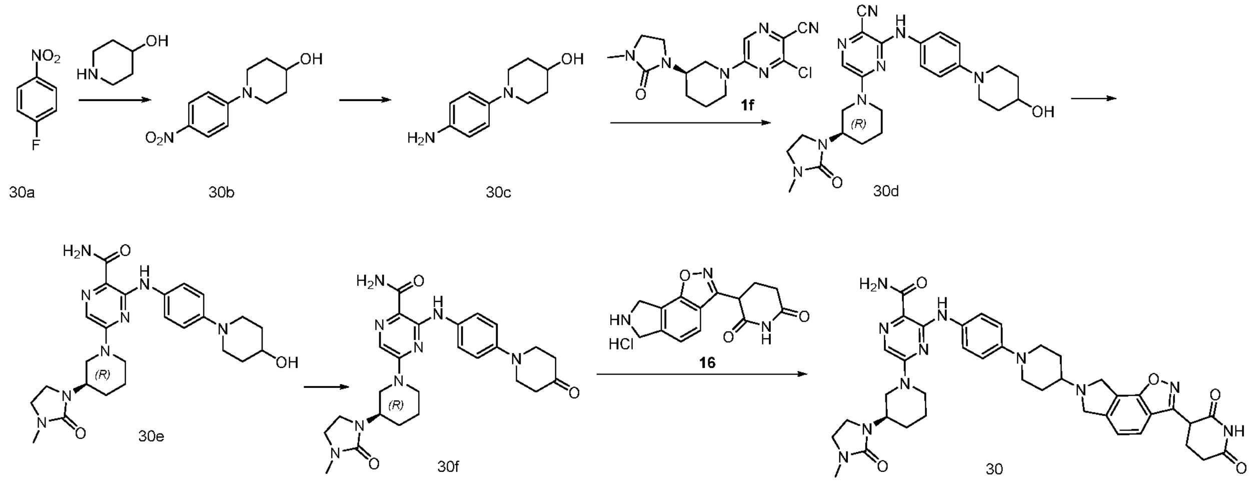

30a 30b 30c 1f 30d 30e 30f 16 30

Step 1: Preparation of intermediate **30b**

**30a** (6.5 g), DMSO (50 mL), piperidin-4-ol (5.13 g, 50.7 mmol), and N,N-diisopropylethylamine (17.86 g, 138 mmol) were added to a reaction flask in sequence, and the mixture was heated to 100 °C and reacted. The reaction solution was poured into ice water and filtered under vacuum, and the filter cake was dried to give **30b** (10.13 g).

MS(ESI, [M+H]+) m/z: 223.2.

Step 2: Preparation of intermediate **30c**

**30b** (10 g), 10% palladium on carbon (0.3 g, 0.282 mmol), and methanol (100 mL) were added to a reaction flask in sequence, and the mixture was purged 3 times with hydrogen and reacted at room temperature under hydrogen atmosphere. After the reaction was completed, as confirmed by TLC, palladium on carbon was removed by filtration under vacuum, and the filtrate was concentrated to give **30c** (8.45 g).

$^1$H NMR (500 MHz, DMSO-d6) δ 6.70 - 6.62 (m, 2H), 6.53 - 6.37 (m, 2H), 4.59 (d, *J* = 4.2 Hz, 1H), 4.51(s, 2H), 3.52 (tq, *J* = 8.5, 4.1 Hz, 1H), 3.20 (ddd, *J* = 12.4, 6.4, 2.7 Hz, 2H), 2.58 (ddd, *J* = 12.6, 10.4, 2.9 Hz, 2H), 1.84 - 1.70 (m, 2H), 1.47 (dtd, *J* = 13.0, 9.7, 3.8 Hz, 2H).

Step 3: Preparation of intermediate **30d**

Intermediate **1f** (5 g), **30c** (3.00 g), cesium carbonate (15.24 g), BINAP (0.971 g), palladium acetate (0.350 g), and 1,4-dioxane (100 mL) were added to a single-necked flask in sequence, and the mixture was heated to 100 °C and reacted under nitrogen atmosphere. After the reaction was completed, the reaction solution was cooled to room temperature, mixed with silica gel, and then purified by column chromatography to give **30d** (6.6 g).

$^1$H NMR (500 MHz, DMSO-*d*6) δ 8.78 (s, 1H), 7.75 (s, 1H), 7.40 - 7.29 (m, 2H), 7.00 - 6.78 (m, 2H), 4.66 (d, *J* = 4.2 Hz, 1H), 4.41 - 4.13 (m, 2H), 3.71 - 3.49 (m, 2H), 3.44 (dt, *J*= 12.3, 4.3 Hz, 2H), 3.31 - 3.19 (m, 4H), 3.00 - 2.83 (m, 2H), 2.77 (ddd, *J* = 12.6, 10.1, 2.9 Hz, 2H), 2.70 (s, 3H), 1.84 - 1.68 (m, 5H), 1.48 (tdd, *J* = 12.8, 9.3, 5.9 Hz, 3H).

Step 4: Preparation of intermediate **30e**

Intermediate **30d** (6.6 g), DMSO (25 mL), and cesium carbonate (4.08 g) were added to a single-necked flask in sequence. 30 wt% hydrogen peroxide (4.24 mL) was added under an ice bath. After the mixture was stirred for 5 min, the ice bath was removed, and the mixture was reacted at room temperature. After the reaction was completed, 300 mL of a saturated sodium sulfite solution was added to the residue to quench the reaction. The color was not changed as detected using a potassium iodide starch reagent, and then 300 mL of ethyl acetate was added for extraction. 100 mL of ethyl acetate was then added for 3 times of extraction. The organic phases were combined, dried over anhydrous sodium sulfate, filtered, and concentrated by evaporation at reduced pressure to remove the solvent. The crude product was separated by silica gel column chromatography to give the target intermediate **30e** (6.4 g).
MS(ESI, [M+H]+) m/z: 495.19

Step 5: Preparation of intermediate **30f**

N,N-diisopropylethylamine (8.49 g, 65.7 mmol) was added to a stirred solution of **30e** (6.5 g) in dichloromethane (60 mL) under an ice bath, and then a solution of sulfur trioxide pyridine (6.27 g) in DMSO (5 mL) was added. The mixture was reacted at room temperature. After the reaction was completed, 300 mL of a saturated sodium sulfite solution was added to the reaction solution to quench the reaction, and ethyl acetate was added for extraction. The organic phase was dried over anhydrous sodium sulfate and concentrated by evaporation at reduced pressure to remove the solvent, thus giving **30f** (5.9 g).

MS(ESI, [M+H]+) m/z: 492.47.

Step 6: Preparation of compound **30**

**30f** (0.15 g), isopropanol (1 mL), 1,2-dichloroethane (5.00 mL), and 16 (0.141 g) were added to a reaction flask in sequence, and sodium cyanoborohydride (0.057 g) was added. The mixture was reacted at room temperature. After the reaction was completed, the reaction solution was purified by silica gel column chromatography to give **30** (15 mg).

MS(ESI, [M+H]+) m/z: 748.35.

$^1$H NMR (500 MHz, DMSO-*d*6) δ 11.10 (s, 1H), 10.98 (s, 1H), 7.72 (d, *J*= 8.3 Hz, 2H), 7.60 (s, 1H), 7.43 (d, *J* = 8.4 Hz, 2H), 7.32 (d, *J* = 8.2 Hz, 1H), 7.29 - 7.22 (m, 1H), 6.91 (d, *J* = 8.6 Hz, 2H), 4.60 (dd, *J* = 11.9, 5.0 Hz, 1H), 4.36 (d, *J* = 12.4 Hz, 1H), 4.27 (d, *J* = 13.5 Hz, 1H), 4.22 (s, 2H), 4.11 (s, 2H), 3.58 (d, *J* = 12.1 Hz, 3H), 3.27 (dq, *J*= 15.0, 8.1, 7.2 Hz, 3H), 2.97 (dt, *J* = 32.4, 12.1 Hz, 2H), 2.77 (t, *J* = 11.8 Hz, 3H), 2.71 (m, 4H), 2.61 (dt, *J* = 17.3, 4.5 Hz, 2H), 2.57 - 2.52 (m, 1H), 2.25 - 2.14 (m, 1H), 2.04 (d, *J* = 12.3 Hz, 2H), 1.86 - 1.71 (m, 3H), 1.70 - 1.50 (m, 3H).

**Example 31:** Synthesis of Compound **31**

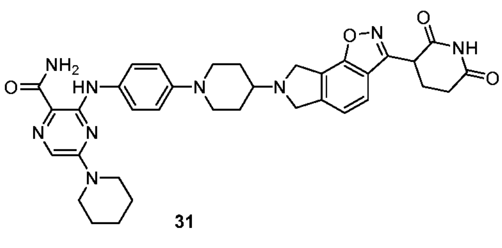

31

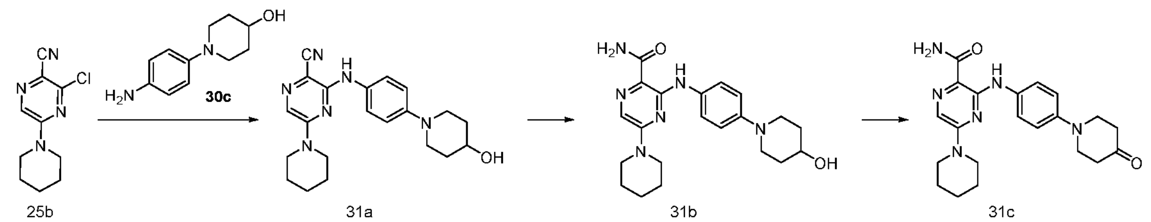


25b 31a 31b 31c

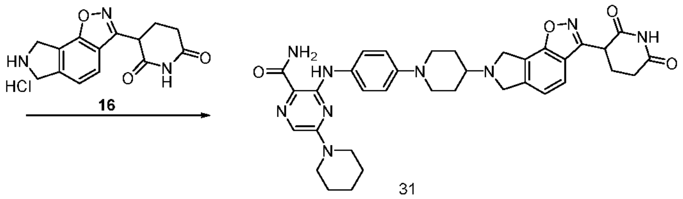


31

Step 1: Preparation of intermediate **31a**

Intermediate **25b** (1 g), **30c** (0.859 g), cesium carbonate (4.39 g), BINAP (0.280 g), palladium acetate (0.101 g), and 1,4-dioxane (10 mL) were added to a single-necked flask in sequence, and the mixture was heated to 100 °C and reacted under nitrogen atmosphere. After the reaction was completed, the reaction solution was cooled to room temperature, mixed with silica gel, and then purified by column chromatography to give **31a** (0.85 g).

MS(ESI, [M+H]+) m/z: 378.43.

Step 2: Preparation of intermediate **31b**

Intermediate **31a** (2.8 g), DMSO (10 mL), and cesium carbonate (2.4 g) were added to a single-necked flask in sequence. 30 wt% hydrogen peroxide (2.52 mL) was added under an ice bath. After the mixture was stirred for 5 min, the ice bath was removed, and the mixture was reacted at room temperature. After the reaction was completed, 300 mL of a saturated sodium sulfite solution was added to the residue to quench the reaction. The color was not changed as detected using a potassium iodide starch reagent, and then 300 mL of ethyl acetate was added for extraction. 100 mL of ethyl acetate was then added for 3 times of extraction. The organic phases were combined, dried over anhydrous sodium sulfate, filtered, and concentrated by evaporation at reduced pressure to remove the solvent. The crude product was separated by silica gel column chromatography to give the target intermediate **31b** (2.85 g).

MS(ESI, [M+H]+) m/z: 396.37.

Step 3: Preparation of intermediate **31c**

N,N-diisopropylethylamine (2.54 g) was added to a stirred solution of **31b** (2.6 g) in dichloromethane (30 mL) under an ice bath, and then a solution of sulfur trioxide pyridine (1.56 g) in DMSO (5 mL) was added. The mixture was reacted at room temperature. After the reaction was completed, 300 mL of a saturated sodium sulfite solution was added to the reaction solution to quench the reaction, and 200 mL of ethyl acetate was added for extraction. The organic phase was dried over anhydrous sodium sulfate and concentrated by evaporation at reduced pressure to remove the solvent, thus giving **31c** (1.85 g).

$^1$H NMR (500 MHz, DMSO-*d*6) δ 11.11 (s, 1H), 7.70 (d, *J* = 2.9 Hz, 1H), 7.61 (s, 1H), 7.52 - 7.43 (m, 2H), 7.27 (d, *J*= 3.0 Hz, 1H), 7.06 - 6.96 (m, 2H), 3.66 (t, *J*= 5.4 Hz, 4H), 3.53 (t, *J*= 6.0 Hz, 4H), 2.42 (t, *J*= 6.0 Hz, 4H), 1.62 (dq, *J* = 34.2, 5.9 Hz, 6H).

Step 4: Preparation of compound **31**

**31c** (0.1 g), isopropanol (1 mL), 1,2-dichloroethane (5.00 mL), and 16 (0.117 g) were added to a reaction flask in sequence, and sodium cyanoborohydride (0.048 g) was added. The mixture was reacted at room temperature. After the reaction was completed, the reaction solution was purified by silica gel column chromatography to give **31** (60 mg).

MS(ESI, [M+H]+) m/z: 649.44.

$^1$H NMR (500 MHz, DMSO-*d*6) δ 11.08 (d, *J* = 15.0 Hz, 2H), 7.72 (d, *J* = 8.0 Hz, 1H), 7.69 (d, *J* = 2.9 Hz, 1H), 7.60 (s, 1H), 7.47 - 7.41 (m, 2H), 7.32 (d, *J* = 8.2 Hz, 1H), 7.26 (d, *J* = 2.9 Hz, 1H), 6.97 - 6.92 (m, 2H), 4.60 (dd, *J* = 11.9, 5.0 Hz, 1H), 4.22 (s, 2H), 4.11 (s, 2H), 3.66 (t, *J* = 5.5 Hz, 4H), 3.59 (d, *J* = 12.0 Hz, 2H), 2.83 - 2.72 (m, 3H), 2.68 - 2.58 (m, 2H), 2.54 (dd, *J* = 10.0, 3.6 Hz, 1H), 2.26 - 2.15 (m, 1H), 2.04 (d, *J* = 12.2 Hz, 2H), 1.73 - 1.50 (m, 8H).

**Examples 32** and **33:** Synthesis of Compounds **32** and **33**

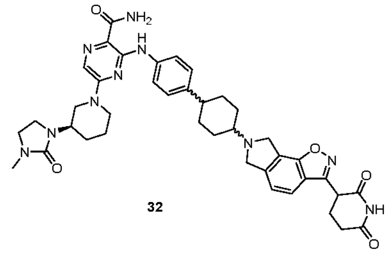

**32**

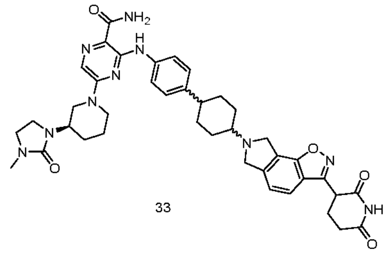

33

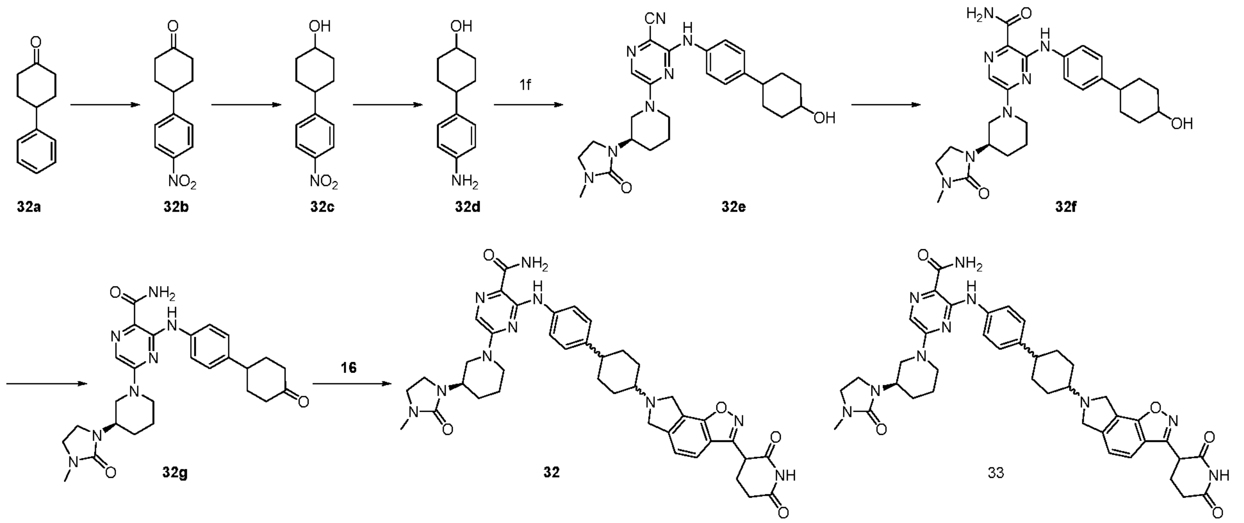


**32a** **32b** **32c** **32d** **32e** **32f** **32g** **32** 33

Step 1: Preparation of intermediate **32b**

**32a** (10 g), dichloromethane (100 mL), and sulfuric acid (11.26 g, 6.15 mL, 115 mmol) were added to a reaction flask in sequence, and nitric acid (4.70 g, 3.11 mL, 74.6 mmol) was added under an ice bath. The mixture was reacted at room temperature. After the reaction was completed, the reaction solution was added to a saturated sodium bicarbonate solution to quench the reaction, and 200 mL of ethyl acetate was added for extraction. The organic phase was dried over anhydrous sodium sulfate, filtered, and concentrated to give a crude product, and the crude product was slurried with a small amount of methyl tert-butyl ether and filtered under vacuum to give **32b** (7.43 g).

$^1H$ NMR (500 MHz, DMSO-*d*6) δ 8.24 - 8.14 (m, 2H), 7.64 - 7.57 (m, 2H), 3.25 (tt, *J* = 12.1, 3.4 Hz, 1H), 2.61 (td, *J*= 14.2, 6.0 Hz, 2H), 2.29 (ddt, *J*= 14.6, 4.3, 2.1 Hz, 2H), 2.09 (ddd, *J* = 12.8, 6.1, 3.1 Hz, 2H), 1.94 (qd, *J* = 13.0, 4.2 Hz, 2H).

Step 2: Preparation of intermediate **32c**

**30b** (7.4 g), MeOH (70 mL), and sodium borohydride (1.277 g) were added to a reaction flask in sequence under an ice bath, and the mixture was reacted at room temperature. After the reaction was completed, the reaction solution was added to a saturated ammonium chloride solution to quench the reaction, and 200 mL of ethyl acetate was added for extraction. The organic phase was dried over anhydrous sodium sulfate, filtered, and concentrated to give **32c** (6.05 g).

$^1H$ NMR (500 MHz, DMSO-*d*6) δ 8.20 - 8.11 (m, 2H), 7.52 (dd, *J* = 8.8, 1.9 Hz, 2H), 4.61 (dd, *J* = 4.4, 1.8 Hz, 1H), 3.48 (ddt, *J*= 10.9, 6.6, 4.5 Hz, 1H), 2.63 (tt, *J* = 12.1, 3.4 Hz, 1H), 1.99 - 1.89 (m, 2H), 1.83 - 1.75 (m, 2H), 1.51 (qd, *J* = 13.0, 11.0, 3.4 Hz, 2H), 1.37 - 1.25 (m, 2H).

Step 3: Preparation of intermediate **32d**

**32c** (6 g), iron powder (4.54 g), ammonium chloride (6 g), MeOH (60 mL), and 20 mL of water were added to

a reaction flask in sequence, and the mixture was warmed to 80 °C and reacted. After the reaction was completed, the solid was removed by filtration under vacuum, 200 mL of ethyl acetate was added to the filtrate, and 200 mL of a saturated sodium carbonate solution was added for extraction. The organic phase was dried over anhydrous sodium sulfate, filtered, and concentrated to give **32d** (2.35 g).

$^1$H NMR (500 MHz, DMSO-d6) δ 6.93 - 6.80 (m, 2H), 6.54 - 6.41 (m, 2H), 4.77 (s, 2H), 4.50 (d, *J* = 4.4 Hz, 1H), 3.40 (ddt, *J* = 15.0, 10.7, 4.3 Hz, 1H), 2.24 (tt, *J* = 12.0, 3.5 Hz, 1H), 1.92 - 1.81 (m, 2H), 1.68 (dt, *J* = 12.8, 2.9 Hz, 2H), 1.43 - 1.30 (m, 2H), 1.30 - 1.19 (m, 2H).

Step 4: Preparation of intermediate **32e**

Intermediate **1f** (1 g), **32d** (0.775 g), cesium carbonate (3.05 g), BINAP (0.194 g), palladium acetate (0.070 g), and 1,4-dioxane (10 mL) were added to a single-necked flask in sequence, and the mixture was heated to 100 °C and reacted under nitrogen atmosphere. After the reaction was completed, the reaction solution was cooled to room temperature, mixed with silica gel, and then purified by column chromatography to give **32e** (1.31 g).

MS(ESI, [M+H]+) m/z: 476.50.

Step 5: Preparation of intermediate **32f**

Intermediate **32e** (1.3 g), DMSO (5 mL), and cesium carbonate (0.891 g) were added to a single-necked flask in sequence. 30 wt% hydrogen peroxide (0.930 mL) was added under an ice bath. After the mixture was stirred for 5 min, the ice bath was removed, and the mixture was reacted at room temperature. After the reaction was completed, 300 mL of a saturated sodium sulfite solution was added to the residue to quench the reaction. The color was not changed as detected using a potassium iodide starch reagent, and then 300 mL of ethyl acetate was added for extraction. 100 mL of ethyl acetate was then added for 3 times of extraction. The organic phases were combined, dried over anhydrous sodium sulfate, filtered, and concentrated by evaporation at reduced pressure to remove the solvent. The crude product was separated by silica gel column chromatography to give the target intermediate **32f** (1.21 g).

MS(ESI, $[M+H]^+$) m/z: 494.47.

Step 6: Preparation of intermediate **32g**

**32f** (1.5 g), IBX (1.702 g), and DMSO (15 mL) were added to a reaction flask in sequence, and the mixture was reacted at room temperature. After the reaction was completed, 200 mL of ethyl acetate was added, and 200 mL of a saturated sodium bicarbonate solution was added for extraction. The organic phase was dried over anhydrous sodium sulfate, filtered, and concentrated to give **32g** (1.02 g).

MS(ESI, [M+H]+) m/z: 492.40.

Step 7: Preparation of compounds **32** and **33**

**32g** (200 mg), isopropanol (1 mL), 1,2-dichloroethane (5.00 mL), and **16** (188 mg) were added to a reaction flask in sequence, and sodium cyanoborohydride (77 mg) was added. The mixture was reacted at room temperature. After the reaction was completed, the reaction solution was purified by silica gel column chromatography (polarity of developing solvents: MeOH:DCM = 0:100 → 1:25) to give **32** (21 mg) and **33** (26 mg) in sequence.

**32:** MS(ESI, [M+H]+) m/z: 747.39.-
$^1$H NMR (500 MHz, DMSO-*d*6) δ 11.16 (s, 1H), 11.10 (s, 1H), 7.78 - 7.69 (m, 2H), 7.63 (s, 1H), 7.46 (d, *J* = 8.2 Hz, 2H), 7.39 - 7.28 (m, 2H), 7.12 (d, *J* = 8.3 Hz, 2H), 4.61 (dd, *J* = 11.9, 4.9 Hz, 1H), 4.44 - 4.33 (m, 1H), 4.24 (d, *J* = 13.5 Hz, 1H), 4.17 (s, 2H), 4.06 (s, 2H), 3.56 (dq, *J* = 11.0, 5.6, 4.3 Hz, 1H), 3.26 (dd, *J* = 9.3, 6.9 Hz, 2H), 3.05 (t, *J* = 7.7 Hz, 2H), 2.94 (q, *J* = 13.3 Hz, 2H), 2.78 (dq, *J* = 17.1, 5.7, 5.2 Hz, 2H), 2.66 - 2.60 (m, 1H), 2.60 - 2.53 (m, 2H), 2.45 (s, 3H), 2.27 - 2.15 (m, 1H), 2.06 (d, *J* = 13.3 Hz, 2H), 1.82 (dt, *J* = 37.4, 12.5 Hz, 4H), 1.68 (dt, *J* = 26.8, 12.6 Hz, 3H), 1.54 (t, *J* = 14.8 Hz, 3H).
**33:** MS(ESI, [M+H]+) m/z: 747.55.
$^1$H NMR (500 MHz, DMSO-*d*6) δ 11.20 (s, 1H), 11.10 (s, 1H), 7.76 (d, *J* = 2.9 Hz, 1H), 7.72 (d, *J* = 8.0 Hz, 1H), 7.66 (s, 1H), 7.50 (d, *J* = 8.2 Hz, 2H), 7.36 - 7.29 (m, 2H), 7.17 (d, *J* = 8.2 Hz, 2H), 4.60 (dd, *J* = 11.9, 5.0 Hz, 1H), 4.32 (dd, *J* = 33.0, 12.7 Hz, 2H), 4.23 (s, 2H), 4.12 (s, 2H), 3.62 (ddt, *J* = 11.2, 8.6, 4.2 Hz, 1H), 3.38 - 3.33 (m, 2H), 3.30 - 3.22 (m, 2H), 3.00 (dt, *J* = 40.8, 11.5 Hz, 2H), 2.82 - 2.68 (m, 4H), 2.66 - 2.59 (m, 1H), 2.58 - 2.51 (m, 2H), 2.47 (d, *J*= 11.2 Hz, 1H), 2.26 - 2.11 (m, 3H), 1.93 - 1.72 (m, 5H), 1.48 (dp, *J* = 49.1, 12.0 Hz, 5H).

**Example 34:** Synthesis of Compound **34**

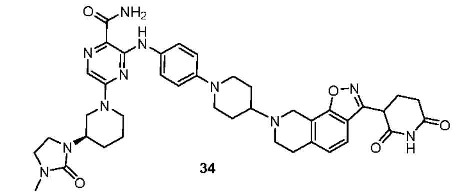

34

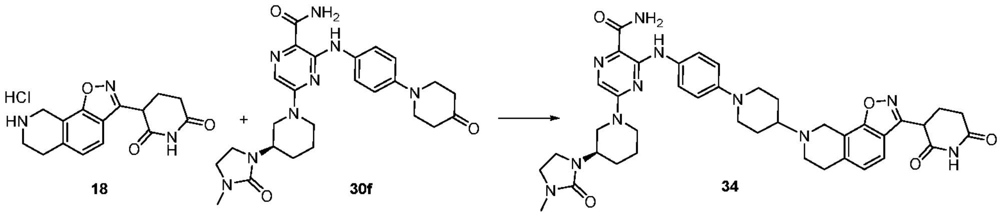

18 + 30f → 34

Compound **18** (116 mg), intermediate **30f** (200 mg), acetic acid (12.21 mg, 0.012 mL, 0.203 mmol), 1,2-dichloroethane (10 mL), and isopropanol (3 mL) were added to a reaction flask in sequence. After the mixture was stirred and reacted at room temperature for 0.5 h, sodium cyanoborohydride (77 mg, 1.220 mmol) was added, and the mixture was stirred and reacted at room temperature. After the reaction was completed, as confirmed by TLC, 2 mL of a saturated sodium bicarbonate solution was added to the reaction solution to neutralize acetic acid, and then 50 mL of dichloromethane and 100 mL were added for extraction. The organic phase was separated, and the aqueous phase was extracted twice with 20 mL of dichloromethane. The organic phases were combined, washed with 100 mL of saturated brine, dried over anhydrous sodium sulfate, and filtered, and the filtrate was purified by silica gel column chromatography to give compound **34** (84 mg).

MS(ESI, $[M+H]^+$) m/z: 761.55.

$^1H$ NMR (500 MHz, DMSO-d6) δ 11.09 (s, 1H), 10.99 (s, 1H), 7.71 (d, J = 2.9 Hz, 1H), 7.59 (d, J = 14.5 Hz, 2H), 7.43 (d, J = 8.7 Hz, 2H), 7.28 (d, J = 3.0 Hz, 1H), 7.14 (d, J = 8.2 Hz, 1H), 6.91 (d, J = 8.7 Hz, 2H), 4.56 (dd, J = 11.9, 5.0 Hz, 1H), 4.41 - 4.32 (m, 1H), 4.27 (d, J = 13.3 Hz, 1H), 3.98 (s, 2H), 3.68 (d, J = 11.7 Hz, 2H), 3.60 (dq, J = 8.1, 5.3, 4.1 Hz, 1H), 3.32 - 3.20 (m, 4H), 3.02 - 2.85 (m, 6H), 2.80 - 2.74 (m, 1H), 2.71 (s, 3H), 2.70 - 2.65 (m, 2H), 2.60 (dt, J = 17.3, 4.3 Hz, 2H), 2.47 (dd, J = 12.3, 4.5 Hz, 1H), 2.19 (dq, J = 13.7, 4.8 Hz, 1H), 1.97 (d, J = 11.9 Hz, 2H), 1.81 (d, J = 11.4 Hz, 2H), 1.78 - 1.63 (m, 3H), 1.59 - 1.50 (m, 1H).

**Example 35:** Synthesis of Compound **35**

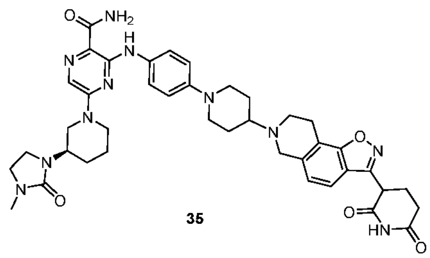

35

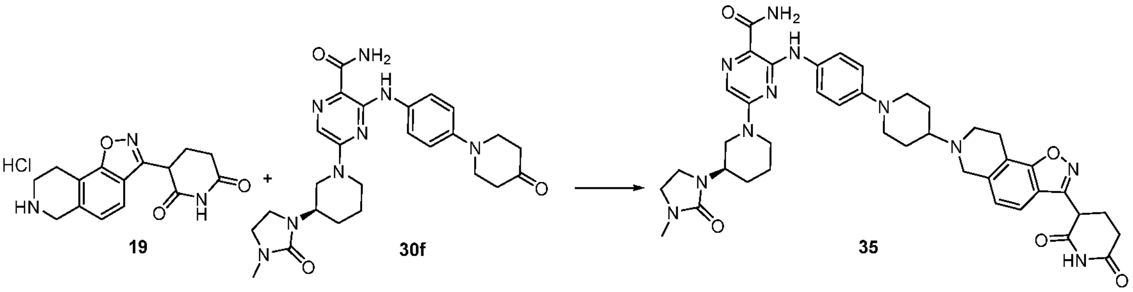

19 30f 35

Compound **19** (113 mg), intermediate **30f** (195 mg), acetic acid (71.4 mg, 0.068 mL, 1.188 mmol), 1,2-dichloroethane (10 mL), and isopropanol (3 mL) were added to a reaction flask in sequence. After the mixture was stirred and reacted at room temperature for 0.5 h, sodium cyanoborohydride (74.7 mg, 1.188 mmol) was added, and the mixture was stirred and reacted at room temperature for 1 h. After the reaction was completed, 2 mL of a saturated sodium bicarbonate solution was added to the reaction solution to neutralize acetic acid, and then 50 mL of dichloromethane and 100 mL of water were added for extraction. The organic phase was separated, and the aqueous phase was extracted twice with 20 mL of dichloromethane. The organic phases were combined, washed with saturated brine, dried over anhydrous sodium sulfate, and filtered, and the filtrate was purified by silica gel column chromatography to give compound **35** (80 mg).

MS(ESI, $[M+H]^+$) m/z: 761.44.

$^1$H NMR (500 MHz, DMSO-d6) δ 11.08 (s, 1H), 10.99 (s, 1H), 7.71 (d, J = 2.9 Hz, 1H), 7.58 (d, J = 21.1 Hz, 2H), 7.43 (d, J = 8.6 Hz, 2H), 7.28 (d, J = 3.0 Hz, 1H), 7.12 (d, J = 8.3 Hz, 1H), 6.90 (d, J = 8.6 Hz, 2H), 4.56 (dd, J = 11.8, 5.0 Hz, 1H), 4.36 (d, J = 12.5 Hz, 1H), 4.27 (d, J = 13.3 Hz, 1H), 3.88 (s, 2H), 3.67 (d, J = 11.7 Hz, 2H), 3.61 (dq, J = 8.0, 5.3, 4.1 Hz, 1H), 3.32 - 3.20 (m, 4H), 3.03 - 2.87 (m, 6H), 2.80 - 2.74 (m, 1H), 2.71 (s, 3H), 2.69 - 2.57 (m, 4H), 2.50 - 2.43 (m, 1H), 2.19 (dq, J = 13.6, 4.8 Hz, 1H), 1.99 - 1.90 (m, 2H), 1.81 (d, J = 10.9 Hz, 2H), 1.75 (dd, J = 11.6, 3.6 Hz, 1H), 1.69 (t, J = 12.3 Hz, 2H), 1.59 - 1.51 (m, 1H).

**Example 36:** Synthesis of Compound **36**

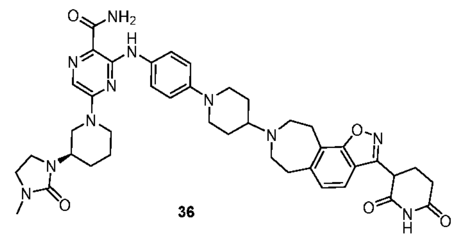

36

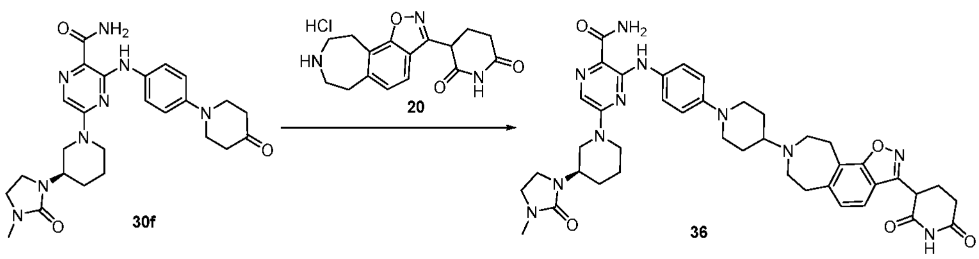

30f 20 36

Step 1: Preparation of compound **36**

**30f** (150 mg), isopropanol (1 mL), 1,2-dichloroethane (5.00 mL), and **20** (102 mg) were added to a reaction flask in sequence, and sodium cyanoborohydride (57 mg) was added. The mixture was reacted at 80 °C. After the reaction was completed, the reaction solution was purified by silica gel column chromatography to give **36** (15 mg).

MS(ESI, [M+H]+) m/z: 776.59.

$^1$H NMR (500 MHz, DMSO-d6) δ 11.09 (s, 1H), 10.98 (s, 1H), 7.71 (d, *J* = 2.9 Hz, 1H), 7.60 (s, 1H), 7.54 (d, *J*

= 8.0 Hz, 1H), 7.41 (d, *J*= 8.6 Hz, 2H), 7.28 (d, *J*= 2.8 Hz, 1H), 7.19 (d, *J*= 8.1 Hz, 1H), 6.88 (d, *J*= 8.5 Hz, 2H), 4.55 (dd, *J*= 11.9, 5.0 Hz, 1H), 4.31 (dd, *J* = 44.9, 11.8 Hz, 2H), 3.68 - 3.55 (m, 3H), 3.27 (dq, *J* = 20.6, 8.1, 7.7 Hz, 4H), 3.19 - 3.10 (m, 2H), 3.03 (s, 2H), 3.00 - 2.87 (m, 2H), 2.75 (dq, *J* = 22.8, 6.1, 5.7 Hz, 5H), 2.70 (s, 4H), 2.66 - 2.55 (m, 3H), 2.49 (s, 1H), 2.18 (dt, *J*= 13.4, 4.6 Hz, 1H), 1.86 - 1.68 (m, 5H), 1.58 (dd, *J*= 35.3, 12.4 Hz, 3H).

**Example 37:** Synthesis of Compound **37**

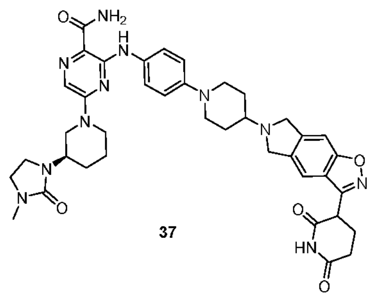

**37**

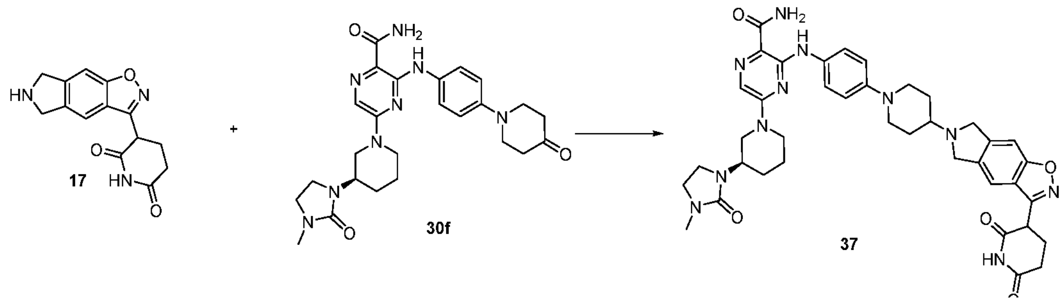

**17** + **30f** → **37**

Step 1: Preparation of compound **37**

**30f** (150 mg), isopropanol (1 mL), 1,2-dichloroethane (5.00 mL), and **17** (140 mg) were added to a reaction flask in sequence, and sodium cyanoborohydride (57 mg) was added. The mixture was reacted at 80 °C. After the reaction was completed, the reaction solution was purified by silica gel column chromatography to give 37 (25 mg).

MS(ESI, [M+H]+) m/z: 748.53.

$^1$H NMR (500 MHz, DMSO-d6) δ 11.09 (s, 1H), 10.98 (s, 1H), 7.71 (d, *J*= 2.9 Hz, 1H), 7.67 (s, 1H), 7.61 (d, *J* = 9.2 Hz, 2H), 7.47 - 7.40 (m, 2H), 7.28 (d, *J* = 2.9 Hz, 1H), 6.95 - 6.87 (m, 2H), 4.56 (dd, *J* = 11.9, 5.0 Hz, 1H), 4.31 (dd, *J* = 44.1, 12.7 Hz, 2H), 4.02 (s, 2H), 3.96 (s, 2H), 3.67 - 3.52 (m, 3H), 3.34 (d, *J* = 4.9 Hz, 1H), 3.26 (ddd, *J* = 13.9, 11.1, 6.9 Hz, 3H), 2.97 (dt, *J* = 33.5, 12.0 Hz, 2H), 2.77 (td, *J* = 11.8, 11.4, 4.7 Hz, 3H), 2.71 (s, 3H), 2.65 - 2.52 (m, 3H), 2.19 (dq, *J* = 13.7, 5.0 Hz, 1H), 2.00 (dd, *J* = 10.6, 5.3 Hz, 2H), 1.85 - 1.71 (m, 3H), 1.59 (p, *J* = 16.0, 13.3 Hz, 3H).

**Examples 38** and **39:** Synthesis of Compounds **38** and **39**

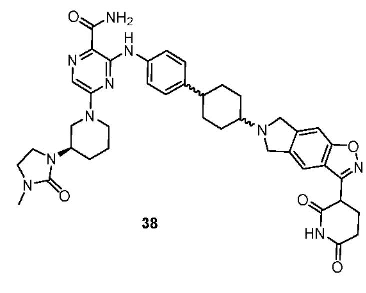

**38**

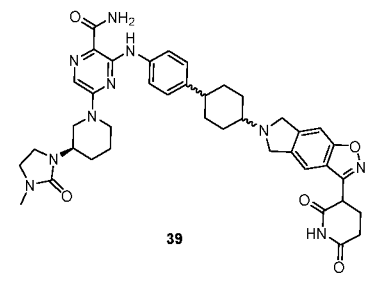

**39**

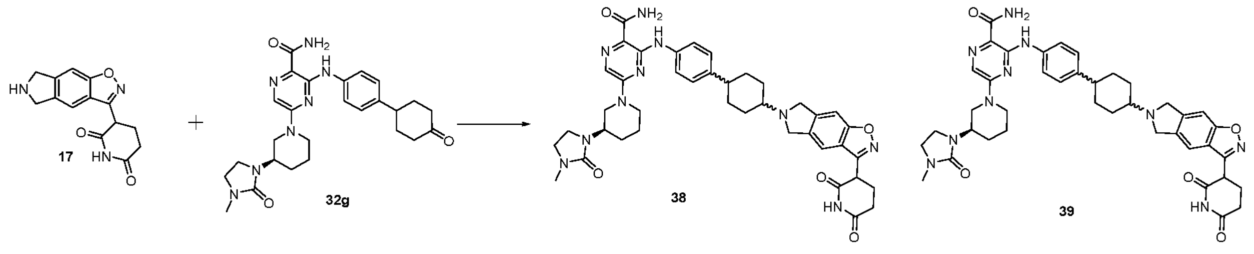

17 32g 38 39

Step 1: Preparation of compounds **38** and **39**

**32g** (130 mg), isopropanol (1 mL), 1,2-dichloroethane (5.00 mL), and **17** (122 mg) were added to a reaction flask in sequence, and sodium cyanoborohydride (49.9 mg) was added. The mixture was reacted at room temperature. After the reaction was completed, the reaction solution was mixed with silica gel and purified by column chromatography (MeOH:DCM = 0:100 → 1:25) to give **38** (17 mg) and **39** (20 mg) in sequence.

**38:** MS(ESI, [M+H]+) m/z: 747.54.

$^1$H NMR (500 MHz, DMSO-*d6*) δ 11.16 (s, 1H), 11.09 (s, 1H), 7.73 (s, 1H), 7.68 (s, 1H), 7.64 (d, *J* = 4.6 Hz, 2H), 7.45 (d, *J* = 8.2 Hz, 2H), 7.31 (s, 1H), 7.11 (d, *J* = 8.2 Hz, 2H), 4.61 - 4.53 (m, 1H), 4.38 (d, *J* = 12.5 Hz, 1H), 4.24 (d, *J* = 13.4 Hz, 1H), 3.95 (d, *J* = 27.6 Hz, 4H), 3.62 - 3.52 (m, 1H), 3.30 - 3.22 (m, 2H), 3.07 (t, *J* = 7.7 Hz, 2H), 2.94 (q, *J* = 12.8, 12.3 Hz, 2H), 2.84 - 2.69 (m, 2H), 2.65 - 2.59 (m, 1H), 2.59 - 2.53 (m, 2H), 2.47 (d, *J* = 3.1 Hz, 3H), 2.25 - 2.17 (m, 1H), 2.02 (d, *J* = 13.6 Hz, 2H), 1.78 (tt, *J* = 26.4, 12.2 Hz, 5H), 1.63 (t, *J* = 13.3 Hz, 2H), 1.54 (d, *J* = 11.7 Hz, 3H).

**39:** MS(ESI, [M+H]+) m/z: 747.53.

$^1$H NMR (500 MHz, DMSO-*d6*) δ 11.20 (s, 1H), 11.09 (s, 1H), 7.76 (d, *J* = 2.9 Hz, 1H), 7.66 (d, *J* = 2.5 Hz, 2H), 7.61 (s, 1H), 7.50 (d, *J* = 8.1 Hz, 2H), 7.33 (d, *J* = 2.8 Hz, 1H), 7.17 (d, *J* = 8.4 Hz, 2H), 4.56 (dd, *J* = 11.9, 5.0 Hz, 1H), 4.32 (dd, *J* = 32.1, 12.1 Hz, 2H), 3.99 (d, *J* = 26.3 Hz, 4H), 3.61 (tt, *J* = 10.0, 3.9 Hz, 1H), 3.38 - 3.33 (m, 2H), 3.26 (dd, *J* = 10.1, 7.1 Hz, 2H), 3.00 (dt, *J* = 42.5, 12.1 Hz, 2H), 2.83 - 2.69 (m, 4H), 2.61 (dt, *J* = 17.3, 4.4 Hz, 1H), 2.46 (d, *J* = 11.6 Hz, 2H), 2.24 - 2.07 (m, 3H), 1.94 - 1.73 (m, 5H), 1.62 - 1.31 (m, 6H).

**Example 40:** Synthesis of Compound **40**

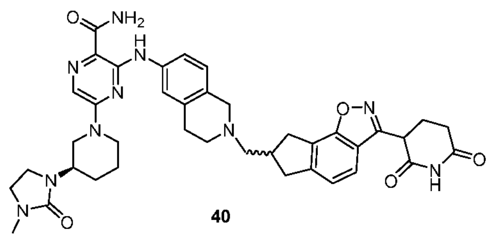

40

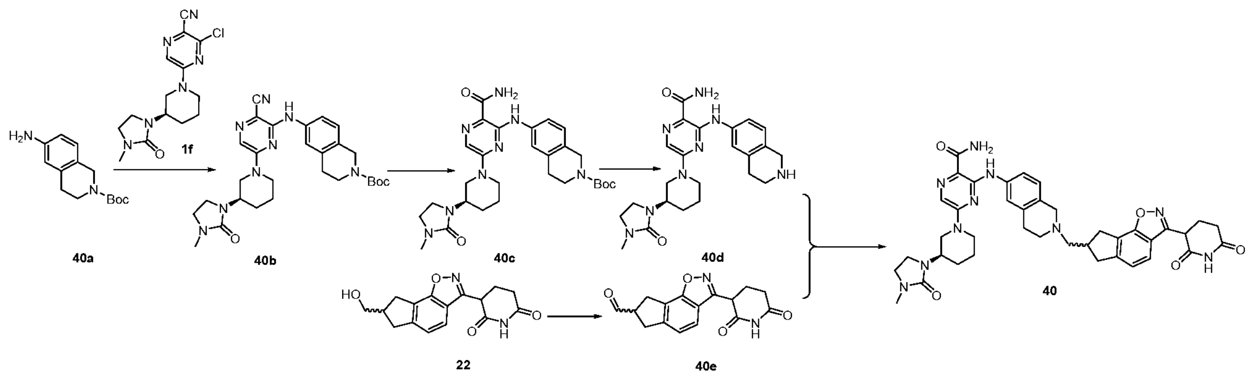

40a 40b 40c 40d 40 22 40e

Step 1: Preparation of intermediate **40b**

Intermediate **1f** (1.5 g), intermediate **40a** (1.161 g), BINAP (0.291 g), cesium carbonate (4.57 g), palladium acetate (0.105 g, 0.468 mmol), and 1,4-dioxane (20 mL) were added to a reaction flask in sequence, and the mixture

was heated to 100 °C and reacted under $N_2$ atmosphere. After the reaction was completed, as confirmed by TLC, the reaction solution was diluted with 20 mL of dichloromethane and purified by silica gel column chromatography to give the target intermediate **40b** (2.2 g).

MS(ESI, $[M+H]^+$) m/z: 533.4.

$^1$H NMR (500 MHz, DMSO-d6) δ 8.98 (s, 1H), 7.83 (s, 1H), 7.43 (d, J = 2.2 Hz, 1H), 7.29 (dd, J = 8.3, 2.2 Hz, 1H), 7.05 (d, J = 8.3 Hz, 1H), 4.44 (s, 2H), 4.38 - 4.19 (m, 2H), 3.57 (dt, J = 11.6, 5.5 Hz, 1H), 3.52 (t, J = 6.0 Hz, 2H), 3.28 (td, J = 6.8, 3.7 Hz, 3H), 3.24 - 3.18 (m, 1H), 2.99 (s, 1H), 2.95 - 2.87 (m, 1H), 2.68 (s, 5H), 1.83 - 1.77 (m, 1H), 1.77 - 1.68 (m, 2H), 1.56 - 1.49 (m, 1H), 1.43 (s, 9H).

Step 2: Preparation of intermediate **40c**

Intermediate **40b** (2.2 g), DMSO (8 mL), methanol (20 mL), cesium carbonate (1.346 g), and water (8 mL) were added to a reaction flask in sequence, and 30 wt% hydrogen peroxide (1.405 g, 1.405 mL, 12.39 mmol) was added under an ice bath. The mixture was allowed to return to room temperature and react. After the reaction was completed, as confirmed by TLC, the reaction solution was poured into an icy saturated sodium sulfite solution to quench the reaction, and ethyl acetate was added for extraction. The organic phase was separated, washed with a saturated sodium chloride solution, dried over anhydrous sodium sulfate, and filtered, and the filtrate was concentrated by evaporation at reduced pressure to remove the solvent, thus giving the target intermediate **40c** (2.1 g).

MS(ESI, $[M+H]^+$) m/z: 551.40.

$^1$H NMR (500 MHz, DMSO-d6) δ 11.28 (s, 1H), 7.81 - 7.75 (m, 1H), 7.70 - 7.60 (m, 2H), 7.38 (s, 1H), 7.23 - 7.16 (m, 1H), 7.06 (d, J = 8.5 Hz, 1H), 4.44 (s, 2H), 4.38 (d, J = 12.5 Hz, 1H), 4.26 (d, J = 13.3 Hz, 1H), 3.63 (dq, J = 11.4, 5.9, 5.2 Hz, 1H), 3.53 (t, J = 5.8 Hz, 2H), 3.35 (s, 1H), 3.33 - 3.20 (m, 3H), 3.01 (t, J = 12.0 Hz, 1H), 2.96 - 2.87 (m, 1H), 2.69 (d, J = 3.9 Hz, 5H), 1.77 (dddd, J = 27.7, 23.7, 12.4, 3.9 Hz, 3H), 1.63 - 1.51 (m, 1H), 1.43 (s, 9H).

Step 3: Preparation of intermediate **40d**

Intermediate **40c** (2.1 g) and TFA (8.70 g, 5.88 mL, 76 mmol) were added to a reaction flask in sequence, and the mixture was reacted at room temperature. After the reaction was completed, as confirmed by TLC, the reaction solution was concentrated by evaporation at reduced pressure to remove the solvent, 20 mL of dichloromethane was added, and the mixture was subjected to rotary evaporation. The process was repeated 2 times. The residue was completely dissolved in 10 mL of dichloromethane, then the solution was added dropwise to 100 mL of an icy 1 M sodium hydroxide solution, and 100 mL of dichloromethane was added for extraction. The organic phase was separated, and the aqueous phase was extracted twice with 50 mL of dichloromethane. The organic phases were combined, washed with saturated brine, dried over anhydrous sodium sulfate, and filtered, and the filtrate was concentrated by evaporation at reduced pressure to remove the solvent, thus giving the target intermediate **40d** (1.7 g).

MS(ESI, $[M+H]^+$) m/z: 451.45.

$^1$H NMR (500 MHz, DMSO-d6) δ 11.25 (d, J = 19.0 Hz, 1H), 7.77 (dd, J = 5.6, 2.8 Hz, 1H), 7.67 (d, J = 6.0 Hz, 1H), 7.57 (dd, J = 7.5, 2.2 Hz, 1H), 7.36 (dq, J = 6.2, 3.1 Hz, 1H), 7.16 (ddd, J = 34.2, 8.2, 2.3 Hz, 1H), 6.98 (dd, J = 11.8, 8.3 Hz, 1H), 4.37 (d, J = 12.5 Hz, 1H), 4.28 (d, J = 13.3 Hz, 1H), 3.94 (s, 1H), 3.61 (tdd, J = 15.2, 9.5, 5.2 Hz, 2H), 3.37 - 3.18 (m, 5H), 3.10 - 3.04 (m, 2H), 2.94 (td, J = 12.8, 2.8 Hz, 1H), 2.75 (d, J = 8.0 Hz, 2H), 2.68 (s, 2H), 2.64 (s, 1H), 2.51 (s, 1H), 1.87 - 1.70 (m, 3H), 1.64 - 1.50 (m, 1H).

Step 4: Preparation of intermediate **40e**

Intermediate **22** (50 mg), Dess-Martin periodinane (141 mg, 0.333 mmol), dichloromethane (5 mL), and DMF (1 mL) were added to a reaction flask in sequence, and the mixture was reacted at room temperature. After the reaction was completed, as confirmed by TLC, the reaction solution was extracted three times with 50 mL of water and 20 mL of ethyl acetate. The organic phases were combined, washed with 50 mL of a saturated sodium chloride solution, dried over anhydrous sodium sulfate, and filtered, and the filtrate was concentrated by evaporation at reduced pressure to remove the solvent, thus giving the target intermediate **40e** (50 mg).

MS(ESI, $[M+H]^+$) m/z: 298.31.

Step 5: Preparation of compound **40**

Intermediate **40e** (50 mg), 1,2-dichloroethane (6 mL), isopropanol (2 mL), intermediate **40d** (83 mg), acetic acid (5.00 mg, 0.083 mmol), and sodium cyanoborohydride (20.93 mg, 0.333 mmol) were added to a reaction flask in sequence, and the mixture was stirred and reacted at room temperature. After the reaction was completed, as confirmed by TLC, 2 mL of a saturated sodium bicarbonate solution was added to the reaction solution to neutralize acetic acid,

and then 50 mL of dichloromethane and 100 mL were added for extraction. The organic phase was separated, and the aqueous phase was extracted twice with 20 mL of dichloromethane. The organic phases were combined, washed with saturated brine, dried over anhydrous sodium sulfate, and filtered, and the filtrate was purified by silica gel column chromatography to give compound **40** (26 mg).

MS(ESI, $[M+H]^+$) m/z: 733.42.

$^1$H NMR (500 MHz, DMSO-d6) δ 11.23 (s, 1H), 11.08 (d, J = 1.8 Hz, 1H), 7.77 (d, J = 2.8 Hz, 1H), 7.67 (s, 1H), 7.61 (d, J = 8.1 Hz, 1H), 7.58 (s, 1H), 7.35 (d, J = 2.9 Hz, 1H), 7.27 (d, J = 8.1 Hz, 1H), 7.17 - 7.10 (m, 1H), 6.97 (d, J = 8.3 Hz, 1H), 4.57 (ddd, J = 11.9, 4.9, 1.7 Hz, 1H), 4.38 (d, J = 12.5 Hz, 1H), 4.32 - 4.24 (m, 1H), 3.66 - 3.53 (m, 3H), 3.35 (s, 1H), 3.31 - 3.13 (m, 5H), 3.10 - 2.83 (m, 6H), 2.76 (dt, J = 17.2, 5.8 Hz, 3H), 2.67 (s, 5H), 2.63 (t, J = 3.6 Hz, 1H), 2.59 (s, 1H), 2.46 (d, J = 5.8 Hz, 1H), 2.18 (dt, J = 13.1, 4.5 Hz, 1H), 1.78 (dtd, J = 22.0, 11.4, 6.5 Hz, 3H), 1.57 (q, J = 12.3, 11.5 Hz, 1H).

**Example 41:** Synthesis of Compound **41**

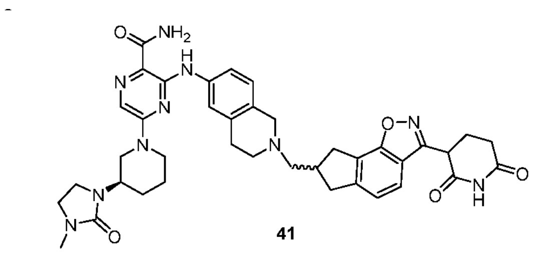

41

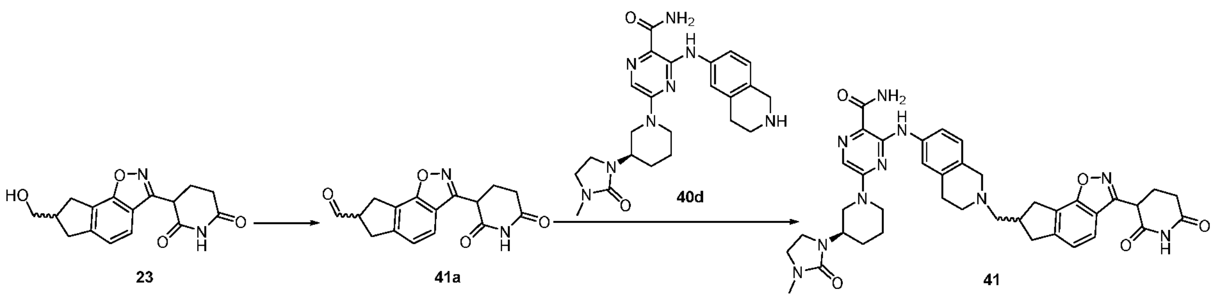

23 41a 40d 41

Step 1: Preparation of intermediate **41a**

Intermediate **23** (50 mg), Dess-Martin periodinane (141 mg, 0.333 mmol), dichloromethane (5 mL), and DMF (1 mL) were added to a reaction flask in sequence, and the mixture was reacted at room temperature. After the reaction was completed, as confirmed by TLC, the reaction solution was extracted three times with 50 mL of water and 20 mL of ethyl acetate. The organic phases were combined, washed with a saturated sodium chloride solution, dried over anhydrous sodium sulfate, and filtered, and the filtrate was concentrated by evaporation at reduced pressure to remove the solvent, thus giving the target intermediate **41a** (50 mg).

MS(ESI, $[M+H]^+$) m/z: 298.29.

Step 2: Preparation of compound **41**

Intermediate **41a** (50 mg), 1,2-dichloroethane (6 mL), isopropanol (2 mL), intermediate **40d** (83 mg), acetic acid (5.00 mg, 0.083 mmol), and sodium cyanoborohydride (20.93 mg, 0.333 mmol) were added to a reaction flask in sequence, and the mixture was stirred and reacted at room temperature. After the reaction was completed, as confirmed by TLC, 2 mL of a saturated sodium bicarbonate solution was added to the reaction solution to neutralize acetic acid, and then 50 mL of dichloromethane and 100 mL of water were added for extraction. The organic phase was separated, and the aqueous phase was extracted twice with 20 mL of dichloromethane. The organic phases were combined, washed with saturated brine, dried over anhydrous sodium sulfate, and filtered, and the filtrate was purified by silica gel column chromatography to give compound **41** (26 mg).

MS(ESI, $[M+H]^+$) m/z: 733.40.

$^1$H NMR (500 MHz, DMSO-d6) δ 11.23 (s, 1H), 11.09 (s, 1H), 7.79 - 7.74 (m, 1H), 7.67 (s, 1H), 7.63 - 7.56 (m,

2H), 7.38 - 7.33 (m, 1H), 7.27 (d, J = 8.1 Hz, 1H), 7.14 (d, J = 8.2 Hz, 1H), 6.97 (d, J = 8.3 Hz, 1H), 4.57 (dd, J = 12.0, 4.9 Hz, 1H), 4.38 (d, J = 12.5 Hz, 1H), 4.32 - 4.25 (m, 1H), 3.67 - 3.52 (m, 3H), 3.30 - 3.14 (m, 5H), 3.12 - 2.84 (m, 6H), 2.83 - 2.68 (m, 5H), 2.67 (s, 3H), 2.64 - 2.61 (m, 1H), 2.59 (s, 1H), 2.47 (d, J = 14.4 Hz, 1H), 2.18 (dt, J = 13.4, 4.6 Hz, 1H), 1.86 - 1.72 (m, 3H), 1.57 (d, J = 12.7 Hz, 1H).

**Example 42:** Synthesis of Compound **42**

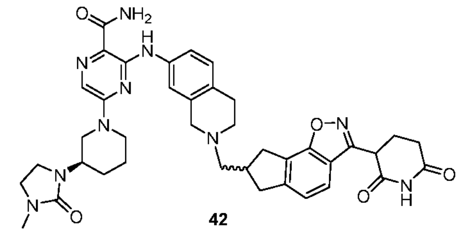

**42**

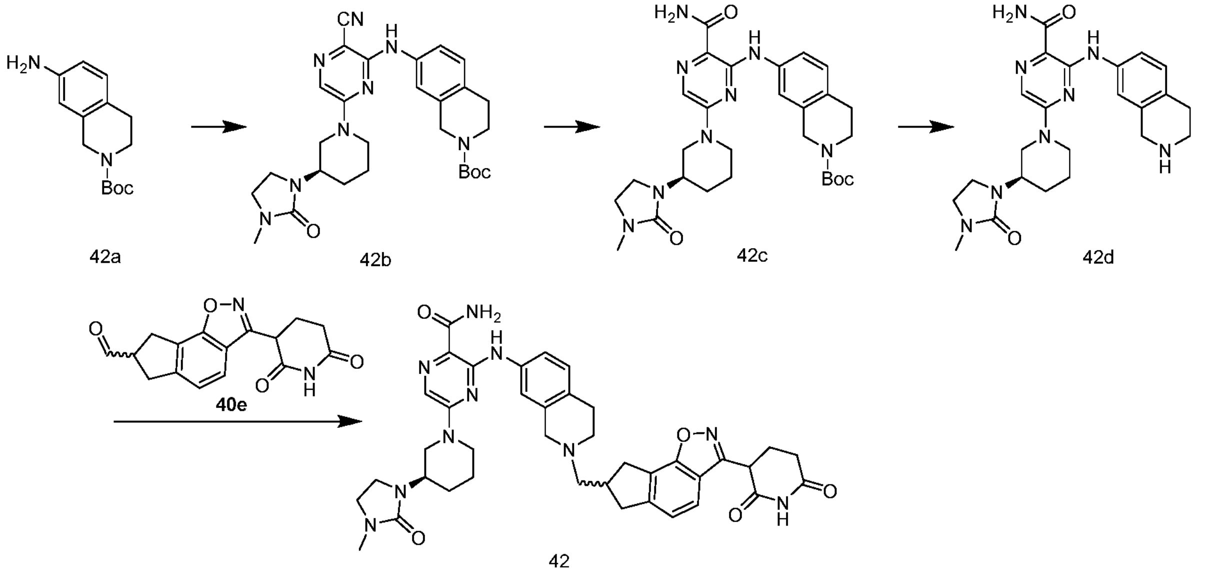

42a 42b 42c 42d

**40e**

42

Step 1: Preparation of intermediate **42b**

**42a** (1.16 g), **1f** (1.50 g), BINAP (0.29 g), cesium carbonate (4.57 g), palladium acetate (0.10 g), and 1,4-dioxane (50 mL) were added to a reaction flask in sequence, and the mixture was purged three times with nitrogen, heated to 100 °C, and reacted. After the reaction was completed, as confirmed by TLC, the reaction solution was cooled to room temperature, and 80 L of ethyl acetate and 140 mL of water were added to the residue. The organic phase was separated, washed with a saturated sodium chloride solution, dried over anhydrous sodium sulfate, filtered, and concentrated. The concentrate was separated and purified by silica gel column chromatography to give intermediate **42b** (2.2 g).

MS(ESI, $[M+H]^+$) *m/z*: 533.5.

Step 2: Preparation of intermediate **42c**

Intermediate **42b** (2.2 g), cesium carbonate (1.34 g), water (10 mL), DMSO (30 mL), and methanol (10 mL) were added to a reaction flask in sequence. The reaction solution was cooled to 0 °C, and 30 wt% hydrogen peroxide (1.40 g) was added dropwise. After the dropwise addition was completed, the mixture was allowed to react at room temperature. After the reaction was completed, as confirmed by TLC, 100 mL of water was added to the reaction solution, and then a saturated aqueous sodium sulfite solution was added to neutralize hydrogen peroxide. The mixture was stirred for 10 min and filtered, and the filter cake was collected and dried to give intermediate **42c** (1.9 g).

MS(ESI, $[M+H]^+$) *m/z*: 551.4.

Step 3: Preparation of intermediate **42d**

Intermediate **42c** (0.30 g), dichloromethane (20 mL), and trifluoroacetic acid (1 mL) were added to a reaction flask in sequence, and the mixture was reacted at room temperature. After the reaction was completed, as confirmed by TLC, 50 mL of water was added to the reaction solution, the pH was adjusted to about 8 with a saturated aqueous sodium bicarbonate solution, and the product was extracted with DCM: MEOH = 10:1. The organic phase was separated, dried over anhydrous sodium sulfate, filtered, and concentrated to give intermediate **42d** (0.20 g).
MS(ESI, $[M+H]^+$) *m/z*: 451.2.

Step 4: Preparation of compound **42**

Intermediate **42d** (76 mg), **40e** (50 mg), dichloroethane/isopropanol (5:1, 20 mL), glacial acetic acid (10 mg), and sodium cyanoborohydride (21 mg) were added to a reaction flask in sequence, and the mixture was reacted at room temperature. After the reaction was completed, as confirmed by TLC, dichloromethane and water were added to the reaction solution. The organic phase was separated, washed with a saturated sodium chloride solution, dried over anhydrous sodium sulfate, filtered, and concentrated. The concentrate was separated and purified by silica gel column chromatography to give compound **42** (20 mg).
MS(ESI, $[M+H]^+$) *m/z*: 733.5.
1H NMR (500 MHz, DMSO-d6) δ 11.22 (s, 1H), 11.08 (d, *J* = 1.8 Hz, 1H), 7.76 (d, *J* = 2.8 Hz, 1H), 7.66 (s, 1H), 7.62 (d, *J* = 8.0 Hz, 1H), 7.44 (d, *J* = 2.2 Hz, 1H), 7.34 (d, *J* = 2.8 Hz, 1H), 7.27 (d, *J* = 8.1 Hz, 1H), 7.21 (d, *J* = 8.6 Hz, 1H), 7.03 (d, *J*= 8.3 Hz, 1H), 4.57 (ddd, *J* = 11.9, 5.0, 2.5 Hz, 1H), 4.30 (dd, *J* = 31.6, 12.6 Hz, 2H), 3.63 - 3.48 (m, 3H), 3.31 - 3.22 (m, 3H), 3.19 (dd, *J* = 9.0, 6.6 Hz, 2H), 3.12 (d, *J* = 8.4 Hz, 1H), 3.06 (t, *J* = 11.8 Hz, 1H), 2.99 (d, *J* = 6.9 Hz, 1H), 2.97 - 2.91 (m, 2H), 2.88 (dd, *J* = 16.5, 4.7 Hz, 1H), 2.81 - 2.72 (m, 4H), 2.68 (d, *J* = 5.1 Hz, 1H), 2.65 - 2.61 (m, 1H), 2.59 (d, *J* = 1.2 Hz, 3H), 2.18 (dq, *J* = 8.7, 4.2 Hz, 1H), 1.83 - 1.73 (m, 3H), 1.61 - 1.52 (m, 1H).

**Example 43:** Synthesis of Compound **43**

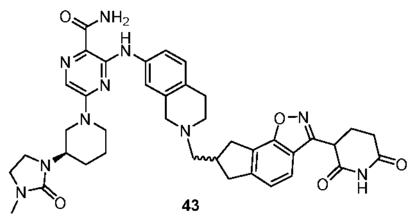

**43**

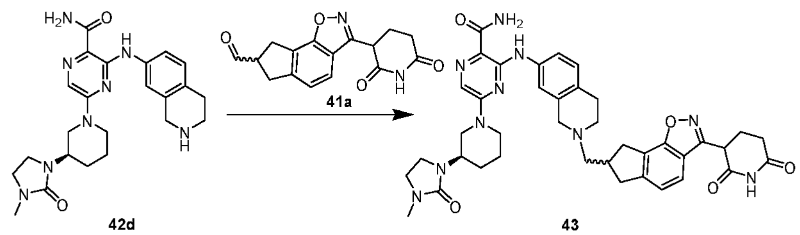


Step 1: Preparation of compound **43**

Intermediate **42d** (76 mg), **41a** (50 mg), dichloroethane/isopropanol (5:1, 20 mL), glacial acetic acid (10 mg), and sodium cyanoborohydride (21 mg) were added to a reaction flask in sequence, and the mixture was reacted at room temperature. After the reaction was completed, as confirmed by TLC, dichloromethane and water were added to the reaction solution. The organic phase was separated, washed with a saturated sodium chloride solution, dried over anhydrous sodium sulfate, filtered, and concentrated. The concentrate was separated and purified by silica gel column chromatography to give compound **43** (24 mg).
MS(ESI, $[M+H]^+$) *m/z*: 733.5.
1H NMR (500 MHz, DMSO-*d6*) δ 11.22 (s, 1H), 11.08 (s, 1H), 7.76 (d, *J* = 2.8 Hz, 1H), 7.66 (s, 1H), 7.62 (d, *J* = 8.0 Hz, 1H), 7.44 (d, *J*= 2.2 Hz, 1H), 7.35 (d, *J*= 2.8 Hz, 1H), 7.27 (d, *J*= 8.1 Hz, 1H), 7.21 (d, *J*= 8.3 Hz, 1H), 7.03 (d, *J* = 8.3 Hz, 1H), 4.57 (ddd, *J* = 11.9, 5.0, 2.6 Hz, 1H), 4.30 (dd, *J* = 31.1, 12.8 Hz, 2H), 3.64 - 3.55 (m, 2H), 3.51 (d, *J* =

15.2 Hz, 1H), 3.31 - 3.25 (m, 2H), 3.25 - 3.21 (m, 1H), 3.21 - 3.14 (m, 2H), 3.13 - 3.02 (m, 2H), 2.99 (d, *J* = 6.5 Hz, 1H), 2.96 (s, 1H), 2.94 - 2.85 (m, 2H), 2.83 - 2.71 (m, 4H), 2.67 (t, *J* = 5.7 Hz, 1H), 2.62 (t, *J* = 3.8 Hz, 1H), 2.59 (d, *J* = 2.8 Hz, 3H), 2.18 (dq, *J* = 8.6, 4.5 Hz, 1H), 1.83 - 1.71 (m, 3H), 1.56 (d, *J* = 14.2 Hz, 2H).

**Example 44:** Synthesis of Compound **44**

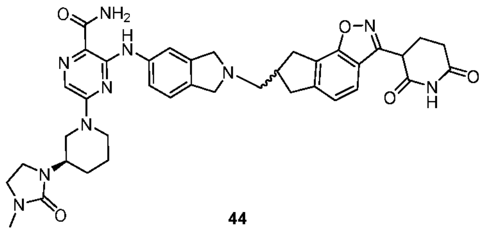

44

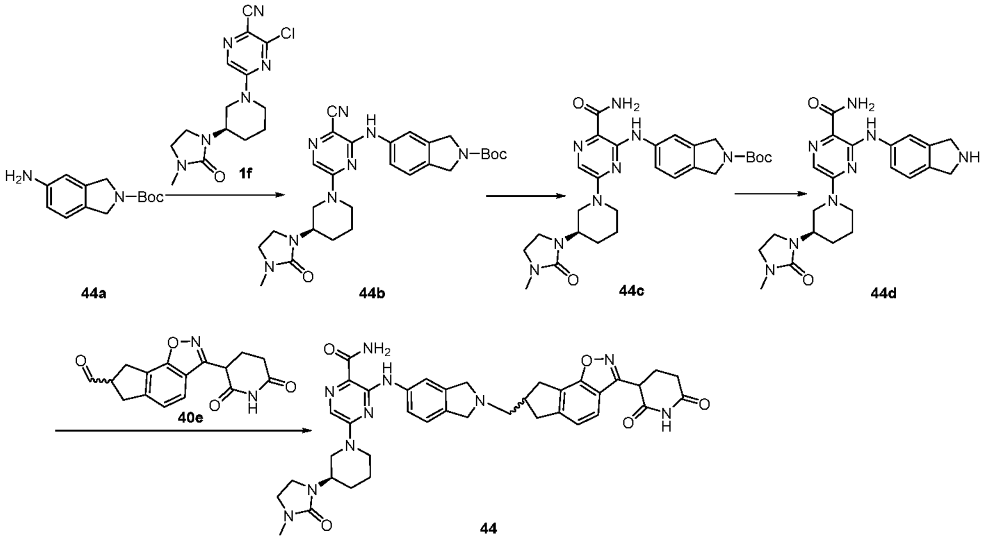

1f

44a 44b 44c 44d

40e

44

Step 1: Preparation of intermediate **44b**

Intermediate **1f** (1.5 g), intermediate **44a** (1.315 g), BINAP (0.291 g, 0.468 mmol), cesium carbonate (4.57 g), palladium acetate (0.105 g, 0.468 mmol), and 1,4-dioxane (20 mL) were added to a reaction flask in sequence, and the mixture was heated to 100 °C and reacted under $N_2$ atmosphere. After the reaction was completed, as confirmed by TLC, the reaction solution was diluted with 20 mL of dichloromethane and purified by silica gel column chromatography to give the target intermediate **44b** (1.99 g).

MS(ESI, $[M+H]^+$) m/z: 519.34.

Step 2: Preparation of intermediate **44c**

Intermediate **44b** (1.99 g), DMSO (8 mL), methanol (20 mL), cesium carbonate (1.250 g), and water (8 mL) were added to a reaction flask in sequence, and 30 wt% hydrogen peroxide (1.305 g, 1.305 mL, 11.51 mmol) was added under an ice bath. The mixture was allowed to return to room temperature and react. After the reaction was completed, as confirmed by TLC, the reaction solution was poured into 200 mL of an icy saturated sodium sulfite solution to quench the reaction, and 200 mL of ethyl acetate was added for extraction. The organic phase was separated, and the aqueous phase was extracted twice with 50 mL of ethyl acetate. The organic phases were combined, washed with a saturated sodium chloride solution, dried over anhydrous sodium sulfate, and filtered, and the filtrate was concentrated by evaporation at reduced pressure to remove the solvent, thus giving the target intermediate **44c** (1.8 g).

MS(ESI, $[M+H]^+$) m/z: 537.40.

$^1$H NMR (500 MHz, DMSO-d6) $\delta$ 11.35 (d, J = 3.5 Hz, 1H), 7.79 (d, J = 2.6 Hz, 1H), 7.69 (d, J = 2.5 Hz, 1H), 7.36 (dd, J = 12.2, 2.6 Hz, 2H), 7.23 (q, J = 8.3 Hz, 2H), 4.53 (q, J = 11.5, 10.6 Hz, 4H), 4.39 (d, J = 14.0 Hz, 1H), 4.25

(t, J = 13.3 Hz, 1H), 3.67 - 3.57 (m, 1H), 3.34 (d, J = 5.9 Hz, 1H), 3.28 (h, J = 5.7 Hz, 2H), 3.04 (td, J = 11.7, 6.7 Hz, 1H), 2.96 (td, J = 12.9, 2.5 Hz, 1H), 2.70 (d, J = 10.8 Hz, 3H), 1.78 (h, J = 12.7, 11.1 Hz, 3H), 1.57 (dt, J = 15.8, 4.9 Hz, 1H), 1.46 (d, J = 4.7 Hz, 9H).

Step 3: Preparation of intermediate **44d**

Intermediate **44c** (1.8 g) and TFA (7.65 g, 5.17 mL, 67.1 mmol) were added to a reaction flask in sequence, and the mixture was reacted at room temperature. After the reaction was completed, as confirmed by TLC, the reaction solution was concentrated by evaporation at reduced pressure to remove the solvent. The residue was completely dissolved in 10 mL of dichloromethane, then the solution was added dropwise to 100 mL of an icy 1 M sodium hydroxide solution, and 100 mL of dichloromethane was added for extraction. The organic phase was separated, and the aqueous phase was extracted twice with 50 mL of dichloromethane. The organic phases were combined, washed with saturated brine, dried over anhydrous sodium sulfate, and filtered, and the filtrate was concentrated by evaporation at reduced pressure to remove the solvent, thus giving the target intermediate **44d** (1.36 g).

MS(ESI, $[M+H]^+$) m/z: 437.30.

$^1H$ NMR (500 MHz, DMSO-d6) δ 11.28 (s, 1H), 7.77 (d, J = 2.8 Hz, 1H), 7.67 (s, 1H), 7.63 (d, J = 2.0 Hz, 1H), 7.35 (d, J = 3.1 Hz, 1H), 7.23 (dd, J = 8.0, 2.0 Hz, 1H), 7.15 (d, J = 8.0 Hz, 1H), 4.36 (d, J = 12.8 Hz, 1H), 4.27 (d, J = 13.4 Hz, 1H), 4.02 (s, 4H), 3.60 (dtd, J = 14.9, 10.6, 5.2 Hz, 1H), 3.30 - 3.20 (m, 3H), 3.04 (t, J = 11.8 Hz, 1H), 2.94 (td, J = 12.9, 2.6 Hz, 1H), 2.69 (s, 3H), 1.83 - 1.73 (m, 3H), 1.60 - 1.52 (m, 1H).

Step 4: Preparation of compound **44**

Intermediate **40e** (50 mg), 1,2-dichloroethane (6 mL), isopropanol (2 mL), intermediate **44d** (72.7 mg), acetic acid (5.00 mg, 0.083 mmol), and sodium cyanoborohydride (20.93 mg, 0.333 mmol) were added to a reaction flask in sequence, and the mixture was stirred and reacted at room temperature. After the reaction was completed, as confirmed by TLC, 2 mL of a saturated sodium bicarbonate solution was added to the reaction solution, and then 50 mL of dichloromethane and 100 mL of water were added for extraction. The organic phase was separated, and the aqueous phase was extracted twice with 20 mL of dichloromethane. The organic phases were combined, washed with saturated brine, dried over anhydrous sodium sulfate, and filtered, and the filtrate was purified by silica gel column chromatography to give compound **44** (40 mg).

MS(ESI, $[M+H]^+$) m/z: 719.50.

$^1H$ NMR (500 MHz, DMSO-d6) δ 11.30 (d, J = 3.0 Hz, 1H), 11.09 (s, 1H), 7.77 (d, J = 2.9 Hz, 1H), 7.67 (d, J = 4.0 Hz, 2H), 7.62 (d, J = 8.0 Hz, 1H), 7.36 (d, J = 2.9 Hz, 1H), 7.28 (d, J = 8.1 Hz, 1H), 7.22 (d, J = 8.3 Hz, 1H), 7.15 (d, J = 8.1 Hz, 1H), 4.57 (dd, J = 11.9, 4.9 Hz, 1H), 4.37 (d, J = 12.7 Hz, 1H), 4.26 (d, J = 13.3 Hz, 1H), 3.87 (s, 4H), 3.61 (tt, J = 10.3, 4.7 Hz, 1H), 3.29 (d, J = 9.6 Hz, 2H), 3.22 (t, J = 7.4 Hz, 3H), 3.05 - 2.86 (m, 5H), 2.77 (ddt, J = 18.5, 12.1, 5.9 Hz, 3H), 2.64 - 2.58 (m, 4H), 2.46 (d, J = 4.3 Hz, 1H), 2.23 - 2.15 (m, 1H), 1.86 - 1.69 (m, 3H), 1.61 -1.51 (m, 1H), 1.24 (d, J = 6.3 Hz, 3H).

**Example 45:** Synthesis of Compound **45**

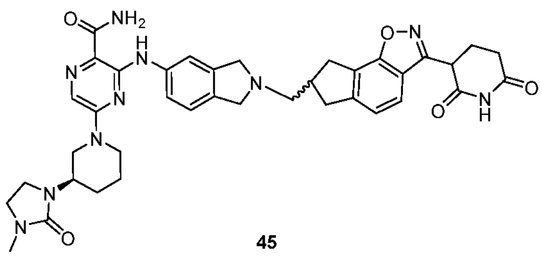

45

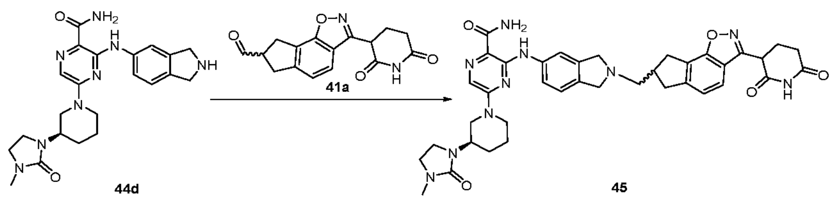

41a
44d
45

Intermediate **41a** (50 mg), 1,2-dichloroethane (6 mL), isopropanol (2 mL), intermediate **44d** (72.7 mg), acetic acid (5.00 mg, 0.083 mmol), and sodium cyanoborohydride (20.93 mg, 0.333 mmol) were added to a reaction flask in sequence, and the mixture was stirred and reacted at room temperature. After the reaction was completed, as confirmed by TLC, 2 mL of a saturated sodium bicarbonate solution was added to the reaction solution, and then 50 mL of dichloromethane and 100 mL of water were added for extraction. The organic phase was separated, and the aqueous phase was extracted twice with 20 mL of dichloromethane. The organic phases were combined, washed with saturated brine, dried over anhydrous sodium sulfate, and filtered, and the filtrate was purified by silica gel column chromatography to give compound **45** (60 mg).

MS(ESI, $[M+H]^+$) m/z: 719.40.

$^1$H NMR (500 MHz, DMSO-d6) δ 11.30 (d, J = 2.9 Hz, 1H), 11.09 (s, 1H), 7.77 (d, J = 2.8 Hz, 1H), 7.67 (d, J = 4.0 Hz, 2H), 7.62 (d, J = 8.0 Hz, 1H), 7.36 (d, J = 2.9 Hz, 1H), 7.28 (d, J = 8.1 Hz, 1H), 7.22 (d, J = 8.3 Hz, 1H), 7.15 (d, J = 8.1 Hz, 1H), 4.57 (dd, J = 11.9, 5.0 Hz, 1H), 4.37 (d, J = 12.6 Hz, 1H), 4.26 (d, J = 13.3 Hz, 1H), 3.87 (s, 4H), 3.61 (dq, J = 11.0, 5.5, 5.0 Hz, 1H), 3.31 - 3.25 (m, 2H), 3.25 - 3.16 (m, 3H), 3.12 - 2.82 (m, 6H), 2.81 - 2.70 (m, 3H), 2.65 - 2.58 (m, 4H), 2.49 - 2.44 (m, 1H), 2.19 (dp, J = 12.9, 4.5 Hz, 1H), 1.85 - 1.71 (m, 3H), 1.56 (qd, J = 13.0, 12.4, 6.2 Hz, 1H).

**Example 46:** Synthesis of Compound **46**

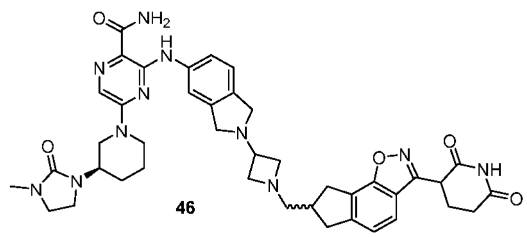

46

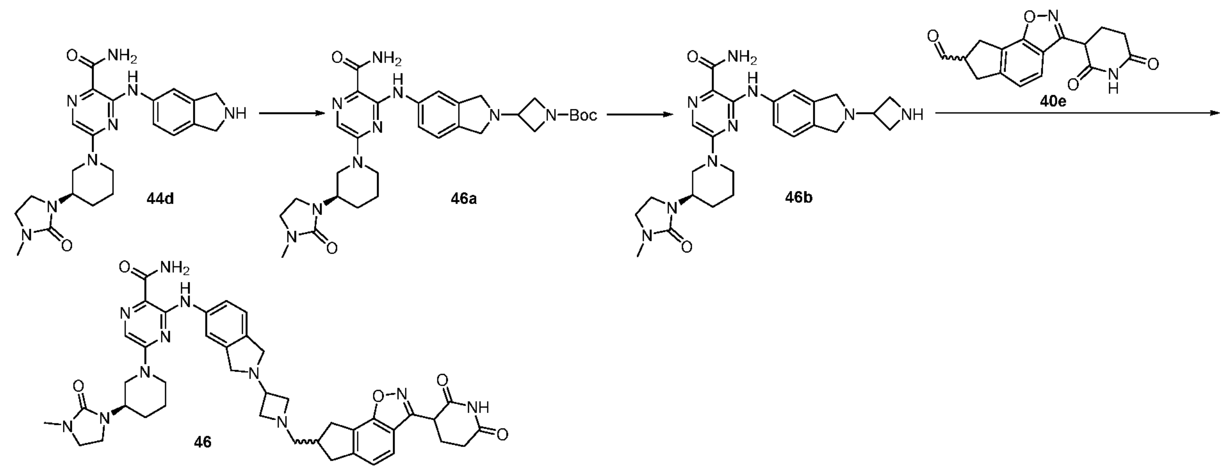

44d
46a
46b
40e
46

Step 1: Preparation of intermediate **46a**

Intermediate **44d** (700 mg), 1-Boc-3-azetidinone (549 mg), 1,2-dichloroethane (3 mL), isopropanol (1 mL), acetic acid (48.1 mg, 0.802 mmol), and sodium cyanoborohydride (202 mg, 3.21 mmol) were added to a reaction flask in sequence, and the mixture was stirred and reacted at room temperature. After the reaction was completed, as confirmed

by TLC, 20 mL of a saturated sodium bicarbonate solution was added to the reaction solution, and then 100 mL of dichloromethane and 100 mL of water were added for extraction. The organic phase was separated, and the aqueous phase was extracted twice with 20 mL of dichloromethane. The organic phases were combined, washed with saturated brine, dried over anhydrous sodium sulfate, and filtered, and the filtrate was purified by silica gel column chromatography to give the target intermediate **46a** (601 mg).

MS(ESI, $[M+H]^+$) m/z: 592.51.

Step 2: Preparation of intermediate **46b**

Intermediate **46a** (601 mg) and TFA (2316 mg, 1565 μL, 20.31 mmol) were added to a reaction flask in sequence, and the mixture was reacted at room temperature for 1 h. After the reaction was completed, the reaction solution was concentrated by evaporation at reduced pressure to remove the solvent. The residue was completely dissolved in 10 mL of dichloromethane, then the solution was added dropwise to 100 mL of an icy 1 M sodium hydroxide solution, and 100 mL of dichloromethane was added for extraction. The organic phase was separated, and the aqueous phase was extracted twice with 50 mL of dichloromethane. The organic phases were combined, washed with saturated brine, dried over anhydrous sodium sulfate, and filtered, and the filtrate was concentrated by evaporation at reduced pressure to remove the solvent, thus giving the target intermediate **46b** (434 mg).

MS(ESI, $[M+H]^+$) m/z: 492.30.

$^1$H NMR (500 MHz, DMSO-d6) δ 11.30 (d, J = 6.8 Hz, 1H), 7.77 (dd, J = 5.3, 2.8 Hz, 1H), 7.70 - 7.61 (m, 2H), 7.35 (t, J = 2.9 Hz, 1H), 7.29 - 7.18 (m, 1H), 7.14 (dd, J = 11.9, 8.1 Hz, 1H), 4.35 (d, J = 12.6 Hz, 1H), 4.27 (d, J = 11.8 Hz, 1H), 3.87 - 3.69 (m, 8H), 3.63 - 3.58 (m, 1H), 3.36 - 3.29 (m, 3H), 3.28 - 3.23 (m, 2H), 3.03 (qd, J = 10.2, 8.9, 5.7 Hz, 2H), 2.99 - 2.92 (m, 1H), 2.68 (s, 3H), 1.84 - 1.74 (m, 3H), 1.60 - 1.52 (m, 1H).

Step 3: Preparation of compound **46**

Intermediate **40e** (50 mg), 1,2-dichloroethane (6 mL), isopropanol (2 mL), intermediate **46b** (83 mg), acetic acid (5.00 mg, 0.083 mmol), and sodium cyanoborohydride (20.93 mg, 0.333 mmol) were added to a reaction flask in sequence, and the mixture was stirred and reacted at room temperature. After the reaction was completed, as confirmed by TLC, 2 mL of a saturated sodium bicarbonate solution was added to the reaction solution to neutralize acetic acid, and then 50 mL of dichloromethane and 100 mL of water were added for extraction. The organic phase was separated, and the aqueous phase was extracted twice with 20 mL of dichloromethane. The organic phases were combined, washed with saturated brine, dried over anhydrous sodium sulfate, and filtered, and the filtrate was purified by silica gel column chromatography to give compound **46** (20 mg).

MS(ESI, $[M+H]^+$) m/z: 774.47.

$^1$H NMR (500 MHz, DMSO-d6) δ 11.31 (s, 1H), 11.08 (s, 1H), 7.80 - 7.74 (m, 1H), 7.66 (d, J = 8.8 Hz, 2H), 7.61 (d, J = 8.0 Hz, 1H), 7.39 - 7.33 (m, 1H), 7.25 (d, J = 8.1 Hz, 1H), 7.21 (d, J = 8.2 Hz, 1H), 7.14 (d, J = 8.1 Hz, 1H), 4.56 (dd, J = 11.8, 5.0 Hz, 1H), 4.37 (d, J = 12.4 Hz, 1H), 4.26 (d, J = 13.4 Hz, 1H), 3.83 - 3.73 (m, 4H), 3.61 (dt, J = 10.9, 5.7 Hz, 1H), 3.49 (d, J = 15.5 Hz, 3H), 3.28 - 3.11 (m, 5H), 2.99 (dt, J = 42.1, 11.7 Hz, 4H), 2.90 - 2.72 (m, 4H), 2.69 (s, 3H), 2.62 (q, J = 4.2 Hz, 2H), 2.59 (d, J = 4.0 Hz, 1H), 2.49 - 2.43 (m, 1H), 2.18 (dq, J = 13.7, 4.8 Hz, 1H), 1.84 - 1.74 (m, 3H), 1.56 (d, J = 12.8 Hz, 1H).

**Example 47:** Synthesis of Compound **47**

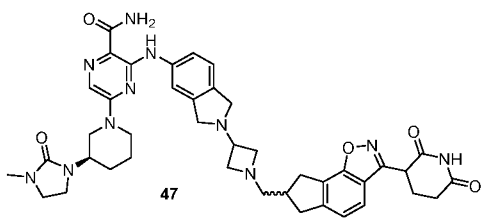

47

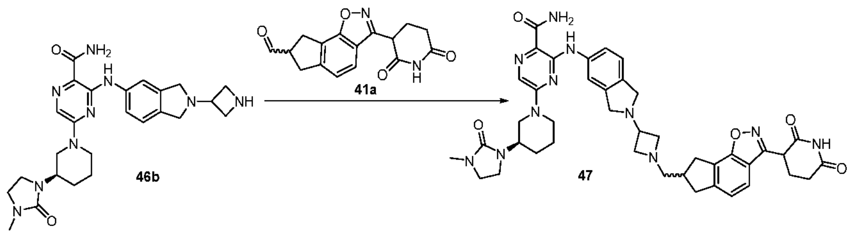


46b 47

Intermediate **41a** (50 mg), 1,2-dichloroethane (6 mL), isopropanol (2 mL), intermediate **46b** (83 mg), acetic acid (5.00 mg, 0.083 mmol), and sodium cyanoborohydride (20.93 mg, 0.333 mmol) were added to a reaction flask in sequence, and the mixture was stirred and reacted at room temperature. After the reaction was completed, as confirmed by TLC, 2 mL of a saturated sodium bicarbonate solution was added to the reaction solution, and then 50 mL of dichloromethane and 100 mL of water were added for extraction. The organic phase was separated, and the aqueous phase was extracted twice with 20 mL of dichloromethane. The organic phases were combined, washed with saturated brine, dried over anhydrous sodium sulfate, and filtered, and the filtrate was purified by silica gel column chromatography to give compound **47** (30 mg).

MS(ESI, $[M+H]^+$) m/z: 774.49.

$^1$H NMR (500 MHz, DMSO-d6) δ 11.31 (s, 1H), 11.08 (s, 1H), 7.77 (d, J = 2.8 Hz, 1H), 7.66 (d, J = 8.4 Hz, 2H), 7.61 (d, J = 8.0 Hz, 1H), 7.36 (d, J = 2.9 Hz, 1H), 7.23 (dd, J = 19.6, 8.1 Hz, 2H), 7.14 (d, J = 8.2 Hz, 1H), 4.56 (dd, J = 11.8, 5.0 Hz, 1H), 4.37 (d, J = 12.6 Hz, 1H), 4.26 (d, J = 13.4 Hz, 1H), 3.78 (d, J = 11.1 Hz, 4H), 3.60 (dq, J = 10.1, 5.4, 4.8 Hz, 1H), 3.47 (s, 3H), 3.35 (s, 1H), 3.30 - 3.12 (m, 5H), 2.99 (dt, J = 41.9, 11.7 Hz, 4H), 2.91 - 2.71 (m, 4H), 2.69 (s, 3H), 2.61 (q, J = 4.2 Hz, 2H), 2.58 (t, J = 4.1 Hz, 1H), 2.49 - 2.42 (m, 1H), 2.18 (dq, J = 14.0, 5.0 Hz, 1H), 1.84 - 1.73 (m, 3H), 1.57 (t, J = 12.1 Hz, 1H).

**Example 48:** Synthesis of Compound **48**

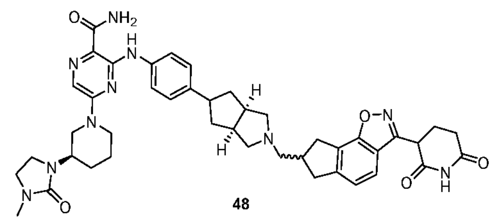

48

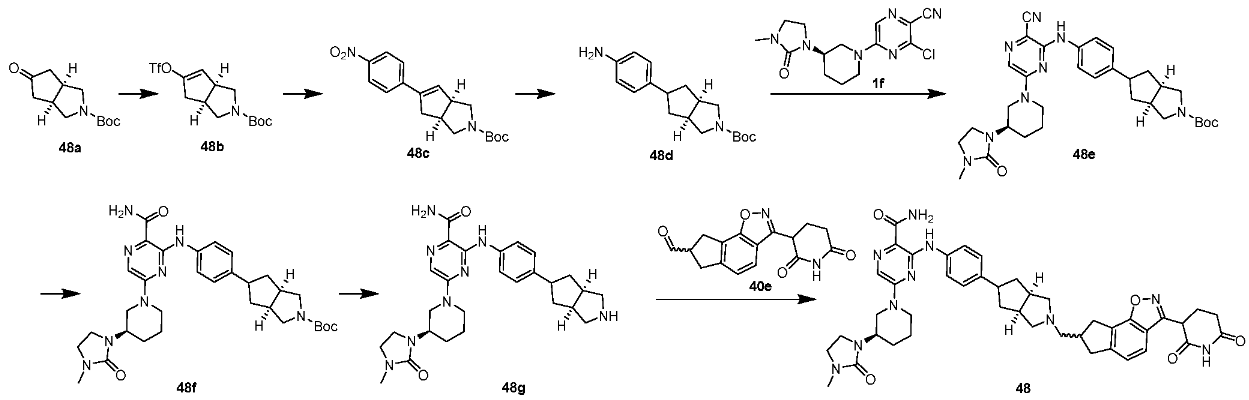


48a 48b 48c 48d 48e 48f 48g 48

Step 1: Preparation of intermediate **48b**

Lithium bis(trimethylsilyl)amide (26.6 mL) was added dropwise to a stirred solution of **48a** (1.16 g) in THF (30 mL) at -78 °C, and the mixture was maintained at this temperature and reacted for 1 h. *N*-phenylbis(trifluoromethanesulfonyl)imide (1.50 g) was dissolved in THF (30 mL), and the solution was added dropwise to the reaction solution. After the dropwise addition was completed, the mixture was allowed to react at room temperature. After the reaction was completed, as confirmed by TLC, the reaction solution was poured into 100 mL of water and extracted twice with petroleum

ether (100 mL each time). The organic phases were combined, washed with saturated sodium chloride, dried over anhydrous sodium sulfate, filtered, and concentrated to give intermediate **48b** (6.2 g).
$^1$H NMR (500 MHz, DMSO-$d_6$) δ 5.76 (d, *J* = 2.0 Hz, 1H), 3.51 (dd, *J* = 11.3, 8.5 Hz, 1H), 3.29 (q, *J* = 7.4 Hz, 3H), 3.00 (dd, *J*= 11.3, 6.1 Hz, 1H), 2.93 - 2.77 (m, 2H), 2.35 - 2.28 (m, 1H), 1.33 (s, 9H).

Step 2: Preparation of intermediate **48c**

Intermediate **48b** (5.0 g), 4-nitrophenylboronic acid (2.33 g), potassium carbonate (3.87 g), $PdCl_2$(dppf) (0.51 g), water (10 mL), and 1,4-dioxane (50 mL) were added to a reaction flask in sequence, and the mixture was purged three times with nitrogen, heated to 100 °C and reacted. After the reaction was completed, as confirmed by TLC, the reaction solution was cooled to room temperature, and 80 L of ethyl acetate and 140 mL of water were added to the residue. The organic phase was separated, washed with a saturated sodium chloride solution, dried over anhydrous sodium sulfate, filtered, and concentrated. The concentrate was separated and purified by silica gel column chromatography to give intermediate **48c** (4.2 g).
MS(ESI, $[M+H]^+$) *m*/*z*: 330.21.

Step 3: Preparation of intermediate **48d**

Intermediate **48c** (0.30 g), 10% palladium on carbon (0.84 g), methanol (60 mL), and dichloromethane (30 mL) were added to a reaction flask in sequence, and the mixture was purged three times with hydrogen and reacted at room temperature. After the reaction was completed, as confirmed by TLC, palladium on carbon was removed by filtration under vacuum, and the residue was concentrated to give intermediate **48d** (3.72 g).
MS(ESI, $[M+H]^+$) *m*/*z*: 302.21.

Step 4: Preparation of intermediate **48e**

Intermediate **48d** (1.88 g), **1f** (2.0 g), BINAP (0.39 g), cesium carbonate (6.09 g), palladium acetate (0.14 g), and dioxane (50 mL) were added to a reaction flask in sequence, and the mixture was purged three times with nitrogen, heated to 100 °C, and reacted. After the reaction was completed, as confirmed by TLC, the reaction solution was cooled to room temperature, and 80 L of ethyl acetate and 140 mL of water were added to the residue. The organic phase was separated, washed with a saturated sodium chloride solution, dried over anhydrous sodium sulfate, filtered, and concentrated. The concentrate was separated and purified by silica gel column chromatography to give intermediate **48e** (3.0 g).
MS(ESI, $[M+H]^+$) *m*/*z*: 587.3.

Step 5: Preparation of intermediate **48f**

Intermediate **48e** (3.0 g), cesium carbonate (1.43 g), water (10 mL), DMSO (35 mL), and methanol (20 mL) were added to a reaction flask in sequence. The reaction solution was cooled to 0 °C, and 30 wt% hydrogen peroxide (1.49 g) was added dropwise. After the dropwise addition was completed, the mixture was allowed to react at room temperature. After the reaction was completed, as confirmed by TLC, 100 mL of water was added to the reaction solution, and then a saturated aqueous sodium sulfite solution was added to neutralize hydrogen peroxide. The mixture was stirred for 10 min and filtered, and the filter cake was collected and dried to give intermediate **48f** (2.0 g).
MS(ESI, $[M+H]^+$) *m*/*z*: 605.4.

Step 6: Preparation of intermediate **48g**

Intermediate **48f** (0.45 g), dichloromethane (20 mL), and trifluoroacetic acid (3 mL) were added to a reaction flask in sequence, and the mixture was reacted at room temperature. After the reaction was completed, as confirmed by TLC, water (50 mL) was added to the reaction solution, and the pH was adjusted to about 8 with a saturated aqueous sodium bicarbonate solution. The mixture was stirred for 10 min and filtered, and the filter cake was collected and dried to give intermediate **48g** (0.20 g).
MS(ESI, $[M+H]^+$) *m*/*z*: 505.5.

Step 7: Preparation of compound **48**

Intermediate **48g** (85 mg), **40e** (50 mg), dichloroethane/isopropanol (5:1, 20 mL), glacial acetic acid (10 mg), and sodium cyanoborohydride (21 mg) were added to a reaction flask in sequence, and the mixture was reacted at room

temperature. After the reaction was completed, as confirmed by TLC, 50 mL of dichloromethane and 100 mL of water were added to the reaction solution. The organic phase was separated, washed with a saturated sodium chloride solution, dried over anhydrous sodium sulfate, filtered, and concentrated. The concentrate was separated and purified by silica gel column chromatography to give compound **48** (32 mg).

MS(ESI, $[M+H]^+$) *m/z:* 787.5.

1H NMR (500 MHz, DMSO-*d6*) δ 11.22 (s, 1H), 11.08 (s, 1H), 7.76 (d, *J* = 2.9 Hz, 1H), 7.66 (s, 1H), 7.60 (d, *J* = 8.0 Hz, 1H), 7.52 (d, *J* = 8.3 Hz, 2H), 7.34 (d, *J* = 2.8 Hz, 1H), 7.26 (d, *J* = 8.0 Hz, 1H), 7.18 (d, *J* = 8.1 Hz, 2H), 4.56 (dd, *J* = 11.8, 5.0 Hz, 1H), 4.40 (d, *J* = 12.4 Hz, 1H), 4.34 - 4.26 (m, 1H), 3.68 - 3.58 (m, 1H), 3.28 - 3.14 (m, 4H), 3.03 (t, *J* = 12.0 Hz, 1H), 3.00 - 2.90 (m, 2H), 2.90 - 2.74 (m, 4H), 2.72 (d, *J* = 3.0 Hz, 3H), 2.66 (d, *J* = 8.9 Hz, 2H), 2.59 (dd, *J* = 17.7, 4.8 Hz, 3H), 2.48 - 2.42 (m, 2H), 2.30 - 2.13 (m, 5H), 1.89 - 1.72 (m, 3H), 1.65 - 1.53 (m, 1H), 1.41 *(d, J*= 8.1 Hz, 2H).

**Example 49:** Synthesis of Compound **49**

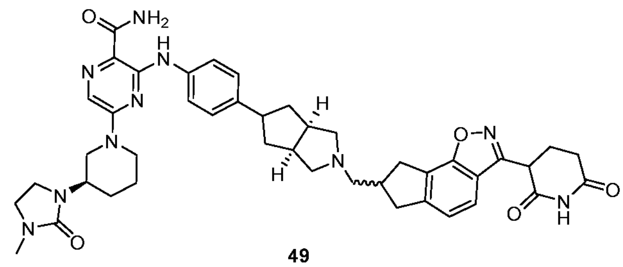

49

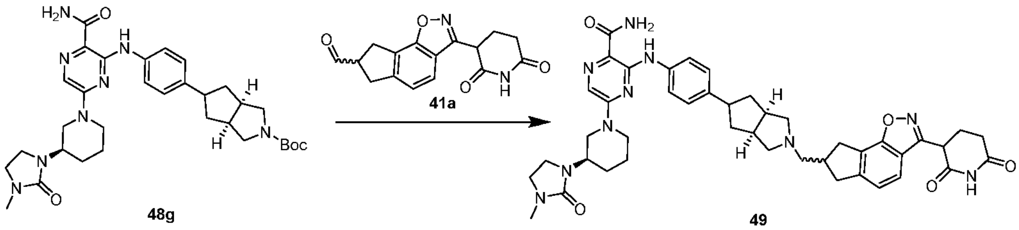

48g

41a

49

Step 1: Preparation of compound **49**

Intermediate **48g** (85 mg), **41a** (50 mg), dichloroethane/isopropanol (5:1, 20 mL), glacial acetic acid (10 mg), and sodium cyanoborohydride (21 mg) were added to a reaction flask in sequence, and the mixture was reacted at room temperature. After the reaction was completed, as confirmed by TLC, 50 mL of dichloromethane and 100 mL of water were added to the reaction solution. The organic phase was separated, washed with a saturated sodium chloride solution, dried over anhydrous sodium sulfate, filtered, and concentrated. The concentrate was separated and purified by silica gel column chromatography to give compound **49** (50 mg).

MS(ESI, $[M+H]^+$) *m/z*: 787.5.

1H NMR (500 MHz, DMSO-*d6*) δ 11.22 (s, 1H), 11.08 (s, 1H), 7.76 (d, *J* = 2.8 Hz, 1H), 7.66 (s, 1H), 7.60 (d, *J* = 8.0 Hz, 1H), 7.56 - 7.46 (m, 2H), 7.34 (d, *J* = 2.8 Hz, 1H), 7.27 (d, *J* = 8.0 Hz, 1H), 7.18 (d, *J* = 8.6 Hz, 2H), 4.56 (dd, *J* = 11.8, 4.9 Hz, 1H), 4.39 (d, *J* = 12.6 Hz, 1H), 4.34 - 4.26 (m, 1H), 3.63 (t, *J* = 11.5 Hz, 1H), 3.29 - 3.13 (m, 4H), 3.04 (t, *J* = 11.7 Hz, 1H), 2.99 - 2.90 (m, 2H), 2.89 - 2.74 (m, 4H), 2.72 (d, *J* = 3.0 Hz, 3H), 2.66 (d, *J* = 8.8 Hz, 2H), 2.63 - 2.54 (m, 3H), 2.46 (d, *J* = 12.4 Hz, 2H), 2.27 - 2.14 (m, 5H), 1.90 - 1.72 (m, 3H), 1.60 - 1.54 (m, 1H), 1.47 - 1.32 (m, 3H).

**Example 50:** Synthesis of Compound **50**

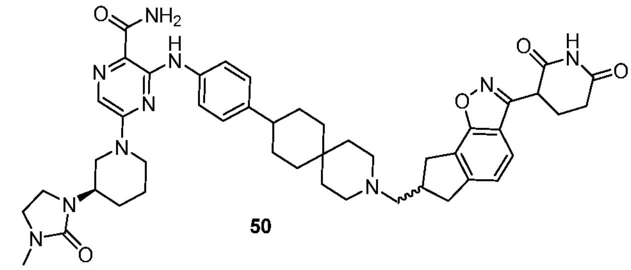

50

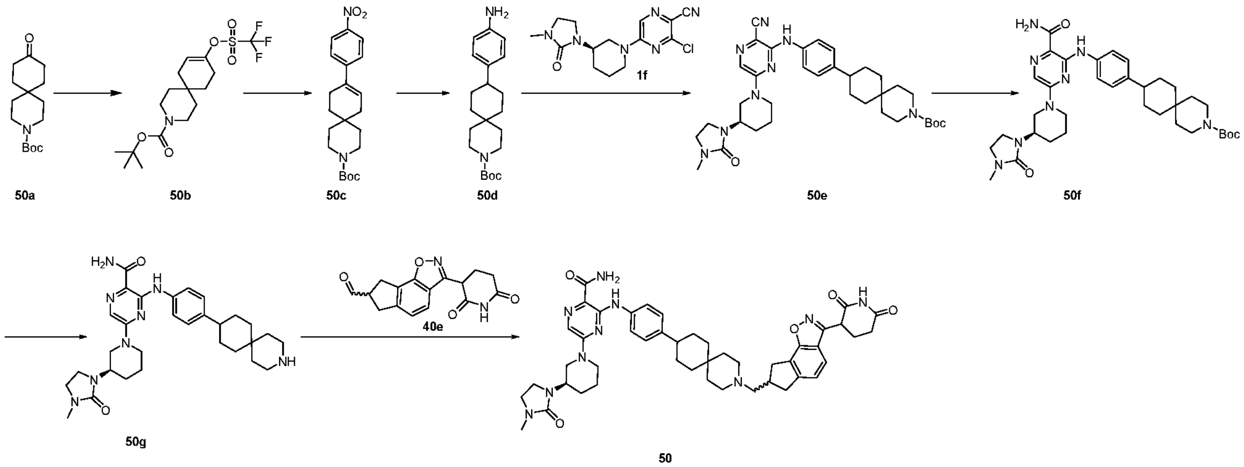

50a 50b 50c 50d 1f 50e 50f 50g 40e 50

Step 1: Preparation of intermediate **50b**

Lithium bis(trimethylsilyl)amide (2.82 g, 16.85 mL) was slowly added dropwise to a solution of **50a** (3 g) in THF (30 mL) at -78 °C under nitrogen atmosphere. After the dropwise addition was completed, the mixture was reacted at -78 °C for 1 h. A solution of *N*-phenylbis(trifluoromethanesulfonyl)imide (4.81 g) in THF (30 mL) was slowly added to the reaction solution. After the addition was completed, the mixture was warmed to room temperature and reacted. After the reaction was completed, 200 mL of petroleum ether was added to the system, and 200 mL of saturated brine was added for extraction. The organic phase was dried over anhydrous sodium sulfate, filtered, and concentrated to give **50b** (4.51 g).

$^1$H NMR (500 MHz, DMSO-*d6*) δ 5.64 (td, *J* = 4.1, 2.0 Hz, 1H), 3.18 (dd, *J* = 13.6, 5.9 Hz, 2H), 3.11 (dd, *J* = 13.3, 6.4 Hz, 2H), 2.13 (tq, *J* = 6.4, 2.0 Hz, 2H), 1.91 (dt, *J* = 4.9, 2.7 Hz, 2H), 1.46 (t, *J* = 6.5 Hz, 2H), 1.22 (s, 9H), 1.14 (t, *J* = 5.9 Hz, 4H).

Step 2: Preparation of intermediate **50c**

4-nitrophenylboronic acid (1.6 g), **50b** (4.59 g), potassium carbonate (3.97 g), Pd(dppf)$_2$Cl$_2$ (0.783 g), 1,4-dioxane (30 mL), and water (8 mL) were added to a reaction flask in sequence, and the mixture was purged several times with nitrogen, heated to 90 °C and reacted. After the reaction was completed, about 200 mL of methyl *tert*-butyl ether was added, and the mixture was washed with saturated brine. The organic phase was dried over anhydrous sodium sulfate, filtered, and concentrated to give a crude product, and the crude product was slurried with petroleum ether to give **50c** (3.83 g).

$^1$H NMR (500 MHz, DMSO-*d6*) δ 8.08 - 7.94 (m, 2H), 7.59 - 7.46 (m, 2H), 6.24 (td, *J* = 4.1, 2.0 Hz, 1H), 3.23 (dt, *J* = 11.9, 5.6 Hz, 2H), 3.11 (d, *J* = 7.4 Hz, 2H), 2.29 - 2.18 (m, 2H), 1.98 (dt, *J* = 4.6, 2.5 Hz, 2H), 1.46 (t, *J* = 6.4 Hz, 2H), 1.23 (s, 9H), 1.17 (t, *J* = 5.8 Hz, 4H).

Step 3: Preparation of intermediate **50d**

**50c** (3.83 g), 10% palladium on carbon (1 g), and methanol (50 mL) were added to a reaction flask in sequence, and the mixture was purged 3 times with hydrogen and reacted overnight at room temperature under hydrogen atmosphere. After the reaction was completed, the reaction solution was filtered under vacuum, and the filtrate was concentrated to give **50d** (3.6 g).

$^1$H NMR (500 MHz, DMSO-*d6*) δ 6.87 (d, *J*= 8.1 Hz, 2H), 6.47 (d, *J*= 8.0 Hz, 2H), 4.85 (s, 2H), 3.30 (q, *J*= 5.9

Hz, 4H), 2.26 (tt, *J*= 10.2, 4.8 Hz, 1H), 1.73 (d, *J*= 13.0 Hz, 2H), 1.50 (pd, *J*= 7.7, 7.1, 2.8 Hz, 6H), 1.39 (s, 9H), 124 (dd, *J* = 7.0, 4.5 Hz, 2H), 1.16 (td, *J* = 13.0, 5.1 Hz, 2H).

Step 4: Preparation of intermediate **50e**

Intermediate **1f** (1.6 g), **50d** (2.84 g), cesium carbonate (4.88 g), BINAP (0.311 g), palladium acetate (0.112 g), and 1,4-dioxane (10 mL) were added to a single-necked flask in sequence, and the mixture was heated to 100 °C and reacted under nitrogen atmosphere. After the reaction was completed, the reaction solution was cooled to room temperature and purified by silica gel column chromatography to give **50e** (2.6 g).

$^1H$ NMR (500 MHz, DMSO-*d*6) δ 8.93 (s, 1H), 7.81 (s, 1H), 7.48 - 7.41 (m, 2H), 7.13 (d, *J*= 8.5 Hz, 2H), 4.41 - 4.16 (m, 2H), 3.56 (q, *J* = 6.0, 4.9 Hz, 1H), 3.23 (ddd, *J* = 10.9, 7.6, 4.7 Hz, 8H), 2.91 (dd, *J* = 21.2, 9.0 Hz, 2H), 2.70 (s, 3H), 2.45 - 2.35 (m, 1H), 1.83 - 1.70 (m, 5H), 1.64 - 1.46 (m, 7H), 1.39 (s, 9H), 1.30 - 1.17 (m, 4H).

Step 5: Preparation of intermediate **50f**

Intermediate **50e** (2.5 g), DMSO (10 mL), and cesium carbonate (1.29 g) were added to a single-necked flask in sequence. 30 wt% hydrogen peroxide (2.254 mL) was added under an ice bath. After the mixture was stirred for 5 min, the ice bath was removed, and the mixture was reacted at room temperature. After the reaction was completed, 300 mL of a saturated sodium sulfite solution was added to the residue to quench the reaction. The color was not changed as detected using a potassium iodide starch reagent, and then 300 mL of ethyl acetate was added for extraction. 100 mL of ethyl acetate was then added for 3 times of extraction. The organic phases were combined, dried over anhydrous sodium sulfate, filtered, and concentrated by evaporation at reduced pressure to remove the solvent. The crude product was separated by silica gel column chromatography to give the target intermediate **50f** (2.41 g).

MS(ESI, $[M+H]^+$) m/z: 647.47.

Step 6: Preparation of intermediate **50g**

**50f** (2.4 g), dichloromethane (10 mL), and TFA (8.46 g, 5.72 mL, 74.2 mmol) were added to a single-necked flask in sequence, and the mixture was reacted at room temperature. After the reaction was completed, the system was added to 200 mL of a saturated sodium bicarbonate solution, and then 200 mL of dichloromethane was added for extraction. The organic phase was dried over anhydrous sodium sulfate, filtered, and concentrated by evaporation at reduced pressure to remove the solvent, thus giving **50g** (1.73 g).

MS(ESI, [M+H]+) m/z: 547.48.

Step 7: Preparation of compound **50**

**40e** (60 mg), isopropanol (1 mL), 1,2-dichloroethane (5.00 mL), and **50g** (109 mg) were added to a reaction flask in sequence, and sodium cyanoborohydride (25.1 mg) was added. The mixture was reacted at room temperature. After the reaction was completed, the reaction solution was purified by silica gel column chromatography to give **50** (56 mg).

MS(ESI, $[M+H]^+$) m/z: 829.57

$^1H$ NMR (500 MHz, DMSO-*d*6) δ 11.20 (s, 1H), 11.09 (s, 1H), 7.75 (d, *J* = 2.9 Hz, 1H), 7.65 (s, 1H), 7.61 (d, *J* = 8.1 Hz, 1H), 7.49 (d, *J*= 8.1 Hz, 2H), 7.33 (d, *J*= 2.9 Hz, 1H), 7.27 (d, *J*= 8.1 Hz, 1H), 7.16 (d, *J*= 8.2 Hz, 2H), 4.63 - 4.51 (m, 1H), 4.44 - 4.23 (m, 2H), 3.61 (dt, *J* = 11.1, 6.6 Hz, 1H), 3.30 (d, *J*= 8.0 Hz, 1H), 3.27 - 3.13 (m, 5H), 3.11 - 2.73 (m, 7H), 2.71 (s, 3H), 2.66 - 2.52 (m, 4H), 2.49 - 2.33 (m, 4H), 2.18 (dt, *J* = 13.2, 4.6 Hz, 1H), 1.90 - 1.71 (m, 5H), 1.70 - 1.47 (m, 7H), 1.41 (s, 2H), 1.26 - 1.15 (m, 2H).

**Example 51:** Synthesis of Compound **51**

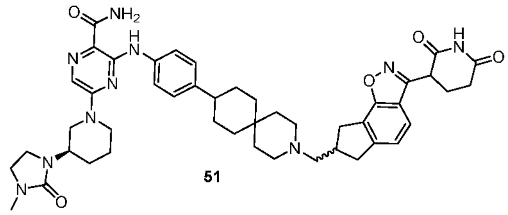

51

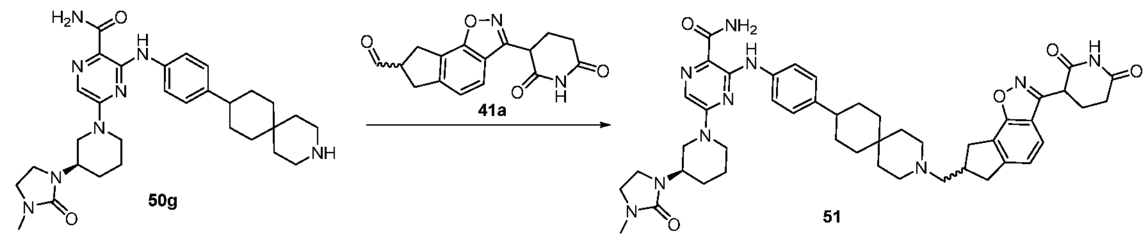



50g

51

Step 1: Preparation of compound **51**

**41a** (60 mg), isopropanol (1 mL), 1,2-dichloroethane (5.00 mL), and **50g** (109 mg) were added to a reaction flask in sequence, and sodium cyanoborohydride (25.1 mg) was added. The mixture was reacted at room temperature. After the reaction was completed, the reaction solution was purified by silica gel column chromatography to give **51** (42 mg).

MS(ESI, [M+H]+) m/z: 829.59.

$^{1}$H NMR (500 MHz, DMSO-*d6*) δ 11.20 (s, 1H), 11.09 (s, 1H), 7.75 (d, *J* = 2.9 Hz, 1H), 7.65 (s, 1H), 7.61 (d, *J* = 8.1 Hz, 1H), 7.49 (d, *J* = 8.1 Hz, 2H), 7.33 (d, *J* = 2.9 Hz, 1H), 7.27 (d, *J* = 8.1 Hz, 1H), 7.16 (d, *J* = 8.2 Hz, 2H), 4.63 - 4.51 (m, 1H), 4.44 - 4.23 (m, 2H), 3.61 (dt, *J* = 11.1, 6.6 Hz, 1H), 3.30 (d, *J* = 8.0 Hz, 1H), 3.27 - 3.13 (m, 5H), 3.11 - 2.73 (m, 7H), 2.71 (s, 3H), 2.66 - 2.52 (m, 4H), 2.49 - 2.33 (m, 4H), 2.18 (dt, *J* = 13.2, 4.6 Hz, 1H), 1.90 - 1.71 (m, 5H), 1.70 - 1.47 (m, 7H), 1.41 (s, 2H), 1.26 - 1.15 (m, 2H).

**Example 52:** Synthesis of Compound **52**

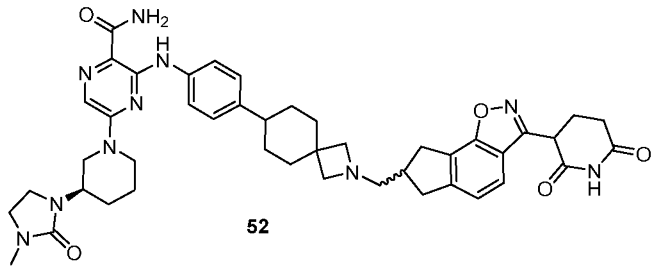

52

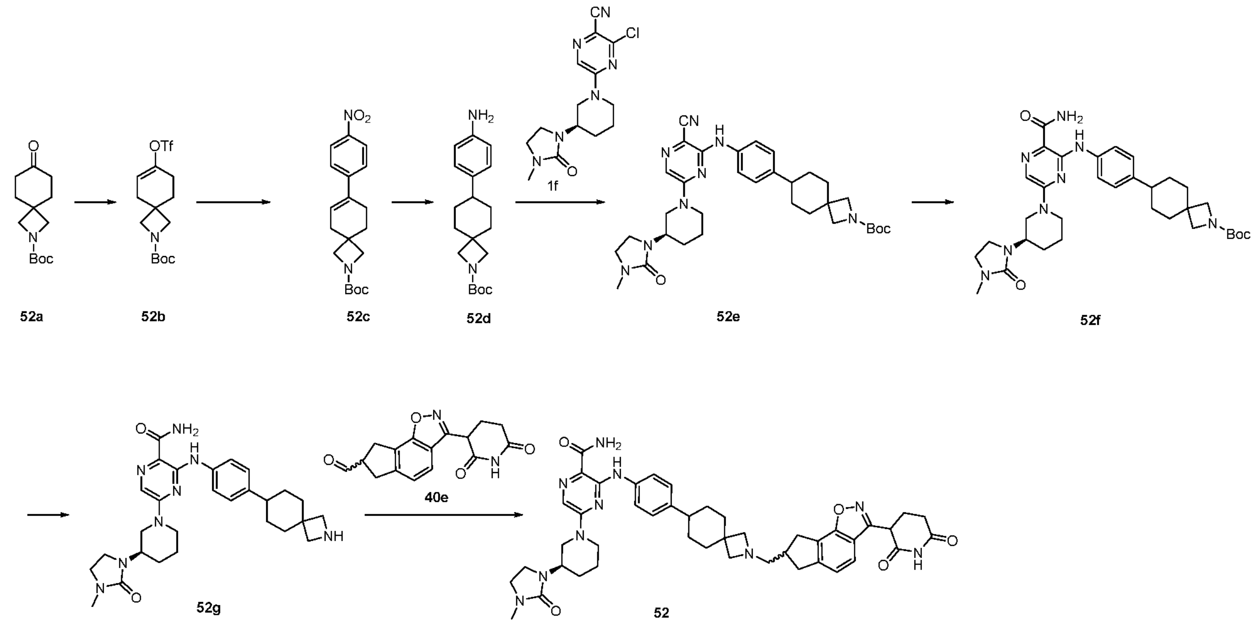

52a 52b 52c 52d 1f 52e 52f 52g 40e 52

Step 1: Preparation of intermediate **52b**

Lithium bis(trimethylsilyl)amide (1 M, 21.75 mL) was slowly added dropwise to a solution of **52a** (4 g) in THF (30 mL) at -78 °C under nitrogen atmosphere. After the dropwise addition was completed, the mixture was reacted at -78 °C for 1 h. A solution of N-phenylbis(trifluoromethanesulfonyl)imide (7.17 g) in THF (30 mL) was slowly added to the reaction solution. After the addition was completed, the mixture was warmed to room temperature and reacted. After the reaction was completed, 200 mL of petroleum ether was added to the system, and 200 mL of saturated brine was added for extraction. The organic phase was dried over anhydrous sodium sulfate, filtered, and concentrated to give **52b** (5 g).

$^1$H NMR (500 MHz, DMSO-*d6*) δ 5.88 - 5.77 (m, 1H), 3.62 (s, 2H), 3.52 (d, *J* = 8.1 Hz, 2H), 2.40 (d, *J* = 4.2 Hz, 4H), 1.90 (t, *J* = 6.1 Hz, 2H), 1.37 (s, 9H).

Step 2: Preparation of intermediate **52c**

4-nitrophenylboronic acid (5.03 g), **52b** (5 g), potassium carbonate (5.58 g), Pd(dppf)$_2$Cl$_2$ (2.199 g), 1,4-dioxane (50 mL), and water (10 mL) were added to a reaction flask in sequence, and the mixture was purged several times with nitrogen, heated to 90 °C, and reacted. After the reaction was completed, about 200 mL of methyl *tert*-butyl ether was added, and the mixture was extracted with 200 mL of saturated brine. The organic phase was dried over anhydrous sodium sulfate, filtered, and concentrated to give a crude product, and the crude product was slurried with petroleum ether to give **50c** (4 g).

$^1$H NMR (500 MHz, DMSO-*d6*) δ 8.23 - 8.12 (m, 2H), 7.75 - 7.62 (m, 2H), 6.37 (td, *J* = 3.9, 1.9 Hz, 1H), 3.65 (s, 2H), 3.56 (d, *J* = 6.8 Hz, 2H), 2.52 (t, *J* = 4.2 Hz, 2H), 2.46 (dt, *J* = 4.6, 2.4 Hz, 2H), 1.89 (t, *J* = 6.3 Hz, 2H), 1.38 (s, 9H).

Step 3: Preparation of intermediate **52d**

**52c** (2 g), 10% palladium on carbon (300 mg), and methanol (30 mL) were added to a reaction flask in sequence, and the mixture was purged 3 times with hydrogen and reacted overnight at room temperature under hydrogen atmosphere. After the reaction was completed, palladium on carbon was removed by filtration under vacuum, and the filtrate was concentrated to give **52d** (1.92 g).

$^1$H NMR (500 MHz, DMSO-*d6*) δ 6.89 - 6.80 (m, 2H), 6.52 - 6.41 (m, 2H), 4.79 (s, 2H), 3.59 (s, 2H), 3.52 - 3.40 (m, 2H), 2.24 (ddd, *J* = 12.0, 8.6, 3.4 Hz, 1H), 1.87 (d, *J* = 12.7 Hz, 2H), 1.67 - 1.56 (m, 2H), 1.48 (td, *J* = 12.9, 3.5 Hz, 2H), 1.38 (s, 9H), 1.31 (qd, *J* = 12.9, 3.0 Hz, 2H).

Step 4: Preparation of intermediate **52e**

Intermediate **1f** (2 g), **52d** (1.915 g), cesium carbonate (5.91 g), BINAP (0.377 g), palladium acetate (0.136 g), and 1,4-dioxane (20 mL) were added to a single-necked flask in sequence, and the mixture was heated to 100 °C and

reacted under nitrogen atmosphere. After the reaction was completed, the reaction solution was cooled to room temperature and purified by silica gel column chromatography to give **52e** (2.58 g).

$^1$H NMR (500 MHz, DMSO-*d*6) δ 8.93 (s, 1H), 7.81 (s, 1H), 7.44 (d, *J*= 8.3 Hz, 2H), 7.10 (d, *J*= 8.2 Hz, 2H), 5.76 (s, 2H), 4.23 (d, *J* = 13.2 Hz, 2H), 3.70 - 3.42 (m, 5H), 3.28 - 3.19 (m, 4H), 2.94 (d, *J* = 14.8 Hz, 2H), 2.71 (s, 3H), 2.43 - 2.29 (m, 1H), 1.91 (d, *J* = 12.7 Hz, 2H), 1.82 - 1.66 (m, 5H), 1.59 - 1.47 (m, 3H), 1.38 (s, 9H).

Step 5: Preparation of intermediate **52f**

Intermediate **52e** (2.5 g), DMSO (10 mL), and cesium carbonate (1.36 g) were added to a single-necked flask in sequence. 30 wt% hydrogen peroxide (2.3 mL) was added under an ice bath. After the mixture was stirred for 5 min, the ice bath was removed, and the mixture was reacted at room temperature. After the reaction was completed, 300 mL of a saturated sodium sulfite solution was added to the residue to quench the reaction. The color was not changed as detected using a potassium iodide starch reagent, and then 300 mL of ethyl acetate was added for extraction. 100 mL of ethyl acetate was then added for 3 times of extraction. The organic phases were combined, dried over anhydrous sodium sulfate, filtered, and concentrated by evaporation at reduced pressure to remove the solvent. The crude product was separated by silica gel column chromatography to give the target intermediate **52f** (1.86 g).

MS(ESI, [M+H]+) m/z: 619.44.

Step 6: Preparation of intermediate **52g**

**52f** (1.86 g), dichloromethane (20 mL), and TFA (6.95 mL) were added to a single-necked flask in sequence, and the mixture was reacted at room temperature. After the reaction was completed, the system was added to 200 mL of a saturated sodium bicarbonate solution, and then 200 mL of dichloromethane was added for extraction. The organic phase was dried over anhydrous sodium sulfate, filtered, and concentrated by evaporation at reduced pressure to remove the solvent, thus giving **52g** (1.6 g).

MS(ESI, [M+H]+) m/z: 519.35.

Step 7: Preparation of compound **52**

**40e** (60 mg), isopropanol (1 mL), 1,2-dichloroethane (5.00 mL), and **52g** (104 mg) were added to a reaction flask in sequence, and sodium cyanoborohydride (25.1 mg) was added. The mixture was reacted at room temperature. After the reaction was completed, the reaction solution was purified by silica gel column chromatography to give **52** (35 mg).

MS(ESI, [M+H]+) m/z: 801.66.

$^1$H NMR (500 MHz, DMSO-$d_6$) δ 11.20 (s, 1H), 11.09 (s, 1H), 7.75 (d, *J* = 2.8 Hz, 1H), 7.65 (s, 1H), 7.61 (d, *J* = 8.1 Hz, 1H), 7.49 (d, *J*= 8.2 Hz, 2H), 7.33 (d, *J*= 2.9 Hz, 1H), 7.26 (d, *J*= 8.1 Hz, 1H), 7.13 (d, *J*= 8.2 Hz, 2H), 4.57 (dd, *J*= 11.8, 5.0 Hz, 1H), 4.33 (dd, *J* = 41.6, 13.0 Hz, 2H), 3.61 (ddt, *J*= 11.2, 8.4, 4.6 Hz, 1H), 3.45 - 3.33 (m, 3H), 3.30 - 3.09 (m, 5H), 3.09 - 2.91 (m, 4H), 2.91 - 2.82 (m, 2H), 2.72 (s, 5H), 2.60 (dt, *J* = 17.1, 4.3 Hz, 2H), 2.55 - 2.51 (m, 1H), 2.49 - 2.44 (m, 1H), 2.44 - 2.35 (m, 1H), 2.22 - 2.14 (m, 1H), 1.99 (s, 2H), 1.86 - 1.79 (m, 2H), 1.79 - 1.73 (m, 1H), 1.71 (dd, *J*= 12.2, 7.7 Hz, 2H), 1.61 - 1.47 (m, 3H), 1.39 (d, *J*= 12.7 Hz, 2H).

**Example 53:** Synthesis of Compound **53**

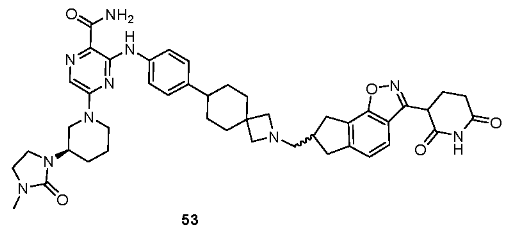

53

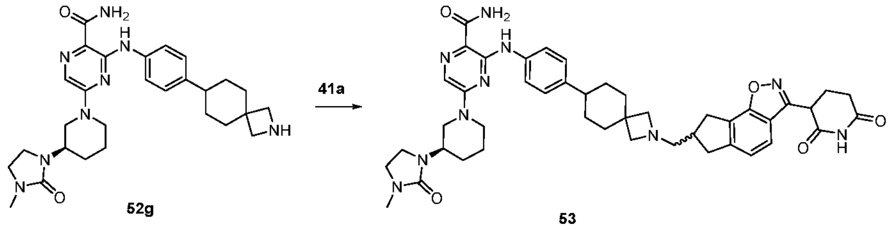

52g 53

Step 1: Preparation of compound **53**

**41a** (60 mg), isopropanol (1 mL), 1,2-dichloroethane (5.00 mL), and **52g** (104 mg) were added to a reaction flask in sequence, and sodium cyanoborohydride (25.1 mg) was added. The mixture was reacted at room temperature. After the reaction was completed, the reaction solution was purified by silica gel column chromatography to give **53** (45 mg).

MS(ESI, [M+H]+) m/z: 801.53.

$^1$H NMR (500 MHz, DMSO-$d_6$) δ 11.20 (s, 1H), 11.09 (s, 1H), 7.75 (d, *J* = 2.8 Hz, 1H), 7.65 (s, 1H), 7.61 *(d, J*= 8.1 Hz, 1H), 7.49 (d, *J*= 8.2 Hz, 2H), 7.33 (d, *J*= 2.9 Hz, 1H), 7.26 (d, *J*= 8.1 Hz, 1H), 7.13 (d, *J*= 8.2 Hz, 2H), 4.57 (dd, *J*= 11.8, 5.0 Hz, 1H), 4.33 (dd, *J* = 41.6, 13.0 Hz, 2H), 3.61 (ddt, *J*= 11.2, 8.4, 4.6 Hz, 1H), 3.45 - 3.33 (m, 3H), 3.30 - 3.09 (m, 5H), 3.09 - 2.91 (m, 4H), 2.91 - 2.82 (m, 2H), 2.72 (s, 5H), 2.60 (dt, *J* = 17.1, 4.3 Hz, 2H), 2.55 - 2.51 (m, 1H), 2.49 - 2.44 (m, 1H), 2.44 - 2.35 (m, 1H), 2.22 - 2.14 (m, 1H), 1.99 (s, 2H), 1.86 - 1.79 (m, 2H), 1.79 - 1.73 (m, 1H), 1.71 (dd, *J*= 12.2, 7.7 Hz, 2H), 1.61 - 1.47 (m, 3H), 1.39 (d, *J*= 12.7 Hz, 2H).

**Example 54:** Synthesis of Compound **54**

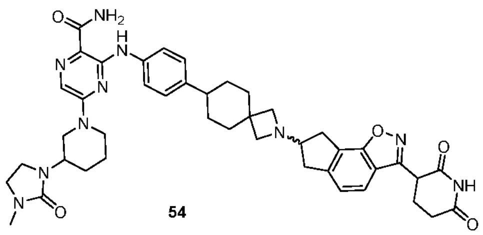
54

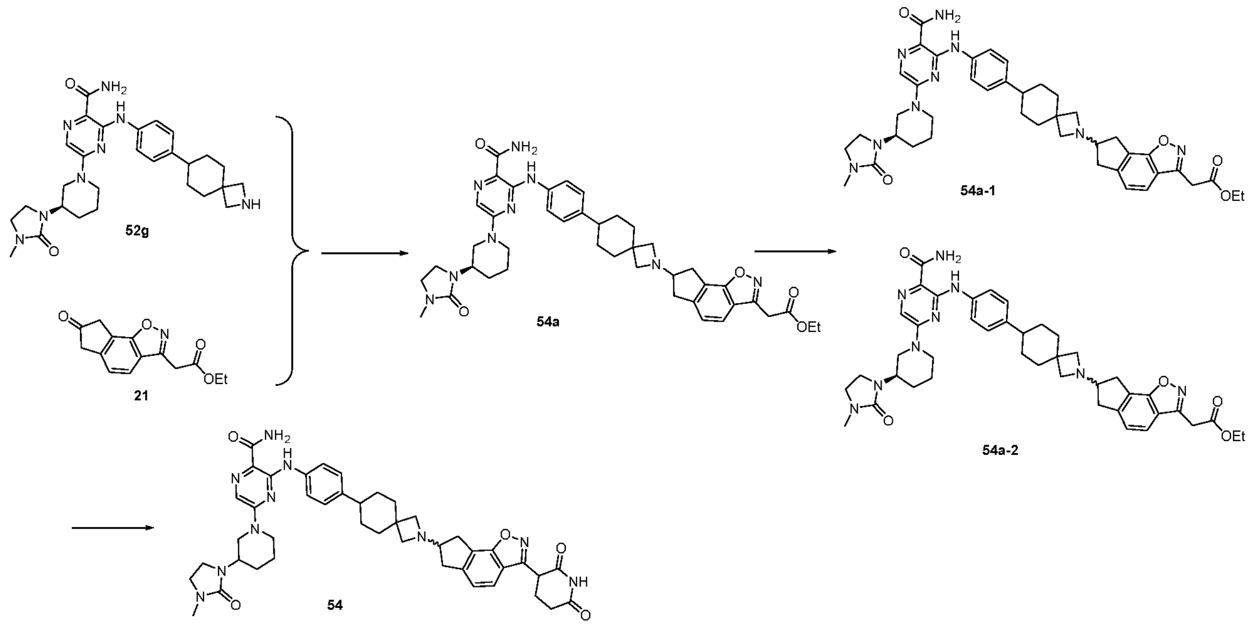
52g 21 54a 54a-1 54a-2 54

Step 1: Preparation of intermediates **54a-1** and **54a-2**

Intermediate **21** (300 mg), intermediate **52g** (600 mg), 1,2-dichloroethane (10 mL), isopropanol (3 mL), and sodium cyanoborohydride (291 mg) were added to a reaction flask in sequence, and the mixture was reacted overnight at room temperature. After the reaction was completed, 5 mL of a saturated sodium bicarbonate solution was added to the reaction solution, and then dichloromethane and saturated brine were added for extraction. The organic phase was separated, and the aqueous phase was extracted with dichloromethane. The organic phases were combined, washed with saturated brine, dried over anhydrous sodium sulfate, and concentrated, and the residue was purified by silica gel column chromatography to give **54a. 54a** was separated by high performance liquid chromatography to give intermediate prepeak **54a-1** (170 mg) and postpeak **54a-2** (160 mg) in sequence. The conditions of preparative chromatography were as follows: instrument and preparative column: YMC high pressure preparative chromatograph was used, and the preparative column was CHIRALART Cellulose-SC. Mobile phase system: ethanol-dichloromethane (15:70)/n-hexane-0.1% diethylamine, isocratic elution: ethanol-dichloromethane (3:2)/n-hexane-0.1% diethylamine = 85/15.

**54a-1,, 54a-2:** MS(ESI, $[M+H]^+$) m/z: 762.59.

Step 2: Preparation of compound **54**

Intermediate **54a-1** (100 mg), acrylamide (15 mg) and tetrahydrofuran (5 mL) were added to a reaction flask in sequence, and the mixture was cooled to 0 °C under $N_2$ atmosphere. Potassium *tert*-butoxide (1 M, 0.079 mL) was added, and the mixture was reacted at 0 °C. After the reaction was completed, as confirmed by TLC, the reaction solution was added dropwise to an icy saturated aqueous ammonium chloride solution, and 50 mL of ethyl acetate was added for extraction. The organic phase was dried over anhydrous sodium sulfate, filtered, and concentrated, and the residue was purified by silica gel column chromatography to give compound **54** (25 mg).

MS(ESI, [M+H]+) m/z: 787.36.

$^1$H NMR (500 MHz, DMSO-*d*6) δ 11.20 (s, 1H), 11.09 (d, *J*= 1.5 Hz, 1H), 7.75 (d, *J*= 2.8 Hz, 1H), 7.65 (s, 1H), 7.62 (d, *J* = 7.4 Hz, 1H), 7.48 (d, *J* = 8.3 Hz, 2H), 7.33 (d, *J* = 2.8 Hz, 1H), 7.26 (d, *J* = 8.0 Hz, 1H), 7.12 (d, *J* = 8.2 Hz, 2H), 4.57 (dd, *J* = 11.9, 5.0 Hz, 1H), 4.32 (dd, *J* = 40.5, 12.7 Hz, 2H), 3.65 - 3.56 (m, 1H), 3.43 - 3.33 (m, 2H), 3.26 (dd, *J* = 9.0, 7.3 Hz, 2H), 3.18 - 2.89 (m, 9H), 2.77 (ddd, *J* = 17.1, 11.9, 5.2 Hz, 3H), 2.71 (s, 3H), 2.60 (dq, *J* = 17.4, 4.0 Hz, 1H), 2.51-2.53 (m, 1H), 2.42 - 2.33 (m, 1H), 2.25 - 2.13 (m, 1H), 1.99 - 1.86 (m, 2H), 1.85 - 1.79 (m, 2H), 1.76 (dt, *J* = 7.0, 3.1 Hz, 1H), 1.67 (d, *J* = 12.1 Hz, 2H), 1.61 - 1.44 (m, 3H), 1.44 - 1.32 (m, 2H).

**Example 55:** Synthesis of Compound **55**

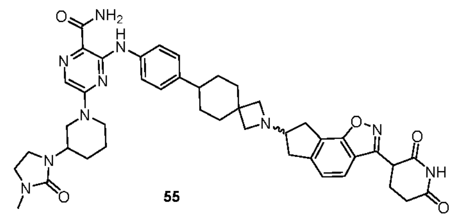

55

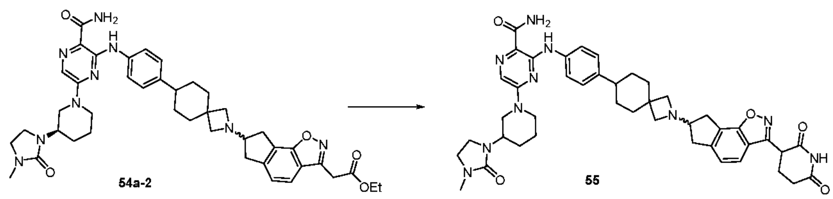

54a-2

55

Step 1: Preparation of compound **55**

Intermediate **54a-2** (100 mg), acrylamide (15 mg) and tetrahydrofuran (5 mL) were added to a reaction flask in sequence, and the mixture was cooled to 0 °C under $N_2$ atmosphere. Potassium *tert*-butoxide (1 M, 0.079 mL) was added, and the mixture was reacted at 0 °C. After the reaction was completed, as confirmed by TLC, the reaction solution was added dropwise to an icy saturated aqueous ammonium chloride solution, and 50 mL of ethyl acetate was added for extraction. The organic phase was dried over anhydrous sodium sulfate, filtered, and concentrated, and the residue

was purified by silica gel column chromatography to give compound **55** (33 mg).

MS(ESI, $[M+H]^+$) m/z: 787.46.

$^1H$ NMR (500 MHz, DMSO-*d*6) δ 11.20 (s, 1H), 11.09 (d, *J*= 1.5 Hz, 1H), 7.75 (d, *J*= 2.8 Hz, 1H), 7.65 (s, 1H), 7.62 (d, *J* = 7.4 Hz, 1H), 7.48 (d, *J* = 8.3 Hz, 2H), 7.33 (d, *J* = 2.8 Hz, 1H), 7.26 (d, *J* = 8.0 Hz, 1H), 7.12 (d, *J* = 8.2 Hz, 2H), 4.57 (dd, *J* = 11.9, 5.0 Hz, 1H), 4.32 (dd, *J* = 40.5, 12.7 Hz, 2H), 3.65 - 3.56 (m, 1H), 3.43 - 3.33 (m, 2H), 3.26 (dd, *J* = 9.0, 7.3 Hz, 2H), 3.18 - 2.89 (m, 9H), 2.77 (ddd, *J* = 17.1, 11.9, 5.2 Hz, 3H), 2.71 (s, 3H), 2.60 (dq, *J* = 17.4, 4.0 Hz, 1H), 2.51-2.53 (m, 1H), 2.42 - 2.33 (m, 1H), 2.25 - 2.13 (m, 1H), 1.99 - 1.86 (m, 2H), 1.85

**[1006]** - 1.79 (m, 2H), 1.76 (dt, *J* = 7.0, 3.1 Hz, 1H), 1.67 (d, *J*= 12.1 Hz, 2H), 1.61 - 1.44 (m, 3H), 1.44 - 1.32 (m, 2H).

**Example 56:** Synthesis of Compound **56**

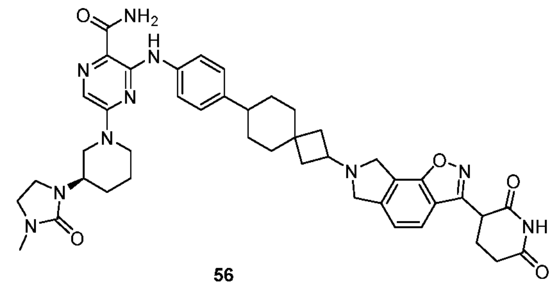

**56**

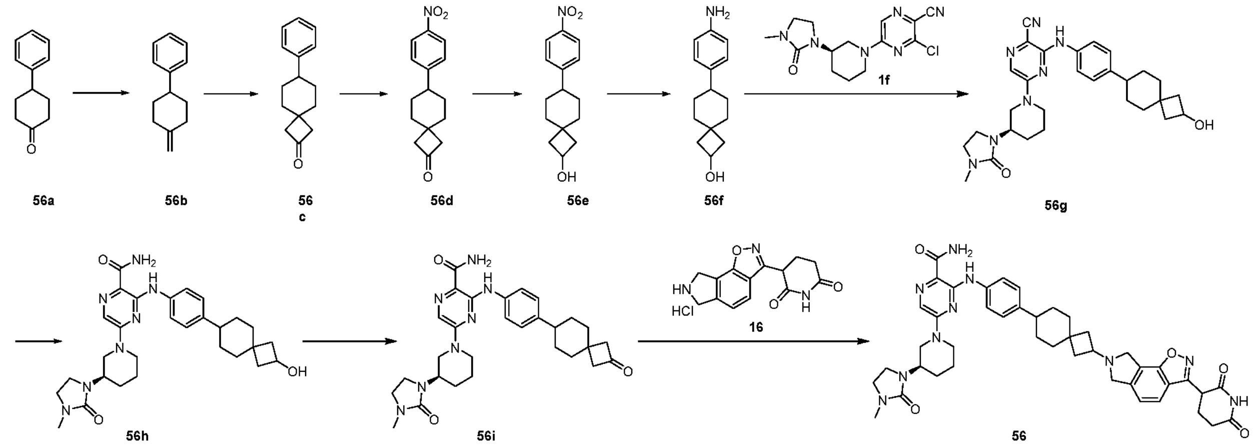


Step 1: Preparation of compound **56b**

Lithium diisopropylamide (2 M, 21.52 mL) was slowly added dropwise to a stirred solution of methyl triphenyl phosphonium bromide (13.33 g, 37.3 mmol) in THF (50 mL) at 0 °C under nitrogen atmosphere, and the mixture was reacted at room temperature for 10 min. **56a** (5 g, 28.7 mmol) was added to the above reaction solution, and the mixture was reacted at room temperature. After the reaction was completed, 200 mL of petroleum ether and 100 mL of water were added to the reaction solution for extraction. The organic phase was dried over anhydrous sodium sulfate, filtered, and concentrated to give **56b** (4.6 g).

Step 2: Preparation of compound **56c**

**56b** (7.43 g), Cu-Zn (16.68 g, 129 mmol), and 100 mL of diethyl ether were added to a reaction flask in sequence. After the mixture was purged with nitrogen, trichloroacetyl chloride (9.63 mL) was added, and the mixture was reacted under an ice bath. After the reaction was completed, the reaction solution was added to about 300 mL of a saturated sodium bicarbonate solution to quench the reaction, and the mixture was slurried with 200 mL of ethyl acetate. The solid was removed by filtration under vacuum, and the mother liquor was extracted. The organic phase was dried over anhydrous sodium sulfate, filtered, and concentrated. Zinc powder (8.46 g, 129 mmol) was added to the concentrate, 30 mL of acetic acid and 15 mL of water were added, and the mixture was reacted under reflux. After the reaction was

completed, the reaction solution was poured into 300 mL of a saturated sodium bicarbonate solution, and the mixture was slurried with 200 mL of petroleum ether. Filtration under vacuum was performed, and the mother liquor was extracted for liquid separation. The organic phase was dried over anhydrous sodium sulfate, filtered, and concentrated to give **56c** (4.5 g).

$^1H$ NMR (500 MHz, DMSO-*d*6) δ 7.31 - 7.25 (m, 2H), 7.25 - 7.21 (m, 2H), 7.20 - 7.14 (m, 1H), 2.83 (q, *J*= 2.7, 2.0 Hz, 2H), 2.80 - 2.74 (m, 2H), 2.57 - 2.51 (m, 1H), 1.83 - 1.74 (m, 4H), 1.70 (td, *J* = 13.6, 12.6, 3.6 Hz, 2H), 1.52 - 1.40 (m, 2H).

Step 3: Preparation of intermediate **56d**

**56c** (4.5 g), dichloromethane (50 mL), and sulfuric acid (2.251 mL) were added to a reaction flask in sequence, and nitric acid (1.62 mL) was added under an ice bath. The mixture was reacted at room temperature. After the reaction was completed, the reaction solution was added to a saturated sodium bicarbonate solution, and 200 mL of ethyl acetate was added for extraction. The organic phase was dried over anhydrous sodium sulfate, filtered, and concentrated, and the concentrate was slurried with a small amount of methyl *tert*-butyl ether and filtered under vacuum to give **56d** (1.68 g).

$^1H$ NMR (500 MHz, DMSO-d6) δ 7.28 (t, *J* = 7.5 Hz, 2H), 7.25 - 7.21 (m, 2H), 2.82 (d, *J* = 2.8 Hz, 2H), 2.78 - 2.75 (m, 2H), 2.54 (dt, *J* = 12.2, 3.3 Hz, 1H), 1.85 - 1.69 (m, 8H).

Step 4: Preparation of intermediate **56e**

**56d** (1.68 g), MeOH (20 mL), and sodium borohydride (0.245 g) were added to a reaction flask in sequence under an ice bath, and the mixture was reacted at room temperature. After the reaction was completed, the reaction solution was added to a saturated ammonium chloride solution, and 200 mL of ethyl acetate was added for extraction. The organic phase was dried over anhydrous sodium sulfate, filtered, and concentrated to give **56e** (1.75 g).

$^1H$ NMR (500 MHz, DMSO-d6) δ 8.21 - 8.12 (m, 2H), 7.55 - 7.47 (m, 2H), 4.87 (d, *J* = 6.3 Hz, 1H), 4.11 - 4.07 (m, 1H), 2.60 (tt, *J* = 11.7, 3.2 Hz, 1H), 2.27 (td, *J* = 7.2, 3.6 Hz, 1H), 2.01 (dq, *J*= 7.4, 4.1, 3.6 Hz, 1H), 1.68 - 1.46 (m, 10H).

Step 5: Preparation of intermediate **56f**

**56e** (3.2 g), iron powder (2.5 g), ammonium chloride (3 g), 30 mL of MeOH, and 10 mL of water were added to a reaction flask in sequence, and the mixture was warmed to 80 °C and reacted. After the reaction was completed, the solid was removed by filtration under vacuum, 200 mL of ethyl acetate was added to the mother liquor, and 200 mL of a saturated sodium carbonate solution was added for extraction. The organic phase was dried over anhydrous sodium sulfate, filtered, and concentrated to give **56f** (2.09 g).

MS(ESI, $[M+H]^+$) m/z: 232.05.

Step 6: Preparation of intermediate **56g**

Intermediate **1f** (1.4 g), **56f** (1.363 g), cesium carbonate (4.27 g), BINAP (0.272 g), palladium acetate (0.098 g), and 1,4-dioxane (10 mL) were added to a single-necked flask in sequence, and the mixture was heated to 100 °C and reacted under nitrogen atmosphere. After the reaction was completed, the reaction solution was cooled to room temperature, mixed with silica gel, and then purified by column chromatography to give **56g** (1.5 g).

MS(ESI, $[M+H]^+$) m/z: 516.48.

Step 7: Preparation of intermediate **56h**

Intermediate **56g** (1.5 g), DMSO (5 mL), and cesium carbonate (0.948 g) were added to a single-necked flask in sequence. 30 wt% hydrogen peroxide (2 mL) was added under an ice bath. After the mixture was stirred for 5 min, the ice bath was removed, and the mixture was reacted at room temperature. After the reaction was completed, 300 mL of a saturated sodium sulfite solution was added to the residue to quench the reaction. The color was not changed as detected using a potassium iodide starch reagent, and then 300 mL of ethyl acetate was added for extraction. 100 mL of ethyl acetate was then added for 3 times of extraction. The organic phases were combined, dried over anhydrous sodium sulfate, filtered, and concentrated by evaporation at reduced pressure to remove the solvent. The crude product was separated by silica gel column chromatography to give the target intermediate **56h** (1.35 g).

MS(ESI, [M+H]+) m/z: 534.50.

Step 8: Preparation of intermediate **56i**

**56h** (1 g), IBX (1.574 g), and DMSO (15 mL) were added to a reaction flask in sequence, and the mixture was reacted at room temperature. After the reaction was completed, 200 mL of ethyl acetate was added, and 200 mL of a saturated sodium bicarbonate solution was added for extraction. The organic phase was dried over anhydrous sodium sulfate, filtered, and concentrated to give **56i** (980 mg).
MS(ESI, [M+H]+) m/z: 532.49.

Step 9: Preparation of compound **56**

**56i** (70 mg), isopropanol (1 mL), 1,2-dichloroethane (5.00 mL), and **16** (60.9 mg) were added to a reaction flask in sequence, and sodium cyanoborohydride (25 mg) was added. The mixture was reacted at room temperature. After the reaction was completed, the reaction solution was mixed with silica gel and purified by column chromatography to give **56** (21 mg).
MS(ESI, $[M+H]^+$) m/z: 787.48.
$^1$H NMR (500 MHz, DMSO-*d*6) δ 11.20 (s, 1H), 11.10 (s, 1H), 7.79 - 7.74 (m, 1H), 7.72 (d, *J* = 8.1 Hz, 1H), 7.65 (s, 1H), 7.49 (d, *J* = 8.0 Hz, 2H), 7.38 - 7.27 (m, 2H), 7.14 (d, *J* = 8.1 Hz, 2H), 4.60 (dd, *J* = 11.9, 5.0 Hz, 1H), 4.33 (dd, *J* = 47.2, 12.8 Hz, 2H), 4.10 (s, 2H), 4.07 - 3.91 (m, 2H), 3.61 (q, *J* = 9.2, 6.4 Hz, 1H), 3.42 - 3.34 (m, 2H), 3.27 (d, *J* = 7.0 Hz, 2H), 2.99 (dt, *J* = 34.2, 11.8 Hz, 2H), 2.85 - 2.69 (m, 4H), 2.61 (dt, *J* = 17.4, 4.2 Hz, 1H), 2.56 - 2.51 (m, 1H), 2.39 (t, *J* = 11.0 Hz, 1H), 2.25 - 2.08 (m, 2H), 1.97 - 1.32 (m, 16H).

**Example 57:** Synthesis of Compound **57**

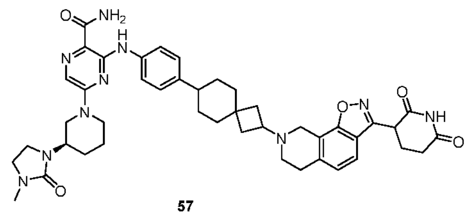

**57**

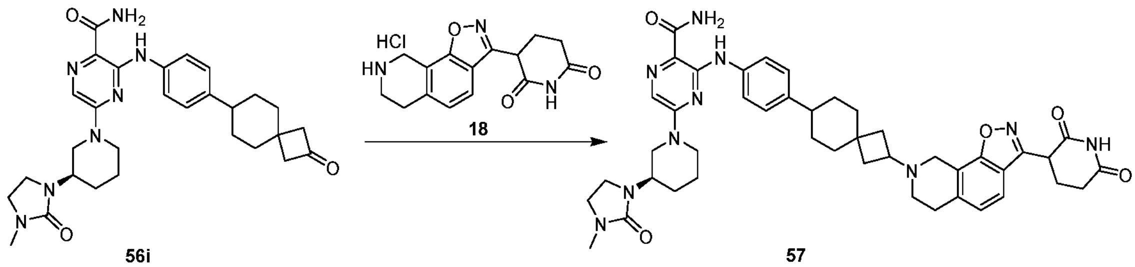


**56i** **57**

Step 1: Preparation of compound **57**

**56i** (70 mg), isopropanol (1 mL), 1,2-dichloroethane (5.00 mL), and **18** (50 mg) were added to a reaction flask in sequence, and sodium cyanoborohydride (25 mg) was added. The mixture was reacted at room temperature. After the reaction was completed, the reaction solution was mixed with silica gel and purified by column chromatography to give **57** (28 mg).
MS(ESI, [M+H]+) m/z: 801.49.
$^1$H NMR (500 MHz, DMSO-*d*6) δ 11.20 (d, *J*= 2.3 Hz, 1H), 11.09 (s, 1H), 7.75 (d, *J*= 2.8 Hz, 1H), 7.65 (s, 1H), 7.59 (d, *J* = 8.1 Hz, 1H), 7.49 (d, *J* = 8.2 Hz, 2H), 7.33 (d, *J* = 2.8 Hz, 1H), 7.14 (dd, *J* = 8.4, 3.7 Hz, 3H), 4.56 (dd, *J* = 11.9, 5.0 Hz, 1H), 4.33 (dd, *J* = 44.6, 13.0 Hz, 2H), 3.69 (s, 2H), 3.61 (t, *J* = 7.6 Hz, 1H), 3.39 - 3.32 (m, 2H), 3.28 (qd, *J*= 8.0, 4.1 Hz, 2H), 3.06 - 2.90 (m, 5H), 2.82 - 2.70 (m, 4H), 2.67 - 2.57 (m, 3H), 2.44 - 2.35 (m, 1H), 2.25 - 2.15 (m, 2H), 2.01 - 1.94 (m, 1H), 1.91 -1.32 (m, 15H).

**Example 58:** Synthesis of Compound **58**

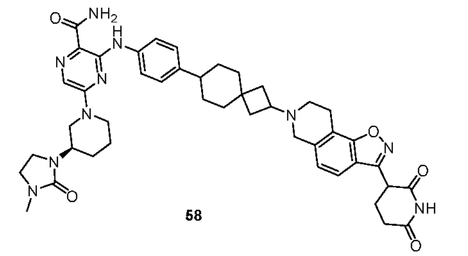

**58**

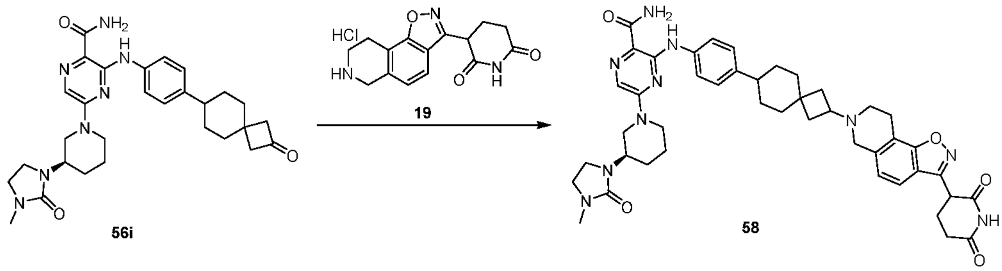



**56i** **58**

Step 1: Preparation of compound **58**

**56i** (70 mg), isopropanol (1 mL), 1,2-dichloroethane (5.00 mL), and **19** (50 mg) were added to a reaction flask in sequence, and sodium cyanoborohydride (25 mg) was added. The mixture was reacted at room temperature. After the reaction was completed, the reaction solution was mixed with silica gel and purified by column chromatography to give **58** (15 mg).

MS(ESI, [M+H]+) m/z: 801.47.

$^1$H NMR (500 MHz, DMSO-d6) δ 11.28 - 11.14 (m, 1H), 11.09 (s, 1H), 7.75 (d, *J* = 2.9 Hz, 1H), 7.65 (s, 1H), 7.58 (d, *J*= 8.0 Hz, 1H), 7.49 (d, *J* = 8.1 Hz, 2H), 7.33 (d, *J* = 3.0 Hz, 1H), 7.14 (d, *J* = 8.4 Hz, 3H), 4.56 (dd, *J* = 11.9, 5.1 Hz, 1H), 4.37 (d, *J*= 12.6 Hz, 1H), 4.29 (d, *J*= 13.2 Hz, 1H), 3.68 - 3.51 (m, 3H), 3.45 - 3.24 (m, 6H), 3.15 - 2.84 (m, 5H), 2.80 - 2.56 (m, 6H), 2.38 (d, *J* = 14.4 Hz, 1H), 2.19 (dq, *J* = 15.9, 7.2, 5.9 Hz, 2H), 1.95 (s, 1H), 1.87 - 1.33 (m, 14H).

**Example 59:** Synthesis of Compound **59**

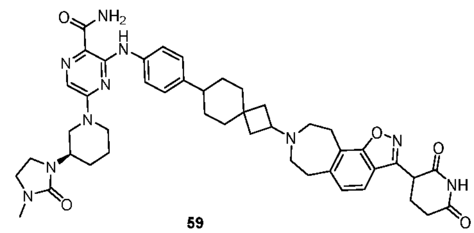

**59**

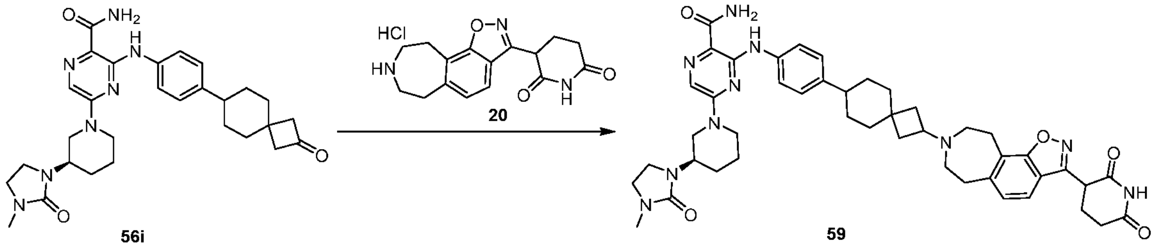



**56i** **59**

Step 1: Preparation of compound **59**

**56i** (70 mg), isopropanol (1 mL), 1,2-dichloroethane (5.00 mL), and **20** (53 mg) were added to a reaction flask in sequence, and sodium cyanoborohydride (25 mg) was added. The mixture was reacted at room temperature. After the reaction was completed, the reaction solution was mixed with silica gel and purified by column chromatography to give **59** (24 mg).

MS(ESI, [M+H]+) m/z: 815.42.

$^1$H NMR (500 MHz, DMSO-*d*6) δ 11.20 (d, *J* = 2.4 Hz, 1H), 11.08 (s, 1H), 7.75 (d, *J* = 2.9 Hz, 1H), 7.65 (s, 1H), 7.54 (d, *J* = 8.0 Hz, 1H), 7.49 (d, *J* = 8.2 Hz, 2H), 7.33 (d, *J* = 2.9 Hz, 1H), 7.19 (d, *J* = 8.1 Hz, 1H), 7.13 (d, *J* = 8.2 Hz, 2H), 4.55 (dd, *J*= 11.9, 5.0 Hz, 1H), 4.47 - 4.23 (m, 2H), 3.62 (ddt, *J*= 11.3, 8.6, 4.0 Hz, 1H), 3.37 (d, *J* = 8.3 Hz, 2H), 3.31 - 3.24 (m, 2H), 3.19 - 3.11 (m, 2H), 3.09 - 2.92 (m, 4H), 2.84 - 2.71 (m, 5H), 2.60 (dt, *J* = 17.3, 4.2 Hz, 1H), 2.49 - 2.33 (m, 5H), 2.22 - 2.10 (m, 2H), 1.93 - 1.38 (m, 16H).

**Example 60:** Synthesis of Compound **60**

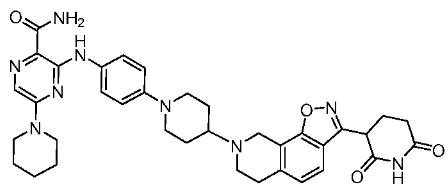

**60**

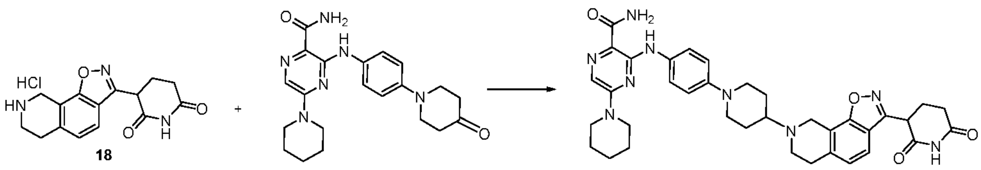

**18** + **31c** **60**

Step 1: Preparation of compound **60**

**31c** (100 mg), isopropanol (1 mL), 1,2-dichloroethane (5.00 mL), and **18** (82 mg) were added to a reaction flask in sequence, and sodium cyanoborohydride (31 mg) was added. The mixture was reacted at room temperature. After the reaction was completed, the reaction solution was mixed with silica gel and purified by column chromatography to give **60** (45 mg).

MS(ESI, [M+H]+) m/z: 664.4.

$^1$H NMR (500 MHz, DMSO-d6) δ 11.08 (d, *J* = 6.4 Hz, 2H), 7.69 (d, *J* = 2.9 Hz, 1H), 7.60 (s, 1H), 7.58 (d, *J* = 8.1 Hz, 1H), 7.48 - 7.39 (m, 2H), 7.26 (d, *J* = 3.0 Hz, 1H), 7.14 (d, *J* = 8.2 Hz, 1H), 6.97 - 6.89 (m, 2H), 4.56 (dd, *J* = 11.9, 5.0 Hz, 1H), 3.98 (s, 2H), 3.70 (d, *J* = 11.7 Hz, 2H), 3.65 (t, *J* = 5.4 Hz, 4H), 2.94 (t, *J* = 5.4 Hz, 2H), 2.87 (t, *J* = 5.6 Hz, 2H), 2.76 (td, *J* = 12.0, 5.9 Hz, 1H), 2.72 - 2.57 (m, 4H), 2.48 (d, *J* = 4.1 Hz, 1H), 2.24 - 2.13 (m, 1H), 1.97 (d, *J* = 11.7 Hz, 2H), 1.68 (ddt, *J* = 22.6, 11.1, 4.6 Hz, 4H), 1.58 (h, *J* = 5.5, 4.4 Hz, 4H).

**Example 61:** Synthesis of Compound **61**

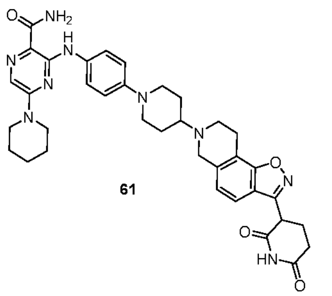

61

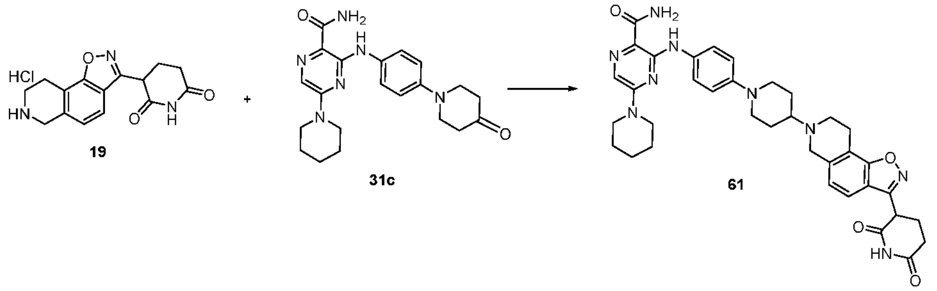

19 31c 61

Step 1: Preparation of compound **61**

**31c** (100 mg), isopropanol (1 mL), 1,2-dichloroethane (5.00 mL), and **19** (82 mg) were added to a reaction flask in sequence, and sodium cyanoborohydride (31 mg) was added. The mixture was reacted at room temperature. After the reaction was completed, the reaction solution was mixed with silica gel and purified by column chromatography to give **61** (41 mg).

MS(ESI, [M+H]+) m/z: 664.78.

$^{1}$H NMR (500 MHz, DMSO-*d*6) δ 11.08 (d, *J* = 4.3 Hz, 2H), 7.69 (d, *J* = 3.0 Hz, 1H), 7.61 (s, 1H), 7.57 (d, *J* = 8.2 Hz, 1H), 7.44 (d, *J* = 8.7 Hz, 2H), 7.26 (d, *J* = 2.9 Hz, 1H), 7.12 (d, *J* = 8.3 Hz, 1H), 6.93 (d, *J* = 8.7 Hz, 2H), 4.55 (dd, *J* = 11.9, 5.0 Hz, 1H), 3.89 (s, 2H), 3.70 (d, *J* = 11.7 Hz, 2H), 3.66 (t, *J* = 5.3 Hz, 4H), 2.97 (d, *J* = 5.7 Hz, 2H), 2.90 (t, *J* = 5.7 Hz, 2H), 2.77 (ddd, *J* = 17.2, 11.9, 5.3 Hz, 1H), 2.71 - 2.56 (m, 4H), 2.49 - 2.43 (m, 1H), 2.19 (dt, *J*= 13.4, 4.6 Hz, 1H), 1.97 - 1.88 (m, 2H), 1.66 (td, *J* = 12.0, 4.5 Hz, 4H), 1.58 (q, *J*= 5.7, 5.2 Hz, 4H).

**Example 62:** Synthesis of Compound **62**

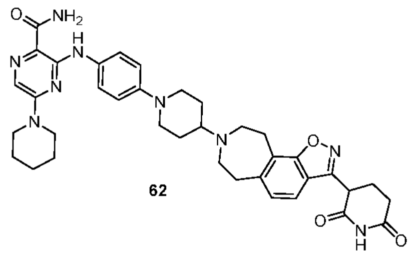

62

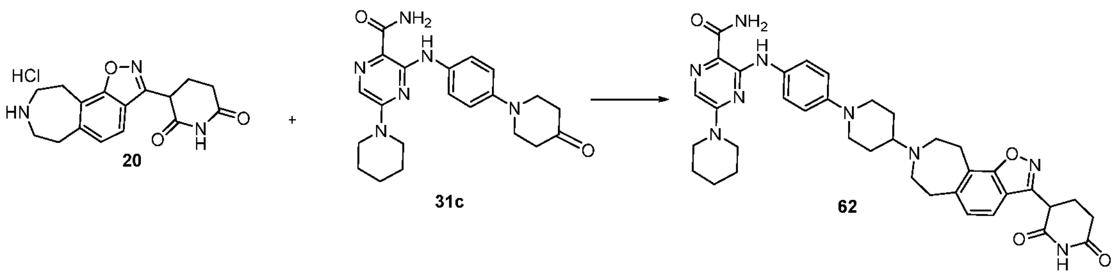

20 31c 62

Step 1: Preparation of compound **62**

**31c** (100 mg), isopropanol (1 mL), 1,2-dichloroethane (5.00 mL), and **20** (85 mg) were added to a reaction flask in sequence, and sodium cyanoborohydride (31 mg) was added. The mixture was reacted at 80 °C. After the reaction was completed, the reaction solution was mixed with silica gel and purified by column chromatography to give **62** (23 mg).
MS(ESI, [M+H]+) m/z: 678.81.
$^{1}$H NMR (500 MHz, DMSO-*d6*) δ 11.07 (d, *J* = 13.3 Hz, 2H), 7.69 (s, 1H), 7.60 (s, 1H), 7.54 (d, *J* = 8.0 Hz, 1H), 7.42 (d, *J* = 8.4 Hz, 2H), 7.26 (s, 1H), 7.19 (d, *J* = 8.1 Hz, 1H), 6.90 (d, *J* = 8.4 Hz, 2H), 4.55 (dd, *J* = 11.9, 4.9 Hz, 1H), 3.66 (q, *J* = 7.1, 5.2 Hz, 6H), 3.14 (d, *J* = 5.2 Hz, 2H), 3.08 - 2.97 (m, 2H), 2.86 - 2.70 (m, 5H), 2.68 - 2.56 (m, 5H), 2.18 (dd, *J*= 13.3, 5.8 Hz, 1H), 1.76 (d, *J*= 11.9 Hz, 2H), 1.69 - 1.48 (m, 8H).

**Examples 63** and **64:** Synthesis of Compounds **63** and **64**

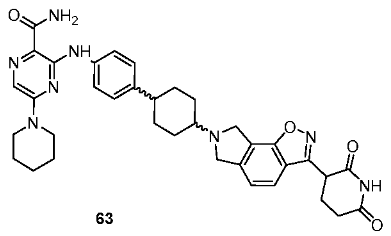

63

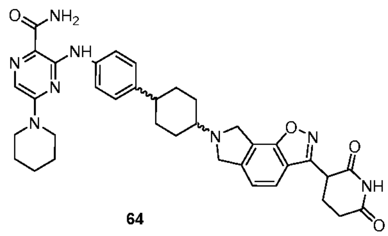

64

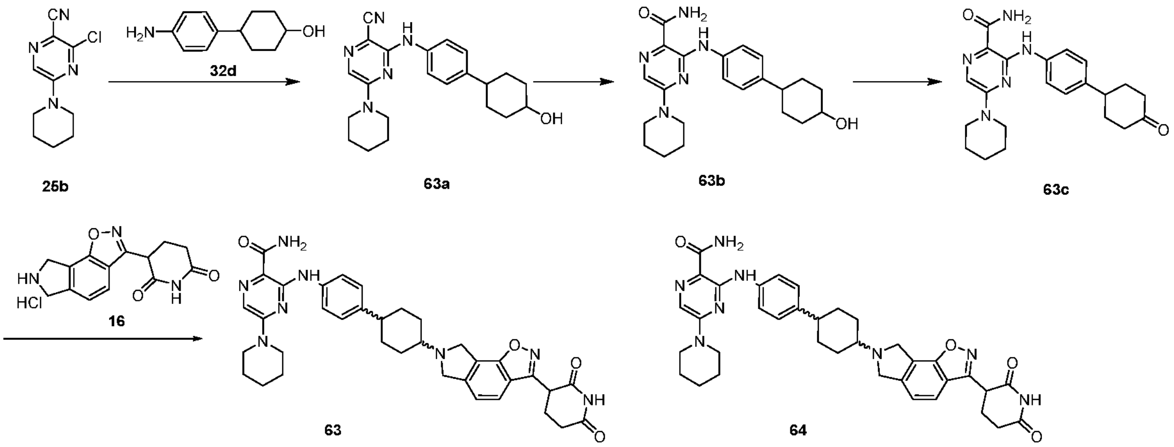


25b 63a 63b 63c 63 64

Step 1: Preparation of intermediate **63a**

Intermediate **25b** (2 g), **32d** (2.58 g), cesium carbonate (5.85 g), BINAP (0.559 g), palladium acetate (0.202 g), and 1,4-dioxane (20 mL) were added to a single-necked flask in sequence, and the mixture was heated to 100 °C and reacted under nitrogen atmosphere. After the reaction was completed, the reaction solution was cooled to room temperature, mixed with silica gel, and then purified by column chromatography to give **63a** (3.2 g).
MS(ESI, [M+H]+) m/z: 378.25.

Step 2: Preparation of intermediate **63b**

Intermediate **63a** (3.2 g), DMSO (30 mL), and cesium carbonate (2.76 g) were added to a single-necked flask in sequence. 30 wt% hydrogen peroxide (2.88 mL) was added under an ice bath. After the mixture was stirred for 5 min, the ice bath was removed, and the mixture was reacted at room temperature. After the reaction was completed, 300 mL of a saturated sodium sulfite solution was added to the residue. The color was not changed as detected using a potassium iodide starch reagent, and then 300 mL of ethyl acetate was added for extraction. 100 mL of ethyl acetate was then added for 3 times of extraction. The organic phases were combined, dried over anhydrous sodium sulfate, filtered, and concentrated by evaporation at reduced pressure to remove the solvent. The crude product was separated by silica gel column chromatography to give the target intermediate **63b** (3.3 g). MS(ESI, [M+H]+) m/z: 396.18.

Step 3: Preparation of intermediate **63c**

**63b** (3.3 g), IBX (7.01 g), and DMSO (30 mL) were added to a reaction flask in sequence, and the mixture was reacted at room temperature. After the reaction was completed, 200 mL of ethyl acetate was added, and 200 mL of a saturated sodium bicarbonate solution was added for extraction. The organic phase was dried over anhydrous sodium sulfate, filtered, and concentrated to give **63c** (770 mg).
MS(ESI, [M+H]+) m/z: 394.21.

Step 4: Preparation of compounds **63** and **64**

**63c** (0.22 g), isopropanol (1 mL), 1,2-dichloroethane (5.00 mL), and **16** (0.172 g) were added to a reaction flask in sequence, and sodium cyanoborohydride (0.105 g) was added. The mixture was reacted at room temperature. After the reaction was completed, the reaction solution was mixed with silica gel and purified by column chromatography (ratio of developing solvents: MeOH:DCM = 0:100 → 1:25) to give **63** (32 mg) and **64** (82 mg) in sequence.
**63:** MS(ESI, [M+H]+) m/z: 649.55.
$^1$H NMR (500 MHz, DMSO-*d*6) δ 11.28 (s, 1H), 11.10 (s, 1H), 8.01 - 7.60 (m, 3H), 7.52 (d, *J* = 8.4 Hz, 2H), 7.37 - 7.25 (m, 2H), 7.20 (d, *J*= 8.2 Hz, 2H), 4.60 (dd, *J*= 11.9, 5.0 Hz, 1H), 4.16 (d, *J* = 53.1 Hz, 4H), 3.68 (t, *J* = 5.4 Hz, 4H), 2.77 (td, *J* = 12.0, 5.9 Hz, 1H), 2.67 - 2.51 (m, 4H), 2.28 - 2.09 (m, 3H), 1.88 (d, *J* = 12.4 Hz, 2H), 1.73 - 1.33 (m, 10H).
**64:** MS(ESI, [M+H]+) m/z: 649.53.
$^1$H NMR (500 MHz, DMSO-*d*6) δ 11.26 (s, 1H), 11.10 (s, 1H), 7.73 (d, *J* = 8.4 Hz, 2H), 7.64 (d, *J* = 4.9 Hz, 1H), 7.48 (d, *J* = 7.9 Hz, 2H), 7.38 - 7.22 (m, 2H), 7.15 (d, *J*= 8.0 Hz, 2H), 4.61 (dd, *J* = 11.7, 4.9 Hz, 1H), 4.18 (s, 2H), 4.08 (d, *J* = 5.2 Hz, 2H), 3.73 - 3.56 (m, 4H), 2.91 - 2.70 (m, 2H), 2.60 (ddd, *J* = 31.6, 17.5, 8.7 Hz, 3H), 2.32 - 2.18 (m, 1H), 2.06 (d, *J*= 13.3 Hz, 2H), 1.87 (p, *J* = 12.2, 9.0 Hz, 2H), 1.76 - 1.43 (m, 10H).

**Examples 65** and **66:** Synthesis of Compounds **65** and **66**

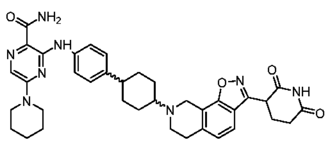

65

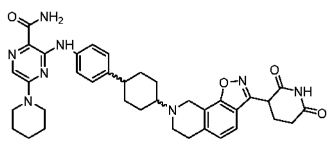

66

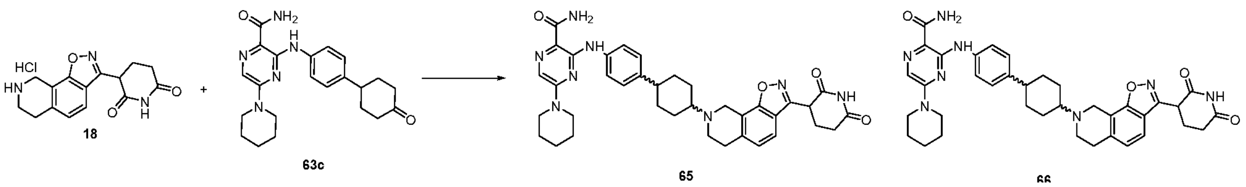

18

63c

65

66

Step 1: Preparation of compounds **65** and **66**

**63c** (150 mg), isopropanol (1 mL), 1,2-dichloroethane (5.00 mL), and **18** (123 mg) were added to a reaction flask in sequence, and sodium cyanoborohydride (71.9 mg) was added. The mixture was reacted at room temperature. After the reaction was completed, the reaction solution was mixed with silica gel and purified by column chromatography (ratio of developing solvents: MeOH:DCM = 0:100 → 1:25) to give **65** (33 mg) and **66** (29 mg) in sequence.
**65:** MS(ESI, [M+H]+) m/z: 663.34.
$^1$H NMR (500 MHz, DMSO-*d*6) δ 11.25 (s, 1H), 11.08 (s, 1H), 7.71 (d, *J* = 2.8 Hz, 1H), 7.64 (s, 1H), 7.60 (d, *J* = 8.1 Hz, 1H), 7.48 (d, *J*= 8.2 Hz, 2H), 7.29 (d, *J*= 2.8 Hz, 1H), 7.16 (d, *J* = 8.2 Hz, 3H), 4.56 (dd, *J*= 11.9, 5.0 Hz, 1H), 3.91 (s, 2H), 3.65 (t, *J* = 5.4 Hz, 4H), 2.97 (s, 2H), 2.84 (t, *J* = 5.5 Hz, 2H), 2.77 (ddd, *J* = 17.2, 12.0, 5.3 Hz, 1H), 2.70 - 2.60 (m, 2H), 2.60 - 2.53 (m, 1H), 2.49 - 2.44 (m, 1H), 2.23 - 2.09 (m, 3H), 1.87 (q, *J*= 14.1, 11.7 Hz, 2H), 1.70 - 1.50 (m, 10H).
**66:** MS(ESI, [M+H]+) m/z: 663.56.
$^1$H NMR (500 MHz, DMSO-*d*6) δ 11.28 (s, 1H), 11.09 (s, 1H), 7.73 (d, *J* = 2.8 Hz, 1H), 7.66 (s, 1H), 7.58 (d, *J* = 8.1 Hz, 1H), 7.55 - 7.49 (m, 2H), 7.31 (d, *J* = 2.8 Hz, 1H), 7.24 - 7.16 (m, 2H), 7.14 (d, *J* = 8.2 Hz, 1H), 4.56 (dd, *J* = 11.9, 5.0 Hz, 1H), 3.99 (s, 2H), 3.68 (t, *J* = 5.4 Hz, 4H), 2.94 (d, *J* = 5.7 Hz, 2H), 2.88 (t, *J*= 5.5 Hz, 2H), 2.77 (ddd, *J* =

17.2, 12.0, 5.3 Hz, 1H), 2.71 - 2.56 (m, 2H), 2.53 (s, 1H), 2.47 (dd, *J* = 12.3, 4.3 Hz, 1H), 2.19 (dq, *J* = 13.6, 4.8 Hz, 1H), 2.00 (s, 2H), 1.91 (d, *J*= 7.3 Hz, 2H), 1.72 - 1.48 (m, 10H).

**Examples 67** and **68:** Synthesis of Compounds **67** and **68**

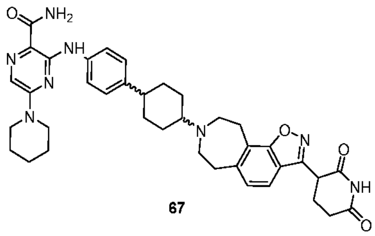

**67**

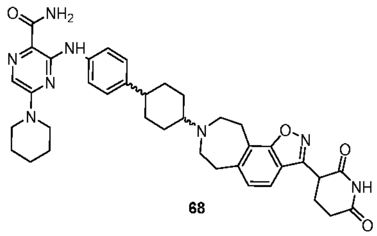

**68**

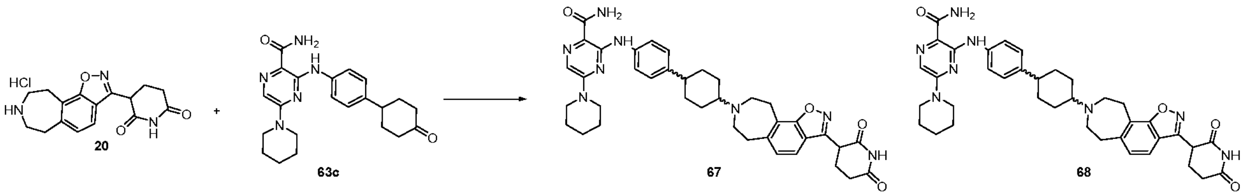

**20** **63c** **67** **68**

Step 1: Preparation of compounds **67** and **68**

**63c** (200 mg), isopropanol (1 mL), 1,2-dichloroethane (5.00 mL), and **20** (171 mg) were added to a reaction flask in sequence, and sodium cyanoborohydride (96 mg) was added. The mixture was reacted at 80 °C. After the reaction was completed, the reaction solution was mixed with silica gel and purified by column chromatography (ratio of developing solvents: MeOH:DCM = 0:100 → 1:25) to give **67** (18 mg) and **68** (42 mg) in sequence.

**67:** MS(ESI, [M+H]+) m/z: 677.52.

$^1$H NMR (500 MHz, DMSO-*d*6) δ 11.27 (s, 1H), 11.08 (s, 1H), 7.73 (d, *J* = 2.8 Hz, 1H), 7.65 (s, 1H), 7.54 (d, *J* = 8.0 Hz, 1H), 7.50 (d, *J* = 8.3 Hz, 2H), 7.31 (d, *J* = 2.8 Hz, 1H), 7.17 (dd, *J* = 15.3, 8.2 Hz, 3H), 5.76 (s, 1H), 4.55 (dd, *J* = 11.9, 5.0 Hz, 1H), 3.67 (t, *J* = 5.4 Hz, 4H), 3.19 - 3.10 (m, 2H), 3.08 - 2.98 (m, 2H), 2.76 (dtd, *J* = 20.0, 11.1, 10.1, 4.9 Hz, 4H), 2.61 (dt, *J* = 17.3, 4.3 Hz, 2H), 2.53 (d, *J* = 4.6 Hz, 1H), 2.40 (d, *J* = 3.3 Hz, 1H), 2.18 (dq, *J* = 13.5, 4.8 Hz, 1H), 1.82 (d, *J* = 11.5 Hz, 4H), 1.62 (dq, *J* = 33.7, 5.9, 5.4 Hz, 6H), 1.46 (q, *J* = 10.2 Hz, 4H).

**68:** MS(ESI, [M+H]+) m/z: 677.49.

$^1$H NMR (500 MHz, DMSO-*d*6) δ 11.27 (s, 1H), 11.08 (s, 1H), 7.73 (d, *J* = 2.8 Hz, 1H), 7.66 (s, 1H), 7.52 (dd, *J* = 8.3, 2.0 Hz, 3H), 7.31 (d, *J* = 2.9 Hz, 1H), 7.20 (d, *J* = 8.3 Hz, 2H), 7.15 (d, *J* = 8.1 Hz, 1H), 4.53 (dd, *J* = 11.9, 5.0 Hz, 1H), 3.67 (t, *J* = 5.4 Hz, 4H), 3.21 - 3.10 (m, 2H), 3.03 (d, *J* = 8.1 Hz, 2H), 2.94 - 2.68 (m, 7H), 2.60 (dt, *J* = 17.3, 4.2 Hz, 1H), 2.46 (dd, *J* = 12.2, 4.3 Hz, 1H), 2.18 (dt, *J* = 13.3, 4.6 Hz, 1H), 2.01 - 1.79 (m, 4H), 1.60 (ddt, *J* = 22.5, 16.9, 8.6 Hz, 10H).

**Example 69:** Synthesis of Compound **69**

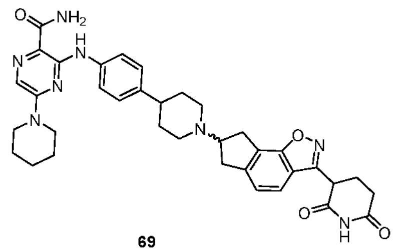

**69**

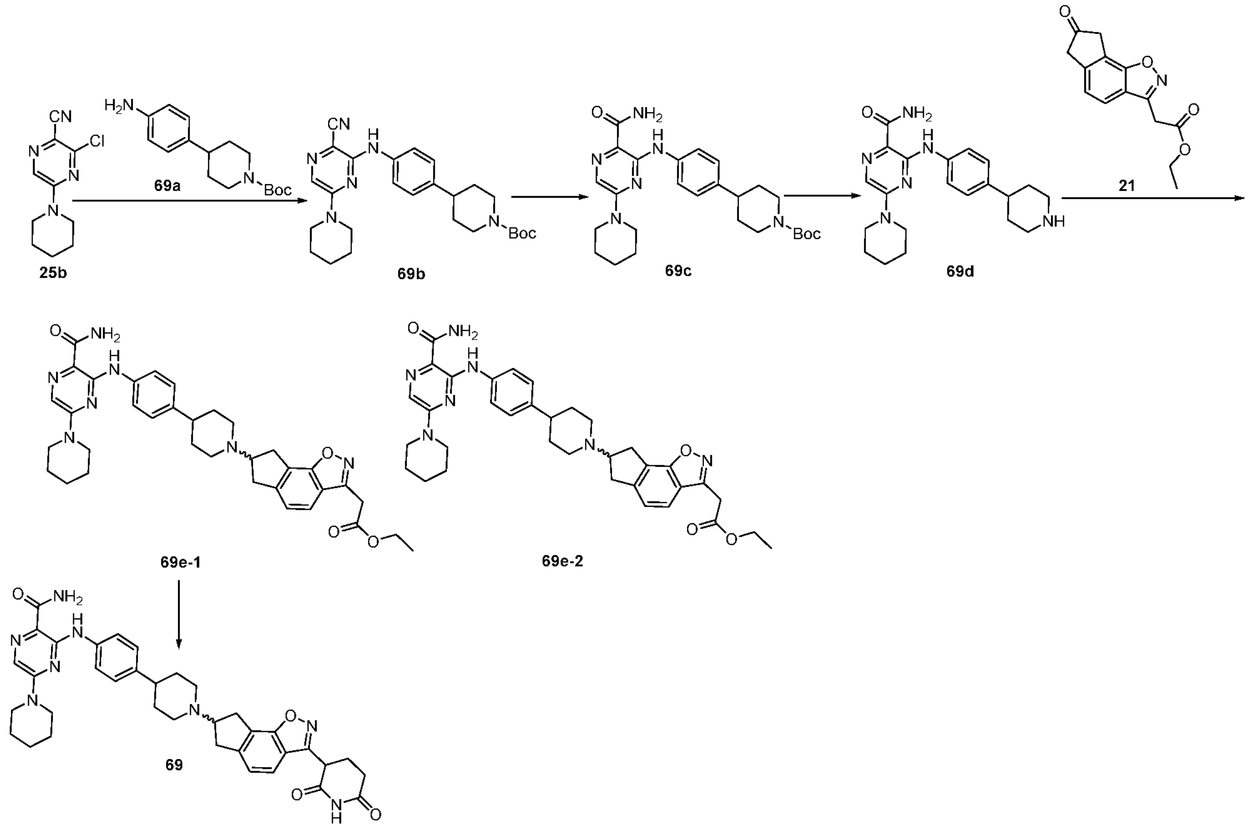


25b 69b 69c 69d

69e-1 69e-2

69

Step 1: Preparation of intermediate **69b**

Intermediate **25b** (4.77 g), intermediate **69a** (5.92 g), cesium carbonate (20.94 g), palladium acetate (0.481 g, 2.14 mmol), BINAP (1.334 g), and 1,4-dioxane (100 mL) were added to a reaction flask in sequence, and the mixture was heated at 100 °C under nitrogen atmosphere. After the reaction was completed, 200 mL of water was added to the reaction solution, and 200 mL of ethyl acetate was added for extraction twice. The organic phases were combined and washed with saturated brine once, and the extract was dried over anhydrous sodium sulfate, filtered, subjected to rotary evaporation, mixed with silica gel, and purified by column chromatography to give the target intermediate **69b** (5.53 g).
$^1$H NMR (500 MHz, DMSO-d6) δ 8.91 (s, 1H), 7.82 (s, 1H), 7.50 - 7.44 (m, 2H), 7.18 - 7.12 (m, 2H), 4.14 - 4.02 (m, 2H), 3.62 (t, J = 5.4 Hz, 4H), 2.79 (s, 2H), 2.63 (tt, J = 12.1, 3.5 Hz, 1H), 1.78 - 1.70 (m, 2H), 1.63 (q, J = 6.4 Hz, 2H), 1.54 (dt, J = 9.6, 5.4 Hz, 4H), 1.46 (dd, J = 12.5, 4.1 Hz, 2H), 1.42 (s, 9H).

Step 3: Preparation of intermediate **69c**

Intermediate **69b** (5.40 g), cesium carbonate (3.60 g), dimethyl sulfoxide (20 mL), and methanol (40 mL) were added to a reaction flask in sequence, and 30 wt% hydrogen peroxide (1.305 g, 1.305 mL, 11.51 mmol) was added under an ice bath. The mixture was stirred and reacted at room temperature. After the reaction was completed, 100 mL of a sodium thiosulfate solution was added to the reaction solution. The reaction solution was subjected to rotary evaporation, water (100 mL) was added to the residue, and 200 mL of dichloromethane was added for extraction twice. The organic phases were combined and washed with saturated brine, the extract was dried over anhydrous sodium sulfate, filtered, and subjected to rotary evaporation to dryness, thus giving the target intermediate **69c** (5.60 g).
$^1$H NMR (500 MHz, DMSO-d6) δ 11.29 (s, 1H), 7.78 - 7.71 (m, 1H), 7.66 (s, 1H), 7.52 (d, J = 8.1 Hz, 2H), 7.32 (s, 1H), 7.18 (d, J = 8.1 Hz, 2H), 4.04 (d, J = 8.0 Hz, 2H), 3.67 (t, J = 5.3 Hz, 4H), 2.79 (s, 2H), 2.66 - 2.59 (m, 1H), 1.74 (d, J = 12.8 Hz, 2H), 1.66 (q, J = 5.6 Hz, 2H), 1.62 - 1.55 (m, 4H), 1.51 - 1.44 (m, 2H), 1.42 (s, 9H).

Step 4: Preparation of intermediate **69d**

Intermediate **69c** (5.55 g) and dichloromethane (30 mL) were added to a reaction flask, and trifluoroacetic acid (35.87 g, 24.07 mL, 314.6 mmol) was added dropwise under an ice-water bath. After the dropwise addition was completed, the mixture was stirred at room temperature. After the reaction was completed, the reaction solution was subjected to

rotary evaporation to remove most trifluoroacetic acid, 100 mL of water was added to the residue, a saturated sodium carbonate solution was added until no obvious bubbles were present, and the mixture was extracted 3-4 times with 200 mL of a mixed solvent of dichloromethane and methanol (10:1, v/v). The organic phases were combined, and the extract was dried over anhydrous sodium sulfate, filtered, and subjected to rotary evaporation to dryness, thus giving the target intermediate **69d** (4.30 g).

$^1$H NMR (500 MHz, DMSO-d6) δ 11.32 (s, 1H), 7.77 - 7.73 (m, 1H), 7.68 (s, 1H), 7.56 (d, J = 8.1 Hz, 2H), 7.35 - 7.31 (m, 1H), 7.17 (d, J = 8.1 Hz, 2H), 3.68 (t, J = 5.4 Hz, 4H), 3.36 (s, 2H), 2.97 (td, J = 12.8, 2.9 Hz, 2H), 2.78 (dt, J = 24.5, 3.6 Hz, 1H), 1.92 (d, J = 13.5 Hz, 2H), 1.81 - 1.53 (m, 9H).

Step 5: Preparation of intermediates **69e-1** and **69e-2**

Intermediate **21** (167 mg), intermediate **69d** (245 mg), 1,2-dichloroethane (10 mL), isopropanol (3 mL), acetic acid (38.7 mg, 0.644 mmol), and sodium cyanoborohydride (121 mg, 1.932 mmol) were added to a reaction flask in sequence, and the mixture was reacted overnight at room temperature. After the reaction was completed, 5 mL of a saturated sodium bicarbonate solution was added to the reaction solution to neutralize acetic acid, and then 100 mL of dichloromethane and 100 mL of water were added for extraction. The organic phase was separated, and the aqueous phase was extracted twice with 50 mL of dichloromethane. The organic phases were combined, washed with saturated brine, dried over anhydrous sodium sulfate, and filtered, and the filtrate was purified by silica gel column chromatography (dichloromethane:MeOH) to give the target intermediate **69i** (211 mg). Intermediate **69i** was separated by high performance liquid chromatography to give intermediates **69e-1** (54 mg) and **69e-2** (56 mg) in sequence. The conditions of preparative chromatography were as follows:

Instrument and preparative column: YMC K-prep Lab100g high pressure preparative chromatograph was used, and the preparative column was Whelk-01 (10 μm, 30*250 mm). Mobile phase system: ethanol-dichloromethane (3:2)/n-hexane-0.1% diethylamine, isocratic elution: ethanol-dichloromethane (3:2)/n-hexane-0.1% diethylamine = 50/50.
Intermediate **69e-1:** MS(ESI, [M+H]+) m/z: 624.55.
Intermediate **69e-2:** MS(ESI, [M+H]+) m/z: 624.54.

Step 7: Preparation of compound **69**

Intermediate **69e-1** (54 mg), acrylamide (6.15 mg, 0.087 mmol), and tetrahydrofuran (5 mL) were added to a reaction flask in sequence, and the mixture was cooled to 0 °C under $N_2$ atmosphere. Potassium *tert*-butoxide (1 moL/L, 0.061 mL, 0.061 mmol) was added, and the mixture was reacted at 0 °C for 1 h. After the reaction was completed, the reaction solution was added dropwise to an icy saturated aqueous ammonium chloride solution, and 50 mL of ethyl acetate was added for extraction. The organic phase was separated, and the aqueous phase was extracted twice with 20 mL of ethyl acetate. The organic phases were combined, washed with saturated brine, dried over anhydrous sodium sulfate, and filtered, and the filtrate was purified by silica gel column chromatography (dichloromethane:MeOH = 0-4%) to give compound **69** (14 mg).

MS(ESI, [M+H]+) m/z: 649.62.

$^1$H NMR (500 MHz, DMSO-d6) δ 11.28 (s, 1H), 11.09 (s, 1H), 7.73 (s, 1H), 7.69 - 7.59 (m, 2H), 7.52 (d, J = 8.0 Hz, 2H), 7.35 - 7.24 (m, 2H), 7.19 (d, J = 8.1 Hz, 2H), 4.57 (d, J = 11.7 Hz, 1H), 3.67 (s, 4H), 3.46 - 3.38 (m, 1H), 3.29 (s, 1H), 3.21 (d, J = 16.1 Hz, 1H), 3.02 (t, J = 11.8 Hz, 4H), 2.76 (d, J = 14.0 Hz, 1H), 2.60 (d, J = 19.9 Hz, 1H), 2.16 (dd, J = 24.4, 12.4 Hz, 3H), 1.70 (dd, J = 76.6, 23.6 Hz, 11H).

**Example 70:** Synthesis of Compound **70**

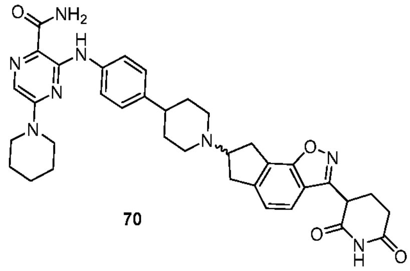

**70**

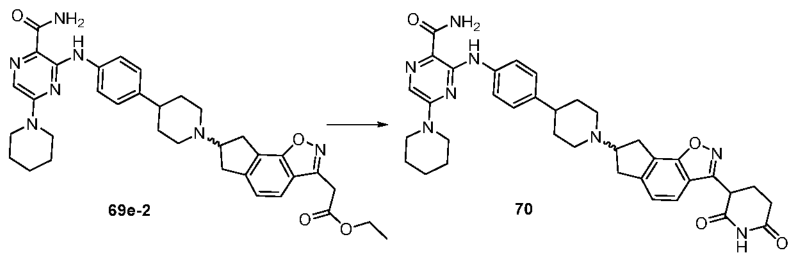

Intermediate **69e-2** (56 mg), acrylamide (6.38 mg, 0.090 mmol), and tetrahydrofuran (5 mL) were added to a reaction flask in sequence, and the mixture was cooled to 0 °C under $N_2$ atmosphere. Potassium *tert*-butoxide (1 moL/L, 0.063 mL, 0.063 mmol) was added, and the mixture was reacted at 0 °C. After the reaction was completed, as confirmed by TLC, the reaction solution was added dropwise to an icy saturated aqueous ammonium chloride solution, and 50 mL of ethyl acetate was added for extraction. The organic phase was separated, and the aqueous phase was extracted twice with 20 mL of ethyl acetate. The organic phases were combined, washed with saturated brine, dried over anhydrous sodium sulfate, and filtered, and the filtrate was purified by silica gel column chromatography (dichloromethane:MeOH = 0-4%) to give compound **70** (25 mg).

MS(ESI, [M+H]+) m/z: 649.56.

$^1$H NMR (500 MHz, DMSO-d6) δ 11.28 (s, 1H), 11.09 (s, 1H), 7.73 (d, J = 2.9 Hz, 1H), 7.66 (s, 1H), 7.63 (d, J = 8.0 Hz, 1H), 7.52 (d, J = 8.1 Hz, 2H), 7.31 (d, J = 2.9 Hz, 1H), 7.27 (d, J = 8.1 Hz, 1H), 7.19 (d, J = 8.2 Hz, 2H), 4.57 (dd, J = 11.8, 5.0 Hz, 1H), 3.67 (t, J = 5.3 Hz, 4H), 3.43 (p, J = 7.6 Hz, 1H), 3.29 (d, J = 7.7 Hz, 1H), 3.21 (dd, J = 16.3, 7.6 Hz, 1H), 3.05 - 2.97 (m, 4H), 2.77 (ddd, J = 17.2, 11.9, 5.3 Hz, 1H), 2.61 (dt, J = 17.3, 4.3 Hz, 1H), 2.48 - 2.44 (m, 1H), 2.22 - 2.10 (m, 3H), 1.81 - 1.74 (m, 2H), 1.72 - 1.53 (m, 9H).

**Example 71:** Synthesis of Compound **71**

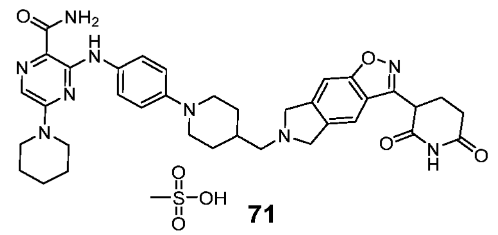

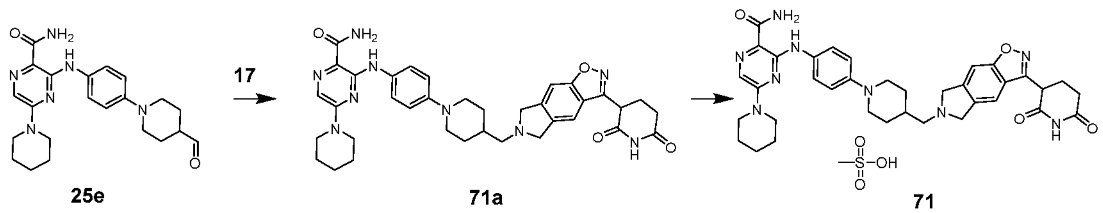


Step 1: Preparation of intermediate **71a**

Intermediate **25e** (138 mg), **17** (80 mg), sodium acetate (24 mg), and dichloroethane/isopropanol (5:1, 25 mL) were added to a reaction flask in sequence. After the mixture was stirred at room temperature for 30 min, sodium cyanoborohydride (21 mg) was added, and the mixture was reacted at room temperature. After the reaction was completed, as confirmed by TLC, 50 mL of dichloromethane and 100 mL of water were added to the reaction solution. The organic phase was separated, washed with a saturated sodium chloride solution, dried over anhydrous sodium sulfate, filtered, and concentrated. The concentrate was separated and purified by silica gel column chromatography to give compound **71a** (72 mg).

MS(ESI, [M+H]$^+$) *m/z*: 664.5.

Step 2: Preparation of compound **71**

Intermediate **71a** (72 mg), methanesulfonic acid (11 mg), and methanol (20 mL) were added to a reaction flask in sequence, and the mixture was reacted at room temperature. After the reaction was completed, as confirmed by TLC, the reaction solution was concentrated to give compound **71** (83 mg).

MS(ESI, $[M+H]^+$) *m/z*: 664.5.

1H NMR (500 MHz, DMSO-*d*6) δ 11.14 (s, 1H), 10.49 (s, 1H), 7.89 (d, *J*= 5.0 Hz, 1H), 7.86 (s, 1H), 7.69 (d, *J* = 39.8 Hz, 2H), 7.52 (s, 2H), 7.36 - 7.24 (m, 1H), 7.00 (s, 1H), 5.12 - 4.88 (m, 2H), 4.80 - 4.57 (m, 3H), 3.75 - 3.61 (m, 6H), 3.17 (s, 1H), 2.87 - 2.59 (m, 3H), 2.31 (s, 3H), 2.27 - 2.17 (m, 1H), 1.98 (s, 3H), 1.73 - 1.53 (m, 6H), 1.47 (s, 1H).

**Example 72:** Synthesis of Compound **72**

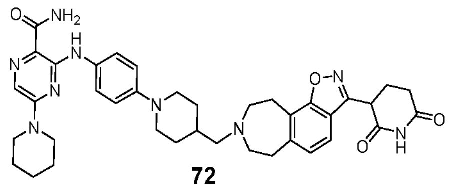

**72**

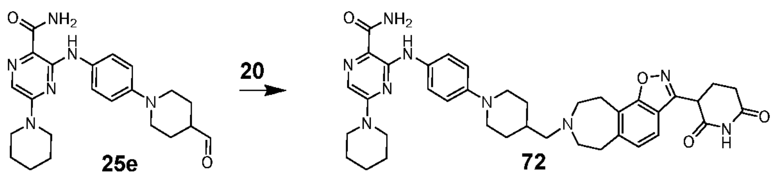


**25e** **72**

Step 1: Synthesis of compound **72**

Intermediate **25e** (102 mg), **20** (84 mg), dichloroethane/isopropanol (5:1, 25 mL), and sodium acetate (20 mg) were added to a reaction flask in sequence. After the mixture was stirred at room temperature for 30 min, sodium cyanoborohydride (31 mg) was added, and the mixture was reacted at room temperature. After the reaction was completed, as confirmed by TLC, 50 mL of dichloromethane and 100 mL of water were added to the reaction solution. The organic phase was separated, washed with a saturated sodium chloride solution, dried over anhydrous sodium sulfate, filtered, and concentrated. The concentrate was separated and purified by silica gel column chromatography to give compound **72** (65 mg).

MS(ESI, $[M+H]^+$) *m/z*: 692.4.

$^1H$ NMR (500 MHz, DMSO-*d6*) δ 11.07 (d, *J* = 10.7 Hz, 2H), 7.69 (d, *J* = 2.9 Hz, 1H), 7.60 (s, 1H), 7.54 (d, *J* = 7.9 Hz, 1H), 7.47 - 7.39 (m, 2H), 7.26 (d, *J* = 2.8 Hz, 1H), 7.18 (d, *J* = 8.1 Hz, 1H), 6.97 - 6.87 (m, 2H), 4.55 (dd, *J*= 11.9, 5.0 Hz, 1H), 3.69 - 3.58 (m, 6H), 3.20 - 3.12 (m, 2H), 3.10 - 2.98 (m, 2H), 2.77 (ddd, *J*= 17.2, 12.0, 5.3 Hz, 1H), 2.69 - 2.58 (m, 6H), 2.47 (dd, *J* = 12.2, 4.2 Hz, 1H), 2.35 (d, *J*= 7.1 Hz, 2H), 2.18 (dq, *J* = 13.7, 5.0 Hz, 1H), 1.88 - 1.80 (m, 2H), 1.65 (q, *J*= 6.2 Hz, 3H), 1.58 (q, *J* = 5.6, 5.2 Hz, 4H), 1.31 - 1.25 (m, 2H).

**Examples 73** and **74:** Synthesis of Compounds **73** and **74**

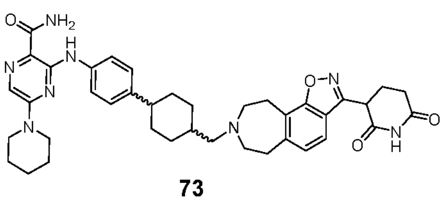 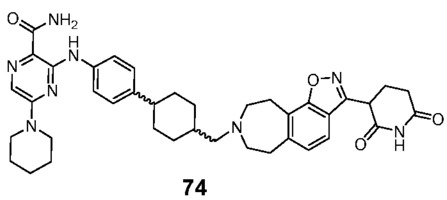

**73** **74**

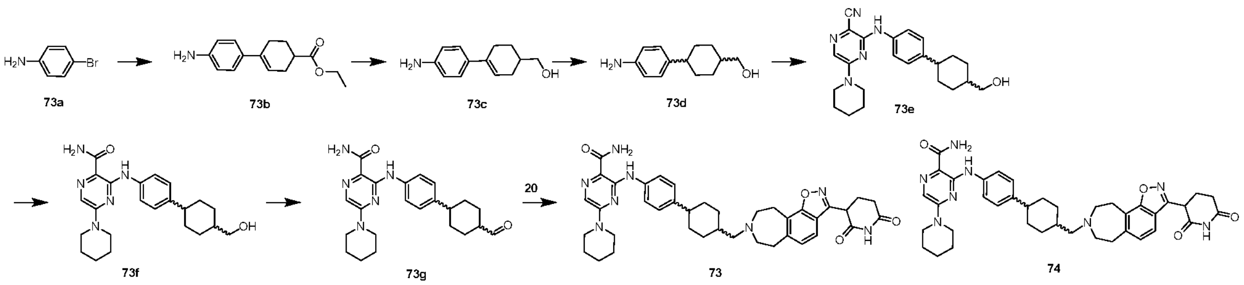


73a 73b 73c 73d 73e 73f 73g 73 74

Step 1: Preparation of intermediate **73b**

Intermediate **73a** (2.0 g), ethyl 4-(4,4,5,5-tetramethyl-1,3,2-dioxolan-2-yl)benzoate (3.26 g), potassium carbonate (3.21 g), $PdCl_2$(dppf) (0.85 g), water (5 mL), and 1,4-dioxane (25 mL) were added to a reaction flask in sequence, and the mixture was purged three times with nitrogen, heated to 100 °C, and reacted. After the reaction was completed, as confirmed by TLC, the reaction solution was cooled to room temperature, and 80 L of ethyl acetate and 140 mL of water were added to the residue. The organic phase was separated, washed with a saturated sodium chloride solution, dried over anhydrous sodium sulfate, filtered, and concentrated. The concentrate was separated and purified by silica gel column chromatography to give intermediate **73b** (2.0 g).

MS(ESI, $[M+H]^+$) *m/z*: 246.1.

1H NMR (500 MHz, DMSO-*d6*) δ 7.11 - 7.05 (m, 2H), 6.54 - 6.47 (m, 2H), 5.89 (td, *J* = 3.3, 1.5 Hz, 1H), 4.08 (qd, *J* = 7.1, 2.1 Hz, 2H), 2.58 - 2.52 (m, 1H), 2.45 - 2.23 (m, 4H), 2.08 - 2.01 (m, 1H), 1.66 (dddd, *J* = 12.8, 11.0, 9.9, 5.8 Hz, 1H), 1.19 (t, *J* = 7.1 Hz, 3H), 1.07 (s, 2H).

Step 2: Preparation of intermediate **73c**

A solution of lithium aluminum hydride in tetrahydrofuran (9.8 mL, 1 M) was added dropwise to a stirred solution of **73b** in tetrahydrofuran (20 mL) at 0 °C. After the dropwise addition was completed, the mixture was reacted at 0 °C. After the reaction was completed, as confirmed by TLC, the reaction solution was slowly added dropwise to 100 mL of ice water, and 60 mL of ethyl acetate was added. The organic phase was separated, washed with a saturated sodium chloride solution, dried over anhydrous sodium sulfate, filtered, and concentrated to give intermediate **73c** (0.68 g).

MS(ESI, $[M+H]^+$) *m/z:* 204.4.

Step 3: Preparation of intermediate **73d**

Intermediate **73c** (0.6 g), 10% palladium on carbon (0.3 g), glacial acetic acid (0.1 g), and methanol (30 mL) were added to a reaction flask in sequence, and the mixture was purged three times with hydrogen and reacted overnight. The palladium on carbon was removed by filtration under vacuum, and the residue was concentrated to give intermediate **73d** (0.62 g).

MS(ESI, $[M+H]^+$) *m/z*: 206.1.

Step 4: Preparation of intermediate **73e**

**25b** (0.4 g), **73d** (0.62 g), BINAP (0.11 g), cesium carbonate (1.75 g), palladium acetate (0.04 g), and 1,4-dioxane (30 mL) were added to a reaction flask in sequence, and the mixture was purged three times with nitrogen, heated to 100 °C, and reacted. After the reaction was completed, as confirmed by TLC, the reaction solution was cooled to room temperature, and 80 L of ethyl acetate and 140 mL of water were added to the residue. The organic phase was separated, washed with a saturated sodium chloride solution, dried over anhydrous sodium sulfate, filtered, and concentrated. The concentrate was separated and purified by silica gel column chromatography to give intermediate **73e** (0.63 g).

MS(ESI, $[M+H]^+$) *m/z*: 392.3.

1H NMR (500 MHz, DMSO-*d6*) δ 8.87 (s, 1H), 7.82 (s, 1H), 7.47 - 7.41 (m, 2H), 7.18 - 7.09 (m, 2H), 4.43 - 4.29 (m, 1H), 3.62 (t, *J*= 5.5 Hz, 4H), 3.45 (dd, *J*= 7.3, 5.4 Hz, 1H), 3.25 (t, *J*= 5.8 Hz, 1H), 1.86 - 1.77 (m, 2H), 1.76 - 1.66 (m, 2H), 1.63 (q, *J*=6.5 Hz, 3H), 1.58 - 1.49 (m, 6H), 1.46 - 1.35 (m, 1H), 1.02 (qd, *J*= 13.1, 3.7 Hz, 1H), 0.90 - 0.79 (m, 1H).

Step 5: Preparation of intermediate **73f**

Intermediate **73e** (0.63 g), cesium carbonate (0.52 g), water (5 mL), DMSO (20 mL), and methanol (5 mL) were

added to a reaction flask in sequence. The reaction solution was cooled to 0 °C, and 30 wt% hydrogen peroxide (0.54 g) was added dropwise. After the dropwise addition was completed, the mixture was allowed to react at room temperature. After the reaction was completed, as confirmed by TLC, 50 mL of water was added to the reaction solution, and then a saturated aqueous sodium sulfite solution was added to neutralize 30 wt% hydrogen peroxide. The mixture was stirred for 10 min and filtered, and the filter cake was collected and dried to give intermediate **73f** (0.55 g).

MS(ESI, $[M+H]^+$) *m/z*: 410.4.

Step 6: Preparation of intermediate **73g**

Intermediate **73f** (0.55 g), dichloromethane (25 mL), and *N,N*-diisopropylethylamine (0.46 g) were added to a reaction flask in sequence, and the mixture was cooled to 0 °C. Sulfur trioxide pyridine (0.87 g) was weighed out and dissolved in DMSO (2 mL), and the solution was added dropwise to the reaction solution. After the dropwise addition was completed, the mixture was allowed to react at room temperature. After the reaction was completed, as confirmed by TLC, ethyl acetate (80 mL) and water (120 mL) were added to the reaction solution to separate the organic phase of the filtrate. The organic phase was separated, washed with a saturated sodium chloride solution, dried over anhydrous sodium sulfate, filtered, and concentrated. The concentrate was separated and purified by silica gel column chromatography to give intermediate **73g** (0.38 g).

MS(ESI, $[M+H]^+$) *m/z*: 408.3.

Step 7: Preparation of compounds **73** and **74**

Intermediate **73g** (250 mg), **20** (206 mg), dichloroethane/isopropanol (5:1, 25 mL), and sodium acetate (50 mg) were added to a reaction flask in sequence. After the mixture was stirred at room temperature for 30 min, sodium cyanoborohydride (77 mg) was added, and the mixture was reacted at room temperature. After the reaction was completed, as confirmed by TLC, 50 mL of dichloromethane and 100 mL of water were added to the reaction solution. The organic phase was separated, washed with a saturated sodium chloride solution, dried over anhydrous sodium sulfate, filtered, and concentrated. The concentrate was separated and purified by silica gel column chromatography to give a mixture of compounds 73 and 74, which was subjected to preparative resolution to give compound **73** (15 mg) and compound **74** (20 mg) in sequence.

The conditions of preparative chromatography were as follows: instrument and preparative column: YMC high pressure preparative chromatograph was used, and the preparative column was Ulitimate Polar RP. Mobile phase system: acetonitrile/10 nM aqueous ammonium acetate solution; isocratic elution: acetonitrile/10 nM aqueous ammonium acetate solution = 45/55.

Compound 73:

MS(ESI, $[M+H]^+$) *m/z*: 691.5.

1H NMR (500 MHz, DMSO-*d*6) δ 11.27 (s, 1H), 11.08 (s, 1H), 7.69 (d, *J* = 36.5 Hz, 2H), 7.53 (dd, *J* = 13.3, 8.0 Hz, 3H), 7.31 (s, 1H), 7.18 (d, *J*= 8.0 Hz, 3H), 4.55 (dd, *J*= 11.9, 50 Hz, 1H), 3.68 (t, *J*= 5.3 Hz, 4H), 3.10 (dt, *J* = 56.7, 4.7 Hz, 4H), 2.77 (ddd, *J* = 17.1, 12.2, 5.4 Hz, 1H), 2.63 (dd, *J* = 18.8, 7.7 Hz, 5H), 2.43 (d, *J* = 12.4 Hz, 2H), 2.30 (d, *J* = 6.8 Hz, 2H), 2.22 - 2.12 (m, 1H), 1.88 (dd, *J* = 51.3, 12.7 Hz, 4H), 1.72 - 1.63 (m, 2H), 1.65 - 1.52 (m, 5H), 1.46 (q, *J* = 12.6 Hz, 2H), 1.03 (q, *J* = 12.6 Hz, 2H).

Compound 74:

MS(ESI, $[M+H]^+$) *m/z*: 691.5.

1H NMR (500 MHz, DMSO-d6) δ 11.29 (s, 1H), 11.09 (s, 1H), 7.73 (d, *J* = 2.9 Hz, 1H), 7.66 (s, 1H), 7.53 (t, *J* = 8.5 Hz, 3H), 7.31 (d, *J* = 2.9 Hz, 1H), 7.20 (dd, *J* = 17.3, 8.1 Hz, 3H), 4.55 (dd, *J* = 11.9, 5.0 Hz, 1H), 3.68 (t, *J* = 5.3 Hz, 4H), 3.11 (dt, *J* = 56.6, 5.0 Hz, 4H), 2.77 (ddd, *J* = 17.1, 12.0, 5.3 Hz, 1H), 2.72 - 2.56 (m, 5H), 2.52 (s, 2H), 2.47 (dd, *J* = 12.2, 4.3 Hz, 2H), 2.18 (dt, *J* = 13.0, 4.9 Hz, 1H), 1.96 (s, 1H), 1.72 (d, *J* = 7.4 Hz, 2H), 1.66 (dt, *J* = 11.1, 5.6 Hz, 4H), 1.63 - 1.54 (m, 8H).

**Example 75:** Synthesis of Compound **75**

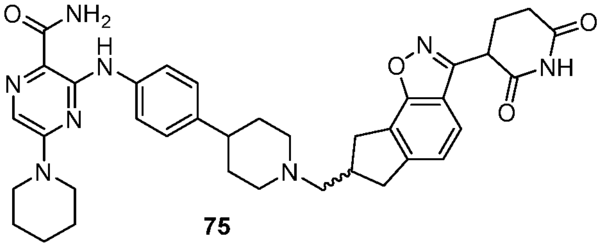

75

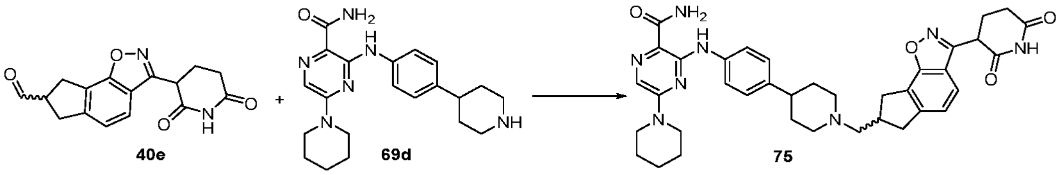

40e 69d 75

Intermediate **40e** (80 mg), 1,2-dichloroethane (6 mL), isopropanol (2 mL), intermediate **69d** (101 mg), acetic acid (8.00 mg, 0.133 mmol), and sodium cyanoborohydride (33.5 mg, 0.533 mmol) were added to a reaction flask in sequence, and the mixture was stirred and reacted at room temperature. After the reaction was completed, 2 mL of a saturated sodium bicarbonate solution was added to the reaction solution to neutralize acetic acid, and then 50 mL of dichloromethane and 100 mL of water were added for extraction. The organic phase was separated, and the aqueous phase was extracted twice with 20 mL of dichloromethane. The organic phases were combined, washed with saturated brine, dried over anhydrous sodium sulfate, and filtered, and the filtrate was purified by silica gel column chromatography (dichloromethane:MeOH = 0-5%) to give compound **75** (40 mg).

MS(ESI, [M+H]+) m/z: 663.4.

$^1$H NMR (500 MHz, DMSO-d6) δ 11.29 (s, 1H), 11.09 (s, 1H), 7.74 (d, J = 2.9 Hz, 1H), 7.66 (s, 1H), 7.62 (d, J = 8.0 Hz, 1H), 7.53 (d, J = 8.1 Hz, 2H), 7.32 (d, J = 2.9 Hz, 1H), 7.27 (d, J = 8.1 Hz, 1H), 7.20 (d, J = 8.1 Hz, 2H), 4.57 (dd, J = 11.8, 5.0 Hz, 1H), 3.68 (t, J = 5.2 Hz, 4H), 3.20 (td, J = 17.8, 15.2, 7.4 Hz, 2H), 3.02 (s, 2H), 2.97 - 2.68 (m, 5H), 2.60 (dd, J = 17.6, 4.4 Hz, 1H), 2.46 (d, J = 4.3 Hz, 1H), 2.37 (s, 2H), 2.19 (dq, J = 13.5, 4.5 Hz, 1H), 2.13 - 1.94 (m, 2H), 1.85 - 1.71 (m, 3H), 1.66 (q, J = 6.0 Hz, 3H), 1.60 (q, J = 5.5, 5.1 Hz, 4H).

**Example 76:** Synthesis of Compound **76**

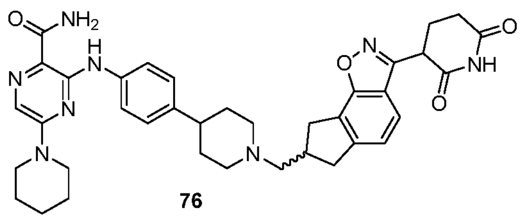

76

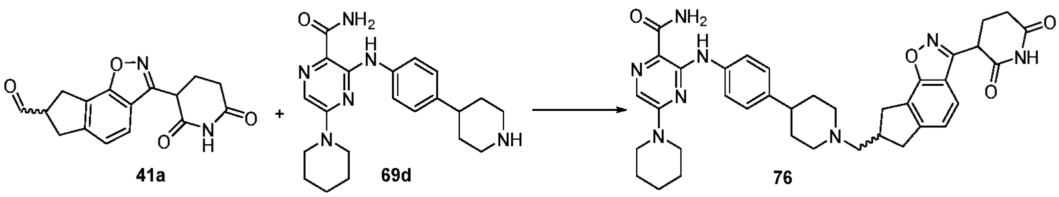

41a 69d 76

Intermediate **41a** (80 mg), 1,2-dichloroethane (6 mL), isopropanol (2 mL), intermediate **69d** (101 mg), acetic acid (8.00 mg, 0.133 mmol), and sodium cyanoborohydride (33.5 mg, 0.533 mmol) were added to a reaction flask in sequence, and the mixture was stirred and reacted at room temperature. After the reaction was completed, 2 mL of a saturated sodium bicarbonate solution was added to the reaction solution to neutralize acetic acid, and then 50 mL of dichloromethane and 100 mL of water were added for extraction. The organic phase was separated, and the aqueous phase was extracted twice with 20 mL of dichloromethane. The organic phases were combined, washed with saturated brine, dried over anhydrous sodium sulfate, and filtered, and the filtrate was purified by silica gel column chromatography

(dichloromethane:MeOH = 0-5%) to give compound 76 (62 mg).

MS(ESI, [M+H]+) m/z: 663.4.

$^1$H NMR (500 MHz, DMSO-d6) δ 11.30 (s, 1H), 11.09 (s, 1H), 7.74 (d, J = 2.9 Hz, 1H), 7.66 (s, 1H), 7.62 (d, J = 8.0 Hz, 1H), 7.54 (d, J = 8.1 Hz, 2H), 7.32 (d, J = 2.9 Hz, 1H), 7.28 (d, J = 8.1 Hz, 1H), 7.21 (d, J = 8.1 Hz, 2H), 4.57 (ddd, J = 11.9, 5.0, 1.5 Hz, 1H), 3.68 (t, J = 5.4 Hz, 4H), 3.21 (dd, J = 26.7, 12.2 Hz, 2H), 3.05 (s, 2H), 2.98 - 2.74 (m, 5H), 2.64 - 2.59 (m, 1H), 2.49 - 2.45 (m, 1H), 2.19 (dp, J = 13.3, 4.4 Hz, 3H), 2.14 - 1.88 (m, 2H), 1.78 (s, 3H), 1.66 (q, J = 5.9, 5.5 Hz, 3H), 1.59 (dq, J = 8.4, 5.4, 4.9 Hz, 4H).

**Example 77:** Synthesis of Compound **77**

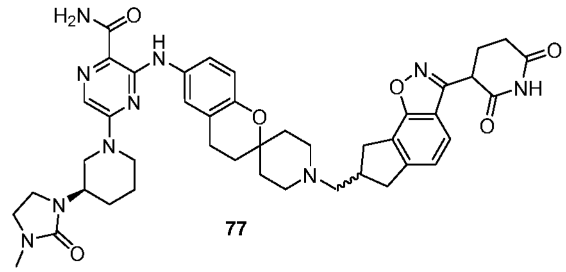

**77**

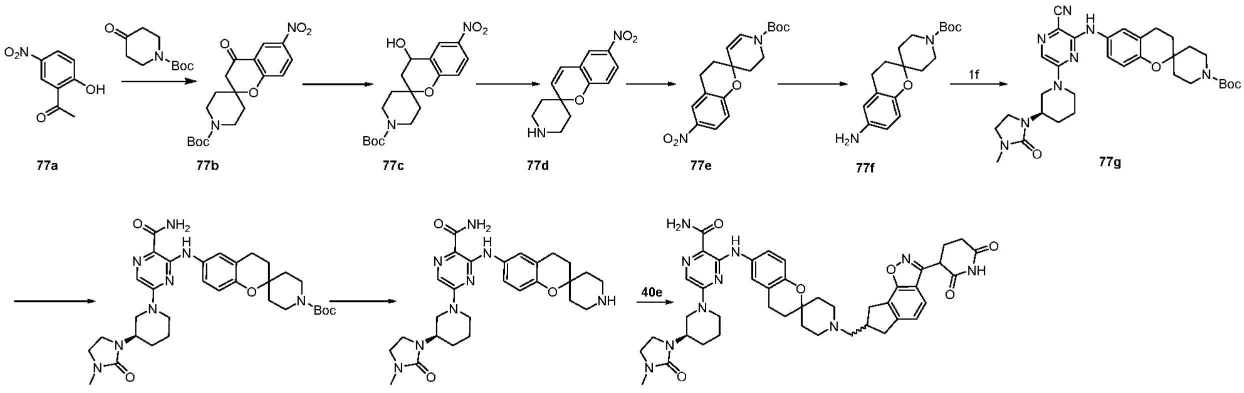


77a 77b 77c 77d 77e 77f 77g

77h 77i 77

Step 1: Preparation of intermediate **77b**

**77a** (10 g), *tert*-butyl 4-oxopiperidine-1-carboxylate (11.00 g), pyrrolidine (1.178 g), and toluene (180 mL) were added to a reaction flask in sequence, and the mixture was reacted at 120 °C. After the reaction was completed, a mixed solvent of 500 mL of ethyl acetate and 300 mL of water was poured into the reaction solution. The organic phase was separated, dried over anhydrous sodium sulfate, filtered, and concentrated by evaporation at reduced pressure to remove the solvent, and the residue was purified by silica gel column chromatography to give **77b** (16.57 g).

$^1$H NMR (500 MHz, DMSO) δ 8.47 (d, *J* = 2.9 Hz, 1H), 8.42 (dd, *J* = 9.0, 2.9 Hz, 1H), 7.34 (d, *J* = 9.1 Hz, 1H), 3.74 (d, *J*= 13.4 Hz, 2H), 3.13 (s, 2H), 3.01 (s, 2H), 1.91 (dq, *J* = 14.8, 3.0 Hz, 2H), 1.70 (ddd, *J*= 13.9, 11.6, 4.7 Hz, 2H), 1.40 (s, 9H).

Step 2: Preparation of intermediate **77c**

**77b** (16 g), THF (80 mL), and sodium borohydride (3.34 g, 88 mmol) were added to a reaction flask in sequence under nitrogen atmosphere, and the mixture was reacted at room temperature. After the reaction was completed, the reaction solution was poured into a mixed solution of water (200 mL) and ethyl acetate (300 mL) containing crushed ice. The organic phase was separated, dried over anhydrous sodium sulfate, filtered, and concentrated to give **77c** (19.28 g).

$^1$H NMR (500 MHz, DMSO) δ 8.34 (dd, *J* = 3.0, 0.9 Hz, 1H), 8.05 (dd, *J* = 9.1, 2.9 Hz, 1H), 7.01 (d, *J* = 9.0 Hz, 1H), 5.83 (d, *J* = 6.0 Hz, 1H), 4.79 (dt, *J* = 9.5, 5.9 Hz, 1H), 3.70 (dd, *J* = 25.1, 13.6 Hz, 2H), 3.33 (s, 1H), 3.08 (s, 1H), 2.22 (dd, *J* = 13.6, 6.1 Hz, 1H), 1.84 - 1.68 (m, 4H), 1.61 (ddd, *J* = 13.7, 11.1, 4.6 Hz, 1H), 1.41 (s, 9H). Step 3: Preparation of intermediate **77d**

**77c** (5 g), trifluoroacetic acid (40 mL), and triethylsilane (18.46 g, 25.4 mL, 159 mmol) were added to a reaction flask in sequence, and the mixture was heated to 70 °C and reacted. After the reaction was completed, the reaction solution was concentrated by evaporation at reduced pressure to remove the solvent, 200 mL of dichloromethane and 200 mL of water were added to the residue, and the pH was adjusted to 11-12 with sodium hydroxide. The organic phase was separated, dried over anhydrous sodium sulfate, filtered, and concentrated by evaporation at reduced pressure to remove the solvent, and the residue was purified by silica gel column chromatography to give **77d** (3.18 g).
MS(ESI, $[M+H]^+$) m/z: 247.1.

Step 4: Preparation of intermediate **77e**

**77d** (3 g), dichloromethane (100 mL), triethylamine (5.71 mL), and Boc anhydride (3.29 g) were added to a reaction flask, and the mixture was reacted at room temperature. After the reaction was completed, the reaction solution was mixed with silica gel and purified by column chromatography to give **77e** (4.25 g).
MS(ESI, [M+H]+) m/z: 347.2.

Step 5: Preparation of intermediate **77f**

**77e** (4.25 g), 10% palladium on carbon (500 mg), and methanol (20 mL) were added to a reaction flask in sequence, and the mixture was purged with hydrogen and reacted overnight at room temperature. After the reaction was completed, the reaction solution was filtered under vacuum, and the filtrate was concentrated to give **77f** (3.02 g).
MS(ESI, $[M+H]^+$) m/z: 319.3.

Step 6: Preparation of intermediate **77g**

Intermediate **1f** (1 g), **77f** (1.191 g), cesium carbonate (3.05 g), BINAP (0.194 g), palladium acetate (0.070 g), and 1,4-dioxane (20 mL) were added to a single-necked flask in sequence, and the mixture was heated to 100 °C and reacted under nitrogen atmosphere. After the reaction was completed, the reaction solution was cooled to room temperature, mixed with silica gel, and then purified by column chromatography to give **77g** (1.6 g).
$^1$H NMR (500 MHz, DMSO-*d6*) δ 8.80 (s, 1H), 7.77 (s, 1H), 7.30 (d, *J* = 2.6 Hz, 1H), 7.16 (dd, *J* = 8.7, 2.6 Hz, 1H), 6.70 (d, *J* = 8.8 Hz, 1H), 4.36 - 4.16 (m, 2H), 3.70 (d, *J* = 12.8 Hz, 2H), 3.60 - 3.50 (m, 1H), 3.25 (dddd, *J* = 23.3, 21.0, 11.5, 6.5 Hz, 4H), 3.17 - 2.79 (m, 4H), 2.67 (s, 5H), 1.82 - 1.63 (m, 7H), 1.57 - 1.46 (m, 3H), 1.41 (s, 9H).

Step 7: Preparation of intermediate **77h**

Intermediate **77g** (1.6 g), DMSO (20 mL), and cesium carbonate (1.03 g) were added to a single-necked flask in sequence. 30 wt% hydrogen peroxide (0.9 mL) was added under an ice bath. After the mixture was stirred for 5 min, the ice bath was removed, and the mixture was reacted at room temperature. After the reaction was completed, 300 mL of a saturated sodium sulfite solution was added to the residue. The color was not changed as detected using a potassium iodide starch reagent, and then 300 mL of ethyl acetate was added for extraction. 100 mL of ethyl acetate was then added for 3 times of extraction. The organic phases were combined, dried over anhydrous sodium sulfate, filtered, and concentrated by evaporation at reduced pressure to remove the solvent. The crude product was separated by silica gel column chromatography to give the target intermediate 77h (1.73 g).
MS(ESI, [M+H]+) m/z: 621.55.

Step 8: Preparation of intermediate **77i**

**77h** (1.7 g), dichloromethane (20 mL), and TFA (5 mL) were added to a single-necked flask in sequence, and the mixture was reacted at room temperature. After the reaction was completed, the system was added to 200 mL of a saturated sodium bicarbonate solution, and then 200 mL of dichloromethane was added for extraction. The organic phase was dried over anhydrous sodium sulfate, filtered, and concentrated by evaporation at reduced pressure to remove the solvent, thus giving **77i** (1.21 g).
MS(ESI, [M+H]+) m/z: 521.49.

Step 9: Preparation of compound **77**

**40e** (60 mg), isopropanol (1 mL), 1,2-dichloroethane (5.00 mL), and **77i** (104 mg) were added to a reaction flask in sequence, and sodium cyanoborohydride (37 mg) was added. The mixture was reacted at room temperature. After the reaction was completed, the reaction solution was mixed with silica gel and purified by column chromatography

to give 77 (23 mg).

MS(ESI, [M+H]+) m/z: 803.64.

$^1$H NMR (500 MHz, DMSO-$d_6$) δ 11.08 (s, 1H), 11.04 (s, 1H), 7.73 (s, 1H), 7.61 (d, *J* = 10.4 Hz, 2H), 7.48 (s, 1H), 7.37 - 7.22 (m, 2H), 7.10 (d, *J* = 8.5 Hz, 1H), 6.69 (d, *J*= 8.6 Hz, 1H), 4.56 (dd, *J* = 11.5, 50 Hz, 1H), 4.31 (dd, *J* = 56.0, 12.6 Hz, 2H), 3.61 (dt, *J* = 11.3, 6.6 Hz, 1H), 3.35 (s, 2H), 3.30 - 3.13 (m, 4H), 2.93 (ddd, *J* = 53.2, 41.3, 14.0 Hz, 5H), 2.79 - 2.55 (m, 9H), 2.45 (d, *J* = 4.0 Hz, 1H), 2.37 (s, 4H), 2.18 (d, *J*= 13.8 Hz, 1H), 1.90 - 1.53 (m, 10H).

**Example 78:** Synthesis of Compound **78**

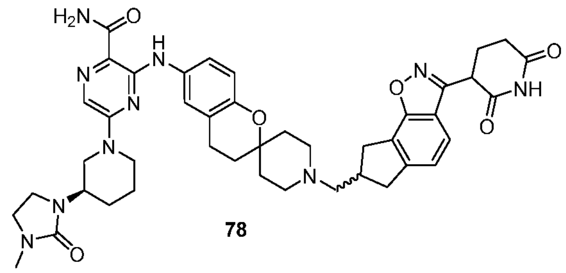

**78**

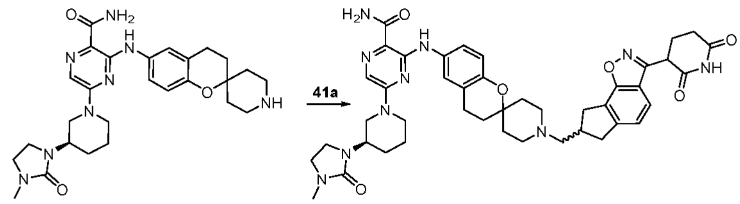


**77i** **78**

Step 1: Preparation of compound **78**

**41a** (60 mg), isopropanol (1 mL), 1,2-dichloroethane (5.00 mL), and **77i** (104 mg) were added to a reaction flask in sequence, and sodium cyanoborohydride (37 mg) was added. The mixture was reacted at room temperature. After the reaction was completed, the reaction solution was mixed with silica gel and purified by column chromatography to give **78** (10 mg).

MS(ESI, [M+H]+) m/z: 803.69.

$^1$H NMR (500 MHz, DMSO-$d_6$) δ 11.08 (s, 1H), 11.04 (s, 1H), 7.73 (s, 1H), 7.61 (d, *J* = 10.4 Hz, 2H), 7.48 (s, 1H), 7.37 - 7.22 (m, 2H), 7.10 (d, *J*= 8.5 Hz, 1H), 6.69 (d, *J*= 8.6 Hz, 1H), 4.56 (dd, *J* = 11.5, 50 Hz, 1H), 4.31 (dd, *J* = 56.0, 12.6 Hz, 2H), 3.61 (dt, *J* = 11.3, 6.6 Hz, 1H), 3.35 (s, 2H), 3.30 - 3.13 (m, 4H), 2.93 (ddd, *J* = 53.2, 41.3, 14.0 Hz, 5H), 2.79 - 2.55 (m, 9H), 2.45 (d, *J* = 4.0 Hz, 1H), 2.37 (s, 4H), 2.18 (d, *J* = 13.8 Hz, 1H), 1.90 - 1.53 (m, 10H).

**Example 79:** Synthesis of Compound **79**

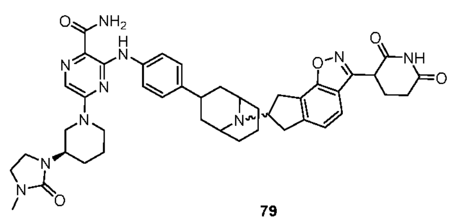

**79**

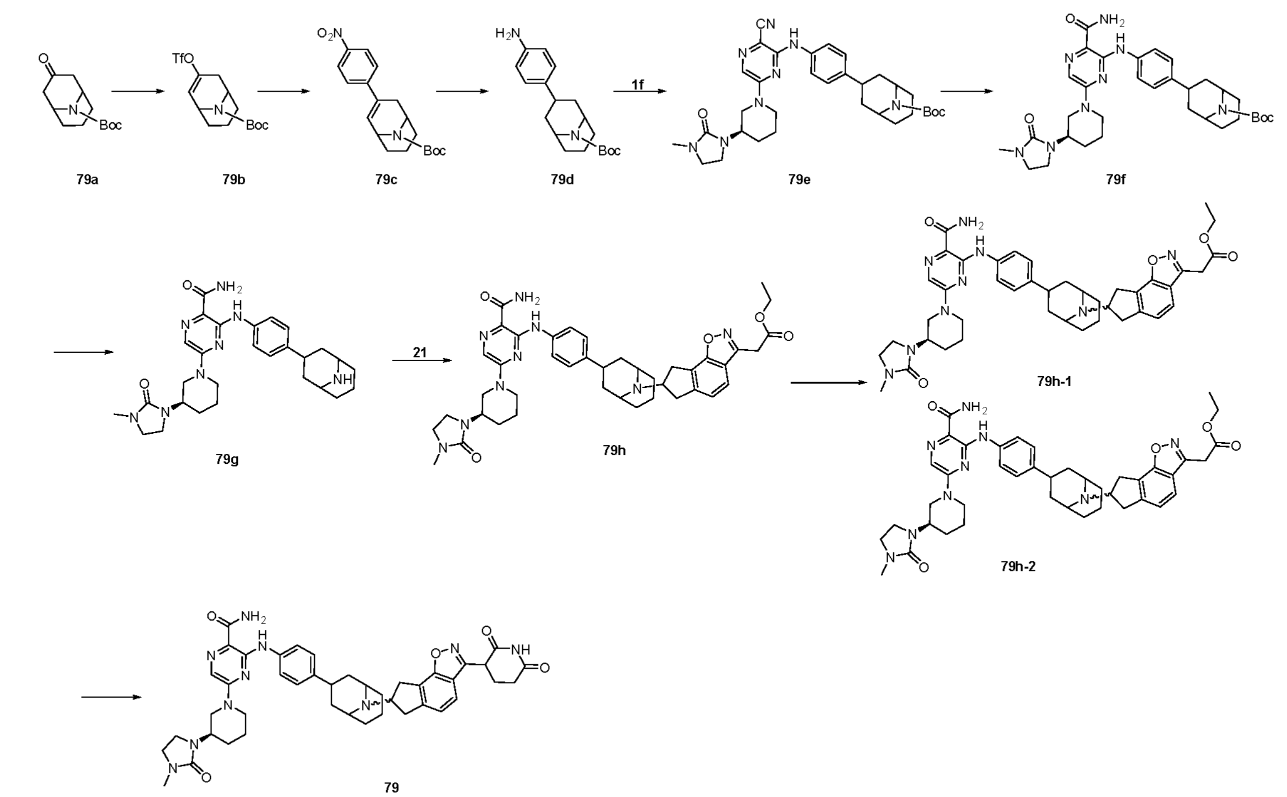


79a 79b 79c 79d 79e 79f 79g 79h 79h-1 79h-2 79

Step 1: Preparation of intermediate **79b**

Lithium bis(trimethylsilyl)amide (1 M, 16.3 mL) was slowly added dropwise to a solution of 79a (3 g) in THF (30 mL) at -78 °C under nitrogen atmosphere. After the dropwise addition was completed, the mixture was reacted at -78 °C for 1 h. A solution of N-phenylbis(trifluoromethanesulfonyl)imide (5.37 g) in THF (30 mL) was slowly added to the reaction solution. After the addition was completed, the mixture was warmed to room temperature and reacted. After the reaction was completed, 200 mL of petroleum ether was added to the system, and saturated brine was added for extraction. The organic phase was dried over anhydrous sodium sulfate, filtered, and concentrated to give **79b** (9 g).

$^1$H NMR (500 MHz, DMSO-d6) δ 6.03 (d, *J* = 5.9 Hz, 1H), 4.71 (d, *J* = 26.5 Hz, 1H), 4.41 (d, *J* = 21.2 Hz, 1H), 2.80 - 2.67 (m, 1H), 2.23 (d, *J*= 18.0 Hz, 1H), 1.72 - 1.46 (m, 6H), 1.39 (s, 9H).

Step 2: Preparation of intermediate **79c**

4-nitrophenylboronic acid (2.82 g), **79b** (7 g), potassium carbonate (3.91 g), Pd(dppf)$_2$Cl$_2$ (0.7 g), 1,4-dioxane (50 mL), and water (10 mL) were added to a reaction flask in sequence, and the mixture was purged several times with nitrogen, heated to 90 °C, and reacted. After the reaction was completed, about 200 mL of methyl tert-butyl ether and 200 mL of saturated brine were added for extraction. The organic phase was dried over anhydrous sodium sulfate, filtered, and concentrated to give a crude product, and the crude product was slurried with petroleum ether and filtered under vacuum to give **79c** (4 g).

$^1$H NMR (500 MHz, DMSO-d6) δ 8.29 - 8.09 (m, 2H), 7.82 - 7.61 (m, 2H), 6.50 (dd, *J* = 11.0, 5.7 Hz, 1H), 4.84 - 4.59 (m, 1H), 4.46 (dd, *J* = 21.3, 7.3 Hz, 1H), 2.84 (ddt, *J* = 17.8, 7.5, 1.7 Hz, 1H), 2.42 (d, *J* = 17.8 Hz, 1H), 1.77 - 1.54 (m, 5H), 1.49 (dd, *J*= 6.7, 3.2 Hz, 1H), 1.42 (d, *J*= 1.6 Hz, 9H).

Step 3: Preparation of intermediate **79d**

79c (4 g), 10% palladium on carbon (2 g), and methanol (30 mL) were added to a reaction flask in sequence, and the mixture was purged with hydrogen and reacted overnight at room temperature. After the reaction was completed, the reaction solution was filtered under vacuum, and the filtrate was concentrated to give **79d** (2.3 g). $^1$H NMR (500 MHz, DMSO-d6) δ 6.96 - 6.87 (m, 2H), 6.53 - 6.37 (m, 2H), 5.05 (s, 2H), 4.37 - 4.25 (m, 2H), 2.16 (tt, *J* = 13.1, 5.3 Hz, 1H), 2.01 (dttt, *J*= 13.3, 7.8, 5.2, 3.2 Hz, 3H), 1.56 - 1.34 (m, 16H).

Step 4: Preparation of intermediate **79e**

Intermediate **1f** (2.3 g), **79d** (2.2 g), cesium carbonate (6.8 g), BINAP (0.4 g), palladium acetate (0.15 g), and 1,4-dioxane (20 mL) were added to a single-necked flask in sequence, and the mixture was heated to 100 °C and reacted under nitrogen atmosphere. After the reaction was completed, the reaction solution was cooled to room temperature, mixed with silica gel, and then purified by column chromatography to give **79e** (2.58 g).
MS(ESI, [M+H]+) m/z: 601.5.

Step 5: Preparation of intermediate **79f**

Intermediate **79e** (3.2 g), DMSO (30 mL), and cesium carbonate (1.73 g) were added to a single-necked flask in sequence. 30 wt% hydrogen peroxide (2 mL) was added under an ice bath. After the mixture was stirred for 5 min, the ice bath was removed, and the mixture was reacted at room temperature. After the reaction was completed, 300 mL of a saturated sodium sulfite solution was added to the residue. The color was not changed as detected using a potassium iodide starch reagent, and then 300 mL of ethyl acetate was added for extraction. 100 mL of ethyl acetate was then added for 3 times of extraction. The organic phases were combined, dried over anhydrous sodium sulfate, filtered, and concentrated by evaporation at reduced pressure to remove the solvent. The crude product was separated by silica gel column chromatography to give the target intermediate **79f** (4.3 g).
MS(ESI, [M+H]+) m/z: 619.44.

Step 6: Preparation of intermediate **79g**

**79f** (3.5 g), dichloromethane (20 mL), and TFA (5 mL) were added to a single-necked flask in sequence, and the mixture was reacted at room temperature. After the reaction was completed, the system was added to 200 mL of a saturated sodium bicarbonate solution, and then 200 mL of dichloromethane was added for extraction. The organic phase was dried over anhydrous sodium sulfate, filtered, and concentrated by evaporation at reduced pressure to remove the solvent, thus giving **79g** (2.5 g).
MS(ESI, [M+H]+) m/z: 519.39.

Step 7: Preparation of compounds **79h-1** and **79h-2**

Intermediate **21** (300 mg), intermediate **79g** (600 mg), 1,2-dichloroethane (10 mL), isopropanol (3 mL), and sodium cyanoborohydride (291 mg) were added to a reaction flask in sequence, and the mixture was reacted overnight at room temperature. After the reaction was completed, 5 mL of a saturated sodium bicarbonate solution was added to the reaction solution to neutralize acetic acid, and then 100 mL of dichloromethane and 100 mL of water were added for extraction. The organic phase was separated, and the aqueous phase was extracted twice with 50 mL of dichloromethane. The organic phases were combined, washed with saturated brine, dried over anhydrous sodium sulfate, and concentrated, and the residue was purified by silica gel column chromatography to give **79h. 79h** was separated by high performance liquid chromatography to give intermediates **79h-1** (163 mg) and **79h-2** (155 mg) in sequence.
The conditions of preparative chromatography were as follows: instrument and preparative column: YMC high pressure preparative chromatograph was used, and the preparative column was REFLECTI-Cellulose-SC. Mobile phase system: ethanol-dichloromethane (1:7)/n-hexane-0.1% diethylamine, isocratic elution: ethanol-dichloromethane (1:7)/n-hexane-0.1% diethylamine = 50/50.
**79h-1, 79h-2:** MS(ESI, $[M+H]^+$) m/z: 762.64.

Step 8: Preparation of compound **79**

Intermediate **79h-1** (100 mg), acrylamide (7.46 mg) and tetrahydrofuran (5 mL) were added to a reaction flask in sequence, and the mixture was cooled to 0 °C under $N_2$ atmosphere. Potassium tert-butoxide (1 M, 0.079 mL) was added, and the mixture was reacted at 0 °C for 1 h. After the reaction was completed, the reaction solution was added dropwise to an icy saturated aqueous ammonium chloride solution, and 50 mL of ethyl acetate was added for extraction. The organic phase was dried over anhydrous sodium sulfate, filtered, and concentrated, and the residue was purified by silica gel column chromatography to give compound **79** (50 mg).
MS(ESI, [M+H]+) m/z: 787.50.
$^1$H NMR (500 MHz, DMSO-*d*6) δ 11.23 (s, 1H), 11.09 (s, 1H), 7.76 (d, *J* = 3.0 Hz, 1H), 7.65 (d, *J* = 7.6 Hz, 2H), 7.51 (d, *J*= 8.0 Hz, 2H), 7.37 - 7.30 (m, 1H), 7.26 (t, *J*= 6.0 Hz, 3H), 4.58 (dd, *J*= 12.0, 5.0 Hz, 1H), 4.34 (dd, *J* = 45.6, 12.7 Hz, 2H), 4.10 (s, 1H), 3.62 (tt, *J*= 11.1, 4.0 Hz, 1H), 3.48 - 3.33 (m, 4H), 3.30 - 3.20 (m, 3H), 3.15 - 2.89 (m, 3H), 2.71 (s, 8H), 2.61 (dt, *J*= 17.4, 4.2 Hz, 1H), 2.31 - 1.90 (m, 6H), 1.87 - 1.71 (m, 3H), 1.63 - 1.43 (m, 4H), 1.00 (s, 2H).

**Example 80:** Synthesis of Compound **80**

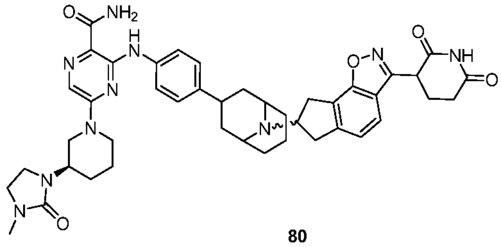

**80**

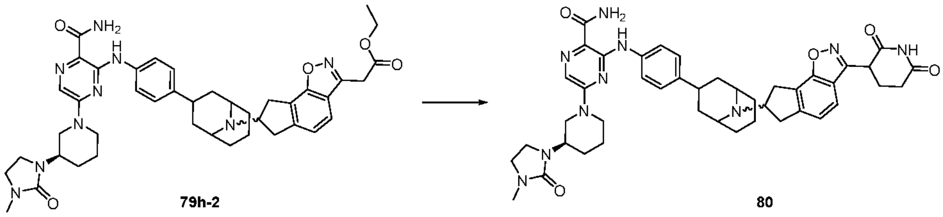

**79h-2** **80**

Step 1: Preparation of compound **80**

Intermediate **79h-2** (100 mg), acrylamide (7.46 mg) and tetrahydrofuran (5 mL) were added to a reaction flask in sequence, and the mixture was cooled to 0 °C under $N_2$ atmosphere. Potassium tert-butoxide (1 M, 0.079 mL) was added, and the mixture was reacted at 0 °C. After the reaction was completed, the reaction solution was added dropwise to an icy saturated aqueous ammonium chloride solution, and 50 mL of ethyl acetate was added for extraction. The organic phase was dried over anhydrous sodium sulfate, filtered, and concentrated, and the residue was purified by silica gel column chromatography to give compound **80** (45 mg).

MS(ESI, [M+H]+) m/z: 787.49.

$^1$H NMR (500 MHz, DMSO-*d*6) δ 11.23 (s, 1H), 11.09 (s, 1H), 7.76 (d, *J* = 3.0 Hz, 1H), 7.65 (d, *J* = 7.6 Hz, 2H), 7.51 (d, *J* = 8.0 Hz, 2H), 7.37 - 7.30 (m, 1H), 7.26 (t, *J* = 6.0 Hz, 3H), 4.58 (dd, *J* = 12.0, 5.0 Hz, 1H), 4.34 (dd, *J* = 45.6, 12.7 Hz, 2H), 4.10 (s, 1H), 3.62 (tt, *J* = 11.1, 4.0 Hz, 1H), 3.48 - 3.33 (m, 4H), 3.30 - 3.20 (m, 3H), 3.15 - 2.89 (m, 3H), 2.71 (s, 8H), 2.61 (dt, *J*= 17.4, 4.2 Hz, 1H), 2.31 - 1.90 (m, 6H), 1.87 - 1.71 (m, 3H), 1.63 - 1.43 (m, 4H), 1.00 (s, 2H).

**Example 81:** Synthesis of Compound **81**

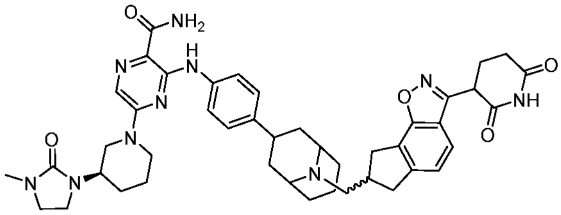

**81**

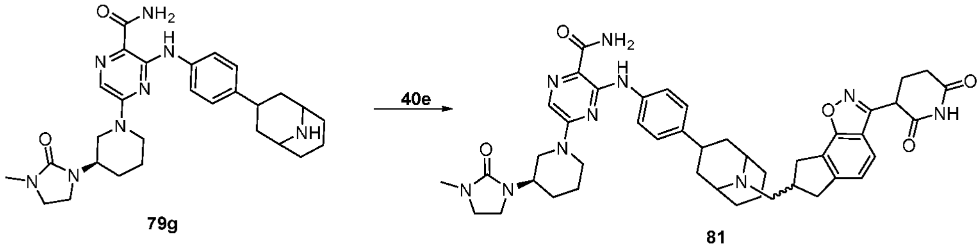


**79g** **81**

Step 1: Preparation of compound **81**

**40e** (100 mg), isopropanol (1 mL), 1,2-dichloroethane (5.00 mL), and **79g** (190 mg) were added to a reaction flask in sequence, and sodium cyanoborohydride (63 mg) was added. The mixture was reacted at room temperature. After the reaction was completed, the reaction solution was mixed with silica gel and purified by column chromatography to give **81** (35 mg).

MS(ESI, [M+H]+) m/z: 801.63.

$^1$H NMR (500 MHz, DMSO-*d*6) δ 11.21 (s, 1H), 11.09 (s, 1H), 7.81 - 7.72 (m, 1H), 7.66 (s, 1H), 7.61 (d, *J* = 8.0 Hz, 1H), 7.52 (d, *J* = 8.1 Hz, 2H), 7.34 (s, 1H), 7.26 (dd, *J* = 16.3, 8.1 Hz, 3H), 4.57 (dd, *J* = 11.8, 5.0 Hz, 1H), 4.35 (dd, *J*= 61.3, 12.9 Hz, 2H), 3.63 (td, *J*= 10.9, 10.3, 5.5 Hz, 1H), 3.34 (d, *J*= 7.2 Hz, 2H), 3.27 (dd, *J*= 10.6, 7.1 Hz, 2H), 3.24 - 2.91 (m, 8H), 2.91 - 2.56 (m, 10H), 2.23 - 1.99 (m, 4H), 1.80 (dd, *J*= 33.4, 12.5 Hz, 5H), 1.63 - 1.42 (m, 4H), 1.01 *(d, J*= 12.4 Hz, 2H).

**Example 82:** Synthesis of Compound **82**

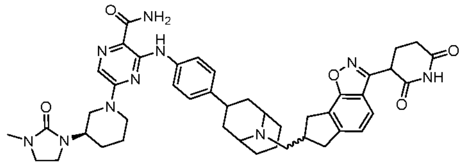

82

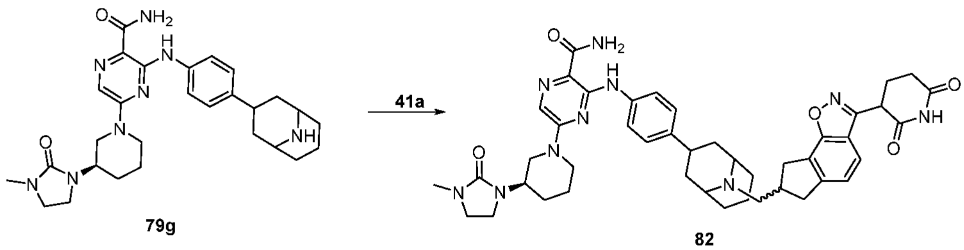


79g

82

Step 1: Preparation of compound **82**

**41a** (100 mg), isopropanol (1 mL), 1,2-dichloroethane (5.00 mL), and **79g** (190 mg) were added to a reaction flask in sequence, and sodium cyanoborohydride (18 mg) was added. The mixture was reacted at room temperature. After the reaction was completed, the reaction solution was mixed with silica gel and purified by column chromatography to give **82** (35 mg).

MS(ESI, [M+H]+) m/z: 801.51.

$^1$H NMR (500 MHz, DMSO-*d*6) δ 11.21 (s, 1H), 11.09 (s, 1H), 7.81 - 7.72 (m, 1H), 7.66 (s, 1H), 7.61 (d, *J* = 8.0 Hz, 1H), 7.52 (d, *J* = 8.1 Hz, 2H), 7.34 (s, 1H), 7.26 (dd, *J* = 16.3, 8.1 Hz, 3H), 4.57 (dd, *J* = 11.8, 5.0 Hz, 1H), 4.35 (dd, *J* = 61.3, 12.9 Hz, 2H), 3.63 (td, *J*= 10.9, 10.3, 5.5 Hz, 1H), 3.34 (d, *J*= 7.2 Hz, 2H), 3.27 (dd, *J* = 10.6, 7.1 Hz, 2H), 3.24 - 2.91 (m, 8H), 2.91 - 2.56 (m, 10H), 2.23 - 1.99 (m, 4H), 1.80 (dd, *J*= 33.4, 12.5 Hz, 5H), 1.63 - 1.42 (m, 4H), 1.01 *(d, J*= 12.4 Hz, 2H).

**Example 83:** Preparation of Compound **83**

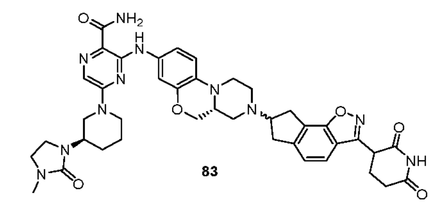

83

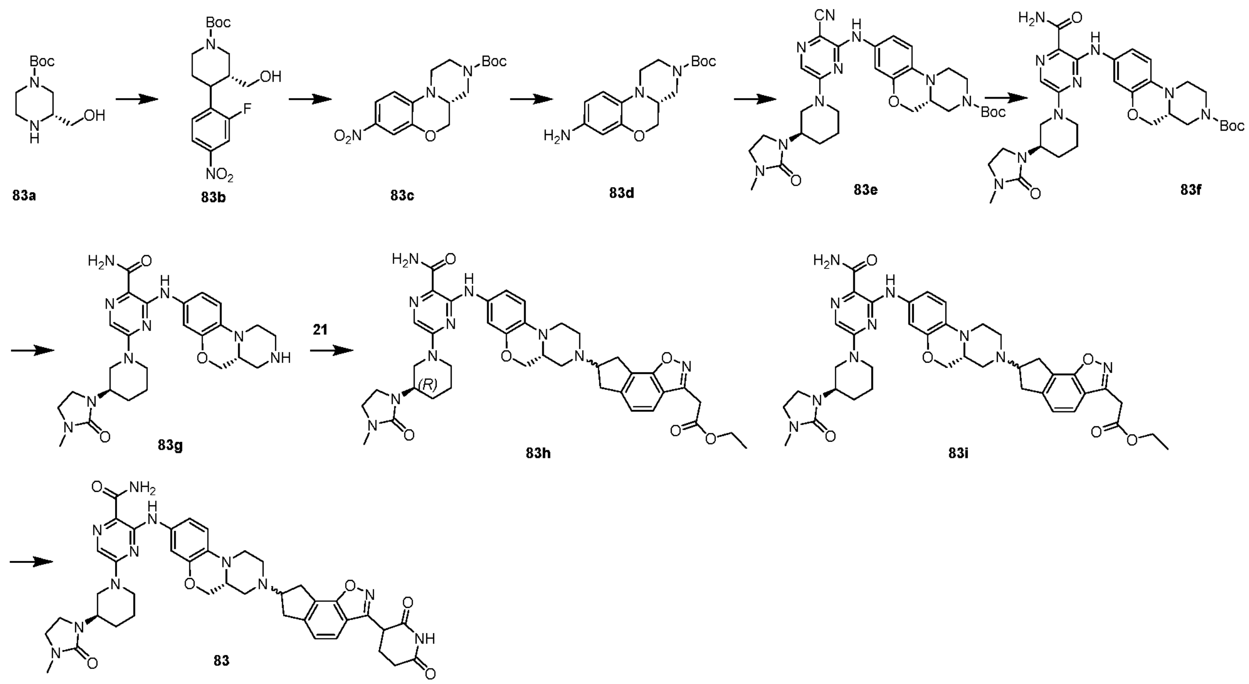


83a 83b 83c 83d 83e 83f 83g 83h 83i 83

Step 1: Preparation of intermediate **83b**

Intermediate **83a** (2.7 g), 3,4-difluoronitrobenzene (2.0 g), potassium hydroxide (2.1 g), and dimethyl sulfoxide (25 mL) were added to a reaction flask in sequence, and the mixture was reacted at room temperature for 4 h. 60 mL of ethyl acetate and 100 mL of water were added to the reaction solution. The organic phase was separated, washed with a saturated sodium chloride solution, dried over anhydrous sodium sulfate, filtered, and concentrated. The concentrate was separated and purified by silica gel column chromatography to give intermediate **83b** (3.4 g). MS(ESI, [M+H]+) m/z: 356.1.

$^1$H NMR (500 MHz, DMSO-d6) δ 8.16 (dd, J = 11.0, 2.8 Hz, 1H), 8.14 - 8.09 (m, 1H), 7.44 (t, J = 8.8 Hz, 1H), 4.14 (d, J = 6.1 Hz, 2H), 3.99 (d, J = 6.1 Hz, 1H), 3.72 (d, J = 12.6 Hz, 1H), 3.00 - 2.79 (m, 3H), 2.59 (tt, J = 11.1, 4.6 Hz, 2H), 1.39 (s, 9H).

Step 2: Preparation of intermediate **83c**

Intermediate **83b** (2.0 g), potassium hydroxide (0.95 g), and dimethyl sulfoxide (25 mL) were added to a reaction flask in sequence, and the mixture was heated to 70 °C and reacted for 16 h. The reaction solution was cooled to room temperature, and 50 mL of ethyl acetate and 100 mL of water were added to the reaction solution. The organic phase was separated, washed with a saturated sodium chloride solution, dried over anhydrous sodium sulfate, filtered, and concentrated. The concentrate was separated and purified by silica gel column chromatography to give intermediate **83c** (2.1 g).

$^1$H NMR (500 MHz, DMSO-d6) δ 7.77 (dd, J = 9.1, 2.6 Hz, 1H), 7.51 (d, J = 2.6 Hz, 1H), 7.04 (d, J = 9.2 Hz, 1H), 4.41 (dd, J = 11.1, 3.2 Hz, 1H), 4.05 - 3.92 (m, 4H), 3.37 (ddd, J = 14.9, 7.4, 3.5 Hz, 3H), 2.91 (td, J = 12.2, 3.3 Hz, 1H), 1.43 (s, 9H).

Step 3: Preparation of intermediate **83d**

Intermediate **83c** (2.1 g), 10% palladium on carbon (1.0 g), and methanol (30 mL) were added to a reaction flask in sequence, and the mixture was purged with hydrogen and reacted overnight. The reaction solution was filtered under vacuum, and the filtrate was concentrated to give intermediate **83d** (1.8 g).

MS(ESI, [M+H]+) m/z: 306.2.

Step 4: Preparation of intermediate **83e**

**1f** (1.50 g), **83d** (1.57 g), BINAP (0.29 g), cesium carbonate (4.57 g), palladium acetate (0.11 g), and 1,4-dioxane (40 mL) were added to a reaction flask in sequence, and the mixture was purged three times with nitrogen,

heated to 100 °C, and reacted for 4 h. The reaction solution was cooled to room temperature, and 50 mL of ethyl acetate and 100 mL of water were added to the residue. The organic phase was separated, washed with a saturated sodium chloride solution, dried over anhydrous sodium sulfate, filtered, and concentrated. The concentrate was separated and purified by silica gel column chromatography to give intermediate **83e** (1.67 g).

MS(ESI, [M+H]+) m/z: 590.4.

1H NMR (500 MHz, DMSO-d6) δ 8.75 (s, 1H), 7.76 (s, 1H), 7.04 (dd, J = 8.8, 2.5 Hz, 1H), 6.92 (d, J = 2.4 Hz, 1H), 6.79 (d, J = 8.9 Hz, 1H), 4.28 (dd, J = 10.8, 2.7 Hz, 3H), 4.05 - 3.82 (m, 3H), 3.70 (d, J = 11.9 Hz, 1H), 3.52 (td, J = 10.3, 4.8 Hz, 1H), 3.30 - 3.20 (m, 4H), 3.07 - 2.85 (m, 4H), 2.67 (s, 3H), 2.62 - 2.52 (m, 1H), 2.48 (d, J = 3.4 Hz, 2H), 1.82 - 1.70 (m, 3H), 1.42 (s, 9H).

Step 5: Preparation of intermediate **83f**

Intermediate **83e** (0.90 g), cesium carbonate (0.50 g), water (5 mL), DMSO (20 mL), and methanol (5 mL) were added to a reaction flask in sequence. The reaction solution was cooled to 0 °C, and 30 wt% hydrogen peroxide (0.52 g) was added dropwise. After the dropwise addition was completed, the mixture was allowed to react at room temperature for 1 h. 50 mL of water was added to the reaction solution, and then a saturated aqueous sodium sulfite solution was added to neutralize hydrogen peroxide. The mixture was stirred for 10 min and filtered, and the filter cake was collected and dried to give intermediate **83f** (0.85 g).

MS(ESI, $[M+H]^+$) m/z: 608.51.

Step 6: Preparation of intermediate **83g**

Intermediate **83f** (0.85 g) and trifluoroacetic acid (15 mL) were added to a reaction flask in sequence, and the mixture was reacted at room temperature for 2 h. The pH was adjusted to about 8 with a saturated aqueous sodium bicarbonate solution. The mixture was stirred for 10 min and filtered, and the filter cake was collected and dried to give intermediate **83g** (0.70 g).

MS(ESI, [M+H]+) m/z: 508.4.

1H NMR (500 MHz, DMSO-d6) δ 11.05 (s, 1H), 7.73 (d, J = 2.9 Hz, 1H), 7.61 (s, 1H), 7.30 (d, J = 2.8 Hz, 1H), 7.07 (d, J = 2.4 Hz, 1H), 6.98 (dd, J = 8.8, 2.5 Hz, 1H), 6.80 (d, J = 8.9 Hz, 1H), 4.30 (d, J = 13.0 Hz, 2H), 4.20 (dd, J = 10.7, 2.7 Hz, 1H), 3.89 (dd, J = 10.7, 8.7 Hz, 1H), 3.67 (d, J = 11.9 Hz, 1H), 3.56 (tt, J = 10.3, 4.2 Hz, 1H), 3.36 - 3.29 (m, 3H), 3.25 (dt, J = 8.3, 2.9 Hz, 2H), 3.19 - 2.98 (m, 5H), 2.97 - 2.82 (m, 2H), 2.67 (s, 3H), 2.60 (td, J = 12.0, 3.1 Hz, 1H), 1.79 (q, J = 6.7, 4.9 Hz, 3H), 1.60 - 1.50 (m, 1H).

Step 6: Preparation of intermediates **83h** and **83i**

Intermediate **83g** (0.44 g), **21** (206 mg), dichloroethane/isopropanol (5:1, 25 mL), and glacial acetic acid (10 mg) were added to a reaction flask in sequence. After the mixture was stirred at room temperature for 30 min, sodium cyanoborohydride (0.11 g) was added, and the mixture was reacted at room temperature for 2 h. 50 mL of DCM and 100 mL of water were added to the reaction solution. The organic phase was separated, washed with a saturated sodium chloride solution, dried over anhydrous sodium sulfate, filtered, and concentrated. The concentrate was separated and purified by silica gel column chromatography to give a mixture of intermediates **83h** and **83i** (0.32 g), which was subjected to resolution by preparative chromatography to give the prepeak **83h** (0.13 g) and the postpeak **83i** (0.14 g) in sequence.

The conditions of preparative chromatography were as follows: instrument and preparative column: YMC high pressure preparative chromatograph was used, and the preparative column was REGIS Cellulose-C. Mobile phase system: dichloromethane/n-hexane/ethanol; isocratic elution: dichloromethane/n-hexane/ethanol = 45/40/15. MS(ESI, $[M+H]^+$) m/z: 751.6.

Step 7: Preparation of compound **83**

Intermediate **83i** (120 mg) and acrylamide (22 mg) were added to a reaction flask in sequence, and the mixture was cooled to 0 °C. A solution of potassium *tert*-butoxide in tetrahydrofuran (0.25 mL, 1 M) was added dropwise, and the mixture was reacted at 0 °C. After the reaction was completed, as confirmed by TLC, the reaction solution was poured into 40 mL of a cold aqueous ammonium chloride solution, and dichloroethane (40 mL each time) was added for three times of extraction. The organic phases were combined, washed with water and saturated sodium chloride, dried over anhydrous sodium sulfate, and filtered, and the concentrate was separated and purified by silica gel column chromatography to give compound **83** (20 mg).

MS(ESI, [M+H]+) m/z: 776.4.

$^1$H NMR (500 MHz, DMSO-d6) δ 11.09 (s, 1H), 11.04 (s, 1H), 7.73 (d, J = 2.9 Hz, 1H), 7.67 - 7.58 (m, 2H),

7.32 - 7.24 (m, 2H), 7.05 (d, J = 2.4 Hz, 1H), 6.98 (dd, J = 8.7, 2.5 Hz, 1H), 6.79 (d, J = 9.0 Hz, 1H), 4.57 (dd, J = 11.9, 5.0 Hz, 1H), 4.33 - 4.20 (m, 3H), 3.91 (dd, J = 10.6, 8.9 Hz, 1H), 3.68 (d, J = 11.3 Hz, 1H), 3.55 (dq, J = 10.5, 5.9 Hz, 1H), 3.45 (q, J = 7.7 Hz, 1H), 3.28 - 3.15 (m, 4H), 3.12 - 3.00 (m, 5H), 3.01 - 2.86 (m, 3H), 2.77 (ddd, J = 17.2, 11.9, 5.4 Hz, 1H), 2.68 (s, 3H), 2.61 (ddd, J = 13.4, 7.2, 3.4 Hz, 2H), 2.53 (d, J = 5.6 Hz, 1H), 2.47 (d, J = 8.3 Hz, 1H), 2.29 (dd, J = 12.2, 9.0 Hz, 1H), 2.19 (dt, J = 13.8, 5.4 Hz, 1H), 1.89 (t, J = 10.5 Hz, 1H), 1.78 (dq, J = 10.8, 5.8, 3.2 Hz, 3H), 1.55 (d, J = 12.4 Hz, 1H).

**Example 84:** Synthesis of Compound **84**

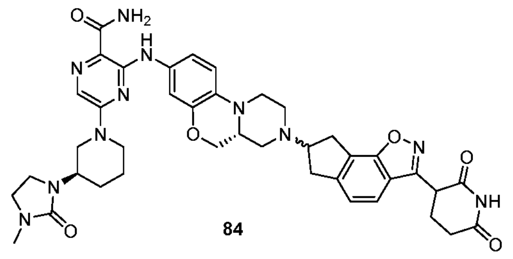

**84**

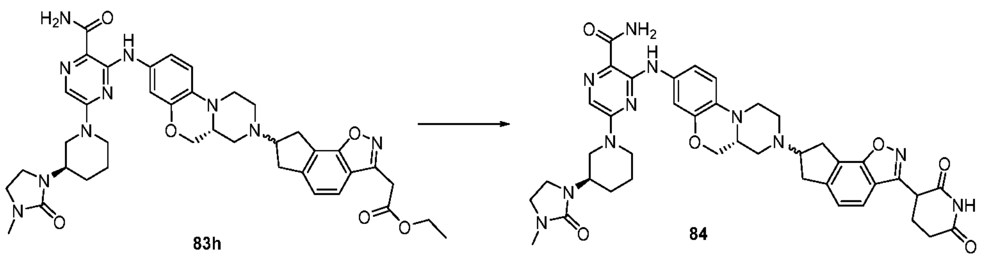

**83h** **84**

Step 1: Preparation of compound **84**

Intermediate **83h** (130 mg) and acrylamide (22 mg) were added to a reaction flask in sequence, and the mixture was cooled to 0 °C. A solution of potassium tert-butoxide in tetrahydrofuran (0.26 mL, 1 M) was added dropwise, and the mixture was reacted at 0 °C. After the reaction was completed, as confirmed by TLC, the reaction solution was poured into 40 mL of a cold aqueous ammonium chloride solution, and dichloroethane (40 mL each time) was added for three times of extraction. The organic phases were combined, washed with water and saturated sodium chloride, dried over anhydrous sodium sulfate, and filtered, and the concentrate was separated and purified by silica gel column chromatography to give compound **84** (18 mg).

MS(ESI, $[M+H]^+$) *m/z:* 776.4.

1H NMR (500 MHz, DMSO-*d6*) δ 11.09 (s, 1H), 11.04 (s, 1H), 7.77 - 7.69 (m, 1H), 7.68 - 7.58 (m, 2H), 7.33 - 7.24 (m, 2H), 7.06 (d, *J*= 2.5 Hz, 1H), 6.97 (dd, *J*= 8.8, 2.5 Hz, 1H), 6.79 (d, *J*= 8.8 Hz, 1H), 4.57 (dd, *J*= 11.9, 5.0 Hz, 1H), 4.34 - 4.21 (m, 3H), 3.92 (t, *J* = 9.7 Hz, 1H), 3.68 (d, *J* = 11.2 Hz, 1H), 3.55 (td, *J* = 10.5, 10.1, 5.7 Hz, 1H), 3.42 (t, *J*= 7.7 Hz, 1H), 3.36 (d, *J*= 5.3 Hz, 2H), 3.28 - 3.20 (m, 3H), 3.05 (dd, *J*= 19.7, 11.7 Hz, 6H), 2.93 (t, *J*= 12.7 Hz, 1H), 2.77 (ddd, *J*= 17.2, 11.9, 5.3 Hz, 1H), 2.68 (s, 3H), 2.62 (td, *J*= 12.9, 11.9, 7.0 Hz, 2H), 2.53 (d, *J*= 6.9 Hz, 1H), 2.47 (d, *J*= 9.5 Hz, 1H), 2.28 (t, *J* = 10.7 Hz, 1H), 2.19 (q, *J*= 8.0, 6.6 Hz, 1H), 1.89 (t, *J* = 10.9 Hz, 1H), 1.79 (q, *J* = 13.5, 10.7 Hz, 3H), 1.55 (d, *J* = 11.6 Hz, 1H).

**Example 85:** Synthesis of Compound **85**

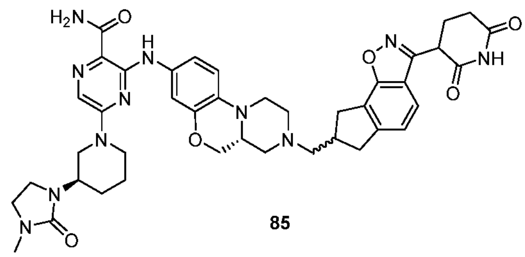

85

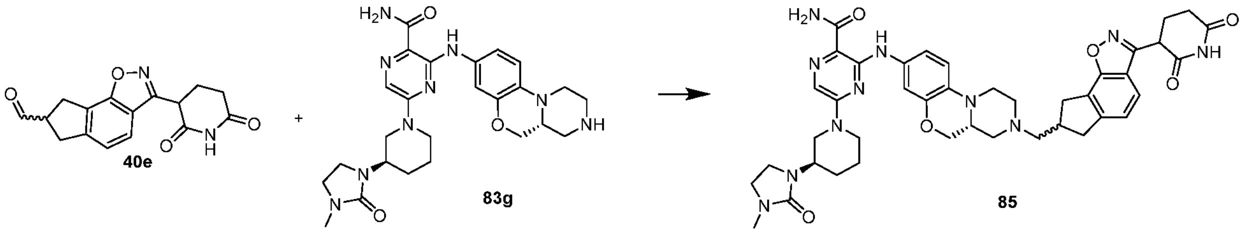

40e 83g 85

Step 1: Preparation of compound **85**

Intermediate **83g** (102 mg), **40e** (60 mg), dichloroethane/isopropanol (5:1, 25 mL), and glacial acetic acid (10 mg) were added to a reaction flask in sequence. After the mixture was stirred at room temperature for 30 min, sodium cyanoborohydride (13 mg) was added, and the mixture was reacted at room temperature for 2 h. 50 mL of dichloromethane and 100 mL of water were added to the reaction solution. The organic phase was separated, washed with a saturated sodium chloride solution, dried over anhydrous sodium sulfate, filtered, and concentrated. The concentrate was separated and purified by silica gel column chromatography to give compound **85** (42 mg).

MS(ESI, $[M+H]^+$) *m/z:* 790.4.

1H NMR (500 MHz, DMSO-*d*6) δ 11.08 (s, 1H), 11.03 (s, 1H), 7.73 *(d, J*= 2.9 Hz, 1H), 7.61 *(d, J*= 7.5 Hz, 2H), 7.32 - 7.25 (m, 2H), 7.05 (d, *J*= 2.4 Hz, 1H), 6.98 (dd, *J*= 8.8, 2.4 Hz, 1H), 6.79 (d, *J*= 8.8 Hz, 1H), 4.57 (ddd, *J* = 11.8, 4.9, 1.5 Hz, 1H), 4.29 (s, 2H), 4.22 (dd, *J* = 10.6, 2.7 Hz, 1H), 3.94 - 3.87 (m, 1H), 3.65 (d, *J* = 11.5 Hz, 1H), 3.59 - 3.52 (m, 1H), 3.34 (d, *J*= 8.1 Hz, 3H), 3.24 (dd, *J*= 9.1, 7.2 Hz, 3H), 3.17 (d, *J*= 5.3 Hz, 1H), 3.08 (t, *J* = 11.8 Hz, 1H), 3.04 - 2.98 (m, 2H), 2.98 - 2.88 (m, 4H), 2.84 (dd, *J* = 16.2, 5.1 Hz, 1H), 2.77 (ddd, *J* = 17.1, 11.9, 5.3 Hz, 1H), 2.67 (s, 3H), 2.64 - 2.57 (m, 2H), 2.40 (t, *J* = 7.3 Hz, 2H), 2.22 - 2.13 (m, 2H), 1.78 (p, *J* = 10.7, 9.8 Hz, 4H), 1.55 (d, *J* = 14.9 Hz, 1H).

**Example 86:** Synthesis of Compound **86**

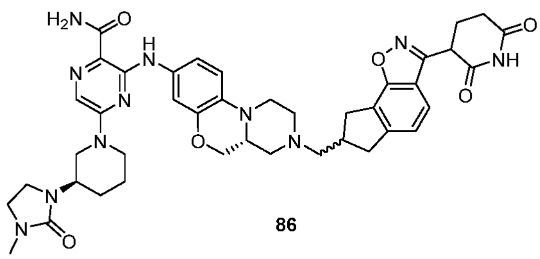

86

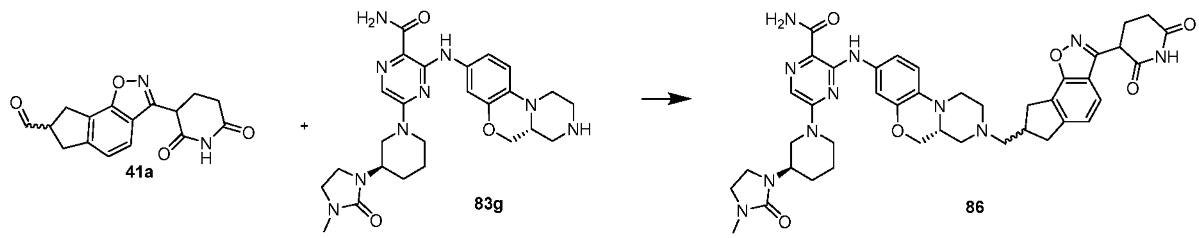

41a 83g 86

Step 1: Preparation of compound **86**

Intermediate **83g** (102 mg), **41a** (60 mg), dichloroethane/isopropanol (5:1, 25 mL), and glacial acetic acid (10 mg) were added to a reaction flask in sequence. After the mixture was stirred at room temperature for 30 min, sodium cyanoborohydride (13 mg) was added, and the mixture was reacted at room temperature for 2 h. 50 mL of dichloromethane and 100 mL of water were added to the reaction solution. The organic phase was separated, washed with a saturated sodium chloride solution, dried over anhydrous sodium sulfate, filtered, and concentrated. The concentrate was separated and purified by silica gel column chromatography to give compound 86 (38 mg).

MS(ESI, $[M+H]^+$) *m/z:* 790.4.

1H NMR (500 MHz, DMSO-*d*6) δ 11.08 (d, *J*= 1.6 Hz, 1H), 11.03 (s, 1H), 7.73 (d, *J* = 2.9 Hz, 1H), 7.61 (d, *J* = 7.7 Hz, 2H), 7.32 - 7.24 (m, 2H), 7.05 (d, *J* = 2.4 Hz, 1H), 6.97 (dd, *J* = 8.8, 2.5 Hz, 1H), 6.79 (d, *J* = 8.9 Hz, 1H), 4.57 (ddd, *J* = 11.9, 5.0, 1.8 Hz, 1H), 4.29 (s, 2H), 4.21 (dd, *J* = 10.5, 2.7 Hz, 1H), 3.90 (dd, *J* = 10.7, 9.0 Hz, 1H), 3.65 (d, *J* = 11.4 Hz, 1H), 3.59 - 3.52 (m, 1H), 3.34 (d, *J* = 8.1 Hz, 3H), 3.28 - 3.22 (m, 3H), 3.20 - 3.15 (m, 1H), 3.08 (t, *J*= 11.8 Hz, 1H), 3.01 (d, *J*= 10.3 Hz, 2H), 2.93 (t, *J*= 15.0 Hz, 4H), 2.84 (dd, *J* = 16.0, 5.1 Hz, 1H), 2.77 (ddd, *J* = 17.1, 11.9, 5.3 Hz, 1H), 2.67 (s, 3H), 2.65 - 2.57 (m, 2H), 2.40 (dd, *J* = 7.6, 4.4 Hz, 2H), 2.17 (ddd, *J* = 14.7, 10.1, 3.5 Hz, 2H), 1.77 (q, *J* = 11.3, 10.4 Hz, 4H), 1.55 (d, *J*= 11.3 Hz, 1H).

**Example 87:** Synthesis of Compound **87**

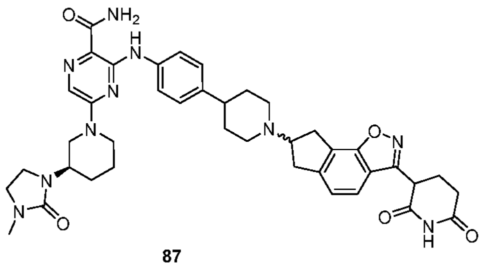

87

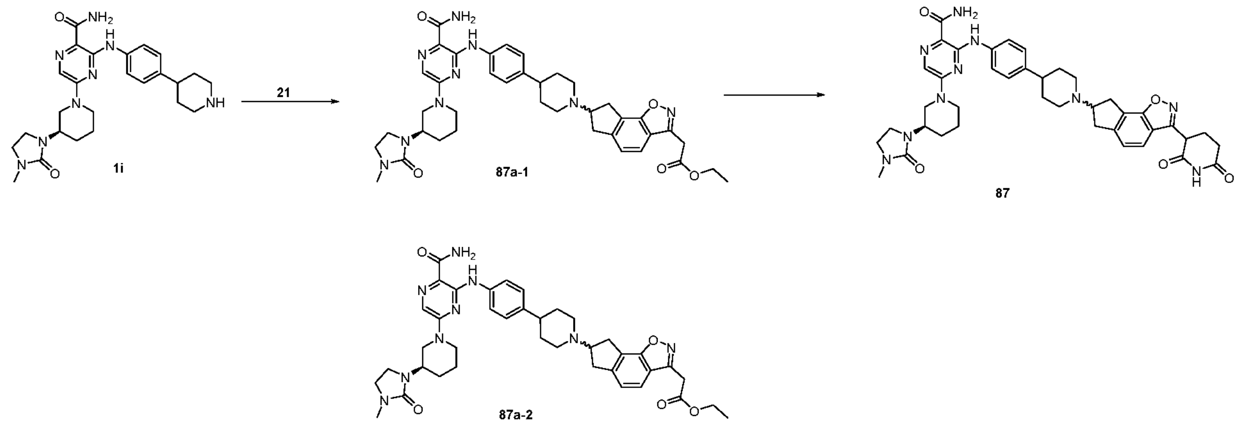


1i

87a-1

87

87a-2

Step 1: Preparation of compounds **87a-1** and **87a-2**

Intermediate **21** (300 mg), intermediate **1i** (600 mg), 1,2-dichloroethane (10 mL), isopropanol (3 mL), and sodium cyanoborohydride (291 mg) were added to a reaction flask in sequence, and the mixture was reacted overnight at room temperature. After the reaction was completed, 5 mL of a saturated sodium bicarbonate solution was added to the reaction solution to neutralize acetic acid, and then 100 mL of dichloromethane and 100 mL of water were added for extraction. The organic phase was separated, and the aqueous phase was extracted twice with 50 mL of dichloromethane. The organic phases were combined, washed with saturated brine, dried over anhydrous sodium sulfate, and concentrated, and the residue was purified by silica gel column chromatography to give a racemic modification. The racemic modification was separated by high performance liquid chromatography to give intermediates **87a-1** (150 mg) and **87a-2** (130 mg) in sequence.

The conditions of preparative chromatography were as follows: instrument and preparative column: YMC high

pressure preparative chromatograph was used, and the preparative column was (R,R)-Whelk-O1 (4.6×250 mm, 5 μm). Mobile phase system: ethanol-dichloromethane (3:2)/n-hexane-0.1% diethylamine, isocratic elution: ethanol-dichloromethane (3:2)/n-hexane-0.1% diethylamine = 50/50.

**87a-1, 87a-2:** MS(ESI, [M+H]+) m/z: 722. 70.

Step 2: Preparation of compound **87**

Intermediate **87a-1** (100 mg), acrylamide (15 mg) and tetrahydrofuran (5 mL) were added to a reaction flask in sequence, and the mixture was cooled to 0 °C under $N_2$ atmosphere. Potassium tert-butoxide (1 M, 0.079 mL) was added, and the mixture was reacted at 0 °C. After the reaction was completed, the reaction solution was added dropwise to an icy saturated aqueous ammonium chloride solution, and 50 mL of ethyl acetate was added for extraction. The organic phase was dried over anhydrous sodium sulfate, filtered, and concentrated, and the residue was purified by silica gel column chromatography to give compound **87** (35 mg).

MS(ESI, [M+H]+) m/z: 747.61.

$^1$H NMR (500 MHz, DMSO-*d6*) δ 11.20 (s, 1H), 11.09 (s, 1H), 7.76 (d, *J*= 2.8 Hz, 1H), 7.70 - 7.58 (m, 2H), 7.50 (d, *J*= 8.3 Hz, 2H), 7.38 - 7.31 (m, 1H), 7.27 (d, *J*= 8.1 Hz, 1H), 7.17 (d, *J*= 8.3 Hz, 2H), 4.57 (dd, *J*= 11.8, 5.0 Hz, 1H), 4.32 (dd, *J* = 31.0, 12.8 Hz, 2H), 3.61 (dt, *J* = 11.0, 6.1 Hz, 1H), 3.50 - 3.39 (m, 1H), 3.35 (d, *J* = 6.9 Hz, 3H), 3.25 (ddt, *J* = 26.3, 15.8, 7.7 Hz, 4H), 3.00 (dq, *J* = 33.1, 12.2, 11.6 Hz, 6H), 2.84 - 2.67 (m, 4H), 2.61 (dt, *J* = 17.4, 4.3 Hz, 1H), 2.46 (s, 1H), 2.26 - 2.09 (m, 3H), 1.88 - 1.49 (m, 8H).

**Example 88:** Synthesis of Compound **88**

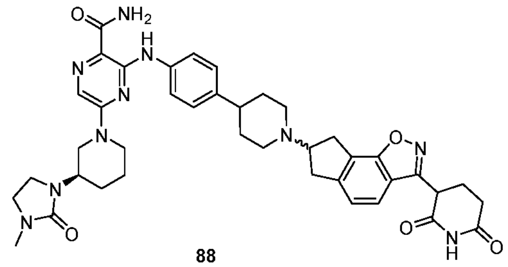

88

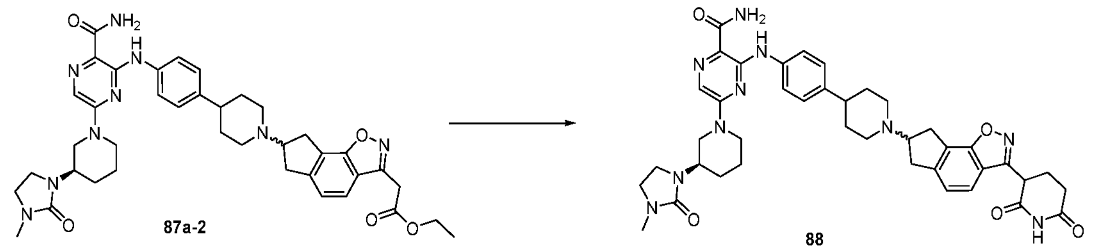

87a-2

88

Step 1: Preparation of compound **88**

Intermediate **87a-2** (100 mg), acrylamide (15 mg), and tetrahydrofuran (5 mL) were added to a reaction flask in sequence, and the mixture was cooled to 0 °C under $N_2$ atmosphere. Potassium tert-butoxide (1 M, 0.079 mL) was added, and the mixture was reacted at 0 °C. After the reaction was completed, the reaction solution was added dropwise to an icy saturated aqueous ammonium chloride solution, and 50 mL of ethyl acetate was added for extraction. The organic phase was dried over anhydrous sodium sulfate, filtered, and concentrated, and the residue was purified by silica gel column chromatography to give compound **88** (50 mg).

MS(ESI, [M+H]+) m/z: 747.55.

$^1$H NMR (500 MHz, DMSO-*d6*) δ 11.20 (s, 1H), 11.09 (s, 1H), 7.76 (d, *J*= 2.8 Hz, 1H), 7.70 - 7.58 (m, 2H), 7.50 (d, *J*= 8.3 Hz, 2H), 7.38 - 7.31 (m, 1H), 7.27 (d, *J*= 8.1 Hz, 1H), 7.17 (d, *J*= 8.3 Hz, 2H), 4.57 (dd, *J*= 11.8, 5.0 Hz, 1H), 4.32 (dd, *J* = 31.0, 12.8 Hz, 2H), 3.61 (dt, *J* = 11.0, 6.1 Hz, 1H), 3.50 - 3.39 (m, 1H), 3.35 (d, *J* = 6.9 Hz, 3H), 3.25 (ddt, *J* = 26.3, 15.8, 7.7 Hz, 4H), 3.00 (dq, *J* = 33.1, 12.2, 11.6 Hz, 6H), 2.84 - 2.67 (m, 4H), 2.61 (dt, *J* = 17.4, 4.3 Hz, 1H), 2.46 (s, 1H), 2.26 - 2.09 (m, 3H), 1.88 - 1.49 (m, 8H).

**Example** 89: Preparation of Compound **89**

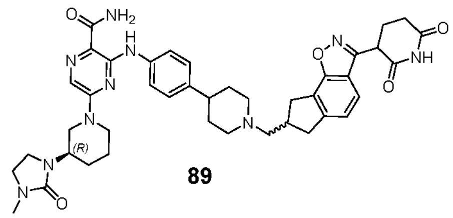

**89**

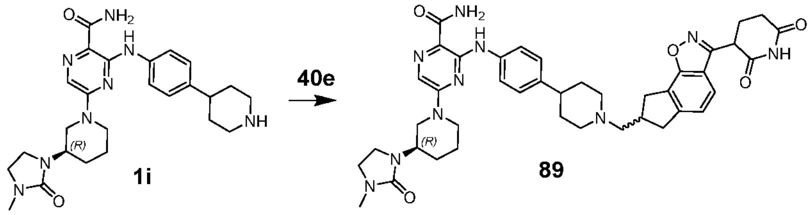


**1i** **89**

Step 1: Preparation of compound **89**

Intermediate **1i** (96 mg), **40e** (60 mg), dichloroethane/isopropanol (5:1, 25 mL), and glacial acetic acid (10 mg) were added to a reaction flask in sequence. After the mixture was stirred at room temperature for 30 min, sodium cyanoborohydride (25.3 mg) was added, and the mixture was reacted at room temperature for 2 h. 50 mL of DCM and 100 mL of water were added to the reaction solution. The organic phase was separated, washed with a saturated sodium chloride solution, dried over anhydrous sodium sulfate, filtered, and concentrated. The concentrate was separated and purified by silica gel column chromatography to give compound 89 (25 mg).

MS(ESI, $[M+H]^+$) *m/z:* 761.6.

1H NMR (500 MHz, DMSO-*d6*) δ 11.18 (s, 1H), 11.08 (s, 1H), 7.75 (s, 1H), 7.66 (s, 1H), 7.61 (d, *J* = 7.9 Hz, 1H), 7.50 (d, *J* = 8.0 Hz, 2H), 7.32 (s, 1H), 7.27 (d, *J* = 8.1 Hz, 1H), 7.18 (d, *J* = 8.2 Hz, 2H), 4.56 (dd, *J* = 11.5, 4.9 Hz, 1H), 4.37 (d, *J* = 12.1 Hz, 1H), 4.29 (d, *J* = 12.9 Hz, 1H), 3.62 (d, *J* = 12.6 Hz, 1H), 3.35 (s, 2H), 3.26 (d, *J* = 10.2 Hz, 4H), 3.24 - 3.12 (m, 2H), 3.05 - 2.97 (m, 3H), 2.97 - 2.81 (m, 4H), 2.74 (s, 3H), 2.63 (s, 1H), 2.44 (s, 1H), 2.36 (d, *J* = 7.1 Hz, 2H), 2.24 - 2.14 (m, 1H), 2.03 (t, *J* = 11.4 Hz, 2H), 1.87 - 1.72 (m, 5H), 1.65 (d, *J* = 13.4 Hz, 2H), 1.57 (s, 1H).

**Example 90:** Preparation of Compound **90**

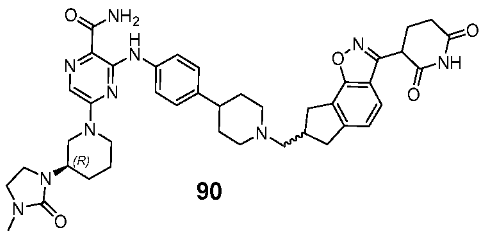

**90**

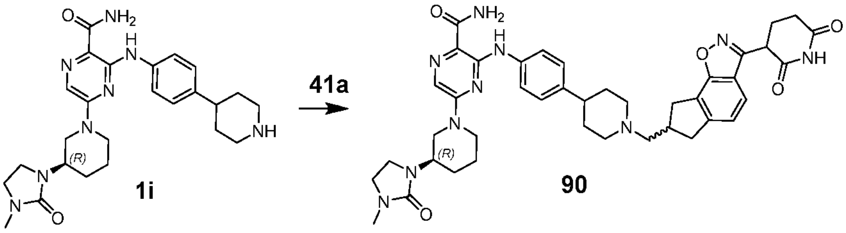


**1i** **90**

Step 1: Preparation of compound **90**

Intermediate **1i** (110 mg), **41a** (65 mg), dichloroethane/isopropanol (5:1, 25 mL), and glacial acetic acid (10 mg) were added to a reaction flask in sequence. After the mixture was stirred at room temperature for 30 min, sodium cyanoborohydride (27.6 mg) was added, and the mixture was reacted at room temperature for 2 h. 50 mL of DCM and 100 mL of water were added to the reaction solution. The organic phase was separated, washed with a saturated sodium chloride solution, dried over anhydrous sodium sulfate, filtered, and concentrated. The concentrate was separated and purified by silica gel column chromatography to give compound **90** (31 mg).

MS(ESI, $[M+H]^+$) *m/z:* 761.6.

$^1H$ NMR (500 MHz, DMSO-*d*6) δ 11.18 (s, 1H), 11.08 (s, 1H), 7.75 (d, *J* = 2.8 Hz, 1H), 7.66 (s, 1H), 7.61 (d, *J* = 8.0 Hz, 1H), 7.53 - 7.47 (m, 2H), 7.32 (d, *J* = 2.9 Hz, 1H), 7.27 (d, *J*= 8.1 Hz, 1H), 7.18 (d, *J* = 8.2 Hz, 2H), 4.57 (ddd, *J* = 11.8, 5.0, 1.5 Hz, 1H), 4.37 (d, *J* = 12.5 Hz, 1H), 4.29 (d, *J* = 13.3 Hz, 1H), 3.62 (dq, *J* = 8.2, 5.5, 4.1 Hz, 1H), 3.38 - 3.32 (m, 2H), 3.31 - 3.24 (m, 4H), 3.24 - 3.14 (m, 2H), 3.01 (dd, *J* = 14.6, 6.2 Hz, 3H), 2.97 - 2.82 (m, 4H), 2.75 (s, 3H), 2.64 - 2.57 (m, 1H), 2.47 - 2.41 (m, 1H), 2.36 (d, *J* = 7.3 Hz, 2H), 2.19 (dq, *J* = 8.6, 4.4, 3.8 Hz, 1H), 2.03 (t, *J* = 11.4 Hz, 2H), 1.86 - 1.72 (m, 5H), 1.66 (dd, *J* = 16.9, 7.3 Hz, 2H), 1.57 (q, *J* = 12.2 Hz, 1H).

**Example 91:** Synthesis of Compound **91**

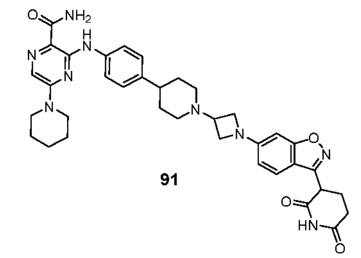

**91**

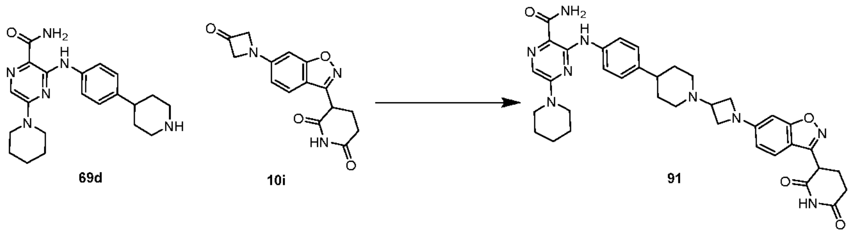

**69d** **10i** **91**

Step 1: Preparation of compound **91**

Intermediate **69d** (150 mg), intermediate **10i** (177 mg), dichloroethane (10 mL), isopropanol (2 mL), and glacial acetic acid (11.84 mg, 0.011 mL) were added to a reaction flask. After the mixture was stirred at room temperature for 30 min, sodium cyanoborohydride (74.3 mg) was added, and the mixture was stirred overnight at room temperature. After the reaction was completed, 100 mL of water was added to the reaction solution, and the mixture was extracted twice with 100 mL of dichloromethane. The extracts were combined, dried over anhydrous sodium sulfate, filtered under vacuum, and concentrated, and the residue was purified by silica gel column chromatography to give compound **91** (50 mg).

Q-TOF(ESI, $[M+H]^+$) *m/z:* 664.3371

$^1H$ NMR (500 MHz, DMSO-$d_6$) δ 11.28 (s, 1H), 11.04 (s, 1H), 7.73 (s, 1H), 7.66 (s, 1H), 7.56 (d, *J* = 8.6 Hz, 1H), 7.51 (d, *J* = 8.1 Hz, 2H), 7.31 (s, 1H), 7.18 (d, *J* = 8.1 Hz, 2H), 6.56 - 6.47 (m, 2H), 4.44 (dd, *J* = 11.4, 5.0 Hz, 1H), 4.04 (t, *J* = 7.4 Hz, 2H), 3.73 (t, *J* = 6.5 Hz, 2H), 3.67 (t, *J* = 5.2 Hz, 4H), 2.93 (d, *J* = 10.5 Hz, 2H), 2.74 (ddd, *J* = 17.1, 11.5, 5.3 Hz, 1H), 2.58 (dt, *J* = 17.4, 4.5 Hz, 1H), 2.43 (ddt, *J* = 16.1, 11.8, 4.2 Hz, 2H), 2.22 - 2.13 (m, 1H), 1.96 (t, *J* = 11.4 Hz, 2H), 1.78 *(d, J*= 12.4 Hz, 2H), 1.68 - 1.62 (m, 3H), 1.62 - 1.55 (m, 5H).

**Example 92:** Synthesis of Compound **92**

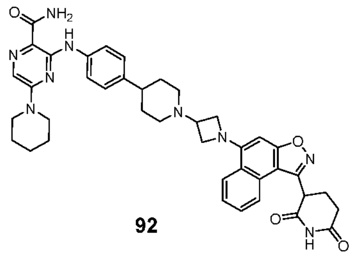

**92**

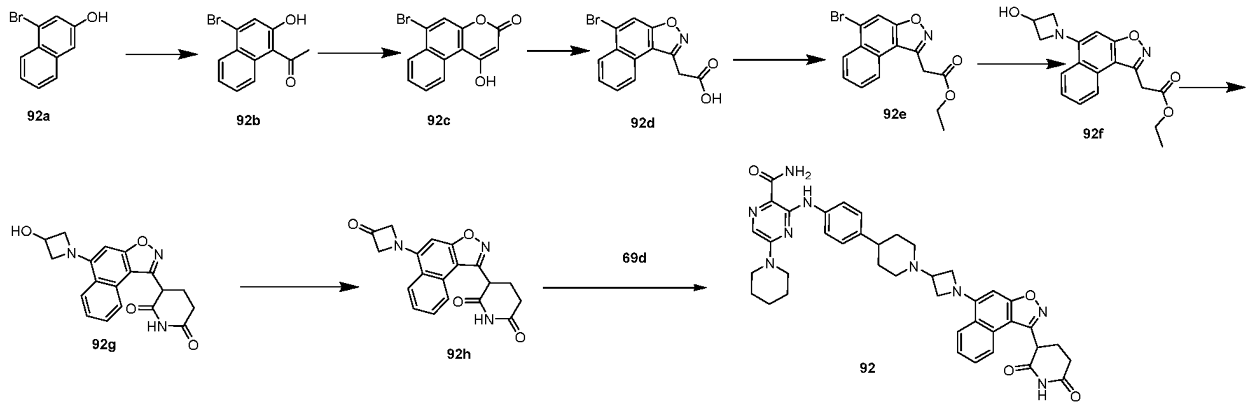


Step **1:** Preparation of intermediate **92b**

Intermediate **92a** (20 g), 1,2-dichloroethane (200 mL), acetyl chloride (7.04 g), and aluminum trichloride (17.93 g) were added to a reaction flask in sequence, and the mixture was heated to 85 °C and reacted. After the reaction was completed, as confirmed by TLC, the reaction solution was cooled to room temperature, slowly added to 1 M diluted hydrochloric acid with ice, and extracted with 200 mL dichloromethane. The organic phase was separated, and the aqueous phase was extracted twice with dichloromethane. The organic phases were combined, dried over anhydrous sodium sulfate, and filtered, and the filtrate was concentrated by evaporation at reduced pressure to remove the solvent. The residue was separated by silica gel column chromatography to give intermediate **92b** (21.1 g).

MS(ESI, $[M-H]^+$) *m/z:* 262.9
$^1H$ NMR (500 MHz, DMSO-$d_6$) $\delta$ 10.81 (s, 1H), 8.10 - 8.04 (m, 1H), 7.73 (dt, *J* = 8.5, 1.0 Hz, 1H), 7.62 (s, 1H), 7.56 (ddd, *J* = 8.4, 6.7, 1.4 Hz, 1H), 7.49 (ddd, *J* = 8.1, 6.8, 1.2 Hz, 1H), 2.61 (s, 3H).

Step **2:** Preparation of intermediate **92c**

Intermediate **92b** (21 g), diethyl carbonate (46.4 g), and toluene (200 mL) were added to a reaction flask in sequence. The reaction solution was cooled to 0 °C, and sodium hydride (9.43 g) was added in portions. The mixture was heated to 120 °C and reacted. After the reaction was completed, as confirmed by TLC, the reaction solution was cooled to room temperature and slowly poured into 1 L of stirred ice water. The mixture was extracted with 500 mL of ethyl acetate, and the organic phase was discarded. The aqueous phase was adjusted to pH = 3 with 3 N hydrochloric acid, the mixture was extracted 3 times with 500 mL of ethyl acetate, and the organic phases were combined. The organic phase was dried over anhydrous sodium sulfate, filtered and concentrated by evaporation at reduced pressure to remove the solvent, thus giving intermediate **92c** (20.2 g).

MS(ESI, $[M-H]^+$) *m/z:* 288.9
$^1H$ NMR (500 MHz, DMSO-$d_6$) $\delta$ 8.07 (dd, J = 8.4, 1.2 Hz, 1H), 7.85 - 7.81 (m, 1H), 7.62 - 7.56 (m, 2H), 7.50 (ddd, J = 8.2, 6.9, 1.3 Hz, 1H), 4.08 (s, 2H).

Step **3:** Preparation of intermediate **92d**

Intermediate **92c** (20 g), an aqueous hydroxylamine solution (21.05 mL), and ethanol (200 mL) were added to a reaction flask in sequence, and the mixture was reacted at 85 °C. After the reaction was completed, as confirmed by TLC, the reaction solution was cooled to room temperature and concentrated by evaporation at reduced pressure to remove the solvent, and 200 mL of ethyl acetate and 200 mL of a saturated aqueous sodium carbonate solution were added to the residue for extraction. The organic phase was separated and the aqueous phase was collected, adjusted to pH of 2-3 with a 3 M aqueous HCl solution, and extracted with 300 mL of ethyl acetate. The organic phase was collected, dried over anhydrous sodium sulfate, filtered, and concentrated by evaporation at reduced pressure to remove the solvent, thus giving intermediate **92d** (18.5 g).

MS(ESI, $[M-H]^+$) *m/z:* 303.9
$^1$H NMR (500 MHz, DMSO-$d_6$) $\delta$ 13.09 (s, 1H), 8.51 (s, 1H), 8.37 (dd, J = 8.4, 1.2 Hz, 1H), 8.28 - 8.21 (m, 1H), 7.87 (ddd, J = 8.3, 7.0, 1.3 Hz, 1H), 7.79 (ddd, J = 8.3, 7.0, 1.3 Hz, 1H), 4.43 (s, 2H).

Step 4: Preparation of intermediate **92e**

Intermediate **92d** (18 g), ethanol (150 mL), and sulfuric acid (3.13 mL) were added to a reaction flask in sequence, and the mixture was heated to 85 °C and reacted. After the reaction was completed, as confirmed by TLC, the reaction solution was cooled to room temperature and concentrated by evaporation at reduced pressure to remove the solvent. 300 mL of ethyl acetate and 300 mL of water were added to the residue for dilution, and a saturated aqueous sodium bicarbonate solution was added to adjust pH = 7. The organic phase was separated, and the aqueous phase was extracted twice with 500 mL of ethyl acetate. The organic phases were combined, dried over anhydrous sodium sulfate, filtered, and concentrated by evaporation at reduced pressure to remove the solvent, thus giving intermediate **92e** (17.42 g).

MS(ESI, $[M+H]^+$) *m/z:* 334.12
$^1$H NMR (500 MHz, DMSO-$d_6$) $\delta$ 8.52 (s, 1H), 8.38 (dd, J = 8.4, 1.2 Hz, 1H), 8.19 (dd, J = 8.1, 1.0 Hz, 1H), 7.86 (ddd, J = 8.2, 7.0, 1.3 Hz, 1H), 7.79 (ddd, J = 8.2, 7.0, 1.2 Hz, 1H), 4.54 (s, 2H), 4.15 (q, J = 7.1 Hz, 2H), 1.16 (t, J = 7.1 Hz, 3H).

Step **5:** Preparation of intermediate **92f**

Intermediate **92e** (4 g), azetidin-3-ol (1.050 g), palladium acetate (0.537 g), xantphos (2.77 g), potassium phosphate (7.62 g), and 1,4-dioxane (30 mL) were added to a reaction flask in sequence, and the mixture was heated to 100 °C and reacted under $N_2$ atmosphere. After the reaction was completed, as confirmed by TLC, the reaction solution was cooled to room temperature, and 100 mL of ethyl acetate and 200 mL of water were added to the system. The organic phase was separated, and the aqueous phase was extracted 3 times with 50 mL of ethyl acetate. The organic phases were combined, dried over anhydrous sodium sulfate, and filtered, and the filtrate was concentrated by evaporation at reduced pressure to remove the solvent. The crude product was separated by silica gel column chromatography to give intermediate **92f** (3.03 g).

MS(ESI, $[M+H]^+$) *m/z:* 327.28
$^1$H NMR (500 MHz, DMSO-$d_6$) $\delta$ 8.07 (dd, J = 44.6, 8.4 Hz, 2H), 7.59 (dt, J = 77.7, 7.6 Hz, 2H), 6.78 (s, 1H), 5.74 (d, J = 6.4 Hz, 1H), 4.64 (h, J = 5.9 Hz, 1H), 4.52 (t, J = 7.3 Hz, 2H), 4.14 (q, J = 7.1 Hz, 2H), 3.93 (dd, J = 8.2, 4.7 Hz, 2H), 1.17 (t, J = 7.0 Hz, 3H).

Step **6:** Preparation of intermediate **92g**

Intermediate **92f** (580 mg), acrylamide (250 mg), and tetrahydrofuran (10 mL) were added to a reaction flask in sequence. The mixture was purged 3 times with nitrogen, and potassium tert-butoxide (299 mg) was added at -15 °C. The mixture was reacted at room temperature. After the reaction was completed, as confirmed by TLC, the reaction solution was added dropwise to a saturated aqueous ammonium chloride solution to quench the reaction, and 50 mL of ethyl acetate was added for extraction. The organic phase was separated, and the aqueous phase was extracted 3 times with 50 mL of ethyl acetate. The organic phases were combined, dried over anhydrous sodium sulfate, filtered, and concentrated by evaporation at reduced pressure to remove the solvent. The residue was separated by silica gel column chromatography to give intermediate **92g** (0.41 g).

MS(ESI, [M-H]+) *m/z:* 350.12
$^1$H NMR (500 MHz, DMSO-$d_6$) δ 8.11 (t, *J* = 7.6 Hz, 2H), 7.66 (ddd, *J*= 8.2, 6.9, 1.2 Hz, 1H), 7.53 (ddd, *J* = 8.3, 6.9, 1.2 Hz, 1H), 6.80 (s, 1H), 5.74 (d, J = 6.4 Hz, 1H), 4.91 (dd, *J* = 10.9, 4.9 Hz, 1H), 4.64 (ddd, *J* = 11.4, 6.5, 4.9 Hz, 1H), 4.52 (dt, *J* = 9.8, 7.5 Hz, 2H), 3.92 (dt, *J* = 8.0, 5.2 Hz, 2H), 2.81 (ddd, *J* = 16.9, 11.2, 5.3 Hz, 1H), 2.68 - 2.58 (m, 2H), 2.41 - 2.27 (m, 2H).

Step **7:** Preparation of intermediate **92h**

Intermediate **92g** (410 mg), acetonitrile (10 mL), and IBX (980 mg) were added to a reaction flask in sequence, and the mixture was heated to 85 °C and reacted under $N_2$ atmosphere. After the reaction was completed, as confirmed by TLC, the reaction solution was cooled to room temperature and filtered, and the filtrate was concentrated by evaporation at reduced pressure to remove the solvent. The crude product was separated by silica gel column chromatography to give intermediate **92h** (0.39 g).

MS(ESI, [M-H]+) *m/z:* 348.21
$^1$H NMR (500 MHz, DMSO-*$d_6$*) δ 11.11 (s, 1H), 8.22 - 8.14 (m, 2H), 7.70 (ddd, J = 8.2, 6.9, 1.2 Hz, 1H), 7.57 (ddd, J = 8.4, 7.0, 1.2 Hz, 1H), 7.14 (s, 1H), 5.21 - 5.08 (m, 4H), 4.96 (dd, J = 11.1, 4.8 Hz, 1H), 2.82 (ddd, J = 17.0, 11.5, 5.3 Hz, 1H), 2.61 (dt, J = 17.2, 4.4 Hz, 1H), 2.57 - 2.52 (m, 1H), 2.36 (dt, J = 13.1, 4.7 Hz, 1H).

Step **8:** Preparation of compound **92**

Intermediate **92h** (100 mg), intermediate **69d** (110 mg), and 1,2-dichloroethane (10 mL) were added to a reaction flask in sequence, acetic acid (15.78 mg) was added dropwise, and the mixture was stirred at room temperature for 20 min. Sodium cyanoborohydride (49.5 mg) was added, and the mixture was reacted at room temperature. After the reaction was completed, as confirmed by TLC, 20 mL of dichloromethane and 50 mL of water were added to the system for extraction. The organic phase was separated, and the aqueous phase was extracted 3 times with dichloromethane/MeOH = 10:1. The organic phases were combined, dried over anhydrous sodium sulfate, filtered, concentrated by evaporation at reduced pressure to remove the solvent, and separated and purified by silica gel column chromatography to give compound **92** (40 mg).

Q-TOF (ESI, [M+H]+) *m/z:* 714.3511
$^1$H NMR (500 MHz, DMSO-$d_6$) δ 11.29 (d, J = 5.0 Hz, 1H), 11.10 (s, 1H), 8.14 (dd, J = 22.1, 8.3 Hz, 2H), 7.73 (s, 1H), 7.71 - 7.60 (m, 2H), 7.52 (dd, J = 8.6, 5.0 Hz, 3H), 7.31 (s, 1H), 7.19 (d, J = 8.2 Hz, 2H), 6.82 (d, J = 5.4 Hz, 1H), 4.92 (dd, J = 10.9, 5.0 Hz, 1H), 4.41 (q, J = 8.0 Hz, 2H), 4.05 (q, J = 6.5 Hz, 2H), 3.76 - 3.62 (m, 4H), 3.34 (d, J = 3.3 Hz, 2H), 2.99 (d, J = 10.4 Hz, 2H), 2.81 (ddd, J = 16.9, 11.1, 5.1 Hz, 1H), 2.60 (dt, J = 17.1, 4.1 Hz, 1H), 2.47 (s, 1H), 2.42 - 2.29 (m, 1H), 1.99 (t, J = 11.1 Hz, 2H), 1.79 (d, J = 12.4 Hz, 2H), 1.62 (dp, J = 26.5, 5.3, 4.6 Hz, 8H).

**Example 93:** Synthesis of Compound **93**

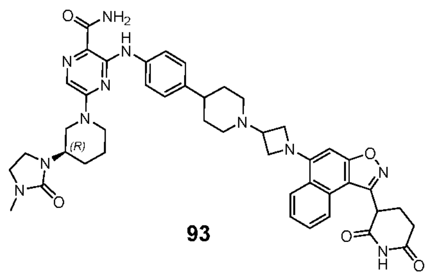

**93**

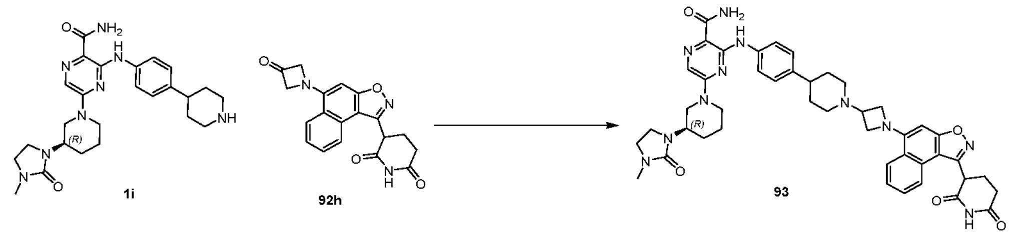

Step **1:** Preparation of compound **93**

Intermediate **92h** (100 mg), intermediate **1i** (88 mg), 1,2-dichloroethane (10 mL), and isopropanol (1.000 mL) were added to a reaction flask in sequence, acetic acid (12.55 mg) was added dropwise, and the mixture was stirred at room temperature for 20 min. Sodium cyanoborohydride (39.4 mg) was added, and the mixture was reacted at room temperature. After the reaction was completed, as confirmed by TLC, 20 mL of dichloromethane and 50 mL of water were added to the system for extraction. The organic phase was separated, and the aqueous phase was extracted 3 times with dichloromethane/MeOH = 10:1. The organic phases were combined, dried over anhydrous sodium sulfate, filtered, concentrated by evaporation at reduced pressure to remove the solvent, and separated and purified by silica gel column chromatography to give compound **93** (40 mg).

Q-TOF (ESI, $[M+H]^+$) *m/z:* 812.3996
$^1$H NMR (500 MHz, DMSO-$d_6$) δ 11.20 (s, 1H), 11.10 (s, 1H), 8.14 (dd, J = 21.3, 8.0 Hz, 2H), 7.75 (d, J = 2.7 Hz, 1H), 7.66 (d, J = 5.6 Hz, 2H), 7.51 (dd, J = 15.8, 8.1 Hz, 3H), 7.33 (d, J = 2.7 Hz, 1H), 7.16 (d, J = 8.3 Hz, 2H), 6.81 (s, 1H), 4.92 (dd, J = 10.8, 5.0 Hz, 1H), 4.41 (q, J = 8.0 Hz, 2H), 4.31 (dd, J = 30.8, 12.8 Hz, 2H), 4.10 - 4.00 (m, 2H), 3.37-3.33 (m, 2H), 3.32-3.29 (m, 2H), 3.28 - 3.19 (m, 2H), 3.10 - 2.90 (m, 4H), 2.81 (ddd, J = 16.9, 11.2, 5.5 Hz, 1H), 2.69 (s, 3H), 2.60 (dt, J = 17.3, 4.5 Hz, 1H), 2.55-2.52 (m, 1H), 2.49-2.45 (m, 1H), 2.34 (dd, J = 11.9, 7.4 Hz, 1H), 2.06 - 1.94 (m, 2H), 1.80 (d, J = 11.4 Hz, 5H), 1.70 - 1.50 (m, 3H).

**Example 94:** Synthesis of Compound **94**

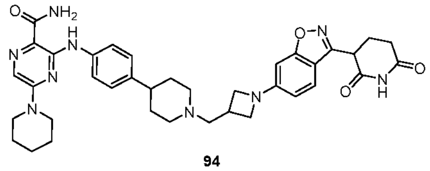

94

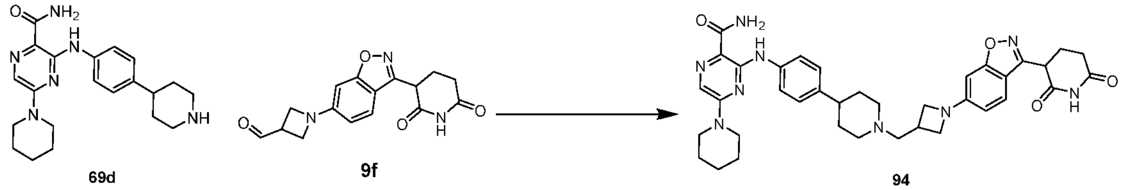

Step 1: Preparation of compound **94**

Intermediate **69d** (80 mg), intermediate **9f** (99 mg), dichloromethane (5 mL), and methanol (1 mL) were first added to the reaction solution, and then glacial acetic acid (12.6 mg, 12 μL) was added. After the mixture was stirred for 10 min, sodium cyanoborohydride (26.4 mg) was added. The mixture was stirred overnight at room temperature. A small amount of a NaHCOs solution was added to the reaction solution to quench the reaction, and then the mixture was purified by silica gel column chromatography to give compound **94** (80 mg).

MS(ESI, $[M+H]^+$) *m/z:* 677.96
$^1$H NMR (500 MHz, DMSO-$d_6$) δ 11.30 (s, 1H), 11.04 (s, 1H), 7.74 (d, *J* = 2.9 Hz, 1H), 7.66 (s, 1H), 7.54 (t, *J* = 9.3 Hz, 3H), 7.32 (d, *J* = 3.0 Hz, 1H), 7.19 (d, *J* = 8.1 Hz, 2H), 6.54 - 6.46 (m, 2H), 4.44 (dd, *J* = 11.4, 5.0 Hz, 1H), 4.06

(t, *J*= 7.8 Hz, 2H), 3.72 - 3.59 (m, 6H), 3.34 (s, 3H), 2.74 (ddd, *J*= 17.0, 11.5, 5.3 Hz, 2H), 2.63 - 2.52 (m, 2H), 2.42 (qd, *J* = 12.0, 4.6 Hz, 2H), 2.17 (dq, *J* = 14.1, 5.0 Hz, 2H), 1.78 (s, 3H), 1.62 (dq, *J* = 32.7, 5.6, 5.2 Hz, 8H).

**Example 95:** Synthesis of Compound **95**

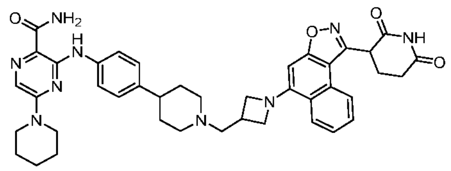

**95**

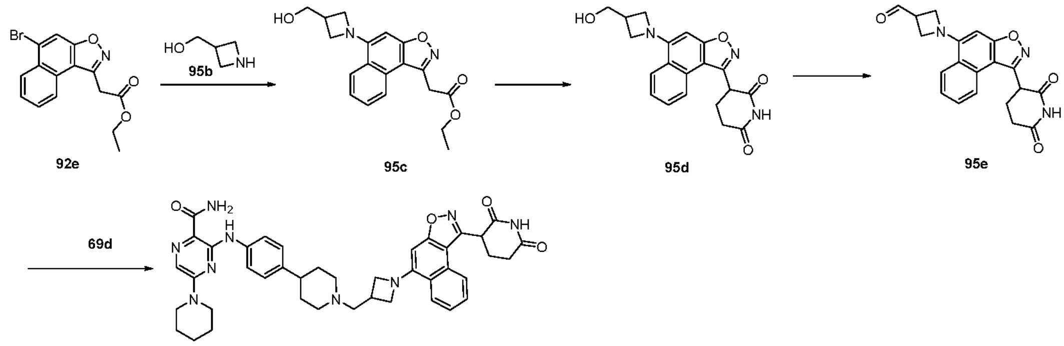


92e 95c 95d 95e

95

Step 1: Preparation of intermediate **95c**

Intermediate **92e** (3.0 g), intermediate **95b** (1.173 g), potassium phosphate (5.72 g), palladium acetate (0.302 g), xantphos (1.558 g), and 1,4-dioxane (50 mL) were added to a reaction flask, and the mixture was heated at 100 °C under nitrogen atmosphere. After the reaction was completed, 10 mL of water was added to the reaction solution, and 30 mL of ethyl acetate was added for extraction. The organic phases were combined and washed with saturated brine, and the extract was dried over anhydrous sodium sulfate, filtered, subjected to rotary evaporation, mixed with silica gel, and purified by column chromatography to give intermediate **95c** (3.0 g).

MS(ESI, $[M+H]^+$) m/z: 341.30

$^1$H NMR (500 MHz, DMSO-$d_6$) δ 8.13 (dd, *J* = 8.6, 1.2 Hz, 1H), 8.01 (dd, *J*= 8.3, 1.2 Hz, 1H), 7.66 (ddd, *J*= 8.2, 5.7, 1.2 Hz, 1H), 7.50 (ddd, *J* = 8.4, 6.9, 1.3 Hz, 1H), 7.35 - 7.25 (m, 1H), 6.70 (s, 1H), 4.83 (t, *J* = 5.3 Hz, 1H), 4.31 (t, *J* = 8.0 Hz, 4H), 4.14 (q, *J*= 7.1 Hz, 2H), 4.01 (dd, *J* = 7.8, 5.5 Hz, 2H), 3.68 - 3.60 (m, 1H), 2.85 (tt, *J* = 8.1, 5.7 Hz, 1H), 1.17 (t, *J* = 7.1 Hz, 3H).

Step 2: Preparation of intermediate **95d**

Intermediate **95c** (1.5 g) and acrylamide (0.5 g) were added to a reaction flask. The reaction apparatus was sealed, then anhydrous tetrahydrofuran (30 mL) was added, and a solution of potassium tert-butoxide in THF was slowly added dropwise (the internal temperature was controlled at 0 °C). After the dropwise addition was completed, the temperature was maintained at 0 °C and the mixture was reacted for 30 min, and then the mixture was reacted at room temperature. After the reaction was completed, as confirmed by TLC, the reaction solution was added dropwise to a mixed system of a saturated ammonium chloride solution-ethyl acetate under stirring. The organic phase was separated, dried over anhydrous sodium sulfate, filtered, mixed with silica gel, and purified by column chromatography to give intermediate **95d** (581 mg).

MS(ESI, $[M+H]^+$) *m/z:* 366.30

$^1$H NMR (500 MHz, DMSO-$d_6$) δ 11.09 (s, 1H), 8.16 - 8.11 (m, 1H), 7.65 (ddd, *J* = 8.2, 6.9, 1.2 Hz, 1H), 7.54 - 7.47 (m, 2H), 6.73 (s, 1H), 4.90 (dd, *J* = 10.9, 4.9 Hz, 1H), 4.82 (t, *J* = 5.4 Hz, 1H), 4.31 (q, *J*= 7.9 Hz, 2H), 4.01 (td, *J* = 7.9, 5.4 Hz, 2H), 3.65 (t, *J* = 5.8 Hz, 2H), 2.89 - 2.75 (m, 2H), 2.59 (dt, *J* = 17.3, 4.6 Hz, 1H), 2.46 (dd, *J* = 10.9, 4.3

Hz, 1H), 2.33 (dq, *J* = 13.8, 5.0 Hz, 1H).

Step 3: Preparation of intermediate **95e**

Intermediate **95d** (280 mg), Dess-Martin periodinane (584 mg), and dichloromethane (20 mL) were added to a reaction flask, and the mixture was stirred at room temperature. After the reaction was completed, the reaction solution was filtered under vacuum, and the filter cake was washed with dichloromethane. The filtrate and the washing solution were combined and subjected to rotary evaporation to dryness, thus giving intermediate **95e** (300 mg).
MS(ESI, $[M+H]^+$) *m/z:* 364.2

Step 4: Preparation of compound **95**

**69d** (100 mg), intermediate **95e** (150 mg), 1,2-dichloroethane (10 mL), and isopropanol (2 mL) were first added to the reaction solution, and then glacial acetic acid (14.2 mg, 14 μL) was added. After the mixture was stirred for 10 min, sodium cyanoborohydride (29.7 mg) was added. The mixture was stirred at room temperature. After the reaction was completed, a small amount of a NaHCOs solution was added to the reaction solution to quench the reaction, and then the mixture was purified by silica gel column chromatography to give compound **95** (32 mg).

MS(ESI, $[M+H]^+$) *m/z:* 728.40
$^1$H NMR (500 MHz, DMSO-*$d_6$*) δ 11.29 (s, 1H), 11.10 (s, 1H), 8.12 (dd, *J* = 18.8, 8.4 Hz, 2H), 7.74 (s, 1H), 7.66 (q, *J* = 7.1, 5.8 Hz, 2H), 7.52 (d, *J* = 8.1 Hz, 3H), 7.32 (s, 1H), 7.19 (d, *J* = 8.2 Hz, 2H), 6.76 (s, 1H), 4.91 (dd, *J* = 10.9, 4.9 Hz, 1H), 4.42 (q, *J* = 8.6 Hz, 2H), 3.93 (q, *J* = 6.7 Hz, 2H), 3.68 (t, *J* = 5.4 Hz, 4H), 3.00 (t, *J* = 12.8 Hz, 3H), 2.81 (ddd, *J* = 17.1, 11.0, 5.2 Hz, 2H), 2.59 (dt, *J* = 17.4, 4.6 Hz, 2H), 2.47 - 2.43 (m, 1H), 2.33 (q, *J* = 6.4, 5.8 Hz, 1H), 2.09 (s, 2H), 1.76 (d, *J* = 11.0 Hz, 2H), 1.66 (q, *J* = 8.0 Hz, 4H), 1.61 - 1.56 (m, 4H).

**Example 96:** Synthesis of Compound **96**

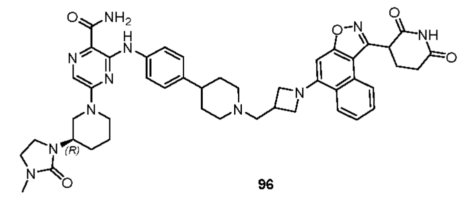

96

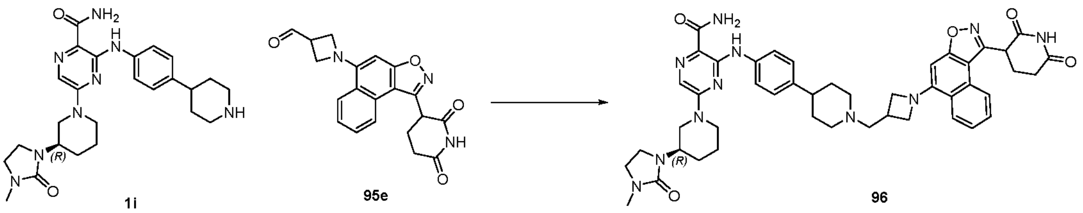

1i 95e 96

Step 1: Preparation of compound **96**

Intermediate **1i** (120 mg), intermediate **95e** (150 mg), 1,2-dichloroethane (10 mL), and isopropanol (2 mL) were first added to the reaction solution, and then glacial acetic acid (13.5 mg, 13 μL) was added. After the mixture was stirred for 10 min, sodium cyanoborohydride (28.4 mg) was added. The mixture was stirred at room temperature. After the reaction was completed, a small amount of a NaHCOs solution was added to the reaction solution to quench the reaction, and then the mixture was purified by silica gel column chromatography to give compound **96** (65 mg).

MS(ESI, $[M+H]^+$) *m/z:* 826.52
$^1$H NMR (500 MHz, DMSO-$d_6$) δ 11.20 (s, 1H), 11.10 (s, 1H), 8.12 (dd, *J* = 19.4, 8.4 Hz, 2H), 7.76 *(d, J*= 2.9 Hz, 1H), 7.65 (d, *J*= 9.4 Hz, 2H), 7.51 (dd, *J* = 11.1, 8.1 Hz, 3H), 7.33 (d, *J*= 2.9 Hz, 1H), 7.17 (d, *J*= 8.3 Hz, 2H), 6.75 (s, 1H), 4.91 (dd, *J*= 10.8, 5.0 Hz, 1H), 4.46 - 4.32 (m, 3H), 4.29 (d, *J* = 13.3 Hz, 1H), 3.92 (q, *J* = 6.6 Hz, 2H), 3.62 (ddt, *J*= 11.0, 8.7, 4.0 Hz, 1H), 3.31 - 3.22 (m, 3H), 3.08 - 2.91 (m, 4H), 2.81 (ddd, *J*= 16.8, 11.2, 5.3 Hz, 1H), 2.72

(s, 3H), 2.66 (d, *J* = 7.3 Hz, 2H), 2.63 - 2.51 (m, 2H), 2.45 (ddt, *J* = 24.5, 12.0, 2.9 Hz, 3H), 2.37 - 2.29 (m, 1H), 2.07 (td, *J*= 11.6, 2.4 Hz, 2H), 1.86 - 1.71 (m, 5H), 1.68 - 1.52 (m, 3H).

**Example** 97: Synthesis of Compound **97**

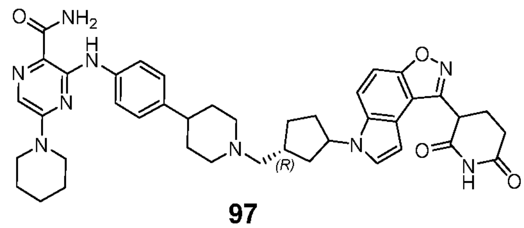

**97**

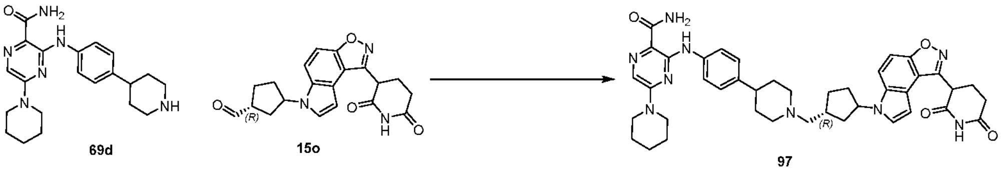

69d 15o 97

Step **1:** Preparation of compound **97**

Intermediate **69d** (100 mg), intermediate **15o** (144 mg), dichloromethane (10 mL), and MeOH (1.000 mL) were added to a reaction flask in sequence, acetic acid (15.78 mg) was added dropwise, and the mixture was stirred at room temperature for 20 min. Sodium cyanoborohydride (49.5 mg) was added, and the mixture was reacted at room temperature. After the reaction was completed, as confirmed by TLC, 20 mL of dichloromethane and 50 mL of water were added to the system for extraction. The organic phase was separated, and the aqueous phase was extracted 3 times with dichloromethane/MeOH = 10:1. The organic phases were combined, dried over anhydrous sodium sulfate, filtered, and concentrated by evaporation at reduced pressure to remove the solvent, and the crude product was separated by silica gel column chromatography to give compound **97** (40 mg).

Q-TOF (ESI, $[M+H]^+$) *m/z:* 730.3822
$^1$H NMR (500 MHz, DMSO-$d_6$) $\delta$ 11.28 (s, 1H), 11.18 (s, 1H), 7.91 (dd, *J*= 9.2, 2.2 Hz, 1H), 7.78 - 7.69 (m, 2H), 7.66 (s, 1H), 7.50 (dd, *J*= 10.9, 8.6 Hz, 3H), 7.31 (d, *J*= 2.9 Hz, 1H), 7.17 (d, *J*= 8.3 Hz, 2H), 6.58 (dd, *J* = 7.8, 3.1 Hz, 1H), 5.04 (p, *J*= 8.0 Hz, 1H), 4.69 (dd, *J* = 11.8, 5.1 Hz, 1H), 3.67 (t, *J*= 5.3 Hz, 4H), 3.01 (s, 2H), 2.85 (ddd, *J*= 17.4, 12.1, 5.4 Hz, 1H), 2.63 (dt, *J*= 17.2, 4.2 Hz, 1H), 2.48 - 2.39 (m, 3H), 2.38 (s, 3H), 2.23 (ddt, *J* = 15.5, 11.3, 6.7 Hz, 2H), 2.09 - 1.87 (m, 5H), 1.74 (d, *J*= 11.5 Hz, 2H), 1.69 - 1.54 (m, 9H).

**Example 98:** Synthesis of Compounds **98-1** and **98-2**

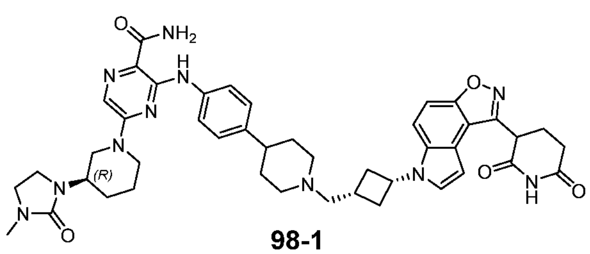

**98-1**

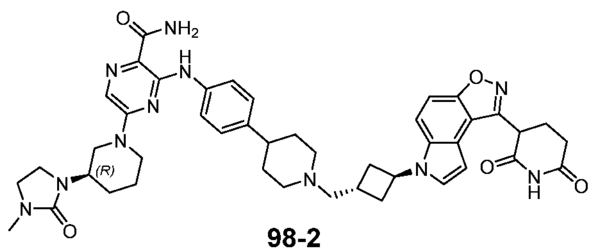

**98-2**

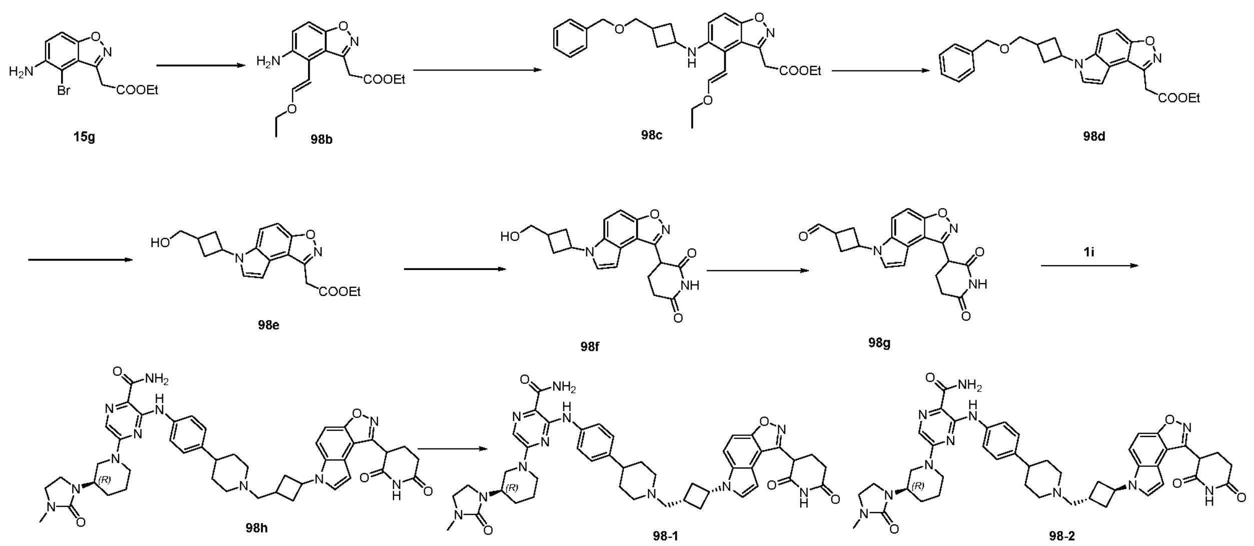


15g 98b 98c 98d 98e 98f 98g 98h 98-1 98-2

Step 1: Preparation of intermediate **98b**

Intermediate **15g** (30.0 g), ethoxyvinyl borate (24.83 g), cesium carbonate (94.25 g), palladium acetate (2.16 g), Ruphos (9.00 g), and 1,4-dioxane (500 mL) were added to a reaction flask, and the mixture was stirred at 100 °C. After the reaction was completed, as confirmed by TLC, 1000 mL of water was added to the reaction solution, and the mixture was extracted twice with 500 mL of ethyl acetate. The organic phases were combined, dried over anhydrous sodium sulfate, filtered under vacuum and concentrated, and the residue was purified by silica gel column chromatography to give intermediate **98b** (14.50 g).

MS(ESI, [M+H]+) m/z: 291.11.

$^1$H NMR (500 MHz, DMSO-$d_6$) δ 7.29 (d, *J* = 8.8 Hz, 1H), 7.01 (d, *J* = 8.8 Hz, 1H), 6.70 (d, *J* = 13.1 Hz, 1H), 5.64 (d, *J* = 13.1 Hz, 1H), 4.89 (s, 2H), 4.11 - 4.04 (m, 2H), 3.95 - 3.89 (m, 2H), 1.29 (t, *J* = 7.0 Hz, 3H), 1.18 (t, *J* = 7.1 Hz, 3H).

Step 2: Preparation of intermediate **98c**

Intermediate **98b** (5.0 g), 3-(benzyloxymethyl)-1-cyclobutanone (4.42 g), methanol (50 mL), and glacial acetic acid (0.465 g, 7.75 mmol) were added to a reaction flask. After the mixture was stirred at room temperature for 5 min, sodium cyanoborohydride (1.606 g, 25.6 mmol) was added, and the mixture was heated at 60 °C. After the reaction was completed, the reaction solution was extracted twice with 300 mL of water and 300 mL of dichloromethane. The extracts were combined, dried over anhydrous sodium sulfate, filtered under vacuum, and concentrated, and the residue was purified by silica gel column chromatography to give intermediate **98c** (4.73 g). MS(ESI, [M+H]+) m/z: 465.30.

Step 3: Preparation of intermediate **98d**

Intermediate **98c** (4.60 g), dichloromethane (50 mL), and hydrochloric acid (5.94 mL, 4 mol/L, 23.76 mmol) were added to a reaction flask in sequence, and the mixture was reacted at room temperature. After the reaction was completed, as confirmed by TLC, 200 mL of an aqueous NaHCOs solution and 200 mL of dichloromethane were added to the reaction solution. The organic phase was separated, dried over anhydrous sodium sulfate, filtered, and concentrated. The concentrate was separated and purified by silica gel column chromatography to give intermediate **98d** (3.56 g). MS(ESI, [M+H]$^+$) *m/z:* 419.33

Step 4: Preparation of intermediate **98e**

Intermediate **98d** (3.50 g), 10% palladium on carbon catalyst (1.75 g), MeOH (35 mL), and hydrochloric acid (2.01 mL, 4 mol/L) were added to a reaction flask in sequence, and the mixture was reacted at room temperature under $H_2$ atmosphere. After the reaction was completed, as confirmed by TLC, the reaction solution was filtered under vacuum, and the filtrate was concentrated and separated and purified by silica gel column chromatography to give intermediate **98e** (2.05 g).
MS(ESI, [M+H]$^+$) *m/z:* 329.32

Step 5: Preparation of intermediate **98f**

Intermediate **98e** (2.0 g), acrylamide (0.9 g), and anhydrous tetrahydrofuran (30 mL) were added to a reaction flask in sequence. A solution of potassium *tert*-butoxide in THF (1.03 g, 9.14 mL, 9.14 mmol) was slowly added under an ice bath, and the mixture was reacted for 1 h. The reaction solution was added to 100 mL of a saturated aqueous ammonium chloride solution to quench the reaction, and the mixture was extracted with 100 mL of dichloromethane. The organic phase was separated, dried over anhydrous sodium sulfate, filtered, and concentrated. The concentrate was separated and purified by silica gel column chromatography to give intermediate **98f** (1.40 g).

MS(ESI, $[M+H]^+$) *m/z:* 354.35.

$^1$H NMR (500 MHz, DMSO-$d_6$) δ 11.18 (s, 1H), 7.89 - 7.70 (m, 2H), 7.48 (dd, *J*= 9.1, 1.5 Hz, 1H), 6.60 (dd, *J* = 6.2, 3.1 Hz, 1H), 5.27 - 4.93 (m, 1H), 4.76 - 4.62 (m, 2H), 3.55 (dt, *J* = 79.3, 5.5 Hz, 2H), 3.30 (d, *J* = 9.6 Hz, 1H), 2.85 (ddd, *J* = 17.4, 12.1, 5.4 Hz, 1H), 2.67 - 2.51 (m, 3H), 2.47 - 2.38 (m, 2H), 2.22 (ddd, *J* = 13.6, 10.8, 6.6 Hz, 2H).

Step 6: Preparation of intermediate **98g**

Intermediate **98f** (1.2 g), acetonitrile (10.00 mL), and IBX oxidant (1.43 g) were added to a reaction flask in sequence, and the mixture was reacted at 80 °C. After the reaction was completed, as confirmed by TLC, the reaction solution was filtered, and the filtrate was concentrated to give intermediate **98g** (1.39 g).

MS(ESI, $[M+H]^+$) *m/z:* 352.36.

Step 7: Preparation of compounds **98-1** and **98-2**

Intermediate **1i** (480 mg), intermediate **98g** (500 mg), dichloroethane (10 mL), isopropanol (2 mL), and glacial acetic acid (35.6 mg, 0.034 mL) were added to a reaction flask. After the mixture was stirred at room temperature for 30 min, sodium triacetoxyborohydride (503 mg) was added, and the mixture was stirred overnight at room temperature. After the reaction was completed, 100 mL of water was added to the reaction solution, and the mixture was extracted twice with 100 mL of dichloromethane. The extracts were combined, dried over anhydrous sodium sulfate, filtered under vacuum, and concentrated, and the residue was purified by silica gel column chromatography to give compound **98h** (544 mg). Compound **98h** was separated by high performance liquid chromatography to give compound **98-1** (248 mg) and compound **98-2** (165 mg) in sequence. The conditions of preparative chromatography were as follows:

Instrument and preparative column: Shimadzu LC-20AD high performance liquid chromatograph was used, and the preparative column was Uitimate XB-Phenyl (4.6×250 mm, 10 μm). Mobile phase system: acetonitrile/10 mM ammonium acetate, isocratic elution: acetonitrile/10 mM ammonium acetate = 60/40.

MS(ESI, [M+H]+) m/z: 814.66.

Compound **98-1:** $^1$H NMR (500 MHz, DMSO-$d_6$) δ 11.20 (d, *J* = 19.5 Hz, 2H), 7.84 (d, *J* = 9.1 Hz, 1H), 7.78 (dd, *J* = 12.5, 3.0 Hz, 2H), 7.66 (s, 1H), 7.51 (dd, *J* = 11.1, 8.3 Hz, 3H), 7.36 - 7.31 (m, 1H), 7.17 (d, *J*= 8.2 Hz, 2H), 6.61 (d, *J* = 3.1 Hz, 1H), 5.05 - 4.95 (m, 1H), 4.70 (dd, *J* = 11.8, 5.1 Hz, 1H), 4.35 (d, *J* = 12.3 Hz, 1H), 4.32 - 4.25 (m, 1H), 3.62 (tt, *J* = 10.8, 4.3 Hz, 1H), 3.30 - 3.20 (m, 4H), 3.04 (t, *J* = 11.7 Hz, 2H), 2.96 (t, *J* = 12.5 Hz, 2H), 2.86 (ddd, *J* = 17.2, 12.1, 5.4 Hz, 2H), 2.76 (s, 2H), 2.71 (s, 3H), 2.63 (dt, *J* = 17.1, 4.2 Hz, 2H), 2.43 (td, *J* = 12.6, 4.6 Hz, 2H), 2.23 (dq, *J* = 13.6, 4.8 Hz, 2H), 2.15 (s, 3H), 1.87 - 1.65 (m, 7H), 1.56 (d, *J* = 12.1 Hz, 2H). Compound 98-2: $^1$H NMR (500 MHz, DMSO-$d_6$) δ 11.19 (d, *J* = 4.6 Hz, 2H), 7.89 - 7.83 (m, 2H), 7.76 (d, *J* = 2.9 Hz, 1H), 7.66 (s, 1H), 7.49 (dd, *J* = 8.7, 5.8 Hz, 3H), 7.33 (d, *J* = 2.9 Hz, 1H), 7.17 (d, *J* = 8.2 Hz, 2H), 6.61 (d, *J* = 3.1 Hz, 1H), 5.30 (p, *J* = 7.6 Hz, 1H), 4.70 (dd, *J*= 11.9, 5.1 Hz, 1H), 4.36 (d, *J* = 12.4 Hz, 1H), 4.28 (d, *J* = 13.2 Hz, 1H), 3.61 (ddt, *J* = 11.1, 8.5, 4.2 Hz, 1H), 3.29 (d, *J* = 7.7 Hz, 2H), 3.25 (dd, *J* = 9.7, 7.2 Hz, 2H), 2.99 (dt, *J* = 37.5, 12.1 Hz, 4H), 2.85 (ddd, *J* = 17.3, 12.2, 5.4 Hz, 1H), 2.71 (s, 3H), 2.66 - 2.60 (m, 3H), 2.59 - 2.54 (m, 2H), 2.47 - 2.35 (m, 4H), 2.23 (dq, *J* = 13.7, 4.9 Hz, 1H), 2.05 (d, *J*= 27.3 Hz, 2H), 1.85 - 1.72 (m, 5H), 1.70 - 1.50 (m, 4H).

**Example 99:** Synthesis of Compounds **99-1** and **99-2**

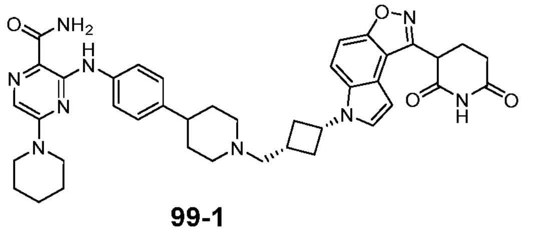

**99-1**

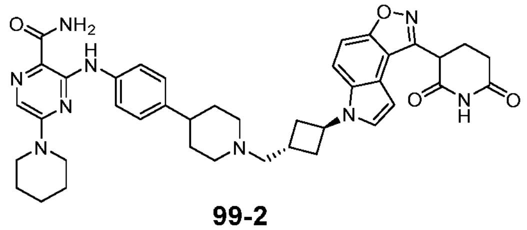

**99-2**

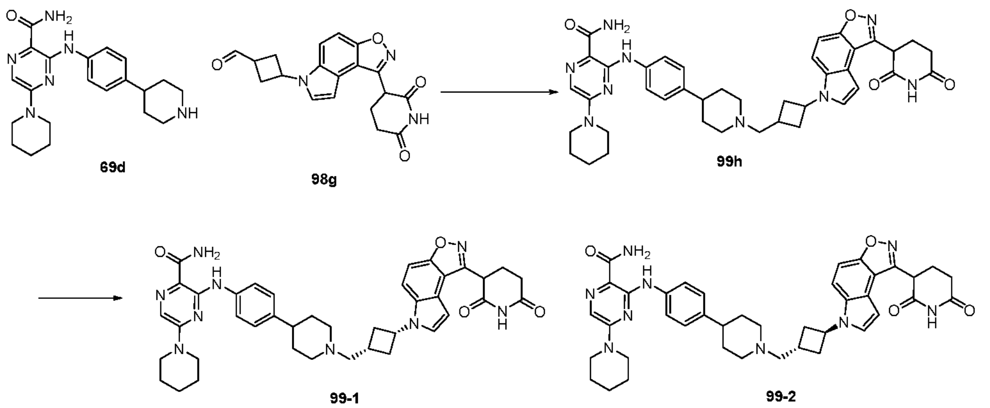

69d 98g 99h

99-1 99-2

Step 1: Preparation of compounds **99-1** and **99-2**

Intermediate **69d** (200 mg), intermediate **98g** (200 mg), dichloroethane (10 mL), isopropanol (2 mL), and glacial acetic acid (14.2 mg, 0.013 mL) were added to a reaction flask. After the mixture was stirred at room temperature (30 min), sodium cyanoborohydride (503 mg) was added, and the mixture was stirred overnight at room temperature. After the reaction was completed, 100 mL of water was added to the reaction solution, and the mixture was extracted twice with 100 mL of dichloromethane. The extracts were combined, dried over anhydrous sodium sulfate, filtered under vacuum, and concentrated, and the residue was purified by silica gel column chromatography to give compound **99h** (200 mg). Compound **99h** was separated by high performance liquid chromatography to give compound **99-1** (95 mg) and compound **99-2** (37 mg) in sequence. The conditions of preparative chromatography were as follows:

Instrument and preparative column: Shimadzu LC-20AD high performance liquid chromatograph was used, and the preparative column was Uitimate XB-Phenyl (4.6×250 mm, 10 μm). Mobile phase system: acetonitrile/10 mM ammonium acetate, isocratic elution: acetonitrile/10 mM ammonium acetate = 60/40.

MS(ESI, $[M+H]^+$) *m/z:* 716.44

Compound **99-1:** $^1$H NMR (500 MHz, DMSO-$d_6$) δ 11.29 (s, 1H), 11.18 (s, 1H), 7.87 - 7.77 (m, 2H), 7.74 *(d, J*= 2.9 Hz, 1H), 7.66 (s, 1H), 7.51 (dd, *J* = 19.4, 8.5 Hz, 3H), 7.32 (d, *J* = 2.9 Hz, 1H), 7.19 (d, *J* = 8.1 Hz, 2H), 6.60 (d, *J* = 3.1 Hz, 1H), 4.99 (p, *J* = 8.5 Hz, 1H), 4.70 (dd, *J* = 11.9, 5.1 Hz, 1H), 3.67 (t, *J* = 5.3 Hz, 4H), 2.98 (s, 2H), 2.85 (td, *J* = 12.1, 6.0 Hz, 1H), 2.74 (d, *J* = 9.5 Hz, 2H), 2.63 (dt, *J* = 17.1, 4.2 Hz, 1H), 2.43 (tt, *J* = 12.2, 6.2 Hz, 3H), 2.23 (dq, *J* = 13.8, 4.9 Hz, 1H), 2.18 - 1.97 (m, 4H), 1.76 (s, 2H), 1.66 (q, *J* = 6.0 Hz, 4H), 1.59 (q, *J* = 5.5 Hz, 4H), 1.23 (s, 2H).

Compound **99-2:** $^1$H NMR (500 MHz, DMSO-$d_6$) δ 11.29 (s, 1H), 11.19 (s, 1H), 7.85 (d, *J* = 9.5 Hz, 2H), 7.74 (s, 1H), 7.66 (s, 1H), 7.51 (dd, *J* = 18.6, 8.4 Hz, 3H), 7.32 (s, 1H), 7.19 (d, *J* = 8.1 Hz, 2H), 6.61 (d, *J* = 3.3 Hz, 1H), 5.29 (p, *J* = 7.5 Hz, 1H), 4.70 (dd, *J* = 11.7, 5.1 Hz, 1H), 3.68 (d, *J* = 5.5 Hz, 4H), 3.04 (s, 2H), 2.86 (ddd, *J* = 17.1, 11.7, 5.3 Hz, 1H), 2.73 - 2.60 (m, 6H), 2.42 (dt, *J*= 19.5, 11.1 Hz, 3H), 2.28 - 2.20 (m, 1H), 2.04 (d, *J* = 52.3 Hz, 2H), 1.78 (d, *J* = 11.3 Hz, 2H), 1.72 - 1.63 (m, 4H), 1.59 (s, 4H).

**Example 100:** Synthesis of Compound **100**

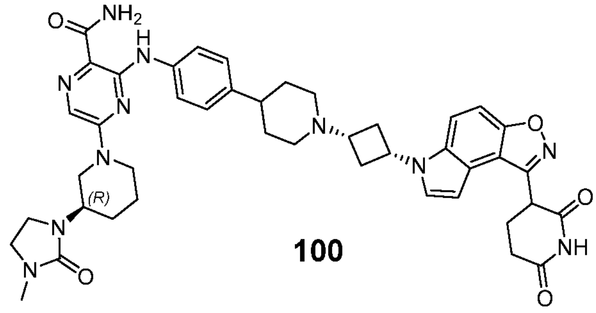

100

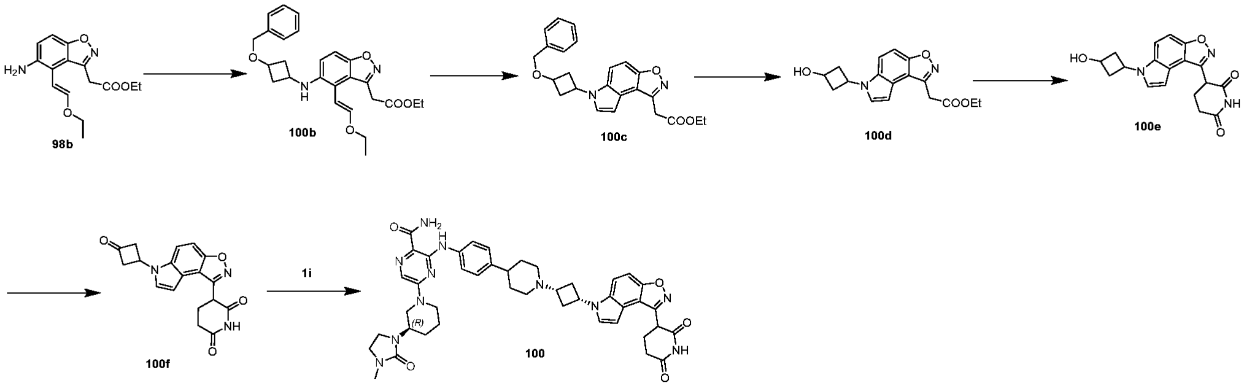


98b 100b 100c 100d 100e 100f 100

Step 1: Preparation of intermediate **100b**

Intermediate **98b** (5.3 g), 3-(benzyloxy)-1-cyclobutanone (3.38 g), methanol (50 mL), and glacial acetic acid (0.384 g) were added to a reaction flask. After the mixture was stirred at room temperature for 5 min, sodium cyanoborohydride (1.606 g) was added, and the mixture was stirred overnight at room temperature. After the reaction was completed, the reaction solution was extracted twice with 300 mL of water and 300 mL of dichloromethane. The extracts were combined, dried over anhydrous sodium sulfate, filtered under vacuum, and concentrated, and the residue was purified by silica gel column chromatography to give intermediate **100b** (3.95 g).

MS(ESI, [M+H]+) m/z: 451.38.

$^1$H NMR (500 MHz, DMSO-$d_6$) δ 7.41 - 7.37 (m, 1H), 7.35 - 7.32 (m, 4H), 7.30 - 7.28 (m, 1H), 6.91 - 6.76 (m, 1H), 6.67 (t, *J* = 13.3 Hz, 1H), 5.65 (dd, *J* = 13.0, 7.8 Hz, 1H), 4.92 (dd, *J* = 10.5, 6.5 Hz, 1H), 4.42 - 4.37 (m, 2H), 4.21 - 4.11 (m, 2H), 4.10 - 4.04 (m, 4H), 3.94 (q, *J* = 7.0 Hz, 2H), 2.73 (dtdd, *J* = 9.2, 6.7, 4.8, 2.9 Hz, 2H), 2.37 (tt, *J* = 10.7, 4.9 Hz, 1H), 1.85 (qd, *J*= 8.5, 2.8 Hz, 1H), 1.30 (td, *J*= 7.0, 1.6 Hz, 3H), 1.20 - 1.17 (m, 3H).

Step 2: Preparation of intermediate **100c**

Intermediate **100b** (3.95 g), dichloromethane (40 mL), and hydrochloric acid (6.58 mL, 4 mol/L) were added to a reaction flask in sequence, and the mixture was reacted at room temperature. After the reaction was completed, as confirmed by TLC, 200 mL of an aqueous NaHCOs solution and 200 mL of dichloromethane were added to the reaction solution. The organic phase was separated, dried over anhydrous sodium sulfate, filtered, and concentrated. The concentrate was separated and purified by silica gel column chromatography to give intermediate **100c** (2.51 g).

MS(ESI, [M+H]$^+$) m/z: 405.3

$^1$H NMR (500 MHz, DMSO-$d_6$) δ 7.87 - 7.75 (m, 2H), 7.47 (dd, *J*= 9.1, 7.2 Hz, 1H), 7.41 - 7.27 (m, 6H), 6.69 (t, *J* = 3.6 Hz, 1H), 4.48 (s, 2H), 4.42 - 4.33 (m, 1H), 4.26 (d, *J* = 1.4 Hz, 2H), 4.14 (qd, *J* = 7.1, 2.3 Hz, 2H), 4.05 - 3.96 (m, 1H), 2.97 (dtd, *J* = 9.1, 6.7, 2.9 Hz, 1H), 2.69 (dt, *J* = 8.5, 5.4 Hz, 1H), 2.34 (ddd, *J* = 11.7, 9.1, 7.4 Hz, 1H), 1.18 (td, *J* = 7.1, 3.0 Hz, 3H).

Step 3: Preparation of intermediate **100d**

Intermediate **100c** (1.5 g), 10% palladium on carbon catalyst (0.75 g), MeOH (30 mL), and hydrochloric acid (0.926 mL, 4 mol/L) were added to a reaction flask in sequence, and the mixture was reacted at room temperature under $H_2$ atmosphere. After the reaction was completed, as confirmed by TLC, the reaction solution was filtered under vacuum,

and the filtrate was concentrated and separated and purified by silica gel column chromatography to give intermediate **100d** (0.765 g).

MS(ESI, $[M+H]^+$) m/z: 315.2
$^1$H NMR (500 MHz, DMSO-$d_6$) δ 7.83 - 7.77 (m, 1H), 7.77 - 7.71 (m, 1H), 7.46 (dd, *J* = 9.1, 4.1 Hz, 1H), 6.67 (t, *J* = 2.8 Hz, 1H), 5.25 (tt, *J* = 8.2, 5.8 Hz, 1H), 4.61 (tt, *J* = 9.3, 7.2 Hz, 1H), 4.31 - 4.24 (m, 2H), 4.12 (dq, *J* = 13.5, 7.1 Hz, 2H), 4.07 - 4.00 (m, 1H), 2.93 (dtd, *J* = 9.7, 6.9, 2.9 Hz, 1H), 2.71 - 2.59 (m, 1H), 2.53 (dd, *J* = 8.2, 4.2 Hz, 1H), 2.29 - 2.22 (m, 1H), 1.18 (td, *J*= 7.1, 1.5 Hz, 3H).

Step 4: Preparation of intermediate **100e**

Intermediate **100d** (1.7 g), acrylamide (0.461 g), and anhydrous tetrahydrofuran (30 mL) were added to a reaction flask in sequence. Potassium tert-butoxide (0.91 g) was slowly added under an ice bath, and the mixture was reacted for 1 h. The reaction solution was added to 100 mL of a saturated aqueous ammonium chloride solution to quench the reaction, and the mixture was extracted with 100 mL of dichloromethane. The organic phase was separated, dried over anhydrous sodium sulfate, filtered, and concentrated. The concentrate was separated and purified by silica gel column chromatography to give intermediate **100e** (0.84 g).
MS(ESI, $[M+H]^+$) m/z: 340.2.

Step 5: Preparation of intermediate **100f**

Intermediate **100e** (400 mg), acetonitrile (10.00 mL), and IBX oxidant (353 mg) were added to a reaction flask in sequence, and the mixture was reacted at 80 °C. After the reaction was completed, as confirmed by TLC, the reaction solution was directly filtered to remove solids, and the filtrate was concentrated to give intermediate **100f** (410 mg).
MS(ESI, $[M+H]^+$) m/z: 338.3.

Step 6: Preparation of compound **100**

Intermediate **1i** (200 mg), intermediate **100f** (165 mg), dichloroethane (10 mL), isopropanol (2 mL), and glacial acetic acid (11.29 mg, 0.011 mL) were added to a reaction flask. After the mixture was stirred at room temperature for 15 min, sodium cyanoborohydride (47.27 mg) was added, and the mixture was stirred overnight at room temperature. After the reaction was completed, 100 mL of water was added to the reaction solution, and the mixture was extracted twice with 100 mL of dichloromethane. The extracts were combined, dried over anhydrous sodium sulfate, filtered under vacuum, and concentrated, and the residue was purified by silica gel column chromatography to give 160 mg of a mixture. The mixture was separated by high performance liquid chromatography to give compound **100** (62 mg). The conditions of preparative chromatography were as follows: Instrument and preparative column: Shimadzu LC-20AD high performance liquid chromatograph was used, and the preparative column was Uitimate XB-Phenyl (4.6×250 mm, 10 μm). Mobile phase system: acetonitrile/10 mM ammonium acetate, isocratic elution: acetonitrile/10 mM ammonium acetate = 60/40.
MS(ESI, [M+H]+) m/z: 800.43.
$^1$H NMR (500 MHz, DMSO-$d_6$) δ 11.20 (d, *J* = 11.3 Hz, 2H), 7.87 (d, *J* = 8.7 Hz, 1H), 7.77 (s, 2H), 7.66 (s, 1H), 7.51 (d, *J*= 8.2 Hz, 3H), 7.34 (s, 1H), 7.17 (d, *J*= 8.0 Hz, 2H), 6.62 (s, 1H), 4.87 (s, 1H), 4.70 (dd, *J*= 11.8, 5.1 Hz, 1H), 4.35 (d, *J* = 12.7 Hz, 1H), 4.29 (d, *J* = 12.7 Hz, 1H), 3.66 - 3.58 (m, 1H), 3.26 (p, *J* = 7.6, 6.6 Hz, 2H), 3.11 - 2.94 (m, 4H), 2.93 - 2.75 (m, 4H), 2.75 - 2.67 (m, 4H), 2.64 (dd, *J* = 17.3, 3.9 Hz, 1H), 2.44 (dd, *J* = 12.6, 4.6 Hz, 2H), 2.30 - 2.16 (m, 3H), 2.08 (d, *J*= 1.8 Hz, 1H), 1.92 (s, 2H), 1.79 (d, *J*= 14.8 Hz, 5H), 1.68 - 1.53 (m, 3H).

**Example 101:** Synthesis of Compound **101**

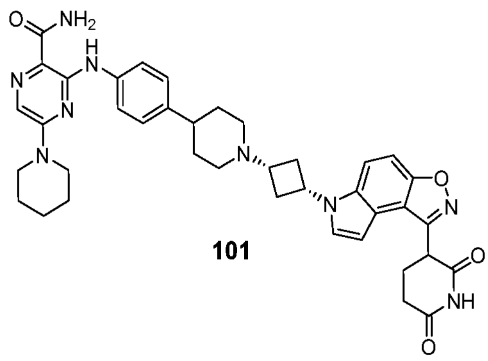

101

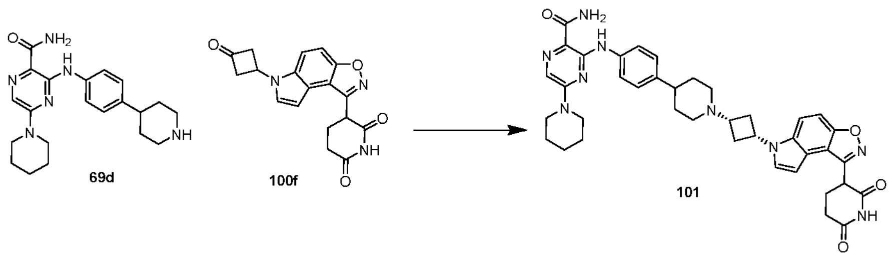

Step 1: Preparation of compound **101**

Intermediate **69d** (150 mg), intermediate **100f** (160 mg), dichloroethane (10 mL), isopropanol (2 mL), and glacial acetic acid (11.84 mg, 0.011 mL) were added to a reaction flask. After the mixture was stirred at room temperature (30 min), sodium cyanoborohydride (74.3 mg) was added, and the mixture was stirred overnight at room temperature. After the reaction was completed, 100 mL of water was added to the reaction solution, and the mixture was extracted twice with 100 mL of dichloromethane. The extracts were combined, dried over anhydrous sodium sulfate, filtered under vacuum, and concentrated, and the residue was purified by silica gel column chromatography to give 120 mg of a mixture. The mixture was separated by high performance liquid chromatography to give compound **101** (49 mg). The conditions of preparative chromatography were as follows: Instrument and preparative column: Shimadzu LC-20AD high performance liquid chromatograph was used, and the preparative column was Uitimate XB-Phenyl (4.6×250 mm, 10 μm). Mobile phase system: acetonitrile/10 mM ammonium acetate, isocratic elution: acetonitrile/10 mM ammonium acetate = 60/40.

MS(ESI, [M+H]+) m/z: 702.38.

$^1$H NMR (500 MHz, DMSO-$d_6$) δ 11.29 (s, 1H), 11.19 (s, 1H), 7.87 (d, *J* = 9.2 Hz, 1H), 7.75 (dd, *J* = 15.7, 3.0 Hz, 2H), 7.66 (s, 1H), 7.51 (dd, *J* = 10.3, 8.7 Hz, 3H), 7.31 (d, *J* = 2.7 Hz, 1H), 7.19 (d, *J* = 8.3 Hz, 2H), 6.61 (d, *J* = 3.1 Hz, 1H), 4.85 (p, *J* = 8.5 Hz, 1H), 4.70 (dd, *J* = 11.9, 5.1 Hz, 1H), 3.67 (t, *J* = 5.3 Hz, 4H), 2.98 (d, *J* = 10.6 Hz, 2H), 2.86 (td, *J* = 12.0, 6.0 Hz, 1H), 2.79 (q, *J* = 9.8, 8.1 Hz, 2H), 2.65 (ddd, *J* = 17.4, 12.7, 5.7 Hz, 2H), 2.49 - 2.38 (m, 2H), 2.28 - 2.16 (m, 3H), 1.91 (t, *J*= 11.2 Hz, 2H), 1.78 (d, *J*= 11.5 Hz, 2H), 1.65 (dd, *J*= 9.5, 4.7 Hz, 3H), 1.59 (h, *J* = 5.0, 4.5 Hz, 5H).

**Example 102:** Synthesis of Compounds **102-1** and **102-2**

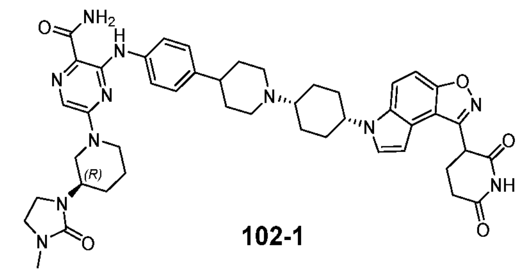

102-1

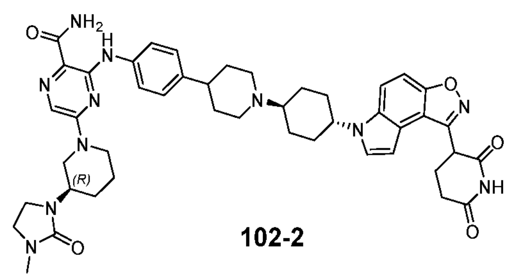

102-2

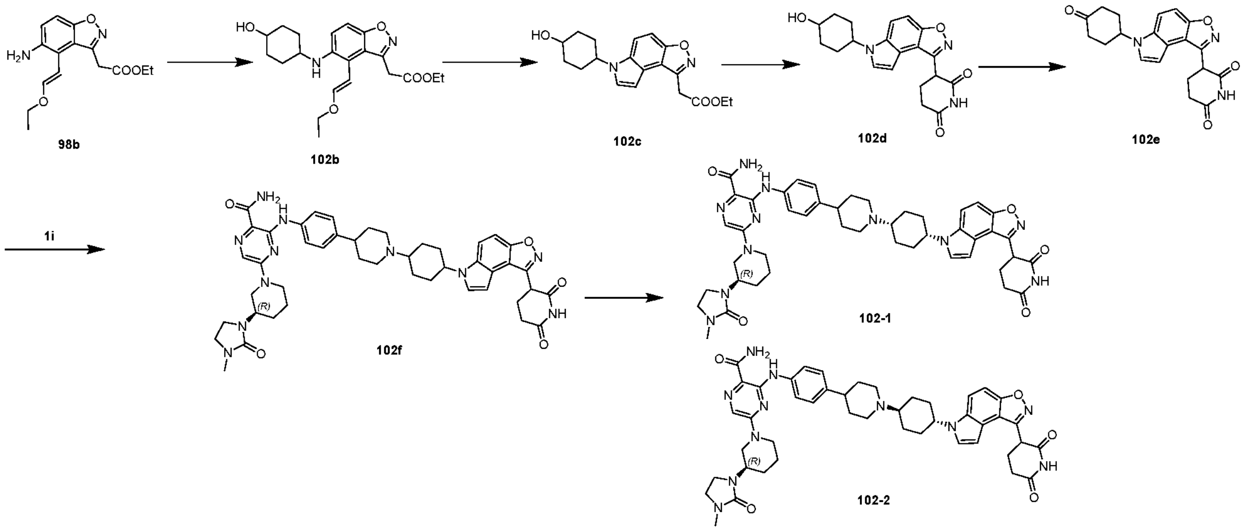


98b 102b 102c 102d 102e 102f 102-1 102-2

Step 1: Preparation of intermediate **102b**

Intermediate **98b** (1.0 g), 4-hydroxycyclohexanone (0.662 g), methanol (20 mL), and glacial acetic acid (0.384 g) were added to a reaction flask. After the mixture was stirred at room temperature for 5 min, sodium cyanoborohydride (1.606 g) was added, and the mixture was stirred overnight at room temperature. After the reaction was completed, 100 mL of water was added to the reaction solution, and the mixture was extracted twice with 100 mL of dichloromethane. The extracts were combined, dried over anhydrous sodium sulfate, filtered under vacuum, and concentrated, and the residue was purified by silica gel column chromatography to give intermediate **102b** (0.71 g).

MS(ESI, $[M+H]^+$) m/z: 389.31.

$^1$H NMR (500 MHz, DMSO-$d_6$) $\delta$ 7.44 - 7.38 (m, 1H), 7.05 (d, *J* = 9.1 Hz, 1H), 6.66 (d, *J* = 13.1 Hz, 1H), 5.63 (d, *J* = 13.1 Hz, 1H), 4.47 - 4.38 (m, 1H), 4.27 (d, *J* = 8.5 Hz, 1H), 4.11 - 4.04 (m, 4H), 3.94 (q, *J* = 7.1 Hz, 2H), 3.70 (q, *J* = 4.0 Hz, 1H), 3.38 (d, *J* = 7.4 Hz, 1H), 2.37 (ddd, *J* = 14.0, 8.2, 5.9 Hz, 1H), 2.22 (ddd, *J*= 15.8, 8.5, 5.8 Hz, 1H), 1.95 - 1.86 (m, 1H), 1.77 - 1.70 (m, 1H), 1.62 (q, *J* = 6.4, 6.0 Hz, 2H), 1.54 (dt, *J* = 6.7, 3.5 Hz, 2H), 1.30 (t, *J* = 7.1 Hz, 3H), 1.19 (d, *J* = 6.9 Hz, 3H).

Step 2: Preparation of intermediate **102c**

Intermediate **102b** (0.45 g), dichloromethane (10 mL), and hydrochloric acid (0.87 mL, 4 mol/L) were added to a reaction flask in sequence, and the mixture was reacted at room temperature for 4 h. 50 mL of an aqueous NaHCOs solution and 50 mL of dichloromethane were added to the reaction solution. The organic phase was separated, dried over anhydrous sodium sulfate, filtered, and concentrated. The concentrate was separated and purified by silica gel column chromatography to give intermediate **102c** (0.24 g).

MS(ESI, $[M+H]^+$) *m/z:* 343.3

$^1$H NMR (500 MHz, DMSO-$d_6$) $\delta$ 7.90 (d, *J* = 9.1 Hz, 1H), 7.67 (d, *J* = 3.1 Hz, 1H), 7.50 - 7.44 (m, 1H), 6.64 (d, *J* = 3.1 Hz, 1H), 4.56 - 4.48 (m, 2H), 4.26 (s, 2H), 4.14 (d, *J* = 7.1 Hz, 2H), 3.94 (p, *J* = 3.1 Hz, 1H), 2.16 (qd, *J* = 12.9, 3.6 Hz, 2H), 1.82 (dt, *J*= 9.5, 3.1 Hz, 2H), 1.78 - 1.70 (m, 4H), 1.20 - 1.17 (m, 3H).

Step 3: Preparation of intermediate **102d**

Intermediate **102c** (0.25 g), acrylamide (0.057 g), and anhydrous tetrahydrofuran (10 mL) were added to a reaction flask in sequence. Potassium tert-butoxide (0.09 g) was slowly added under an ice bath, and the mixture was reacted for 1 h. The reaction solution was added to 50 mL of a saturated aqueous ammonium chloride solution to quench the reaction, and the mixture was extracted with 50 mL of dichloromethane. The organic phase was separated, dried over anhydrous sodium sulfate, filtered, and concentrated. The concentrate was separated and purified by silica gel column chromatography to give intermediate **102d** (0.21 g).

MS(ESI, $[M+H]^+$) *m/z:* 368.2.

$^1$H NMR (500 MHz, DMSO-$d_6$) $\delta$ 11.18 (s, 1H), 7.92 (d, *J* = 9.2 Hz, 1H), 7.70 - 7.66 (m, 1H), 7.50 - 7.46 (m, 1H), 6.56 (d, *J*= 3.1 Hz, 1H), 4.68 (dd, *J* = 11.9, 5.1 Hz, 1H), 4.57 - 4.50 (m, 2H), 3.94 (q, *J* = 3.0 Hz, 1H), 2.85 (ddd, *J* = 17.4, 12.2, 5.4 Hz, 1H), 2.63 (dt, *J* = 17.3, 4.1 Hz, 1H), 2.43 (td, *J* = 12.7, 4.6 Hz, 1H), 2.27 - 2.09 (m, 4H), 1.83 (d, *J*

= 13.0 Hz, 3H), 1.78 - 1.69 (m, 2H).

Step 4: Preparation of intermediate **102e**

Intermediate **102d** (2.3 g), acetonitrile (10.00 mL), and IBX oxidant (2.104 g, 7.51 mmol) were added to a reaction flask in sequence, and the mixture was reacted at 80 °C for 1 h. The reaction solution was directly filtered to remove solids, and the filtrate was concentrated to give intermediate **102e** (1.63 g).

MS(ESI, $[M+H]^+$) m/z: 366.3.

$^1H$ NMR (500 MHz, DMSO-$d_6$) δ 11.18 (s, 1H), 8.04 (d, *J*= 9.1 Hz, 1H), 7.76 - 7.71 (m, 1H), 7.54 (d, *J*= 9.1 Hz, 1H), 6.59 (dd, *J* = 8.9, 3.2 Hz, 1H), 5.15 (tt, *J* = 11.1, 4.2 Hz, 1H), 4.73 - 4.64 (m, 1H), 3.17 (d, *J* = 3.7 Hz, 1H), 2.90 - 2.75 (m, 3H), 2.63 (dt, *J* = 16.3, 3.7 Hz, 1H), 2.44 (tt, *J* = 12.3, 6.1 Hz, 1H), 2.38 - 2.29 (m, 3H), 2.24 (tt, *J* = 9.1, 3.9 Hz, 3H).

Step 5: Preparation of compounds **102-1** and **102-2**

Intermediate 1i (200 mg), intermediate **102e** (183 mg), dichloroethane (10 mL), isopropanol (2 mL), and glacial acetic acid (13 mg, 0.209 mmol) were added to a reaction flask. After the mixture was stirred at room temperature for 5 min, sodium cyanoborohydride (53 mg, 0.836 mmol) was added, and the mixture was stirred overnight at room temperature. After the reaction was completed, 100 mL of water was added to the reaction solution, and the mixture was extracted twice with 100 mL of dichloromethane. The extracts were combined, dried over anhydrous sodium sulfate, filtered under vacuum, and concentrated, and the residue was purified by silica gel column chromatography to give compound **102f** (110 mg), which was then purified by $C_{18}$ reversed-phase column chromatography (10 nM aqueous ammonium acetate solution-acetonitrile = 90%:10% → 10%:90%) to give compound **102-1** (34 mg) and compound **102-2** (35 mg) in sequence.

Compound **102-1:**
Q-TOF(ESI, $[M+H]^+$) *m/z:* 828.4345
$^1H$ NMR (500 MHz, DMSO-$d_6$) δ 11.19 (d, *J* = 12.7 Hz, 2H), 7.94 (d, *J* = 9.3 Hz, 1H), 7.76 (d, *J* = 2.9 Hz, 1H), 7.72 - 7.64 (m, 2H), 7.50 (dd, *J* = 8.8, 6.6 Hz, 3H), 7.34 (d, *J* = 2.9 Hz, 1H), 7.17 (d, *J* = 8.2 Hz, 2H), 6.57 (d, *J* = 3.1 Hz, 1H), 4.68 (dd, *J* = 11.9, 5.2 Hz, 1H), 4.54 (dq, *J* = 11.6, 6.7, 5.6 Hz, 1H), 4.36 (d, *J* = 11.4 Hz, 1H), 4.29 (d, *J* = 12.7 Hz, 1H), 3.62 (tt, *J* = 10.6, 4.3 Hz, 1H), 3.26 (dd, *J* = 9.7, 7.2 Hz, 2H), 3.00 (dt, *J* = 41.7, 12.9 Hz, 4H), 2.85 (td, *J* = 12.2, 6.1 Hz, 1H), 2.73 (d, *J* = 3.9 Hz, 3H), 2.63 (dt, *J* = 17.1, 4.1 Hz, 2H), 2.43 (td, *J* = 12.6, 4.5 Hz, 3H), 2.22 (dq, *J* = 13.7, 4.6 Hz, 1H), 2.10 - 2.03 (m, 2H), 2.02 - 1.85 (m, 5H), 1.80 (q, *J*= 12.2 Hz, 5H), 1.73 - 1.51 (m, 6H).
Compound **102-2:**
Q-TOF(ESI, $[M+H]^+$) *m/z:* 828.4330
$^1H$ NMR (500 MHz, DMSO-$d_6$) δ 11.20 (d, *J* = 11.9 Hz, 2H), 7.97 (d, *J*= 9.2 Hz, 1H), 7.76 (s, 1H), 7.67 (s, 2H), 7.51 (dd, *J* = 18.0, 8.5 Hz, 3H), 7.34 (d, *J* = 2.9 Hz, 1H), 7.21 (d, *J* = 8.1 Hz, 2H), 6.59 (d, *J* = 3.0 Hz, 1H), 4.70 (dd, *J* = 11.8, 5.2 Hz, 2H), 4.38 (s, 1H), 4.30 (d, *J* = 13.1 Hz, 1H), 3.67 - 3.59 (m, 1H), 3.39 - 3.33 (m, 2H), 3.28 - 3.21 (m, 3H), 3.00 (dt, *J* = 34.4, 12.0 Hz, 2H), 2.86 (ddd, *J* = 17.3, 12.0, 5.3 Hz, 1H), 2.71 (s, 3H), 2.63 (dt, *J* = 17.1, 4.2 Hz, 1H), 2.43 (td, *J* = 12.2, 11.7, 3.7 Hz, 2H), 2.30 (s, 1H), 2.23 (dt, *J* = 13.6, 4.8 Hz, 2H), 2.14 (d, *J* = 14.1 Hz, 3H), 2.04 - 1.91 (m, 3H), 1.87 - 1.73 (m, 7H), 1.68 (s, 3H), 1.57 (d, *J* = 12.3 Hz, 1H).

**Example 103:** Synthesis of Compounds **103-1** and **103-2**

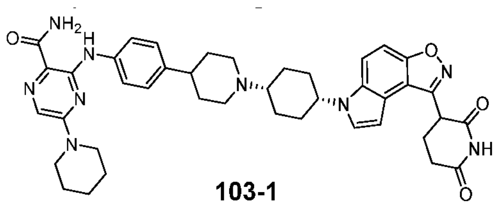
103-1

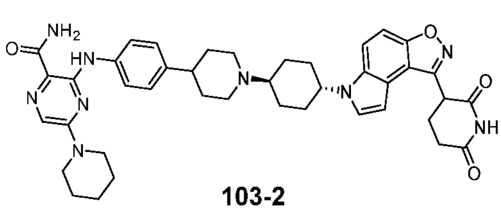
103-2

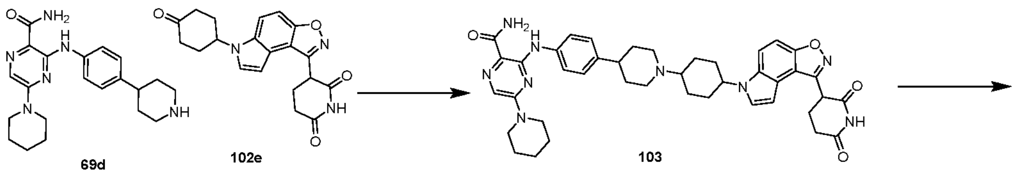
69d 102e 103

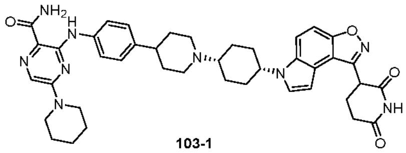
103-1

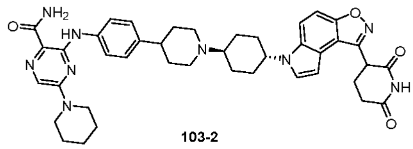
103-2

Step 1: Preparation of compounds **103-1** and **103-2**

Intermediate **69d** (220 mg), intermediate **102e** (254 mg), dichloroethane (10 mL), isopropanol (2 mL), and glacial acetic acid (17 mg, 0.289 mmol) were added to a reaction flask. After the mixture was stirred at room temperature for 5 min, sodium cyanoborohydride (73 mg, 1.156 mmol) was added, and the mixture was stirred overnight at room temperature. After the reaction was completed, 100 mL of water was added to the reaction solution, and the mixture was extracted twice with 100 mL of dichloromethane. The extracts were combined, dried over anhydrous sodium sulfate, filtered under vacuum, and concentrated, and the residue was purified by silica gel column chromatography to give compound **103** (180 mg), which was then purified by $C_{18}$ reversed-phase column chromatography (10 nM aqueous ammonium acetate solution-acetonitrile = 90%:10% → 10%:90%) to give compound **103-1** (68 mg) and compound **103-2** (48 mg) in sequence.

Compound **103-1:**
Q-TOF(ESI, $[M+H]^+$) *m/z:* 730.3833
$^1$H NMR (500 MHz, DMSO-$d_6$) δ 11.30 (s, 1H), 11.19 (s, 1H), 7.97 (d, *J* = 9.2 Hz, 1H), 7.74 (d, *J* = 2.8 Hz, 1H), 7.67 (s, 2H), 7.55 (d, *J*= 8.1 Hz, 2H), 7.49 (d, *J*= 9.0 Hz, 1H), 7.32 (d, *J*= 2.8 Hz, 1H), 7.23 (d, *J*= 8.2 Hz, 2H), 6.58 (d, *J* = 3.1 Hz, 1H), 4.69 (dt, *J* = 18.4, 9.2 Hz, 2H), 3.69 (t, *J* = 5.4 Hz, 4H), 3.20 (d, *J* = 22.9 Hz, 2H), 2.86 (ddd, *J*= 17.2, 12.0, 5.4 Hz, 1H), 2.63 (dt, *J*= 17.2, 4.2 Hz, 1H), 2.44 (tt, *J*= 12.4, 6.4 Hz, 2H), 2.30 (s, 1H), 2.28 - 2.20 (m, 2H), 2.15 (dd, *J*= 29.5, 11.9 Hz, 4H), 1.95 (s, 2H), 1.84 - 1.75 (m, 4H), 1.67 (q, *J* = 5.9 Hz, 5H), 1.60 (t, *J* = 5.5 Hz, 4H).
Compound **103-2:**
Q-TOF(ESI, $[M+H]^+$) *m/z:* 730.3840
$^1$H NMR (500 MHz, DMSO-$d_6$) δ 11.30 (s, 1H), 11.18 (s, 1H), 7.94 (d, *J* = 9.2 Hz, 1H), 7.74 (d, *J* = 3.0 Hz, 1H), 7.72 - 7.65 (m, 2H), 7.52 (dd, *J* = 19.2, 8.6 Hz, 3H), 7.32 (d, *J* = 2.9 Hz, 1H), 7.20 (d, *J* = 8.2 Hz, 2H), 6.57 (d, *J* = 3.1 Hz, 1H), 4.68 (dd, *J* = 11.9, 5.2 Hz, 1H), 4.54 (s, 1H), 3.68 (t, *J* = 5.3 Hz, 4H), 2.91 (s, 2H), 2.85 (ddd, *J* = 17.3, 12.1, 5.4 Hz, 1H), 2.63 (dt, *J* = 17.2, 4.2 Hz, 1H), 2.43 (td, *J* = 12.5, 4.4 Hz, 2H), 2.22 (dq, *J* = 13.8, 4.9 Hz, 1H), 2.06 (s, 2H), 2.04 - 1.94 (m, 2H), 1.90 (d, *J* = 11.2 Hz, 5H), 1.81 (s, 3H), 1.71 - 1.63 (m, 4H), 1.60 (q, *J* = 5.6 Hz, 4H).

**Example 104:** Synthesis of Compound **104**

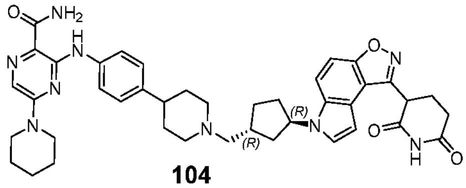
**104**

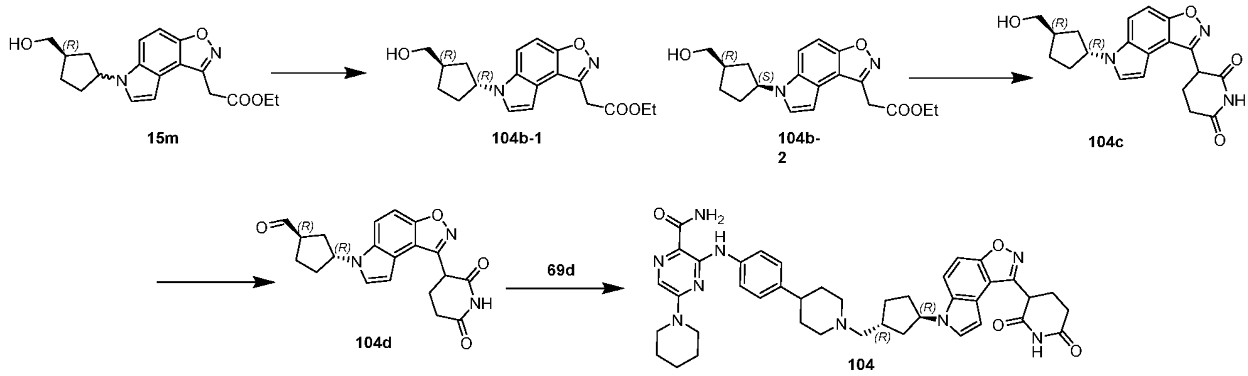


15m 104b-1 104b-2 104c 104d 104

Step 1: Preparation of intermediates **104b-1** and **104b-2**

Intermediate **15m** was separated by high performance liquid chromatography to give intermediates **104b-1** (0.77 g) and **104b-2** (1.51 g) in sequence. The conditions of preparative chromatography were as follows:
Instrument and preparative column: YMC K-prep Lab100g high pressure preparative chromatograph was used, and the preparative column was YMC Sil SLG12S11-2530 (30×250 mm, 10 $\mu$m). Mobile phase system: ethanol/n-hexane, isocratic elution: ethanol/n-hexane = 60/40.

Intermediate **104b-1:**

MS(ESI, $[M+H]^+$) *m/z:* 343.3.
$^1$H NMR (500 MHz, DMSO-$d_6$) $\delta$ 7.91 - 7.84 (m, 1H), 7.68 (d, *J* = 3.1 Hz, 1H), 7.47 (d, *J* = 9.1 Hz, 1H), 6.65 (d, *J* = 3.1 Hz, 1H), 5.05 (p, *J* = 7.1 Hz, 1H), 4.61 (t, *J* = 5.3 Hz, 1H), 4.26 (s, 2H), 4.14 (q, *J* = 7.1 Hz, 2H), 3.40 (ddd, *J* = 6.7, 5.2, 1.5 Hz, 2H), 2.42 - 2.31 (m, 1H), 2.22 (tdd, *J* = 13.3, 9.0, 6.0 Hz, 1H), 2.01 - 1.89 (m, 4H), 1.46 (tdd, *J*= 13.4, 7.5, 4.5 Hz, 1H), 1.18 (td, *J* = 7.1, 3.8 Hz, 3H).

Intermediate **104b-2:**

MS(ESI, $[M+H]^+$) *m/z:* 343.2.
$^1$H NMR (500 MHz, DMSO-$d_6$) $\delta$ 7.90 (d, *J* = 9.0 Hz, 1H), 7.72 (d, *J*= 3.2 Hz, 1H), 7.46 (d, *J*= 9.1 Hz, 1H), 6.65 (d, *J* = 3.1 Hz, 1H), 5.02 (dq, *J* = 9.5, 7.6 Hz, 1H), 4.62 (t, *J* = 5.2 Hz, 1H), 4.26 (s, 2H), 4.16 - 4.12 (m, 2H), 3.44 (ddd, *J*= 6.5, 5.2, 1.6 Hz, 2H), 2.32 (dt, *J*= 12.4, 7.3 Hz, 1H), 2.20 (dddd, *J*= 25.0, 12.5, 8.0, 6.0 Hz, 2H), 1.97 - 1.75 (m, 2H), 1.72 - 1.59 (m, 2H), 1.18 (t, *J* = 7.1 Hz, 3H).

Step 2: Preparation of intermediate **104c**

Intermediate **104b-1** (0.63 g), acrylamide (0.128 g), and anhydrous tetrahydrofuran (20 mL) were added to a reaction flask in sequence. Potassium *tert*-butoxide (0.203 g, 1.805 mmol) was slowly added under an ice bath, and the mixture was reacted for 1 h. The reaction solution was added to 100 mL of a saturated aqueous ammonium chloride solution to quench the reaction, and the mixture was extracted with 100 mL of dichloromethane. The organic phase was separated, dried over anhydrous sodium sulfate, filtered, and concentrated. The concentrate was separated and purified by silica gel column chromatography to give intermediate **104c** (0.45 g).
MS(ESI, $[M+H]^+$) *m/z:* 368.2.
$^1$H NMR (500 MHz, DMSO-$d_6$) $\delta$ 11.18 (s, 1H), 7.89 (d, *J*= 9.2 Hz, 1H), 7.69 (d, *J*= 3.1 Hz, 1H), 7.49 (d, *J*= 9.1 Hz, 1H), 6.56 (d, *J* = 3.1 Hz, 1H), 5.05 (p, *J* = 7.1 Hz, 1H), 4.69 (dd, *J* = 11.9, 5.1 Hz, 1H), 4.64 - 4.57 (m, 1H), 3.40 (ddd, *J* = 6.7, 5.3, 1.5 Hz, 2H), 2.85 (ddd, *J* = 17.3, 12.1, 5.4 Hz, 1H), 2.63 (dt, *J* = 17.3, 4.2 Hz, 1H), 2.44 (qd, *J* = 12.4, 4.4 Hz, 1H), 2.35 (dq, *J* = 13.4, 6.8, 6.3 Hz, 1H), 2.27 - 2.18 (m, 2H), 2.04 - 1.89 (m, 4H), 1.47 (tt, *J* = 11.9, 3.8 Hz, 1H).

Step 3: Preparation of intermediate **104d**

Intermediate **104c** (0.45 g), acetonitrile (20.00 mL), and IBX oxidant (0.514 g) were added to a reaction flask in sequence, and the mixture was reacted at 80 °C for 1 h. The reaction solution was directly filtered, and the filtrate was concentrated to give intermediate **104d** (0.56 g).
MS(ESI, $[M+H]^+$) *m/z:* 366.3.

Step 4: Preparation of compound **104**

Intermediate **69d** (400 mg), intermediate **104d** (499 mg), dichloroethane (20 mL), and isopropanol (4 mL) were added to a reaction flask, and then sodium triacetoxyborohydride (334 mg, 1.577 mmol) was added. The mixture was stirred overnight at room temperature. After the reaction was completed, 100 mL of water was added to the reaction solution, and the mixture was extracted twice with 100 mL of dichloromethane. The extracts were combined, dried over anhydrous sodium sulfate, filtered under vacuum, and concentrated, and the residue was purified by silica gel column chromatography and $C_{18}$ reversed-phase column chromatography to give compound **104** (266 mg).

Q-TOF(ESI, $[M+H]^+$) *m/z:* 730.3839
$^1$H NMR (500 MHz, DMSO-$d_6$) δ 11.28 (s, 1H), 11.18 (s, 1H), 7.91 (d, *J* = 9.1 Hz, 1H), 7.72 (dd, *J* = 10.2, 3.0 Hz, 2H), 7.66 (s, 1H), 7.50 (dd, *J* = 8.8, 6.8 Hz, 3H), 7.31 (d, *J* = 2.9 Hz, 1H), 7.18 (d, *J* = 8.3 Hz, 2H), 6.57 (d, *J* = 3.1 Hz, 1H), 5.12 (p, *J* = 7.1 Hz, 1H), 4.69 (dd, *J* = 11.9, 5.1 Hz, 1H), 3.67 (t, *J* = 5.3 Hz, 4H), 3.00 (s, 2H), 2.85 (ddd, *J* = 17.3, 12.1, 5.4 Hz, 1H), 2.63 (dt, *J* = 17.1, 4.2 Hz, 1H), 2.43 (td, *J* = 12.5, 4.5 Hz, 3H), 2.34 (s, 2H), 2.25 (tp, *J* = 14.0, 5.1, 4.3 Hz, 2H), 1.98 (tdd, *J* = 22.0, 13.4, 6.9 Hz, 6H), 1.73 (s, 2H), 1.68 - 1.53 (m, 8H), 1.44 (dq, *J* = 11.9, 8.0 Hz, 1H).

**Example 105:** Synthesis of Compound **105**

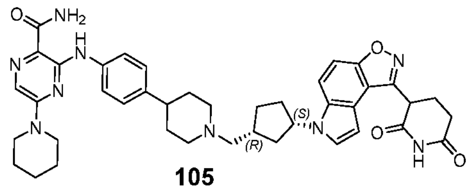

**105**

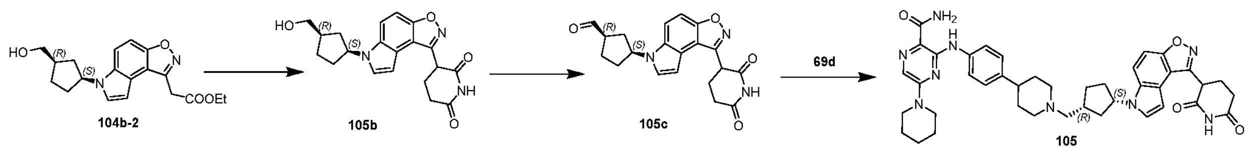


104b-2 105b 105c 105

Step 1: Preparation of intermediate **105b**

Intermediate **104b-2** (0.35 g), acrylamide (0.078 g), and anhydrous tetrahydrofuran (20 mL) were added to a reaction flask in sequence. Potassium *tert*-butoxide (0.123 g, 1.1 mmol) was slowly added under an ice bath, and the mixture was reacted for 1 h. The reaction solution was added to 100 mL of a saturated aqueous ammonium chloride solution to quench the reaction, and the mixture was extracted with 100 mL of dichloromethane. The organic phase was separated, dried over anhydrous sodium sulfate, filtered, and concentrated. The concentrate was separated and purified by silica gel column chromatography to give intermediate **105b** (0.27 g).
MS(ESI, $[M+H]^+$) *m/z:* 368.2.
$^1$H NMR (500 MHz, DMSO-$d_6$) δ 11.18 (s, 1H), 7.91 (d, *J* = 9.2 Hz, 1H), 7.72 (d, *J* = 3.2 Hz, 1H), 7.48 (d, *J* = 9.1 Hz, 1H), 6.57 (d, *J* = 3.1 Hz, 1H), 5.02 (p, *J* = 8.1 Hz, 1H), 4.69 (dd, *J* = 11.9, 5.1 Hz, 1H), 4.62 (t, *J* = 5.2 Hz, 1H), 3.46 - 3.41 (m, 2H), 2.85 (ddd, *J* = 17.4, 12.1, 5.4 Hz, 1H), 2.63 (dt, *J* = 17.2, 4.2 Hz, 1H), 2.44 (qd, *J* = 12.2, 4.4 Hz, 1H), 2.31 (dt, *J* = 12.2, 7.3 Hz, 1H), 2.21 (ddt, *J* = 26.5, 12.5, 5.5 Hz, 3H), 1.94 - 1.79 (m, 2H), 1.64 (tdd, *J* = 12.4, 5.9, 3.3 Hz, 2H).

Step 2: Preparation of intermediate **105c**

Intermediate **105b** (0.27 g), acetonitrile (20.00 mL), and IBX oxidant (0.309 g) were added to a reaction flask in sequence, and the mixture was reacted at 80 °C for 1 h. The reaction solution was directly filtered, and the filtrate was concentrated to give intermediate **105c** (0.25 g).
MS(ESI, $[M+H]^+$) *m/z:* 366.3.

Step 3: Preparation of compound **105**

Intermediate **69d** (270 mg), intermediate **105c** (389 mg), dichloroethane (20 mL), and isopropanol (4 mL) were added to a reaction flask, and then sodium triacetoxyborohydride (301 mg, 1.419 mmol) was added. The mixture was stirred overnight at room temperature. After the reaction was completed, 100 mL of water was added to the reaction solution, and the mixture was extracted twice with 100 mL of dichloromethane. The extracts were combined, dried over anhydrous sodium sulfate, filtered under vacuum, and concentrated, and the residue was purified by silica gel column chromatography and $C_{18}$ reversed-phase column chromatography to give compound **105** (181 mg).

Q-TOF(ESI, $[M+H]^+$) *m/z:* 730.3855
$^1$H NMR (500 MHz, DMSO-$d_6$) δ 11.28 (s, 1H), 11.18 (s, 1H), 7.91 (d, *J* = 9.2 Hz, 1H), 7.74 (t, *J* = 3.8 Hz, 2H), 7.66 (s, 1H), 7.50 (dd, *J* = 12.1, 8.6 Hz, 3H), 7.31 (d, *J* = 2.9 Hz, 1H), 7.18 (d, *J* = 8.1 Hz, 2H), 6.59 (d, *J* = 3.1 Hz, 1H), 5.04 (q, *J* = 8.2 Hz, 1H), 4.69 (dd, *J* = 11.8, 5.1 Hz, 1H), 3.67 (t, *J* = 5.2 Hz, 4H), 3.01 (s, 2H), 2.85 (ddd, *J* = 17.4, 12.0, 5.4 Hz, 1H), 2.63 (dt, *J* = 17.2, 4.2 Hz, 1H), 2.49 - 2.29 (m, 6H), 2.23 (dq, *J* = 14.3, 5.9, 5.1 Hz, 2H), 2.09 - 1.88 (m, 4H), 1.75 (s, 2H), 1.65 (q, *J* = 6.0, 5.6 Hz, 4H), 1.58 (h, *J* = 7.1, 5.6 Hz, 6H).

**Test Example 1: Effect of Compounds on BTK Degradation in OCI-LY10 Cells**

The detection was carried out using the Total-BTK Kits (cisbio, 63ADK064PEH), and 4× Supplemented Lysis buffer was prepared using the Blocking reagent stock solution (100×) and Lysis buffer stock solution (4×). Premixed antibody solutions were prepared using the Detection buffer, Total-BTK d2 antibody, and Total-BTK Eu Cryptate antibody.

OCI-LY10 cells in a good growth state were collected and added to a centrifuge tube, adjusted to a cell density of $4.17 \times 10^6$ cells/mL, and seeded in a 384-well plate (12 μL/well). The cells were incubated overnight in a cell incubator. Compounds were added using a nanoliter pipettor such that the final concentrations of the compounds were 100 nM-0.098 nM; the addition was performed in duplicate, and meanwhile, a control was set. After another 24 (or 6) h of incubation in the cell incubator, the 4× Supplemented Lysis buffer was added at 4 μL/well to lyse the cells. After the cells were shaken for 40 min at room temperature, the Premixed antibody solutions were added at 4 μL/well, and the cells were left to stand overnight at room temperature. Fluorescence values of the cells were detected at 665 nm/620 nm using an Envision microplate reader, the four-parameter analysis was performed, the dose-response curves were fit, and $DC_{50}$ (the concentration of the drug when the degradation rate reaches 50%) and Dmax (the maximum degradation rate) were calculated. The experimental results are shown in Table 1.

Table 1

| Compound | OCI-LY10 cell | | Compound | BTK | Compound | BTK | Compound | BTK |
|---|---|---|---|---|---|---|---|---|
| | BTK degradation | | | degradation in OCI-LY10 cells | | degradation in OCI-LY10 cells | | degradation in OCI-LY10 cells |
| | $DC_{50}$ (nM) | Dmax (%) | | Dmax (%) | | Dmax (%) | | Dmax (%) |
| 1 | <0.098 | 99 | 24 | >90 | 52 | >90 | 88 | >90 |
| 2 | <0.098 | 99 | 26 | >90 | 54 | >90 | 89 | >90 |
| 3 | <0.098 | 99 | 28 | >90 | 55 | >90 | 90 | >90 |
| 6 | <0.098 | 99 | 29 | >90 | 56 | >90 | 93 | >90 |
| 7 | <0.098 | 99 | 30 | >90 | 57 | >90 | 94 | >90 |
| 8 | <0.098 | 99 | 31 | >90 | 58 | >90 | 96 | >90 |
| 9 | <0.098 | 99 | 33 | >90 | 59 | >90 | 97 | >90 |
| 10 | <0.098 | 99 | 35 | >90 | 62 | >90 | 100 | >90 |
| 11 | <0.098 | 99 | 40 | >90 | 72 | >90 | 102-1 | >90 |
| 12 | 0.098 | 99 | 41 | >90 | 76 | >90 | 103-2 | >90 |
| 13 | <0.098 | 100 | 44 | >90 | 77 | >90 | 98-1 | >90 |
| 14 | <0.098 | 100 | 45 | >90 | 78 | >90 | 98-2 | >90 |
| 15 | <0.098 | 100 | 46 | >90 | 79 | >90 | 99-1 | >90 |
| 27 | <0.098 | 97 | 47 | >90 | 80 | >90 | | |
| 34 | <0.098 | 97 | 48 | >90 | 81 | >90 | | |
| 36 | <0.098 | 95 | 49 | >90 | 82 | >90 | | |
| 53 | <0.098 | 95.97 | 50 | >90 | 86 | >90 | | |
| 102-2 | <0.098 | 97.11 | 51 | >90 | 87 | >90 | | |

**Test Example 2: Inhibitory Activity on *In Vitro* Cell Proliferation**

2.1 Assay for inhibitory activity on OCI-LY10 cell proliferation

OCI-LY10 cells in a good growth state were collected and added to a centrifuge tube, adjusted to a cell density of 1 × 105 cells/mL, and seeded in a 96-well plate (100 μL/well). The cells were incubated overnight in a cell incubator. Compounds were added using a nanoliter pipettor such that the final concentrations of the compounds were 5000 nM-0.31 nM; the addition was performed in duplicate, and meanwhile, a control was set. After another 72 h of incubation in the cell incubator, 10 μL of CCK-8 (CK04, PP799) was added. After 2.5 h of incubation in the cell incubator, absorbance values were detected at 450 nm using an Envision microplate reader, the four-parameter analysis was performed, the dose-response curves were fit, and IC50 was calculated.

2.2 Assay for inhibitory activity on TMD8-BTK(C481S) cell proliferation

TMD8-BTK(C481S) cells in a good growth state were collected and added to a centrifuge tube, adjusted to a cell density of 8 × $10^4$ cells/mL, and seeded in a 96-well plate (100 μL/well). The cells were incubated overnight in a cell incubator. Compounds were added using a nanoliter pipettor such that the final concentrations of the compounds were 5000 nM-0.31 nM; the addition was performed in duplicate, and meanwhile, a control was set. After another 72 h of incubation in the cell incubator, 10 μL of CCK-8 (CK04, PP799) was added. After 4 h of incubation in the cell incubator, absorbance values were detected at 450 nm using an Envision microplate reader, the four-parameter analysis was performed, the dose-response curves were fit, and IC50 was calculated.

2.3 Assay for inhibitory activity on OCI-LY10-BTK(C481S) cell proliferation

OCI-LY10(C481S) cells in the logarithmic growth phase were collected and added to a centrifuge tube, adjusted to a cell density of 5 × 104 cells/mL, and seeded in a 96-well plate (100 μL/well). Meanwhile, compounds were added using a nanoliter pipettor such that the final concentrations of the compounds were 5000 nM-0.31 nM; the addition was performed in duplicate, and meanwhile, a control was set. After another 72 h of incubation in the cell incubator, the detection reagent CCK-8 (manufacturer: Dojindo Laboratories, Beijing; 10 μL/well) was added. After 3 h of incubation in the cell incubator, absorbance values were detected at 450 nm using a PerkinElmer Envision microplate reader, the four-parameter analysis was performed, the dose-response curves were fit, and IC50 was calculated. The results are shown in Table 2.

Table 2

| Compound | OCI-LY10 | TMD8-BTK$^{C481S}$ | OCI-LY10-BTK$^{C481S}$ |
|---|---|---|---|
| | IC50 (nM) | IC50 (nM) | |
| 1 | <0.31 | 35 | |
| 2 | | 18 | |
| 3 | <0.31 | | |
| 4 | | 27 | |
| 6 | <0.31 | 27 | |
| 7 | <0.31 | | |
| 8 | <0.31 | 6.9 | |
| 9 | <0.31 | 1.2 | |
| 10 | <0.31 | 2.4 | |
| 11 | <0.31 | 14 | |
| 13 | <0.31 | | <0.31 |
| 14 | <0.31 | | <0.31 |
| 15 | <0.31 | | <0.31 |
| 24 | <0.31 | | 0.5 |

(continued)

| Compound | OCI-LY10 | TMD8-BTK$^{C481S}$ | OCI-LY10-BTK$^{C481S}$ |
|---|---|---|---|
| | IC50 (nM) | IC50 (nM) | |
| 26 | <0.31 | | 0.26 |
| 27 | <0.31 | | 0.062 |
| 28 | <0.31 | | 0.29 |
| 29 | <0.31 | | 0.35 |
| 30 | <0.31 | | 0.44 |
| 33 | <0.31 | | 0.37 |
| 34 | <0.31 | | 0.19 |
| 35 | <0.31 | | 0.12 |
| 36 | <0.31 | | 0.024 |
| 40 | | | 0.51 |
| 41 | <0.31 | | 0.34 |
| 44 | | | 0.56 |
| 46 | | | 0.66 |
| 47 | | | 0.62 |
| 48 | | 5.7 | |
| 49 | | 6.8 | |
| 50 | | 4 | |
| 51 | | 4.7 | |
| 52 | | 3.7 | |
| 53 | <0.31 | 2.8 | |
| 54 | | 9.7 | |
| 55 | | 6.1 | |
| 56 | | 33 | |
| 58 | | 21 | |
| 59 | | 12 | |
| 60 | | 9.4 | |
| 61 | | 13 | |
| 62 | <0.31 | 8.2 | |
| 67 | | 32 | |
| 69 | | 5.7 | |
| 70 | | 15 | |
| 72 | | 2.6 | |
| 74 | | 23 | |
| 75 | | 9.8 | |
| 76 | | 11 | |
| 77 | <0.31 | 23 | |
| 79 | <0.31 | 12 | |

(continued)

| Compound | OCI-LY10 | TMD8-BTK$^{C481S}$ | OCI-LY10-BTK$^{C481S}$ |
|---|---|---|---|
| | IC50 (nM) | IC50 (nM) | |
| 80 | <0.31 | 11 | |
| 81 | <0.31 | 11 | |
| 82 | <0.31 | 11 | |
| 87 | <0.31 | 11 | |
| 88 | | 29 | |
| 89 | <0.31 | 20 | |
| 90 | <0.31 | 1.2 | |
| 93 | | | 0.7 |
| 94 | <0.31 | | 0.53 |
| 96 | <0.31 | | 0.31 |
| 97 | | | 0.78 |
| 100 | <0.31 | 0.57 | |
| 104 | | 16 | |
| 105 | | 6.9 | |

**Test Example 3: *In Vitro* Kinase Inhibition and Selectivity**

3.1 Inhibitory activity on BTK(WT)

The Asssy Buffer was prepared, and the composition of the Asssy Buffer was 50 mM Hepes (Gibco, 15630), 10 mM $MgCl_2$, 2 mM DTT, 1 mM EGTA, and 0.010% Tween 20. BTK(WT) kinase (Life, PR5442A), ATP (Sigma, A7699), and ULight-poly GT (PE, TRF0100-M) working solutions were prepared using the Asssy Buffer. EDTA and Eu-labeled anti-phosphotyrosine (PT66) antibody (PE, AD0069) working solutions were prepared using the Detection Buffer (PE, CR97-100). 6 μL of BTK(WT) kinase at the corresponding concentration (final concentration: 0.003 ng/μL) was added to the compound groups and the control group, while 6 μL of the Asssy Buffer was added to the blank group, and then the compounds (with the concentration set to 1000 nM at the maximum, 4-fold dilution, 7 concentration gradients) were each added using a nanoliter pipettor. The mixture was incubated at room temperature for 30 min, and then 4 μL of the mixture of ATP (final concentration: 10 μM) and ULight-poly GT (final concentration: 100 nM) was added. After incubation at room temperature for 2 h, 5 μL of EDTA (final concentration: 10 mM) was added to stop the reaction, and finally 5 μL of antibody (final concentration: 2 nM) was added. After incubation at room temperature for 1 h, signal values were detected at 665/615 nM, the four-parameter analysis was performed, the dose-response curves were fit, and IC50 was calculated.

3.2 Inhibitory activity on BTK(C481S)

The Asssy Buffer was prepared, and the composition of the Asssy Buffer was 50 mM Hepes (Gibco, 15630), 10 mM $MgCl_2$, 2 mM DTT, 1 mM EGTA, and 0.010% Tween 20. BTK(C481S) kinase (Carna Biosciences, 08-547), ATP (Sigma, A7699), and ULight-poly GT (PE, TRF0100-M) working solutions were prepared using the Asssy Buffer. EDTA and Eu-labeled anti-phosphotyrosine (PT66) antibody (PE, AD0069) working solutions were prepared using the Detection Buffer. 6 μL of BTK(C481S) kinase at the corresponding concentration (final concentration: 0.005 ng/μL) was added to the compound groups and the control group, while 6 μL of the Asssy Buffer was added to the blank group, and then the compounds (with the concentration set to 1000 nM at the maximum, 4-fold dilution, 7 concentration gradients) were added using a nanoliter pipettor. The mixture was incubated at room temperature for 30 min, and then 4 μL of the mixture of ATP (final concentration: 10 μM) and ULight-poly GT (final concentration: 100 nM) was added. After incubation at room temperature for 2 h, 5 μL of EDTA (final concentration: 10 mM) was added to stop the reaction, and finally 5 μL of antibody (final concentration: 2 nM) was added. After incubation at room temperature for 1 h, signal values were detected at 665/615 nM, the four-parameter analysis was performed, the dose-response curves were fit, and IC50 was

calculated.

3.3 Inhibitory activity on EGFR

The Asssy Buffer was prepared, and the composition of the Asssy Buffer was 50 mM Hepes (Gibco, 15630), 10 mM $MgCl_2$, 2 mM DTT, 1 mM EGTA, and 0.010% Tween 20. EGFR kinase (Carna, 08-115), ATP (Sigma, A7699), and ULight-poly GT (PE, TRF0100-M) working solutions were prepared using the Asssy Buffer. EDTA and Eu-labeled anti-phosphotyrosine (PT66) antibody (PE, AD0069) working solutions were prepared using the Detection Buffer. 6 μL of EGFR kinase at the corresponding concentration (final concentration: 0.006 ng/μL) was added to the compound groups and the control group, while 6 μL of the Asssy Buffer was added to the blank group, and then the compounds (with the concentration set to 1000 nM at the maximum, 4-fold dilution, 7 concentration gradients) were added using a nanoliter pipettor. The mixture was incubated at room temperature for 30 min, and then 4 μL of the mixture of ATP (final concentration: 5 μM) and ULight-poly GT (final concentration: 100 nM) was added. After incubation at room temperature for 2 h, 5 μL of EDTA (final concentration: 10 mM) was added to stop the reaction, and finally 5 μL of antibody (final concentration: 2 nM) was added. After incubation at room temperature for 1 h, signal values were detected at 665/615 nM, the four-parameter analysis was performed, the dose-response curves were fit, and IC50 was calculated.

3.4 Inhibitory activity on TEC

The Asssy Buffer was prepared, and the composition of the Asssy Buffer was 50 mM Hepes (Gibco, 15630), 10 mM $MgCl_2$, 2 mM DTT, 1 mM EGTA, and 0.010% Tween 20. TEC kinase (Carna, 08-115), ATP (Sigma, A7699), and ULight-poly GT (PE, TRF0100-M) working solutions were prepared using the Asssy Buffer. EDTA and Eu-labeled anti-phosphotyrosine (PT66) antibody (PE, AD0069) working solutions were prepared using the Detection Buffer. 6 μL of TEC kinase at the corresponding concentration (final concentration: 0.01 ng/μL) was added to the compound groups and the control group, while 6 μL of the Asssy Buffer was added to the blank group, and then the compounds (with the concentration set to 1000 nM at the maximum, 4-fold dilution, 7 concentration gradients) were added using a nanoliter pipettor. The mixture was incubated at room temperature for 30 min, and then 4 μL of the mixture of ATP (final concentration: 10 μM) and ULight-poly GT (final concentration: 100 nM) was added. After incubation at room temperature for 2 h, 5 μL of EDTA (final concentration: 10 mM) was added to stop the reaction, and finally 5 μL of antibody (final concentration: 2 nM) was added. After incubation at room temperature for 1 h, signal values were detected at 665/615 nM, the four-parameter analysis was performed, the dose-response curves were fit, and IC50 was calculated. The results are shown in Table 3.

Table 3

| Compound | BTK | $BTK^{C481S}$ | EGFR |
|---|---|---|---|
| | IC50 (nM) | | |
| 10 | 7.1 | 19.6 | 3317 |
| 27 | 0.96 | 9 | 109 |
| 33 | 11 | 22 | 785 |
| 50 | 0.42 | 0.81 | 69 |
| 59 | 5.4 | 9.1 | 279 |

**Test Example 4: *In Vitro* Liver Microsomal Stability**

Liver microsome incubation samples were prepared by mixing a PBS buffer (pH 7.4), a liver microsome solution (0.5 mg/mL), a test compound, and an NADPH + $MgCl_2$ solution and incubated at 37 °C and 300 rpm for 1 h. Zero-hour samples were prepared by mixing a PBS buffer (pH 7.4), a liver microsome solution (0.5 mg/mL), and a test compound. An acetonitrile solution containing an internal standard was added to the samples, and supernatants were prepared by protein precipitation, diluted, and then assayed by LC/MS/MS. The results are shown in Table 4.

Table 4. *In vitro* metabolic stability of related compounds in liver microsomes of various species

| Compound | Mouse liver microsome | Human liver microsome |
|---|---|---|
| | Residuals at 60 min (%) | |
| 7 | 42.7% | |
| 8 | 54.7% | |
| 10 | 65.4% | |
| 13 | 56.41% | |
| 14 | 62.41% | |
| 27 | | 56.0% |
| 33 | 71.0% | 55.0% |
| 50 | 63.1% | |
| 59 | 47.0% | |
| 69 | 85.0% | 80.0% |
| 72 | | 94.0% |
| 79 | 61.19% | |
| 80 | 51.56% | |
| 90 | 54.63% | |
| 93 | 72.0% | 53.0% |
| 94 | 75.0% | 81.0% |

**Test Example 5: Mouse Pharmacokinetics**

ICR mice weighing 18-22 g were randomized into groups of 9 after 3-5 days of acclimatization and then intragastrically given the test compound solution at a dose of 10 mg/kg. Plasma samples to be tested were prepared by taking blood from the orbit at time points of 15 min, 30 min, 1 h, 2 h, 3 h, 4 h, 6 h, 8 h, 10 h, and 24 h. 20 μL of each plasma sample to be detected and a standard curve sample were taken, and an acetonitrile solution containing an internal standard was added. Supernatants were obtained by protein precipitation, diluted, and then assayed by LC/MS/MS. Pharmacokinetic parameters were fitted using a non-compartmental model (see Table 5).

Table 5

| Compound | Mouse i.v. 1 mpk | | | Mouse i.g. 10 mpk | | | |
|---|---|---|---|---|---|---|---|
| | AUC (ng*h/mL) | t1/2 (h) | Cl (L/hr/kg) | AUC (ng*h/mL) | t1/2 (h) | Cmax (ng/mL) | F (%) |
| 9 | 2971 | 1.5 | 0.331 | 13697 | 2.6 | 1392 | 46.10% |
| 10 | 6138 | 1.6 | 0.159 | 35589 | 2.9 | 3848 | 57.98% |
| 27 | 1758 | 1.4 | 0.56 | 7687 | 2.4 | 766 | 44.0% |
| 33 | 7987 | 2.6 | 0.13 | 20971 | 3.2 | 1555 | 26.0% |
| 69 | 8033 | 2.6 | 0.12 | 41431 | 7 | 2732 | 51.6% |
| 72 | 1287 | 2.3 | 0.72 | 8301 | 4.6 | 634 | 64.50% |
| 89 | 9703 | 2.8 | 0.103 | 23740 | 3 | 1881 | 24.5% |
| 90 | 8882 | 2.7 | 0.11 | 18944 | 3 | 2550 | 21.0% |

**Test Example 6:** Study on *In Vivo* Drug Efficacy

TMD-8 mouse subcutaneous xenograft tumors: TMD-8 (WT) cells at a concentration of 1 × $10^8$/mL were

inoculated under aseptic conditions into the right side armpit of NOD-SCID mice at 0.1 mL/mouse. After subcutaneous grafting, the animals were divided into groups with 6 animals in each group when the tumor volume reached about 188 $mm^3$ (or $200mm^3$):
model group: vehicle, 6 mice; compound administration groups: 30 mg/kg, qd, i.g., 6 mice.

The vehicle or the test compound was intragastrically given at a volume of 4mL/kg, once daily for 16 consecutive days. The tumor volume was measured 2-3 times a week, and meanwhile, the mice were weighed and the data were recorded; the general behaviors of the animals were observed every day. After all dosing was complete, the animals were sacrificed on day 17, and the tumors were removed and weighed. The results are shown in Table 6. The tumor volume and the tumor growth inhibition were calculated using the following formulae:

$$\text{Tumor volume (TV)} = (\text{Length} \times \text{Width}^2)/2.$$

$$\text{Tumor growth inhibition (TGI)} = (1 - \text{tumor weight of treatment group/tumor weight of model group}) \times 100\%.$$

Table 6 Therapeutic effect of compounds on mouse TMD-8 xenograft tumors

| Compound | TV on d0 ($mm^3$) Mean±SD | TV on d12 ($mm^3$) Mean±SD | TV on d15 ($mm^3$) Mean±SD | TGI (%) d17 |
|---|---|---|---|---|
| Model group | 226±27 | 1347±290 | 1629±252 | - |
| 10 | 226±29 | 235±57 | 209±60 | 97.5% |
| 105 | 226±28 | 253±36 | 229±49 | 95.4% |
| 104 | 226±27 | 286±77 | 269±61 | 96.0% |
| 69 | 226±21 | 246±56 | 199±72 | 96.4% |
| 72 | 226±21 | 246±52 | 122±78 | 97.9% |

**Test Example 7:** Binding Activity for CRBN Protein

Reagent: CEREBLON BINDING KITS, purchased from Cisbio

Preparation of working solutions:

1) Preparation of Human Cereblon WT GST-tagged protein working solution according to requirements of the kit

The Human Cereblon WT GST-tagged protein stock solution was diluted from 45X to 1/2X with the PROTAC binding Buffer

2) Preparation of GST Eu Cryptate Antibody+Thalidomide-Red reagent working solution according to requirements of the kit

a. preparing 1X GST Eu Cryptate Antibody: the GST Eu Cryptate Antibody stock solution was diluted from 50X to 1X with the PROTAC binding Buffer;
b. preparing 1X Thalidomide-Red reagent: the Thalidomide-Red reagent stock solution was diluted from 50X to 1X with the PROTAC binding Buffer;
c. sufficiently mixing 1X GST Eu Cryptate Antibody and 1X Thalidomide-Red reagent at a ratio of 1:1.

Test process: the whole experiment was carried out at room temperature. A 384-well white low-volume microplate was used; for the experimental groups and the negative group, the 1/2X Human Cereblon WT GST-tagged protein working solution was added at 5 μL/well, and for the blank group, the PROTAC binding Buffer was added at 5 μL/well. Then, the compounds (at an initial concentration of 10 μM, 7 concentration gradients by 1:3 serial dilution) were added to the experimental groups using an ultra microsyringe. Finally, 10 μL of GST Eu Cryptate Antibody+Thalidomide-Red

reagent working solution was added to all groups, and after incubation for 3 h at room temperature, signal values were detected at 665 nm/620 nm using a PerkinElmer Envision HTS multi-label plate reader. The inhibition rate was calculated by the following formula: inhibition rate (%) = (mean value of negative control group - mean value of experimental group)/(mean value of negative control group - mean value of blank group) × 100%. The curves were fit using the four-parameter logistic model with the logarithm of the compound concentration as the abscissa and the inhibition rate as the ordinate, and $IC_{50}$ values were calculated. The test compounds of the present application have binding activity for CRBN protein ($IC_{50}$ < 5 μM).

**Test Example** 8: *In Vitro* Test for IKZF1/IKZF3/GSPT1 Degradation Levels in Multiple Myeloma Cell

The regulation of the levels of proteins IKZF1/IKZF3/GSPT1 in multiple myeloma cell MM.1S by compounds was studied by using the Western Blot method.

Experimental scheme: MM.1S cells were seeded in a 6-well plate and then treated with the test compounds, and the cells were collected and lysed on ice with RIPA buffer containing a compound protease inhibitor (Roche). Supernatants were collected by centrifugation and protein quantification was performed (BCA protein assay kit, Thermo). An equal amount of 20 μg of total protein was separated by SDS-PAGE, transferred to a PVDF membrane, and then blocked at room temperature by the addition of 5% skimmed milk powder (prepared with 0.1% TBS-T). Primary antibodies anti-IKZF1, anti-IKZF-3, anti-GSPT1, and anti-Actin were each diluted with 5% BSA (prepared with 0.1% TBS-T) according to a ratio, and the PVDF membrane was incubated in the primary antibody overnight at 4 °C. The next day, after incubation with a HRP-labeled secondary antibody at room temperature for a period of time, the band on the membrane was detected using the ECL chemiluminescent substrate (Thermo) to determine whether the compound had the ability to degrade IKZF1/IKZF3/GSPT1 protein. The test compounds of the present application have the activity in degrading IKZF1, IKZF3, and GSPT1 proteins. The above examples are only preferred examples of the present application and are not intended to limit the scope of the present application. It will be appreciated by those skilled in the art that various modifications and changes may be made based on the examples described above, and any modifications, substitutions, combinations, etc., made within the spirit and conception of the present application shall all fall within the protection scope of the present application.

## Claims

**1.** A compound of formula I, a stereoisomer thereof, or a pharmaceutically acceptable salt thereof,

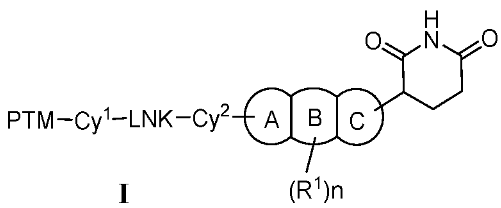

I

wherein,

ring A is absent or selected from the group consisting of $C_{5-10}$ cycloalkenyl, 5- to 10-membered heterocycloalkenyl, phenyl, and 5- to 6-membered heteroaryl, or
ring B is selected from phenyl;
ring C is selected from the group consisting of isoxazolyl and furanyl;
each R' is independently selected from the group consisting of halogen, -OH, $-NH_2$, -CN, $C_{1-4}$ alkyl, $C_{1-4}$ alkoxy, and $C_{1-4}$ haloalkyl;
n is selected from the group consisting of 0, 1, 2, and 3;
$Cy^1$ is selected from the group consisting of a bond, $C_{3-12}$ cycloalkyl, and 4- to 12-membered heterocycloalkyl, wherein the $C_{3-12}$ cycloalkyl or 4- to 12-membered heterocycloalkyl is optionally substituted with one or more $R^a$; LNK is selected from the group consisting of a bond, $C_{1-12}$ alkylene, and $C_{1-12}$ heteroalkylene;
$Cy^2$ is absent or selected from the group consisting of $C_{3-12}$ cycloalkyl and 4- to 12-membered heterocycloalkyl, wherein the cycloalkyl or heterocycloalkyl is optionally substituted with one or more $R^b$;
each $R^a$ and each $R^b$ are independently selected from the group consisting of halogen, -OH, $-NH_2$, -CN, $C_{1-4}$ alkyl, $C_{1-4}$ alkoxy, $C_{1-4}$ haloalkyl, $C_{1-4}$ alkylamino, and di-$C_{1-4}$ alkylamino;
PTM is selected from the group consisting of drugs and derivatives thereof that bind to a target protein.

2. The compound, the stereoisomer thereof, or the pharmaceutically acceptable salt thereof according to claim 1, wherein PTM is selected from the group consisting of drugs and derivatives thereof that act on AR, ER, kinase, phosphatase, MDM2, human BET bromodomain protein, Hsp90, HDAC, human lysine methyltransferase, RAF receptor, FKBP, angiogenic factor, immunosuppression-related receptor or protein, arene receptor, thyroid hormone receptor, HIV protease, HIV integrase, HCV protease, HBV protease, or acyl protein thioesterase 1 and/or 2;

optionally, PTM is selected from the group consisting of drugs and derivatives thereof that act on ALK, BET, CDK, PARP, EGFR, γ-secretase, CBFβ-SMMHC, WEE1, MEK, BCR-ABL, MET, RAS, BTK, VEGFR, JAK, HER2, HDAC, Akt, PI3K, mTOR, AR, ER, PDEδ, SRC, MDM2, RAF, IRAK4, STATS and c-Myc; or
PTM is selected from the group consisting of drugs and derivatives thereof that act on ALK, BRD4, CDK4/6, PARP, EGFR, γ-secretase, CBFβ-SMMHC, WEEl, MEK, BCR-ABL, MET, KRAS, EGFR, BTK, AR, ER, PDEδ, JAK, MDM2 or RAF; or
PTM is selected from the group consisting of drugs and derivatives thereof that act on BTK or WEE1; or
PTM is selected from the group consisting of drugs and derivatives thereof that act on BTK.

3. A compound of formula II-1, a stereoisomer thereof, or a pharmaceutically acceptable salt thereof,

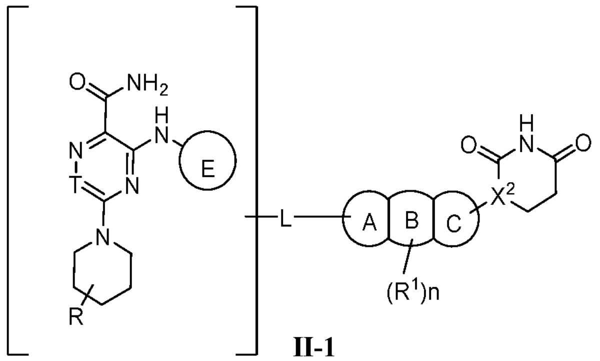

II-1

wherein,

T is selected from the group consisting of CH and N;
R is selected from the group consisting of hydrogen and 5- to 6-membered heterocycloalkyl, wherein the heterocycloalkyl is optionally substituted with =O or $C_{1-6}$ alkyl;
ring E is selected from the group consisting of phenyl, benzocycloalkenyl, and benzoheterocycloalkenyl;
$X^2$ is selected from the group consisting of CH and N;
L is selected from a connecting group,
wherein ring A, ring B, ring C, $R^1$, or n is as defined in claim 1.

4. The compound, the stereoisomer thereof, or the pharmaceutically acceptable salt thereof according to claim 3, wherein L is selected from the group consisting of $-Cy^1-LNK-Cy^2-LNK-$, $-Cy^1-LNK-Cy^2-$, and $-Cy^1-Cy^2-LNK-$, wherein $Cy^1$, LNK, and $Cy^2$ are as defined in claim 1.

5. The compound, the stereoisomer thereof, or the pharmaceutically acceptable salt thereof according to any one of claims 1-4, being selected from a compound of formula I'a, a stereoisomer thereof, or a pharmaceutically acceptable salt thereof,

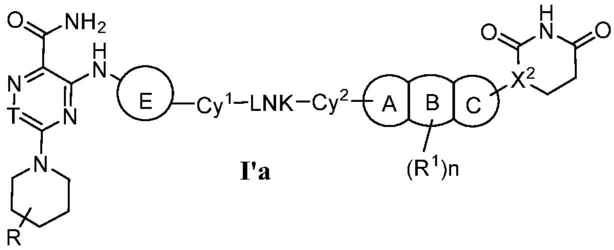

I'a

wherein,

T is selected from the group consisting of CH and N;
R is selected from the group consisting of hydrogen and 5- to 6-membered heterocycloalkyl, wherein the heterocycloalkyl is optionally substituted with =O or $C_{1-6}$ alkyl;
ring E is selected from the group consisting of phenyl, benzocycloalkenyl, and benzoheterocycloalkenyl;
$X^2$ is selected from the group consisting of CH and N;
ring A, ring B, ring C, $R^1$, n, $Cy^1$, LNK, and $Cy^2$ are as defined in claim 1 or 3.

**6.** The compound, the stereoisomer thereof, or the pharmaceutically acceptable salt thereof according to any one of claims 1-5, wherein $X^2$ is selected from CH; or, $X^2$ is selected from N.

**7.** The compound, the stereoisomer thereof, or the pharmaceutically acceptable salt thereof according to any one of claims 1-6, wherein ring E is selected from the group consisting of phenyl, benzo $C_{5-12}$ cycloalkenyl, and benzo 5- to 12-membered heterocycloalkenyl; or

ring E is selected from the group consisting of phenyl, benzo $C_{5-6}$ cycloalkenyl, and benzo 5- to 11-membered heterocycloalkenyl; or
ring E is selected from the group consisting of phenyl, benzo 5-membered heterocycloalkenyl, benzo 6-membered heterocycloalkenyl, benzo 10-membered heterocycloalkenyl, and benzo 11-membered heterocycloalkenyl.

**8.** The compound, the stereoisomer thereof, or the pharmaceutically acceptable salt thereof according to any one of claims 1-7, being selected from a compound of formula I', a stereoisomer thereof, or a pharmaceutically acceptable salt thereof,

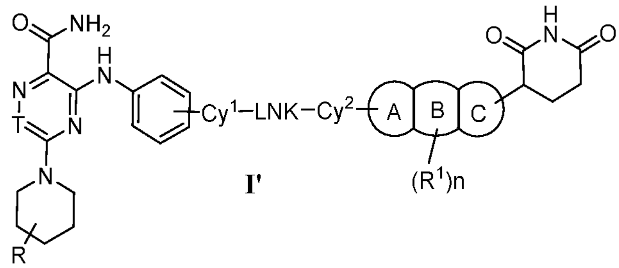

I'

wherein,

T is selected from the group consisting of CH and N;
R is selected from the group consisting of hydrogen and 5- to 6-membered heterocycloalkyl, wherein the heterocycloalkyl is optionally substituted with =O or $C_{1-6}$ alkyl.

**9.** The compound, the stereoisomer thereof, or the pharmaceutically acceptable salt thereof according to any one of claims 1-8, wherein ring A is absent or selected from the group consisting of $C_{5-8}$ cycloalkenyl, 5- to 10-membered heterocycloalkenyl, phenyl, and 5-membered heteroaryl; ring A is absent or selected from the group consisting of $C_{5-7}$ cycloalkenyl, 5- to 9-membered heterocycloalkenyl, phenyl, and 5-membered heteroaryl; ring A is absent or selected from the group consisting of $C_{5-6}$ cycloalkenyl, 5- to 9-membered heterocycloalkenyl, phenyl, and 5-membered heteroaryl; or

ring A is absent or selected from the group consisting of $C_{5-6}$ cycloalkenyl, 5- to 9-membered heterocycloalkenyl, phenyl, pyrrolyl, pyrazolyl, furanyl, and oxazolyl; or
ring A is absent or selected from the group consisting of $C_5$ cycloalkenyl, $C_6$ cycloalkenyl, 5-, 6-, 7-, 8-, or 9-membered heterocycloalkenyl, phenyl, pyrrolyl, pyrazolyl, furanyl, and oxazolyl; or
ring A is absent or selected from the group consisting of cyclopentenyl, bicyclohexenyl, dihydropyrrolyl, tetrahydropyridinyl, tetrahydroazepinyl, dihydrooxazinyl, azaspirooctenyl, azaspirononenyl, phenyl, pyrrolyl, pyrazolyl, furanyl, and oxazolyl; or
ring A is selected from the group consisting of $C_{5-6}$ cycloalkenyl and 5- to 9-membered heterocycloalkenyl; or
ring A is selected from the group consisting of cyclopentenyl, bicyclohexenyl, dihydropyrrolyl, tetrahydropyridinyl,

tetrahydroazepinyl, dihydrooxazinyl, azaspirooctenyl, and azaspirononenyl.

**10.** The compound, the stereoisomer thereof, or the pharmaceutically acceptable salt thereof according to any one of claims 1-9, wherein the structural moiety

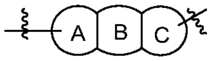

is selected from the group consisting of

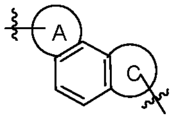 and 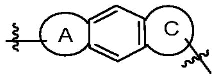;

or

the structural moiety

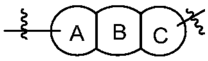

is selected from the group consisting of

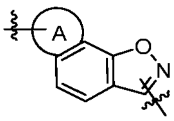,

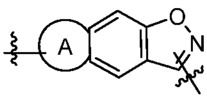, 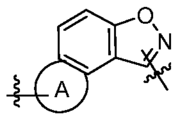, 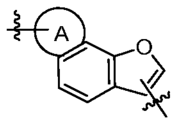, 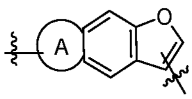, and 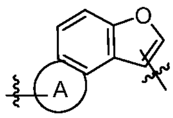;

or
the structural moiety

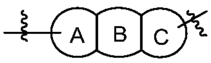

is selected from the group consisting of

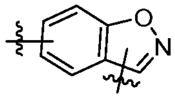,

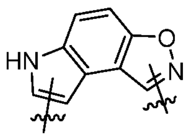, 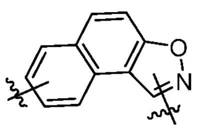, 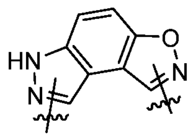, 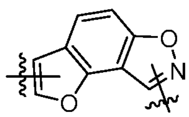, 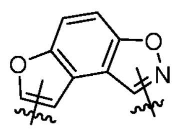, 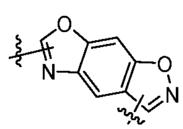,

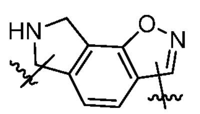, 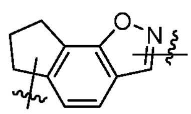, 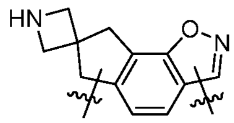, 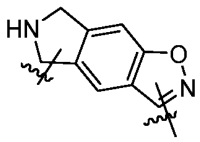, 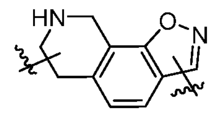,

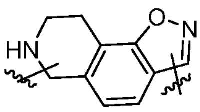, 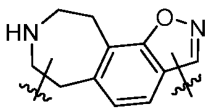, 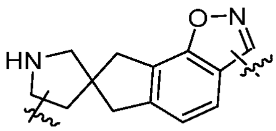, 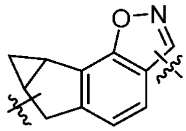, 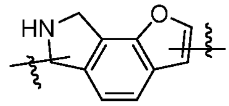,

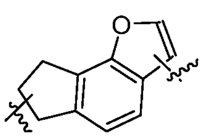, 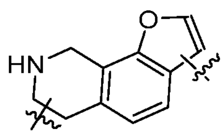, 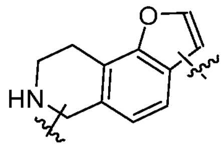, 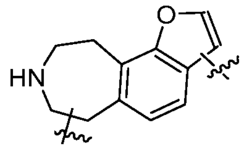, 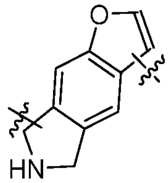,

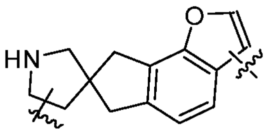, 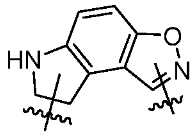, 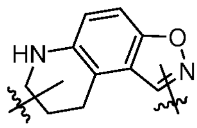, and 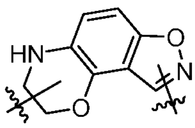;

or
the structural moiety

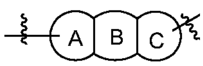

is selected from the group consisting of

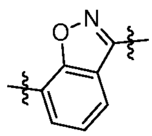, 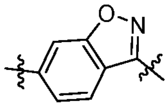,

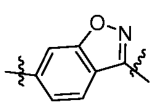, 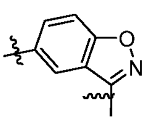, 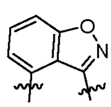, 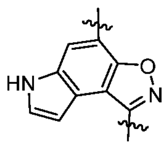, 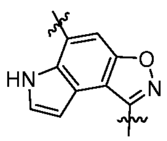, 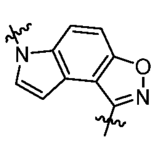, 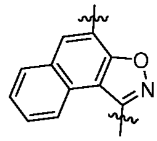,

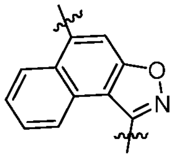, 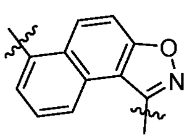, 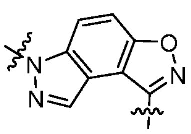, 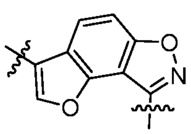, 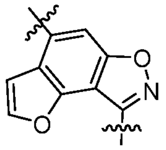, 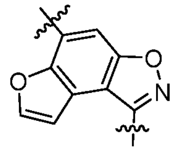,

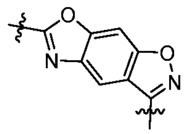, 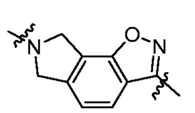, 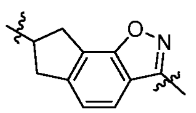, 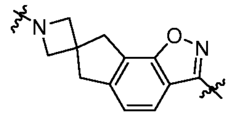, 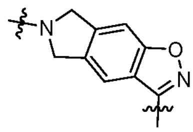,

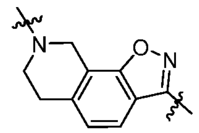, 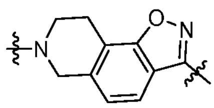, 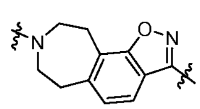, 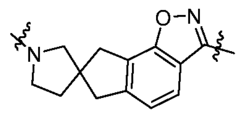, 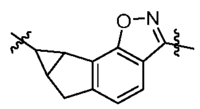,

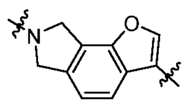, 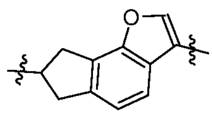, 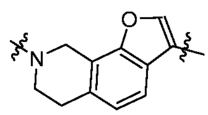, 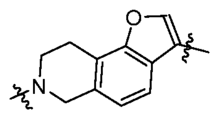, 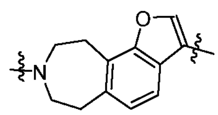,

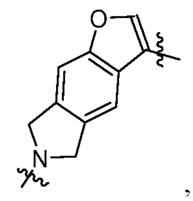, 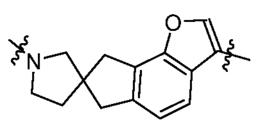, 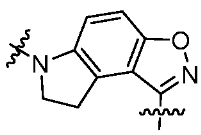, 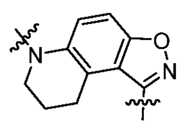, and 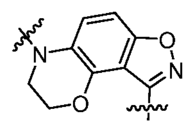.

**11.** The compound, the stereoisomer thereof, or the pharmaceutically acceptable salt thereof according to any one of claims 1-10, wherein each R' is independently selected from the group consisting of halogen, -OH, $-NH_2$, -CN, $C_{1-3}$ alkyl, $C_{1-3}$ alkoxy, and $C_{1-3}$ haloalkyl; or

each R' is independently selected from the group consisting of fluorine, chlorine, bromine, -OH, $-NH_2$, and -CN; or
each R' is independently selected from the group consisting of fluorine, chlorine, and bromine; or
each R' is independently selected from fluorine.

**12.** The compound, the stereoisomer thereof, or the pharmaceutically acceptable salt thereof according to any one of claims 1-11, wherein n is selected from the group consisting of 0, 1, and 2; or, n is selected from the group consisting of 0 and 1.

**13.** The compound, the stereoisomer thereof, or the pharmaceutically acceptable salt thereof according to any one of claims 1-12, wherein the structural moiety

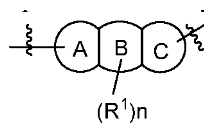

is selected from the group consisting of

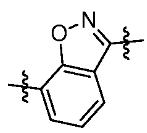, 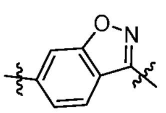, 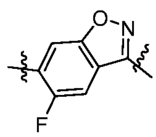, 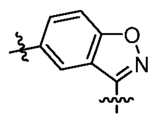, 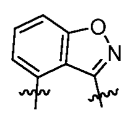, 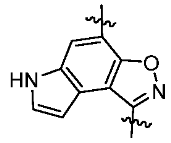, 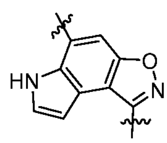,

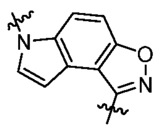, 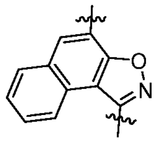, 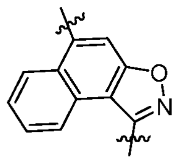, 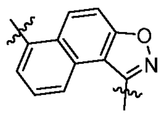, 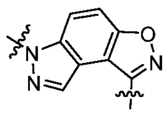, 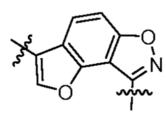,

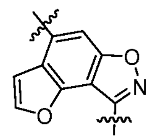, 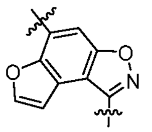, 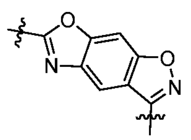, 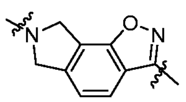, 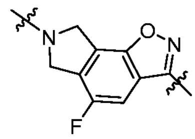, 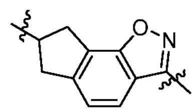,

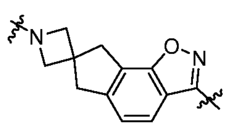, 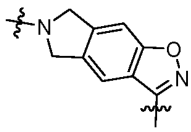, 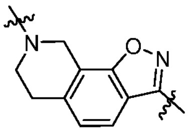, 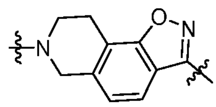, 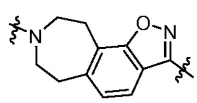,

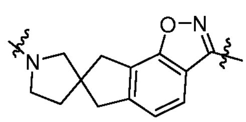, 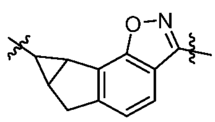, 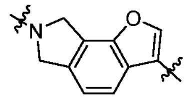, 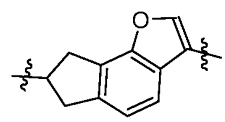, 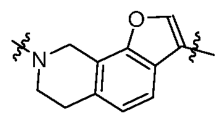,

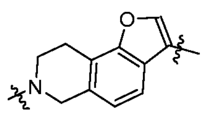, 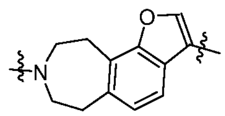, 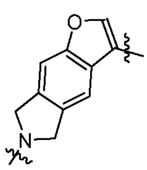, 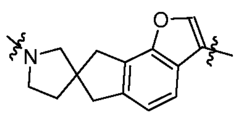, 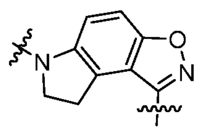,

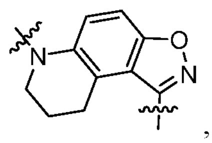, and 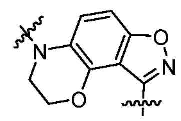.

**14.** The compound, the stereoisomer thereof, or the pharmaceutically acceptable salt thereof according to any one of claims 1-13, wherein the structural moiety -$Cy^1$-LNK-$Cy^2$- is selected from the group consisting of -$Cy^1$-, -$Cy^1$-LNK, -$Cy^1$-$Cy^2$-, -$Cy^1$-LNK-$Cy^2$-, -$Cy^2$-, and -LNK-$Cy^2$-; or
the structural moiety -$Cy^1$-LNK-$Cy^2$- is selected from the group consisting of -$Cy^1$-, -$Cy^2$-$Cy^2$-, and -$Cy^2$-.

**15.** The compound, the stereoisomer thereof, or the pharmaceutically acceptable salt thereof according to any one of claims 1-14, wherein $Cy^1$ is selected from the group consisting of a bond and the following groups optionally substituted with one or more $R^a$: $C_{4-11}$ cycloalkyl or 4- to 11-membered heterocycloalkyl; or

$Cy^1$ is selected from the group consisting of a bond and the following groups optionally substituted with one or more $R^a$: $C_{6-9}$ cycloalkyl or 4- to 11-membered heterocycloalkyl; or
$Cy^1$ is selected from the group consisting of a bond and the following groups optionally substituted with one or more $R^a$: $C_6$ cycloalkyl, $C_9$ cycloalkyl, and 4-, 5-, 6-, 7-, 8-, 9-, 10-, or 11-membered heterocycloalkyl; or
$Cy^1$ is selected from the group consisting of a bond and the following groups optionally substituted with one or more $R^a$: $C_6$ cycloalkyl, $C_9$ cycloalkyl, and 4-, 6-, or 8- to 11-membered heterocycloalkyl; or
$Cy^1$ is selected from a bond; or
$Cy^1$ is selected from the group consisting of $C_6$ cycloalkyl and $C_9$ cycloalkyl optionally substituted with one or more $R^a$; or
$Cy^1$ is selected from the group consisting of 4-, 6-, and 8- to 11-membered heterocycloalkyl optionally substituted with one or more $R^a$; or

$Cy^1$ is selected from the group consisting of 8-, 9-, 10-, and 11-membered heterocycloalkyl optionally substituted with one or more $R^a$; or

$Cy^1$ is selected from the group consisting of a bond and the following groups optionally substituted with one or more $R^a$: cyclohexyl, spirononanyl, azetidinyl, octahydrocyclopentapyrrolyl, piperidinyl, monoazaspirononanyl, diazaspirononanyl, azabicyclononanyl, monoazaspiroundecanyl, or diazaspiroundecanyl.

**16.** The compound, the stereoisomer thereof, or the pharmaceutically acceptable salt thereof according to any one of claims 1-15, wherein LNK is selected from the group consisting of a bond, $C_{1-6}$ alkylene, and $C_{1-6}$ heteroalkylene; or

LNK is selected from the group consisting of a bond and $C_{1-4}$ alkylene; or
LNK is selected from the group consisting of a bond and $C_{1-3}$ alkylene; or
LNK is selected from the group consisting of a bond and $-CH_2-$.

**17.** The compound, the stereoisomer thereof, or the pharmaceutically acceptable salt thereof according to any one of claims 1-16, wherein $Cy^2$ is absent or selected from the group consisting of $C_{4-11}$ cycloalkyl and 4- to 11-membered heterocycloalkyl, wherein the cycloalkyl or heterocycloalkyl is optionally substituted with one or more $R^b$; or

$Cy^2$ is absent or selected from the group consisting of $C_{4-6}$ cycloalkyl and 4- to 6-membered heterocycloalkyl, wherein the cycloalkyl or heterocycloalkyl is optionally substituted with one or more $R^b$; or
$Cy^2$ is absent or selected from the group consisting of cyclobutyl, cyclopentyl, cyclohexyl, azetidinyl, pyrrolidinyl, and piperidinyl, wherein the cyclobutyl, cyclopentyl, azetidinyl, pyrrolidinyl, or piperidinyl is optionally substituted with one or more $R^b$; or
$Cy^2$ is selected from the group consisting of

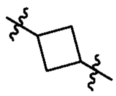, 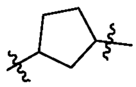, 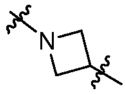, 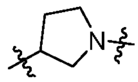, 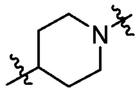,

and

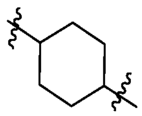.

**18.** The compound, the stereoisomer thereof, or the pharmaceutically acceptable salt thereof according to any one of claims 1-17, wherein $R^a$ and $R^b$ are each independently selected from the group consisting of halogen, -OH, $-NH_2$, -CN, $C_{1-3}$ alkyl, $C_{1-3}$ alkoxy, $C_{1-3}$ haloalkyl, $C_{1-3}$ alkylamino, and di-$C_{1-3}$ alkylamino; or

$R^a$ and $R^b$ are each independently selected from the group consisting of halogen, -OH, $-NH_2$, -CN, and $C_{1-3}$ alkyl; or
$R^a$ and $R^b$ are each independently selected from the group consisting of halogen, -OH, $-NH_2$, and -CN.

**19.** The compound, the stereoisomer thereof, or the pharmaceutically acceptable salt thereof according to any one of claims 1-18, wherein R is selected from the group consisting of hydrogen and 5- to 6-membered heterocycloalkyl, wherein the heterocycloalkyl is optionally substituted with =O or $C_{1-3}$ alkyl; or

R is selected from the group consisting of hydrogen and 5-membered heterocycloalkyl, wherein the heterocycloalkyl is optionally substituted with =O or $C_{1-3}$ alkyl; or
R is selected from the group consisting of hydrogen and imidazolinyl, wherein the imidazolinyl is optionally substituted with =O or methyl; or
R is selected from the group consisting of hydrogen and

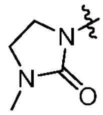.

**20.** The compound, the stereoisomer thereof, or the pharmaceutically acceptable salt thereof according to any one of claims 1-19, wherein the compound of formula I, formula II, formula I'a, or formula I' is selected from the group consisting of compounds of formula I'-1A, formula I'-2A, formula I'-1A-1, formula I'-2A-1, formula I'-3A-1, formula I'-3A-2, formula I'-4A-1, and formula I'-4A-2, wherein

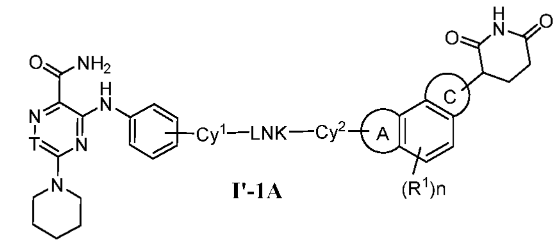

I'-1A

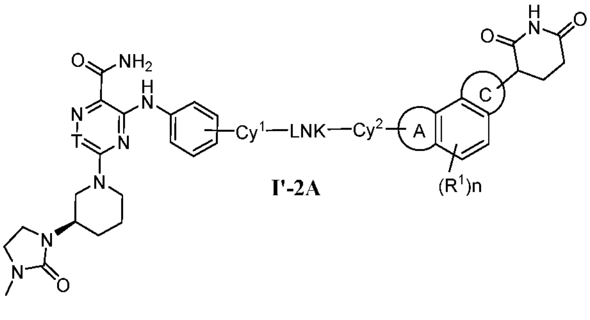

I'-2A

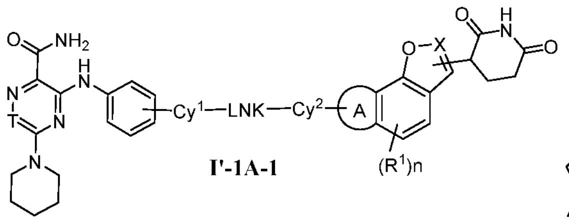

I'-1A-1

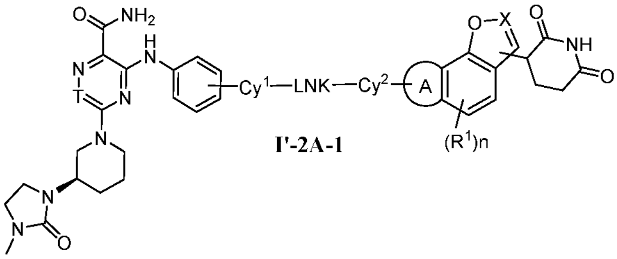

I'-2A-1

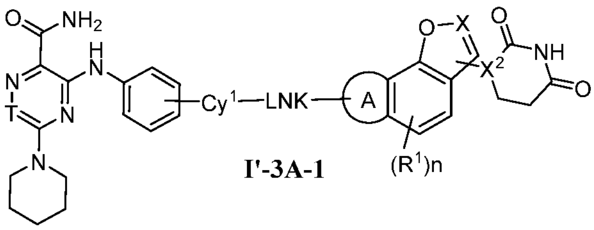

I'-3A-1

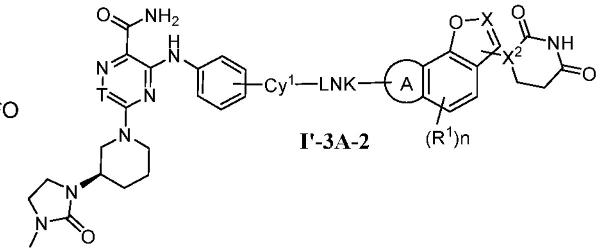

I'-3A-2

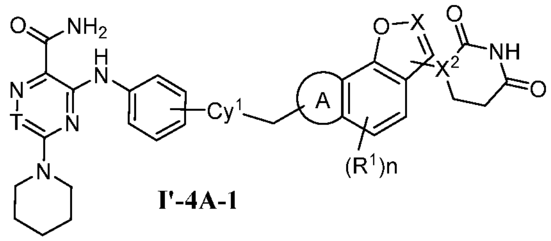

I'-4A-1

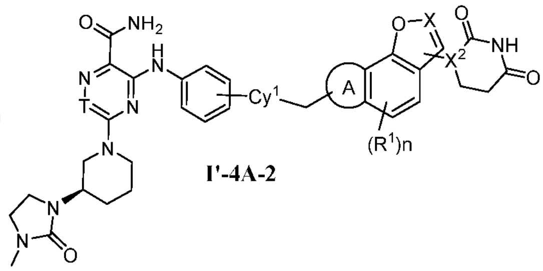

I'-4A-2

X is selected from the group consisting of CH and N.

**21.** A compound of the following formula, a stereoisomer thereof, or a pharmaceutically acceptable salt thereof:

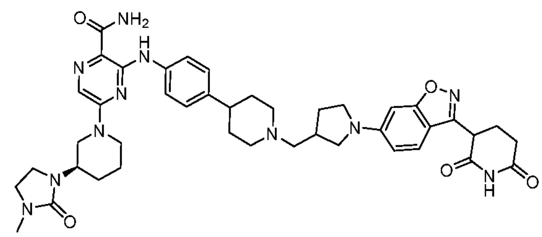

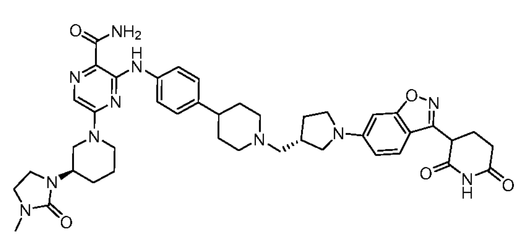

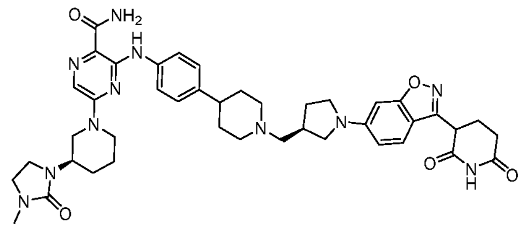

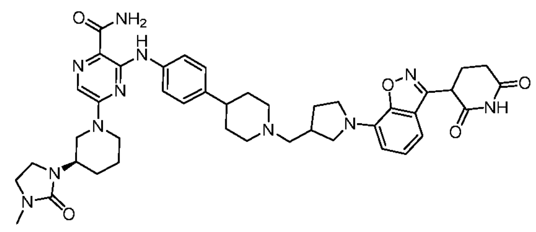

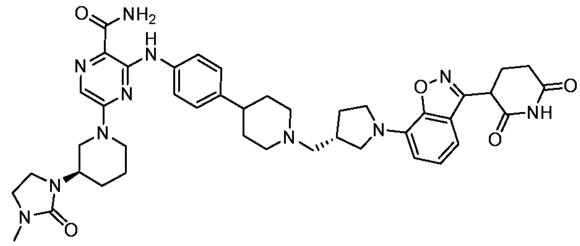

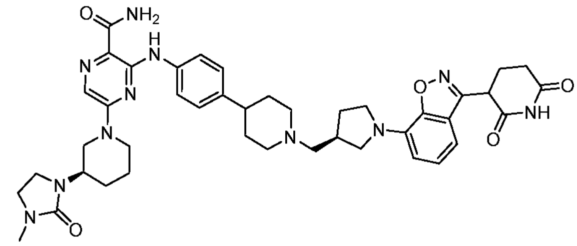

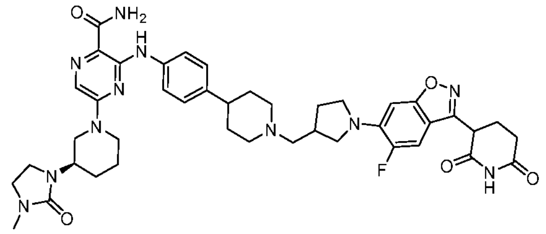

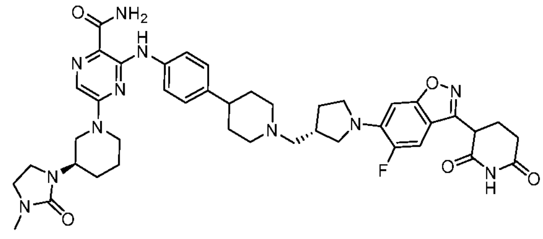

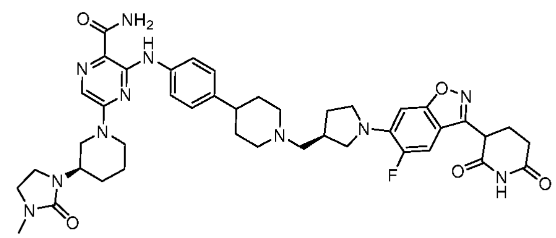

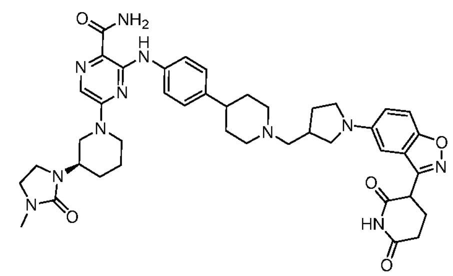

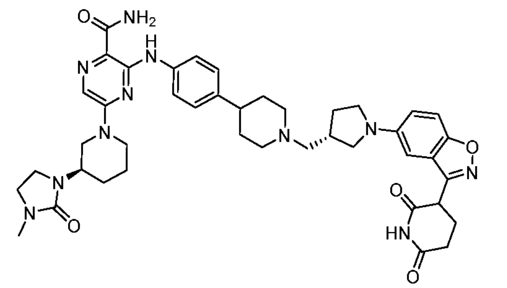

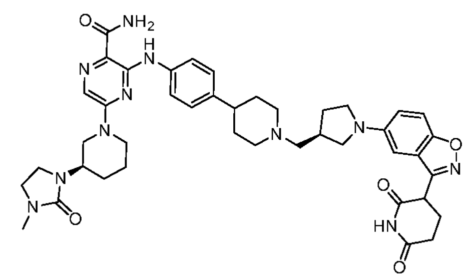

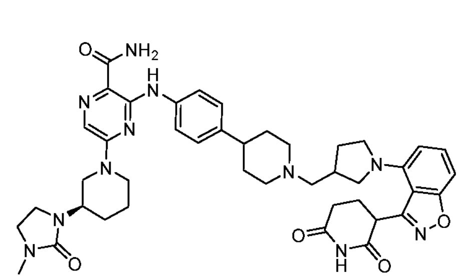

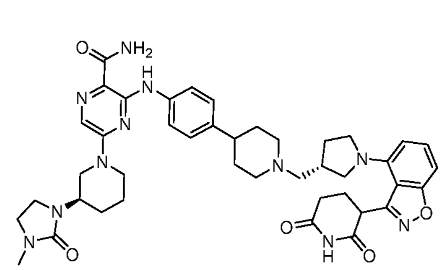

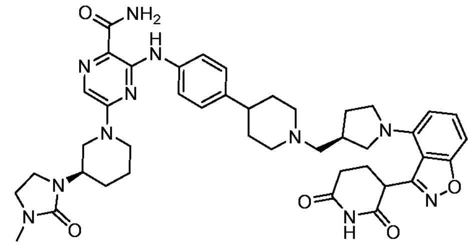

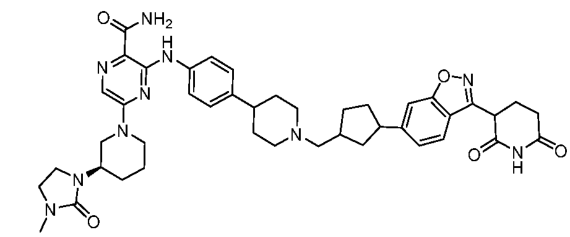

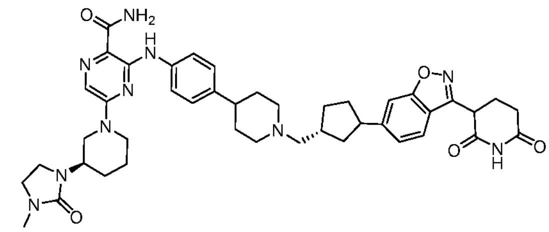

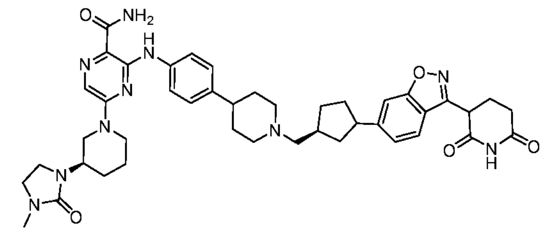

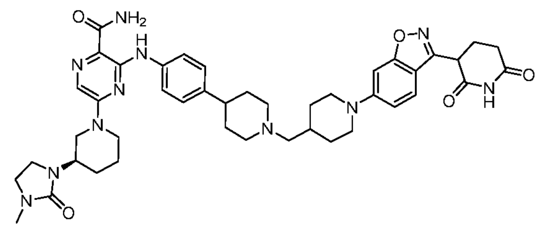

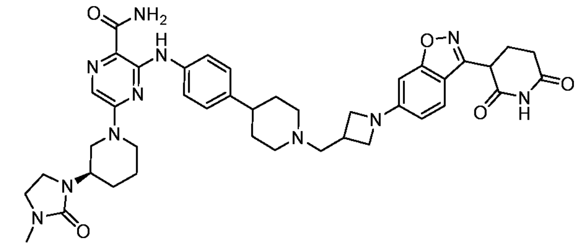

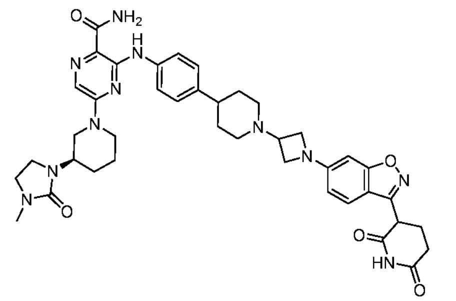

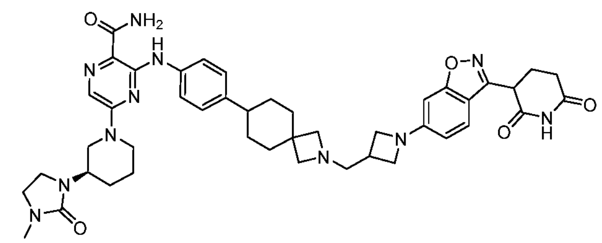

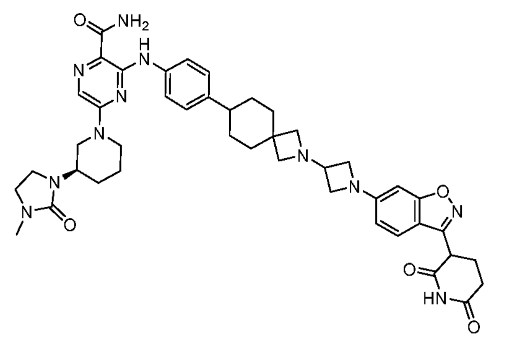

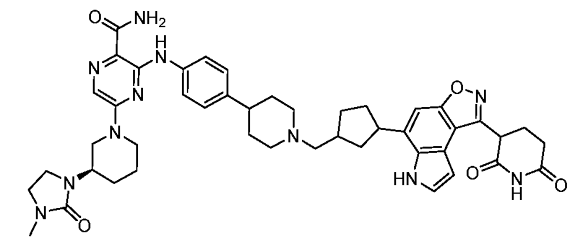

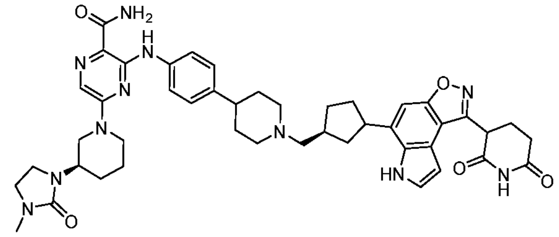

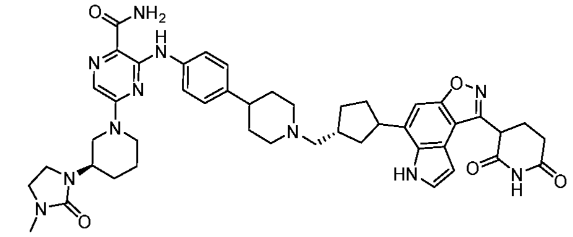

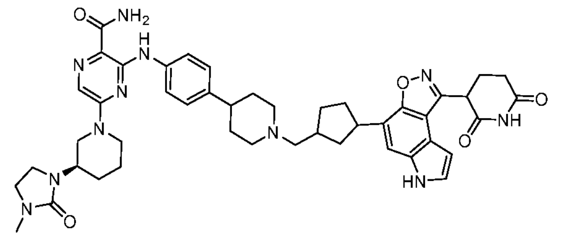

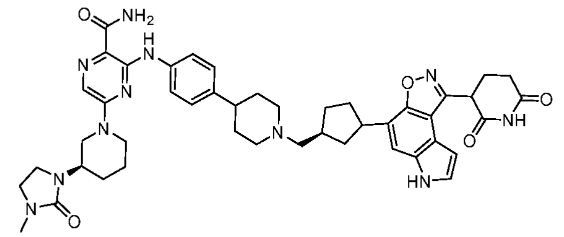

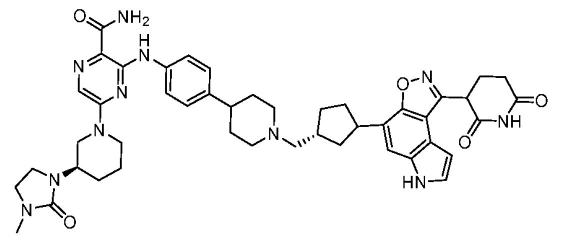

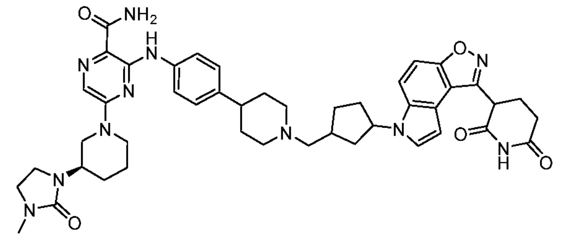

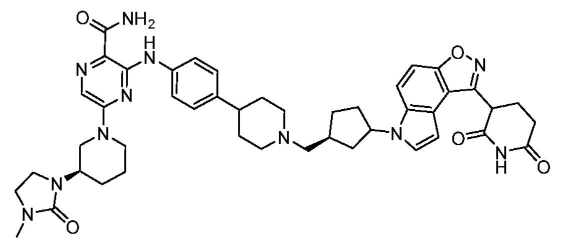

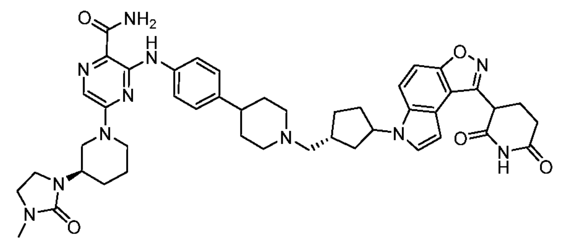

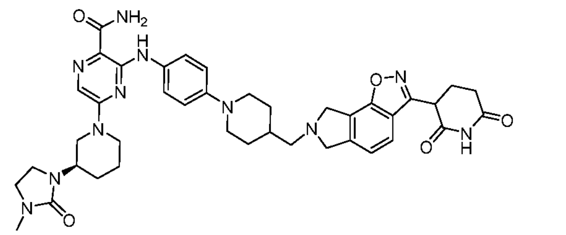

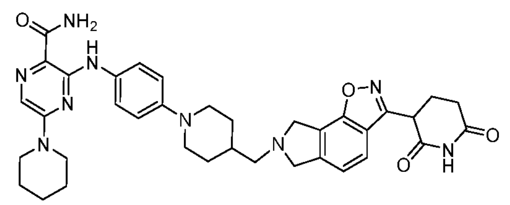

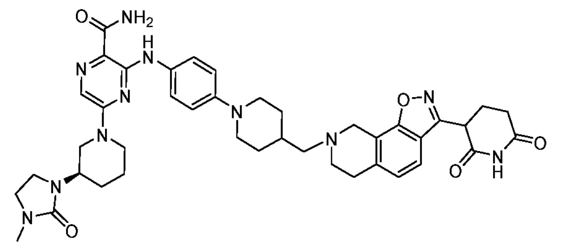

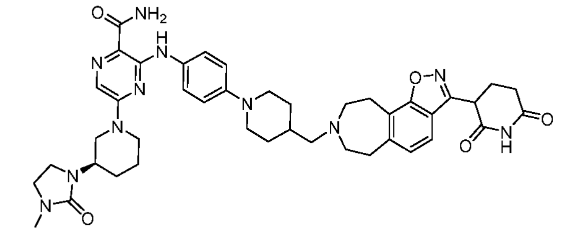

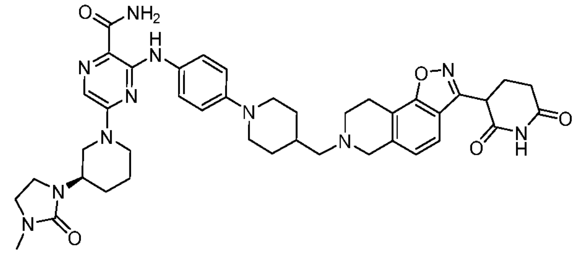

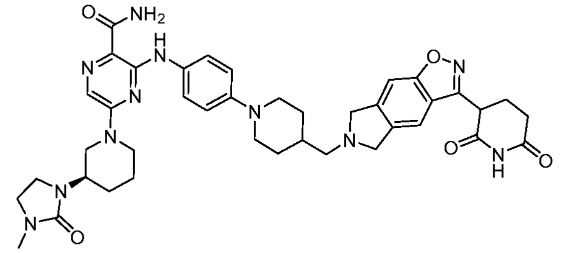

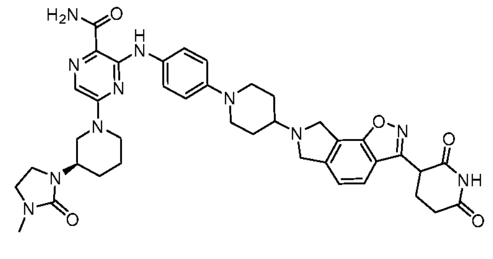

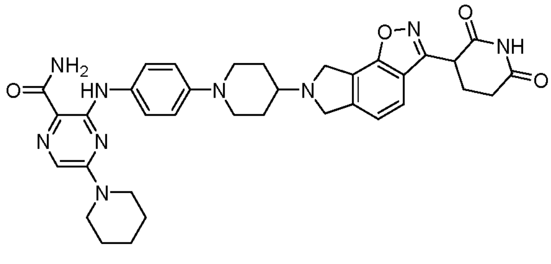

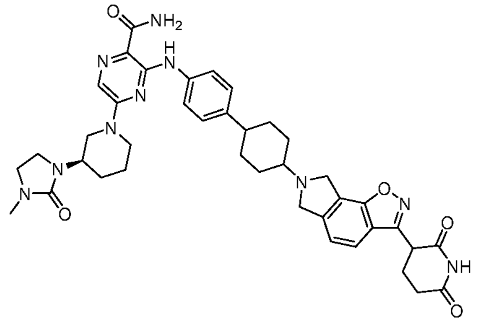

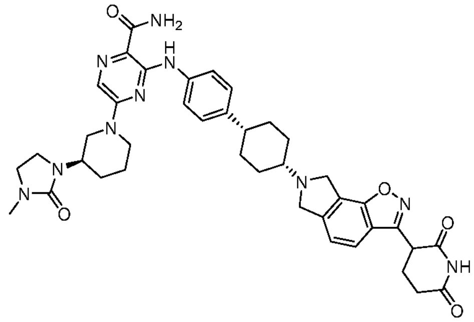

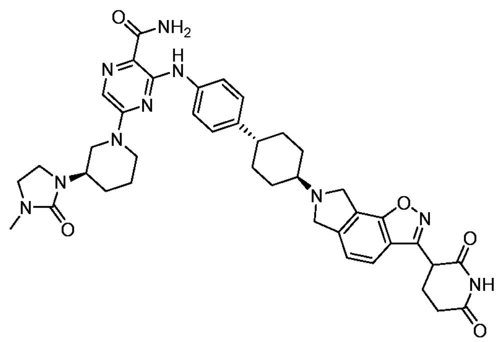

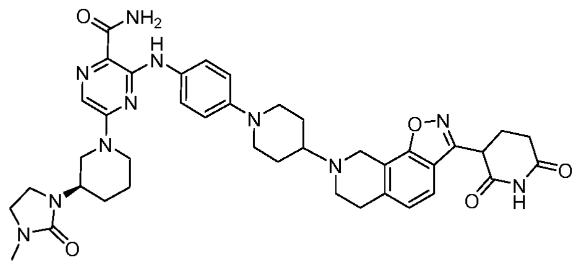

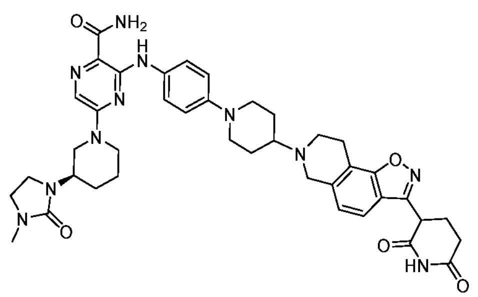

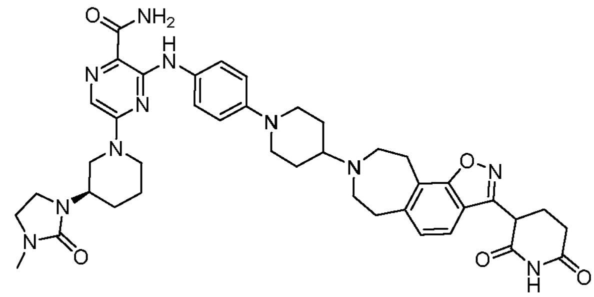

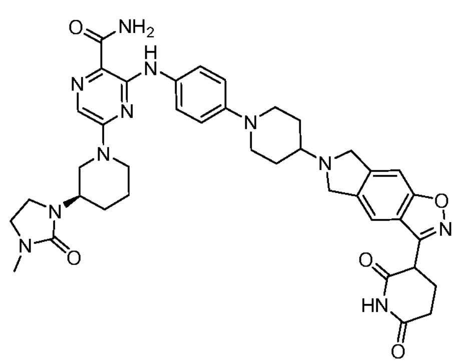

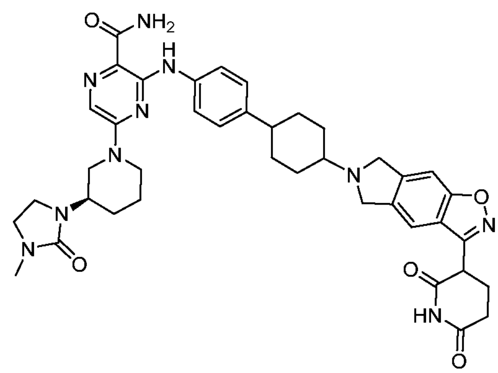

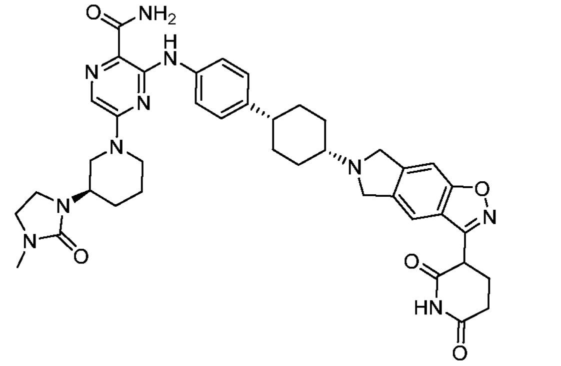

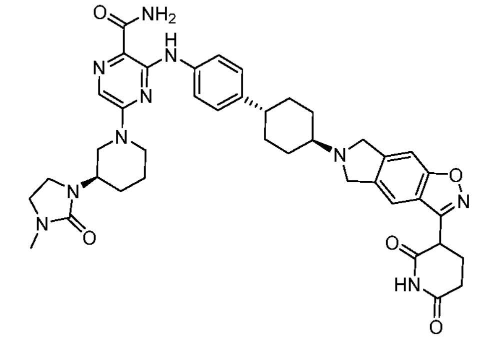

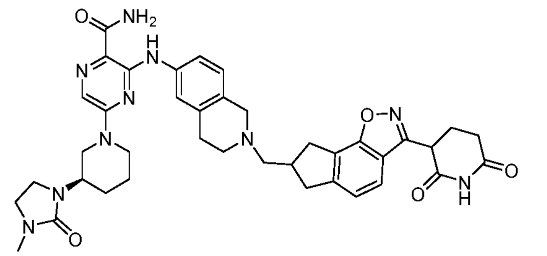

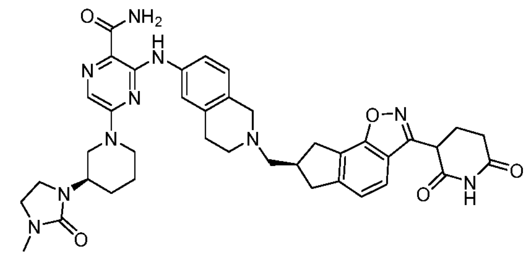

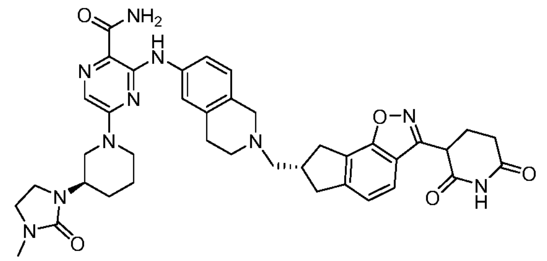

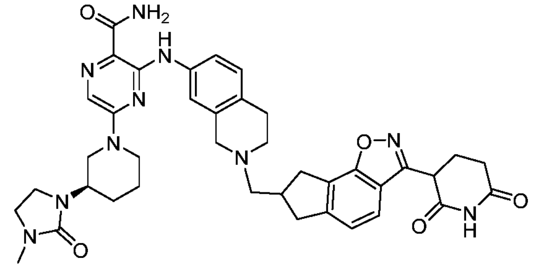

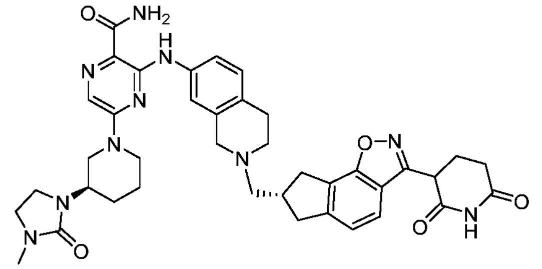

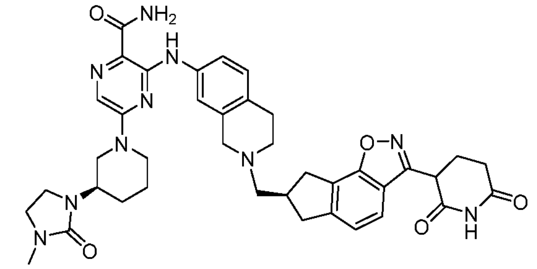

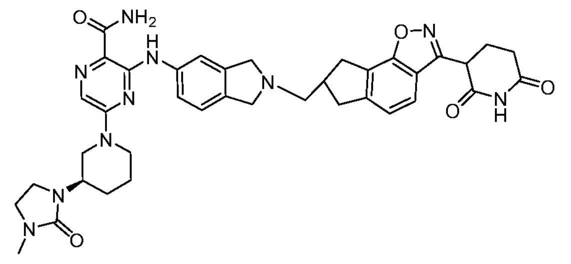

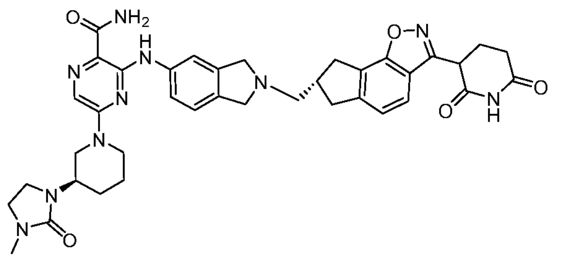

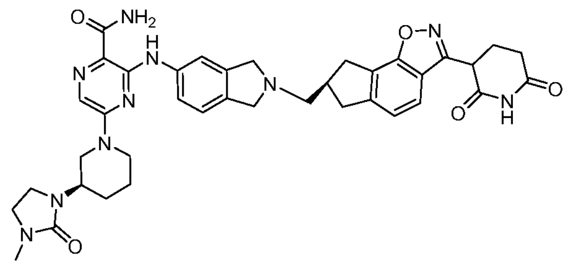

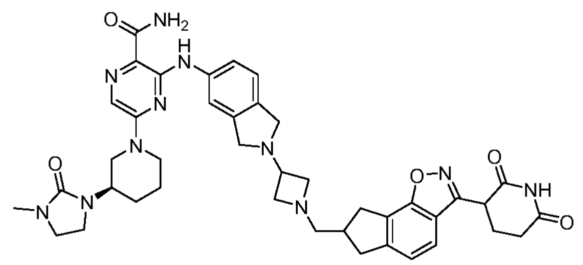

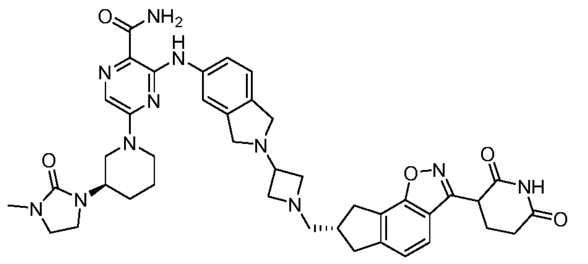

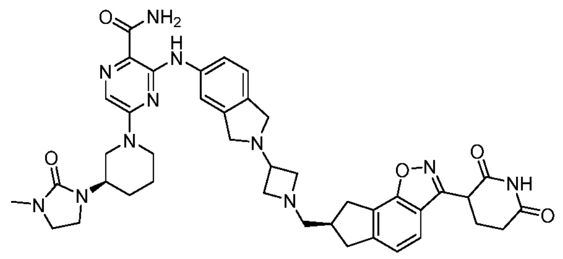

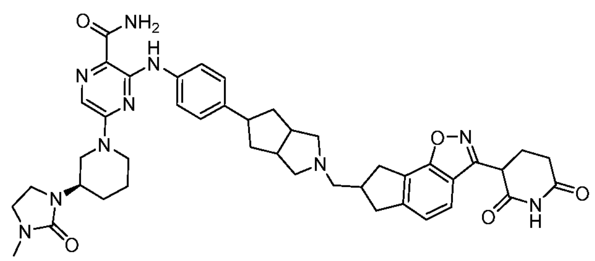

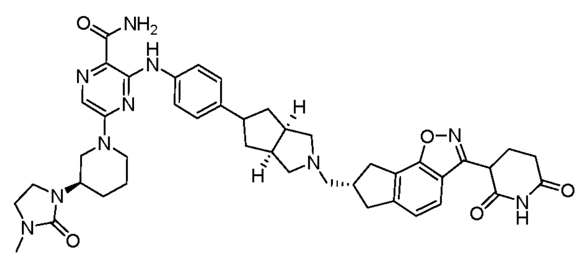

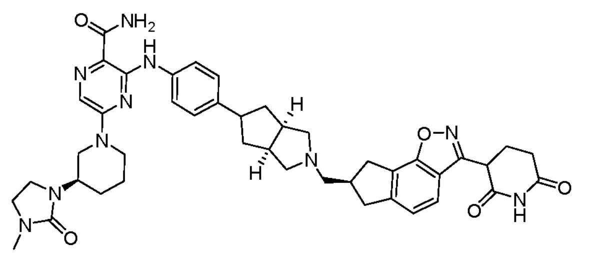

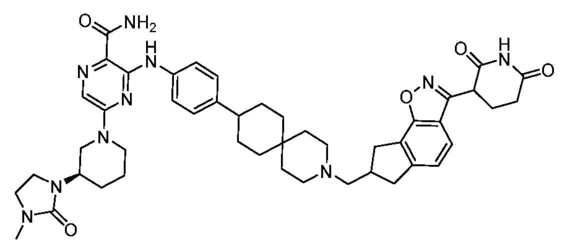

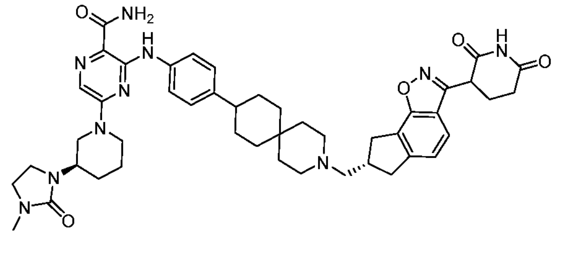

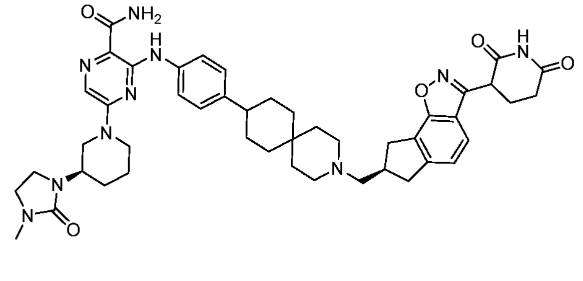

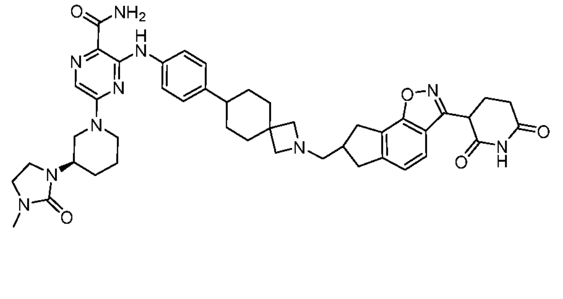

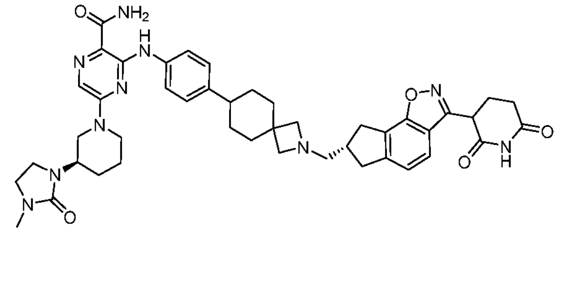

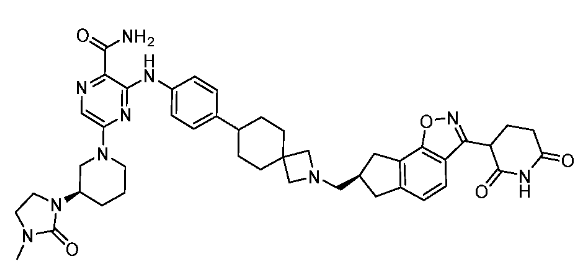

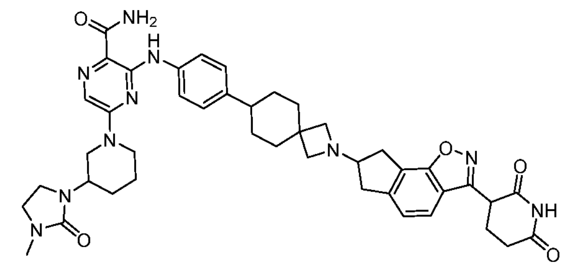

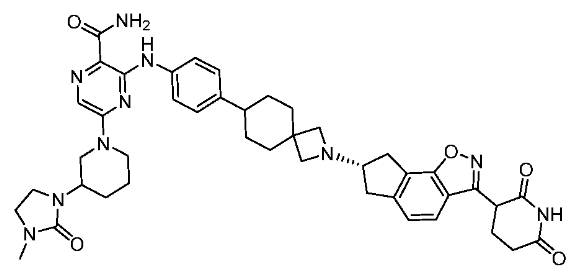

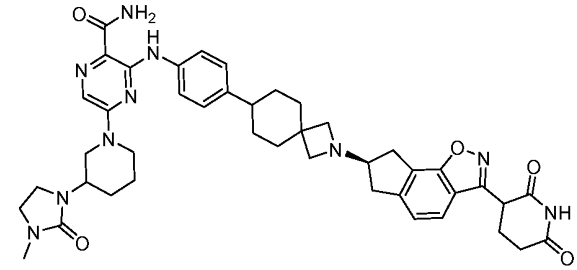

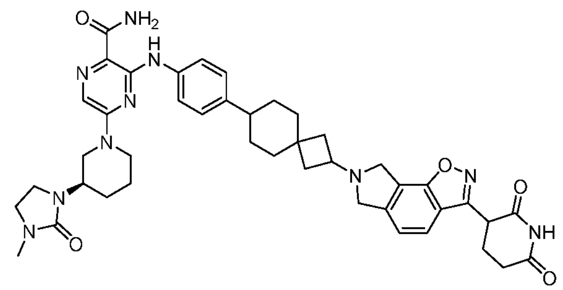

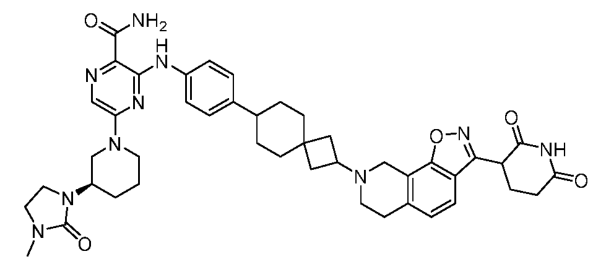

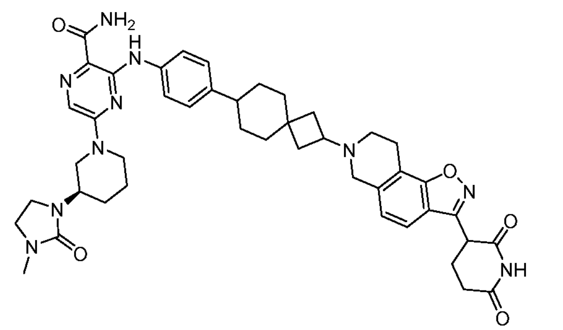

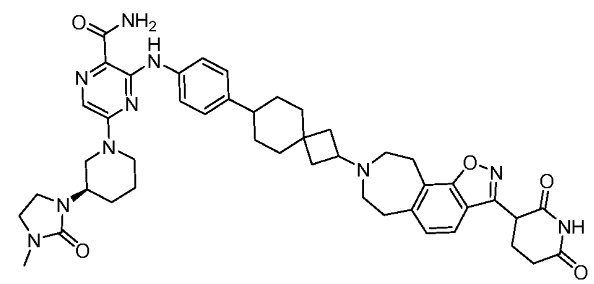

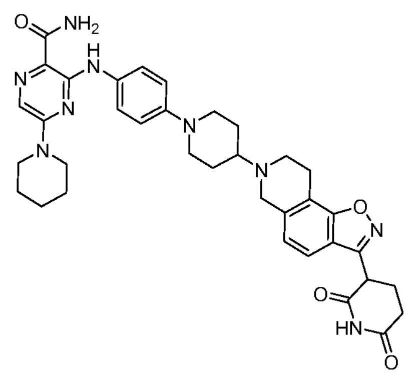

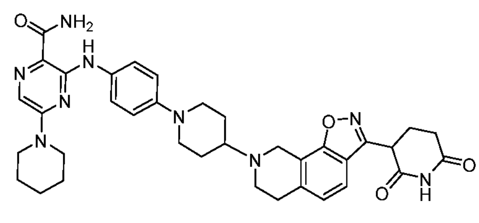

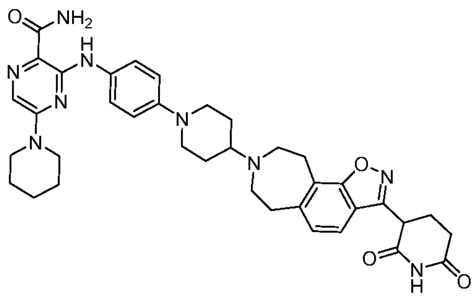

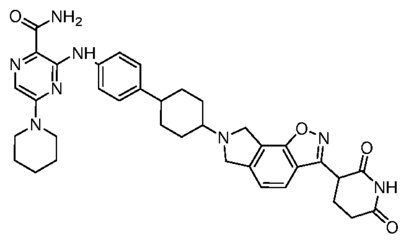

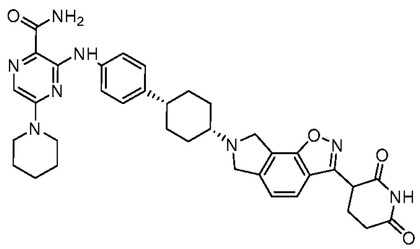

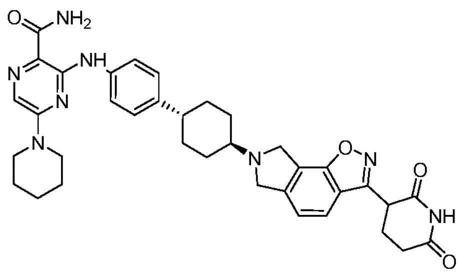

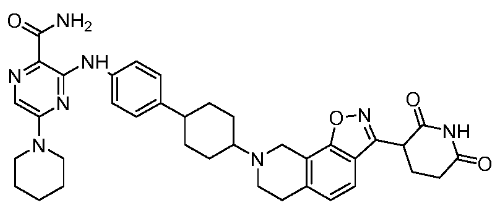

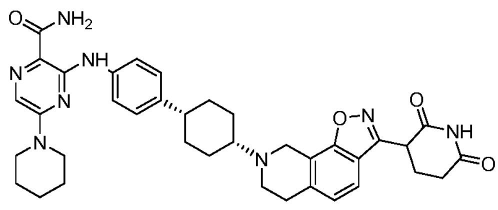

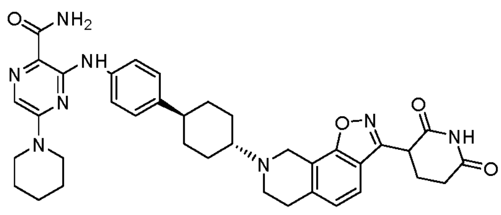

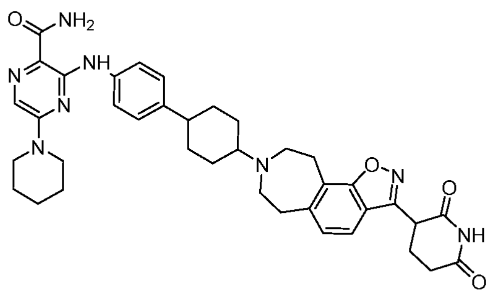

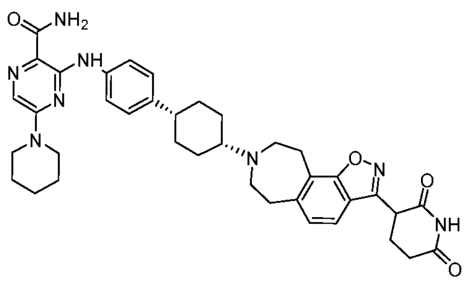

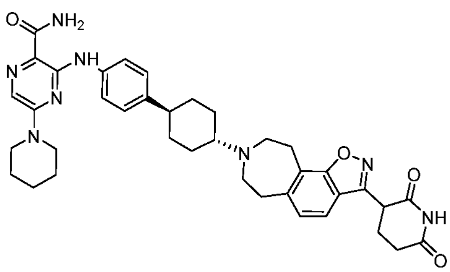

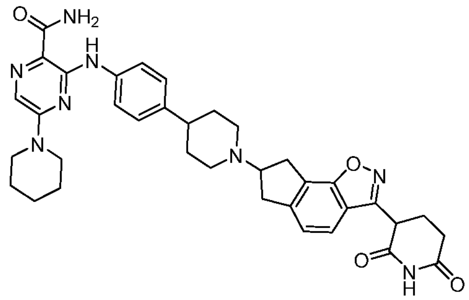

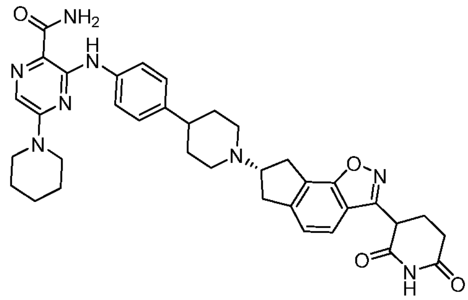

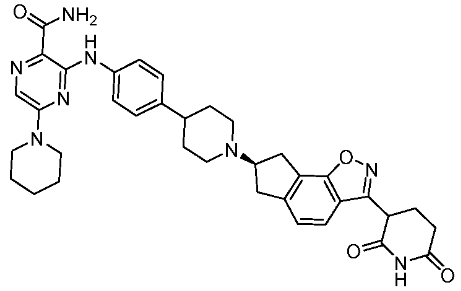

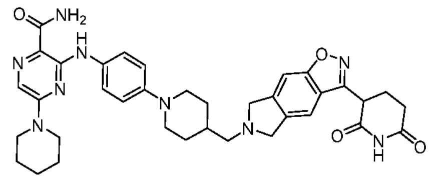

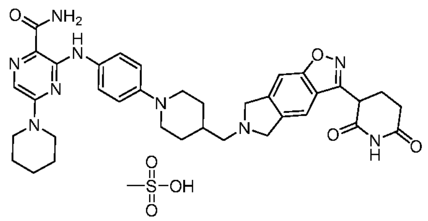

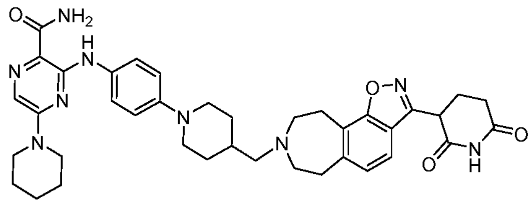

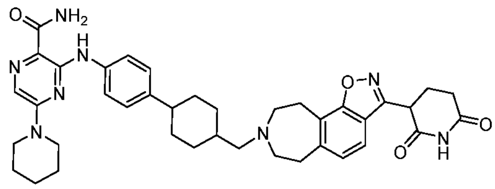

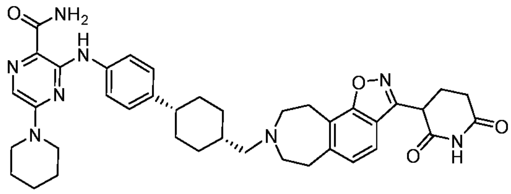

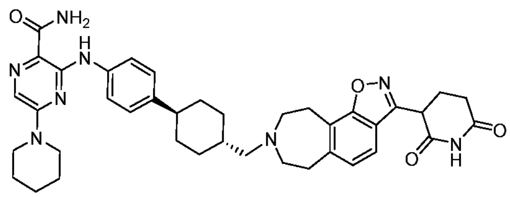

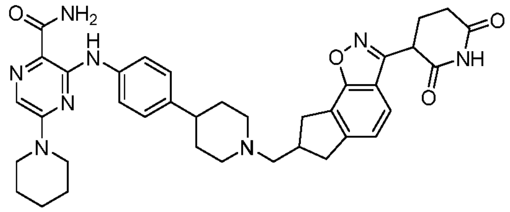

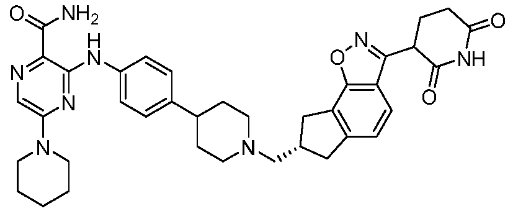

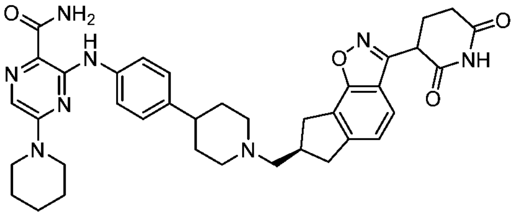

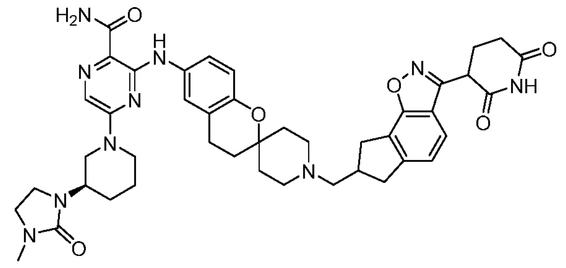

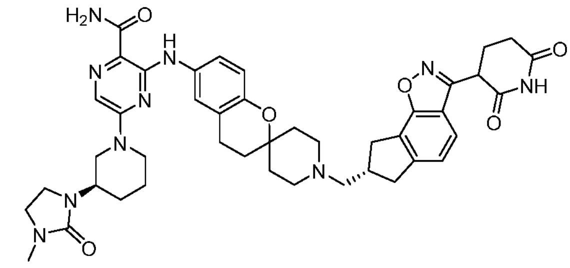

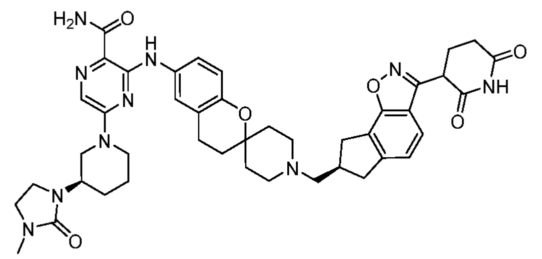

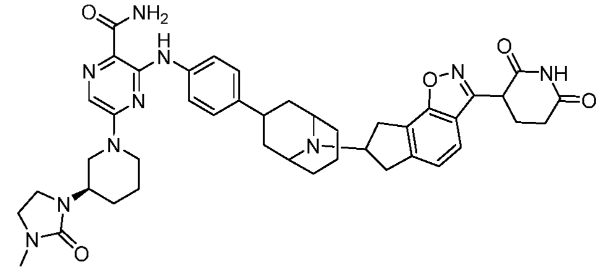

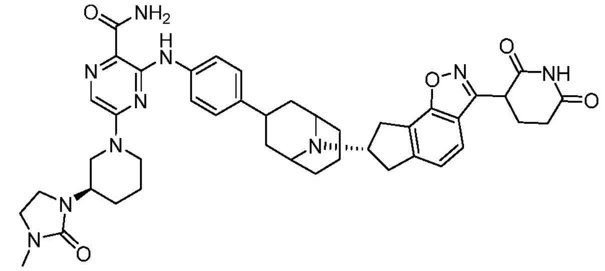

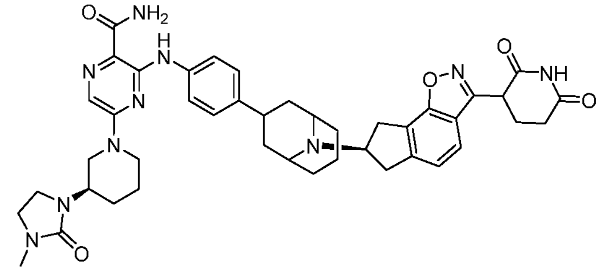

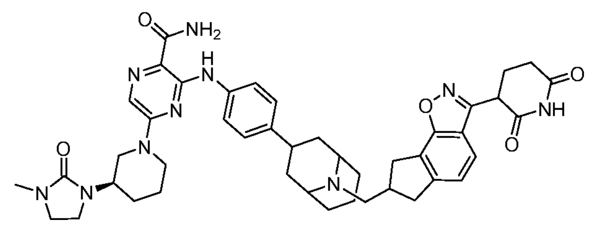

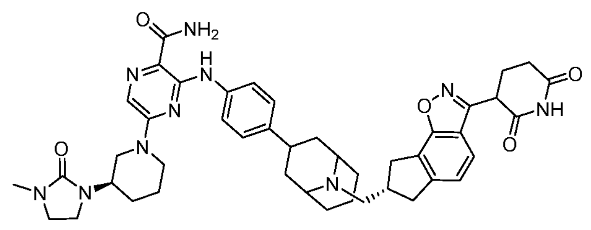

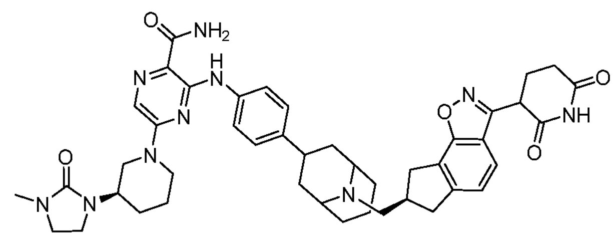

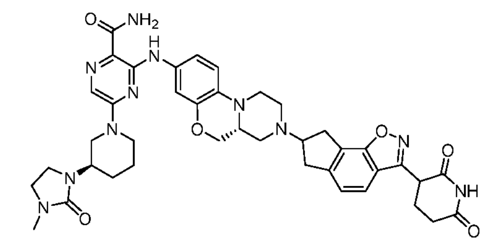

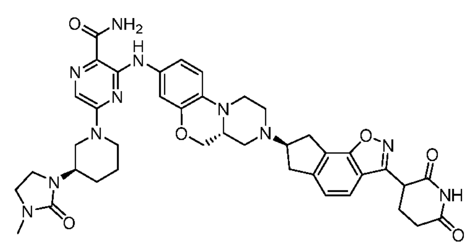

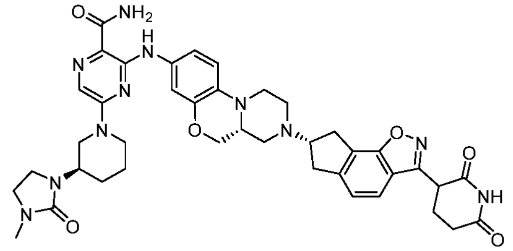

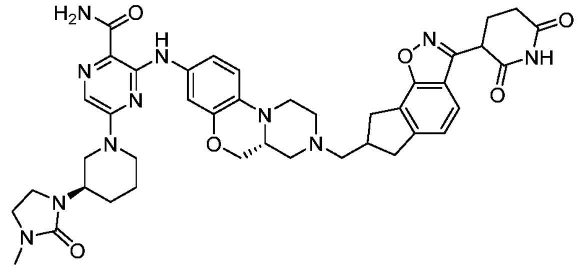

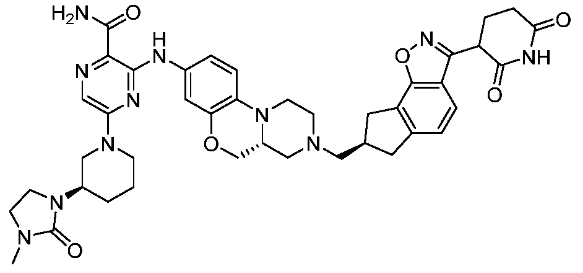

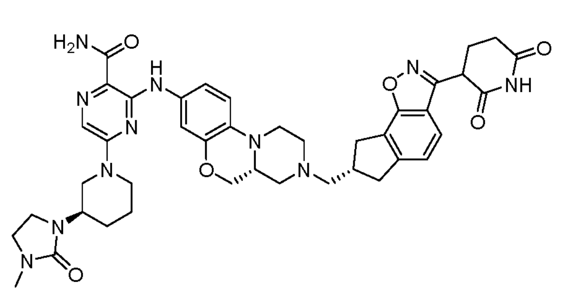

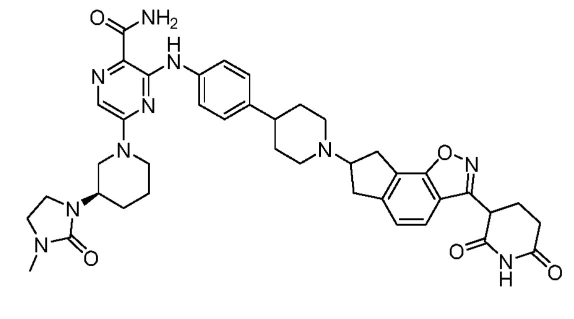

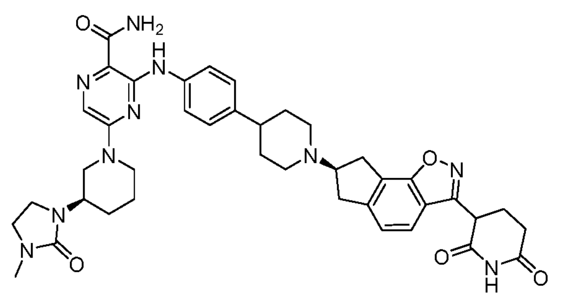

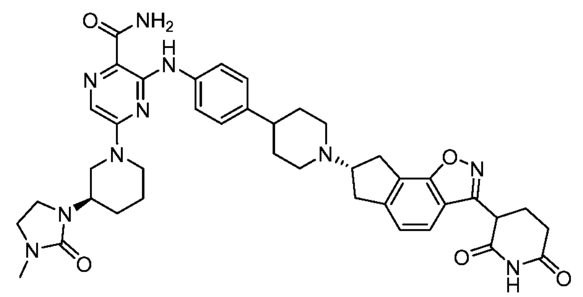

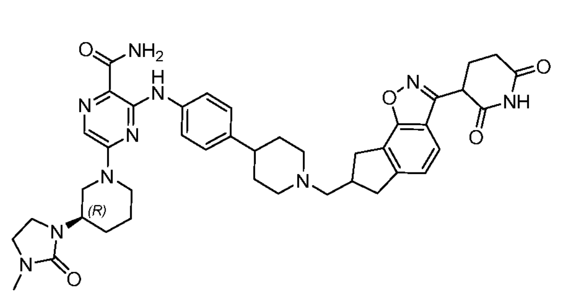

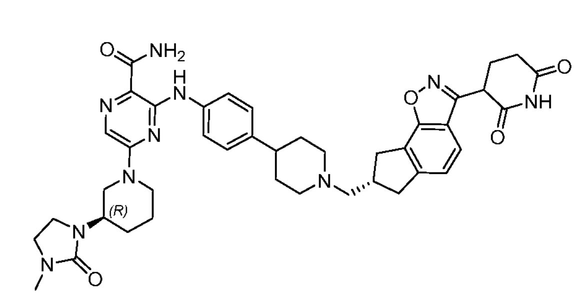

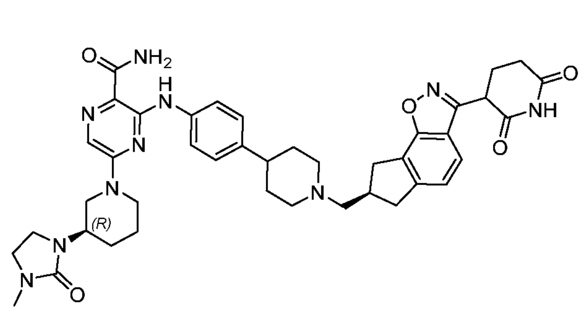

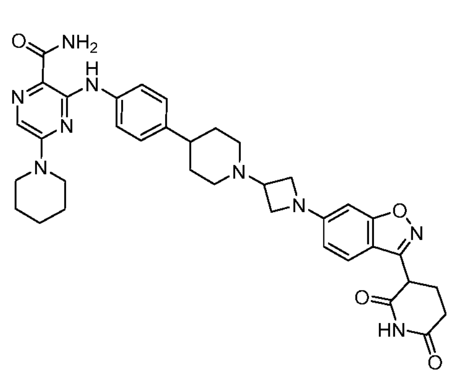

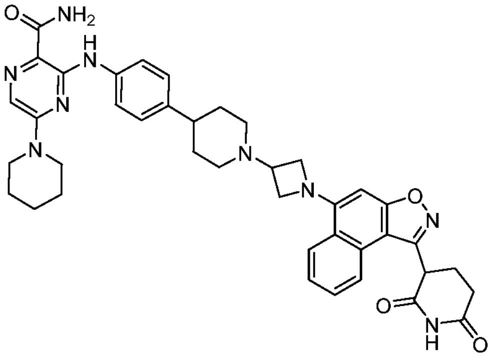

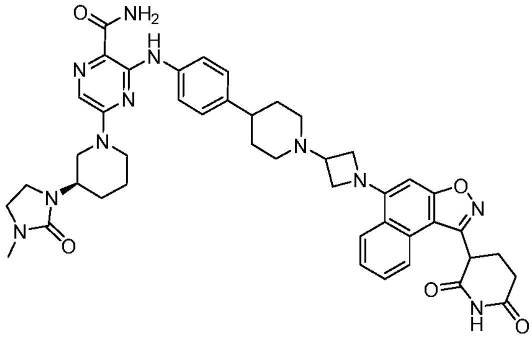

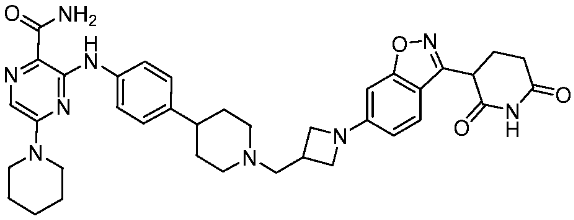

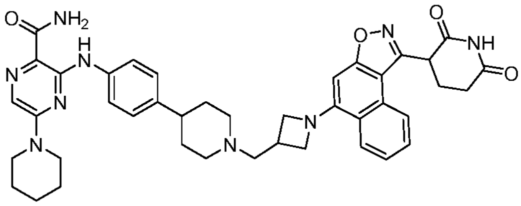

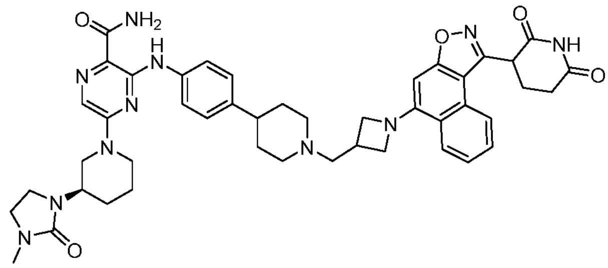

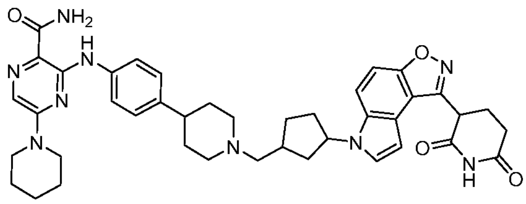

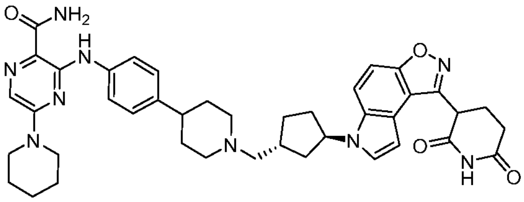

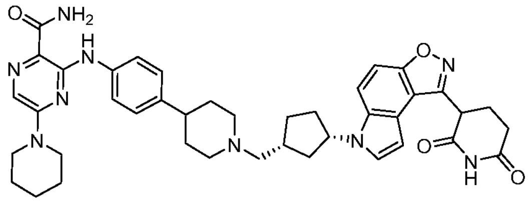

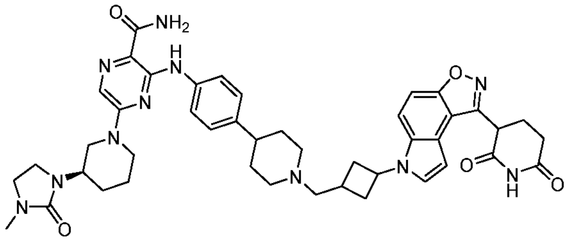

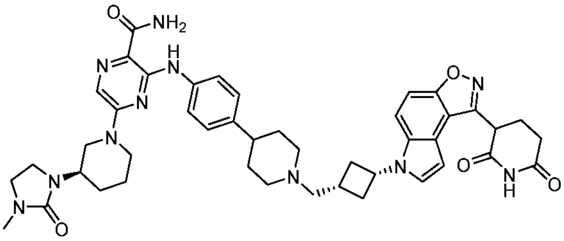

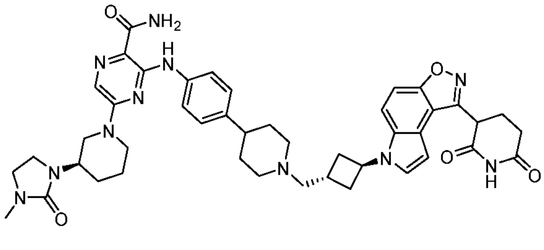

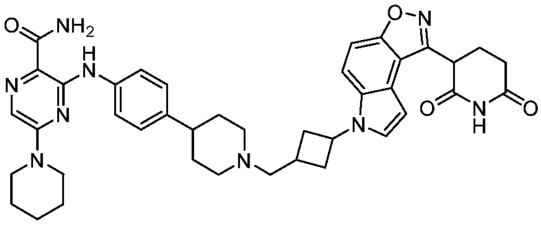

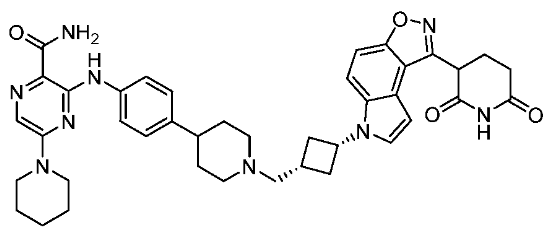

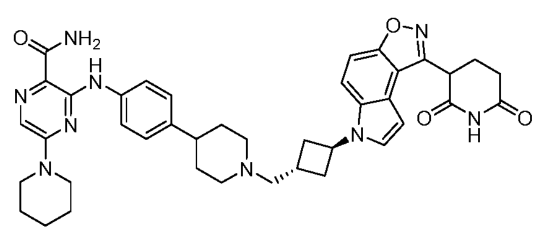

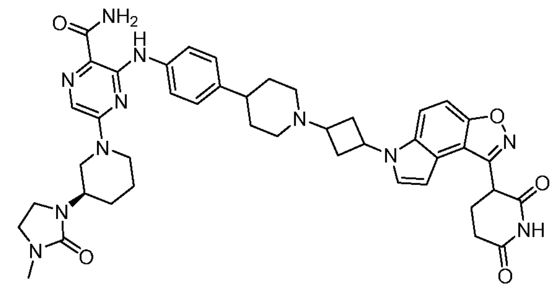

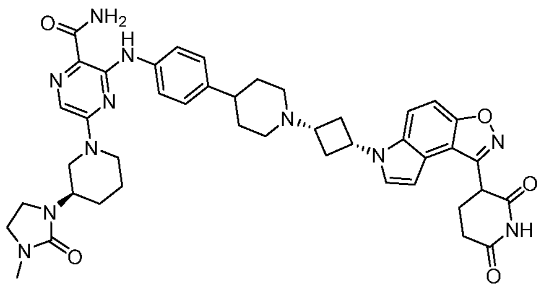

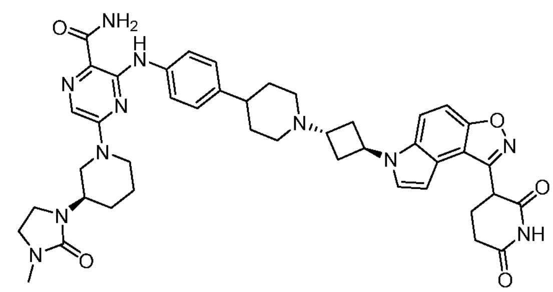

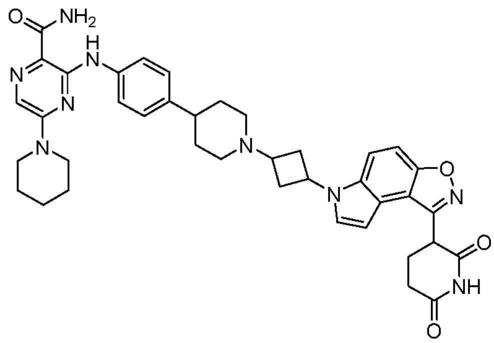

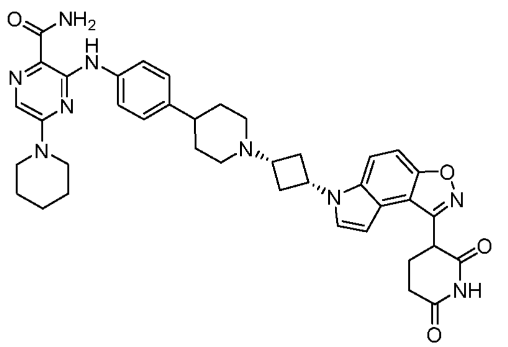

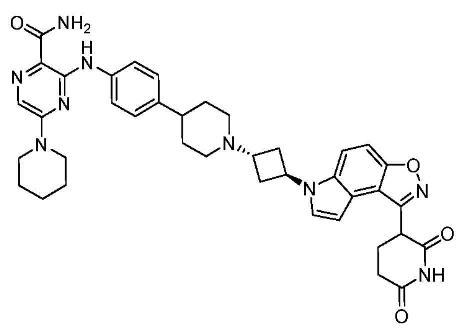

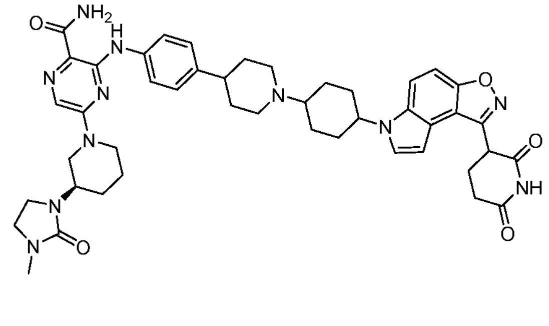

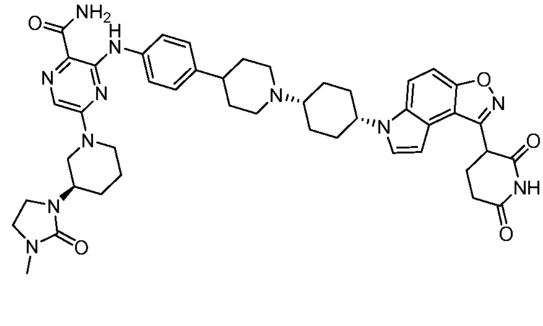

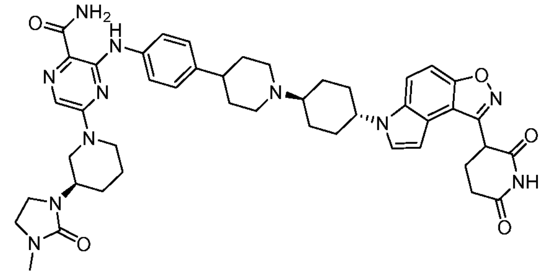

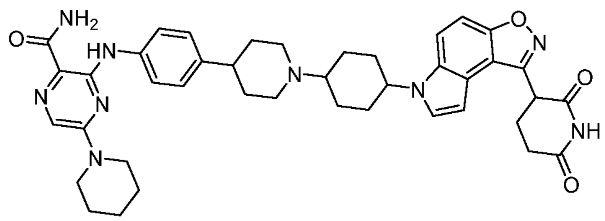

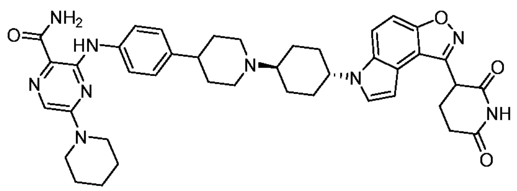

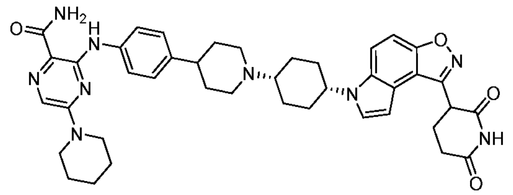

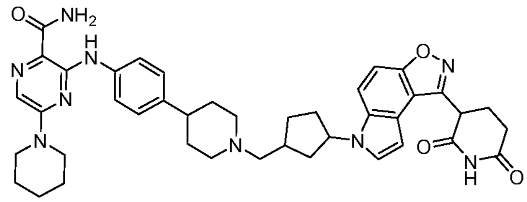

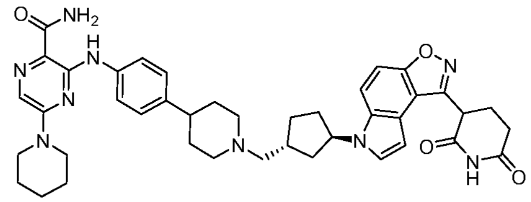

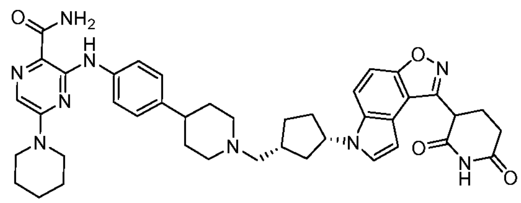

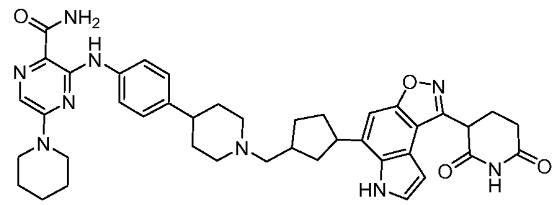

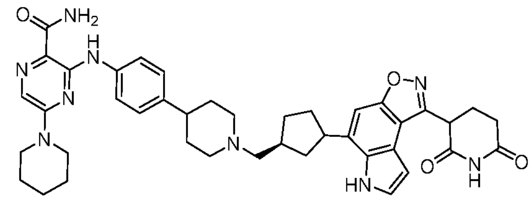

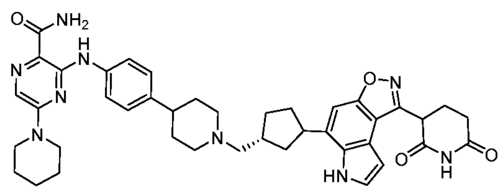

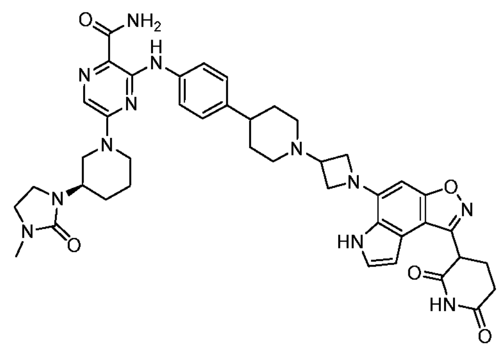

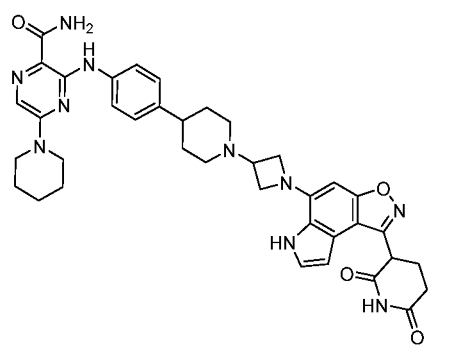

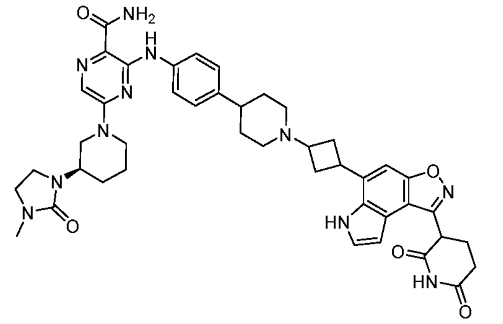

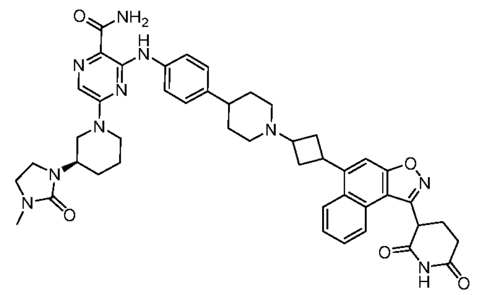

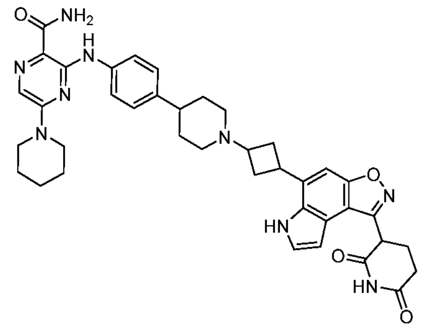

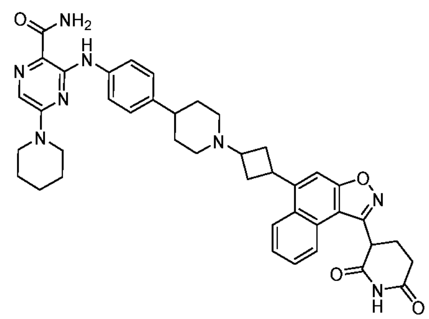

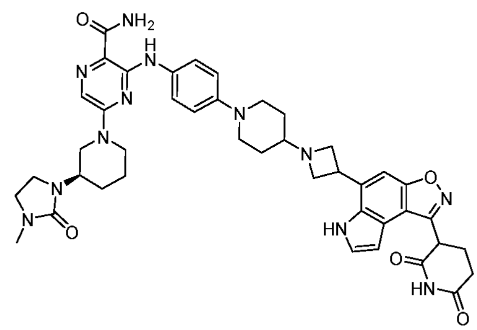

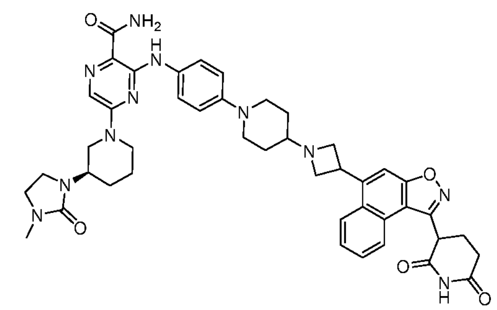

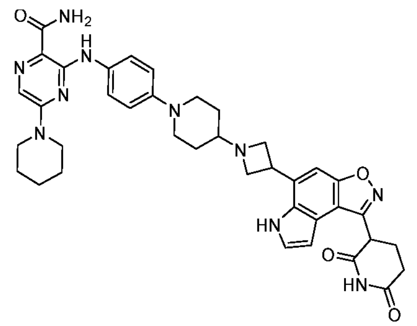

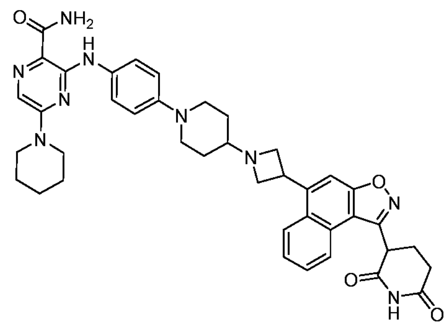

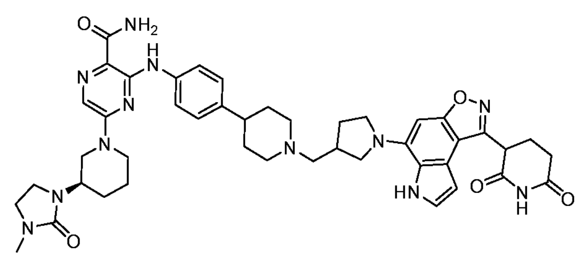

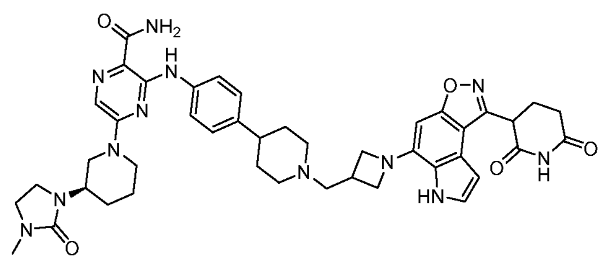

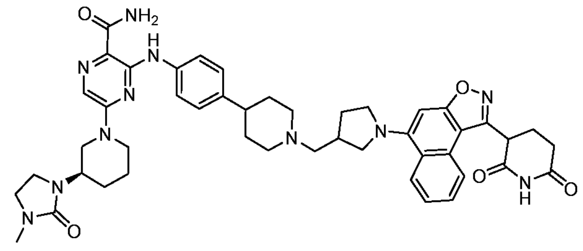

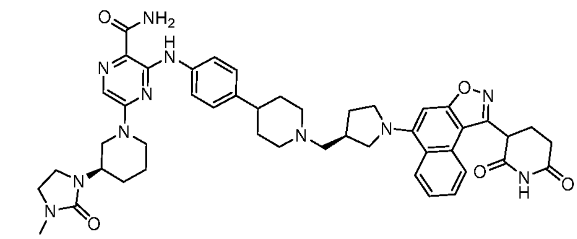

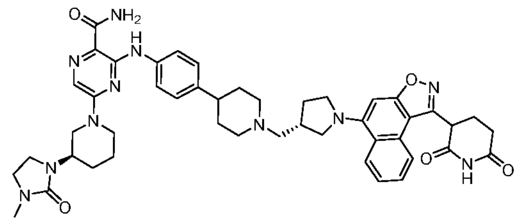

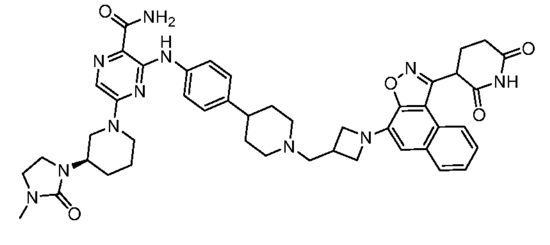

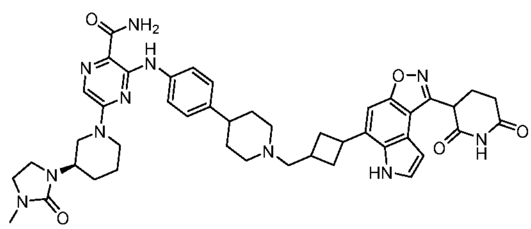

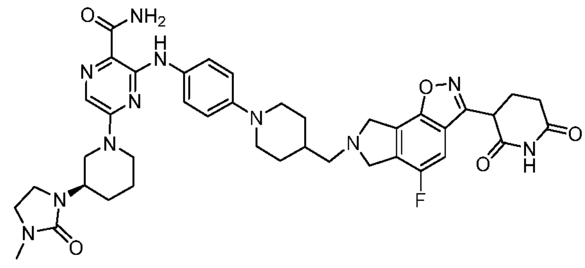

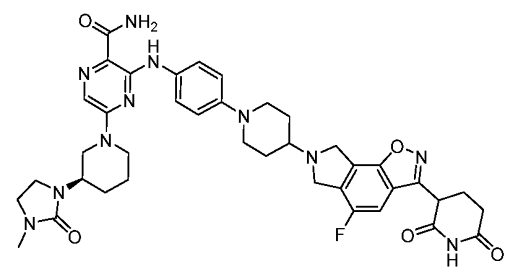

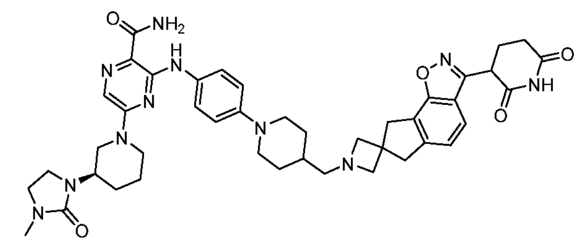

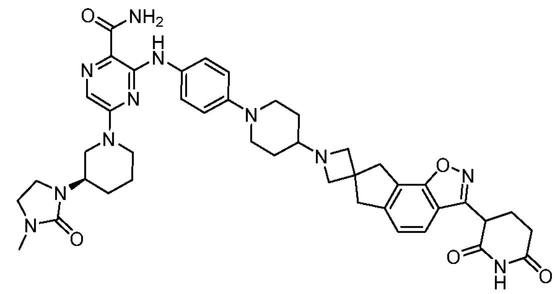

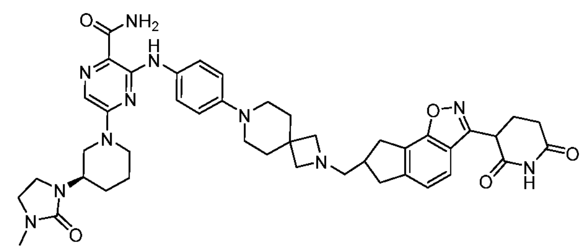

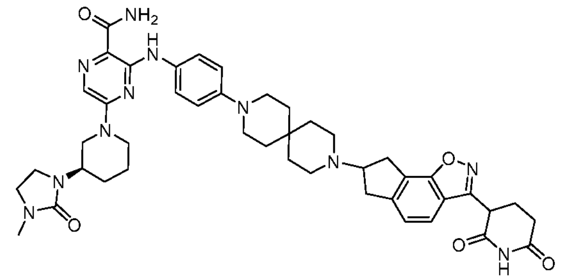 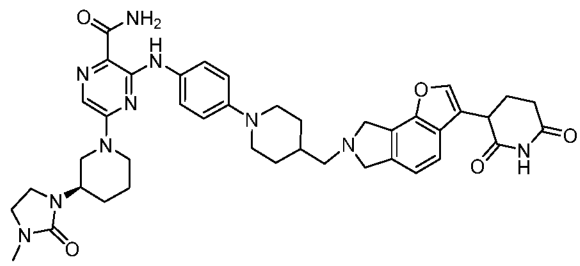

or

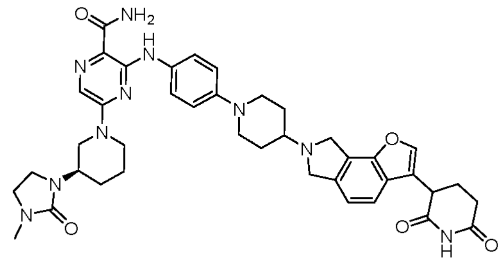.

**22.** A compound of formula I", a stereoisomer thereof, or a pharmaceutically acceptable salt thereof,

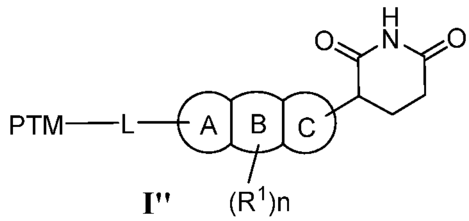

I''

wherein,

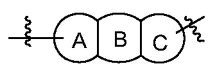

is selected from the group consisting of

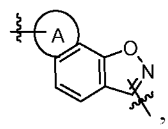, 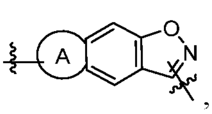, 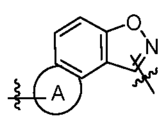,

and

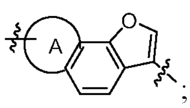;

ring A is selected from the group consisting of $C_{5-10}$ cycloalkenyl, 5- to 10-membered heterocycloalkenyl, phenyl, and 5- to 6-membered heteroaryl;
PTM is as defined in claim 1 or 2;
L is selected from a connecting group;

R' and n are as defined in any one of claims 1 and 9-10.

**23.** The compound, the stereoisomer thereof, or the pharmaceutically acceptable salt thereof according to claim 22, wherein ring A is selected from the group consisting of $C_{5-8}$ cycloalkenyl, 5- to 10-membered heterocycloalkenyl, phenyl, and 5- to 6-membered heteroaryl; or ring A is selected from the group consisting of $C_{5-7}$ cycloalkenyl, 5- to 10-membered heterocycloalkenyl, phenyl, and 5- to 6-membered heteroaryl; or ring A is selected from the group consisting of $C_{5-6}$ cycloalkenyl, 5- to 10-membered heterocycloalkenyl, phenyl, and 5- to 6-membered heteroaryl; or ring A is selected from the group consisting of $C_{5-6}$ cycloalkenyl, 5- to 9-membered heterocycloalkenyl, phenyl, pyrrolyl, pyrazolyl, furanyl, and oxazolyl; or

ring A is selected from the group consisting of $C_5$ cycloalkenyl, $C_6$ cycloalkenyl, 5-, 6-, 7-, 8-, or 9-membered heterocycloalkenyl, phenyl, pyrrolyl, pyrazolyl, furanyl, and oxazolyl; or
ring A is selected from the group consisting of cyclopentenyl, bicyclohexenyl, dihydropyrrolyl, tetrahydropyridinyl, tetrahydroazepinyl, azaspirooctenyl, dihydrooxazinyl, azaspirononenyl, phenyl, pyrrolyl, pyrazolyl, furanyl, and oxazolyl; or
ring A is selected from the group consisting of $C_{5-6}$ cycloalkenyl and 5- to 9-membered heterocycloalkenyl; or ring A is selected from the group consisting of cyclopentenyl, bicyclohexenyl, dihydropyrrolyl, tetrahydropyridinyl, dihydrooxazinyl, tetrahydroazepinyl, azaspirooctenyl, and azaspirononenyl.

**24.** The compound, the stereoisomer thereof, or the pharmaceutically acceptable salt thereof according to any one of claims 1-23, wherein PTM is selected from

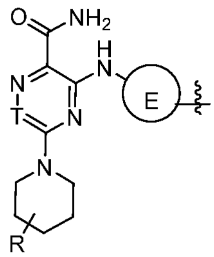
;

or, PTM is selected from

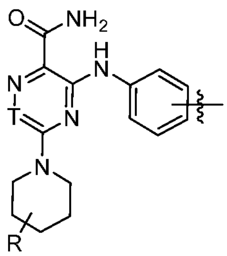
.

**25.** A compound of formula I'''-1a or I'''-2a, a moiety, a stereoisomer thereof, a derivative thereof, or a pharmaceutically acceptable salt thereof: wherein,

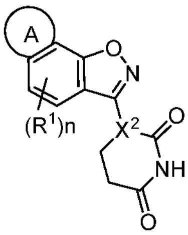
I'''-1a,
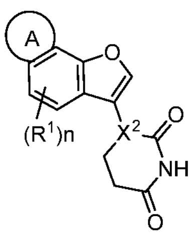
I'''-2a,

ring A is selected from the group consisting of $C_{5-10}$ cycloalkenyl and 5- to 10-membered heterocycloalkenyl;
n is as defined in claim 1 or 12;

each R' is independently selected from the group consisting of halogen, -OH, $-NH_2$, -CN, =O, $C_{1-4}$ alkoxy, -CHO, $C_{3-6}$ cycloalkyl, 3- to 10-membered heterocycloalkyl, and $C_{1-4}$ alkyl, wherein the $C_{3-6}$ cycloalkyl, 3- to 10-membered heterocycloalkyl, or $C_{1-4}$ alkyl is optionally substituted with halogen, -OH, $-NH_2$, or $C_{1-4}$ alkyl-OH; or, each R' is independently selected from the group consisting of halogen, -OH, $-NH_2$, -CN, =O, $C_{1-4}$ alkoxy, -CHO, $C_{3-6}$ cycloalkyl, and $C_{1-4}$ alkyl, wherein the $C_{3-6}$ cycloalkyl or $C_{1-4}$ alkyl is optionally substituted with halogen, -OH, $-NH_2$, or $C_{1-4}$ alkyl-OH;
$X^2$ is selected from the group consisting of CH and N.

**26.** The compound, the stereoisomer thereof, the derivative thereof, or the pharmaceutically acceptable salt thereof according to claim 25, wherein each R' is independently selected from the group consisting of halogen, -OH, $-NH_2$, -CN, =O, $C_{1-3}$ alkoxy, -CHO, $C_{3-4}$ cycloalkyl, and $C_{1-3}$ alkyl, wherein the $C_{3-4}$ cycloalkyl or $C_{1-3}$ alkyl is optionally substituted with halogen, -OH, $-NH_2$, or $C_{1-3}$ alkyl-OH; or

each R' is independently selected from the group consisting of halogen, -OH, $-NH_2$, -CN, =O, methoxy, -CHO, cyclobutyl, and methyl, wherein the cyclobutyl or methyl is optionally substituted with halogen, -OH, $-NH_2$, or $CH_2OH$; or
each R' is independently selected from the group consisting of F, -OH, $-NH_2$, $-CH_2OH$, =O, -CHO, $-CH_2NH_2$, and

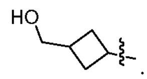.

**27.** The compound, the moiety, the stereoisomer thereof, the derivative thereof, or the pharmaceutically acceptable salt thereof according to claim 25, wherein the compound of formula I'''-1a or I'''-2a is selected from a compound of formula 1'''-1 or 1'''-2, respectively:

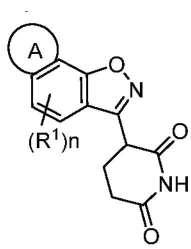 I'''-1, 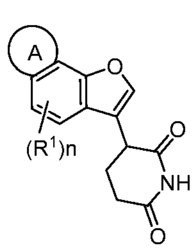 I'''-2,

wherein,

ring A is selected from the group consisting of $C_{5-10}$ cycloalkenyl and 5- to 10-membered heterocycloalkenyl; optionally, R' and n are as defined in any one of claims 1 and 11-12.

**28.** The compound, the moiety, the isomer thereof, the derivative thereof, or the pharmaceutically acceptable salt thereof according to claim 27, wherein ring A is selected from the group consisting of $C_{5-8}$ cycloalkenyl and 5- to 10-membered heterocycloalkenyl; or ring A is selected from the group consisting of $C_{5-7}$ cycloalkenyl and 5- to 10-membered heterocycloalkenyl; or ring A is selected from the group consisting of $C_{5-6}$ cycloalkenyl and 5- to 10-membered heterocycloalkenyl; or ring A is selected from the group consisting of $C_{5-6}$ cycloalkenyl and 5- to 9-membered heterocycloalkenyl; or

ring A is selected from the group consisting of $C_5$ cycloalkenyl, $C_6$ cycloalkenyl, and 5-, 6-, 7-, 8-, or 9-membered heterocycloalkenyl; or
ring A is selected from the group consisting of cyclopentenyl, bicyclohexenyl, dihydropyrrolyl, tetrahydropyridinyl, tetrahydroazepinyl, dihydrooxazinyl, azaspirooctenyl, and azaspirononenyl; or
ring A is selected from the group consisting of

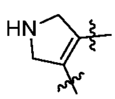, 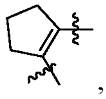, 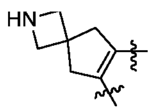, 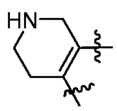, 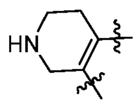,

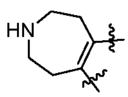, 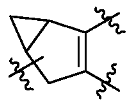, 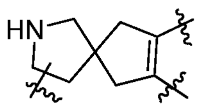, 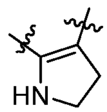, 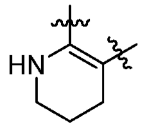, and 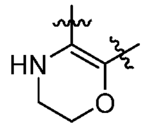;

or

ring A is selected from the group consisting of

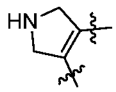, 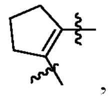, 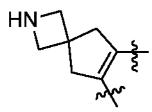, 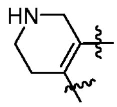, 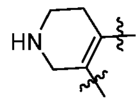,

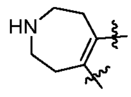, 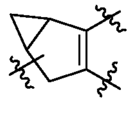, and 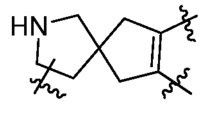.

**29.** A compound of the following formula, a moiety, a stereoisomer thereof, a derivative thereof, or a pharmaceutically acceptable salt thereof:

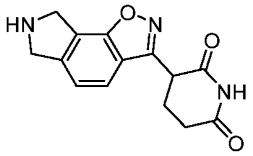, 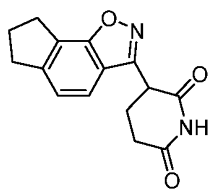, 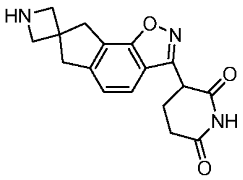, 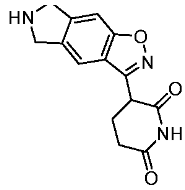, 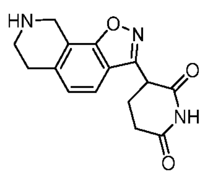,

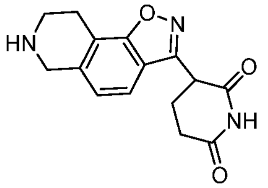, 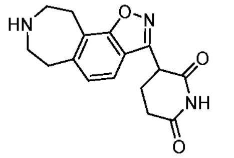, 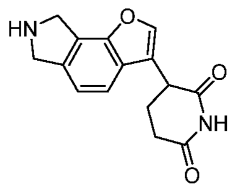, 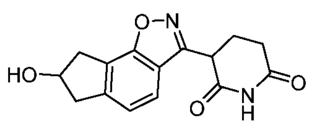,

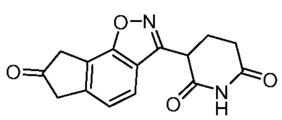, 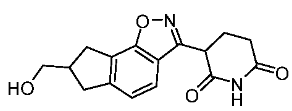, 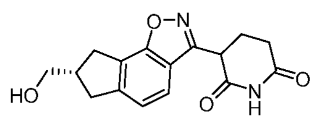,

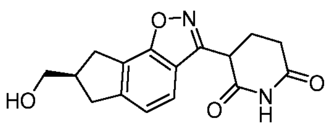, 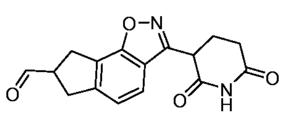, 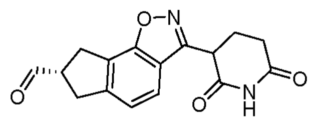,

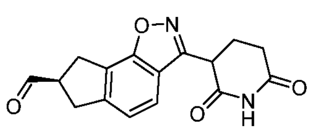, 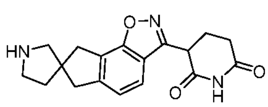, 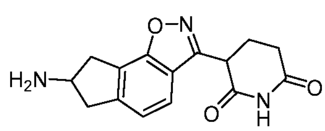, 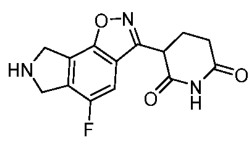,

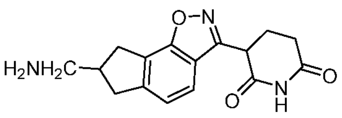, 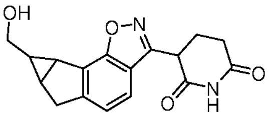, 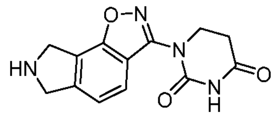, 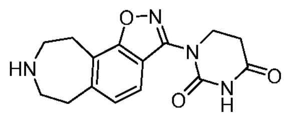,

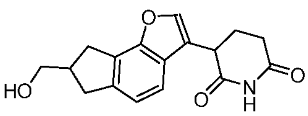, 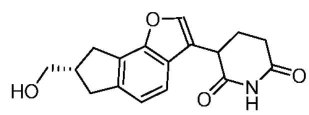, 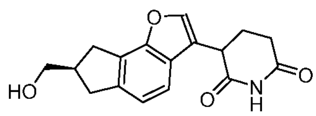,

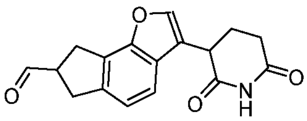, 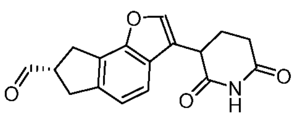, 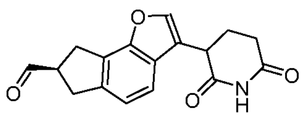, 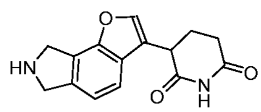,

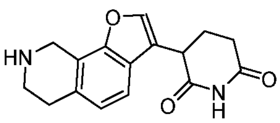, 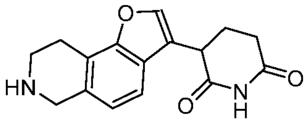, 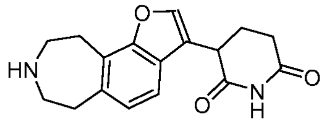, 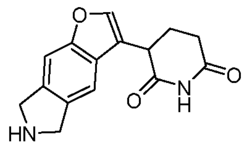,

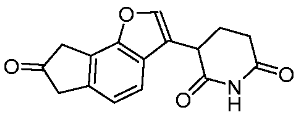, 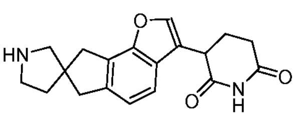, 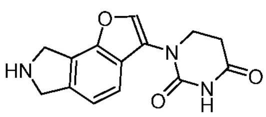, 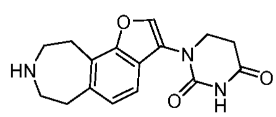,

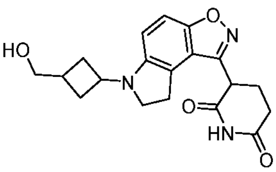, 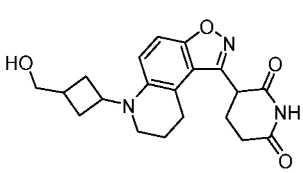, or 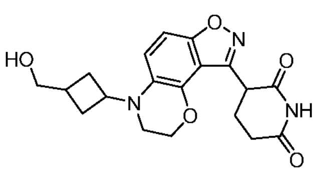.

**30.** A compound of formula I‴-1c or I‴-2d, a moiety, a stereoisomer thereof, a derivative thereof, or a pharmaceutically acceptable salt thereof:

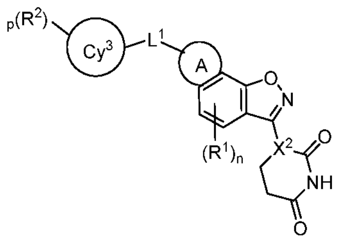 I‴-1c, 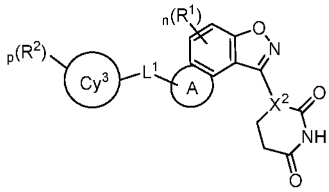 I‴-1d

wherein,

ring A, n, and $R^1$ are as defined in any one of claims 1-2, 9, 11-12, 22-23, 25, and 27-28;
$X^2$ is selected from the group consisting of CH and N;
L' is selected from $C_{0-3}$ alkylene;
$Cy^3$ is selected from the group consisting of $C_{3-8}$ cycloalkyl and 3- to 8-membered heterocycloalkyl;
$R^2$ is selected from the group consisting of -CHO, OH, SH, $NH_2$, COOH, and $C_{1-6}$ alkyl substituted with one or more SH, OH, or $NH_2$;
p is selected from the group consisting of 0, 1, 2, and 3.

**31.** The compound, the moiety, the stereoisomer thereof, the derivative thereof, or the pharmaceutically acceptable salt thereof according to claim 30, wherein ring A is selected from the group consisting of $C_{5-9}$ cycloalkenyl, 5- to 9-membered heterocycloalkenyl, phenyl, and 5- to 6-membered heteroaryl; ring A is selected from the group consisting of $C_{5-7}$ cycloalkenyl, 5- to 9-membered heterocycloalkenyl, phenyl, and 5- to 6-membered heteroaryl; ring A is selected from the group consisting of 5- to 9-membered heterocycloalkenyl, phenyl, and 5- to 6-membered heteroaryl; or

ring A is selected from the group consisting of 5- to 6-membered heterocycloalkenyl, phenyl, and 5-membered heteroaryl; or
ring A is selected from the group consisting of dihydropyrrolyl, tetrahydropyridinyl, dihydrooxazinyl, phenyl, pyrrolyl, pyrazolyl, and furanyl.

**32.** The compound, the moiety, the stereoisomer thereof, the derivative thereof, or the pharmaceutically acceptable salt thereof according to claim 30 or 31, wherein the moiety

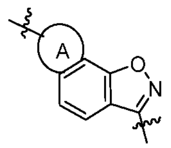

is selected from the group consisting of

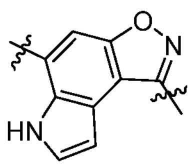 , 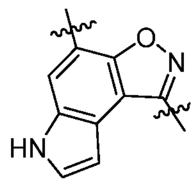 , 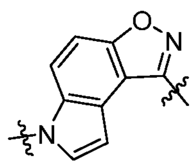 , 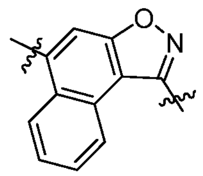 , 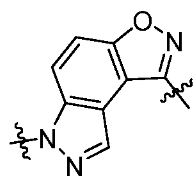 ,

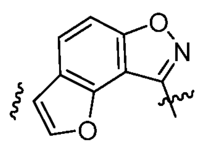 , 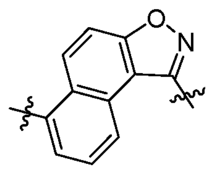 , 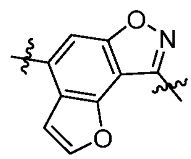 , 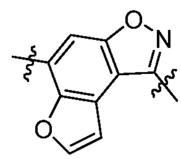 , 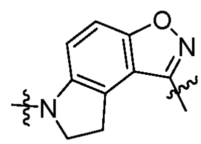 ,

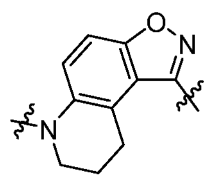 , and 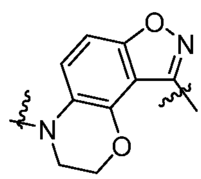 .

**33.** The compound, the moiety, the stereoisomer thereof, the derivative thereof, or the pharmaceutically acceptable salt thereof according to any one of claims 30-32, wherein L' is selected from the group consisting of a bond and $-CH_2-$.

**34.** The compound, the moiety, the stereoisomer thereof, the derivative thereof, or the pharmaceutically acceptable salt thereof according to any one of claims 30-33, wherein $Cy^3$ is selected from the group consisting of $C_{3-6}$ cycloalkyl and 4- to 6-membered heterocycloalkyl; or

$Cy^3$ is selected from the group consisting of $C_{4-6}$ cycloalkyl, 4-membered heterocycloalkyl, and 6-membered heterocycloalkyl; or
$Cy^3$ is selected from the group consisting of cyclobutyl, cyclopentyl, cyclohexyl, azetidinyl, and piperidinyl.

**35.** The compound, the moiety, the stereoisomer thereof, the derivative thereof, or the pharmaceutically acceptable salt thereof according to any one of claims 30-34, wherein $R^2$ is selected from the group consisting of -CHO, OH, and

$C_{1-3}$ alkyl substituted with one or more OH or $NH_2$; or

$R^2$ is selected from the group consisting of -CHO, OH, and methyl substituted with one or more OH; or
$R^2$ is selected from the group consisting of -CHO, OH, and $-CH_2OH$.

**36.** The compound, the moiety, the stereoisomer thereof, the derivative thereof, or the pharmaceutically acceptable salt thereof according to any one of claims 30-35, wherein p is selected from the group consisting of 0, 1 and 2; or

p is selected from the group consisting of 0 and 1; or
p is selected from 0; or, p is selected from 1.

**37.** The compound, the moiety, the stereoisomer thereof, the derivative thereof, or the pharmaceutically acceptable salt thereof according to claim 30, wherein the structural moiety

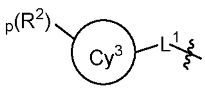

is selected from the group consisting of

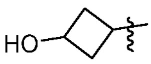, 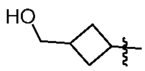, 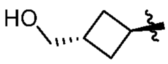, 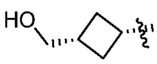, 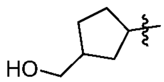, 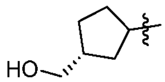,

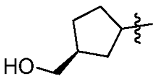, 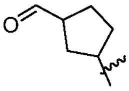, 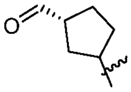, 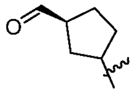, 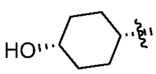, 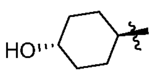, 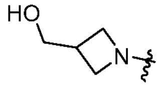,

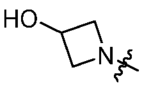, 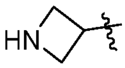, 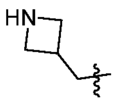, and 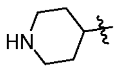.

**38.** A compound of the following formula, a moiety, a stereoisomer thereof, a derivative thereof, or a pharmaceutically acceptable salt thereof,

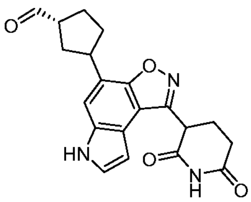
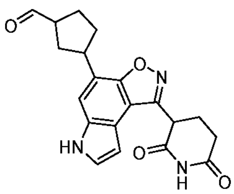
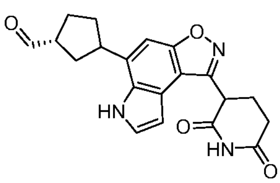
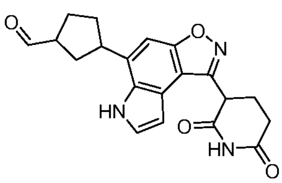

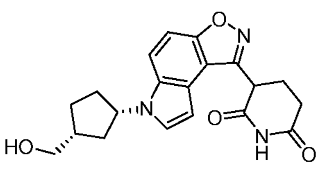

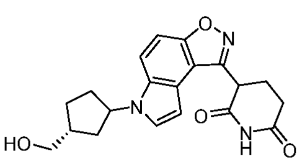
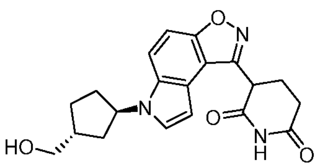
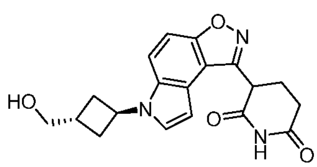

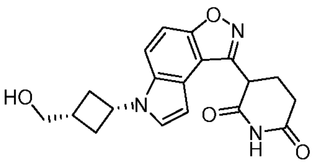 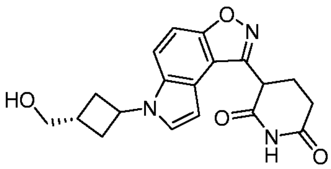 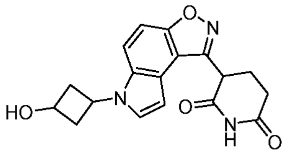 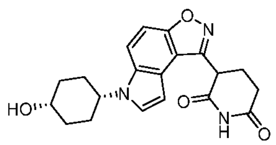

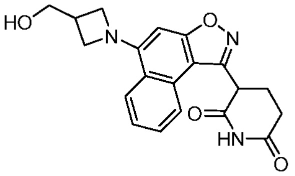 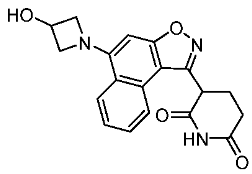 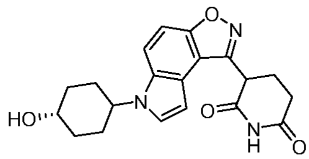 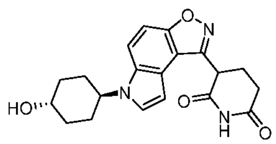

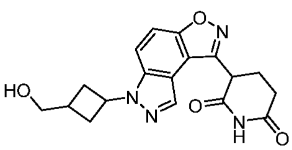 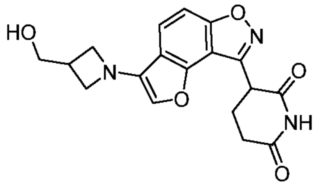 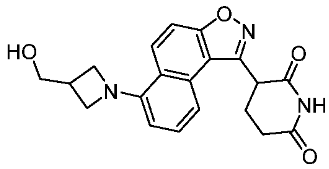 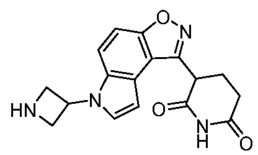

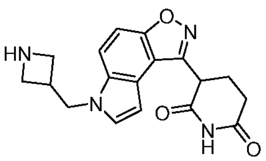 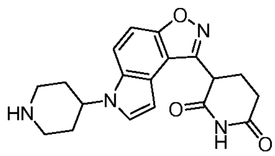 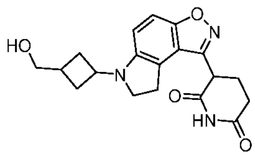 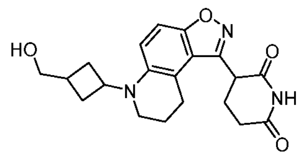

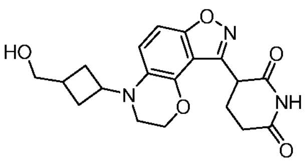 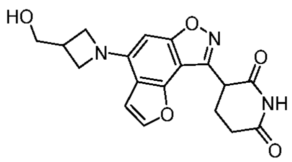 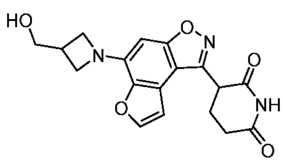 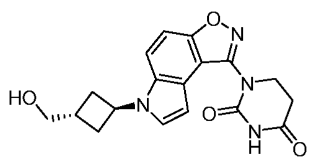

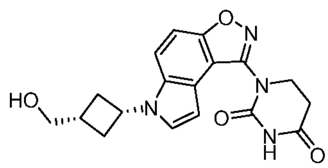 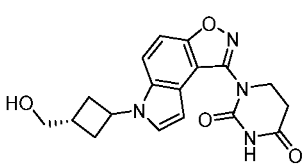 or 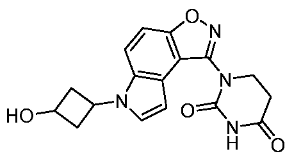.

**39.** Use of the compound, the moiety, the stereoisomer thereof, or the derivative thereof according to any one of claims 25-38 in a Protac molecule;

optionally use of the compound, the moiety, the stereoisomer thereof, or the derivative thereof for constituting part of a Protac molecule;

optionally the compound, the moiety, the stereoisomer thereof, or the derivative thereof present in the form of a Protac molecule;

optionally use of the compound, the moiety, the stereoisomer thereof, or the derivative thereof for degrading a protein, wherein the compound, the moiety, the stereoisomer thereof, or the derivative thereof degrading the protein in the form of a Protac molecule;

optionally use of the compound, the moiety, the stereoisomer thereof, or the derivative thereof for degrading a protein in the form of a Protac molecule; or

use of the compound, the moiety, the stereoisomer thereof, or the derivative thereof for preparing a protein degrader; or

use of the compound, the moiety, the stereoisomer thereof, or the derivative thereof for preparing a Protac molecule.

**40.** A pharmaceutical composition, comprising the compound, the moiety, the stereoisomer thereof, the derivative thereof, or the pharmaceutically acceptable salt thereof according to any one of claims 1-38.

**41.** Use of the compound, the moiety, the stereoisomer thereof, the derivative thereof, or the pharmaceutically acceptable salt thereof according to any one of claims 1-38 or the pharmaceutical composition thereof according to claim 40 for preparing a medicament for preventing or treating a disorder treated by degrading a target protein that binds to a targeting ligand.

**42.** Use of the compound, the moiety, the stereoisomer thereof, the derivative thereof, or the pharmaceutically acceptable salt thereof according to any one of claims 1-38 or the pharmaceutical composition thereof according to claim 40 for preparing a medicament for preventing or treating a disorder treated by binding to cereblon protein *in vivo.*

**43.** Use of the compound, the moiety, the stereoisomer thereof, the derivative thereof, or the pharmaceutically acceptable salt thereof according to any one of claims 1-38 or the pharmaceutical composition thereof according to claim 40 for preparing a medicament for preventing or treating a BTK-related disease, wherein

optionally, the BTK-related disease is selected from the group consisting of disorders treated by degrading a protein that binds to a BTK target protein ligand;
optionally, the BTK-related disease is selected from the group consisting of disorders treated by binding to cereblon protein *in vivo;*
optionally, the disease is selected from the group consisting of autoimmune diseases, inflammatory diseases, and cancer.

## INTERNATIONAL SEARCH REPORT

International application No.
**PCT/CN2022/132769**

**A. CLASSIFICATION OF SUBJECT MATTER**

C07D 498/04(2006.01)i; C07D 498/00(2006.01)i; C07D 491/04(2006.01)i; A61K 31/423(2006.01)i; A61K 31/343(2006.01)i; A61K 31/4523(2006.01)i; A61K 31/4525(2006.01)i; A61P 35/00(2006.01)i; A61P 37/00(2006.01)i

According to International Patent Classification (IPC) or to both national classification and IPC

**B. FIELDS SEARCHED**

Minimum documentation searched (classification system followed by classification symbols)

C07D,A61K,A61P

Documentation searched other than minimum documentation to the extent that such documents are included in the fields searched

Electronic data base consulted during the international search (name of data base and, where practicable, search terms used)

WPI, CNPAT, CAPLUS(STN), REG(STN), MARPAT(STN), CNKI, 万方, WANFANG: 蛋白降解, 调节剂, 靶蛋白, 苯并呋喃, 苯并异噁唑, 癌, 肿瘤, 免疫, 正大天晴, degradat+, targeted protein?, targeted chimera, protac, BTK, benzofuran, benzoisoxazole, cancer?, tumor?, tumour?, immun+, structural formula search

**C. DOCUMENTS CONSIDERED TO BE RELEVANT**

| Category* | Citation of document, with indication, where appropriate, of the relevant passages | Relevant to claim No. |
|---|---|---|
| X | WO 2021185291 A1 (MEDSHINE DISCOVERY INC.) 23 September 2021 (2021-09-23) claims 1, 9-11, 14 and 19-20 | 1-2, 39-43 |
| Y | WO 2021185291 A1 (MEDSHINE DISCOVERY INC.) 23 September 2021 (2021-09-23) claims 1, 9-11, 14 and 19-20 | 3-24, 39-43 |
| X | WO 2020048547 A1 (MEDSHINE DISCOVERY INC.) 12 March 2020 (2020-03-12) claims 1 and 11-15, and description, page 1 | 25-29, 39-43 |
| X | WO 2020048546 A1 (MEDSHINE DISCOVERY INC.) 12 March 2020 (2020-03-12) claims 1 and 11-15, and description, page 1 | 25-29, 39-43 |
| X | WO 2020048548 A1 (CHIA TAI TIANQING PHARMACEUTICAL GROUP CO., LTD. et al.) 12 March 2020 (2020-03-12) claims 1 and 18-23 | 25-43 |
| Y | CN 113412259 A (NURIX THERAPEUTICS, INC.) 17 September 2021 (2021-09-17) claims 1, 38, 48 and 102-106, and description, abstract | 3-24, 39-43 |
| Y | WO 2021113557 A1 (NURIX THERAPEUTICS, INC.) 10 June 2021 (2021-06-10) claims 1, 22, 93 and 104, and description, abstract | 3-24, 39-43 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 January 2023** | **17 January 2023** |
| Name and mailing address of the ISA/CN<br>**China National Intellectual Property Administration (ISA/CN)**<br>**No. 6, Xitucheng Road, Jimenqiao, Haidian District, Beijing 100088, China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/132769**

**C. DOCUMENTS CONSIDERED TO BE RELEVANT**

| Category* | Citation of document, with indication, where appropriate, of the relevant passages | Relevant to claim No. |
|---|---|---|
| A | WO 2021018018 A1 (BEIGENE, LTD. et al.) 04 February 2021 (2021-02-04) claim 1, and description, abstract | 1-43 |

Form PCT/ISA/210 (second sheet) (January 2015)

**INTERNATIONAL SEARCH REPORT**
**Information on patent family members**

International application No.
**PCT/CN2022/132769**

| Patent document cited in search report | Publication date (day/month/year) | Patent family member(s) | Publication date (day/month/year) |
|---|---|---|---|
| WO 2020048547 A1 | 12 March 2020 | JP 2022502355 A | 11 January 2022 |
| | | KR 20210057091 A | 20 May 2021 |
| | | US 2021317138 A1 | 14 October 2021 |
| | | EP 3848371 A1 | 14 July 2021 |
| WO 2020048546 A1 | 12 March 2020 | US 2021317109 A1 | 14 October 2021 |
| | | EP 3848363 A1 | 14 July 2021 |
| | | KR 20210056397 A | 18 May 2021 |
| | | JP 2021535184 A | 16 December 2021 |
| WO 2020048548 A1 | 12 March 2020 | CA 3111649 A1 | 12 March 2020 |
| | | AU 2019334065 A1 | 15 April 2021 |
| | | CN 115109074 A | 27 September 2022 |
| | | IL 281296 A | 29 April 2021 |
| | | EP 3848367 A1 | 14 July 2021 |
| | | BR 112021004269 A2 | 25 May 2021 |
| | | CN 112533916 A | 19 March 2021 |
| | | US 2022119376 A1 | 21 April 2022 |
| | | KR 20210057767 A | 21 May 2021 |
| | | JP 2021536480 A | 27 December 2021 |
| | | SG 11202102271 W A | 29 April 2021 |
| CN 113412259 A | 17 September 2021 | AU 2019360928 A1 | 13 May 2021 |
| | | BR 112021007115 A2 | 20 July 2021 |
| | | WO 2020081450 A1 | 23 April 2020 |
| | | KR 20210077719 A | 25 June 2021 |
| | | US 11479556 B1 | 25 October 2022 |
| | | EP 3867242 A1 | 25 August 2021 |
| | | IL 281904 A | 31 May 2021 |
| | | CA 3115526 A1 | 23 April 2020 |
| | | JP 2022508705 A | 19 January 2022 |
| WO 2021113557 A1 | 10 June 2021 | BR 112022010349 A2 | 16 August 2022 |
| | | CA 3160713 A1 | 10 June 2021 |
| | | US 2021198280 A1 | 01 July 2021 |
| | | CN 115003304 A | 02 September 2022 |
| | | KR 20220112273 A | 10 August 2022 |
| | | EP 4069237 A1 | 12 October 2022 |
| | | IL 293797 A | 01 August 2022 |
| | | AU 2020397920 A1 | 16 June 2022 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202111371514
- CN 202111667477
- CN 202210824373
- CN 202211261799
- CN 202211378992
- CN 202211413934

### Non-patent literature cited in the description

- Greene's Protective Groups in Organic Synthesis. John Wiley & Sons, Inc